# EUROPEAN PATENT APPLICATION

(11) **EP 4 663 630 A1**
(43) Date of publication of application: **17.12.2025**
(21) Application number: 24778407.7
(22) Date of filing: 27.03.2024
(51) Int. Cl.: C07D 235/26, A61K 31/4184, A61K 31/5377, A61K 31/4439, A61P 43/00, A61P 1/16, A61P 11/00, A61P 13/12, A61P 35/00, C07D 405/04

(54) **NOVEL BENZIMIDAZOLONE DERIVATIVE COMPOUND AS AUTOTAXIN INHIBITOR**

(30) Priority: 31.03.2023 KR 20230043118
(71) Applicant: LG Chem, Ltd., Seoul 07336 (KR)
(72) Inventor: CHOI, Eun Sil, Daejeon 34122 (KR); KIM, Young Kwan, Daejeon 34122 (KR); KANG, So Yeong, Daejeon 34122 (KR); BAEK, Ji Soo, Daejeon 34122 (KR); KOO, Jae Young, Daejeon 34122 (KR); KWON, Oh Hwan, Daejeon 34122 (KR)
(74) Representative: Hoffmann Eitle
(86) International application number: PCT/IB2024/052923
(87) International publication number: WO 2024/201308

(57) **Abstract**

The present invention relates to a novel benzimidazolone derivative compound as an autotaxin inhibitor.

## Description

### [TECHNICAL FIELD]

### [Cross-reference to Related Applications]

This application claims the benefit of Korean Patent Application No. 10-2023-0043118, filed on March 31, 2023, the disclosure of which is incorporated herein in its entirety by reference.

The present invention relates to a novel compound as an autotaxin inhibitor. Specifically, the present invention relates to a novel benzimidazolone derivative compound as an autotaxin inhibitor.

### [BACKGROUND ART]

Autotaxin (ATX), also referred to as ectonucleotide pyrophosphatase/phosphodiesterase 2 or lysophospholipase D, is an enzyme that causes an increase in lysophosphatidic acid (LPA), and is a secretory enzyme that plays an important role in the conversion of lysophosphatidylcholine (LPC) to LPA, which is a bioactive signaling molecule. The lysophosphatidic acid (LPA) is a bioactive lipid that exhibits its biological activity by transmitting signals through a specific G protein-coupled receptor (LPA1-6) and affects the migration, proliferation, and survival of various cell types. Since the level of plasma lysophosphatidic acid (LPA) is associated with autotaxin (ATX) activity, the autotaxin is an important source of extracellular lysophosphatidic acid (LPA).

In particular, recent studies have found that lysophosphatidic acid (LPA) is involved in various physiological activities, and in cancer patients, LPA excessively produced by the action of autotaxin promotes the growth, migration, metastasis, invasion, and colony formation of cancer cells. In particular, it has been reported that autotaxin is excessively produced and secreted in cancer cells of cancer patients such as renal cell carcinoma, ovarian cancer and breast cancer with severe metastasis, thyroid carcinoma, Hodgkin lymphoma, neuroblastoma, invasive glioblastoma multiform, prostate cancer and skin cancer (melanoma), and the secreted autotaxin converts LPC into LPA, and the produced LPA is about 40 times that of a normal person, thereby promoting the growth, metastasis, and invasion of various cancer cells.

In addition, the LPA produced by autotaxin binds to receptors present on cells to induce angiogenesis and sclerosis. It is known that the concentration of LPA and LPA receptors is increased in various diseases such as fibrosis disease, proliferative disease, inflammatory disease, autoimmune disease, respiratory disease, cardiovascular disease, neurodegenerative disease, skin disease, and diseases associated with abnormal angiogenesis.

Therefore, an inhibitor targeting autotaxin, which is an enzyme involved in the production of lysophosphatidic acid (LPA), is expected to have the potential to treat various diseases, including cancer and fibrosis. However, there is a limitation in that the existing autotaxin inhibitor has a low *in vivo* exposure, and thus it is difficult to exert a medicinal effect, or it competes with lysophosphatidylcholine (LPC), which is a substrate of the autotaxin enzyme, and thus the autotaxin inhibitory activity decreases. Accordingly, there is a need to develop a novel autotaxin inhibitor that shows excellent inhibitory activity and has high *in vivo* exposure.

### [DISCLOSURE OF THE INVENTION]

### [TECHNICAL PROBLEM]

An aspect of the present invention provides a novel compound having an autotaxin inhibitory activity.

Another aspect of the present invention provides a compound useful for the prevention or treatment of autotaxin-mediated diseases.

### [TECHNICAL SOLUTION]

According to an aspect of the present invention, there is provided a compound having a chemical structure of Formula 1 below or a pharmaceutically acceptable salt thereof.

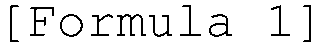

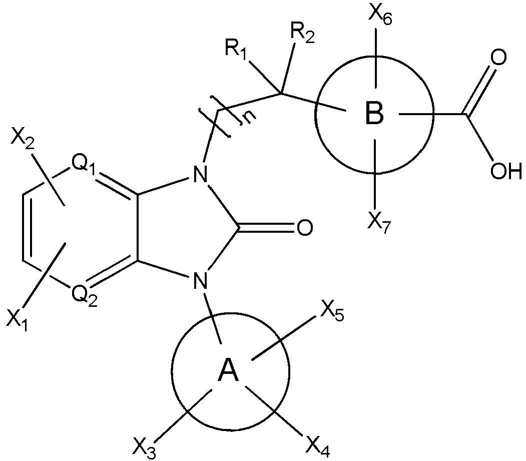

According to another aspect of the present invention, there is provided a pharmaceutical composition including: the compound of Formula 1 above or a pharmaceutically acceptable salt thereof; and a pharmaceutically acceptable carrier.

### [ADVANTAGEOUS EFFECTS]

The compounds according to the present invention have the excellent effect of inhibiting the activity of autotaxin, which is an enzyme involved in the production of lysophosphatidic acid, and thus can be effectively used as active ingredients of a pharmaceutical composition for the prevention or treatment of autotaxin-mediated diseases.

However, the effects of the present invention are not limited to the above-mentioned effects and other effects not mentioned will be clearly understood from the following description by a person skilled in the art.

### [MODE FOR CARRYING OUT THE INVENTION]

First, terms as used herein are defined.

The following terms herein have the following meanings unless otherwise indicated. Any term that is not defined has a meaning that is understood in the art.

Throughout the specification, a part "including" an element means that it may further include other elements rather than exclude other elements unless otherwise indicated.

In a structural formula of the specification, a symbol "-" that binds an atom and/or a group may mean a single bond, and a symbol "=" may mean a double bond. The symbols may be omitted, and may be indicated when necessary, for example, when a binding atom or a binding position is specified.

As used herein, the term "halogen" refers to fluorine (F), chlorine (Cl), bromine (Br), and iodine (I).

As used herein, the term "alkyl" refers to an aliphatic hydrocarbon group that does not include a double bond nor a triple bond, and unless otherwise indicated, the alkyl may have 1 to 20 carbon atoms, 1 to 19 carbon atoms, 1 to 18 carbon atoms, 1 to 17 carbon atoms, 1 to 16 carbon atoms, 1 to 15 carbon atoms, 1 to 14 carbon atoms, 1 to 13 carbon atoms, 1 to 12 carbon atoms, 1 to 11 carbon atoms, 1 to 10 carbon atoms, for example, 1 to 6 carbon atoms, and in particular, 1 to 4 carbon atoms, and may be linear or branched. Specific examples of the alkyl group may include a methyl group, an ethyl group, a propyl group, an n-propyl group, an isopropyl group, a butyl group, an n-butyl group, an isobutyl group, a tert-butyl group, a sec-butyl group, a 1-methylbutyl group, a 1-ethylbutyl group, a pentyl group, an n-pentyl group, an isopentyl group, a neopentyl group, a tert-pentyl group, a hexyl group, an n-hexyl group, a 1-methylpentyl group, a 2-methylpentyl group, a 4-methyl-2-pentyl group, a 3,3-dimethyl butyl group, a 2-ethylbutyl group, a heptyl group, an n-heptyl group, a 1-methylhexyl group, an octyl group, an n-octyl group, a *tert-*octyl group, a 1-methylheptyl group, a 2-ethylhexyl group, a 2-propylpentyl group, an n-nonyl group, a 2,2-dimethylheptyl group, a 1-ethylpropyl group, a 1,1-dimethylpropyl group, an isohexyl group, a 4-methylhexyl group, a 5-methylhexyl group, a benzyl group, etc., but are not limited thereto.

As used herein, the term "alkenyl" refers to an aliphatic hydrocarbon group including at least one double bond, and unless otherwise indicated, the alkenyl may have 2 to 20 carbon atoms, 2 to 19 carbon atoms, 2 to 18 carbon atoms, 2 to 17 carbon atoms, 2 to 16 carbon atoms, 2 to 15 carbon atoms, 2 to 14 carbon atoms, 2 to 13 carbon atoms, 2 to 12 carbon atoms, 2 to 11 carbon atoms, 2 to 10 carbon atoms, for example, 2 to 6 carbon atoms, and in particular, 2 to 4 carbon atoms, and may be linear or branched. Specific examples of the alkenyl group may include a vinyl group, a 1-propenyl group, an isopropenyl group, a 1-butenyl group, a 2-butenyl group, a 3-butenyl group, a 1-pentenyl group, a 2-pentenyl group, a 3-pentenyl group, a 3-methyl-1-butenyl group, a 1,3-butadienyl group, an allyl group, a 1-phenylvinyl-1-yl group, a 2-phenylvinyl-1-yl group, a 2,2-diphenylvinyl-1-yl group, a 2-phenyl-2-(naphthyl-1-yl)vinyl-1-yl group, a 2,2-bis(diphenyl-1-yl)vinyl-1-yl group, a stilbenyl group, a styrenyl group, and the like, but are not limited thereto.

As used herein, the term "alkynyl" refers to an aliphatic hydrocarbon group including at least one triple bond, and unless otherwise indicated, the alkynyl may have 2 to 20 carbon atoms, 2 to 19 carbon atoms, 2 to 18 carbon atoms, 2 to 17 carbon atoms, 2 to 16 carbon atoms, 2 to 15 carbon atoms, 2 to 14 carbon atoms, 2 to 13 carbon atoms, 2 to 12 carbon atoms, 2 to 11 carbon atoms, 2 to 10 carbon atoms, for example, 2 to 6 carbon atoms, and in particular, 2 to 4 carbon atoms, and may be linear or branched. Specific examples of the alkynyl group include an ethynyl group, a propynyl group, a butynyl group, a pentynyl group, a hexynyl group, and the like, but are not limited thereto.

In addition, as used herein, the term "cycloalkyl" refers to a cyclic aliphatic hydrocarbon group that does not include a double bond nor a triple bond, and unless otherwise indicated, the cycloalkyl may have 3 to 30 carbon atoms, 3 to 28 carbon atoms, 3 to 26 carbon atoms, 3 to 24 carbon atoms, 3 to 22 carbon atoms, 3 to 20 carbon atoms, 3 to 18 carbon atoms, 3 to 16 carbon atoms, 3 to 14 carbon atoms, 3 to 12 carbon atoms, 3 to 10 carbon atoms, for example, 3 to 8 carbon atoms, and in particular, 3 to 6 carbon atoms, and may be monocyclic or polycyclic. The polycyclic group refers to a group in which a cycloalkyl group is directly linked to or fused with another cyclic group, wherein the other cyclic group may be a cycloalkyl group, but may be another type of cyclic group, for example, a heterocycloalkyl group, an aryl group, a heteroaryl group, or the like. Specific examples of the cycloalkyl group may include a cyclopropyl group, a cyclobutyl group, a cyclopentyl group, a 3-methylcyclopentyl group, a 2,3-dimethylcyclopentyl group, a cyclohexyl group, a 3-methylcyclohexyl group, a 4-methylcyclohexyl group, a 2,3-dimethylcyclohexyl group, a 3,4,5-trimethylcyclohexyl group, a 4-tert-butylcyclohexyl group, a cycloheptyl group, a cyclooctyl group, a cyclononyl group, a cyclodecyl group, a cycloundecyl group, a cyclododecyl group, a bicyclo[2.2.1]heptyl group, a bicyclo[2.2.2]octyl group, a bicyclo[3.2.2]nonyl group, a bicyclo[4.4.0]decyl group, a bicyclo[4.1.0]heptyl group, and the like, but are not limited thereto.

As used herein, the term "cycloalkenyl" refers to a cyclic aliphatic hydrocarbon group including at least one double bond, and unless otherwise indicated, the cycloalkenyl may have 3 to 30 carbon atoms, 3 to 28 carbon atoms, 3 to 26 carbon atoms, 3 to 24 carbon atoms, 3 to 22 carbon atoms, 3 to 20 carbon atoms, 3 to 18 carbon atoms, 3 to 16 carbon atoms, 3 to 14 carbon atoms, 3 to 12 carbon atoms, 3 to 10 carbon atoms, for example, 3 to 8 carbon atoms, and in particular, 3 to 6 carbon atoms, and may be monocyclic or polycyclic. The polycyclic group refers to a group in which a cycloalkenyl group is directly linked to or fused with other cyclic group, wherein the other cyclic group may be a cycloalkyl group, but may be another type of cyclic group, for example, a heterocycloalkyl group, an aryl group, a heteroaryl group, or the like. Specific examples of the cycloalkenyl group include a cyclopentenyl group, a cyclohexenyl group, a cyclopenta-1,3-dienyl group, a cycloheptenyl group, a cyclooctenyl group, a cycloocta-1,4-dienyl group, and the like, but are not limited thereto.

As used herein, the term "aryl" refers to an aromatic hydrocarbon group, and unless otherwise indicated, may have 6 to 30 carbon atoms, 6 to 28 carbon atoms, 6 to 26 carbon atoms, 6 to 24 carbon atoms, 6 to 22 carbon atoms, 6 to 20 carbon atoms, 6 to 18 carbon atoms, 6 to 16 carbon atoms, 6 to 14 carbon atoms, for example, 6 to 12 carbon atoms, and may be monocyclic or polycyclic. The polycyclic group refers to a group in which an aryl group is directly linked or fused with another cyclic group, wherein the other cyclic group may be an aryl group, but may be other types of cyclic groups such as a cycloalkyl group, a heterocycloalkyl group, and a heteroaryl group. In addition, the aryl group includes a spiro group. Specific examples of the aryl group may include a phenyl group, a biphenyl group, a triphenyl group, a naphthyl group, an anthryl group, a chrysenyl group, a phenanthrenyl group, a perylenyl group, a fluoranthenyl group, a triphenylenyl group, a phenalenyl group, a pyrenyl group, a tetracenyl group, a pentacenyl group, a fluorenyl group, an indenyl group, an acenaphthylenyl group, a benzofluorenyl group, a spirobifluorenyl group, a 2,3-dihydro-1H-indenyl group, a fused cyclic group thereof, and the like, but are not limited thereto.

As used herein, the terms "heterocycloalkyl," "heterocycloalkenyl," and "heteroaryl" mean that at least one atom constituting the above-described ring of cycloalkyl, cycloalkenyl, and aryl is replaced with a heteroatom such as O, S, Se, N, Si, or the like, respectively.

As used herein, the term "substitution" means that a hydrogen atom bonded to a carbon atom in a structure is replaced with another substituent, and the substituted position is not limited as long as it is a position at which the hydrogen atom is substituted, that is, a position at which a substituent can be substituted, and when the hydrogen atoms are substituted at two or more positions, two or more substituents may be the same as or different from each other.

As used herein, the term "pharmaceutically acceptable" means that it may be approved or is preferably approved by a regulatory agency of a Federal or a state government or listed in the U.S. Pharmacopoeia or other generally recognized pharmacopoeia for use in animals, more specifically in humans, since significant toxic effect can be avoided when used with a common medicinal dosage.

The term "pharmaceutically acceptable salt" as used herein refers to a salt of a compound of the present invention that is pharmaceutically acceptable and has a preferable biological or pharmacological activity of a parent compound. Examples of such salts include, but are not limited to, acid addition salts formed of inorganic acids (e.g., hydrochloric acid, hydrobromic acid, sulfuric acid, phosphoric acid, nitric acid, and the like), and salts formed of organic acids such as acetic acid, oxalic acid, tartaric acid, succinic acid, malic acid, fumaric acid, maleic acid, ascorbic acid, trifluoroacetic acid, benzoic acid, tannic acid, pamoic acid, alginic acid, polyglutamic acid, naphthalene sulfonic acid, naphthalene disulfonic acid, and poly-galacturonic acid. The compounds may also be administered as pharmaceutically acceptable quaternary salts known by those skilled in the art, and in particular, include chloride, bromide, iodide, -O-alkyl, toluenesulfonate, methylsulfonate, sulfonate, phosphate, or carboxylate (e.g., benzoate, succinate, acetate, glycolate, maleate, malate, fumarate, citrate, tartrate, ascorbate, cinnamoate, mandeloate, and diphenylacetate). The compound of the formula of the present invention may include not only pharmaceutically acceptable salts, but also all salts, hydrates, and solvates that can be prepared by typical methods.

The term "hydrate" as used herein refers to a compound of the present invention or a salt thereof that includes a stoichiometric or non-stoichiometric amount of water bound by non-covalent intermolecular forces.

The term "solvate" as used herein refers to a compound of the present invention or a salt thereof that includes a stoichiometric or non-stoichiometric amount of solvent bound by non-covalent intermolecular forces. Preferred solvents are volatile, non-toxic, and/or acceptable for administration to humans.

The term "prodrug" as used herein refers to a substance that is converted into a parent drug in vivo. This refers to a compound of the present invention that can hydrolyze, oxidize, or otherwise react under biological conditions (in vitro or in vivo) in order to provide an active compound, in particular the compound of the present invention. Examples of the prodrug include compounds that are biohydrolyzed to produce compounds of the present invention, including biohydrolyzable moieties such as biohydrolyzable amides, biohydrolyzable esters, biohydrolyzable carbamates, biohydrolyzable carbonates, biohydrolyzable ureides, and biohydrolyzable phosphate analogs, but are not limited to these specific embodiments. Such a prodrug includes compounds easily prepared on the basis of various known documents.

The term "isomer" as used herein refers to a compound of the present invention or a salt thereof that has the same chemical formula or molecular formula but is structurally or sterically different. Such isomers include all of structural isomers such as tautomers, R or S isomers having an asymmetric carbon center, stereoisomers such as geometric isomers (trans or cis), and enantiomers. In addition, all of these isomers and mixtures thereof also fall within the scope of the present invention.

As used herein, the phrase "pharmaceutically acceptable carrier" refers to a diluent, an adjuvant, an additive or a carrier administered with a compound of the present invention.

As used herein, "prevention" refers to a reduction in the risk of acquiring a disease or disorder (i.e., causing one or more clinical symptoms of the disease not to develop in an individual that is exposed to or predisposed to the disease but does not yet experience or display the symptoms of the disease).

As used herein, "treatment" refers to relieving a disease or disorder (i.e., arresting or reducing the progression of the disease or one or more clinical symptoms of the disease), improving one or more physical parameters which may not be discernible by the individual, or modulating the disease or disorder physically (e.g., stabilizing discernible symptoms), mentally (e.g., stabilizing physical parameters), or both.

Hereinafter, the present invention will be described in detail.

### 1. Novel Benzimidazolone Derivative Compound

According to an aspect of the present invention, there is provided a compound having a chemical structure of Formula 1 below, or a pharmaceutically acceptable salt, an isomer, a solvate, a hydrate, or a prodrug thereof:

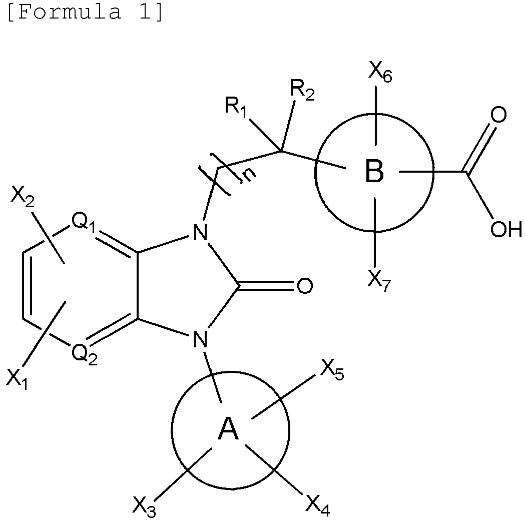

In Formula 1 above, Q₁ and Q₂ may each independently be any one selected from the group consisting of carbon and nitrogen.

In Formula 1 above, X₁ and X₂ may each independently be any one selected from the group consisting of: hydrogen; halogen; substituted or unsubstituted alkyl; substituted or unsubstituted cycloalkyl; and substituted or unsubstituted alkoxy. Specifically, X₁ and X₂ above may each independently be any one selected from the group consisting of: hydrogen; halogen; alkyl unsubstituted or substituted with halogen; cycloalkyl unsubstituted or substituted with halogen; and alkoxy unsubstituted or substituted with halogen. For example, X₁ and X₂ above may each independently be any one selected from the group consisting of: hydrogen; halogen; alkyl unsubstituted or substituted with halogen; unsubstituted cycloalkyl; and alkoxy substituted with halogen. In particular, X₁ and X₂ above may each independently be any one selected from the group consisting of: hydrogen; halogen; alkyl having 1 to 3 carbon atoms unsubstituted or substituted with halogen; unsubstituted cycloalkyl having 3 to 6 carbon atoms; and alkoxy having 1 to 3 carbon atoms substituted with halogen.

In Formula 1 above, A may be aryl; or heteroaryl. For example, A above may be any one selected from the group consisting of: monocyclic or polycyclic aryl; and monocyclic or polycyclic heteroaryl. Specifically, A above may be any one selected from the group consisting of: phenyl; benzodioxolyl; pyridinyl; pyrimidinyl; pyrazolyl; dihydroindenyl; isoindolinyl; benzothiazolyl; dihydroisobenzofuranyl; and dihydrobenzoxazinyl. In particular, A above may be any one selected from the group consisting of: phenyl; benzo[*d*][1,3]dioxolyl; pyridin-2-yl; pyridin-3-yl; pyrimidin-5-yl; 1*H*-pyrazol-4-yl; 2,3-dihydro-1*H*-inden-5-yl; isoindolin-5-yl; benzo[*d*]thiazol-2-yl; 1,3-dihydroisobenzofuran-5-yl; and 3,4-dihydro-2*H-*benzo[*b*] [1,4]oxazin-7-yl.

In Formula 1 above, X₃, X₄, and X₅ may each independently be any one selected from the group consisting of hydrogen; halogen; hydroxy; oxo; carboxy; substituted or unsubstituted alkoxy; substituted or unsubstituted thio; substituted or unsubstituted amino; substituted or unsubstituted carbamoyl; substituted or unsubstituted sulfonyl; substituted or unsubstituted alkyl; substituted or unsubstituted cycloalkyl; substituted or unsubstituted tricycloalkyl; substituted or unsubstituted heterocycloalkyl; substituted or unsubstituted oxoheterocycloalkyl; substituted or unsubstituted heterobicycloalkyl; substituted or unsubstituted cycloalkenyl; substituted or unsubstituted heterocycloalkenyl; substituted or unsubstituted oxaazabicycloalkyl; substituted or unsubstituted aryl; substituted or unsubstituted heteroaryl; substituted or unsubstituted heterocycloalkylcarbonyl; and substituted or unsubstituted heterobicycloalkylcarbonyl. Specifically, X₃, X₄, and X₅ above may each independently be any one selected from the group consisting of: hydrogen; halogen; hydroxy; oxo; carboxy; substituted or unsubstituted alkoxy; alkylthio; substituted or unsubstituted amino; substituted or unsubstituted carbamoyl; substituted or unsubstituted sulfonyl; substituted or unsubstituted alkyl; substituted or unsubstituted cycloalkyl; substituted or unsubstituted tricycloalkyl; substituted or unsubstituted morpholino; substituted or unsubstituted pyrrolidinyl; substituted or unsubstituted azetidinyl; substituted or unsubstituted piperazinyl; substituted or unsubstituted piperidinyl; substituted or unsubstituted oxazepanyl; substituted or unsubstituted oxetanyl; substituted or unsubstituted tetrahydrofuranyl; substituted or unsubstituted tetrahydropyranyl; substituted or unsubstituted oxopiperidinyl; substituted or unsubstituted oxooxazolidinyl; substituted or unsubstituted oxooxaazaspiroalkanyl; substituted or unsubstituted azaspiroalkanyl; substituted or unsubstituted oxaazaspiroalkanyl; substituted or unsubstituted diazaspiroalkanyl; substituted or unsubstituted cycloalkenyl; substituted or unsubstituted dihydropyranyl; substituted or unsubstituted dihydrofuranyl; substituted or unsubstituted oxaazabicycloalkyl; substituted or unsubstituted phenyl; substituted or unsubstituted oxadiazolyl; substituted or unsubstituted pyrazolyl; substituted or unsubstituted pyrrolidinecarbonyl; substituted or unsubstituted piperazinecarbonyl; substituted or unsubstituted piperidinecarbonyl; substituted or unsubstituted morpholinecarbonyl; substituted or unsubstituted azetidinecarbonyl; and substituted or unsubstituted oxaazaspiroalkanecarbonyl. The substituted functional groups in X₃, X₄, and X₅ above may each independently be substituted with at least one selected from the group consisting of: halogen; hydroxy; alkoxy; alkoxycarbonyl; sulfonyl; alkylsulfonyl; haloalkylsulfonyl; oxo; alkyl; haloalkyl; hydroxyalkyl; cycloalkyl; halocycloalkyl; heterocycloalkyl; alkyloxoheterocycloalkyl; aryl; heteroaryl; haloalkylheteroaryl; acetyl; alkylcarbonyl; haloalkylcarbonyl; cycloalkylcarbonyl; halocycloalkylcarbonyl; heterocycloalkylcarbonyl; haloheterocycloalkylcarbonyl; hydroxyheterocycloalkylcarbonyl; alkylheterocycloalkylcarbonyl; alkylamino; cycloalkylamino; cycloalkylalkylamino; and alkylaminocarbonyl. For example, X₃, X₄, and X₅ above may each independently be any one selected from the group consisting of: hydrogen; halogen; hydroxy; oxo; carboxy; methoxy; ethoxy; propoxy; isopropoxy; cyclobutylmethoxy; cyclopropylmethoxy; methylthio; dimethylamino; isobutyrylamido; methylsulfonamido; (2,2,2-trifluoroethyl)sulfonamido; methylcarbamoyl; dimethylcarbamoyl; (2,2,2-trifluoroethyl)carbamoyl; methylsulfonyl; morpholinosulfonyl; piperidin-1-ylsulfonyl; pyrrolidin-1-ylsulfonyl; *N,N*-dimethylsulfamoyl; *N-*cyclopropylsulfamoyl; *N*-(cyclobutylmethyl)sulfamoyl; methyl; propyl; isopropyl; *tert*-butyl; butyl; 2-hydroxypropan-2-yl; trifluoromethyl; 2,2,2-trifluoroethyl; 2-hydroxy-2-methylpropyl; 2-fluoro-2-methylpropyl; 1-hydroxy-2-methylpropan-2-yl; 1-fluoro-2-methylpropan-2-yl; (2-oxopyrrolidin-1-yl)methyl; ((3-(trifluoromethyl)-1*H*-pyrazol-4-yl)methyl; (4-(trifluoromethyl)-1*H*-imidazol-1-yl)methyl; (3-methyl-2-oxo-imidazolin-1-yl)methyl; oxetan-3-ylmethyl; 2-( dimethylamino)-2-oxoethyl; 2-oxo-2-(pyrrolidin-1-yl)ethyl; morpholinomethyl; 2-morpholino-2-oxoethyl; 2-(4,4-difluoropiperidin-1-yl)-2-oxoethyl; 2-(3-hydroxyazetidin-1-yl)-2-oxoethyl; 2-(2-methylmorpholino)-2-oxoethyl; 2-(3-methylmorpholino)-2-oxoethyl; cyclopropylmethyl; cyclobutylmethyl; cyclopropyl; 1-(hydroxymethyl)cyclopropyl; 1-(hydroxymethyl)cyclobutyl; 1-hydroxycyclobutyl; cyclohexyl; (3r,5r,7r)-adamantan-1-yl; morpholino; 3-methyloxetan-3-yl; 3-hydroxyoxetan-3-yl; tetrahydro-2*H*-pyran-4-yl; tetrahydrofuran-3-yl; 2-oxo-piperidin-1-yl; pyrrolidin-1-yl; 3-hydroxyazetidin-1-yl; 3,3-difluoroazetidin-1-yl; 3,3-difluoropyrrolidin-1-yl; 4-methylpiperazin-1-yl; 4,4-difluoropiperidin-1-yl; 4-(cyclopropanecarbonyl)piperazin-1-yl; 4-(cyclobutanecarbonyl)piperazin-1-yl; 4-(isopropoxycarbonyl)piperazin-1-yl; 4-(4,4-difluorocyclohexane-1-carbonyl)piperazin-1-yl; 1-(oxetan-3-yl)piperidin-4-yl; 1-(cyclopropanecarbonyl)piperidin-4-yl; 1-(cyclobutanecarbonyl)piperidin-4-yl; 1-propionylpiperidin-4-yl; 4-propionylpiperazin-1-yl; 3,6-dihydro-2*H*-pyran-4-yl; 2,5-dihydrofuran-3-yl; 2-azaspiro[3.3]heptan-2-yl; 7-oxa-2-azaspiro[3.5]nonan-2-yl; 2-oxa-6-azaspiro[3.3]heptan-6-yl; 2-oxa-5-azabicyclo[2.2.1]heptan-5-yl; 8-oxa-3-azabicyclo[3.2.1]octan-3-yl; 1,4-oxazepan-4-yl; 7-acetyl-2,7-diazaspiro[3.5]nonan-2-yl; 7-(tert-butoxycarbonyl)-2,7-diazaspiro[3.5]nonan-2-yl; 4,4-dimethyl-2-oxo-oxazolidin-3-yl; 5-oxo-6-oxa-4-azaspiro[2.4]heptan-4-yl; cyclohexen-1-yl; 4-methyl-cyclohexen-1-yl; 4',4'-dimethyl-cyclohexen-1-yl; phenyl; chlorophenyl; 5-cyclopropyl-1,3,4-oxadiazol-2-yl; 5-cyclopentyl-1,3,4-oxadiazol-2-yl; 5-cyclohexyl-1,3,4-oxadiazol-2-yl; 5-cycloheptyl-1,3,4-oxadiazol-2-yl; 5-(tetrahydro-2*H*-pyran-4-yl)-1,3,4-oxadiazol-2-yl; 5-(4,4-difluorocyclohexyl)-1,3,4-oxadiazol-2-yl; 1-propyl-1*H-*pyrazol-4-yl; piperidine-1-carbonyl; morpholine-4-carbonyl; 3-methylmorpholine-4-carbonyl; pyrrolidine-1-carbonyl; azetidine-1-carbonyl; 3-hydroxyazetidine-1-carbonyl; 3,3-difluoroazetidine-1-carbonyl; 3-methoxypyrrolidine-1-carbonyl; 3,3-difluoropyrrolidine-1-carbonyl; 4-methylpiperazine-1-carbonyl; 4-hydroxypiperidine-1-carbonyl; 4-methoxypiperidine-1-carbonyl; 4,4-difluoropiperidine-1-carbonyl; 7-oxa-2-azaspiro[3.5]nonane-2-carbonyl; and 2-oxa-6-azaspiro[3.3]heptane-6-carbonyl.

In Formula 1 above, n may be an integer of 0 to 3. Specifically, n may be an integer of 0 to 2, for example, n may be 0 or 1.

In Formula 1 above, R₁ and R₂ may each independently be any one selected from the group consisting of: hydrogen; a substituted or unsubstituted alkyl group; and a substituted or unsubstituted cycloalkyl group, or R₁ and R₂ above may be linked to each other to form a substituted or unsubstituted hydrocarbon ring. Specifically, R₁ and R₂ above may each independently be hydrogen; or a substituted or unsubstituted alkyl group; or R₁ and R₂ above may be linked to each other to form any one selected from the group consisting of: substituted or unsubstituted cycloalkyl; substituted or unsubstituted heterocycloalkyl; substituted or unsubstituted aryl; and substituted or unsubstituted heterocycloaryl. For example, R₁ and R₂ above may each independently be hydrogen; or a substituted or unsubstituted alkyl group having 1 to 6 carbon atoms, or R₁ and R₂ above may be linked to each other to form substituted or unsubstituted cycloalkyl. In particular, R₁ and R₂ above may each independently be hydrogen; or methyl; or R₁ and R₂ above may be linked to each other to form a cycloalkyl having 3 to 6 carbon atoms.

In Formula 1 above, B may be aryl; or heteroaryl. Specifically, B above may be phenyl; or heteroaryl including at least one selected from the group consisting of N and O. For example, B above may be phenyl; pyridine; oxazole; or isoxazole.

In Formula 1 above, X₆ and X₇ may each independently be any one selected from the group consisting of: hydrogen; halogen; hydroxy; oxo; substituted or unsubstituted alkoxy; substituted or unsubstituted amino; substituted or unsubstituted alkyl; and substituted or unsubstituted cycloalkyl. Specifically, X₆ and X₇ above may each independently be any one selected from the group consisting of: hydrogen; halogen; hydroxy; oxo; and substituted or unsubstituted alkyl. For example, X₆ and X₇ above may each independently be hydrogen or halogen.

Meanwhile, unless specifically specified, the substituted functional groups in X₁, X₂, X₆, X₇, R₁, and R₂ above may be substituted with at least one selected from the group consisting of halogen, alkyl, hydroxyalkyl, alkoxyalkyl, haloalkyl, cycloalkyl, heterocycloalkyl, -OR₃, -(CO)-R₃, - (CO)-OR₃, alkylamino, and oxo. Here, R₃ may be hydrogen or alkyl.

In particular, IUPAC names of specific examples of the compounds having the chemical structure of Formula 1 above may be presented in Table 1 below, but are not limited thereto.

**[Table 1]**

| **Examples** | **Compound IUPAC Name)** |
|---|---|
| 1 | 3-((5-chloro-2-oxo-3-phenyl-2,3-dihydro-1*H*- |
| | benzo[*d*]imidazol-1-yl)methyl)benzoic acid |
| 2 | 3-((5-chloro-2-oxo-3-(4-propylphenyl)-2,3-dihydro-1*H-*benzo[*d*]imidazol-1-yl)methyl)benzoic acid |
| 3 | 3-((5-chloro-3-(4-fluorophenyl)-2-oxo-2,3-dihydro-*1H*-benzo[*d*]imidazol-1-yl)methyl)benzoic acid |
| 4 | 3-((5-chloro-2-oxo-3-(4-(trifluoromethyl)phenyl)-2,3-dihydro-1*H*-benzo[*d*]imidazol-1-yl)methyl)benzoic acid |
| 5 | 3-((5-chloro-3-(4-methoxyphenyl)-2-oxo-2,3-dihydro-1*H*-benzo[*d*]imidazol-1-yl)methyl)benzoic acid |
| 6 | 3-((3-([1,1'-biphenyl]-3-yl)-5-chloro-2-oxo-2,3-dihydro-1*H-*benzo[*d*]imidazol-1-yl)methyl)benzoic acid |
| 7 | 3-((5-chloro-3-(4'-chloro-[1,1'-biphenyl]-4-yl)-2-oxo-2,3-dihydro-*1H-*benzo[*d*]imidazol-1-yl)methyl)benzoic acid |
| 8 | 3-((5-chloro-2-oxo-3-(*p-*tolyl)-2,3-dihydro-*1H-*benzo[*d*]imidazol-1-yl)methyl)benzoic acid |
| 9 | 3-((3-(benzo[*d*][1,3]dioxol-5-yl)-5-chloro-2-oxo-2,3-dihydro-*1H*-benzo[*d*]imidazol-1-yl)methyl)benzoic acid |
| 10 | 3-((5-chloro-3-(4-(dimethylamino)phenyl)-2-oxo-2,3-dihydro-*1H-*benzo[*d*]imidazol-1-yl)methyl)benzoic acid |
| 11 | 3-((5-chloro-3-(4-isobutylamidophenyl)-2-oxo-2,3-dihydro-*1H-*benzo[*d*]imidazol-1-yl)methyl)benzoic acid |
| 12 | 3-((3-(4-(*tert*-butyl)phenyl)-5-chloro-2-oxo-2,3-dihydro-*1H-*benzo[*d*]imidazol-1-yl)methyl)benzoic acid |
| 13 | 3-((5-chloro-3-(4-hydroxyphenyl)-2-oxo-2,3-dihydro-*1H*-benzo[*d*]imidazol-1-yl)methyl)benzoic acid |
| 14 | 3-((5-chloro-3-(4-isopropoxyphenyl)-2-oxo-2,3-dihydro-*1H*-benzo[*d*]imidazol-1-yl)methyl)benzoic acid |
| 15 | 3-((3-([1,1'-biphenyl]-4-yl)-5-chloro-2-oxo-2,3-dihydro-*1H-*benzo[*d*]imidazol-1-yl)methyl)benzoic acid |
| 16 | 3-((3-(4-butylphenyl)-5-chloro-2-oxo-2,3-dihydro-*1H-*benzo[*d*]imidazol-1-yl)methyl)benzoic acid |
| 17 | 3-((5-chloro-3-(4-isopropylphenyl)-2-oxo-2,3-dihydro-*1H*-benzo[*d*]imidazol-1-yl)methyl)benzoic acid |
| 18 | 3-((5-chloro-3-(4-(2-hydroxypropan-2-yl)phenyl)-2-oxo-2,3-dihydro-*1H-*benzo[*d*]imidazol-1-yl)methyl)benzoic acid |
| 19 | 3-((5-chloro-3-(4-(methylsulfonamido)phenyl)-2-oxo-2,3-dihydro-*1H-*benzo[*d*]imidazol-1-yl)methyl)benzoic acid |
| 20 | 3-((5-chloro-3-(4-ethoxyphenyl)-2-oxo-2,3-dihydro-*1H*-benzo[d]imidazol-1-yl)methyl)benzoic acid |
| 21 | 3-((5-chloro-3-(4-(dimethylcarbamoyl)phenyl)-2-oxo-2,3-dihydro-*1H-*benzo[*d*]imidazol-1-yl)methyl)benzoic acid |
| 22 | 3-((5-chloro-3-(4-(methylcarbamoyl)phenyl)-2-oxo-2,3-dihydro-*1H-*benzo[*d*]imidazol-1-yl)methyl)benzoic acid |
| 23 | 3-((5-chloro-2-oxo-3-(4-((2,2,2-trifluoroethyl)carbamoyl)pheny l)-2,3-dihydro-*1H-*benzo[*d*]imidazol-1-yl)methyl)benzoic acid |
| 24 | 3-((5-chloro-4-fluoro-3-(4-morpholinophenyl)-2-oxo-2,3-dihydro-*1H*-benzo[*d*]imidazol-1-yl)methyl)benzoic acid |
| 25 | 3-((5-chloro-3-(4-(dimethylamino)phenyl)-4-fluoro-2-oxo-2,3-dihydro-*1H-*benzo[*d*]imidazol-1-yl)methyl)benzoic acid |
| 26 | 3-((5-chloro-2-oxo-3-(4-((2,2,2-trifluoroethyl)sulfonamido)phe nyl)-2,3-dihydro-*1H-*benzo[*d*]imidazol-1-yl)methyl)benzoic acid |
| 27 | 3-((5-chloro-3-(4-(cyclobutylmethoxy)phenyl)-2-oxo-2,3-dihydro-*1H-*benzo[*d*]imidazol-1-yl)methyl)benzoic acid |
| 28 | 3-((5-chloro-2-oxo-3-(4-(2-oxo-piperidin-1-yl)phenyl)-2,3-dihydro-*1H-*benzo[*d*]imidazol-1-yl)methyl)benzoic acid |
| 29 | 3-((5-chloro-2-oxo-3-(4-(pyrrolidin-1-yl)phenyl)-2,3-dihydro-*1H*-benzo[*d*]imidazol-1-yl)methyl)benzoic acid |
| 30 | 3-((5-chloro-3-(4-(cyclobutylmethoxy)-3-fluorophenyl)-2-oxo-2,3-dihydro-*1H*-benzo[*d*]imidazol-1-yl)methyl)benzoic acid |
| 31 | 3-(1-(5-chloro-2-oxo-3-(4-propylphenyl)-2,3-dihydro-*1H-*benzo[*d*]imidazol-1-yl)cyclopropyl)benzoic acid |
| 32 | 3-((5-chloro-2-oxo-3-phenyl-2,3-dihydro-*1H-*benzo[*d*]imidazol-1-yl)methyl)-4-fluorobenzoic acid |
| 33 | 3-((5-chloro-2-oxo-3-phenyl-2,3-dihydro-1*H-*benzo[*d*]imidazol-1-yl)methyl)-2-fluorobenzoic acid |
| 34 | 3-((5-chloro-2-oxo-3-phenyl-2,3-dihydro-1*H-*benzo[*d*]imidazol-1-yl)methyl)-5-fluorobenzoic acid |
| 35 | 5-((5-chloro-2-oxo-3-phenyl-2,3-dihydro-1*H-*benzo[d*]*imidazol-1-yl)methyl)-2-fluorobenzoic acid |
| 36 | 3-((5-chloro-3-(4-(dimethylcarbamoyl)-3-fluorophenyl)-2-oxo-2,3-dihydro-*1H*-benzo[*d*]imidazol-1-yl)methyl)benzoic acid |
| 37 | 3-((6-chloro-3-(4-(dimethylamino)phenyl)-4-fluoro-2-oxo-2,3-dihydro-*1H-*benzo[*d*]imidazol-1-yl)methyl)benzoic acid |
| 38 | 3-((5-chloro-3-(4-(dimethylcarbamoyl)phenyl)-2-oxo-2,3-dihydro-*1H-*benzo[*d*]imidazol-1-yl)methyl)-2-fluorobenzoic acid |
| 39 | 3-((5-chloro-3-(4-(dimethylamino)phenyl)-2-oxo-2,3-dihydro-*1H-*benzo[d*]*imidazol-1-yl)methyl)-2-fluorobenzoic acid |
| 40 | 3-((5-chloro-3-(4-isopropoxyphenyl)-2-oxo-2,3-dihydro-*1H*-benzo[*d*]imidazol-1-yl)methyl)-2-fluorobenzoic acid |
| 41 | 3-((5-chloro-3-(4-(methylsulfonamido)phenyl)-2-oxo-2,3-dihydro-*1H-*benzo[*d*]imidazol-1-yl)methyl)-2-fluorobenzoic acid |
| 42 | 3-((5-chloro-3-(4-(cyclopropylmethoxy)phenyl)-2-oxo-2,3-dihydro-*1H-*benzo[*d*]imidazol-1-yl)methyl)benzoic acid |
| 43 | 3-((5-chloro-2-oxo-3-(4-(piperidine-1-carbonyl)phenyl)-2,3-dihydro-*1H*-benzo[*d*]imidazol-1-yl)methyl)benzoic acid |
| 44 | 3-((5-chloro-3-(4-(morpholine-4-carbonyl)phenyl)-2-oxo-2,3-dihydro-*1H*-benzo[*d*]imidazol-1-yl)methyl)benzoic acid |
| 45 | 3-((5-chloro-2-oxo-3-(4-(pyrrolidine-1-carbonyl)phenyl)-2,3-dihydro-*1H*-benzo[*d*]imidazol-1-yl)methyl)benzoic acid |
| 46 | 4-(3-(3-carboxybenzyl)-6-chloro-2-oxo-2,3-dihydro-*1H-*benzo[*d*]imidazol-1-yl)-3-fluorobenzoic acid |
| 47 | 3-((5-chloro-3-(4-morpholinophenyl)-2-oxo-2,3-dihydro-*1H*-benzo[*d*]imidazol-1-yl)methyl)benzoic acid |
| 48 | 5-((5-chloro-4-fluoro-3-(4-morpholinophenyl)-2-oxo-2,3-dihydro-*1H*-benzo[*d*]imidazol-1-yl)methyl)-2-fluorobenzoic acid |
| 49 | 3-((5-chloro-4-fluoro-3-(4-morpholinophenyl)-2-oxo-2,3-dihydro-*1H*-benzo[*d*]imidazol-1-yl)methyl)-5-fluorobenzoic acid |
| 50 | 3-((5-chloro-4-fluoro-3-(4-morpholinophenyl)-2-oxo-2,3-dihydro-*1H*-benzo[*d*]imidazol-1-yl)methyl)-4-fluorobenzoic acid |
| 51 | 3-((5-chloro-4-fluoro-3-(4-morpholinophenyl)-2-oxo-2,3-dihydro-*1H*-benzo[*d*]imidazol-1-yl)methyl)-2-fluorobenzoic acid |
| 52 | 6-((5-chloro-4-fluoro-3-(4-morpholinophenyl)-2-oxo-2,3-dihydro-*1H*-benzo[*d*]imidazol-1-yl)methyl)picolinic acid |
| 53 | 3-((5-chloro-4-fluoro-3-(4-(3-hydroxyazetidin-1-yl)phenyl)-2-oxo-2,3-dihydro-*1H-*benzo[*d*]imidazol-1-yl)methyl)benzoic acid |
| 54 | 3-((5-chloro-3-(4-(dimethylcarbamoyl)phenyl)-7-fluoro-2-oxo-2,3-dihydro-*1H-*benzo[*d*]imidazol-1-yl)methyl)benzoic acid |
| 55 | 3-((5-chloro-3-(4-(methylthio)phenyl)-2-oxo-2,3-dihydro-*1H*-benzo[*d*]imidazol-1-yl)methyl)-2-fluorobenzoic acid |
| 56 | 3-((5-chloro-2-oxo-3-(4-(pyrrolidine-1-carbonyl)phenyl)-2,3-dihydro-*1H*-benzo[*d*]imidazol-1-yl)methyl)-2-fluorobenzoic acid |
| 57 | 3-((5-chloro-2-oxo-3-(4-(piperidine-1-carbonyl)phenyl)-2,3-dihydro-*1H*-benzo[*d*]imidazol-1-yl)methyl)-2-fluorobenzoic acid |
| 58 | 3-((3-(4-(azetidine-1-carbonyl)phenyl)-5-chloro-2-oxo-2,3-dihydro-*1H-*benzo[*d*]imidazol-1-yl)methyl)benzoic acid |
| 59 | 3-((5-chloro-3-(4-(3-methoxypyrrolidine-1-carbonyl)phenyl)-2-oxo-2,3-dihydro-*1H*-benzo[*d*]imidazol-1-yl)methyl)benzoic acid |
| 60 | 3-((5-chloro-3-(4-(4-methylpiperazine-1-carbonyl)phenyl)-2-oxo-2,3-dihydro-*1H-*benzo[*d*]imidazol-1-yl)methyl)benzoic acid |
| 61 | 3-((5-chloro-3-(4-(4-hydroxypiperidine-1-carbonyl)phenyl)-2-oxo-2,3-dihydro-*1H*-benzo[*d*]imidazol-1-yl)methyl)benzoic acid |
| 62 | 3-((5-chloro-3-(4-(4-methoxypiperidine-1-carbonyl)phenyl)-2-oxo-2,3-dihydro-*1H*-benzo[*d*]imidazol-1-yl)methyl)benzoic acid |
| 63 | 3-(1-(5-chloro-2-oxo-3-(4-(pyrrolidine-1-carbonyl)phenyl)-2,3-dihydro-*1H*-benzo[*d*]imidazol-1-yl)cyclopropyl)benzoic acid |
| 64 | 3-(1-(5-chloro-2-oxo-3-(4-(piperidine-1-carbonyl)phenyl)-2,3-dihydro-*1H*-benzo[*d*]imidazol-1-yl)cyclopropyl)benzoic acid |
| 65 | 3-((5-chloro-3-(4-(dimethylamino)phenyl)-7-fluoro-2-oxo-2,3-dihydro-1*H-*benzo[*d*]imidazol-1-yl)methyl)-5-fluorobenzoic acid |
| 66 | 3-((5-chloro-3-(4-(dimethylamino)phenyl)-7-fluoro-2-oxo-2,3-dihydro-*1H-*benzo[*d*]imidazol-1-yl)methyl)benzoic acid |
| 67 | 3-(1-(5-chloro-2-oxo-3-(4-(pyrrolidine-1-carbonyl)phenyl)-2,3-dihydro-*1H*-benzo[*d*]imidazol-1-yl)cyclopropyl)-2-fluorobenzoic acid |
| 68 | 3-(1-(5-chloro-2-oxo-3-(4-(piperidine-1-carbonyl)phenyl)-2,3-dihydro-*1H*-benzo[*d*]imidazol-1-yl)cyclopropyl)-2-fluorobenzoic acid |
| 69 | 3-(1-(5-chloro-3-(4-(dimethylamino)phenyl)-2-oxo-2,3-dihydro-*1H-*benzo[*d*]imidazol-1-yl)cyclopropyl)benzoic acid |
| 70 | 3-(1-(5-chloro-3-(4-(dimethylamino)phenyl)-2-oxo-2,3-dihydro-*1H-*benzo[*d*]imidazol-1-yl)cyclopropyl)-2-fluorobenzoic acid |
| 71 | 3-(1-(5-chloro-3-(4-(dimethylcarbamoyl)phenyl)-2-oxo-2,3-dihydro-*1H-*benzo[*d*]imidazol-1-yl)cyclopropyl)benzoic acid |
| 72 | 3-(1-(5-chloro-3-(4-(dimethylcarbamoyl)phenyl)-2-oxo-2,3-dihydro-*1H-*benzo[*d*]imidazol-1-yl)cyclopropyl)-2-fluorobenzoic acid |
| 73 | 3-((5-chloro-2-oxo-3-(pyridin-3-yl)-2,3-dihydro-*1H-*benzo[*d*]imidazol-1-yl)methyl)benzoic acid |
| 74 | 3-((5-chloro-3-(6-fluoropyridin-3-yl)-2-oxo-2,3-dihydro-*1H*-benzo[*d*]imidazol-1-yl)methyl)benzoic acid |
| 75 | 3-((5-chloro-3-(6-ethoxypyridin-3-yl)-2-oxo-2,3-dihydro-*1H*-benzo[*d*]imidazol-1-yl)methyl)benzoic acid |
| 76 | 3-((5-chloro-2-oxo-3-(6-propoxypyridin-3-yl)-2,3-dihydro-*1H*-benzo[*d*]imidazol-1-yl)methyl)benzoic acid |
| 77 | 3-((5-chloro-3-(6-(3-hydroxyazetidin-1-yl)pyridin-3-yl)-2-oxo-2,3-dihydro-*1H-*benzo[d]imidazol-1-yl)methyl)benzoic acid |
| 78 | 3-((5-chloro-3-(6-morpholinopyridin-3-yl)-2-oxo-2,3-dihydro-*1H-*benzo[*d*]imidazol-1-yl)methyl)benzoic acid |
| 79 | 3-((5-chloro-3-(6-(dimethylamino)pyridin-3-yl)-2-oxo-2,3-dihydro-*1H-*benzo[*d*]imidazol-1-yl)methyl)benzoic acid |
| 80 | 3-((5-chloro-3-(6-(3,3-difluoropyrrolidin-1-yl)pyridin-3-yl)-2-oxo-2,3-dihydro-1*H*-benzo[*d*]imidazol-1-yl)methyl)benzoic acid |
| 81 | 3-((5-chloro-3-(6-(4-methylpiperazin-1-yl)pyridin-3-yl)-2-oxo-2,3-dihydro-*1H-*benzo[*d*]imidazol-1-yl)methyl)benzoic acid |
| 82 | 3-((5-chloro-3-(6-(4,4-difluoropiperidin-1-yl)pyridin-3-yl)-2-oxo-2,3-dihydro-*1H*-benzo[*d*]imidazol-1-yl)methyl)benzoic acid |
| 83 | 3-((5-chloro-3-(2-morpholinopyrimidin-5-yl)-2-oxo-2,3-dihydro-*1H-*benzo[*d*]imidazol-1-yl)methyl)benzoic acid |
| 84 | 3-((5-chloro-2-oxo-3-(1-propyl-*1H*-pyrazol-4-yl)-2,3-dihydro-*1H*-benzo[*d*]imidazol-1-yl)methyl)benzoic acid |
| 85 | 3-((5-cyclopropyl-2-oxo-3-(1-(tetrahydro-2*H*-pyran-4-yl)-*1H-*pyrazol-4-yl)-2,3-dihydro-*1H-*benzo[*d*]imidazol-1-yl)methyl)benzoic acid |
| 86 | 3-((5-chloro-2-oxo-3-(1-(tetrahydro-2*H*-pyran-4-yl)-*1H-*pyrazol-4-yl)-2,3-dihydro-*1H-*benzo[*d*]imidazol-1-yl)methyl)benzoic acid |
| 87 | 3-((5-chloro-2-oxo-3-(1-(2,2,2-trifluoroethyl)-*1H-*pyrazol-4-yl)-2,3-dihydro-*1H-*benzo[*d*]imidazol-1-yl)methyl)benzoic acid |
| 88 | 3-((5-chloro-4-fluoro-2-oxo-3-(1-propyl-*1H*-pyrazol-4-yl)-2,3-dihydro-*1H-*benzo[*d*]imidazol-1-yl)methyl)benzoic acid |
| 88 | 3-((5-chloro-3-(1-isopropyl-*1H*-pyrazol-4-yl)-2-oxo-2,3-dihydro-*1H*-benzo[*d*]imidazol-1-yl)methyl)benzoic acid |
| 90 | 3-((5-chloro-3-(1-cyclopropylmethyl-*1H*-pyrazol-4-yl)-2-oxo-2,3-dihydro-*1H-*benzo[*d*]imidazol-1-yl)methyl)benzoic acid |
| 91 | 3-((5-chloro-3-(1-cyclobutylmethyl-*1H*-pyrazol-4-yl)-2-oxo-2,3-dihydro-*1H-*benzo[*d*]imidazol-1-yl)methyl)benzoic acid |
| 92 | 3-((5-chloro-4-fluoro-2-oxo-3-(1-propyl-*1H*-pyrazol-4-yl)-2,3-dihydro-*1H-*benzo[d*]*imidazol-1-yl)methyl)-2-fluorobenzoic acid |
| 93 | 3-(1-(3-(4-(7-oxa-2-azaspiro[3.5]nonane-2-carbonyl)phenyl)-5-chloro-2-oxo-2,3-dihydro-*1H-*benzo[*d*]imidazol-1-yl)cyclopropyl)benzoic acid |
| 94 | 3-((5-chloro-3-(4-(4,4-difluoropiperidin-1-yl)phenyl)-2-oxo-2,3-dihydro-*1H*-benzo[*d*]imidazol-1-yl)methyl)benzoic acid |
| 95 | 3-((5-chloro-3-(4-(4-(cyclopropanecarbonyl)piperazi n-1-yl)phenyl)-2-oxo-2,3-dihydro-*1H*-benzo[*d*]imidazol-1-yl)methyl)benzoic acid |
| 96 | 3-((5-chloro-3-(4-(4-(isopropoxycarbonyl)piperazin-1-yl)phenyl)-2-oxo-2,3-dihydro-*1H*-benzo[*d*]imidazol-1-yl)methyl)benzoic acid |
| 97 | 3-((5-chloro-3-(4-(4-methylpiperazin-1-yl)phenyl)-2-oxo-2,3-dihydro-*1H-*benzo[*d*]imidazol-1-yl)methyl)benzoic acid |
| 98 | 3-(1-(5-chloro-3-(4-cyclohexylphenyl)-2-oxo-2,3-dihydro-*1H*-benzo[*d*]imidazol-1-yl)cyclopropyl)benzoic acid |
| 99 | 3-(1-(5-chloro-2-oxo-3-(4-(tetrahydro-2*H*-pyran-4-yl)phenyl)-2,3-dihydro-*1H-*benzo[*d*]imidazol-1-yl)cyclopropyl)benzoic acid |
| 100 | 3-((5-chloro-3-(4-(1-(cyclopropanecarbonyl)piperidi n-4-yl)phenyl)-2-oxo-2,3-dihydro-*1H*-benzo[*d*]imidazol-1-yl)methyl)benzoic acid |
| 101 | 3-((5-chloro-3-(4-(1-(cyclobutanecarbonyl)piperidin -4-yl)phenyl)-2-oxo-2,3-dihydro-*1H*-benzo[*d*]imidazol-1-yl)methyl)benzoic acid |
| 102 | 3-((5-chloro-2-oxo-3-(4-(1-propionylpiperidin-4-yl)phenyl)-2,3-dihydro-*1H-*benzo[*d*]imidazol-1-yl)methyl)benzoic acid |
| 103 | 3-((5-chloro-2-oxo-3-(4-(tetrahydro-*2H*-pyran-4-yl)phenyl)-2,3-dihydro-*1H-*benzo[*d*]imidazol-1-yl)methyl)benzoic acid |
| 104 | 3-((5-chloro-3-(4-(3,6-dihydro-2H-pyran-4-yl)phenyl)-2-oxo-2,3-dihydro-*1H-*benzo[*d*]imidazol-1-yl)methyl)benzoic acid |
| 105 | 3-((5-chloro-3-(4-(3,3-difluoropyrrolidin-1-yl)phenyl)-4-fluoro-2-oxo-2,3-dihydro-*1H*-benzo[*d*]imidazol-1-yl)methyl)benzoic acid |
| 106 | 3-(1-(5-chloro-2-oxo-3-(4-(tetrahydro-2*H*-pyran-4-yl)phenyl)-2,3-dihydro-*1H-*benzo[*d*]imidazol-1-yl)cyclopropyl)-2-fluorobenzoic acid |
| 107 | 3-(1-(3-(4-(7-oxa-2-azaspiro[3.5]nonane-2-carbonyl)phenyl)-5-chloro-2-oxo-2,3-dihydro-*1H-*benzo[*d*]imidazol-1-yl)cyclopropyl)-2-fluorobenzoic acid |
| 108 | 3-((5-chloro-2-oxo-3-(4-(tetrahydro-2*H*-pyran-4-yl)phenyl)-2,3-dihydro-*1H-*benzo[*d*]imidazol-1-yl)methyl)-2-fluorobenzoic acid |
| 109 | 3-((5-chloro-2-oxo-3-(2',3',4',5'-tetrahydro-[1,1'-biphenyl]-4-yl)-2,3-dihydro-*1H*-benzo[*d*]imidazol-1-yl)methyl)benzoic acid |
| 110 | 3-((5-chloro-3-(4-(2,5-dihydrofuran-3-yl)phenyl)-2-oxo-2,3-dihydro-1*H-*benzo[*d*]imidazol-1-yl)methyl)-2-fluorobenzoic acid |
| 111 | 3-((5-chloro-3-(4'-methyl-2',3',4',5'-tetrahydro-[1,1'-biphenyl]-4-yl)-2-oxo-2,3-dihydro-*1H*-benzo[*d*]imidazol-1-yl)methyl)-2-fluorobenzoic acid |
| 112 | 3-((5-chloro-3-(4',4'-dimethyl-2',3',4',5'-tetrahydro-[1,1'-biphenyl]-4-yl)-2-oxo-2,3-dihydro-*1H-*benzo[*d*]imidazol-1-yl)methyl)-2-fluorobenzoic acid |
| 113 | 3-(2-(5-chloro-3-(4-(dimethylcarbamoyl)phenyl)-2-oxo-2,3-dihydro-*1H-*benzo[*d*]imidazol-1-yl)ethyl)benzoic acid |
| 114 | 3-((5-chloro-3-(2,2-dimethyl-3-oxo-2,3-dihydro-*1H*-inden-5-yl)-2-oxo-2,3-dihydro-*1H-*benzo[*d*]imidazol-1-yl)methyl)benzoic acid |
| 115 | 3-((5-chloro-3-(2,2-dimethyl-1-oxo-2,3-dihydro-*1H*-inden-5-yl)-2-oxo-2,3-dihydro-*1H-*benzo[*d*]imidazol-1-yl)methyl)benzoic acid |
| 116 | 3-((5-chloro-3-(2-methyl-1-oxoisoindolin-5-yl)-2-oxo-2,3-dihydro-*1H*-benzo[*d*]imidazol-1-yl)methyl)benzoic acid |
| 117 | 3-((5-chloro-3-(2-methyl-1-oxoisoindolin-5-yl)-2-oxo-2,3-dihydro-*1H*-benzo[*d*]imidazol-1-yl)methyl)-2-fluorobenzoic acid |
| 118 | 3-((5-chloro-3-(4-(4,4-difluoropiperidin-1-yl)phenyl)-2-oxo-2,3-dihydro-*1H*-benzo[*d*]imidazol-1-yl)methyl)-2-fluorobenzoic acid |
| 119 | 3-((5-chloro-3-(4-(3,3-difluoropyrrolidin-1-yl)phenyl)-4-fluoro-2-oxo-2,3-dihydro-*1H*-benzo[*d*]imidazol-1-yl)methyl)-2-fluorobenzoic acid |
| 120 | 3-((5-chloro-4-fluoro-3-(4-(3-hydroxyazetidin-1-yl)phenyl)-2-oxo-2,3-dihydro*-1H-*benzo[*d*]imidazol-1-yl)methyl)-2-fluorobenzoic acid |
| 121 | 3-(1-(5-chloro-3-(4-(3,6-dihydro-2*H*-pyran-4-yl)phenyl)-2-oxo-2,3-dihydro-*1H-*benzo[*d*]imidazol-1-yl)cyclopropyl)benzoic acid |
| 122 | 3-(1-(5-chloro-3-(4-(3,6-dihydro-2*H*-pyran-4-yl)phenyl)-2-oxo-2,3-dihydro-*1H-*benzo[*d*]imidazol-1-yl)cyclopropyl)-2-fluorobenzoic acid |
| 123 | 3-(1-(5-chloro-3-(4-(2,5-dihydrofuran-3-yl)phenyl)-2-oxo-2,3-dihydro-*1H-*benzo[*d*]imidazol-1-yl)cyclopropyl)-2-fluorobenzoic acid |
| 124 | 3-(1-(5-chloro-3-(4-(2,5-dihydrofuran-3-yl)phenyl)-2-oxo-2,3-dihydro-*1H-*benzo[*d*]imidazol-1-yl)cyclopropyl)benzoic acid |
| 125 | 4-((5-chloro-4-fluoro-3-(4-morpholinophenyl)-2-oxo-2,3-dihydro-*1H*-benzo[*d*]imidazol-1-yl)methyl)benzoic acid |
| 126 | 3-(1-(5-chloro-3-(4-(morpholine-4-carbonyl)phenyl)-2-oxo-2,3-dihydro-*1H*-benzo[*d*]imidazol-1-yl)cyclopropyl)benzoic acid |
| 127 | (*S*)-3-(1-(5-chloro-3-(4-(3-methylmorpholine-4-carbonyl)phenyl)-2-oxo-2,3-dihydro-*1H*-benzo[*d*]imidazol-1-yl)cyclopropyl)benzoic acid |
| 128 | 3-(1-(5-chloro-3-(4-(3-hydroxyazetidine-1-carbonyl)phenyl)-2-oxo-2,3-dihydro-*1H*-benzo[*d*]imidazol-1-yl)cyclopropyl)benzoic acid |
| 129 | 3-(1-(5-chloro-3-(4-(3,3-difluoroazetidine-1-carbonyl)phenyl)-2-oxo-2,3-dihydro-*1H-*benzo[*d*]imidazol-1-yl)cyclopropyl)benzoic acid |
| 130 | 3-(1-(5-chloro-3-(4-(3,3-difluoropyrrolidine-1-carbonyl)phenyl)-2-oxo-2,3-dihydro*-1H*-benzo[*d*]imidazol-1-yl)cyclopropyl)benzoic acid |
| 131 | 3-(1-(5-chloro-3-(4-(4,4-difluoropiperidine-1-carbonyl)phenyl)-2-oxo-2,3-dihydro-*1H*-benzo[*d*]imidazol-1-yl)cyclopropyl)benzoic acid |
| 132 | 3-(1-(5-chloro-3-(4-(3,3-difluoropyrrolidine-1-carbonyl)phenyl)-2-oxo-2,3-dihydro-*1H*-benzo[*d*]imidazol-1-yl)cyclopropyl)-2-fluorobenzoic acid |
| 133 | 3-(1-(5-chloro-3-(4-(4,4-difluoropiperidine-1-carbonyl)phenyl)-2-oxo-2,3-dihydro-*1H*-benzo[*d*]imidazol-1-yl)cyclopropyl)-2-fluorobenzoic acid |
| 134 | 3-(1-(5-chloro-3-(4-(3-hydroxyazetidine-1-carbonyl)phenyl)-2-oxo-2,3-dihydro-*1H*-benzo[*d*]imidazol-1-yl)cyclopropyl)-2-fluorobenzoic acid |
| 135 | 3-(1-(3-(4-(2-oxa-6-azaspiro[3.3]heptane-6-carbonyl)phenyl)-5-chloro-2-oxo-2,3-dihydro-*1H-*benzo[*d*]imidazol-1- |
| | yl)cyclopropyl)-2-fluorobenzoic acid |
| 136 | (S)-3-(1-(5-chloro-3-(4-(3-methylmorpholine-4-carbonyl)phenyl)-2-oxo-2,3-dihydro-*1H*-benzo[*d*]imidazol-1-yl)cyclopropyl)-2-fluorobenzoic acid |
| 137 | 3-(1-(3-(4-(2-oxa-6-azaspiro[3.3]heptane-6-carbonyl)phenyl)-5-chloro-2-oxo-2,3-dihydro-*1H-*benzo[*d*]imidazol-1-yl)cyclopropyl)benzoic acid |
| 138 | 3-(1-(5-chloro-3-(4-(5-(4,4-difluorocyclohexyl)-1,3,4-oxadiazol-2-yl)phenyl)-2-oxo-2,3-dihydro-*1H-*benzo[*d*]imidazol-1-yl)cyclopropyl)benzoic acid |
| 139 | 3-(1-(5-chloro-3-(4-(2-(dimethylamino)-2-oxoethyl)phenyl)-2-oxo-2,3-dihydro-*1H-*benzo[*d*]imidazol-1-yl)cyclopropyl)benzoic acid |
| 140 | 3-((5-chloro-3-(4-(4-(cyclopropanecarbonyl)piperazi n-1-yl)phenyl)-2-oxo-2,3-dihydro-*1H*-benzo[*d*]imidazol-1-yl)methyl)-2-fluorobenzoic acid |
| 141 | 3-((5-chloro-2-oxo-3-(4-(4-propionylpiperazin-1-yl)phenyl)-2,3-dihydro-*1H-*benzo[*d*]imidazol-1-yl)methyl)-2-fluorobenzoic acid |
| 142 | 3-((5-chloro-3-(4-(4-(cyclobutanecarbonyl)piperazin -1-yl)phenyl)-2-oxo-2,3-dihydro-*1H*-benzo[*d*]imidazol-1-yl)methyl)-2-fluorobenzoic acid |
| 143 | 3-((5-chloro-3-(4-(4-(4,4-difluorocyclohexane-1-carbonyl)piperazin-1-yl)phenyl)-2-oxo-2,3-dihydro-*1H*-benzo[d]imidazol-1- |
| | yl)methyl)-2-fluorobenzoic acid |
| 144 | 3-(1-(3-(benzo[*d*]thiazol-2-yl)-5-chloro-2-oxo-2,3-dihydro-*1H*-benzo[*d*]imidazol-1-yl)cyclopropyl)-2-fluorobenzoic acid |
| 145 | 3-(1-(5-chloro-3-(4-(morpholinomethyl)phenyl)-2-oxo-2,3-dihydro-*1H-*benzo[*d*]imidazol-1-yl)cyclopropyl)benzoic acid |
| 146 | 3-(1-(5-chloro-2-oxo-3-(4-(pyrrolidin-1-yl)phenyl)-2,3-dihydro-*1H*-benzo[*d*]imidazol-1-yl)cyclopropyl)benzoic acid |
| 147 | 3-(1-(5-chloro-3-(4-morpholinophenyl)-2-oxo-2,3-dihydro-*1H*-benzo[*d*]imidazol-1-yl)cyclopropyl)benzoic acid |
| 148 | 3-(1-(3-(4-(7-oxa-2-azaspiro[3.5]nonan-2-yl)phenyl)-5-chloro-2-oxo-2,3-dihydro-*1H*-benzo[*d*]imidazol-1-yl)cyclopropyl)benzoic acid |
| 149 | 3-(1-(3-(4-(2-oxa-6-azaspiro[3.3]heptan-6-yl)phenyl)-5-chloro-2-oxo-2,3-dihydro-*1H*-benzo[*d*]imidazol-1-yl)cyclopropyl)benzoic acid |
| 150 | 3-((5-chloro-3-(4-(1-(cyclopropanecarbonyl)piperidi n-4-yl)phenyl)-4-fluoro-2-oxo-2,3-dihydro-*1H-*benzo[*d*]imidazol-1-yl)methyl)-2-fluorobenzoic acid |
| 151 | 3-((5-chloro-4-fluoro-2-oxo-3-(4-(1-propionylpiperidin-4-yl)phenyl)-2,3-dihydro-*1H-*benzo[*d*]imidazol-1-yl)methyl)-2-fluorobenzoic acid |
| 152 | 3-((5-chloro-3-(4-(1-(cyclobutanecarbonyl)piperidin -4-yl)phenyl)-4-fluoro-2-oxo-2,3-dihydro-1*H*- |
| | benzo[*d*]imidazol-1-yl)methyl)-2-fluorobenzoic acid |
| 153 | 3-(2-(5-chloro-3-(4-(dimethylamino)phenyl)-2-oxo-2,3-dihydro-*1H-*benzo[*d*]imidazol-1-yl)propan-2-yl)benzoic acid |
| 154 | 3-(2-(5-chloro-3-(4-(3-hydroxyazetidin-1-yl)phenyl)-2-oxo-2,3-dihydro-*1H-*benzo[*d*]imidazol-1-yl)propan-2-yl)benzoic acid |
| 155 | 3-(1-(5-chloro-2-oxo-3-(4-(tetrahydro-2*H*-pyran-4-yl)phenyl)-2,3-dihydro-*1H-*benzo[*d*]imidazol-1-yl)cyclobutyl)benzoic acid |
| 156 | 3-(1-(5-chloro-3-(4-(dimethylamino)phenyl)-2-oxo-2,3-dihydro-*1H-*benzo[*d*]imidazol-1-yl)cyclobutyl)benzoic acid |
| 157 | 3-(1-(5-chloro-3-(4-(dimethylcarbamoyl)phenyl)-2-oxo-2,3-dihydro-*1H-*benzo[*d*]imidazol-1-yl)cyclobutyl)benzoic acid |
| 158 | 3-(2-(5-chloro-2-oxo-3-(4-(tetrahydro-*2H*-pyran-4-yl)phenyl)-2,3-dihydro-*1H-*benzo[*d*]imidazol-1-yl)propan-2-yl)benzoic acid |
| 159 | 3-(1-(5-chloro-3-(4-(3-hydroxyazetidin-1-yl)phenyl)-2-oxo-2,3-dihydro-*1H-*benzo[*d*]imidazol-1-yl)cyclopropyl)benzoic acid |
| 160 | 3-(1-(5-chloro-3-(4-(4,4-difluoropiperidin-1-yl)phenyl)-2-oxo-2,3-dihydro-*1H*-benzo[*d*]imidazol-1-yl)cyclopropyl)benzoic acid |
| 161 | 3-((6-chloro-1-(4-(dimethylamino)phenyl)-2-oxo-1,2-dihydro-*3H*-imidazo[4,5- |
| | *b*]pyridin-3-yl)methyl)benzoic acid |
| 162 | 3-((6-chloro-1-(4-morpholinophenyl)-2-oxo-1,2-dihydro-3*H-*imidazo[4,5-*b*]pyridin-3-yl)methyl)benzoic acid |
| 163 | 3-((5-chloro-3-(5-cyclopropylpyridin-2-yl)-2-oxo-2,3-dihydro-*1H-*benzo[*d*]imidazol-1-yl)methyl)benzoic acid |
| 164 | 3-((5-chloro-3-(5-(dimethylamino)pyridin-2-yl)-2-oxo-2,3-dihydro-*1H-*benzo[*d*]imidazol-1-yl)methyl)benzoic acid |
| 165 | 3-(1-(5-chloro-3-(6-(3,3-difluoropyrrolidin-1-yl)pyridin-3-yl)-2-oxo-2,3-dihydro-*1H*-benzo[*d*]imidazol-1-yl)cyclopropyl)benzoic acid |
| 166 | 3-((5-chloro-3-(6-(3,3-difluoropyrrolidin-1-yl)pyridin-3-yl)-2-oxo-2,3-dihydro-*1H*-benzo[*d*]imidazol-1-yl)methyl)-2-fluorobenzoic acid |
| 167 | 3-((3-(4-(dimethylamino)phenyl)-5-methyl-2-oxo-2,3-dihydro-*1H-*imidazo[4,5-*b*]pyridin-1-yl)methyl)-2-fluorobenzoic acid |
| 168 | 3-((3-(4-(dimethylamino)phenyl)-5-methyl-2-oxo-2,3-dihydro-*1H-*imidazo[4,5-*b*]pyridin-1-yl)methyl)benzoic acid |
| 169 | 3-((6-chloro-2-oxo-1-(4-(pyrrolidine-1-carbonyl)phenyl)-1,2-dihydro-3*H*-imidazo[4,5-*b*]pyridin-3-yl)methyl)benzoic acid |
| 170 | 3-(1-(5-chloro-2-oxo-3-(4-(2-oxo-2-(pyrrolidin-1-yl)ethyl)phenyl)-2,3-dihydro-*1H*-benzo[*d*]imidazol-1-yl)cyclopropyl)benzoic acid |
| 171 | 3-(1-(5-chloro-2-oxo-3-(4-(2-oxo-2-(piperidin-1-yl)ethyl)phenyl)-2,3-dihydro-*1H*-benzo[*d*]imidazol-1-yl)cyclopropyl)benzoic acid |
| 172 | 3-(1-(5-chloro-3-(4-(2-morpholino-2-oxoethyl)phenyl)-2-oxo-2,3-dihydro-*1H-*benzo[*d*]imidazol-1-yl)cyclopropyl)benzoic acid |
| 173 | 3-(1-(5-chloro-3-(4-(2-(4,4-difluoropiperidin-1-yl)-2-oxoethyl)phenyl)-2-oxo-2,3-dihydro-*1H*-benzo[*d*]imidazol-1-yl)cyclopropyl)benzoic acid |
| 174 | 3-(1-(5-chloro-3-(4-(2-(3-hydroxyazetidin-1-yl)-2-oxoethyl)phenyl)-2-oxo-2,3-dihydro-*1H*-benzo[*d*]imidazol-1-yl)cyclopropyl)benzoic acid |
| 175 | (*R*)-3-(1-(5-chloro-3-(4-(2-(2-methylmorpholino)-2-oxoethyl)phenyl)-2-oxo-2,3-dihydro-*1H*-benzo[*d*]iimidazol-1-yl)cyclopropyl)benzoic acid |
| 176 | (*S*)-3-(1-(5-chloro-3-(4-(2-(2-methylmorpholino)-2-oxoethyl)phenyl)-2-oxo-2,3-dihydro-*1H*-benzo[*d*]imidazol-1-yl)cyclopropyl)benzoic acid |
| 177 | (*S*)-3-(1-(5-chloro-3-(4-(2-(3-methylmorpholino)-2-oxoethyl)phenyl)-2-oxo-2,3-dihydro-*1H*-benzo[*d*]imidazol-1-yl)cyclopropyl)benzoic acid |
| 178 | (*R*)-3-(1-(5-chloro-3-(4-(2-(3-methylmorpholino)-2-oxoethyl)phenyl)-2-oxo-2,3-dihydro-*1H*-benzo[*d*]imidazol-1-yl)cyclopropyl)benzoic acid |
| 179 | 3-(1-(3-(4-((3r,5r,7r)-adamantan-1-yl)phenyl)-5-chloro-2-oxo-2,3-dihydro-*1H-*benzo[*d*]imidazol-1-yl)cyclopropyl)benzoic acid |
| 180 | 3-(1-(5-chloro-3-(4-(methylsulfonyl)phenyl)-2-oxo-2,3-dihydro-*1H-*benzo[*d*]imidazol-1-yl)cyclopropyl)benzoic acid |
| 181 | 3-(1-(3-(4-(2-oxa-5-azabicyclo[2.2.1]heptan-5-yl)phenyl)-5-chloro-2-oxo-2,3-dihydro-*1H*-benzo[*d*]imidazol-1-yl)cyclopropyl)benzoic acid |
| 182 | 3-(1-(3-(4-(8-oxa-3-azabicyclo[3.2.1]octan-3-yl)phenyl)-5-chloro-2-oxo-2,3-dihydro-*1H*-benzo[*d*]imidazol-1-yl)cyclopropyl)benzoic acid |
| 183 | 3-(1-(3-(4-(1,4-oxazepan-4-yl)phenyl)-5-chloro-2-oxo-2,3-dihydro-*1H*-benzo[*d*]imidazol-1-yl)cyclopropyl)benzoic acid |
| 184 | 3-(1-(3-(4-(2-azaspiro[3.3]heptan-2-yl)phenyl)-5-chloro-2-oxo-2,3-dihydro-*1H*-benzo[*d*]imidazol-1-yl)cyclopropyl)benzoic acid |
| 185 | 3-(1-(5-chloro-3-(4-(3,3-difluoroazetidin-1-yl)phenyl)-2-oxo-2,3-dihydro-*1H-*benzo[*d*]imidazol-1-yl)cyclopropyl)benzoic acid |
| 186 | 3-(1-(3-(4-(7-acetyl-2,7-diazaspiro[3.5]nonan-2-yl)phenyl)-5-chloro-2-oxo-2,3-dihydro-*1H*-benzo[*d*]imidazol-1-yl)cyclopropyl)benzoic acid |
| 187 | 3-(1-(5-chloro-3-(4-morpholinophenyl)-2-oxo-2,3-dihydro-*1H*-benzo[*d*]imidazol-1-yl)cyclopropyl)-5-fluorobenzoic acid |
| 188 | 3-(1-(5-chloro-3-(4-(dimethylamino)phenyl)-2-oxo-2,3-dihydro-1*H-*benzo[*d*]imidazol-1- |
| | yl)cyclopropyl)-5-fluorobenzoic acid |
| 189 | 3-(1-(5-chloro-3-(4-(2-(dimethylamino)-2-oxoethyl)phenyl)-2-oxo-2,3-dihydro-*1H*-benzo[*d*]imidazol-1-yl)cyclopropyl)-5-fluorobenzoic acid |
| 190 | 3-(1-(5-chloro-2-oxo-3-(4-(tetrahydro-2*H*-pyran-4-yl)phenyl)-2,3-dihydro-*1H-*benzo[*d*]imidazol-1-yl)cyclopropyl)-5-fluorobenzoic acid |
| 191 | 3-(1-(3-(4-(2-oxa-6-azaspiro[3.3]heptan-6-yl)phenyl)-5-chloro-2-oxo-2,3-dihydro-*1H*-benzo[*d*]imidazol-1-yl)cyclopropyl)-5-fluorobenzoic acid |
| 192 | 3-(1-(3-(4-(8-oxa-3-azabicyclo[3.2.1]octan-3-yl)phenyl)-5-chloro-2-oxo-2,3-dihydro-*1H*-benzo[*d*]imidazol-1-yl)cyclopropyl)-5-fluorobenzoic acid |
| 193 | 3-(1-(3-(4-(7-oxa-2-azaspiro[3.5]nonan-2-yl)phenyl)-5-chloro-2-oxo-2,3-dihydro-*1H*-benzo[*d*]imidazol-1-yl)cyclopropyl)-5-fluorobenzoic acid |
| 194 | 3-(1-(5-chloro-3-(4-(3,3-difluoroazetidin-1-yl)phenyl)-2-oxo-2,3-dihydro-*1H-*benzo[*d*]imidazol-1-yl)cyclopropyl)-5-fluorobenzoic acid |
| 195 | 3-(1-(3-(4-(7-(*tert-*butoxycarbonyl)-2,7-diazaspiro[3.5]nonan-2-yl)phenyl)-5-chloro-2-oxo-2,3-dihydro-*1H*-benzo[*d*]imidazol-1-yl)cyclopropyl)-5-fluorobenzoic acid |
| 196 | 3-(1-(3-(4-(2-azaspiro[3.3]heptan-2-yl)phenyl)-5-chloro-2-oxo-2,3-dihydro-*1H*-benzo[*d*]imidazol-1-yl)cyclopropyl)-5-fluorobenzoic acid |
| 197 | 3-(1-(5-chloro-3-(4-(4,4-difluoropiperidin-1-yl)phenyl)-2-oxo-2,3-dihydro-*1H*-benzo[*d*]imidazol-1-yl)cyclopropyl)-5-fluorobenzoic acid |
| 198 | 5-((5-chloro-4-fluoro-3-(4-morpholinophenyl)-2-oxo-2,3-dihydro-*1H*-benzo[*d*]imidazol-1-yl)methyl)isoxazole-3-carboxylic acid |
| 199 | 2-((5-chloro-4-fluoro-3-(4-morpholinophenyl)-2-oxo-2,3-dihydro-*1H*-benzo[*d*]imidazol-1-yl)methyl)oxazole-4-carboxylic acid |
| 200 | 5-((5-chloro-3-(4-morpholinophenyl)-2-oxo-2,3-dihydro-*1H*-benzo[*d*]imidazol-1-yl)methyl)isoxazole-3-carboxylic acid |
| 201 | 2-((5-chloro-3-(4-morpholinophenyl)-2-oxo-2,3-dihydro-*1H*-benzo[*d*]imidazol-1-yl)methyl)oxazole-4-carboxylic acid |
| 202 | 5-((5-chloro-4-fluoro-3-(4-morpholinophenyl)-2-oxo-2,3-dihydro-*1H*-benzo[*d*]imidazol-1-yl)methyl)-2,3-difluorobenzoic acid |
| 203 | 5-((5-chloro-3-(4-morpholinophenyl)-2-oxo-2,3-dihydro-*1H*-benzo[*d*]imidazol-1-yl)methyl)-2,3-difluorobenzoic acid |
| 204 | 3-((5-chloro-4-fluoro-3-(4-morpholinophenyl)-2-oxo-2,3-dihydro-*1H*-benzo[*d*]imidazol-1-yl)methyl)-2,6-difluorobenzoic acid |
| 205 | 3-((5-chloro-3-(4-morpholinophenyl)-2-oxo-2,3-dihydro-*1H*-benzo[*d*]imidazol-1-yl)methyl)-2,6-difluorobenzoic acid |
| 206 | 3-(1-(3-(4-(dimethylamino)phenyl)-2-oxo-5-(trifluoromethyl)-2,3-dihydro-*1H*-benzo[*d*]imidazol-1-yl)cyclopropyl)benzoic acid |
| 207 | 3-(1-(3-(4-morpholinophenyl)-2-oxo-5-(trifluoromethyl)-2,3-dihydro-*1H*-benzo[*d*]imidazol-1-yl)cyclopropyl)benzoic acid |
| 208 | 3-(1-(3-(4-(dimethylamino)phenyl)-2-oxo-5-(trifluoromethoxy)-2,3-dihydro-1*H*-benzo[*d*]imidazol-1-yl)cyclopropyl)benzoic acid |
| 209 | 3-(1-(3-(4-morpholinophenyl)-2-oxo-5-(trifluoromethoxy)-2,3-dihydro-*1H-*benzo[*d*]imidazol-1-yl)cyclopropyl)benzoic acid |
| 210 | 3-(1-(5-chloro-3-(4-(*N,N-*dimethylsulfamoyl)phenyl)-2-oxo-2,3-dihydro-*1H-*benzo[*d*]imidazol-1-yl)cyclopropyl)benzoic acid |
| 211 | 3-(1-(5-chloro-3-(4-(*N-*cyclopropylsulfamoyl)phenyl)-2-oxo-2,3-dihydro-*1H-*benzo[*d*]imidazol-1-yl)cyclopropyl)benzoic acid |
| 212 | 3-(1-(5-chloro-3-(4-(morpholinosulfonyl)phenyl)-2-oxo-2,3-dihydro-*1H-*benzo[*d*]imidazol-1-yl)cyclopropyl)benzoic acid |
| 213 | 3-(1-(5-chloro-3-(4-(*N-*(cyclobutylmethyl)sulfamoyl)ph enyl)-2-oxo-2,3-dihydro-*1H-*benzo[*d*]imidazol-1-yl)cyclopropyl)benzoic acid |
| 214 | 3-(1-(5-chloro-2-oxo-3-(4-(piperidin-1-ylsulfonylphenyl)-2,3-dihydro- |
| | *1H*-benzo[*d*]imidazol-1-yl)cyclopropyl)benzoic acid |
| 215 | 3-(1-(5-chloro-2-oxo-3-(4-(pyrrolidin-1-ylsulfonyl)phenyl)-2,3-dihydro-*1H*-benzo[*d*]imidazol-1-yl)cyclopropyl)benzoic acid |
| 216 | 3-(1-(5-chloro-3-(4-(2-hydroxy-2-methylpropyl)phenyl)-2-oxo-2,3-dihydro-*1H-*benzo[*d*]imidazol-1-yl)cyclopropyl)benzoic acid |
| 217 | 3-(1-(5-chloro-3-(4-(2-fluoro-2-methylpropyl)phenyl)-2-oxo-2,3-dihydro-*1H-*benzo[*d*]imidazol-1-yl)cyclopropyl)benzoic acid |
| 218 | 3-(1-(5-chloro-3-(4-(1-hydroxy-2-methylpropan-2-yl)phenyl)-2-oxo-2,3-dihydro-*1H*-benzo[*d*]imidazol-1-yl)cyclopropyl)benzoic acid |
| 219 | 3-(1-(5-chloro-3-(4-(1-fluoro-2-methylpropan-2-yl)phenyl)-2-oxo-2,3-dihydro-*1H-*benzo[*d*]imidazol-1-yl)cyclopropyl)benzoic acid |
| 220 | 3-(1-(6-chloro-2-oxo-1-(4-(tetrahydro-*2H*-pyran-4-yl)phenyl)-1,2-dihydro-3*H-*imidazo[4,5-*b*]pyridin-3-yl)cyclopropyl)benzoic acid |
| 221 | 3-(1-(6-chloro-1-(4-(dimethylamino)phenyl)-2-oxo-1,2-dihydro-3*H*-imidazo[4,5-*b*]pyridin-3-yl)cyclopropyl)benzoic acid |
| 222 | 3-(1-(6-chloro-1-(4-morpholinophenyl)-2-oxo-1,2-dihydro-3*H*-imidazo[4,5-*b*]pyridin-3-yl)cyclopropyl)benzoic acid |
| 223 | 3-(1-(6-chloro-1-(4-(dimethylcarbamoyl)phenyl)-2-oxo-1,2-dihydro-3*H-*imidazo[4,5-*b*]pyridin-3-yl)cyclopropyl)benzoic acid |
| 224 | 3-(1-(6-chloro-1-(4-(3-hydroxyazetidine-1-carbonyl)phenyl)-2-oxo-1,2-dihydro-*3H*-imidazo[4,5-*b*]pyridin-3-yl)cyclopropyl)benzoic acid |
| 225 | 3-(1-(1-(4-(7-oxa-2-azaspiro[3.5]nonane-2-carbonyl)phenyl)-6-chloro-2-oxo-1,2-dihydro-3*H-*imidazo[4,5-*b*]pyridin-3-yl)cyclopropyl)benzoic acid |
| 226 | 3-(1-(6-chloro-1-(4-(2-(dimethylamino)-2-oxoethyl)phenyl)-2-oxo-1,2-dihydro-3*H*-imidazo[4,5-*b*]pyridin-3-yl)cyclopropyl)benzoic acid |
| 227 | 3-(1-(6-chloro-1-(4-(methylsulfonyl)phenyl)-2-oxo-1,2-dihydro-3*H*-imidazo[4,5-*b*]pyridin-3-yl)cyclopropyl)benzoic acid |
| 228 | 3-(1-(6-chloro-2-oxo-1-(4-(tetrahydro-2*H*-pyran-4-yl)phenyl)-1,2-dihydro-3*H-*imidazo[4,5-*b*]pyridin-3-yl)cyclopropyl)-2-fluorobenzoic acid |
| 229 | 3-(1-(6-chloro-1-(4-(dimethylamino)phenyl)-2-oxo-1,2-dihydro-3*H-*imidazo[4,5-*b*]pyridin-3-yl)cyclopropyl)-2-fluorobenzoic acid |
| 230 | 3-(1-(6-chloro-1-(4-(morpholinophenyl)-2-oxo-1,2-dihydro-3H-imidazo[4,5-*b*]pyridin-3-yl)cyclopropyl)-2-fluorobenzoic acid |
| 231 | 3-(1-(6-chloro-2-oxo-1-(4-(pyrrolidine-1-carbonyl)phenyl)-1,2-dihydro-3*H*-imidazo[4,5-*b*]pyridin-3- |
| | yl)cyclopropyl)-2-fluorobenzoic acid |
| 232 | 3-(1-(6-chloro-1-(4-(3-hydroxyazetidine-1-carbonyl)phenyl)-2-oxo-1,2-dihydro-3*H*-imidazo[4,5-*b*]pyridin-3-yl)cyclopropyl)-2-fluorobenzoic acid |
| 233 | 3-(1-(1-(4-(2-oxa-6-azaspiro[3.3]heptane-6-carbonyl)phenyl)-6-chloro-2-oxo-1,2-dihydro-3*H-*imidazo[4,5-*b*]pyridin-3-yl)cyclopropyl)-2-fluorobenzoic acid |
| 234 | 3-(1-(5-chloro-2-oxo-3-(4-(1-propionylpiperidin-4-yl)phenyl)-2,3-dihydro-*1H-*benzo[*d*]imidazol-1-yl)cyclopropyl)benzoic acid |
| 235 | 3-(1-(5-chloro-2-oxo-3-(4-(1-propionylpiperidin-4-yl)phenyl)-2,3-dihydro*-1H-*benzo[*d*]imidazol-1-yl)cyclopropyl)-5-fluorobenzoic acid |
| 236 | 3-(1-(5-chloro-2-oxo-3-(4-((2-oxopyrrolidin-1-yl)methyl)phenyl)-2,3-dihydro-*1H*-benzo[*d*]imidazol-1-yl)cyclopropyl)benzoic acid |
| 237 | 3-(1-(5-chloro-2-oxo-3-(4-((4-(trifluoromethyl)-*1H*-imidazol-1-yl)methyl)phenyl)-2,3-dihydro-*1H*-benzo[*d*]imidazol-1-yl)cyclopropyl)benzoic acid |
| 238 | 3-(1-(5-chloro-3-(4-(1-(hydroxymethyl)cyclopropyl)phe nyl)-2-oxo-2,3-dihydro-*1H-*benzo[*d*]imidazol-1-yl)cyclopropyl)benzoic acid |
| 239 | 3-(1-(5-chloro-3-(4-(1-(hydroxymethyl)cyclobutyl)phen yl)-2-oxo-2,3-dihydro*-1H-*benzo[*d*]imidazol-1-yl)cyclopropyl)benzoic acid |
| 240 | 3-(1-(5-chloro-3-(4-(oxetan-3-ylmethyl)phenyl)-2-oxo-2,3-dihydro-*1H*-benzo[*d*]imidazol-1-yl)cyclopropyl)benzoic acid |
| 241 | 3-(1-(5-chloro-3-(4-((3-methyl-2-oxo-imidazolin-1-yl)methyl)phenyl)-2-oxo-2,3-dihydro-*1H*-benzo[*d*]imidazol-1-yl)cyclopropyl)benzoic acid |
| 242 | 3-(1-(5-chloro-3-(4-(4,4-dimethyl-2-oxo-oxazolidin-3-yl)phenyl)-2-oxo-2,3-dihydro-*1H*-benzo[*d*]imidazol-1-yl)cyclopropyl)benzoic acid |
| 243 | 3-(1-(5-chloro-2-oxo-3-(4-(5-oxo-6-oxa-4-azaspiro[2.4]heptan-4-yl)phenyl)-2,3-dihydro-*1H-*benzo[*d*]imidazol-1-yl)cyclopropyl)benzoic acid |
| 244 | 3-(1-(5-chloro-2-oxo-3-(4-((3-(trifluoromethyl)-*1H*-pyrazol-1-yl)methyl)phenyl)-2,3-dihydro-*1H*-benzo[*d*]imidazol-1-yl)cyclopropyl)benzoic acid |
| 245 | 3-(1-(5-chloro-3-(2-methyl-1-oxoisoindolin-5-yl)-2-oxo-2,3-dihydro-*1H*-benzo[*d*]imidazol-1-yl)cyclopropyl)benzoic acid |
| 246 | 3-(1-(5-chloro-3-(4-(2-hydroxypropan-2-yl)phenyl)-2-oxo-2,3-dihydro-*1H-*benzo[*d*]imidazol-1-yl)cyclopropyl)benzoic acid |
| 247 | 3-(1-(5-chloro-3-(4-(3-methyloxetan-3-yl)phenyl)-2-oxo-2,3-dihydro-*1H-*benzo[*d*]imidazol-1-yl)cyclopropyl)benzoic acid |
| 248 | 3-(1-(5-chloro-2-oxo-3-(4-(tetrahydrofuran-3-yl)phenyl)-2,3-dihydro-*1H-*benzo[*d*]imidazol-1-yl)cyclopropyl)benzoic acid |
| 249 | 3-(1-(5-chloro-2-oxo-3-(4-(tetrahydrofuran-3-yl)phenyl)-2,3-dihydro*-1H-*benzo[*d*]imidazol-1- |
| | yl)cyclopropyl)-5-fluorobenzoic acid |
| 250 | 3-(1-(5-chloro-2-oxo-3-(4-(tetrahydrofuran-3-yl)phenyl)-2,3-dihydro-*1H-*benzo[*d*]imidazol-1-yl)cyclopropyl)-2-fluorobenzoic acid |
| 251 | 3-(1-(5-chloro-3-(1,1-dimethyl-1,3-dihydroisobenzofuran-5-yl)-2-oxo-2,3-dihydro-*1H-*benzo[*d*]imidazol-1-yl)cyclopropyl)benzoic acid |
| 252 | 3-(1-(5-chloro-3-(1,1-dimethyl-1,3-dihydroisobenzofuran-5-yl)-2-oxo-2,3-dihydro-*1H-*benzo[*d*]imidazol-1-yl)cyclopropyl)-5-fluorobenzoic acid |
| 253 | 3-(1-(5-chloro-3-(1,1-dimethyl-1,3-dihydroisobenzofuran-5-yl)-2-oxo-2,3-dihydro-*1H-*benzo[*d*]imidazol-1-yl)cyclopropyl)-2-fluorobenzoic acid |
| 254 | 3-(1-(5-chloro-2-oxo-3-(3-oxo-3,4-dihydro-*2H-*benzo[b][1,4]oxazin-7-yl)-2,3-dihydro-*1H*-benzo[d]imidazol-1-yl)cyclopropyl)-5-fluorobenzoic acid |
| 255 | 3-(1-(5-chloro-3-(4-methyl-3,4-dihydro-2*H-*benzo[b][1,4]oxazin-7-yl)-2-oxo-2,3-dihydro-*1H-*benzo[*d*]imidazol-1-yl)cyclopropyl)-5-fluorobenzoic acid |
| 256 | 3-(1-(5-chloro-3-(4-(1-hydroxycyclobutyl)phenyl)-2-oxo-2,3-dihydro-*1H-*benzo[*d*]imidazol-1-yl)cyclopropyl)benzoic acid |
| 257 | 3-(1-(5-chloro-3-(4-(1-hydroxycyclobutyl)phenyl)-2-oxo-2,3-dihydro-*1H-*benzo[*d*]imidazol-1-yl)cyclopropyl)-5-fluorobenzoic acid |
| 258 | 3-(1-(5-chloro-3-(4-(3-hydroxyoxetan-3-yl)phenyl)-2-oxo-2,3-dihydro-*1H-*benzo[*d*]imidazol-1-yl)cyclopropyl)benzoic acid |
| 259 | 3-((5-chloro-3-(4-(1-(oxetan-3-yl)piperidin-4-yl)phenyl)-2-oxo-2,3-dihydro-*1H-*benzo[*d*]imidazol-1-yl)methyl)benzoic acid |
| 260 | 3-(1-(5-chloro-3-(4-(1-(oxetan-3-yl)piperidin-4-yl)phenyl)-2-oxo-2,3-dihydro-*1H*-benzo[*d*]imidazol-1-yl)cyclopropyl)benzoic acid |
| 261 | 3-(1-(5-chloro-2-oxo-3-(6-(tetrahydro-2*H*-pyran-4-yl)pyridin-3-yl)-2,3-dihydro-*1H*-benzo[*d*]imidazol-1-yl)cyclopropyl)benzoic acid |
| 262 | 3-(1-(5-chloro-2-oxo-3-(6-(tetrahydro-2*H*-pyran-4-yl)pyridin-3-yl)-2,3-dihydro-*1H*-benzo[*d*]imidazol-1-yl)cyclopropyl)-5-fluorobenzoic acid |
| 263 | 3-(1-(5-chloro-3-(4-(5-cyclopropyl-1,3,4-oxadiazol-2-yl)phenyl)-2-oxo-2,3-dihydro-*1H*-benzo[*d*]imidazol-1-yl)cyclopropyl)benzoic acid |
| 264 | 3-(1-(5-chloro-3-(4-(5-cycloheptyl-1,3,4-oxadiazol-2-yl)phenyl)-2-oxo-2,3-dihydro-*1H*-benzo[*d*]imidazol-1-yl)cyclopropyl)benzoic acid |
| 265 | 3-(1-(5-chloro-3-(4-(5-cyclohexyl-1,3,4-oxadiazol-2-yl)phenyl)-2-oxo-2,3-dihydro-*1H*-benzo[*d*]imidazol-1-yl)cyclopropyl)benzoic acid |
| 266 | 3-(1-(5-chloro-3-(4-(5-cyclopentyl-1,3,4-oxadiazol-2-yl)phenyl)-2-oxo-2,3-dihydro-*1H*-benzo[*d*]imidazol-1-yl)cyclopropyl)benzoic acid |
| 267 | 3-(1-(5-chloro-2-oxo-3-(4-(5-(tetrahydro-2*H*-pyran-4-yl)-1,3,4-oxadiazol-2-yl)phenyl)-2,3-dihydro-*1H-*benzo[d]imidazol-1-yl)cyclopropyl)benzoic acid |
| 268 | 3-(1-(5-chloro-2-oxo-3-(4-(5-(tetrahydro-2*H*-pyran-4-yl)-1,3,4-oxadiazol-2-yl)phenyl)-2,3-dihydro-*1H-*benzo[*d*]imidazol-1-yl)cyclopropyl)-2-fluorobenzoic acid |
| 269 | 3-(1-(5-chloro-2-oxo-3-(4-(1-propyl-*1H*-pyrazol-4-yl)phenyl)-2,3-dihydro-*1H-*benzo[*d*]imidazol-1-yl)cyclopropyl)benzoic acid |
| 270 | 3-(1-(6-chloro-2-oxo-1-(4-(1-propyl-*1H*-pyrazol-4-yl)phenyl)-1,2-dihydro-3*H-*imidazo[4,5-*b*]pyridin-3-yl)cyclopropyl)benzoic acid |
| 271 | 3-(1-(6-chloro-1-(4-(dimethylamino)phenyl)-2-oxo-1,2-dihydro-3*H*-imidazo[4,5-*b*]pyridin-3-yl)cyclopropyl)-5-fluorobenzoic acid |
| 272 | 3-(1-(6-chloro-1-(4-(dimethylcarbamoyl)phenyl)-2-oxo-1,2-dihydro-3*H-*imidazo[4,5-*b*]pyridin-3-yl)cyclopropyl)-5-fluorobenzoic acid |
| 273 | 3-(1-(6-chloro-2-oxo-1-(4-(tetrahydro-2*H*-pyran-4-yl)phenyl)-1,2-dihydro-3*H-*imidazo[4,5-*b*]pyridin-3-yl)cyclopropyl)-5-fluorobenzoic acid |
| 274 | 3-(1-(6-chloro-1-(4-morpholinophenyl)-2-oxo-1,2-dihydro-3*H*-imidazo[4,5- |
| | *b*]pyridin-3-yl)cyclopropyl)-5-fluorobenzoic acid |
| 275 | 3-(1-(1-(4-(7-oxa-2-azaspiro[3.5]nonane-2-carbonyl)phenyl)-6-chloro-2-oxo-1,2-dihydro-3*H-*imidazo[4,5-*b*]pyridin-3-yl)cyclopropyl)-5-fluorobenzoic acid |
| 276 | 3-(1-(6-chloro-2-oxo-1-(4-(pyrrolidine-1-carbonyl)phenyl)-1,2-dihydro-3*H*-imidazo[4,5-*b*]pyridin-3-yl)cyclopropyl)-5-fluorobenzoic acid |

The compounds of the present invention having the chemical structure of Formula 1 above inhibit the enzymatic activity of Autotaxin. In a specific experimental example of the present invention, it was confirmed that the compounds having the chemical structure of Formula 1 of the present invention are effective as so-called "autotaxin inhibitors" that inhibit the enzymatic activity of autotaxin. Therefore, the compound having the chemical structure of Formula 1 above, or a pharmaceutically acceptable salt, an isomer, a solvate, a hydrate, or a prodrug thereof according to the present invention may be used as an active ingredient of a pharmaceutical composition for the prevention or treatment of a disease associated with the enzymatic activity of autotaxin.

### 2. Use of Novel Benzimidazolone Derivative Compound

According to another aspect of the present invention, there is provided a pharmaceutical composition containing, as an active ingredient, a compound having the chemical structure of Formula 1 above, or a pharmaceutically acceptable salt, an isomer, a solvate, a hydrate, or a prodrug thereof.

As described above, since the compound having the chemical structure of Formula 1 above has the activity as an autotaxin inhibitor for inhibiting the expression or activity of autotaxin, the pharmaceutical composition of the present invention may be used for the prevention or treatment of a disease mediated by autotaxin, so-called an autotaxin-mediated disease.

In particular, the autotaxin-mediated disease may be a disease caused by the overexpression or overactivation of autotaxin.

In one embodiment, the autotaxin-mediated disease may be a cell proliferative disorder or fibrosis.

The cell proliferative disorder includes, but is not limited to, cancerous hyperproliferative disorders (*e.g.,* brain, lung, squamous cell, bladder, gastric, pancreatic, breast, head, neck, renal, liver, kidney, ovarian, prostate, colorectal, colon, epidermoid, esophageal, testicular, gynecological or thyroid cancer, acute myeloid leukemia, multiple myeloma, mesothelioma, non-small cell lung carcinoma (NSCLC), small cell lung cancer (SCLC), neuroblastoma, and acute lymphoblastic leukemia (ALL))); non-cancerous hyperproliferative disorders (*e.g.,* benign hyperplasia of the skin (*e.g.,* psoriasis), restenosis, and benign prostatic hypertrophy (BPH)); and diseases related to vasculogenesis or angiogenesis (*e.g.,* tumor angiogenesis, hemangioma, glioma, melanoma, Kaposi's sarcoma and ovarian, breast, lung, pancreatic, prostate, colon and epidermoid cancer). The cell proliferative disorder further includes primary and metastatic cancers. In particular, the cell proliferative disorder includes pseudomyxoma, intrahepatic cholangiocarcinoma, hepatoblastoma, liver cancer, colon cancer, testicular cancer, myelodysplastic syndrome, glioblastoma, oral cancer, lip cancer, oropharynx-tonsillar cancer (HPV-associated), oropharynx-hypopharyngeal cancer (non-HPV-associated), mycotic cell carcinoma, basal cell carcinoma, epithelial ovarian cancer, ovarian germ cell tumor, male breast cancer, brain tumor, pituitary adenoma, choledochocholecystic tumors, gallbladder cancer, biliary tract cancer, colorectal cancer, chronic myeloid leukemia, chronic lymphocytic leukemia, retinoblastoma, choroidal melanoma, diffuse large B cell lymphoma, ampullar of vater cancer, bladder cancer, peritoneal cancer, parathyroid cancer, adrenal cancer, nasal and paranasal cavity cancer, non-hodgkin's lymphoma, tongue cancer, astrocytoma, juvenile brain tumor, juvenile lymphoma, juvenile leukemia, small intestine cancer, meningioma, esophageal cancer, glioma, renal pelvis cancer, renal cancer, heart cancer, duodenal cancer, malignant soft tissue cancer, malignant bone cancer, malignant lymphoma, malignant mesothelioma, malignant melanoma, eye cancer, vulvar cancer, ureteral cancer, urethral cancer, cancer of unknown primary site, gastric lymphoma, gastric cancer, gastric carcinoid, gastrointestinal stromal tumor, Wilms' tumor, breast cancer, sarcoma, penile cancer, gestational choriocarcinoma, cervical cancer, endometrial cancer, uterine sarcoma, prostate cancer, metastatic bone tumor, metastatic brain tumor, mediastinal cancer, rectal cancer, neuroendocrine tumor, rectal carcinoid, vaginal cancer, spinal cord tumor, vestibular schwannoma, pancreatic cancer, salivary gland cancer, Paget's disease, squamous cell cancer, adenocarcinoma of lung, lung cancer, squamous cell lung cancer, skin cancer, anal cancer, rhabdomyosarcoma, laryngeal cancer, and pleura cancer, but are not limited thereto, and including a disease caused by metastasis of these diseases, or a variant including a mutation caused by relapse.

In addition, the fibrosis includes diseases such as cystic fibrosis, idiopathic pulmonary fibrosis, liver fibrosis, kidney fibrosis, scleroderma, radiation-induced fibrosis, Peyronie's disease, scarring and other diseases where excessive fibrosis contributes to disease pathology. In addition, the fibrosis is selected from the group consisting of mediastinal fibrosis, myelofibrosis, retroperitoneal fibrosis, progressive massive fibrosis, nephrogenic systemic fibrosis, Crohn's Disease, keloid, systemic sclerosis, arthrofibrosis, Dupuytren's contracture, adhesive capsulitis, fibrosis of the pancreases, fibrosis of the intestine, liver fibrosis, lung fibrosis, kidney fibrosis, cardiac fibrosis, fibrostenosis, cystic fibrosis, idiopathic pulmonary fibrosis, radiation-induced fibrosis, Peyronie's disease and scleroderma or is associated with respiratory disease, abnormal wound healing and repair, scarring, hypertrophic scarring/keloids, scarring post-surgery, cardiac arrest and all conditions where excess or aberrant deposition of fibrous material is associated with disease, injury, implants or surgery. In another embodiment, the fibrosis is selected from the group consisting of liver fibrosis, lung fibrosis, kidney fibrosis, cardiac fibrosis, scarring and scleroderma. In addition, the fibrosis is selected from the group consisting of myelofibrosis, systemic sclerosis, liver fibrosis, pulmonary fibrosis, kidney fibrosis, cardiac fibrosis and radiation-induced fibrosis. For example, kidney fibrosis includes, but is not limited to, diabetic nephropathy, vesicoureteral reflux, tubulointerstitial renal fibrosis, glomerulonephritis or glomerular nephritis, including focal segmental glomerulosclerosis and membranous glomerulonephritis, IgA nephropathy and mesangiocapillary glomerular nephritis. For example, liver fibrosis results in cirrhosis, and includes associated conditions such as chronic viral hepatitis, non-alcoholic fatty liver disease (NAFLD), alcoholic steatohepatitis (ASH), non-alcoholic steatohepatitis (NASH), primary biliary cirrhosis (PBC), biliary cirrhosis, and autoimmune hepatitis. In addition, the fibrosis is selected from keloid, scarring, ocular scarring, hypertrophic scarring, scleroderma, Dupuytren's contracture and Peyronie's disease. For example, the hypertrophic scarring results from a burn. Specifically, the hypertrophic scarring is caused by external injuries. In another embodiment, the hypertrophic scarring is caused by surgical procedures. In one embodiment, the keloid is caused by external injuries. In another embodiment, the keloid is caused by surgical procedures. In a further embodiment, the keloid is a result of a skin injury caused by acne, burns, chicken pox, ear piercing, scratches, surgical cuts or vaccination sites.

In another embodiment, the autotaxin-mediated disease may be an inflammatory disease, an autoimmune disease, a respiratory disease, a cardiovascular disease, a neurodegenerative disease, a skin disease, a disease associated with abnormal angiogenesis, and the like, but is not limited thereto.

The pharmaceutical composition of the present invention may further include a pharmaceutically acceptable carrier or additive.

The active ingredient of the present invention may be administered alone or in combination with any convenient carrier, and the like, and the dosage form may be a single-dose unit or multiple-dose unit. The pharmaceutical composition may be a solid formulation or a liquid formulation. The solid formulation includes, but is not limited to, a powder, a granule, a tablet, a capsule, a suppository, and the like. The solid formulation may include, but is not limited to, a carrier, a flavor, a binder, a preservative, a disintegrant, a lubricant, a filler, and the like. The liquid formulation includes water, a solution such as a propylene glycol solution, a suspension, an emulsion, and the like, but is not limited thereto, and may be prepared by adding suitable colorants, flavors, stabilizers, thickeners, and the like. For example, a powder may be prepared by simply mixing the active ingredient of the present invention with a suitable pharmaceutically acceptable carrier such as lactose, starch, or microcrystalline cellulose. A granule may be prepared by mixing the active ingredient of the present invention, a suitable pharmaceutically acceptable carrier, and a suitable pharmaceutically acceptable binder such as polyvinylpyrrolidone or hydroxypropyl cellulose, and then utilizing wet granulation using a solvent such as water, ethanol, or isopropanol, or dry granulation using a compressive force. Also, a tablet may be prepared by mixing the granule with a suitable pharmaceutically acceptable lubricant such as magnesium stearate, and then tableting the mixture using a tableting machine.

The pharmaceutical composition of the present invention may be administered in the form of oral formulation, injectable formulation (for example, intramuscular, intraperitoneal, intravenous, infusion, subcutaneous, implant), inhalable, intranasal, vaginal, rectal, sublingual, transdermal, topical, *etc.* depending on the disorders to be treated and the individual's conditions, but is not limited thereto. The composition of the present invention may be formulated in a suitable dosage unit formulation including a pharmaceutically acceptable and non-toxic carrier, additive and vehicle, which are generally used in the art, depending on the routes to be administered.

The pharmaceutical composition of the present invention may be administered at a daily dose of about 0.0001 mg/kg to about 10 g/kg, for example, about 0.001 mg/kg to about 1 g/kg. However, the dosage may vary with the degree of purification of the mixture, the condition of a patient (age, sex, body weight, *etc*.), the severity of the condition being treated, and the like. For convenience, a total daily dose of the pharmaceutical composition may be administered in multiple doses a day as needed.

In another aspect, the present invention provides a method of treating an autotaxin-mediated disease, such as a cell proliferative disorder or fibrosis, the method including administering to a subject a therapeutically effective amount of a compound having the chemical structure of Formula 1 above, or a pharmaceutically acceptable salt, an isomer, a solvate, a hydrate or a prodrug thereof.

In still another aspect, the present invention provides a method for inhibiting the activity of autotaxin, the method including administering to a subject a therapeutically effective amount of a compound having the chemical structure of Formula 1 above, or a pharmaceutically acceptable salt, an isomer, a solvate, a hydrate or a prodrug thereof.

Hereinafter, the present invention will be described in detail through Preparation Examples, Examples, and Experimental Examples.

Although Preparation Examples, Examples, and Experimental Examples below specifically illustrate the present invention, the contents of the present invention are not limited by Preparation Examples, Examples, and Experimental Examples below.

### [Preparation Examples]

### Preparation Example 1: Preparation of 3-((5-chloro-2-oxo-2,3-dihydro-1H-benzo[d]imidazol-1-yl)methyl)benzoic acid methyl ester

The process of the following steps A, B, and C gave the title compound.

### Step A: Preparation of 3-(((4-chloro-2-nitrophenyl)amino)methyl)benzoic acid methyl ester

To 3-(aminomethyl)benzoic acid methyl ester (17 g, 103 mmol), K₂CO₃ (28.4 g, 206 mmol), and 4-chloro-1-fluoro-2-nitrobenzene (18 g, 103 mmol) was added 150 mL of DMF, followed by charging with nitrogen gas. The resulting mixture was stirred at room temperature for 2 hours, and then when the reaction was terminated, EtOAC and water were added thereto, and the organic layer was separated and dried over anhydrous MgSO₄. The filtrate was distilled under reduced pressure and then purified by MPLC to give the title compound (20 g, yield: 61%).
¹H NMR (500 MHz, CDCl3) δ 8.45 (brs, 1H), 8.23 (s, 1H), 8.02 (m, 2H), 7.55 (m, 1H), 7.48 (m, 1H), 7.34 (m, 1H), 6.75 (d, 1H), 4.62 (d, 2H), 3.95 (s, 3H)

### Step B: Preparation of 3-(((2-amino-4-chlorophenyl)amino)methyl)benzoic acid methyl ester

To 3-(((4-chloro-2-nitrophenyl)amino)methyl)benzoic acid methyl ester (0.35 g, 1.09 mmol) obtained in step A above and iron (274 mg, 4.91 mmol) were added 1 mL of EtOH and 3 mL of water. The reaction solution was stirred at 100 °C for 3 hours by the addition of 0.6 mL of acetic acid. When the reaction was terminated, the reaction solution was diluted with EtOAc at room temperature, basified with a 5 M aqueous NaOH solution, the resulting solid was removed through Celite, and then an organic layer was washed with water and separated. The filtrate was dried over anhydrous MgSO₄, distilled under reduced pressure to give the title compound (0.32 g, yield: 99%) .
¹H NMR (500 MHz, CDCl3) δ 8.09 (brs, 1H), 7.98 (d, 1H), 7.59 (d, 1H), 7.45 (t, 1H), 7.28 (m, 2H) 6.75 (m, 2H), 6.53 (d, 1H), 4.37 (s, 2H), 3.95 (s, 3H)

### Step C: Preparation of 3-((5-chloro-2-oxo-2,3-dihydro-1H-benzo[d]imidazol-1-yl)methyl)benzoic acid methyl ester

To 3-(((2-amino-4-chlorophenyl)amino)methyl)benzoic acid methyl ester (367 mg, 1.26 mmol) obtained in step B above and CDI (408 mg, 2.52 mmol) was added 15 mL of THF, and the resulting mixture was stirred at 70 °C for 12 hours. After the reaction was terminated, the filtrate was distilled under reduced pressure and then purified by MPLC to give the title compound (240 mg, yield: 60%).
¹H NMR (500 MHz, CDCl3) δ 8.31 (brs, 1H), 8.00 (m, 2H), 7.51 (d, 1H), 7.43 (t, 1H), 7.10 (s, 1H), 7.00 (d, 1H), 6.74 (d, 1H), .5.11 (s, 2H), 3.93 (s, 3H)

### Preparation Example 2: Preparation of 3-((5-chloro-2-oxo-2,3-dihydro-1H-benzo[d]imidazol-1-yl)methyl)-2-fluorobenzoic acid methyl ester

The process of the following steps A, B, and C gave the title compound.

### Step A: Preparation of 3-(((4-chloro-2-nitrophenyl)amino)methyl)-2-fluorobenzoic acid methyl ester

The title compound (2.53 g, yield: 68%) was obtained in the same manner as in step A of Preparation Example 1 by using 3-(aminomethyl)-2-fluorobenzoic acid methyl ester (2.2 g, 12.01 mmol) and 4-chloro-1-fluoro-2-nitrobenzene.
¹H NMR (500 MHz, CDCl3) δ 8.45 (brs, 1H), 8.23 (s, 1H), 8.02 (m, 2H), 7.55 (m, 1H), 7.48 (m, 1H), 7.34 (m, 1H), 6.75 (d, 1H), 4.62 (d, 2H), 3.95 (s, 3H)

### Step B: Preparation of 3-(((2-amino-4-chlorophenyl)amino)methyl)-2-fluorobenzoic acid methyl ester

The title compound (2.36 g, yield: 99%) was obtained in the same manner as in step B of Preparation Example 1 by using 3-(((4-chloro-2-nitrophenyl)amino)methyl)-2-fluorobenzoic acid methyl ester (2.5 g, 7.38 mmol) obtained in step A above.
¹H NMR (500 MHz, CDCl3) δ 7.87 (t, 1H), 7.70 (m, 1H), 7.53 (m, 2H), 7.18 (t, 1H), 6.73 (m, 1H), 6.51 (m, 1H), 4.43 (s, 2H), 3.97 (s, 3H).

### Step C: Preparation of 3-((5-chloro-2-oxo-2,3-dihydro-IH-benzo[d]imidazol-1-yl)methyl)-2-fluorobenzoic acid methyl ester

The title compound (0.66 g, yield: 28%) was obtained in the same manner as in step C of Preparation Example 1 by using 3-(((2-amino-4-chlorophenyl)amino)methyl)-2-fluorobenzoic acid methyl ester (2.36 g, 6.97 mmol) obtained in step B above.
¹H NMR (500 MHz, CDCl3) δ 8.95 (brs, 1H), 7.90 (t, 1H), 7.51 (t, 1H), 7.18 (t, 1H), 7.12 (m, 1H), 7.05 (d, 1H), 6.90 (d, 1H), 5.17 (s, 2H), 3.97 (s, 2H)

### Preparation Example 3: Preparation of 3-(1-(5-chloro-2-oxo-2,3-dihydro-1H-benzo[d]imidazol-1-yl)cyclopropyl)benzoic acid methyl ester

The title compound (97 mg, yield: 9%) was obtained in the same manner as in steps A, B, and C of Preparation Example 1 by using 3-(1-aminocyclopropyl)benzoic acid methyl ester (575 mg, 12.01 mmol) and 4-chloro-1-fluoro-2-nitrobenzene.
¹H NMR (500 MHz, CDCl3) δ 10.67 (brs, 1H), 7.85 (m, 2H), 7.34 (m, 2H), 7.13 (s, 1H), 7.03 (s, 2H), 3.90 (s, 3H), 1.73 (s, 4H)

### Preparation Example 4: Preparation of 3-(1-(5-chloro-2-oxo-2,3-dihydro-1H-benzo[d]imidazol-1-yl)cyclopropyl)-2-fluorobenzoic acid methyl ester

The process of the following steps A and B gave the title compound.

### Step A: Preparation of 3-(1-aminocyclopropyl)-2-fluorobenzoic acid methyl ester

To 3-cyano-2-fluorobenzoic acid methyl ester (5.4 g, 30 mmol) were added 150 mL of Et₂O and Ti(O-Pr)₄ (9.82 mL, 33 mmol), and the resulting mixture was cooled to -78 °C, and then ethyl magnesium bromide (3M Et₂O solution) was slowly added thereto. The resultant mixture was stirred at room temperature for 1 hour, then BF₃-OEt₂ (6.37 mL, 60 mmol) was added thereto, and the resulting mixture was stirred again at room temperature for 1 hour. To the reaction solution was added 90 mL of 1 N aqueous hydrochloric acid solution, and then the reaction solution was rinsed with Et₂O. A water layer was separated, basified with a 2.5 M aqueous NaOH solution, and then extracted with Et₂O to obtain an organic layer. The organic layer was dried over anhydrous Na₂SO₄, then the resulting solid was filtered, and the filtrate was concentrated under reduced pressure to give the title compound (3.1 g, yield: 49%) .
¹H NMR (500 MHz, CDCl3) δ 7.81 (m, 1H), 6.51 (m, 1H), 7.14 (m, 1H), 3.96 (s, 3H), 2.00 (brs, 2H), 1.05 (m, 2H), 0.92 (m, 2H)

### Step B: Preparation of 3-(1-(5-chloro-2-oxo-2,3-dihydro-1H-benzo[d]imidazol-1-yl)cyclopropyl)-2-fluorobenzoic acid methyl ester

The title compound (278 mg, yield: 7%) was obtained in the same manner as in steps A, B, and C of Preparation Example 1 by using 3-(1-aminocyclopropyl)-2-fluorobenzoic acid methyl ester (2.2 g, 10.51 mmol) obtained in step A above and 4-chloro-1-fluoro-2-nitrobenzene.
¹H NMR (500 MHz, CDCl3) δ 8.39 (brs, 1H), 8.09 (t, 1H), 8.56 (t, 1H), 7.44 (d, 1H), 7.20 (t, 1H), 7.11 (d, 1H), 7.03 (s, 1H), 3.93 (s, 3H), 1.65 (m, 4H)

### Preparation Example 5: Preparation of 6-chloro-1-phenyl-1,3-dihydro-2H-benzo[d]imidazol-2-one

The process of the following steps A, B, and C gave the title compound.

### Step A: Preparation of 5-chloro-2-nitro-N-phenylaniline

4-Chloro-2-fluoro-1-nitrobenzene (877.70 mg, 5.00 mmol), aniline (1.37 mL, 15.00 mmol), and potassium carbonate (1.72 g, 12.50 mmol) were dissolved in dimethyl sulfoxide and the resulting mixture was stirred at 50 °C. After the reaction was terminated, water was added thereto, and the reaction mixture was extracted with ethyl acetate to separate an organic layer, and then the organic layer was dried over anhydrous Na₂SO₄. The resulting solid was filtered, and the filtrate was distilled under reduced pressure and then purified by MPLC to give the title compound (1.23 g, yield: 99%).
¹H NMR (400 MHz, CDCl3) δ 9. 55 (s, 1H), 8.17 (m 1H), 7.45 (m, 2H), 7.28 (m, 3H), 7.14 (m, 1H), 6.72 (m, 1H)

### Step B: Preparation of 5-chloro-N¹-phenylbenzene-1,2-diamine

The title compound (436.00 mg, yield: 99%) was obtained in the same manner as in step B of Preparation Example 1 by using 5-chloro-2-nitro-*N*-phenylaniline (500.00 mg, 2.01 mmol) obtained in step A above.
¹H NMR (500 MHz, CDCl3) δ 7.25 (m, 2H), 7.12 (s, 1H), 6.94 (d, 2H), 6.90 (t, 1H), 6.80 (d, 2H), 6.72 (d, 1H), 5.17 (s, 1H), 3.71 (s, 2H)

### Step C: Preparation of 6-chloro-1-phenyl-1,3-dihydro-2H-benzo[d]imidazol-2-one

The title compound (445.90 mg, yield: 91%) was obtained in the same manner as in step C of Preparation Example 1 by using 5-chloro-*N*¹-phenylbenzene-1,2-diamine (437.36 mg, 2.00 mmol) obtained in step B above.
¹H NMR (400 MHz, CDCl3) δ 8.99 (s, 1H), 7.59 (t, 2H), 7.53 (d, 2H), 7.47 (t, 1H), 7.09 (d, 1H), 7.05 (d, 2H)

### Preparation Example 6: Preparation of 3-(aminomethyl)-5-fluorobenzoic acid methyl ester

To 3-(bromomethyl)-5-fluorobenzoic acid methyl ester (1.08 g. 4.37 mmol) was added 15 mL of DMF and sodium azide (0.34 g, 5.25 mmol), and the resulting mixture was stirred at 70 °C for 1 hour. After the reaction was terminated, water and Et₂O were added thereto to separate an organic layer. The organic layer was concentrated under reduced pressure, then 15 mL of MeOH and 15 mL of DCM were added thereto, followed by the addition of 10 wt% Pd/C (0.40 g), and the resulting mixture was stirred under hydrogen atmospheric pressure. After the reaction was terminated, the resulting solid was filtered, and the filtrate was concentrated under reduced pressure to give the title compound (0.61 g, yield: 76%).
¹H-NMR (400 MHz, DMSO-d6) δ7.80 (d, 1H), 7.50 (2H), 3.87 (s, 3H), 3.86 (s, 1H), 3.76 (s, 1H)

### Preparation Example 7: Preparation of 6-chloro-1-(6-propoxypyridin-3-yl)-1,3-dihydro-2H-benzo[d]imidazol-2-one

The process of the following steps A, B, C, D and E gave the title compound.

### Step A: Preparation of 5-nitro-2-propoxypyridine

To 1-propanol (0.934 mL, 12.4 mmol) was added 18 mL of MTBE, and the resulting mixture was cooled to 0 °C and KOtBu (1.39 g, 12.4 mmol) and 2-fluoro-5-nitropyridine (1.61 g, 11.3 mmol) were added thereto. After the reaction was terminated, water was added thereto, and the organic layer was separated and dried over anhydrous Na₂SO₄. The resulting solid was filtered, and the filtrate was distilled under reduced pressure and then purified by MPLC to give the title compound (1.57 g, yield: 76%).
¹H NMR (400 MHz, CDCl3) δ 9.07 (d, 1H), 8.34 (dd, 1H), 6.81 (d, 1H), 4.38 (t, 2H), 1.82 (m, 2H), 1.04 (t, 3H)

### Step B: Preparation of 6-propoxypyridin-3-amine

To 5-nitro-2-propoxypyridine (1.57 g, 8.62 mmol) obtained in step A above were added 15 mL of each of THF, EtOH, and water, iron (3.37 g, 60 mmol) and ammonium chloride (3.23 g, 60 mmol) were added thereto, and the resulting mixture was stirred under reflux for 2 hours. After the reaction was terminated, the mixture was diluted with water and EtOAc, and then the resulting solid was removed through Celite. The organic layer was separated and then dried over anhydrous Na₂SO₄ to give the title compound (1.28 g, yield: 98%).
¹H NMR (400 MHz, CDCl3) δ 7.65 (d, 1H), 7.03 (dd, 1H), 6.60 (d, 1H), 4.14 (t, 2H), 3.35 (brs, 2H), 1.76 (m, 2H), 1.00 (t, 3H)

### Step C: Preparation of N-(5-chloro-2-nitrophenyl)-6-propoxypyridine-3-amine

To 6-propoxypyridine-3-amine (1.28 g, 8.41 mmol) obtained in step B above and 4-chloro-2-fluoro-1-nitrobenzene (1.62 g, 9.25 mmol) were added 28 mL of AN and K₂CO₃ (2.30 g, 19.3 mmol), and the resulting mixture was stirred at 80 °C for 3 days. The resulting solid was filtered and then the filtrate was purified by MPLC to give the title compound (1.05 g, yield: 40%) .
¹H NMR (400 MHz, CDCl3) δ 9.33 (brs, 1H), 8.16 (d, 1H), 8.10 (d, 1H), 7.50 (dd, 1H), 6.84 (2H), 6.72 (dd, 1H), 4.29 (t, 2H), 1.82 (m, 2H), 1.05 (t, 3H)

### Step D: Preparation of 6-chloro-1-(6-propoxypyridin-3-yl)-1,3-dihydro-2H-benzo[d]imidazol-2-one

The title compound (0.70 g, yield: 68%) was obtained in the same manner as in step B of Preparation Example 7 and step C of Preparation Example 3 by using *N*-(5-chloro-2-nitrophenyl)-6-propoxypyridine-3-amine (1.05 g, 3.41 mmol) obtained in step C above.
¹H NMR (400 MHz, CDCl3) δ 8.92 (brs, 1H), 8.30 (d, 1H), 7.70 (dd, 1H), 7.09 (d, 1H), 7.03 (d, 1H), 6.93 (d, 1H), 6.90 (d, 1H), 4.32 (t, 2H), 1.83 (m, 2H), 1.06 (t, 3H)

### Preparation Example 8: Preparation of 6-chloro-1-(4-(dimethylamino)phenyl)-7-fluoro-1,3-dihydro-2H-benzo[d]imidazol-2-one

The process of the following steps A and B gave the title compound.

### Step A: Preparation of N¹-(3-chloro-2-fluoro-6-nitrophenyl)-N⁴,N⁴-dimethylbenzene-1,4-diamine

To *N*¹,*N*¹-dimethylbenzene-1,4-diamine (2.60 g, 19.1 mmol) was added 19 mL of DMF, 1-chloro-2,3-difluoro-4-nitrobenzene (3.70 g, 19.1 mmol), and K₂CO₃ (5.28 g, 38.2 mmol), and then the resulting mixture was stirred at 70 °C for 2 hours. After the reaction was terminated, cold water was added thereto, and the resulting solid was dried to give the title compound (5.92 g, yield: 99%).
¹H NMR (400 MHz, DMSO-d6) δ 8.86 (brs, 1H), 7.90 (dd, 1H), 7.10 (dd, 1H), 6.94 (d, 2H), 6.66 (d, 2H), 2.86 (s, 6H)

### Step B: Preparation of 6-chloro-1-(4-(dimethylamino)phenyl)-7-fluoro-1,3-dihydro-2H-benzo[d]imidazol-2-one

5-Chloro-*N*¹-(4-(dimethylamino)phenyl)-6-fluorobenzene-1,2-diamine was obtained in the same manner as in step B of Preparation Example 7 by using *N*¹-(3-chloro-2-fluoro-6-nitrophenyl)-*N*⁴,*N*⁴-dimethylbenzene-1,4-diamine (2.60 g, 8.39 mmol) obtained in step A above. To this was added 200 mL of DCM, and the resulting mixture was cooled to 0 °C, and DIPEA (8.34 mL, 47.8 mmol) and triphosgene (5.67 g, 19.1 mmol) were sequentially added thereto. After 30 minutes, water was added thereto and an organic layer was separated. The organic layer was dried over anhydrous Na₂SO₄ and purified by MPLC to give the title compound (3.25 g, yield: 56%).
¹H NMR (400 MHz, DMSO-d6) δ 11.42 (brs, 1H), 7.26 (d, 2H), 7.16 (dd, 1H), 6.89 (dd, 1H), 6.82 (m, 2H), 2.97 (s, 6H)

### Preparation Example 9: Preparation of 6-chloro-1-(1-(tetrahydro-2H-pyran-4-yl)-1H-pyrazol-4-yl)-1,3-dihydro-2H-benzo[d]imidazol-2-one

The process of the following steps A, B, and C gave the title compound.

### Step A: Preparation of 1-(tetrahydro-2H-pyran-4-yl)-1H-pyrazol-4-amine

To 4-nitro-1-(tetrahydro-2*H*-pyran-4-yl)-1*H*-pyrazole (1.44 g, 7.30 mmol) was added 50 mL of EtOH, and then the resulting mixture was charged with nitrogen gas. To this was added 10 wt% of Pd-C (0.777 g, 0.730 mmol) and the resulting mixture was stirred under hydrogen atmospheric pressure. After the reaction was terminated, the resulting solid was filtered and the filtrate was purified by MPLC to give the title compound (1.17 g, yield: 96%).
¹H NMR (400 MHz, CDCl3) δ 7.17 (s, 1H), 7.06 (s, 1H), 4.22 (m, 1H), 4.08 (m, 2H), 3.51 (m, 2H), 2.90 (brs, 2H), 2.01 (4H)

### Step B: Preparation of N-(5-chloro-2-nitrophenyl)-1-(tetrahydro-2H-pyran-4-yl)-1H-pyrazol-4-amine

The title compound (0.65 g, yield: 26%) was obtained in the same manner as in step C of Preparation Example 7 by using 1-(tetrahydro-2*H*-pyran-4-yl)-1*H*-pyrazol-4-amine (1.17 g, 7.00 mmol) and 4-chloro-2-fluoro-1-nitrobenzene (1.35 g, 7.70 mmol) obtained in step A above.
¹H NMR (500 MHz, CDCl3) δ 9.09 (brs, 1H), 8.13 (d, 1H), 7.53 (s, 1H), 7.49 (s, 1H), 6.97 (d, 1H), 6.70 (dd, 1H), 4.40 (m, 1H), 4.14 (m, 2H), 3.57 (m, 2H), 2.10 (m, 4H)

### Step C: Preparation of 6-chloro-1-(1-(tetrahydro-2H-pyran-4-yl)-1H-pyrazol-4-yl)-1,3-dihydro-2H-benzo[d]imidazol-2-one

The title compound (0.29 g, yield: 45%) was obtained in the same manner as in step B of Preparation Example 7 and step B of Preparation Example 8 by using *N*-(5-chloro-2-nitrophenyl)-1-(tetrahydro-2*H*-pyran-4-yl)-1*H*-pyrazol-4-amine (0.65 g, 2.01 mmol) obtained in step B above.
¹H NMR (400 MHz, CDCl3) δ 9.00 (brs, 1H), 7.83 (s, 1H), 7.78 (s, 1H), 7.08 (2H), 7.01 (d, 1H), 4.40 (m, 1H), 4.14 (m, 2H), 3.58 (m, 2H), 2.18 (m, 4H)

### Preparation Example 10: Preparation of N-(5-chloro-2-nitrophenyl)-1H-pyrazol-4-amine

The process of the following steps A, B, and C gave the title compound.

### Step A: Preparation of 4-nitro-1H-pyrazole-1-carboxylic acid tert-butyl ester

4-Nitro-1*H*-pyrazole (15.0 g, 133 mmol) was dissolved in 200 mL of DCM, and then dimethylaminopyridine (1.62 g, 13.2 mmol) and BOC₂O (30.4 g, 139 mmol) were added thereto. The resulting mixture was stirred at room temperature for 1 hour, and then a 0.5 N aqueous hydrochloric acid solution was added thereto. An organic layer was separated and dried over Na₂SO₄, and the resulting solid was removed to give the title compound (28.3 g, yield: 100%).
¹H NMR (400 MHz, CDCl3) δ 8.79 (s, 1H), 8.22 (s, 1H), 1.69 (s, 9H)

### Step B: Preparation of 4-amino-1H-pyrazole-1-carboxylic acid tert-butyl ester

The title compound (8.73 g, yield: 94%) was obtained in the same manner as in step A of Preparation Example 9 by using 4-nitro-1*H*-pyrazole-1-carboxylic acid *tert*-butyl ester (10.8 g, 50.7 mmol) obtained in step A above.
¹H NMR (400 MHz, CDCl3) δ 7.55 (s, 1H), 7.41 (s, 1H), 3.12 (brs, 2H), 1.63 (s, 9H)

### Step C: Preparation of N-(5-chloro-2-nitrophenyl)-1H-pyrazol-4-amine

The title compound (1.00 g, yield: 19%) was obtained in the same manner as in step A of Preparation Example 8 by using 4-amino-1*H*-pyrazole-1-carboxylic acid *tert*-butyl ester (4.00 g, 21.8 mmol) obtained in step B above and 4-chloro-2-fluoro-1-nitrobenzen (3.83 g, 21.8 mmol).
¹H NMR (400 MHz, CDCl3) δ 9.14 (brs, 1H), 8.17 (d, 1H), 8.15 (s, 1H), 7.76 (s, 1H), 6.99 (d, 1H), 6.76 (dd, 1H), 1.67 (s, 9H)

### Preparation Example 11: Preparation of 6-chloro-7-fluoro-1-(4-morpholinophenyl)-1,3-dihydro-2H-benzo[d]imidazol-2-one

The process of the following steps A, B, C and D gave the title compound.

### Step A: Preparation of 4-(4-nitrophenyl)morpholine

To 1-fluoro-4-nitrobenzene (5.0 g, 35.4 mmol) was added 17 mL of morpholine, and the resulting mixture was stirred at 80 °C for 4 hours. The reaction solution was poured into 400 mL of cold water and the resulting solid was filtered to give the title compound (7.38 g, yield: 99%).
¹H NMR (400 MHz, DMSO-d6) δ 8.08 (d, 2H), 7.04 (d, 2H), 3.83 (m, 4H), 3.41 (m, 4H)

### Step B: Preparation of 4-morpholinoaniline

The title compound (6.22 g, yield: 98%) was obtained in the same manner as in step B of Preparation Example 7 by using 4-(4 - nitrophenyl)morpholine (7.38 g, 35.40 mmol) obtained in step A above.
¹H NMR (500 MHz, DMSO-d6) δ 6.68 (d, 2H), 6.59 (d, 2H), 4.57 (brs, 2H), 3.67 (m, 4H), 2.86 (m, 4H)

### Step C: Preparation of 3-chloro-2-fluoro-N-(4-morpholinophenyl)-6-nitroaniline

The title compound (2.57 g, yield: 94%) was obtained in the same manner as in step A of Preparation Example 8 by using 4-morpholinoaniline (1.38 g, 7.75 mmol) obtained in step B above and 1-chloro-2,3-difluoro-4-nitrobenzene (1.50 g, 7.75 mmol).
¹H NMR (400 MHz, DMSO-d6) δ 8.33 (brs, 1H), 7.90 (dd, 1H), 7.18 (dd, 1H), 6.95 (d, 2H), 6.87 (d, 2H), 3.73 (m, 4H), 3.03 (m, 4H)

### Step D: Preparation of 6-chloro-7-fluoro-1-(4-morpholinophenyl)-1,3-dihydro-2H-benzo[d]imidazol-2-one

The title compound (1.18 g, yield: 46%) was are used obtained in the same manner as in step B of Preparation Example 8 by using 3-chloro-2-fluoro-*N*-(4-morpholinophenyl)-6-nitroaniline (2.57 g, 7.31 mmol) obtained in step C above.
¹H NMR (400 MHz, DMSO-d6) δ 11.45 (s, 1H), 7.33 (d, 2H), 7.18 (dd, 1H), 7.04 (d, 2H), 6.90 (dd, 1H), 3.77 (m, 4H), 3.18 (m, 4H)

### Preparation Example 12: Preparation of 6-chloro-1-(1-propyl-1H-pyrazol-4-yl)-1,3-dihydro-2H-benzo[d]imidazol-2-one

The process of the following steps A, B, and C gave the title compound.

### Step A: Preparation of 1-propyl-1H-pyrazol-4-amine

To 4-bromo-1-propyl-1*H*-pyrazole (1.00 g, 5.29 mmol), K₂CO₃ (1.09 g, 7.93 mmol), and acetamide (0.63 g, 10.6 mmol) was added 5.3 mL of *n*-butanol, and the resulting mixture was charged with nitrogen gas. To this were added DMEDA (0.074 mL, 0.69 mmol) and CuI (0.131 g, 0.688 mmol), and the resultant mixture was stirred at 100 °C for 16 hours. After the reaction was terminated, the resulting solid was removed through Celite. The filtrate was concentrated, 13 mL of EtOH and an aqueous hydrochloric acid solution (0.87 mL, 10.6 mmol) were added thereto, and the resulting mixture was stirred at 70 °C for 3 days. After the reaction was terminated, the reaction mixture was concentrated under reduced pressure, and the filtrate was neutralized with a 1 N aqueous NaOH solution and extracted with EtOAc. The organic layer was separated and then dried over anhydrous Na₂SO₄ to give the title compound (0.39 g, yield: 59%).
¹H NMR (400 MHz, DMSO-d6) δ 7.01 (s, 1H), 6.88 (s, 1H), 4.00 (brs, 2H), 3.85 (t, 2H), 1.67 (m, 2H), 0.81 (t, 3H)

### Step B: Preparation of N-(5-chloro-2-nitrophenyl)-1-propyl-1H-pyrazol-4-amine

The title compound (0.41 g, yield: 46%) was obtained in the same manner as in step C of Preparation Example 7 by using 1-propyl-1*H*-pyrazol-4-amine (0.39 g, 3.12 mmol) obtained in step A above and 4-chloro-2-fluoro-1-nitrobenzene (0.57 g, 3.27 mmol).
¹H NMR (500 MHz, CDCl3) δ 9.08 (brs, 1H), 8.13 (d, 1H), 7.50 (s, 1H), 7.44 (s, 1H), 6.97 (d, 1H), 6.69 (dd, 1H), 4.00 (t, 2H), 1.83 (m, 2H), 0.88 (t, 3H)

### Step C: Preparation of 6-chloro-1-(1-propyl-1H-pyrazol-4-yl)-1,3-dihydro-2H-benzo[d]imidazol-2-one

The title compound (0.077 g, yield: 18%) was obtained in the same manner as in step B of Preparation Example 7 and step C of Preparation Example 3 by using *N*-(5-chloro-2-nitrophenyl)-1-propyl-1*H*-pyrazol-4-amine (0.41 g, 1.5 mmol) obtained in step B above.
¹H NMR (400 MHz, CDCl3) δ 8.90 (brs, 1H), 7.78 (s, 1H), 7.76 (s, 1H), 7.08 (2H), 7.02 (d, 1H), 4.16 (t, 2H), 1.98 (m, 2H), 1.00 (t, 3H)

### Preparation Example 13: Preparation of 6-chloro-7-fluoro-1-(1-propyl-1H-pyrazol-4-yl)-1,3-dihydro-2H-benzo[d]imidazol-2-one

The process of the following steps A and B gave the title compound.

### Step A: Preparation of N-(3-chloro-2-fluoro-6-nitrophenyl)-1-propyl-1H-pyrazol-4-amine

The title compound (0.25 g, yield: 33%) was obtained in the same manner as in step A of Preparation Example 8 by using 1-propyl-1*H*-pyrazol-4-amine (0.32 g, 2.58 mmol) obtained in step A of Preparation Example 12 and 1-chloro-2,3-difluoro-4-nitrobenzene (0.50 g, 2.58 mmol).
¹H NMR (400 MHz, CDCl3) δ 8.92 (brs, 1H), 7.97 (dd, 1H), 7.42 (s, 1H), 7.34 (s, 1H), 6.82 (dd, 1H), 4.06 (q, 2H), 1.88 (m, 2H), 0.93 (t, 3H)

### Step B: Preparation of 6-chloro-7-fluoro-1-(1-propyl-1H-pyrazol-4-yl)-1,3-dihydro-2H-benzo[d]imidazol-2-one

The title compound (0.14 g, yield: 58%) was obtained in the same manner as in step B of Preparation Example 7 and step C of Preparation Example 3 by using *N*-(3-chloro-2-fluoro-6-nitrophenyl)-1-propyl-1*H*-pyrazol-4-amine (0.25 g, 0.84 mmol) obtained in step A above.
¹H NMR (400 MHz, CDCl3) δ 9.23 (brs, 1H), 7.71 (2H), 7.11 (m, 1H), 6.85 (d, 1H), 4.14 (t, 2H), 1.95 (m, 2H), 0.98 (t, 3H)

### Preparation Example 14: Preparation of 1-(4-(6-chloro-7-fluoro-2-oxo-2,3-dihydro-1H-benzo[d]imidazol-1-yl)phenyl)azetidin-3-yl acetate

The process of the following steps A, B, C, D and E gave the title compound.

### Step A: Preparation of 1-(4-nitrophenyl)azetidin-3-ol

To azetidine-3-ol hydrochloride (1.97 g, 17.9 mmol) and (4-nitrophenyl)boronic acid (3.0 g, 17.9 mmol) were added 180 mL of DCM, 10 mL of TEA, and Cu(OAc)₂ (3.26 g, 17.9 mmol), and the resulting mixture was stirred at room temperature for two days. The reaction solution was concentrated under reduced pressure and then purified by MPLC to give the title compound (0.58 g, yield: 16%).
¹H NMR (400 MHz, CDCl3) δ 8.11 (d, 2H), 6.33 (d, 2H), 4.87 (m, 1H), 4.31 (m, 2H), 3.90 (m, 2H), 2.16 (d, 1H)

### Step B: Preparation of 1-(4-aminophenyl)azetidin-3-ol

The title compound (0.49 g, yield: 99%) was obtained in the same manner as in step B of Preparation Example 7 by using 1-(4-nitrophenyl)azetidin-3-ol (0.58 g, 2.99 mmol) obtained in step A above.
¹H NMR (400 MHz, CDCl3) δ 6.64 (d, 2H), 6.38 (d, 2H), 4.70 (m, 1H), 4.11 (t, 2H), 3.67 (m, 2H), 3.38 (brs, 2H)

### Step C: Preparation of 1-(4-((3-chloro-2-fluoro-6-nitrophenyl)amino)phenyl)azetidin-3-ol

The title compound (0.77 g, yield: 76%) was obtained in the same manner as in step A of Preparation Example 8 by using 1-(4-aminophenyl)azetidin-3-ol (0.493 g, 3 mmol) obtained in step B above and 1-chloro-2,3-difluoro-4-nitrobenzene (0.581 g, 3.00 mmol).
¹H NMR (400 MHz, CDCl3) δ 9.10 (brs, 1H), 7.96 (dd, 1H), 6.98 (m, 2H), 6.81 (dd, 1H), 7.44 (d, 2H), 4.77 (m, 1H), 4.19 (m, 2H), 3.68 (m, 2H), 2.04 (d, 1H)

### Step D: Preparation of 1-(4-((3-chloro-2-fluoro-6-nitrophenyl)amino)phenyl)azetidin-3-yl acetate

To 1-(4-((3-chloro-2-fluoro-6-nitrophenyl)amino)phenyl)azetidin-3-ol (0.77 g, 2.28 mmol) obtained in step C were added 9 mL of DMSO and KOtBu (0.256 g, 2.28 mmol), and the resulting mixture was stirred at room temperature for 30 minutes. To this was added 4.5 mL of EtOAc, and the resultant mixture was stirred at room temperature for 16 hours. To the reaction solution were added water and EtOAc, and then an organic layer was separated and dried over anhydrous Na₂SO₄. This was purified by MPLC to give the title compound (0.367 g, yield: 42%).
¹H-NMR (400 MHz, CDCl3) δ9.08 (brs, 1H), 7.96 (dd, 1H), 7.00 (d, 2H), 6.82 (dd, 1H), 6.45 (d, 2H), 5.31 (m, 1H), 4.25 (m, 2H), 3.83 (m, 2H), 2.11 (s, 3H)

### Step E: Preparation of 1-(4-(6-chloro-7-fluoro-2-oxo-2,3-dihydro-1H-benzo[d]imidazol-1-yl)phenyl)azetidin-3-yl acetate

The title compound (0.21 g, yield: 56%) was obtained in the same manner as in step B of Preparation Example 8 by using 1-(4-((3-chloro-2-fluoro-6-nitrophenyl)amino)phenyl)azetidin-3-yl acetate (0.38 g, 1.00 mmol) obtained in step D above.
¹H-NMR (400 MHz, CDCl3) δ9.14 (brs, 1H), 7.30 (m, 2H), 7.08 (dd, 1H), 6.81 (dd, 1H), 6.55 (d, 2H), 5.34 (m, 1H), 4.30 (m, 2H), 3.88 (m, 2H), 2.12 (s, 3H)

### Preparation Example 15: Preparation of 3-((5-chloro-7-fluoro-2-oxo-2,3-dihydro-1H-benzo[d]imidazol-1-yl)methyl)benzoic acid methyl ester

The process of the following steps A and B gave the title compound.

### Step A: Preparation of 3-(((4-chloro-2-fluoro-6-nitrophenyl)amino)methyl)benzoic acid methyl ester

The title compound (0.88 g, yield: 69%) was obtained in the same manner as in step A of Preparation Example 8 by using 5-chloro-1,2-difluoro-3-nitrobenzene (0.726 g, 3.75 mmol) and 3-(aminomethyl)benzoic acid methyl ester (0.62 g, 3.75 mmol).
¹H-NMR (400 MHz, CDCl3) δ8.11 (brs, 1H), 8.00 (3H), 7.51 (dd, 1H), 7.46 (t, 1H), 7.20 (dd, 1H), 4.77 (m, 2H), 3.96 (s, 3H)

### Step B: Preparation of 3-((5-chloro-7-fluoro-2-oxo-2,3-dihydro-1H-benzo[d]imidazol-1-yl)methyl)benzoic acid methyl ester

The title compound (0.075 g, yield: 18%) was obtained in the same manner as in step B of Preparation Example 8 by using 3-(((4-chloro-2-fluoro-6-nitrophenyl)amino)methyl)benzoic acid methyl ester (0.42 g, 1.24 mmol) obtained in step A above.
¹H-NMR (500 MHz, MeOH-d4) δ7.93 (2H), 7.54 (m, 1H), 7.44 (m, 1H), 6.94 (m, 1H), 6.90 (m, 1H), 5.18 (s, 2H), 3.88 (s, 3H)

### Preparation Example 16: Preparation of 1-(4-((5-chloro-2-nitrophenyl)amino)phenyl)piperidin-2-one

The process of the following steps A and B gave the title compound.

### Step A: Preparation of 1-(4-aminophenyl)piperidin-2-one

To 4-bromoaniline (0.97 g, 5.6 mmol), piperidin-2-one (0.40 g, 4.0 mmol), CuI (38 mg, 0.20 mmol), K₂CO₃ (1.1 g, 8.1 mmol), and DMEDA (36 mg, 0.40 mmol) was added 4 mL of toluene and the resulting mixture was charged with nitrogen gas. The reaction solution was stirred at 110 °C for 16 hours and then the resulting solid was removed through Celite. The filtrate was purified by MPLC to give the title compound (0.21 g, yield: 27%) .
¹H-NMR (400 MHz, CDCl3) δ7.00 (d, 2H), 6.68 (d, 2H), 3.66 (brs, 2H), 3.57 (m, 2H), 2.53 (m, 2H), 1.92 (m, 4H)

### Step B: Preparation of 1-(4-((5-chloro-2-nitrophenyl)amino)phenyl)piperidin-2-one

The title compound (0.12 g, yield: 32%) was obtained in the same manner as in step A of Preparation Example 8 by using 1-(4-aminophenyl)piperidin-2-one (0.21 g, 1.1 mmol) obtained in step A above and 4-chloro-2-fluoro-1-nitrobenzene (0.19 g, 1.1 mmol).
¹H-NMR (400 MHz, CDCl3) δ9.52 (brs, 1H), 8.16 (d, 1H), 7.35 (d, 2H), 7.30 (d, 2H), 7.22 (m, 1H), 6.72 (dd, 1H), 3.69 (m, 2H), 2.59 (m, 2H), 1.98 (m, 4H)

### Preparation Example 17: Preparation of 6-cyclopropyl-1-(1-(tetrahydro-2H-pyran-4-yl)-1H-pyrazol-4-yl)-1,3-dihydro-2H-benzo[d]imidazol-2-one

The process of the following steps A, B, and C gave the title compound.

### Step A: Preparation of 4-cyclopropyl-2-fluoro-1-nitrobenzene

To 4-bromo-2-fluoro-1-nitrobenzene (1.00 g, 4.55 mmol) and cyclopropylboronic acid (0.586 g, 6.82 mmol) was added 12 mL of 1,4-dioxane and 3 mL of water, and the resulting mixture was charged with nitrogen gas. To the mixture were added Na₂CO₃ (1.44 g, 13.6 mmol) and PdCl₂(dppf)-CH₂Cl₂ (0.371 g, 0.455 mmol), and the resulting mixture was stirred at 80 °C for 16 hours. After the reaction was terminated, the resulting solid was filtered through Celite and the filtrate was purified by MPLC to give the title compound (0.52 g, 2.87 mmol, yield: 63%) .
¹H-NMR (400 MHz, CDCl3) δ7.97 (t, 1H), 6.94 (dd, 1H), 6.90 (dd, 1H), 1.96 (m, 1H), 1.18 (m, 2H), 0.83 (m, 2H)

### Step B: Preparation of N-(5-cyclopropyl-2-nitrophenyl)-1-(tetrahydro-2H-pyran-4-yl)-1H-pyrazol-4-amine

The title compound (0.147 g, yield: 31%) was obtained in the same manner as in step A of Preparation Example 8 by using 4-cyclopropyl-2-fluoro-1-nitrobenzene (0.26 g, 1.43 mmol) obtained in step A above and 1-(tetrahydro-2*H*-pyran-4-yl)-1*H-*pyrazol-4-amine (0.24 g, 1.43 mmol) obtained in step A of Preparation Example 9.
¹H-NMR (400 MHz, DMSO-d6) δ9.21 (m, 1H), 8.00 (2H), 7.57 (m, 1H), 6.78 (m, 1H), 6.40 (dd, 1H), 4.41 (m, 1H), 3.97 (m, 2H), 3.50 (m, 2H), 1.90 (5H), 1.00 (m, 2H), 0.71 (m, 2H)

### Step C: Preparation of 6-cyclopropyl-1-(1-(tetrahydro-2H-pyran-4-yl)-1H-pyrazol-4-yl)-1,3-dihydro-2H-benzo[d]imidazol-2-one

The title compound (0.078 g, yield: 56%) was obtained in the same manner as in step B of Preparation Example 7 and step C of Preparation Example 3 by using *N*-(5-cyclopropyl-2-nitrophenyl)-1-(tetrahydro-2*H*-pyran-4-yl)-1*H*-pyrazol-4-amine (0.14 g, 0.43 mmol) obtained in step B above.
¹H-NMR (400 MHz, CDCl3) δ7.20 (brs, 1H), 7.86 (s, 1H), 7.80 (s, 1H), 7.00 (d, 1H), 6/86 (dd, 1H), 6.82 (d, 1H), 4.40 (m, 1H), 4.14 (m, 2H), 3.59 (m, 2H), 2.18 (m, 4H), 1.93 (m, 1H), 0.95 (m, 2H), 0.64 (m, 2H)

### Preparation Example 18: Preparation of 6-chloro-1-(1-isopropyl-1H-pyrazol-4-yl)-1,3-dihydro-2H-benzo[d]imidazol-2-one

The process of the following steps A and B gave the title compound.

### Step A: Preparation of N-(5-chloro-2-nitrophenyl)-1-isopropyl-1H-pyrazol-4-amine

*N*-(5-chloro-2-nitrophenyl)-1*H*-pyrazol-4-amine (250.0 mg, 1.05 mmol) obtained in Preparation Example 10 was dissolved in DMF, 2-iodopropane (214.0 mg, 1.26 mmol) and K₂CO₃ (290.0 mg, 2.10 mmol) were added thereto, and the resulting mixture was stirred at 70 °C. After the reaction was terminated, the resulting solid was filtered, and the filtrate was distilled under reduced pressure and then purified by MPLC to give the title compound (195.0 mg, yield: 67%).
¹H NMR (500 MHz, CDCl3) δ 7.47 (s, 1H), 7.43 (s, 1H), 6.48 (d, 1H), 6.39 (d, 1H), 6.17 (s, 1H), 4.49 (m, 1H), 3.85 (br s, 1H), 1.54 (d, 6H)

### Step B: Preparation of 6-chloro-1-(1-isopropyl-1H-pyrazol-4-yl)-1,3-dihydro-2H-benzo[d]imidazol-2-one

The title compound (15.6 mg, yield: 8%) was obtained in the same manner as in step B of Preparation Example 7 and step C of Preparation Example 1 by using *N*-(5-chloro-2-nitrophenyl)-1-isopropyl-1*H*-pyrazol-4-amine (195.0 mg, 0.695 mmol) obtained in step A above.
¹H NMR (500 MHz, CDCl₃) δ10.52 (m, 1H) 7.80 (s, 1H), 7.77 (s, 1H), 7.08 (m, 3H), 4.60 (m, 1H), 1.60 (d, 6H)

### Preparation Example 19: Preparation of 6-chloro-1-(1-(cyclopropylmethyl)-1H-pyrazol-4-yl)-1,3-dihydro-2H-benzo[d]imidazol-2-one

The process of the following steps A, B, and C gave the title compound.

### Step A: Preparation of N-(5-chloro-2-nitrophenyl)-1-(cyclopropylmethyl)-1H-pyrazol-4-amine

*N*-(5-chloro-2-nitrophenyl)-1*H*-pyrazol-4-amine (250.0 mg, 1.05 mmol) obtained in Preparation Example 10 was dissolved in DMF, (bromomethyl)cyclopropane (170.0 mg, 1.26 mmol) and K₂CO₃ (290.0 mg, 2.10 mmol) were added thereto, and the resulting mixture was stirred at 70 °C. After the reaction was terminated, the resulting solid was filtered, and the filtrate was distilled under reduced pressure and then purified by MPLC to give the title compound (205.0 mg, yield: 67%).

### Step B: Preparation of 6-chloro-1-(1-(cyclopropylmethyl)-1H-pyrazol-4-yl)-1,3-dihydro-2H-benzo[d]imidazol-2-one

The title compound (55.3 mg, yield: 13%) was obtained in the same manner as in step B of Preparation Example 7 and step C of Preparation Example 1 by using *N*-(5-chloro-2-nitrophenyl)-1-(cyclopropylmethyl)-1*H*-pyrazol-4-amine (205.0 mg, 0.70 mmol) obtained in step A above.
¹H NMR (500 MHz, CDCl₃) δ9.02 (m, 1H), 7.90 (s, 1H), 7.77 (s, 1H), 7.09 (d, 2H), 7.03 (m, 1H), 4.07 (d, 2H), 1.36 (m, 1H), 0.72 (m, 2H), 0.45 (m, 2H)

### Preparation Example 20: Preparation of 6-chloro-1-(1-(cyclobutylmethyl)-1H-pyrazol-4-yl)-1,3-dihydro-2H-benzo[d]imidazol-2-one

The process of the following steps A and B gave the title compound.

### Step A: Preparation of N-(5-chloro-2-nitrophenyl)-1-(cyclobutylmethyl)-1H-pyrazol-4-amine

*N*-(5-chloro-2-nitrophenyl)-1*H*-pyrazol-4-amine (250.0 mg, 1.05 mmol) obtained in Preparation Example 10 was dissolved in DMF, (bromomethyl)cyclobutane (187.0 mg, 1.26 mmol) and K₂CO₃ (290.0 mg, 2.10 mmol) were added thereto, and the resulting mixture was stirred at 70 °C. After the reaction was terminated, the resulting solid was filtered, and the filtrate was distilled under reduced pressure and then purified by MPLC to give the title compound (232.0 mg, yield: 72%).
¹H NMR (500 MHz, CDCl3) δ 7.46 (s, 1H), 7.38 (s, 1H), 6.50 (d, 1H), 6.42 (d, 1H), 6.17 (s, 1H), 4.14 (d, 2H), 2.86 (m, 1H), 1.96-1.78 (m, 6H)

### Step B: Preparation of 6-chloro-1-(1-(cyclobutylmethyl)-1H-pyrazol-4-yl)-1,3-dihydro-2H-benzo[d]imidazol-2-one

The title compound (10.0 mg, yield: 5%) was obtained in the same manner as in step B of Preparation Example 7 and step C of Preparation Example 1 by using *N*-(5-chloro-2-nitrophenyl)-1-(cyclobutylmethyl)-1*H*-pyrazol-4-amine (232 mg, 0.76 mmol) obtained in step A above.
¹H NMR (500 MHz, CDCl₃) δ10.49 (br s, 1H), 7.75 (s, 2H), 7.08 (m, 3H), 4.22 (d, 2H), 2.93 (m, 1H), 2.17 (m, 2H), 2.00 (m, 4H)

### Preparation Example 21: Preparation of 4-((5-chloro-2-nitrophenyl)amino)benzoic acid

The process of the following steps A and B gave the title compound.

### Step A: Preparation of 4-((5-chloro-2-nitrophenyl)amino)benzoic acid tert-butyl ester

4-Aminobenzoic acid *tert*-butyl ester (1.2 g, 6.27 mmol) was dissolved in DMF and cooled to 0 °C. NaH (273.0 mg, 6.84 mmol) was added slowly thereto and then stirred for 30 minutes. Thereafter, 4-chloro-2-fluoro-1-nitrobenzene (1.0 g, 5.70 mmol) was added at 0 °C, and the mixture was stirred while raising the temperature to room temperature. After the reaction was terminated, water was slowly added thereto while stirring at 0 °C, and the organic layer was extracted with EtOAc and a saturated aqueous NH₄Cl solution and dried over Na₂SO₄. The resulting solid was filtered, and the filtrate was distilled under reduced pressure and then purified by MPLC to give the title compound (670.0 mg, yield: 34%).
¹H NMR (500 MHz, CDCl₃) δ 9.57 (s, 1H), 8.18 (m, 1H) , 8.06 (d, 2H), 7.31 (m, 3H), 6.82 (d, 1H), 1.61 (s, 9H)

### Step B: Preparation of 4-((5-chloro-2-nitrophenyl)amino)benzoic acid

4-((5-Chloro-2-nitrophenyl)amino)benzoic acid *tert-*butyl ester (670.0 mg, 1.92 mmol) obtained in step A above was dissolved in 20 mL of DCM, and then 8 mL of TFA was slowly added thereto, followed by stirring. After the reaction was terminated, the solvent and the reactant were removed by distillation under reduced pressure to give the title compound (559.0 mg, yield: 99%).

### Preparation Example 22: Preparation of 6-chloro-1-(4-(piperidine-1-carbonyl)phenyl)-1,3-dihydro-2H-benzo[d]imidazol-2-one

The process of the following steps A, B, and C gave the title compound.

### Step A: Preparation of (4-((5-chloro-2-nitrophenyl)amino)phenyl)(piperidin-1-yl)methanone

4-((5-Chloro-2-nitrophenyl)amino)benzoic acid (146.3 mg, 0.50 mmol) obtained in Preparation Example 21 and piperidine (54 µl, 0.55 mmol) were dissolved in DMF and then cooled to 0 °C. HATU (228.0 mg, 0.60 mmol) and DIPEA (262.0 µ L, 1.50 mmol) were added thereto, and then the resulting mixture was stirred while raising the temperature to room temperature. After the reaction was terminated, ice was added thereto and the mixture was stirred, and the resulting solid was filtered and washed with cold water. The obtained solid was dried under vacuum to give the title compound (170.6 mg, yield: 94%).
¹H NMR (500 MHz, CDCl₃) δ 9.55 (s, 1H), 8.18 (t, 1H) , 7.49 (s, 2H), 7.31 (m, 3H), 6.78 (m, 1H), 3.72-3.42 (m, 4H), 1.72-1.55 (m, 6H)

### Step B: Preparation of (4-((2-amino-5-chlorophenyl)amino)phenyl)(piperidin-1-yl)methanone

The title compound (154.1 mg, yield: 99%) was obtained in the same manner as in step B of Preparation Example 7 by using (4-((5-chloro-2-nitrophenyl)amino)phenyl)(piperidin-1-yl)methanone (170.6 mg, 0.47 mmol) in step A above.

### Step C: Preparation of 6-chloro-1-(4-(piperidine-1-carbonyl)phenyl)-1,3-dihydro-2H-benzo[d]imidazol-2-one

(4-((2-amino-5-chlorophenyl)amino)phenyl)(piperidin-1-yl)methanone (154.1 mg, 0.46 mmol) obtained in step B above was dissolved in THF, then CDI (162.0 mg, 1.00 mmol) was added thereto, and the resulting mixture was stirred at 70 °C. After the reaction was terminated, the reaction mixture was cooled to room temperature, and then an organic layer was separated by the addition of water and EtOAc, and dried over Na₂SO₄. The resulting solid was filtered and the filtrate was distilled under reduced pressure to give a mixture of the title compound.

### Preparation Example 23: Preparation of 6-chloro-1-(4-(morpholine-4-carbonyl)phenyl)-1,3-dihydro-2H-benzo[d]imidazol-2-one

The process of the following steps A and B gave the title compound.

### Step A: Preparation of (4-((5-chloro-2-nitrophenyl)amino)phenyl)(morpholino)methanone

The title compound (169.4 mg, yield: 93%) was obtained in the same manner as in step A of Preparation Example 22 by using 4-((5-chloro-2-nitrophenyl)amino) benzoic acid (146.3 mg, 0.50 mmol) in Preparation Example 21 and morpholine (47.9 mg, 0.55 mmol).
¹H NMR (500 MHz, CDCl₃) δ 9.54 (s, 1H), 8.18 (d, 1H), 7.52 (d, 2H), 7.33 (m, 3H), 6.80 (d, 1H), 3.80-3.50 (m, 8H)

### Step B: Preparation of 6-chloro-1-(4-(morpholine-4-carbonyl)phenyl)-1,3-dihydro-2H-benzo[d]imidazol-2-one

A mixture of the title compound was obtained in the same manner as in steps B and C of Preparation Example 22 by using (4-((5-chloro-2-nitrophenyl)amino)phenyl)(morpholino)methanone (169.4 mg, 0.47 mmol) obtained in step A above.

### Preparation Example 24: Preparation of 6-chloro-1-(4-(pyrrolidine-1-carbonyl)phenyl)-1,3-dihydro-2H-benzo[d]imidazol-2-one

The process of the following steps A and B gave the title compound.

### Step A: Preparation of (4-((5-chloro-2-nitrophenyl)amino)phenyl)(pyrrolidin-1-yl)methanone

The title compound (171.5 mg, yield: 99%) was obtained in the same manner as in step A of Preparation Example 22 by using 4-((5-chloro-2-nitrophenyl)amino) benzoic acid (146.3 mg, 0.50 mmol) obtained in Preparation Example 21 and pyrrolidine (39.1 mg, 0.55 mmol).
¹H NMR (400 MHz, CDCl₃) δ 9.55 (s, 1H), 8.18 (d, 1H), 7.63 (d, 2H), 7.31 (d, 2H), 7.24 (s, 1H), 6.79 (d, 1H), 3.68 (m, 2H), 3.51 (m, 2H), 1.99 (m, 4H)

### Step B: Preparation of 6-chloro-1- (4-(pyrrolidine-1-carbonyl)phenyl)-1,3-dihydro-2H-benzo[d]imidazol-2-one

A mixture of the title compound was obtained in the same manner as in steps B and C of Preparation Example 22 by using (4-((5-chloro-2-nitrophenyl)amino)phenyl) (pyrrolidin-1-yl)methanone (171.5 mg, 0.50 mmol) obtained in step A above.

### Preparation Example 25: Preparation of 4-(6-chloro-3-(3-(methoxycarbonyl)benzyl)-2-oxo-2,3-dihydro-1H-benzo[d]imidazol-1-yl)benzoic acid

The process of the following steps A and B gave the title compound.

### Step A: Preparation of 3-((3-(4-(tert-butoxycarbonyl)phenyl)-5-chloro-2-oxo-2,3-dihydro-1H-benzo[d]imidazol-1-yl)methyl)benzoic acid methyl ester

3-((5-chloro-2-oxo-2,3-dihydro-1*H*-benzo[*d*]imidazol-1-yl)methyl)benzoic acid methyl ester (1.0 g, 3.16 mmol) obtained in Preparation Example 1, (4-(*tert-*butoxycarbonyl)phenyl)boronic acid (1.4 g, 6.31 mmol), and copper(II) acetate (1.7 g, 9.47 mmol) were dissolved in DCM (30 mL). Thereafter, TEA (1.3 mL, 9.47 mmol) was added thereto, and the resulting mixture was stirred at room temperature. After the reaction was terminated, a saturated K₂CO₃ solution (1 mL) was added thereto, and the resultant mixture was further stirred for 20 minutes. Then, the reaction solution was filtered using Celite^{®}, and the filtrate was extracted with water and EtOAc to separate an organic layer and the organic layer was dried over Na₂SO₄. The resulting solid was filtered, and the filtrate was distilled under reduced pressure and then purified by MPLC to give the title compound (745.0 mg, yield: 48%) .
¹H NMR (500 MHz, CDCl₃) δ 8.18 (d, 2H), 8.05 (s, 1H), 7.99 (d, 1H), 7.65 (d, 2H), 7.57 (d, 1H), 7.45 (t, 1H), 7.13 (s, 1H), 7.05 (d, 1H), 6.83 (d, 1H), 5.16 (s, 2H), 3.91 (s, 3H), 1.62 (s, 9H)

### Step B: Preparation of 4-(6-chloro-3-(3-(methoxycarbonyl)benzyl)-2-oxo-2,3-dihydro-1H-benzo[d]imidazol-1-yl)benzoic acid

3-((3-(4-(*tert*-butoxycarbonyl)phenyl)-5-chloro-2-oxo-2,3-dihydro-1*H*-benzo[*d*]imidazol-1-yl)methyl)benzoic acid methyl ester (745.0 mg, 1.5 mmol) obtained in step A above was dissolved in 20 mL of DCM, and then 8 mL of TFA was slowly added thereto, followed by stirring. After the reaction was terminated, the solvent and the reactant were removed by distillation under reduced pressure to give the title compound (619.4 mg, yield: 95%).
¹H NMR (500 MHz, DMSO-d6) δ8.14 (d, 2H), 8.01 (s, 1H), 7.89 (d, 1H), 7.64 (d, 2H), 7.66 (d, 1H), 7.51 (t, 1H), 7.29 (d, 1H), 7.19 (m, 2H), 5.23 (s, 2H), 3.84 (s, 3H)

### Preparation Example 26: Preparation of 6-chloro-1-(2-morpholinopyrimidin-5-yl)-1,3-dihydro-2H-benzo[d]imidazol-2-one

The process of the following steps A, B, C, and D gave the title compound.

### Step A: Preparation of 4-(5-nitropyrimidin-2-yl)morpholine

To 2-chloro-5-nitropyrimidine (3.00 g, 18.8 mmol) was added 38 mL of AN and morpholine (3.60 mL, 41.4 mmol), and then the resulting mixture was stirred at room temperature for 1 hour. After the resulting solid was removed, the filtrate was concentrated. To the filtrate were added EtOAc and an aqueous hydrochloric acid solution to separate an organic layer, and the organic layer was dried over anhydrous Na₂SO₄ to give the title compound (2.65 g, yield: 67%).
¹H-NMR (400 MHz, CDCl3) δ9.07 (s, 2H), 4.00 (m, 4H), 3.78 (m, 4H)

### Step B: Preparation of 2-morpholinopyrimidin-5-amine

The title compound (2.27 g, yield: 99%) was obtained in the same manner as in step A of Preparation Example 9 by using 4-(5-nitropyrimidin-2-yl)morpholine (2.65 g, 12.6 mmol) obtained in step A above.
¹H-NMR (400 MHz, CDCl3) δ7.99 (s, 2H), 3.77 (m, 4H), 3.64 (m, 4H)

### Step C: Preparation of N-(5-chloro-2-nitrophenyl)-2-morpholinopyrimidin-5-amine

To 2-morpholinopyrimidin-5-amine (0.50 g, 2.77 mmol) obtained in step B above were added 4-chloro-2-fluoro-1-nitrobenzene (0.97 g, 5.55 mmol) and 14 mL of DMF, and then the resulting mixture was cooled to 0 °C and KOtBu (0.31 g, 2.77 mmol) was added thereto. The reaction solution was stirred under reflux for 3 days, and then an aqueous ammonium chloride solution was added thereto. The resulting solid was filtered and separated by MPLC to give the title compound (0.21 g, yield: 22%).
¹H-NMR (400 MHz, DMSO-d6) δ9.37 (brs, 1H), 8.348 (s, 2H), 8.14 (d, 1H), 6.86 (dd, 1H), 6.80 (d, 1H), 3.73 (m, 4H), 3.68 (m, 4H)

### Step D: Preparation of 6-chloro-1-(2-morpholinopyrimidin-5-yl)-1,3-dihydro-2H-benzo[d]imidazol-2-one

The title compound (0.045 g, yield: 22%) was obtained in the same manner as in step B of Preparation Example 8 by using *N*-(5-chloro-2-nitrophenyl)-2-morpholinopyrimidin-5-amine (0.21 g, 0.625 mmol) obtained in step C above.
¹H-NMR (400 MHz, MeOH-d4) δ8.46 (s, 2H), 7.11 (dd, 1H), 7.08 (dd, 1H), 6.95 (d, 1H), 3.86 (m, 4H), 3.75 (m, 4H)

### Preparation Example 27: Preparation of 5-chloro-1-(4-(dimethylamino)phenyl)-7-fluoro-1,3-dihydro-2H-benzo[d]imidazol-2-one

### Step A: Preparation of N¹-(4-chloro-2-fluoro-6-nitrophenyl)-N⁴,N⁴-dimethylbenzene-1,4-diamine

The title compound (1.08 g, yield: 95%) was obtained in the same manner as in step A of Preparation Example 8 by using 5-chloro-1,2-difluoro-3-nitrobenzene (0.710 g, 3.67 mmol) and *N*¹,*N*¹-dimethylbenzene-1,4-diamine (0.50 g, 3.67 mmol).
¹H-NMR (400 MHz, CDCl3) δ8.94 (brs, 1H), 8.01 (d, 1H), 7.21 (dd, 1H), 6.99 (d, 2H), 6.67 (d, 2H), 2.93 (s, 6H)

### Step B: Preparation of 5-chloro-1-(4-(dimethylamino)phenyl)-7-fluoro-1,3-dihydro-2H-benzo[d]imidazol-2-one

The title compound (0.50 g, yield: 47%) was obtained in the same manner as in step B of Preparation Example 8 by using *N*¹-(4-chloro-2-fluoro-6-nitrophenyl)-*N*⁴,*N*⁴-dimethylbenzene-1,4-diamine (1.08 g, 3.49 mmol) obtained in step A above.
¹H-NMR (400 MHz, CDCl3) δ11.48 (brs, 1H), 8.24 (m, 1H), 7.28 (m, 1H), 7.00 (d, 2H), 6.78 (d, 2H), 3.03 (s, 6H)

### Preparation Example 28: Preparation of 6-chloro-1-(4-(4,4-difluoropiperidin-1-yl)phenyl)-1,3-dihydro-2H-benzo[d]imidazol-2-one

The process of the following steps A, B, and C gave the title compound.

### Step A: Preparation of 4-(4,4-difluoropiperidin-1-yl)aniline

The title compound (6.25 g, yield: 83%) was obtained in the same manner as in step A of Preparation Example 1 and step B of Preparation Example 7 by using 1-fluoro-4-nitrobenzene (5.00 g, 35.4 mmol) and 4,4-difluoropiperidine hydrochloride (5.58 g, 35.4 mmol).
¹H-NMR (500 MHz, DMSO-d6) δ6.73 (d, 2H), 6.50 (d, 2H), 4.62 (brs, 2H), 3.02 (m, 4H), 2.05 (m, 4H)

### Step B: Preparation of 5-chloro-N-(4-(4,4-difluoropiperidin-1-yl)phenyl)-2-nitroaniline

The title compound (1.52 g, yield: 88%) was obtained in the same manner as in step A of Preparation Example 1 by using 4-(4,4-difluoropiperidin-1-yl)aniline (1.00 g, 4.71 mmol) obtained in step A above and 4-chloro-2-fluoro-1-nitrobenzene (0.827 g, 4.71 mmol).
¹H-NMR (400 MHz, DMSO-d6) δ 9.46 (d, 1H), 8.13 (d, 1H), 7.21 (d, 2H), 7.10 (d, 2H), 6.85 (dd, 1H), 6.80 (dd, 1H), 3.47 (m, 4H), 2.06 (m, 4H)

### Step C: Preparation of 6-chloro-1-(4-(4,4-difluoropiperidin-1-yl)phenyl)-1,3-dihydro-2H-benzo[d]imidazol-2-one

The title compound (1.04 g, yield: 69%) was obtained in the same manner as in step B of Preparation Example 7 and step B of Preparation Example 8 by using 5-chloro-*N*-(4-(4,4-difluoropiperidin-1-yl)phenyl)-2-nitroaniline (1.52 g, 4.13 mmol) obtained in step B above.
¹H-NMR (400 MHz, DMSO-d6) δ 11.22 (brs, 1H), 7.34 (d, 2H), 7.15 (d, 2H), 7.05 (2H), 6.84 (d, 1H), 3.43 (m, 4H), 2.08 (m, 4H)

### Preparation Example 29: Preparation of 3-((5-chloro-2-oxo-3-(4-(piperazin-1-yl)phenyl)-2,3-dihydro-1H-benzo[d]imidazol-1-yl)methyl)benzoic acid methyl ester hydrochloride

The process of the following steps A, B, C, D and E gave the title compound.

### Step A: Preparation of 4-(4-aminophenyl)piperazine-1-carboxylic acid tert-butyl ester

The title compound (1.60 g, yield: 51%) was obtained in the same manner as in step A of Preparation Example 1 and step B of Preparation Example 7 by using 1-fluoro-4-nitrobenzene (1.59 g, 11.3 mmol) and piperazine-1-carboxylic acid *tert*-butyl ester (2.10 g, 11.3 mmol).
¹H-NMR (400 MHz, DMSO-d6) δ6.70 (d, 2H), 6.49 (d, 2H), 4.61 (brs, 2H), 3.41 (m, 4H), 2.82 (m, 4H), 1.41 (s, 9H)

### Step B: Preparation of 4-(4-((5-chloro-2-nitrophenyl)amino)phenyl)piperazine-1-carboxylic acid tert-butyl ester

The title compound (2.35 g, yield: 94%) was obtained in the same manner as in step A of Preparation Example 1 by using 4-(4-aminophenyl)piperazine-1-carboxylic acid *tert-*butyl ester (1.60 g, 5.77 mmol) obtained in step A above and 4-chloro-2-fluoro-1-nitrobenzene (1.01 g, 5.77 mmol).
¹H-NMR (400 MHz, DMSO-d6) δ 9.46 (brs, 1H), 8.13 (d, 1H), 7.20 (d, 2H), 7.04 (d, 2H), 6.82 (dd, 1H), 6.80 (m, 1H), 3.48 (m, 4H), 3.14 (m, 4H), 1.42 (s, 9H)

### Step C: Preparation of 4-(4-(6-chloro-2-oxo-2,3-dihydro-1H-benzo[d]imidazol-1-yl)phenyl)piperazine-1-carboxylic acid tert-butyl ester

The title compound (1.0 g, yield: 43%) was obtained in the same manner as in step B of Preparation Example 7 and step B of Preparation Example 8 by using 4-(4-((5-chloro-2-nitrophenyl)amino)phenyl)piperazine-1-carboxylic acid *tert-*butyl ester (2.35 g, 5.34 mmol) obtained in step B above.
¹H-NMR (400 MHz, DMSO-d6) δ 11.22 (brs, 1H), 7.34 (d, 2H), 7.10 (d, 2H), 7.06 (2H), 6.83 (d, 1H), 3.48 (m, 4H), 3.19 (m, 4H), 1.43 (s, 9H)

### Step D: Preparation of 4-(4-(6-chloro-3-(3-(methoxycarbonyl)benzyl)-2-oxo-2,3-dihydro-1H-benzo[d]imidazol-1-yl)phenyl)piperazine-1-carboxylic acid tert-butyl ester

The title compound (1.05 mmol, yield: 78%) was obtained in the same manner as in step C of Preparation Example 7 by using 4-(4-(6-chloro-2-oxo-2,3-dihydro-1*H*-benzo[*d*]imidazol-1-yl)phenyl)piperazine-1-carboxylic acid *tert*-butyl ester (1.0 g, 2.33 g) obtained in step C above and 3-(bromomethyl)benzoic acid methyl ester (0.53 g, 2.33 mmol).
¹H-NMR (400 MHz, CDCl3) δ 8.05 (d, 1H), 7.97 (dd, 1H), 7.55 (dd, 1H), 7.43 (d, 1H), 7.40 (d, 2H), 7.05 (d, 2H), 6.99 (m, 2H), 6.79 (d, 1H), 5.15 (s, 2H), 3.92 (s, 3H), 3.62 (m, 4H), 3.22 (m, 4H), 1.50 (s, 9H)

### Step E: Preparation of 3-((5-chloro-2-oxo-3-(4-(piperazin-1-yl)phenyl)-2,3-dihydro-1H-benzo[d]imidazol-1-yl)methyl)benzoic acid methyl ester hydrochloride

To 4-(4-(6-chloro-3-(3-(methoxycarbonyl)benzyl)-2-oxo-2,3-dihydro-1*H*-benzo[*d*]imidazol-1-yl)phenyl)piperazine-1-carboxylic acid *tert*-butyl ester (1.05 g, 1.82 mmol) obtained in step D above were added 6 mL of DCM and 1.8 mL of hydrochloric acid (4 M EtOAc solution), and then the resulting mixture was stirred at room temperature for 1 hour. The reaction solution was concentrated under reduced pressure, then ethyl ether was added thereto, and the resulting solid was filtered to give the title compound (0.94 g, yield: 99%).
¹H-NMR (400 MHz, DMSO-d6) δ 8.91 (brs, 2H), 8.00 (d, 1H), 7.88 (dd, 1H), 7.67 (dd, 1H), 7.52 (t, 1H), 7.45 (d, 2H), 7.24 (d, 1H), 7.18 (3H), 6.91 (d, 1H), 5.21 (s, 2H), 3.85 (s, 3H), 3.45 (m, 4H), 3.26 (m, 4H)

### Preparation Example 30: Preparation of 3-((5-chloro-2-oxo-3-(4-(piperidin-4-yl)phenyl)-2,3-dihydro-1H-benzo[d]imidazol-1-yl)methyl)benzoic acid methyl ester hydrochloride

The process of the following steps A, B, C and D gave the title compound.

### Step A: Preparation of 4-(4-((5-chloro-2-nitrophenyl)amino)phenyl)piperidine-1-carboxylic acid tert-butyl ester

The title compound (1.65 g, yield: 67%) was obtained in the same manner as in step A of Preparation Example 1 by using 4-(4-aminophenyl)piperidine-1-carboxylic acid *tert-*butyl ester (1.57 g, 5.70 mmol) and 4-chloro-2-fluoro-1-nitrobenzene (1.00 g, 5.70 mmol).
¹H-NMR (400 MHz, CDCl3) δ9.15 (brs, 1H), 8.15 (d, 1H), 7.28 (d, 2H), 7.21 (d, 2H), 7.12 (d, 1H), 6.70 (dd, 1H), 4.28 (m, 2H), 2.83 (m, 2H), 2.70 (m, 1H), 1.85 (m, 2H), 1.65 (m, 2H), 1.49 (s, 9H)

### Step B: Preparation of 4-(4-(6-chloro-2-oxo-2,3-dihydro-1H-benzo[d]imidazol-1-yl)phenyl)piperidine-1-carboxylic acid tert-butyl ester

The title compound (0.65 g, yield: 40%) was obtained in the same manner as in step B of Preparation Example 7 and step B of Preparation Example 8 by using 4-(4-((5-chloro-2-nitrophenyl)amino)phenyl)piperidine-1-carboxylic acid *tert-*butyl ester (1.65 g, 3.81 mmol) obtained in step A above.
¹H-NMR (400 MHz, CDCl3) δ8.79 (brs, 1H), 7.45 (d, 2H), 7.38 (2H), 7.07 (d, 1H), 7.02 (2H), 4.28 (m, 2H), 2.83 (m, 2H), 2.73 (m, 1H), 1.88 (m, 2H), 1.65 (m, 2H), 1.50 (s, 9H)

### Step C: Preparation of 4-(4-(6-chloro-3-(3-(methoxycarbonyl)benzyl)-2-oxo-2,3-dihydro-1H-benzo[d]imidazol-1-yl)phenyl)piperidine-1-carboxylic acid tert-butyl ester

The title compound (0.70 g, yield: 80%) was obtained in the same manner as in step C of Preparation Example 7 by using 4-(4-(6-chloro-2-oxo-2,3-dihydro-1*H*-benzo[*d*]imidazol-1-yl)phenyl)piperidine-1-carboxylic acid tert-butyl ester (0.65 g, 1.52 mmol) obtained in step B above and 3-(bromomethyl)benzoic acid methyl ester (0.35 g, 1.52 mmol).
¹H-NMR (400 MHz, CDCl3) δ 8.05 (d, 1H), 7.98 (dd, 1H), 7.57 (dd, 1H), 7.47 (d, 2H), 7.42 (t, 1H), 7.38 (d, 2H), 7.07 (d, 1H), 7.02 (dd, 1H), 6.81 (d, 1H), 5.16 (s, 2H), 4.29 (m, 2H), 3.91 (s, 3H), 2.83 (m, 2H), 2.73 (m, 1H), 1.87 (m, 2H), 1.66 (m, 2H), 1.50 (s, 9H)

### Step D: Preparation of 3-((5-chloro-2-oxo-3-(4-(piperidin-4-yl)phenyl)-2,3-dihydro-1H-benzo[d]imidazol-1-yl)methyl)benzoic acid methyl ester hydrochloride

To 4-(4-(6-chloro-3-(3-(methoxycarbonyl)benzyl)-2-oxo-2,3-dihydro-1*H*-benzo[*d*]imidazol-1-yl)phenyl)piperidine-1-carboxylic acid tert-butyl ester (0.70 g, 1.21 mmol) obtained in step C above were added 3 mL of DCM and 1.21 mL of hydrochloric acid (4 M EtOAc solution), and then the resulting mixture was stirred at room temperature for 1 hour. The reaction solution was concentrated under reduced pressure, then diethyl ether was added thereto, and the resulting solid was dried to give the title compound (0.48 g, yield: 76%).
¹H-NMR (400 MHz, DMSO-d6) δ8.90 (brs, 2H), 8.01 (d, 1H), 7.89 (dd, 1H), 7.66 (dd, 1H), 7.57 (d, 2H), 7.52 (t, 1H), 7.455 (d, 2H), 7.27 (d, 1H), 7.16 (dd, 1H), 7.03 (d, 1H), 5.23 (s, 2H), 3.85 (s, 3H), 3.33 (m, 2H), 3.03 (m, 2H), 2.97 (m, 1H), 1.98 (m, 2H), 1.90 (m, 2H)

### Preparation Example 31: Preparation of 6-chloro-1-(4-(3,6-dihydro-2H-pyran-4-yl)phenyl)-1,3-dihydro-2H-benzo[d]imidazol-2-one

The process of the following steps A, B, and C gave the title compound.

### Step A: Preparation of 4-(4-nitrophenyl)-3,6-dihydro-2H-pyran

To 4-bromo-1-nitrobenzene (200 mg, 1.0 mmol) and 2-(3,6-dihydro-2*H*-pyran-4-yl)-4,4,5,5-tetramethyl-1,3,2-dioxaborolane (230 mg, 1.1 mmol) were added 3 mL of 1,4-dioxane and 3 mL of an aqueous 1 M sodium carbonate solution, and then the resulting mixture was charged with nitrogen, and a catalytic amount of Pd(PPh₃)₄ was added thereto, followed by stirring under reflux for 16 hours. After the reaction was terminated, the resulting solid was removed through Celite and the filtrate was purified by MPLC to give the title compound (185 mg, yield: 91%).
¹H-NMR (500 MHz, CDCl3) δ 8.22 (d, 2H), 7.55 (d, 2H), 6.36 (s, 1H), 4.39 (s, 2H), 3.98 (t, 2H), 2.57 (s, 2H)

### Step B: Preparation of 4-(3,6-dihydro-2H-pyran-4-yl)aniline

The title compound (138 mg, yield: 90%) was obtained in the same manner as in step B of Preparation Example 7 by using 4-(4-nitrophenyl)-3,6-dihydro-2*H*-pyran (180 mg, 0.88 mmol) obtained in step A above.

### Step C: Preparation of 6-chloro-1-(4-(3,6-dihydro-2H-pyran-4-yl)phenyl)-1,3-dihydro-2H-benzo[d]imidazol-2-one

The title compound (65 mg, yield: 26%) was obtained in the same manner as in step A of Preparation Example 1, step B of Preparation Example 7, and step B of Preparation Example 8 by using 4-(3,6-dihydro-2*H*-pyran-4-yl)aniline (138 mg, 0.78 mmol) obtained in step B above and 4-chloro-2-fluoro-1-nitrobenzene (138 mg, 0.78 mmol).
¹H-NMR (500 MHz, CDCl3) δ 8.18 (d, 1H), 7.50 (d, 2H), 7.26 (d, 2H), 7.19 (s, 1H), 6.75 (d, 1H), 6.20 (s, 1H), 4.38 (m, 2H), 3.98 (m, 2H), 2.57 (m, 2H)

### Preparation Example 32: Preparation of 6-chloro-1-(4-(3,3-difluoropyrrolidin-1-yl)phenyl)-7-fluoro-1,3-dihydro-2H-benzo[d]imidazol-2-one

The process of the following steps A, B, C and D gave the title compound.

### Step A: Preparation of 3,3-difluoro-1-(4-nitrophenyl)pyrrolidine

The title compound (4.28 g, yield: 88%) was obtained in the same manner as in step A of Preparation Example 1 by using 1-fluoro-4-nitrobenzene (3.0 g, 21.3 mmol) and 3,3-difluoropiperidine hydrochloride (3.05 g, 21.26 mmol).
¹H-NMR (400 MHz, DMSO-d6) δ 8.10 (d, 2H), 6.70 (d, 2H), 3.89 (t, 2H), 3.65 (t, 2H), 2.57 (m, 2H)

### Step B: Preparation of 4-(3,3-difluoropyrrolidin-1-yl)aniline

The title compound (2.17 g, yield: 58%) was obtained in the same manner as in step B of Preparation Example 7 by using 3,3-difluoro-1-(4-nitrophenyl)pyrrolidine (4.28 g, 18.8 mmol) obtained in step A above.
¹H-NMR (400 MHz, DMSO-d6) δ6.51 (d, 2H), 6.44 (d, 2H), 4.43 (brs, 2H), 3.50 (t, 2H), 3.30 (t, 2H), 2.45 (m, 2H)

### Step C: Preparation of 3-chloro-N-(4-(3,3-difluoropyrrolidin-1-yl)phenyl)-2-fluoro-6-nitroaniline

The title compound (0.87 g, yield: 48%) was obtained in the same manner as in step A of Preparation Example 1 by using 4-(3,3-difluoropyrrolidin-1-yl)aniline (1.0 g, 5.04 mmol) obtained in step B above and 1-chloro-2,3-difluoro-4-nitrobenzene (0.98 g, 5.04 mmol).
¹H-NMR (400 MHz, CDCl3) δ9.10 (brs, 1H), 7.97 (dd, 1H), 7.03 (d, 2H), 6.83 (dd, 1H), 6.52 (d, 2H), 3.68 (t, 2H), 3.53 (t, 2H), 2.50 (m, 2H)

### Step D: Preparation of 6-chloro-1-(4-(3,3-difluoropyrrolidin-1-yl)phenyl)-7-fluoro-1,3-dihydro-2H-benzo[d]imidazol-2-one

The title compound (0.48 g, yield: 56%) was obtained in the same manner as in step B of Preparation Example 7 and step B of Preparation Example 8 by using 3-chloro-*N*-(4-(3,3-difluoropyrrolidin-1-yl)phenyl)-2-fluoro-6-nitroaniline (0.87 g, 2.34 mmol) obtained in step C above.
¹H-NMR (400 MHz, CDCl3) δ8.21 (brs, 1H), 7.32 (d, 2H), 7.09 (dd, 1H), 6.81 (dd, 1H), 6.63 (d, 2H), 3.73 (t, 2H), 3.58 (t, 2H), 2.52 (m, 2H)

### Preparation Example 33: Preparation of 6-bromo-2,2-dimethyl-2,3-dihydro-1H-inden-1-one

To 6-bromo-2,3-dihydro-1*H*-inden-1-one (1.0 g, 4.74 mmol) were added 31 mL of THF and iodomethane (0.74 mL, 11.8 mmol), and then the resulting mixture was cooled to 0 °C and NaH (0.46 g, 11.3 mmol, 55 wt % in mineral oil) was added thereto. The reaction mixture was stirred at room temperature for 1 hour, then a saturated aqueous ammonium chloride solution was added thereto and the resultant mixture was extracted with EtOAc. An organic layer was separated and purified by MPLC to give the title compound (0.81 g, yield: 71%).
¹H-NMR (400 MHz, CDCl3) δ 7.88 (dd, 1H), 7.59 (dd, 1H), 7.31 (d, 1H), 2.94 (s, 2H), 1.24 (s, 6H)

### Preparation Example 34: Preparation of 5-bromo-2,2-dimethyl-2,3-dihydro-1H-inden-1-one

The title compound (1.06 g, yield: 94%) was obtained in the same manner as in Preparation Example 33 by using 5-bromo-2,3-dihydro-1*H*-inden-1-one (1.0 g, 4.74 mmol).
¹H-NMR (400 MHz, CDCl3) δ7.62 (2H), 7.52 (dd, 1H), 2.98 (s, 2H), 1.23 (s, 6H)

### Preparation Example 35: Preparation of 6-chloro-1-(2-methyl-1-oxoisoindolidin-5-yl)-1,3-dihydro-2H-benzo[d]imidazol-2-one

The process of the following steps A and B gave the title compound.

### Step A: Preparation of 5-((5-chloro-2-nitrophenyl)amino)-2-methylisoindolin-1-one

To 5-amino-2-methylisoindolin-1-one (1.0 g, 6.17 mmol) were added 61 mL of THF and KOtBu (0.69 g, 6.17 mmol), and the resulting mixture was stirred at 70 °C for 30 minutes. To the reaction solution was added 4-chloro-2-fluoro-1-nitrobenzene (1.62 g, 9.25 mmol), followed by stirring at 70 °C for 16 hours. To the reaction solution was added water, and then the reaction solution was extracted with EtOAc, and the organic layer was purified by MPLC to give the title compound (0.307 g, yield: 15%).
¹H-NMR (400 MHz, DMSO-d6) δ 9.60 (brs, 1H), 8.18 (d, 1H), 7.90 (dd, 1H), 7.30 (2H), 7.24 (d, 1H), 6.82 (dd, 1H), 4.42 (s, 2H), 3.22 (s, 3H)

### Step B: Preparation of 6-chloro-1-(2-methyl-1-oxoisoindolidin-5-yl)-1,3-dihydro-2H-benzo[d]imidazol-2-one

The title compound (0.29 g, yield: 96%) was obtained in the same manner as in step B of Preparation Example 7 and step B of Preparation Example 8 by using 5-((5-chloro-2-nitrophenyl)amino)-2-methylisoindolin-1-one (0.307 g, 0.97 mmol) obtained in step A above.
¹H-NMR (400 MHz, DMSO-d6) δ 11.41 (brs, 1H), 7.82 (d, 1H), 7.79 (d, 1H), 7.64 (dd, 1H), 7.10 (3H), 4.55 (s, 2H), 3.11 (s, 3H)

### Preparation Example 36: Preparation of 3-(2-(5-chloro-2-oxo-2,3-dihydro-1H-benzo[d]imidazol-1-yl)ethyl)benzoic acid methyl ester

The process of the following steps A, B, C, and D gave the title compound.

### Step A: Preparation of 3-(cyanomethyl)benzoic acid methyl ester

To 3-(bromomethyl)benzoic acid methyl ester (2.00 g, 8.73 mmol) were added 8 mL of DMSO and KCN (0.60 g, 9.17 mmol), and then the resulting mixture was stirred at room temperature for 16 hours. To the reaction solution was added water and Et₂O, and then an organic layer was separated and purified by MPLC to give the title compound (0.78 g, yield: 51%).
¹H-NMR (400 MHz, CDCl3) δ8.01 (2H), 7.56 (m, 1H), 7.49 (m, 1H), 3.93 (s, 3H), 3.81 (s, 2H)

### Step B: Preparation of 3-(2-aminoethyl)benzoic acid methyl ester

The title compound (0.71 g, yield: 99%) was obtained by referring to WO2016/165014 by using 3-(cyanomethyl)benzoic acid methyl ester (0.70 g, 4.0 mmol) obtained in step A above.
¹H-NMR (400 MHz, DMSO-d6) δ7.83 (2H), 7.81 (brs, 2H), 7.55 (m, 1H), 7.52 (m, 1H), 3.86 (s, 3H), 3.07 (m, 2H), 2.95 (t, 2H)

### Step C: Preparation of 3-(2-((4-chloro-2-nitrophenyl)amino)ethyl)benzoic acid methyl ester

The title compound (0.24 g, yield: 18%) was obtained in the same manner as in step A of Preparation Example 1 by using 3-(2-aminoethyl)benzoic acid methyl ester (0.70 g, 4.0 mmol) obtained in step B above and 4-chloro-1-fluoro-2-nitrobenzene (0.70 g, 7.0 mmol).
¹H-NMR (400 MHz, CDCl3) δ8.16 (d, 1H), 8.04 (m, 1H), 7.93 (2H), 7.43 (2H), 7.30 (dd, 1H), 6.82 (d, 1H), 3.92 (s, 3H), 3.58 (m, 2H), 3.07 (t, 2H)

### Step D: Preparation of 3-(2-(5-chloro-2-oxo-2,3-dihydro-1H-benzo[d]imidazol-1-yl)ethyl)benzoic acid methyl ester

The title compound (0.10 g, yield: 42%) was obtained in the same manner as in step B of Preparation Example 7 and step B of Preparation Example 8 by using 3-(2-((4-chloro-2-nitrophenyl)amino)ethyl)benzoic acid methyl ester (0.24 g, 0.71 mmol) obtained in step C above.
¹H-NMR (400 MHz, CDCl3) δ9.52 (brs, 1H), 7.88 (2H), 7.37 (2H), 7.09 (d, 1H), 7.00 (dd, 1H), 6.72 (dd, 1H), 4.11 (t, 2H), 3.91 (s, 3H), 3.10 (t, 2H)

### Preparation Example 37: Preparation of 2-(4-bromophenyl)-N,N-dimethylacetamide

To 2-(4-bromophenyl)acetic acid (1.0 g, 4.65 mmol) were added 15 mL of DCM, dimethylamine hydrochloride (0.46 g, 5.58 mmol), EDC-HCl (0.98 g, 5.12 mmol), HOBt (0.78 g, 5.12 mmol), and DIPEA (2.03 mL, 11.6 mmol), and then the resulting mixture was stirred at room temperature for 16 hours. To the reaction solution was added water, and then an organic layer was separated and purified by MPLC to give the title compound (1.13 g, yield: 99%).
¹H-NMR (400 MHz, CDCl3) δ7.44 (dd, 2H), 7.13 (dd, 2H), 3.66 (s, 2H), 3.00 (s, 3H), 2.97 (s, 3H)

### Preparation Example 38: Preparation of 1-(4-bromophenyl)azetidin-3-ol

To (4-bromophenyl)boronic acid (0.30 g, 1.49 mmol) were added 15 mL of DCM, Cu(OAc)₂ (0.54 g, 3.0 mol), and TEA (1.25 mL, 9 mmol), and then the resulting mixture was stirred at room temperature for 16 hours. The resulting solid was removed through Celite and then the filtrate was purified by MPLC to give the title compound (0.11 g, yield: 31%).
¹H-NMR (400 MHz, CDCl3) δ7.28 (d, 2H), 6.33 (d, 2H), 4.76 (m, 1H), 4.15 (t, 2H), 3.65 (m, 2H), 2.08 (m, 1H)

### Preparation Example 39: Preparation of 6-(4-bromophenyl)-2-oxa-6-azaspiro[3.3]heptane

The title compound (0.062 g, yield: 24%) was obtained in the same manner as in Preparation Example 38 by using (4-bromophenyl)boronic acid (0.223 g, 1.11 mmol) and 2-oxa-6-azaspiro[3.3]heptane (0.10 g, 1 mmol).
¹H-NMR (400 MHz, CDCl3) δ7.29 (dd, 2H), 6.32 (dd, 2H), 4.83 (s, 4H), 3.99 (s, 4H)

### Preparation Example 40: Preparation of 2-(4-bromophenyl)-7-oxa-2-azaspiro[3.5]nonane

The title compound (0.15 g, yield: 22%) was obtained in the same manner as in Preparation Example 38 by using (4-bromophenyl)boronic acid (0.50 g, 2.49 mmol) and 7-oxa-2-azaspiro[3.5]nonane hydrochloride (0.49 g, 2.74 mmol).
¹H-NMR (500 MHz, CDCl3) δ7.28 (m, 2H), 6.31 (m, 2H), 3.67 (m, 4H), 3.62 (s, 4H), 1.83 (m, 4H)

### Preparation Example 41: Preparation of 3-(2-(5-chloro-2-oxo-2,3-dihydro-1H-benzo[d]imidazol-1-yl)propan-2-yl)benzonitrile

The process of the following steps A, B, C, D, and E gave the title compound.

### Step A: Preparation of (2-(3-bromophenyl)propan-2-yl)carbonic acid tert-butyl ester

To 2-(3-bromophenyl)propan-2-amine hydrochloride (1.0 g, 4.0 mmol) were added 40 mL of THF, BOC₂O (1.0 mL, 4.4 mmol), DIPEA (1.7 mL, 10 mmol), and a catalytic amount of DMAP, and the resulting mixture was stirred at room temperature for 16 hours. The resulting solid was filtered and the filtrate was purified by MPLC to give the title compound (1.2 g, yield: 99%).

### Step B: Preparation of (2-(3-cyanophenyl)propane-2-yl)carbonic acid tert-butyl ester

To (2-(3-bromophenyl)propan-2-yl)carbonic acid *tert-*butyl ester (0.45 g, 1.8 mmol) obtained in step A above were added 6 mL of DMF, Zn(CN)₂ (0.13 g, 0.85 mmol), and Pd(PPh₃)₄ (0.21 g, 0.18 mmol), and the resulting mixture was stirred at 100 °C for 16 hours. To the reaction solution was added EtOAc and the resultant mixture was washed with an aqueous ammonia solution. An organic layer was separated and purified by MPLC to give the title compound (0.20 g, yield: 42%).
¹H-NMR (400 MHz, CDCl3) δ7.68 (d, 1H), 7.64 (dd, 1H), 7.51 (dd, 1H), 7.43 (t, 1H), 5.06 (m, 1H), 1.51 (s, 6H), 1.39-1.10 (9H)

### Step C: Preparation of 3-(2-aminopropan-2-yl)benzonitrile hydrochloride

To (2-(3-cyanophenyl)propan-2-yl)carbonic acid *tert-*butyl ester (0.31 g, 1.2 mmol) obtained in step B above were added 1.2 mL of DCM and 1.2 mL of HCl (4.0 M EtOAc solution), the resulting mixture was stirred at room temperature for 1 hour. The reaction solution was concentrated under reduced pressure to give the title compound (0.20 g, yield: 85%).
¹H-NMR (400 MHz, DMSO-d6) δ8.76 (brs, 3H), 7.05 (d, 1H), 7.92 (dd, 1H), 7.86 (dd, 1H), 7.61 (t, 1H), 1.65 (s, 6H)

### Step D: Preparation of 3-(2-((4-chloro-2-nitrophenyl)amino)propan-2-yl)benzonitrile

The title compound (0.20 g, yield: 62%) was obtained in the same manner as in step A of Preparation Example 1 by using 3-(2-aminopropan-2-yl)benzonitrile hydrochloride (0.20 g, 1 mmol) obtained in step C above and 4-chloro-1-fluoro-2-nitrobenzene (0.18 g, 1 mmol).
¹H-NMR (400 MHz, CDCl3) δ8.61 (brs, 1H), 8.20 (d, 1H), 7.72 (m, 1H), 7.66 (m, 1H), 7.58 (m, 1H), 7.48 (t, 1H), 7.07 (dd, 1H), 6.13 (d, 1H), 1.78 (s, 6H)

### Step E: Preparation of 3-(2-(5-chloro-2-oxo-2,3-dihydro-1H-benzo[d]imidazol-1-yl)propan-2-yl)benzonitrile

The title compound (0.12 g, yield: 64%) was obtained in the same manner as in step B of Preparation Example 7 and step B of Preparation Example 8 by using 3-(2-((4-chloro-2-nitrophenyl)amino)propan-2-yl)benzonitrile (0.20 g, 0.63 mmol) obtained in step D above.
¹H-NMR (400 MHz, CDCl3) δ8.23 (brs, 1H), 7.64 (d, 1H), 7.58 (2H), 7.47 (t, 1H), 7.00 (d, 1H), 6.74 (dd, 1H), 6.10 (dd, 1H), 2.08 (s, 6H)

### Preparation Example 42: Preparation of 3-((5-chloro-2-oxo-3-(4-(piperazin-1-yl)phenyl)-2,3-dihydro-1H-benzo[d]imidazol-1-yl)methyl)-2-fluorobenzoic acid methyl ester hydrochloride

The process of the following steps A and B gave the title compound.

### Step A: Preparation of 4-(4-(6-chloro-3-(2-fluoro-3-(methoxycarbonyl)benzyl)-2-oxo-2,3-dihydro-1H-benzo[d]imidazol-1-yl)phenyl)piperazine-1-carboxylic acid tert-butyl ester

The title compound (0.72 g, yield: 99%) was obtained in the same manner as in step A of Preparation Example 1 by using 4-(4-(6-chloro-2-oxo-2,3-dihydro-1*H*-benzo[*d*]imidazol-1-yl)phenyl)piperazine-1-carboxylic acid tert-butyl ester (0.52 g, 1.21 mmol) obtained in step C of Preparation Example 29 and 3-(bromomethyl)-2-fluorobenzoic acid methyl ester (0.30 g, 1.21 mmol).
¹H-NMR (400 MHz, CDCl3) δ 7.88 (t, 1H), 7.61 (t, 1H), 7.37 (d, 2H), 7.17 (t, 1H), 7.06 (3H), 7.00 (d, 1H), 6.96 (dd, 1H), 5.19 (s, 2H), 3.94 (s, 3H), 3.61 (m, 4H), 3.21 (m, 4H), 1.49 (s, 9H)

### Step B: Preparation of 3-((5-chloro-2-oxo-3-(4-(piperazin-1-yl)phenyl)-2,3-dihydro-1H-benzo[d]imidazol-1-yl)methyl)-2-fluorobenzoic acid methyl ester hydrochloride

To 4-(4-(6-chloro-3-(2-fluoro-3-(methoxycarbonyl)benzyl)-2-oxo-2,3-dihydro-1*H-*benzo[*d*]imidazol-1-yl)phenyl)piperazine-1-carboxylic acid *tert*-butyl ester (0.71 g, 1.2 mmol) obtained in step A above were added 4 mL of DCM and 1.2 mL of hydrochloric acid (4.0 M EtOAc solution), then the resulting mixture was stirred at room temperature for 1 hour, and then the reaction solution was concentrated under reduced pressure to give the title compound (0.55 g, yield: 86%).
¹H-NMR (400 MHz, DMSO-d6) δ8.86 (brs, 2H), 7.83 (td, 1H), 7.58 (td, 1H), 7.43 (d, 2H), 7.30 (t, 1H), 7.24 (m, 1H), 7.18 (3H), 6.91 (d, 1H), 5.22 (s, 2H), 3.86 (s, 3H), 3.45 (m, 4H), 3.27 (m, 4H)

### Preparation Example 43: Preparation of 3-((5-chloro-4-fluoro-2-oxo-3-(4-(piperidin-4-yl)phenyl)-2,3-dihydro-1H-benzo[d]imidazol-1-yl)methyl)-2-fluorobenzoic acid methyl ester hydrochloride

The title compound (0.27 g, yield: 13%) was obtained in the same manner as in Preparation Example 30 by using 4-(4-aminophenyl)piperidine-1-carboxylic acid *tert*-butyl ester (1.00 g, 3.62 mmol), 1-chloro-2,3-difluoro-4-nitrobenzene (0.70 g, 3.62 mmol), and 3-(bromomethyl)-2-fluorobenzoic acid methyl ester.
¹H-NMR (400 MHz, DMSO-d6) δ 8.74 (m, 1H), 8.55 (m, 1H), 7.83 (td, 1H), 7.61 (td, 1H), 7.52 (dd, 2H), 7.38 (d, 2H), 7.30 (2H), 7.13 (d, 1H), 5.24 (s, 2H), 3.86 (s, 3H), 3.40 (m, 2H), 3.04 (m, 2H), 2.95 (m, 1H), 2.02 (m, 2H), 1.87 (m, 2H)

### Preparation Example 44: Preparation of 6-chloro-1-(6-(3,3-difluoropyrrolidin-1-yl)pyridin-3-yl)-1,3-dihydro-2H-benzo[d]imidazol-2-one

The process of the following steps A, B, C, and D gave the title compound.

### Step A: Preparation of 2-(3,3-difluoropyrrolidin-1-yl)-5-nitropyridine

The title compound (1.84 g, yield: 91%) was obtained in the same manner as in step A of Preparation Example 1 by using 2-fluoro-5-nitropyridine (1.26 g, 8.87 mmol) and 3,3-difluoropyrrolidine hydrochloride (1.40 g, 9.47 mmol).
¹H-NMR (400 MHz, DMSO-d6) δ9.01 (d, 1H), 8.30 (dd, 1H), 6.68 (m, 1H), 4.01 (t, 2H), 3.79 (m, 2H), 2.60 (m, 2H)

### Step B: Preparation of 6-(3,3-difluoropyrrolidin-1-yl)pyridin-3-amine

The title compound (1.58 g, yield: 99%) was obtained in the same manner as in step A of Preparation Example 9 by using 2-(3,3-difluoropyrrolidin-1-yl)-5-nitropyridine (1.84 g, 8.03 mmol) obtained in step A above.
¹H-NMR (400 MHz, CDCl3) δ7.77 (d, 1H), 7.01 (dd, 1H), 6.28 (d, 1H) 3.78 (t, 2H), 3.60 (t, 2H), 3.27 (brs, 2H), 2.48 (m, 2H)

### Step C: Preparation of N-(5-chloro-2-nitrophenyl)-6-(3,3-difluoropyrrolidin-1-yl)pyridin-3-amine

To 2-bromo-4-chloronitrobenzene (1.30 g, 5.52 mmol) and 6-(3,3-difluoropyrrolidin-1-yl)pyridin-3-amine (1.0 g, 5.0 mmol) obtained in step B above were added 34 mL of toluene and BINAP (0.38 g, 0.60 mmol), and then the resulting mixture was charged with nitrogen gas. To the mixture were added Cs₂CO₃ (2.62 g, 8.0 mmol) and Pd₂(dba)₃ (0.46 g, 0.50 mmol), and the resulting mixture was stirred at 100 °C for 16 hours. The resulting solid was removed through Celite and the filtrate was purified by MPLC to give the title compound (1.11 g, yield: 63%) .
¹H-NMR (400 MHz, CDCl3) δ9.30 (brs, 1H), 8.17 (d, 1H), 8.12 (dd, 1H), 7.41 (dd, 1H), 6.80 (d, 1H), 6.69 (dd, 1H), 6.45 (dd, 1H), 3.90 (t, 2H), 3.74 (t, 2H), 2.54 (m, 2H)

### Step D: Preparation of 6-chloro-1-(6-(3,3-difluoropyrrolidin-1-yl)pyridin-3-yl)-1,3-dihydro-2H-benzo[d]imidazol-2-one

The title compound (0.48 g, yield: 43%) was obtained in the same manner as in step B of Preparation Example 7 and step B of Preparation Example 8 by using *N*-(5-chloro-2-nitrophenyl)-6-(3,3-difluoropyrrolidin-1-yl)pyridin-3-amine (1.12 g, 3.16 mmol) obtained in step C above.
¹H-NMR (400 MHz, DMSO-d6) δ11.28 (brs, 1H), 8.22 (d, 1H), 7.70 (dd, 1H), 7.07 (2H), 6.82 (d, 1H), 6.71 (dd, 1H), 3.90 (t, 2H), 3.67 (t, 2H), 2.56 (m, 2H)

### Preparation Example 45: Preparation of 3-(4-(dimethylamino)phenyl)-5-methyl-1,3-dihydro-2H-imidazo[4,5-b]pyridin-2-one

The process of the following steps A and B gave the title compound.

### Step A: Preparation of N¹,N¹-dimethyl-N⁴-(6-methyl-3-nitropyridin-2-yl)benzene-1,4-diamine

The title compound (0.77 g, yield: 88%) was obtained in the same manner as in step A of Preparation Example 1 by using 2-fluoro-6-methyl-3-nitropyridine (0.50 g, 3.2 mmol) and *N*¹,*N*¹-dimethylbenzene-1,4-diamine (0.44 g, 3.2 mmol).
¹H-NMR (400 MHz, CDCl3) δ10.15 (brs, 1H), 8.37 (d, 1H), 7.53 (d, 2H), 6.76 (d, 2H), 6.58 (dd, 1H), 2.97 (s, 6H), 2.48 (s, 3H)

### Step B: Preparation of 3-(4-(dimethylamino)phenyl)-5-methyl-1,3-dihydro-2H-imidazo[4,5-b]pyridin-2-one

The title compound (0.20 g, yield: 26%) was obtained in the same manner as in step B of Preparation Example 7 and step B of Preparation Example 8 by using *N*¹,*N*¹-dimethyl-*N*⁴-(6-methyl-3-nitropyridin-2-yl)benzene-1,4-diamine (0.77 g, 2.83 mmol) obtained in step A above.
¹H-NMR (400 MHz, CDCl3) δ8.07 (d, 1H), 7.52 (brs, 1H), 7.42 (dd, 2H), 7.00 (dd, 1H), 6.89 (dd, 2H), 3.05 (s, 6H), 2.50 (s, 3H)

### Preparation Example 46: Preparation of 3-(1-(5-chloro-2-oxo-2,3-dihydro-1H-benzo[d]imidazol-1-yl)cyclobutyl)benzoic acid methyl ester

The process of the following steps A, B, C, D, and E gave the title compound.

### Step A: Preparation of 3-(1-((tert-butoxycarbonyl)amino)cyclobutyl)benzoic acid

*tert*-butyl (1-(3-bromophenyl)cyclobutyl)carbamate (196 mg, 0.60 mmol) was dissolved in 3 mL of DMF, and oxalic acid (81.03 mg, 0.90 mmol), acetic anhydride (92 mg, 0.90 mmol), Pd(OAc)₂ (1.35 mg, 6 µmol), and Xantphos (3.47 mg, 6 µmol) were added thereto, then DIPEA (116 mg, 0.90 mmol) was added thereto, and then the resulting mixture was charged with nitrogen gas, followed by stirring at 100 °C for 5 hours. After the reaction was terminated, the reaction solution was diluted by the addition of EtOAc and water, then 1 N HCl (1 mL) was added thereto, and an organic layer was separated. The organic layer was dried over anhydrous Na₂SO₄ and purified by MPLC to give the title compound (138 mg, yield: 79%).
¹H NMR (500 MHz, CDCl3) δ8.16 (s, 1H), 7.99 (d, 1H), 7.69 (br s, 1H), 7.46 (t, 1H), 2.61 (m, 2H), 2.51 (m, 2H), 2.15 (m, 1H), 1.92 (m, 1H), 1.38 (m, 9H)

### Step B: Preparation of 3-(1-((tert-butoxycarbonyl)amino)cyclobutyl)benzoic acid methyl ester

3-(1-((*tert*-butoxycarbonyl)amino)cyclobutyl)benzoic acid (135 mg, 0.463 mmol) obtained in step A above was dissolved in 1 mL of DMF, Na₂CO₃ (98 mg 0.927 mmol) was added thereto, then iodomethane (43.5 µL, 0.695 mmol) was added to the reaction solution, and the reaction solution was stirred at 40 °C. After the reaction was terminated, EtOAc and water were added thereto, and an organic layer was separated and dried over anhydrous MgSO₄. The filtrate was distilled under reduced pressure and then purified by MPLC to give the title compound (20 mg, yield: 61%).
¹H NMR (500 MHz, CDCl3) δ8.08 (m, 1H), 7.91 (m, 1H), 7.64 (br s, 1H), 7.42 (m, 1H), 5.19 (s, 1H), 3.91 (s, 3H), 2.54 (m, 4H), 2.12 (m, 1H), 1.87 (m, 1H), 1.51 (m, 9H)

### Step C: Preparation of 3-(1-aminocyclobutyl)benzoic acid methyl ester

3-(1-((*tert*-butoxycarbonyl)amino)cyclobutyl)benzoic acid methyl ester (125 mg, 0.409 mmol) obtained in step B above was dissolved in 20 mL of DCM, and 8 mL of TFA was added thereto, followed by stirring at room temperature for 3 hours. After the reaction was terminated, the reaction solution was distilled under reduced pressure to remove TFA and the solvent, and then was carried through to the next step without further purification.

### Step D: Preparation of 3-(1-((4-chloro-2-nitrophenyl)amino)cyclobutyl)benzoic acid methyl ester

The title compound (77.1 mg, yield: 52%) was obtained in the same manner as in step A of Preparation Example 1 by using 4-chloro-1-fluoro-2-nitrobenzene and 3-(1-aminocyclobutyl)benzoic acid methyl ester obtained in step C above.
¹H NMR (500 MHz, CDCl3) δ8.75 (s, 1H), 8.18 (s, 2H), 7.95 (d, 1H), 7.63 (d, 1H), 7.43 (t, 1H), 7.10 (d, 1H), 6.16 (d, 1H), 3.93 (s, 3H), 2.78 (m, 2H), 2.54 (m, 2H), 2.24 (m, 2H)

### Step E: Preparation of 3-(1-(5-chloro-2-oxo-2,3-dihydro-1H-benzo[d]imidazol-1-yl)cyclobutyl)benzoic acid methyl ester

The title compound (26.9 mg, yield: 36%) was obtained in the same manner as in steps B and C, in turn, of Preparation Example 1 by using 3-(1-((4-chloro-2-nitrophenyl)amino)cyclobutyl)benzoic acid methyl ester obtained in step D above.
¹H NMR (500 MHz, CDCl3) δ10.39 (s, 1H), 8.35 (s, 1H), 7.95 (d, 1H), 7.80 (d, 1H), 7.43 (t, 1H), 7.08 (s, 1H), 6.94 (d, 1H), 6.81 (d, 1H), 3.91 (s, 3H), 3.20 (m, 2H), 3.02 (m, 2H), 2.07 (m, 2H)

### Preparation Example 47: Preparation of 3-(2-(5-chloro-2-oxo-2,3-dihydro-1H-benzo[d]imidazol-1-yl)propan-2-yl)benzoic acid methyl ester

The process of the following steps A, B, C, D, and E gave the title compound.

### Step A: Preparation of 3-(2-((tert-butoxycarbonyl)amino)propan-2-yl)benzoic acid

The title compound (139 mg, yield: 20%) was obtained in the same manner as in step A of Preparation Example 46 by using *tert*-butyl (2-(3-bromophenyl)propan-2-yl)carbamate (785 mg, 2.50 mmol).
¹H NMR (500 MHz, CDCl3) δ8.14 (s, 1H), 7.98 (d, 1H), 7.66 (d, 1H), 7.45 (t, 1H), 1.65 (s, 6H), 1.40 (m, 9H)

### Step B: Preparation of 3-(2-((tert-butoxycarbonyl)amino)propan-2-yl)benzoic acid methyl ester

The title compound (145 mg, yield: 99%) was obtained in the same manner as in step B of Preparation Example 46 by using 3-(2-((*tert*-butoxycarbonyl)amino)propan-2-yl)benzoic acid obtained in step A above.
¹H NMR (500 MHz, CDCl3) δ8.07 (s, 1H), 7.90 (d, 1H), 7.60 (d, 1H), 7.40 (t, 1H), 4.99 (br s, 1H), 3.90 (s, 3H), 1.63 (s, 6H), 1.39 (m, 9H)

### Step C: Preparation of 3-(2-aminopropan-2-yl)benzoic acid methyl ester

The title compound was obtained in the same manner as in step C of Preparation Example 46 by using 3-(2-((*tert-*butoxycarbonyl)amino)propan-2-yl)benzoic acid methyl ester obtained in step B above, and then was carried through to the next step without further purification.

### Step D: Preparation of 3-(2-((4-chloro-2-nitrophenyl)amino)propan-2-yl)benzoic acid methyl ester

The title compound (41.6 mg, yield: 24%) was obtained in the same manner as in step A of Preparation Example 1 by using 4-chloro-1-fluoro-2-nitrobenzene and 3-(2-aminopropan-2-yl)benzoic acid methyl ester obtained in step C above.

### Step E: Preparation of 3-(2-(5-chloro-2-oxo-2,3-dihydro-1H-benzo[d]imidazol-1-yl)propan-2-yl)benzoic acid methyl ester

The title compound (23.0 mg, yield: 58%) was obtained in the same manner as in steps B and C, in turn, of Preparation Example 1 by using 3-(2-((4-chloro-2-nitrophenyl)amino)propan-2-yl)benzoic acid methyl ester in step D above.
¹H NMR (500 MHz, CDCl3) δ9.73 (s, 1H), 8.07 (s, 1H), 7.98 (d, 1H), 7.52 (d, 1H), 7.43 (t, 1H), 7.01 (s, 1H), 6.67 (d, 1H), 6.04 (d, 1H), 3.92 (s, 3H), 2.10 (s, 6H)

### Preparation Example 48: Preparation of 3-((6-chloro-2-oxo-1,2-dihydro-3H-imidazo[4,5-b]pyridin-3-yl)methyl)benzoic acid methyl ester

The process of the following steps A and B gave the title compound.

### Step A: Preparation of 3-(((5-chloro-3-nitropyridin-2-yl)amino)methyl)benzoic acid methyl ester

3-(Aminomethyl)benzoic acid methyl ester (775 mg, 4.69 mmol) was dissolved in THF and cooled to 0 °C. NaH (222 mg, 5.54 mmol) was added slowly thereto and then stirred for 30 minutes. Thereafter, 5-chloro-2-fluoro-3-nitropyridine (753 mg, 4.27 mmol) was added at 0 °C, and the resulting mixture was stirred while raising the temperature to room temperature. After the reaction was terminated, water was slowly added thereto while stirring at 0 °C, and the organic layer was extracted with EtOAc and a saturated aqueous NH₄Cl solution and dried over Na₂SO₄. The resulting solid was filtered, and the filtrate was distilled under reduced pressure and then purified by MPLC to give the title compound (448 mg, yield: 33%) .
¹H-NMR (500 MHz, CDCl3) δ8.55 (brs, 1H), 8.45 (s, 1H), 8.39 (s, 1H), 8.04 (s, 1H), 7.98 (d, 1H), 7.56 (d, 1H), 7.44 (s, 1H), 4.98 (d, 2H), 3.93 (s, 3H).

### Step B: Preparation of 3-((6-chloro-2-oxo-1,2-dihydro-3H-imidazo[4,5-b]pyridin-3-yl)methyl)benzoic acid methyl ester

The title compound (45 mg, yield: 41%) was obtained in the same manner as in step B of Preparation Example 8 by using 3-(((5-chloro-3-nitropyridin-2-yl)amino)methyl)benzoic acid methyl ester (110 mg, 0.34 mmol) obtained in step A above.
¹H-NMR (500 MHz, CDCl3) δ10.01 (brs, 1H), 8.15 (s, 1H), 8.05 (s, 1H), 7.97 (d, 1H), 7.67 (d, 1H), 7.42 (t, 1H), 7.36 (s, 1H), 5.21 (d, 2H), 3.92 (s, 3H).

### Preparation Example 49: Preparation of 3-(1-(2-oxo-5-(trifluoromethyl) -2,3-dihydro-1H-benzo[d]imidazol-1-yl)cyclopropyl)benzoic acid methyl ester

The process of the following steps A and B gave the title compound.

### Step A: Preparation of 3-(1-((2-nitro-4-(trifluoromethyl)phenyl)amino)cyclopropyl)benzoic acid methyl ester

The title compound (0.86 g, yield: 94%) was obtained in the same manner as in step A of Preparation Example 1 by using 1-fluoro-2-nitro-4-(trifluoromethyl)benzene (0.50 g, 2.39 mmol) and 3-(1-aminocyclopropyl)benzoic acid methyl ester hydrochloride (0.60 g, 2.63 mmol).
¹H-NMR (400 MHz, CDCl3) δ8.82 (brs, 1H), 7.50 (d, 1H), 7.88 (m, 1H), 7.76 (d, 1H), 7.51 (dd, 1H), 7.30 (2H), 7.04 (dd, 1H), 3.91 (s, 3H), 1.58 (m, 2H), 1.44 (m, 2H)

### Step B: Preparation of 3-(1-(2-oxo-5-(trifluoromethyl)-2,3-dihydro-1H-benzo[d]imidazol-1-yl)cyclopropyl)benzoic acid methyl ester

The title compound (0.29 g, yield: 35%) was obtained in the same manner as in step B of Preparation Example 7 and step B of Preparation Example 8 by using 3-(1-((2-nitro-4-(trifluoromethyl)phenyl)amino)cyclopropyl)benzoic acid methyl ester (0.85 g, 2.23 mmol) obtained in step A above.
¹H-NMR (400 MHz, CDCl3) δ9.75 (brs, 1H), 7.89 (dd, 1H), 7.30 (d, 1H), 7.35 (4H), 7.23 (dd, 1H), 3.89 (s, 3H), 1.72 (m, 4H)

### Preparation Example 50: Preparation of 3-(1-(2-oxo-5-(trifluoromethoxy)-2,3-dihydro-1H-benzo[d]imidazol-1-yl)cyclopropyl)benzoic acid methyl ester

The process of the following steps A and B gave the title compound.

### Step A: Preparation of 3-(1-((2-nitro-4-(trifluoromethoxy)phenyl)amino)cyclopropyl)benzoic acid methyl ester

The title compound (0.86 g, yield: 97%) was obtained in the same manner as in step A of Preparation Example 1 by using 1-fluoro-2-nitro-4-(trifluoromethoxy)benzene (0.50 g, 2.22 mmol) and 3-(1-aminocyclopropyl)benzoic acid methyl ester hydrochloride (0.55 g, 2.44 mmol).
¹H-NMR (400 MHz, CDCl3) δ8.62 (brs, 1H), 8.10 (dd, 1H), 7.88 (m, 1H), 7.76 (d, 1H), 7.40 (2H), 7.21 (dd, 1H), 6.96 (dd, 1H), 3.90 (s, 3H), 1.55 (m, 2H), 1.42 (m, 2H)

### Step B: Preparation of 3-(1-(2-oxo-5-(trifluoromethoxy)-2,3-dihydro-1H-benzo[d]imidazol-1-yl)cyclopropyl)benzoic acid methyl ester

The title compound (0.21 g, yield: 25%) was obtained in the same manner as in step B of Preparation Example 7 and step B of Preparation Example 8 by using 3-(1-((2-nitro-4-(trifluoromethoxy)phenyl)amino)cyclopropyl)benzoic acid methyl ester (0.85 g, 2.14 mmol) obtained in step A above.
¹H-NMR (400 MHz, CDCl3) δ8.81 (brs, 1H), 7.89 (dd, 1H), 7.81 (d, 1H), 7.38 (2H), 7.10 (d, 1H), 7.00 (d, 1H), 6.95 (dd, 1H), 3.89 (s, 3H), 1.69 (m, 4H)

### Preparation Example 51: Preparation of 4-(4-bromophenyl)-1,4-oxazepane

The process of the following steps A and B gave the title compound.

### Step A: Preparation of 4-(1,4-oxazepan-4-yl)aniline

The title compound (0.32 g, yield: 23%) was obtained in the same manner as in step B of Preparation Example 1 and step B of Preparation Example 7 by using 1-fluoro-4-nitrobenzene (1.02 g, 7.23 mmol) and 1,4-oxazepane hydrochloride (0.10 g, 7.25 mmol).
¹H-NMR (400 MHz, DMSO-d6) δ6.50 (m, 4H), 4.36 (brs, 2H), 3.66 (t, 2H), 3.55 (t, 2H), 3.41 (t, 4H), 1.86 (m, 2H)

### Step B: Preparation of 4-(4-bromophenyl)-1,4-oxazepane

To 4-(1,4-oxazepan-4-yl)aniline (0.32 g, 1.66 mmol) obtained in step A above was added a 48% aqueous bromic acid solution (3.3 mL), and then the resulting mixture was cooled to 0 °C. To the mixture was slowly added an aqueous NaNO₂ solution (4 mL, 5 mmol), and the resulting mixture was stirred at room temperature for 15 minutes. The reaction solution was cooled to 0 °C, then CuBr (0.36 g, 2.50 mmol) was added thereto, followed by stirring under reflux at 100 °C for 2 hours. The reaction solution was cooled to room temperature, then was extracted by the addition of an aqueous NaOH solution and EtOAc, and then an organic layer was purified by MPLC to give the title compound (0.14 g, yield: 33%).
¹H-NMR (400 MHz, CDCl3) δ7.27 (d, 2H), 6.58 (d, 2H), 3.81 (m, 2H), 3.67 (m, 2H), 3.58 (m, 4H), 2.01 (m, 2H)

### Preparation Example 52: Preparation of 2-(4-bromophenyl)-2-azaspiro[3.3]heptane

To 1,4-dibromobenzene (0.50 g, 2.12 mmol), 2-azaspiro[3.3]heptane hydrochloride (0.31 g, 2.33 mmol), and BINAP (0.13 g, 0.21 mmol) was added 10 mL of toluene, and then the resulting mixture was charged with nitrogen gas. To the mixture were added Pd₂(dba)₃ (0.10 g, 0.11 mmol) and Cs₂CO₃ (1.52 g, 4.66 mmol) were added, followed by stirring at 85 °C for 16 hours. After the reaction solution was cooled to room temperature, the resulting solid was removed through Celite, and the filtrate was purified by MPLC to give the title compound (0.28 g, yield: 52%).
¹H-NMR (400 MHz, DMSO-d6) δ7.27 (d, 2H), 6.35 (d, 2H), 3.74 (s, 4H), 2.15 (t, 4H), 1.81 (m, 2H)

### Preparation Example 53: Preparation of 6-chloro-7-fluoro-1-(4-morpholinophenyl)-1,3-dihydro-2H-benzo[d]imidazol-2-one

The title compound (3.10 g, yield: 48%) was obtained in the same manner as in step A of Preparation Example 1, step B of Preparation Example 7, and step B of Preparation Example 8 by using 4-morpholinoaniline (3.50 g, 19.6 mmol) and 1-chloro-2,3-difluoro-4-nitrobenzene (3.80 g, 19.6 mmol).
¹H-NMR (400 MHz, DMSO-d6) δ11.41 (s, 1H), 7.32 (m, 2H), 7.17 (dd, 1H), 7.03 (d, 2H), 6.90 (dd, 1H), 3.77 (m, 4H), 3.18 (m, 4H)

### Preparation Example 54: Preparation of 6-chloro-1-(4-morpholinophenyl)-1,3-dihydro-2H-benzo[d]imidazol-2-one

The title compound (7.2 g, yield: 98%) was obtained in the same manner as in step A of Preparation Example 1, step B of Preparation Example 7, and step B of Preparation Example 8 by using 4-morpholinoaniline (4.00 g, 22.4 mmol) and 4-chloro-2-fluoro-1-nitrobenzene (3.94 g, 22.4 mmol).
¹H-NMR (400 MHz, DMSO-d6) δ11.22 (brs, 1H), 7.35 (d, 2H), 7.06 (4H), 6.82 (d, 1H), 3.77 (m, 4H), 3.18 (m, 4H)

### Preparation Example 55: Preparation of 5-(bromomethyl)isoxazole-3-carboxylic acid methyl ester

To 5-methylisoxazole-3-carboxylic acid methyl ester (2.0 g, 14.1 mmol) were added 70 mL of CCl₄, *N*-bromosuccinimide (3.28 g, 18.4 mmol), and azobisbutyronitrile (0.23 g, 1.41 mmol), and the resulting mixture was stirred at 70 °C for 16 hours. The resulting solid was filtered and removed, and then the filtrate was purified by MPLC to give the title compound (1.5 g, yield: 48%).
¹H-NMR (400 MHz, CDCl3) δ6.75 (s, 1H), 4.51 (s, 2H), 4.00 (s, 3H)

### Preparation Example 56: Preparation of 2-(bromomethyl)oxazole-4-carboxylic acid methyl ester

The title compound (1.14 g, yield: 36%) was obtained in the same manner as in Preparation Example 55 by using 2-methyloxazole-4carboxylic acid methyl ester (2.0 g, 14.1 mmol).
¹H-NMR (400 MHz, CDCl3) δ8.25 (s, 1H), 4.48 (s, 2H), 3.93 (s, 3H)

### Preparation Example 57: Preparation of 5-(bromomethyl)-2,3-difluorobenzoic acid ethyl ester

The process of the following steps A and B gave the title compound.

### Step A: Preparation of 2,3-difluoro-5-methylbenzoic acid ethyl ester

To 2,3-difluoro-5-methylbenzoic acid (2.0 g, 11.6 mmol) were added 23 mL of EtOH and a catalytic amount of sulfuric acid, and the resulting mixture was stirred under reflux for 16 hours. The reaction solution was poured into ice-water, and then the resulting solid was dried to give the title compound (2.0 g, yield: 86%).
¹H-NMR (400 MHz, CDCl3) δ7.47 (m, 1H), 7.15 (m, 1H), 4.40 (q, 2H), 2.34 (s, 3H), 1.41 (t, 3H)

### Step B: Preparation of 5-(bromomethyl)-2,3-difluorobenzoic acid ethyl ester

The title compound (1.34 g, yield: 48%) was obtained in the same manner as in Preparation Example 55 by using 2,3-difluoro-5-methyl benzoic acid ethyl ester (2.0 g, 9.99 mmol) obtained in step A above.
¹H-NMR (400 MHz, CDCl3) δ7.72 (m, 1H), 7.41 (m, 1H), 4.43 (s, 2H), 4.41 (q, 2H), 1.41 (t, 3H)

### Preparation Example 58: Preparation of 3-(bromomethyl)-2,6-difluorobenzoic acid ethyl ester

The process of the following steps A and B gave the title compound.

### Step A: Preparation of 2,6-difluoro-3-methylbenzoic acid ethyl ester

To 2,6-difluoro-3-methylbenzoic acid (2.0 g, 11.6 mmol) were added 23 mL of EtOH and a catalytic amount of sulfuric acid, and the resulting mixture was stirred under reflux for 16 hours. The reaction solution was poured into ice-water, and then the resulting solid was dried to give the title compound (1.80 g, yield: 77%).
¹H-NMR (500 MHz, CDCl3) δ7.26 (m, 1H), 6.86 (m, 1H), 4.44 (q, 2H), 2.27 (s, 3H), 1.41 (t, 3H)

### Step B: Preparation of 3-(bromomethyl)-2,6-difluorobenzoic acid ethyl ester

The title compound (0.56 g, yield: 22%) was obtained in the same manner as in Preparation Example 55 by using 2,6-difluoro-3-methyl benzoic acid ethyl ester (1.80 g, 9.0 mmol) obtained in step A above.
¹H-NMR (500 MHz, CDCl3) δ7.48 (m, 1H), 6.92 (m, 1H), 4.47 (s, 2H), 4.43 (q, 2H), 1.40 (t, 3H)

### Preparation Example 59: Preparation of 3-(1-(5-chloro-2-oxo-2,3-dihydro-1H-benzo[d]imidazol-1-yl)cyclopropyl)-5-fluorobenzoic acid ethyl ester

The process of the following steps A and B gave the title compound.

### Step A: Preparation of 3-(1-aminocyclopropyl)-5-fluorobenzoic acid ethyl ester hydrochloride

To 3-cyano-5-fluorobenzoic acid ethyl ester (5.0 g, 25.9 mmol) were added 100 mL of Et₂O and 20 mL of THF, and then the resulting mixture was cooled to -78 °C, and then Ti(OiPr)₄ (10 mL, 34 mmol) and EtMgBr (3.0 M Et₂O solution, 20 mL, 60 mmol) were slowly added thereto. The reaction solution was stirred at room temperature for 1 hour, then cooled to 0 °C, BF₃-Et₂O (6.6 mL, 52 mmol) was then slowly added thereto, and the reaction solution was stirred at room temperature for 1 hour. After the reaction was terminated, an aqueous 3 M HCl solution was slowly added thereto, and an organic layer was extracted with EtOAc. After the organic layer was concentrated, HCl (4 M EtOAc solution) was added thereto, and the resulting solid was dried to give the title compound (2.6 g, yield: 38%).
¹H-NMR (500 MHz, CDCl3) δ 9.06 (brs, 3H), 7.89 (d, 1H), 7.66 (dd, 1H), 7.47 (dd, 1H), 4.36 (q, 2H), 1.68 (m, 4H), 1.38 (t, 3H)

### Step B: Preparation of 3-(1-(5-chloro-2-oxo-2,3-dihydro-1H-benzo[d]imidazol-1-yl)cyclopropyl)-5-fluorobenzoic acid ethyl ester

The title compound (0.12 g, yield: 6%) was obtained in the same manner as in step A of Preparation Example 1, step B of Preparation Example 7, and step B of Preparation Example 8 by using 3-(1-aminocyclopropyl)-5-fluorobenzoic acid ethyl ester hydrochloride (1.62 g, 6.2 mmol) obtained in step A above and 4-chloro-1-fluoro-2-nitrobenzene (1.0 g, 5.7 mmol).
¹H-NMR (400 MHz, CDCl3) δ9.38 (brs, 1H), 7.56 (m, 2H), 7.14 (d, 1H), 7.04 (3H), 4.37 (q, 2H), 1.72 (4H), 1.38 (t, 3H)

### Preparation Example 60: Preparation of 3-(4-bromophenyl)-8-oxa-3-azabicyclo[3.2.1]octane

The title compound (0.11 g, yield: 14%) was obtained in the same manner as in Preparation Example 38 by using (4-bromophenyl)boronic acid (0.66 g, 3.29 mmol) and 8-oxa-3-azabicyclo[3.2.1]octane hydrochloride (0.44 g, 3 mmol).
¹H-NMR (400 MHz, CDCl3) δ7.33 (d, 2H), 6.67 (d, 2H), 4.48 (m, 2H), 3.27 (m, 2H), 2.98 (m, 2H), 1.95 (4H)

### Preparation Example 61: Preparation of 1-(4-bromophenyl)-3,3-difluoroazetidine

The title compound (0.42 g, yield: 80%) was obtained in the same manner as in Preparation Example 52 by using 1,4-dibromobenzene (0.50 g, 2.12 mmol) and 3,3-difluoroazetidine hydrochloride (0.31 g, 2.33 mmol).
¹H-NMR (400 MHz, DMSO-d6) δ7.38 (d, 2H), 6.53 (d, 2H), 4.25 (t, 4H)

### Preparation Example 62: Preparation of 1-(4-bromophenyl)-4,4-difluoropiperidine

The title compound (0.075 g, yield: 18%) was obtained in the same manner as in Preparation Example 38 by using (4-bromophenyl)boronic acid (0.30 g, 1.50 mmol) and 4,4-difluoropiperidine (0.22 g, 1.8 mmol).
¹H-NMR (400 MHz, CDCl3) δ7.36 (d, 2H), 6.82 (d, 2H), 3.32 (m, 4H), 2.08 (m, 4H)

### Preparation Example 63: Preparation of 2-(4-bromophenyl)-2,7-diazaspiro[3.5]nonane-7-carboxylic acid tert-butyl ester

The title compound (1.70 g, yield: 70%) was obtained in the same manner as in Preparation Example 52 by using 1,4-dibromobenzene (1.50 g, 6.36 mmol) and 2,6-diazaspiro[3.5]nonane-7-carboxylic acid tert-butyl ester hydrochloride (1.84 g, 7.00 mmol).
¹H-NMR (400 MHz, CDCl3) δ7.28 (d, 2H), 6.31 (d, 2H), 3.59 (s, 4H), 3.39 (m, 4H), 1.75 (m, 4H), 1.46 (s, 9H)

### Preparation Example 64: Preparation of 2-(4-bromophenyl)-1-(3-hydroxyazetidin-1-yl)ethan-1-one

To 2-(4-bromophenyl)acetic acid (200 mg, 0.93 mmol) and 3-hydroxyazetidine hydrochloride (122 mg, 1.2 mmol) were added 2 mL of DMF, HATU (390 mg, 1.02 mmol), and TEA (0.26 mL, 1.86 mmol), and then the resulting mixture was stirred at room temperature for 16 hours. To the reaction solution was added water, and then the resulting mixture was extracted with EtOAc and purified by MPLC to give the title compound (150 mg, yield: 60%) .
¹H-NMR (500 MHz, CDCl3) 7.46 (d, 2H), 7.16 (d, 2H), 4.65 (m, 1H), 4.32 (t, 1H), 4.25 (t, 1H), 4.00 (dd, 1H), 3.88 (dd, 1H), 3.43 (s, 2H), 2.59 (s, 1H)

### Preparation Example 65: Preparation of 2-(4-bromophenyl)-1-(4,4-difluoropiperidin-1-yl)ethan-1-one

The title compound (102 mg, yield: 69%) was obtained in the same manner as in Preparation Example 64 by using 2-(4-bromophenyl)acetic acid (100 mg, 0.46 mmol) and 4,4-difluoropiperidine (67 mg, 0.56 mmol).
¹H-NMR (500 MHz, CDCl3) δ7.48 (d, 2H), 7.14 (d, 2H), 3.76 (m, 2H), 3.72 (s, 2H), 3.55 (m, 2H), 1.96 (m, 2H), 1.79 (m, 2H)

### Preparation Example 66: Preparation of (S)-2-(4-bromophenyl)-1-(3-methylmorpholino)ethan-1-one

The title compound (160 mg, yield: 58%) was obtained in the same manner as in Preparation Example 64 by using 2-(4-bromophenyl)acetic acid (200 mg, 0.93 mmol) and (S)-3-morpholine (113 mg, 1.11 mmol).
¹H-NMR (500 MHz, CDCl3) δ7.47 (d, 2H), 7.14 (m, 2H), 4.31 (m, 1H), 3.86 (m, 2H), 3.66 (s, 2H), 3.60 (m, 1H), 3.41 (m, 2H), 3.30 (m, 1H), 1.27 (d, 3H)

### Preparation Example 67: Preparation of (R)-2-(4-bromophenyl)-1-(3-methylmorpholino)ethan-1-one

The title compound (160 mg, yield: 58%) was obtained in the same manner as in Preparation Example 64 by using 2-(4-bromophenyl)acetic acid (200 mg, 0.93 mmol) and (*R*)-3-morpholine (113 mg, 1.11 mmol).
¹H-NMR (500 MHz, CDCl3) δ7.47 (d, 2H), 7.14 (m, 2H), 4.30 (m, 1H), 3.86 (m, 2H), 3.66 (s, 2H), 3.62 (m, 1H), 3.38 (m, 2H), 3.31 (m, 1H), 1.27 (d, 3H)

### Preparation Example 68: Preparation of (R)-2-(4-bromophenyl)-1-(2-methylmorpholino)ethan-1-one

The title compound (162 mg, yield: 58%) was obtained in the same manner as in Preparation Example 64 by using 2-(4-bromophenyl)acetic acid (200 mg, 0.93 mmol) and (*R*)-2-morpholine (113 mg, 1.11 mmol).
¹H-NMR (500 MHz, CDCl3) δ7.47 (d, 2H), 7.14 (d, 2H), 4.45 (dd, 1H), 3.82 (dd, 1H), 3.68 (s, 2H), 3.61 (t, 1H), 3.47 (m, 1H), 3.33 (m, 1H), 3.19 (m, 1H), 2.85 (m, 1H), 2.82 (s, 3H), 1.16 (3H)

### Preparation Example 69: Preparation of (S)-2-(4-bromophenyl)-1-(2-methylmorpholino)ethan-1-one

The title compound (134 mg, yield: 48%) was obtained in the same manner as in Preparation Example 64 by using 2-(4-bromophenyl)acetic acid (200 mg, 0.93 mmol) and (*S*)-2-morpholine (113 mg, 1.11 mmol).
¹H-NMR (500 MHz, CDCl3) δ7.47 (d, 2H), 7.14 (d, 2H), 4.45 (dd, 1H), 3.82 (dd, 1H), 3.68 (s, 2H), 3.61 (t, 1H), 3.47 (m, 1H), 3.33 (m, 1H), 3.19 (m, 1H), 2.85 (m, 1H), 2.82 (s, 3H), 1.16 (3H)

### Preparation Example 70: Preparation of 4-bromo-N,N-dimethylbenzenesulfonamide

To 4-bromobenzenesulfonyl chloride (200 mg, 0.78 mmol) were added 15 mL of THF, dimethylamine (0.79 mL, 1.56 mmol), and DIPEA (0.28 mL, 1.56 mmol), and then the resulting mixture was stirred at room temperature. After the reaction was terminated, the solution was washed with water, and then an organic layer was dried to give the title compound (196 mg, yield: 95%).
¹H-NMR (500 MHz, CDCl3) δδ 7.71 (d, 2H), 7.67 (d, 2H), 2.73 (s, 6H)

### Preparation Example 71: Preparation of 4-bromo-N-cyclopropylbenzenesulfonamide

The title compound (244 mg, yield: 90%) was obtained in the same manner as in Preparation Example 70 by using 4-bromobenzenesulfonyl chloride (250 mg, 0.98 mmol) and cyclopropanamine (112 mg, 2.0 mmol).
¹H-NMR (500 MHz, CDCl3) δ7.80 (d, 2H), 7.70 (d, 2H), 4.85 (brs, 1H), 2.28 (m, 1H), 0.64 (m, 2H), 0.63 (m, 2H)

### Preparation Example 72: Preparation of 4-((4-bromophenyl)sulfonyl)morpholine

The title compound (273 mg, yield: 91%) was obtained in the same manner as in Preparation Example 70 by using 4-bromobenzenesulfonyl chloride (250 mg, 0.98 mmol) and morpholine (0.34 mL, 2.0 mmol).
¹H-NMR (500 MHz, CDCl3) 7.73 (d, 2H), 7.64 (d, 2H), 3.77 (m, 4H), 3.02 (m, 4H)

### Preparation Example 73: Preparation of 4-bromo-N-(cyclobutylmethyl)benzenesulfonamide

The title compound (140 mg, yield: 78%) was obtained in the same manner as in Preparation Example 70 by using 4-bromobenzenesulfonyl chloride (150 mg, 0.59 mmol) and cyclobutylmethanamine (75 mg, 0.88 mmol).
¹H-NMR (500 MHz, CDCl3) 7.74 (d, 2H), 7.68 (d, 2H), 4.41 (t, 1H), 2.99 (t, 2H), 2.42 (m, 1H), 2.03 (m, 1H), 1.91 (m, 1H), 1.87 (m, 1H), 1.62 (m, 2H)

### Preparation Example 74: Preparation of 1-((4-bromophenyl)sulfonyl)piperidine

The title compound (149 mg, yield: 83%) was obtained in the same manner as in Preparation Example 70 by using 4-bromobenzenesulfonyl chloride (150 mg, 0.59 mmol) and piperidine (100 mg, 1.17 mmol).
¹H-NMR (500 MHz, CDCl3) δ7.69 (d, 2H), 7.64 (d, 2H), 3.00 (m, 4H), 1.66 (m, 4H), 1.45 (m, 2H)

### Preparation Example 75: Preparation of 1-((4-bromophenyl)sulfonyl)pyrrolidine

The title compound (114 mg, yield: 67%) was obtained in the same manner as in Preparation Example 70 by using 4-bromobenzenesulfonyl chloride (150 mg, 0.59 mmol) and pyrrolidine (62.6 mg, 0.88 mmol).
¹H-NMR (500 MHz, CDCl3) δ7.72 (d, 2H), 7.69 (d, 2H), 3.26 (m, 4H), 1.80 (m, 4H)

### Preparation Example 76: Preparation of 3-(1-(6-chloro-2-oxo-1,2-dihydro-3H-imidazo[4,5-b]pyridin-3-yl)cyclopropyl)benzoic acid methyl ester

The process of the following steps A and B gave the title compound.

### Step A: Preparation of 3-(1-((5-chloro-3-nitropyridin-2-yl)amino)cyclopropyl)benzoic acid methyl ester

The title compound (2.34 g, yield: 84%) was obtained in the same manner as in step A of Preparation Example 1 by using 5-chloro-2-fluoro-3-nitropyridine (1.56 g, 8.86 mmol) and 3-(1-aminocyclopropyl)benzoic acid methyl ester (1.54 g, 8.05 mmol).
¹H-NMR (500 MHz, CDCl3) δ8.55 (brs, 1H), 8.45 (s, 1H), 8.39 (s, 1H), 8.04 (s, 1H), 7.98 (d, 1H), 7.56 (d, 1H), 7.44 (t, 1H), 3.93 (s, 3H), 1.74 (m, 4H).

### Step B: Preparation of 3-(1-(2-oxo-5-(trifluoromethyl)-2,3-dihydro-1H-benzo[d]imidazol-1-yl)cyclopropyl)benzoic acid methyl ester

The title compound (0.90 g, yield: 39%) was obtained in the same manner as in step B of Preparation Example 7 and step B of Preparation Example 8 by using 3-(1-((5-chloro-3-nitropyridin-2-yl)amino)cyclopropyl)benzoic acid methyl ester (2.32 g, 6.68 mmol) obtained in step A above.
¹H-NMR (500 MHz, CDCl3) δ8.15 (s, 1H), 8.05 (s, 1H), 7.97 (d, 1H), 7.67 (d, 1H), 7.42 (t, 1H), 7.36 (m, 1H), 3.89 (s, 3H), 1.72 (m, 4H).

### Preparation Example 77: Preparation of 3-(1-(6-chloro-2-oxo-1,2-dihydro-3H-imidazo[4,5-b]pyridin-3-yl)cyclopropyl)-2-fluorobenzoic acid methyl ester

The title compound (0.26 g, yield: 32%) was obtained in the same manner as in step A of Preparation Example 1, step B of Preparation Example 7, and step B of Preparation Example 8 by using 5-chloro-2-fluoro-3-nitropyridine (0.40 g, 2.28 mmol) and 3-(1-aminocyclopropyl)-2-fluoro benzoic acid methyl ester (0.43 g, 2.07 mmol).

¹H-NMR (500 MHz, CDCl3) δ10.10 (brs, 1H), 8.10 (s, 1H), 7.98 (t, 1H), 7.84 (t, 1H), 7.35 (s, 1H), 7.16 (t, 1H), 3.90 (s, 3H), 1.73 (m, 4H).

### Preparation Example 78: Preparation of 3-(1-(6-chloro-2-oxo-1,2-dihydro-3H-imidazo[4,5-b]pyridin-3-yl)cyclopropyl)-5-fluorobenzoic acid methyl ester

The process of the following steps A and B gave the title compound.

### Step A: Preparation of 3-(1-aminocyclopropyl)-5-fluorobenzoic acid methyl ester

The title compound (4.5 g, yield: 32%) was obtained in the same manner as in steps A and B of Preparation Example 4 by using 3-cyano-5-fluorobenzoic acid methyl ester (12.0 g, 67.0 mmol).
¹H-NMR (400 MHz, CDCl3) δ 7.73 (s, 1H), 7.51 (m, 1H), 7.19 (m, 1H), 3.91 (s, 3H), 1.69 (s, 2H), 1.14 (m, 2H), 1.03 (m, 2H)

### Step B: Preparation of 3-(1-(6-chloro-2-oxo-1,2-dihydro-3H-imidazo[4,5-b]pyridin-3-yl)cyclopropyl)-5-fluorobenzoic acid methyl ester

The title compound (1.1 g, yield: 81%) was obtained in the same manner as in step A of Preparation Example 1, step B of Preparation Example 7, and step B of Preparation Example 8 by using 5-chloro-2-fluoro-3-nitropyridine (1.8 g, 8.6 mmol) and 3-(1-aminocyclopropyl)-5-fluorobenzoic acid methyl ester (1.6 g, 9.5 mmol).
¹H-NMR (400 MHz, CDCl3) δ 8.02 (d, 1H), 7.74 (m, 1H), 7.52 (m, 1H), 7.32 (d, 1H), 7.19 (m, 1H), 3.86 (s, 3H), 1.70 (m, 4H)

### [Examples]

### Example 1: Preparation of 3-((5-chloro-2-oxo-3-phenyl-2,3-dihydro-1H-benzo[d]imidazol-1-yl)methyl)benzoic acid

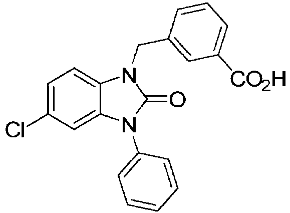

The process of the following steps A and B gave the title compound.

### Step A: Preparation of 3-((5-chloro-2-oxo-3-phenyl-2,3-dihydro-1H-benzo[d]imidazol-1-yl)methyl)benzoic acid methyl ester

To 3-((5-chloro-2-oxo-2,3-dihydro-1H-benzo[*d*]imidazol-1-yl)methyl)benzoic acid methyl ester (30 mg, 0.095 mmol) obtained in step C of Preparation Example 1 was added 1.5 mL of DCM, and then phenyl boronic acid (23 mg, 0.189 mmol), copper(II) acetate (48 mg, 0.265 mmol), and TEA (0.04 mL, 0.284 mmol) were added thereto. After the reaction was terminated, a saturated aqueous K₂CO₃ solution was added thereto, and the resultant mixture was stirred for 20 minutes. The precipitate was removed through Celite, and an organic layer, which was obtained by the extraction with EtOAc, was washed with water. The separated organic layer was dried over anhydrous MgSO₄ and distilled under reduced pressure, and then this was purified by chromatography to give the title compound (27 mg, yield: 72%).

¹H NMR (500 MHz, CDCl₃) δ 8.08 (brs, 1H), 8.01 (d, 1H), 7.58 (m, 5H), 7.45 (m, 2H), 7.10 (s, 1H), 7.04 (d, 1H), 6.84 (d, 1H), 5.19 (s, 2H), 3.94 (s, 3H).

### Step B: Preparation of 3-((5-chloro-2-oxo-3-phenyl-2,3-dihydro-1H-benzo[d]imidazol-1-yl)methyl)benzoic acid

To 3-((5-chloro-2-oxo-3-phenyl-2,3-dihydro-1*H-*benzo[*d*]imidazol-1-yl)methyl)benzoic acid methyl ester (14 mg, 0.035 mmol) obtained in step A above was added 1.5 mL of a mixed solution of THF/MeOH/water (7/2/1). LiOH (1.7 mg, 0.070 mmol) was added thereto, and the resulting mixture was stirred at room temperature for 12 hours, and then when the reaction was terminated, the mixture was diluted with EtOAc. The mixture was extracted with a 1 N aqueous sodium hydroxide solution, and the obtained aqueous solution is adjusted to pH 3 with a 1 N aqueous hydrochloric acid solution and then extracted with EtOAc. The separated organic layer was dried over anhydrous MgSO₄ and distilled under reduced pressure, and then this was purified by chromatography to give the title compound (13 mg, yield: 98%).
¹H NMR (400 MHz, CDCl₃) δ 8.10 (s, 1H), 8.03 (d, 1H), 7.61 (d, 1H), 7.54 (m, 4H), 7.44 (m, 2H), 7.08 (d, 1H), 7.03 (dd, 1H), 6.82 (d, 1H), 5.18 (s, 2H)

### Example 2: Preparation of 3-((5-chloro-2-oxo-3-(4-propylphenyl)-2,3-dihydro-1H-benzo[d]imidazol-1-yl)methyl)benzoic acid

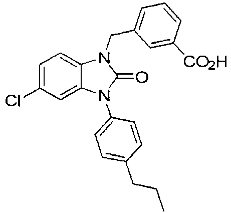

The process of the following steps A and B gave the title compound.

### Step A: Preparation of 3-((5-chloro-2-oxo-3-(4-propylphenyl)-2,3-dihydro-1H-benzo[d]imidazol-1-yl)methyl)benzoic acid methyl ester

To 3-((5-chloro-2-oxo-2,3-dihydro-1*H-*benzo[*d*]imidazol-1-yl)methyl)benzoic acid methyl ester (100 mg, 0.316 mmol) obtained in Preparation Example 1 was added 6 mL of DCM, and then (4-propylphenyl)boronic acid (104 mg, 0.631 mmol), copper(II) acetate (172 mg, 0.947 mmol), and TEA (0.132 mL, 0.947 mmol) were added thereto. After the reaction was terminated, a saturated aqueous K₂CO₃ solution was added thereto, and the resultant mixture was stirred for 20 minutes. Thereafter, the resulting solid was removed through Celite. The filtrate was washed with water, and then an organic layer was separated and dried over Na₂SO₄. The resulting solid was filtered, and the filtrate was distilled under reduced pressure and then purified by MPLC to give the title compound (120 mg, yield: 87%).
MS [M+H] = 435 (M+1)
¹H NMR (500 MHz, CD₃OD) δ 8.08 (s, 1H), 8.00 (d, 1H), 7.59 (d, 1H), 7.45 (d, 3H), 7.38 (d, 2H), 7.08 (s, 1H), 7.03 (d, 1H), 6.82 (d, 1H), 5.18 (s, 2H), 3.94 (s, 3H), 2.68 (t, 2H), 1.72 (m, 2H), 1.02 (t, 3H)

### Step B: Preparation of 3-((5-chloro-2-oxo-3-(4-propylphenyl)-2,3-dihydro-1H-benzo[d]imidazol-1-yl)methyl)benzoic acid

To 3-((5-chloro-2-oxo-3-(4-propylphenyl)-2,3-dihydro-1*H*-benzo[*d*]imidazol-1-yl)methyl)benzoic acid methyl ester (120 mg, 0.276 mmol) obtained in step A above was added 2 mL of THF and 1 N LiOH (2 mL, 2.00 mmol). The resulting mixture was stirred at room temperature for 16 hours, and then a 1 N aqueous hydrochloric acid solution was added thereto. An organic layer was separated and dried over Na₂SO₄. The resulting solid was removed and the filtrate was distilled under reduced pressure to give the title compound (110 mg, yield: 95%).
MS [M+H] = 421 (M+1)
¹H NMR (400 MHz, DMSO-D6) δ7.93 (s, 1H), 7.82 (d, 1H), 7.58 (d, 1H), 7.45 (m, 3H), 7.36 (d, 2H), 7.22 (d, 1H), 7.11 (dd, 1H), 6.96 (d, 1H), 5.17 (s, 2H), 2.60 (t, 2H), 1.61 (td, 2H), 0.90 (t, 3H)

### Example 3: Preparation of 3-((5-chloro-3-(4-fluorophenyl)-2-oxo-2,3-dihydro-1H-benzo[d]imidazol-1-yl)methyl)benzoic acid

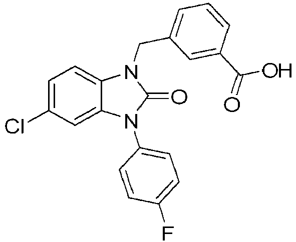

The process of the following steps A and B gave the title compound.

### Step A: Preparation of 3-((5-chloro-3-(4-fluorophenyl)-2-oxo-2,3-dihydro-1H-benzo[d]imidazol-1-yl)methyl)benzoic acid methyl ester

The title compound (0.090 g, yield: 99%) was obtained in the same manner as in step A of Example 2 by using 3-((5-chloro-2-oxo-2,3-dihydro-1*H*-benzo[*d*]imidazol-1-yl)methyl)benzoic acid methyl ester (0.070 g, 0.22 mmol) obtained in Preparation Example 1 and 4-(fluorophenyl)boronic acid (0.062 g, 0.44 mmol).
¹H NMR (400 MHz, CDCl3) δ 8.05 (s, 1H), 7.98 (m, 1H), 7.58-7.50 (3H), 7.43 (t, 1H), 7.26 (m, 2H), 7.06-7.02 (2H), 6.82 (d, 1H), 5.16 (s, 2H), 3.92 (s, 3H)

### Step B: Preparation of 3-((5-chloro-3-(4-fluorophenyl)-2-oxo-2,3-dihydro-1H-benzo[d]imidazol-1-yl)methyl)benzoic acid

The title compound (0.020 g, yield: 23%) was obtained in the same manner as in step B of Example 2 by using 3-((5-chloro-3-(4-fluorophenyl)-2-oxo-2,3-dihydro-1*H-*benzo[*d*]imidazol-1-yl)methyl)benzoic acid methyl ester (0.090 g, 0.22 mmol) obtained in step A above.
¹H NMR (500 MHz, CDCl3) δ 8.10 (s, 1H), 8.04 (dd, 1H), 7.61 (d, 1H), 7.52 (m, 2H), 7.46 (t, 1H), 7.23 (2H), 7.04 (2H), 6.84 (dd, 1H), 5.18 (s, 2H)

### Example 4: Preparation of 3-((5-chloro-2-oxo-3-(4-(trifluoromethyl)phenyl)-2,3-dihydro-1H-benzo[d]imidazol-1-yl)methyl)benzoic acid

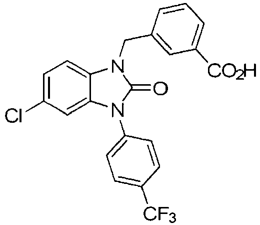

The process of the following steps A and B gave the title compound.

### Step A: Preparation of methyl 3-((5-chloro-2-oxo-3-(4-(trifluoromethyl)phenyl)-2,3-dihydro-1H-benzo[d]imidazol-1-yl)methyl)benzoic acid methyl ester

The title compound (105 mg, yield: 72%) was obtained in the same manner as in step A of Preparation Example 2 by using 3-((5-chloro-2-oxo-2,3-dihydro-1*H*-benzo[*d*]imidazol-1-yl)methyl)benzoic acid methyl ester (100 mg, 0.316 mmol) obtained in Preparation Example 1 and 4-(trifluoromethyl)phenyl)boronic acid (120 mg, 0.631 mmol).
MS [M+H] = 461 (M+1)
¹H NMR (400 MHz, CD₃OD) δδ 8.04 (s, 1H), 7.97 (d, 1H), 7.82 (d, 2H), 7.72 (d, 2H), 7.55 (d, 1H), 7.42 (t, 1H), 7.12 (s, 1H), 7.06 (d, 1H), 6.83 (d, 1H), 5.15 (s, 2H), 3.90 (s, 3H)

### Step B: Preparation of 3-((5-chloro-2-oxo-3-(4-(trifluoromethyl)phenyl)-2,3-dihydro-1H-benzo[d]imidazol-1-yl)methyl)benzoic acid

The title compound (90 mg, yield: 88%) was obtained in the same manner as in step B of Preparation Example 2 by using 3-((5-chloro-2-oxo-3-(4-(trifluoromethyl)phenyl)-2,3-dihydro-1*H*-benzo[*d*]imidazol-1-yl)methyl)benzoic acid methyl ester (105 mg, 0.228 mmol) obtained in step A above.
MS [M+H] = 447 (M+1)
¹H NMR (400 MHz, DMSO-D6) δ7.94 (d, 2H), 7.91 (s, 1H), 7.85 (s, 1H), 7.82 (d, 2H), 7.60 (d, 1H), 7.45 (t, 1H), 7.26 (d, 1H), 7.20 (d, 1H), 7.16 (dd, 1H), 5.19 (s, 2H)

### Example 5: Preparation of 3-((5-chloro-3-(4-methoxyphenyl)-2-oxo-2,3-dihydro-1H-benzo[d]imidazol-1-yl)methyl)benzoic acid

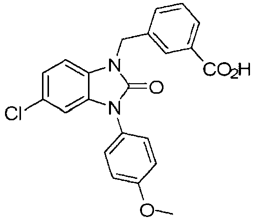

The process of the following steps A and B gave the title compound.

### Step A: Preparation of 3-((5-chloro-3-(4-methoxyphenyl)-2-oxo-2,3-dihydro-1H-benzo[d]imidazol-1-yl)methyl)benzoic acid methyl ester

The title compound (73 mg, yield: 55%) was obtained in the same manner as in step A of Example 2 by using 3-((5-chloro-2-oxo-2,3-dihydro-1*H*-benzo[*d*]imidazol-1-yl)methyl)benzoic acid methyl ester (100 mg, 0.316 mmol) obtained in Preparation Example 1 and 4-(methoxyphenyl)boronic acid (96 mg, 0.631 mmol).
MS [M+H] = 423 (M+1)
¹H NMR (400 MHz, CD₃OD) δδ8.04 (s, 1H), 7.96 (d, 1H), 7.55 (d, 1H), 7.42 (m, 3H), 7.05 (d, 2H), 7.00 (d, 2H), 6.78 (d, 1H), 5.14 (s, 2H), 3.90 (s, 3H), 3.86 (s, 3H)

### Step B: Preparation of 3-((5-chloro-3-(4-methoxyphenyl)-2-oxo-2,3-dihydro-1H-benzo[d]imidazol-1-yl)methyl)benzoic acid

The title compound (62 mg, yield: 88%) was obtained in the same manner as in step B of Example 2 by using 3-((5-chloro-3-(4-methoxyphenyl)-2-oxo-2,3-dihydro-1*H-*benzo[*d*]imidazol-1-yl)methyl)benzoic acid methyl ester (73 mg, 0.173 mmol) obtained in step A above.
MS [M+H] = 409 (M+1)
¹H NMR (400 MHz, DMSO-D6) δ7.93 (s, 1H), 7.82 (dt, 1H), 7.58 (d, 1H), 7.45 (m, 3H), 7.20 (d, 1H), 7.09 (m, 3H), 6.89 (d, 1H), 5.16 (s, 2H), 3.79 (s, 3H)

### Example 6: Preparation of 3-((3-([1,1'-biphenyl]-3-yl)-5-chloro-2-oxo-2,3-dihydro-1H-benzo[d]imidazol-1-yl)methyl)benzoic acid

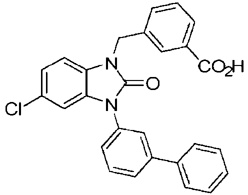

The process of the following steps A and B gave the title compound.

### Step A: Preparation of 3-((3-([1,1'-biphenyl]-3-yl)-5-chloro-2-oxo-2,3-dihydro-1H-benzo[d]imidazol-1-yl)methyl)benzoic acid methyl ester

The title compound (65 mg, yield: 44%) was obtained in the same manner as in step A of Example 2 by using 3-((5-chloro-2-oxo-2,3-dihydro-1*H*-benzo[*d*]imidazol-1-yl)methyl)benzoic acid methyl ester (100 mg, 0.316 mmol) obtained in Preparation Example 1 and [1,1'-biphenyl]-3-ylboronic acid (125 mg, 0.631 mmol).
MS [M+H] = 469 (M+1)
¹H NMR (400 MHz, CD₃OD) δ8.06 (s, 1H), 7.97 (d, 1H), 7.75 (m, 1H), 7.63 (m, 4H), 7.57 (d, 1H), 7.45 (m, 5H), 7.11 (d, 1H), 7.02 (dd, 1H), 6.81 (d, 1H), 5.17 (s, 2H), 3.90 (s, 3H)

### Step B: Preparation of 3-((3-([1,1'-biphenyl]-3-yl)-5-chloro-2-oxo-2,3-dihydro-1H-benzo[d]imidazol-1-yl)methyl)benzoic acid

The title compound (62 mg, yield: 98%) was obtained in the same manner as in step B of Example 2 by using 3-((3-([1,1'-biphenyl]-3-yl)-5-chloro-2-oxo-2,3-dihydro-1*H-*benzo[d]imidazol-1-yl)methyl)benzoic acid methyl ester (65 mg, 0.139 mmol) obtained in step A above.
MS [M+H] = 455 (M+1)
¹H NMR (400 MHz, DMSO-D6) δ7.96 (s, 1H), 7.83 (m, 2H), 7.75 (dt, 1H), 7.70 (m, 2H), 7.65 (t, 1H), 7.60 (d, 1H), 7.55 (dt, 1H), 7.45 (m, 3H), 7.37 (m, 1H), 7.24 (d, 1H), 7.14 (dd, 1H), 7.07 (d, 1H), 5.19 (s, 2H)

### Example 7: Preparation of 3-((5-chloro-3-(4'-chloro-[1,1'-biphenyl]-4-yl)-2-oxo-2,3-dihydro-1H-benzo[d]imidazol-1-yl)methyl)benzoic acid

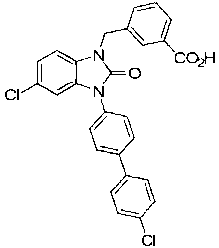

The process of the following steps A and B gave the title compound.

### Step A: Preparation of 3-((5-chloro-3-(4'-chloro-[1,1'-biphenyl]-4-yl)-2-oxo-2,3-dihydro-1H-benzo[d]imidazol-1-yl)methyl)benzoic acid methyl ester

The title compound (66 mg, yield: 42%) was obtained in the same manner as in step A of Example 2 by using 3-((5-chloro-2-oxo-2,3-dihydro-1*H*-benzo[*d*]imidazol-1-yl)methyl)benzoic acid methyl ester (100 mg, 0.316 mmol) obtained in Preparation Example 1 and (4'-chloro-[1,1'-biphenyl]-4-yl)boronic acid (147 mg, 0.631 mmol).
MS [M+H] = 504 (M+1)
¹H NMR (400 MHz, CD₃OD) δ8.05 (s, 1H), 7.97 (d, 1H), 7.72 (d, 2H), 7.61 (d, 2H), 7.55 (t, 3H), 7.44 (t, 3H), 7.13 (d, 1H), 7.02 (dd, 1H), 6.82 (d, 1H), 5.16 (s, 2H), 3.91 (s, 3H)

### Step B: Preparation of 3-((5-chloro-3-(4'-chloro-[1,1'-biphenyl]-4-yl)-2-oxo-2,3-dihydro-1H-benzo[d]imidazol-1-yl)methyl)benzoic acid

The title compound (55 mg, yield: 86%) was obtained in the same manner as in step B of Example 2 by using 3-((5-chloro-3-(4'-chloro-[1,1'-biphenyl]-4-yl)-2-oxo-2,3-dihydro-1*H*-benzo[*d*]imidazol-1-yl)methyl)benzoic acid methyl ester (66 mg, 0.131 mmol) obtained in step A above.
MS [M+H] = 490 (M+1)
¹H NMR (400 MHz, DMSO-D6) δ7.95 (s, 1H), 7.84 (m, 3H), 7.75 (dd, 2H), 7.66 (dd, 2H), 7.59 (d, 1H), 7.53 (dd, 2H), 7.45 (t, 1H), 7.24 (d, 1H), 7.14 (dd, 1H), 7.08 (d, 1H), 5.19 (s, 2H)

### Example 8: Preparation of 3-((5-chloro-2-oxo-3-(p-tolyl)-2,3-dihydro-1H-benzo[d]imidazol-1-yl)methyl)benzoic acid

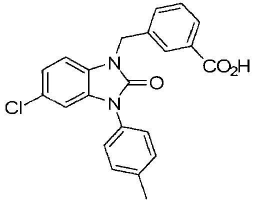

The process of the following steps A and B gave the title compound.

### Step A: Preparation of 3-((5-chloro-2-oxo-3-(p-tolyl)-2,3-dihydro-1H-benzo[d]imidazol-1-yl)methyl)benzoic acid methyl ester

The title compound (86 mg, yield: 67%) was obtained in the same manner as in step A of Example 2 by using 3-((5-chloro-2-oxo-2,3-dihydro-1*H*-benzo[*d*]imidazol-1-yl)methyl)benzoic acid methyl ester (100 mg, 0.316 mmol) obtained in Preparation Example 1 and p-tolylboronic acid (86 mg, 0.631 mmol).
MS [M+H] = 407 (M+1)
¹H NMR (400 MHz, CD₃OD) δδ8.07 (s, 1H), 8.00 (d, 1H), 7.58 (d, 1H), 7.44 (m, 3H), 7.37 (m, 2H), 7.06 (s, 1H), 7.03 (d, 1H), 6.82 (d, 1H), 5.18 (s, 2H), 3.94 (s, 3H), 2.46 (s, 3H)

### Step B: Preparation of 3-((5-chloro-2-oxo-3-(p-tolyl)-2,3-dihydro-1H-benzo[d]imidazol-1-yl)methyl)benzoic acid

The title compound (83 mg, yield: 100%) was obtained in the same manner as in step B of Example 2 by using 3-((5-chloro-2-oxo-3-(*p*-tolyl)-2,3-dihydro-1*H*-benzo[*d*]imidazol-1-yl)methyl)benzoic acid methyl ester (86 mg, 0.211 mmol) obtained in step A above.
MS [M+H] = 393 (M+1)
¹H NMR (500 MHz, DMSO-D6) δ7.98 (s, 1H), 7.87 (d, 1H), 7.63 (d, 1H), 7.50 (d, 1H), 7.46 (d, 2H), 7.40 (d, 2H), 7.26 (d, 1H), 7.16 (d, 1H), 6.98 (s, 1H), 5.21 (s, 2H), 2.42 (d, 3H)

### Example 9: Preparation of 3-((3-(benzo[d][1,3]dioxol-5-yl)-5-chloro-2-oxo-2,3-dihydro-1H-benzo[d]imidazol-1-yl)methyl)benzoic acid

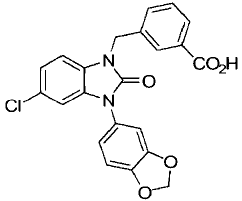

The process of the following steps A and B gave the title compound.

### Step A: Preparation of 3-((3-(benzo[d][1,3]dioxol-5-yl)-5-chloro-2-oxo-2,3-dihydro-1H-benzo[d]imidazol-1-yl)methyl)benzoic acid methyl ester

The title compound (58 mg, yield: 42%) was obtained in the same manner as in step A of Example 2 by using 3-((5-chloro-2-oxo-2,3-dihydro-1*H*-benzo[*d*]imidazol-1-yl)methyl)benzoic acid methyl ester (100 mg, 0.316 mmol) obtained in Preparation Example 1 and benzo[*d*][1,3]dioxol-5-ylboronic acid (105 mg, 0.631 mmol).
MS [M+H] = 437 (M+1)
¹H NMR (500 MHz, CD₃OD) δδ8.07 (s, 1H), 8.00 (d, 1H), 7.57 (d, 1H), 7.45 (t, 1H), 7.00 (m, 5H), 6.81 (d, 1H), 6.09 (s, 2H), 5.17 (s, 2H), 3.94 (s, 3H)

### Step B: Preparation of 3-((3-(benzo[d][1,3]dioxol-5-yl)-5-chloro-2-oxo-2,3-dihydro-1H-benzo[d]imidazol-1-yl)methyl)benzoic acid

The title compound (55 mg, yield: 99%) was obtained in the same manner as in step B of Example 2 by using 3-((3-(benzo[*d*][1,3]dioxol-5-yl)-5-chloro-2-oxo-2,3-dihydro-1*H-*benzo[d]imidazol-1-yl)methyl)benzoic acid methyl ester (58 mg, 0.132 mmol) obtained in step A above.
MS [M+H] = 423 (M+1)
¹H NMR (500 MHz, DMSO-D6) δ7.97 (d, 1H), 7.86 (m, 1H), 7.61 (m, 1H), 7.53 (m, 1H), 7.24 (d, 1H), 7.18 (s, 1H), 7.15 (d, 1H), 7.09 (m, 1H), 7.03 (d, 1H), 6.96 (d, 1H), 6.14 (s, 2H), 5.22 (s, 2H)

### Example 10: Preparation of 3-((5-chloro-3-(4-(dimethylamino)phenyl)-2-oxo-2,3-dihydro-1H-benzo[d]imidazol-1-yl)methyl)benzoic acid

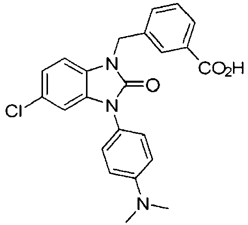

The process of the following steps A and B gave the title compound.

### Step A: Preparation of 3-((5-chloro-3-(4-(dimethylamino)phenyl)-2-oxo-2,3-dihydro-1H-benzo[d]imidazol-1-yl)methyl)benzoic acid methyl ester

The title compound (78 mg, yield: 57%) was obtained in the same manner as in step A of Example 2 by using 3-((5-chloro-2-oxo-2,3-dihydro-1*H*-benzo[*d*]imidazol-1-yl)methyl)benzoic acid methyl ester (100 mg, 0.316 mmol) obtained in Preparation Example 1 and (4-(dimethylamino)phenyl)boronic acid (104 mg, 0.631 mmol).
MS [M+H] = 436 (M+1)
¹H NMR (500 MHz, CD₃OD) δ8.07 (s, 1H), 7.99 (d, 1H), 7.58 (d, 1H), 7.44 (t, 1H), 7.35 (d, 2H), 7.00 (d, 2H), 6.85 (d, 2H), 6.79 (d, 1H), 5.17 (s, 2H), 3.94 (s, 3H), 3.04 (s, 6H)

### Step B: Preparation of 3-((5-chloro-3-(4-(dimethylamino)phenyl)-2-oxo-2,3-dihydro-1H-benzo[d]imidazol-1-yl)methyl)benzoic acid

The title compound (69 mg, yield: 92%) was obtained in the same manner as in step B of Example 2 by using 3-((5-chloro-3-(4-(dimethylamino)phenyl)-2-oxo-2,3-dihydro-1*H-*benzo[*d*]imidazol-1-yl)methyl)benzoic acid methyl ester (78 mg, 0.178 mmol) obtained in step A above.
MS [M+H] = 422 (M+1)
¹H NMR (500 MHz, DMSO-D6) δ7.96 (s, 1H), 7.87 (d, 1H), 7.61 (d, 1H), 7.49 (m, 1H), 7.33 (d, 2H), 7.23 (d, 1H), 7.12 (d, 1H), 6.87 (m, 3H), 5.20 (s, 2H), 2.96 (s, 6H)

### Example 11: Preparation of 3-((5-chloro-3-(4-isobutyramidophenyl)-2-oxo-2,3-dihydro-1H-benzo[d]imidazol-1-yl)methyl)benzoic acid

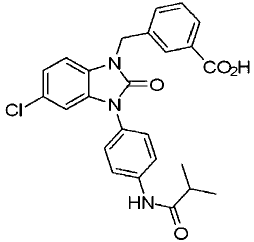

The process of the following steps A and B gave the title compound.

### Step A: Preparation of 3-((5-chloro-3-(4-isobutyramidophenyl)-2-oxo-2,3-dihydro-1H-benzo[d]imidazol-1-yl)methyl)benzoic acid methyl ester

The title compound (78 mg, yield: 52%) was obtained in the same manner as in step A of Example 2 by using 3-((5-chloro-2-oxo-2,3-dihydro-1*H*-benzo[*d*]imidazol-1-yl)methyl)benzoic acid methyl ester (100 mg, 0.316 mmol) obtained in Preparation Example 1 and 4-(isobutyramidophenyl)boronic acid (131 mg, 0.631 mmol).
MS [M+H] = 478 (M+1)
¹H NMR (500 MHz, CD₃OD) δ8.07 (s, 1H), 8.00 (d, 1H), 7.73 (d, 2H), 7.57 (d, 1H), 7.48 (m, 4H), 7.04 (d, 2H), 6.83 (d, 1H), 5.18 (s, 2H), 3.94 (s, 3H), 2.56 (m, 1H), 1.30 (d, 6H)

### Step B: Preparation of 3-((5-chloro-3-(4-isobutyramidophenyl)-2-oxo-2,3-dihydro-1H-benzo[d]imidazol-1-yl)methyl)benzoic acid

The title compound (51 mg, yield: 67%) was obtained in the same manner as in step B of Example 2 by using 3-((5-chloro-3-(4-isobutyramidophenyl)-2-oxo-2,3-dihydro-1*H-*benzo[*d*]imidazol-1-yl)methyl)benzoic acid methyl ester (78 mg, 0.163 mmol) obtained in step A above.
MS [M+H] = 464 (M+1)
¹H NMR (500 MHz, DMSO-D6) δ10.08 (s, 1H), 7.97 (s, 1H), 7.87 (d, 1H), 7.82 (d, 2H), 7.62 (d, 1H), 7.50 (d, 3H), 7.26 (d, 1H), 7.15 (d, 1H), 7.02 (d, 1H), 5.21 (s, 2H), 2.63 (td, 1H), 1.14 (d, 6H)

### Example 12: Preparation of 3-((3-(4-(tert-butyl)phenyl)-5-chloro-2-oxo-2,3-dihydro-1H-benzo[d]imidazol-1-yl)methyl)benzoic acid

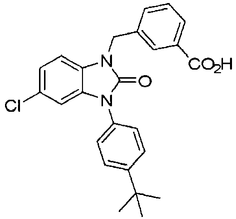

The process of the following steps A and B gave the title compound.

### Step A: Preparation of 3-((3-(4-(tert-butyl)phenyl)-5-chloro-2-oxo-2,3-dihydro-1H-benzo[d]imidazol-1-yl)methyl)benzoic acid methyl ester

The title compound (78 mg, yield: 92%) was obtained in the same manner as in step A of Example 2 by using 3-((5-chloro-2-oxo-2,3-dihydro-1*H*-benzo[*d*]imidazol-1-yl)methyl)benzoic acid methyl ester (60 mg, 0.189 mmol) obtained in Preparation Example 1 and (4-(*tert-*butyl)phenyl)boronic acid (68 mg, 0.379 mmol).
MS [M+H] = 449 (M+1)
¹H NMR (400 MHz, CD₃OD) δ8.04 (s, 1H), 7.96 (d, 1H), 7.54 (dd, 3H), 7.43 (m, 2H), 7.41 (m, 1H), 7.07 (s, 1H), 6.99 (d, 1H), 6.79 (d, 1H), 5.15 (s, 2H), 3.90 (s, 3H), 1.36 (s, 9H)

### Step B: Preparation of 3-((3-(4-(tert-butyl)phenyl)-5-chloro-2-oxo-2,3-dihydro-1H-benzo[d]imidazol-1-yl)methyl)benzoic acid

The title compound (66 mg, yield: 87%) was obtained in the same manner as in step B of Example 2 by using 3-((3-(4-(*tert*-butyl)phenyl)-5-chloro-2-oxo-2,3-dihydro-1*H-*benzo[*d*]imidazol-1-yl)methyl)benzoic acid methyl ester (78 mg, 0.174 mmol) obtained in step A above.
MS [M+H] = 435 (M+1)
¹H NMR (400 MHz, DMSO-D6) δ7.92 (s, 1H), 7.82 (d, 1H), 7.57 (m, 3H), 7.45 (m, 3H), 7.22 (d, 1H), 7.12 (dd, 1H), 6.98 (d, 1H), 5.17 (s, 2H), 1.31 (s, 9H)

### Example 13: Preparation of 3-((5-chloro-3-(4-hydroxyphenyl)-2-oxo-2,3-dihydro-1H-benzo[d]imidazol-1-yl)methyl)benzoic acid

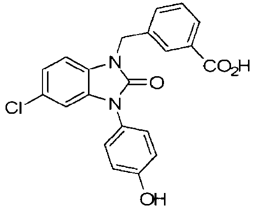

The process of the following steps A and B gave the title compound.

### Step A: Preparation of 3-((5-chloro-3-(4-hydroxyphenyl)-2-oxo-2,3-dihydro-1H-benzo[d]imidazol-1-yl)methyl)benzoic acid methyl ester

The title compound (131 mg, yield: 68%) was obtained in the same manner as in step A of Example 2 by using 3-((5-chloro-2-oxo-2,3-dihydro-1*H*-benzo[*d*]imidazol-1-yl)methyl)benzoic acid methyl ester (150 mg, 0.474 mmol) obtained in Preparation Example 1 and (4-hydroxyphenyl)boronic acid (131 mg, 0.947 mmol).
MS [M+H] = 409 (M+1)
¹H NMR (400 MHz, CD₃OD) δ8.05 (s, 1H), 7.97 (d, 1H), 7.55 (d, 1H), 7.43 (t, 1H), 7.25 (d, 1H), 7.23 (d, 1H), 7.01 (d, 1H), 6.94 (d, 1H), 6.85 (d, 2H), 6.81 (d, 1H), 6.68 (s, 1H), 5.16 (s, 2H), 3.91 (s, 3H)

### Step B: Preparation of 3-((5-chloro-3-(4-hydroxyphenyl)-2-oxo-2,3-dihydro-1H-benzo[d]imidazol-1-yl)methyl)benzoic acid

The title compound (40 mg, yield: 84%) was obtained in the same manner as in step B of Example 2 by using 3-((5-chloro-3-(4-hydroxyphenyl)-2-oxo-2,3-dihydro-1*H-*benzo[*d*]imidazol-1-yl)methyl)benzoic acid methyl ester (50 mg, 0.122 mmol) obtained in step A above.
MS [M+H] = 395 (M+1)
¹H NMR (400 MHz, DMSO-D6) δ9.81 (s, 1H), 7.92 (s, 1H), 7.82 (m, 1H), 7.57 (d, 1H), 7.44 (t, 1H), 7.30 (td, 2H), 7.19 (d, 1H), 7.09 (dd, 1H), 6.89 (td, 2H), 6.86 (d, 1H), 5.15 (s, 2H)

### Example 14: Preparation of 3-((5-chloro-3-(4-isopropoxyphenyl)-2-oxo-2,3-dihydro-1H-benzo[d]imidazol-1-yl)methyl)benzoic acid

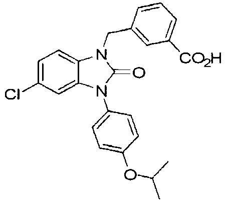

The process of the following steps A and B gave the title compound.

### Step A: Preparation of 3-((5-chloro-3-(4-isopropoxyphenyl)-2-oxo-2,3-dihydro-1H-benzo[d]imidazol-1-yl)methyl)benzoic acid methyl ester

The title compound (50 mg, yield: 70%) was obtained in the same manner as in step A of Example 2 by using 3-((5-chloro-2-oxo-2,3-dihydro-1*H*-benzo[*d*]imidazol-1-yl)methyl)benzoic acid methyl ester (50 mg, 0.158 mmol) obtained in Preparation Example 1 and (4-isopropoxyphenyl)boronic acid (56.8 mg, 0.316 mmol).
MS [M+H] = 451 (M+1)
¹H NMR (400 MHz, CD₃OD) δ8.03 (s, 1H), 7.96 (dd, 1H), 7.55 (d, 1H), 7.39 (m, 3H), 7.03 (s, 1H), 7.00 (m, 3H), 6.78 (d, 1H), 5.14 (s, 2H), 4.59 (m, 1H), 3.90 (s, 3H), 1.37 (d, 6H)

### Step B: Preparation of 3-((5-chloro-3-(4-isopropoxyphenyl)-2-oxo-2,3-dihydro-1H-benzo[d]imidazol-1-yl)methyl)benzoic acid

The title compound (40 mg, yield: 83%) was obtained in the same manner as in step B of Example 2 by using 3-((5-chloro-3-(4-isopropoxyphenyl)-2-oxo-2,3-dihydro-1*H-*benzo[*d*]imidazol-1-yl)methyl)benzoic acid methyl ester (50 mg, 0.111 mmol) obtained in step A above.
MS [M+H] = 437 (M+1)
¹H NMR (500 MHz, DMSO-D6) δ13.08 (s, 1H), 7.97 (s, 1H), 7.87 (d, 1H), 7.63 (d, 1H), 7.50 (d, 3H), 7.47 (d, 2H), 7.27 (d, 1H), 7.16 (d, 1H), 7.01 (s, 1H), 5.22 (s, 2H), 3.00 (m, 1H), 1.27 (d, 6H)

### Example 15: Preparation of 3-((3-([1,1'-biphenyl]-4-yl)-5-chloro-2-oxo-2,3-dihydro-1H-benzo[d]imidazol-1-yl)methyl)benzoic acid

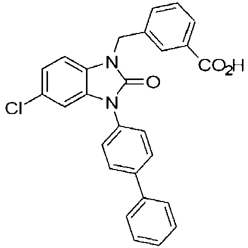

The process of the following steps A and B gave the title compound.

### Step A: Preparation of 3-((3-([1,1'-biphenyl]-4-yl)-5-chloro-2-oxo-2,3-dihydro-1H-benzo[d]imidazol-1-yl)methyl)benzoic acid methyl ester

The title compound (52 mg, yield: 70%) was obtained in the same manner as in step A of Example 2 by using 3-((5-chloro-2-oxo-2,3-dihydro-1*H*-benzo[*d*]imidazol-1-yl)methyl)benzoic acid methyl ester (50 mg, 0.158 mmol) obtained in Preparation Example 1 and [1,1'-biphenyl]-4-ylboronic acid (62.5 mg, 0.316 mmol).
MS [M+H] = 469 (M+1)
¹H NMR (400 MHz, CD₃OD) δ8.06 (s, 1H), 7.99 (d, 1H), 7.75 (d, 2H), 7.63 (m, 4H), 7.58 (d, 1H), 7.47 (t, 2H), 7.43 (d, 1H), 7.38 (d, 1H), 7.13 (d, 1H), 7.02 (d, 1H), 6.81 (d, 1H), 5.17 (s, 2H), 3.91 (s, 3H)

### Step B: Preparation of 3-((3-([1,1'-biphenyl]-4-yl)-5-chloro-2-oxo-2,3-dihydro-1H-benzo[d]imidazol-1-yl)methyl)benzoic acid

The title compound (42 mg, yield: 84%) was obtained in the same manner as in step B of Example 2 by using 3-((3-([1,1'-biphenyl]-4-yl)-5-chloro-2-oxo-2,3-dihydro-1*H-*benzo[*d*]imidazol-1-yl)methyl)benzoic acid methyl ester (52 mg, 0.110 mmol) obtained in step A above.
MS [M+H] = 455 (M+1)
¹H NMR (500 MHz, DMSO-D6) δ13.08 (s, 1H), 8.00 (s, 1H), 7.89 (m, 3H), 7.76 (d, 2H), 7.70 (d, 2H), 7.65 (d, 1H), 7.51 (m, 3H), 7.42 (t, 1H), 7.29 (d, 1H), 7.18 (d, 1H), 7.12 (s, 1H), 5.24 (s, 2H)

### Example 16: Preparation of 3-((3-(4-butylphenyl)-5-chloro-2-oxo-2,3-dihydro-1H-benzo[d]imidazol-1-yl)methyl)benzoic acid

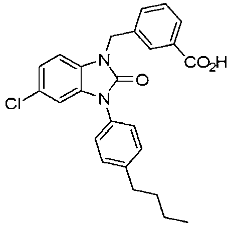

The process of the following steps A and B gave the title compound.

### Step A: Preparation of 3-((3-(4-butylphenyl)-5-chloro-2-oxo-2,3-dihydro-1H-benzo[d]imidazol-1-yl)methyl)benzoic acid methyl ester

The title compound (55 mg, yield: 77%) was obtained in the same manner as in step A of Example 2 by using 3-((5-chloro-2-oxo-2,3-dihydro-1*H*-benzo[*d*]imidazol-1-yl)methyl)benzoic acid methyl ester (50 mg, 0.158 mmol) obtained in Preparation Example 1 and (4-butylphenyl)boronic acid (56.2 mg, 0.316 mmol).
MS [M+H] = 449 (M+1)
¹H NMR (500 MHz, CD₃OD) δ8.07 (s, 1H), 8.00 (d, 1H), 7.58 (d, 1H), 7.45 (m, 3H), 7.37 (d, 2H), 7.08 (s, 1H), 7.03 (d, 1H), 6.82 (d, 1H), 5.18 (s, 2H), 3.94 (s, 3H), 2.71(t, 2H), 1.67 (m, 2H), 1.43 (m, 2H), 0.98 (t, 3H)

### Step B: Preparation of 3-((3-(4-butylphenyl)-5-chloro-2-oxo-2,3-dihydro-1H-benzo[d]imidazol-1-yl)methyl)benzoic acid

The title compound (46 mg, yield: 87%) was obtained in the same manner as in step B of Example 2 by using 3-((3-(4-butylphenyl)-5-chloro-2-oxo-2,3-dihydro-1*H*-benzo[*d*]imidazol-1-yl)methyl)benzoic acid methyl ester (54.6 mg, 0.122 mmol) obtained in step A above.
MS [M+H] = 435 (M+1)
¹H NMR (500 MHz, DMSO-D6) 513.07 (s, 1H), 7.98 (s, 1H), 7.87 (d, 1H), 7.63 (d, 1H), 7.49 (t, 3H), 7.41 (d, 2H), 7.27 (d, 1H), 7.16 (d, 1H), 7.00 (s, 1H), 5.22 (s, 2H), 2.67 (t, 2H), 1.62 (m, 2H), 1.36 (td, 2H), 0.93 (t, 3H)

### Example 17: Preparation of 3-((5-chloro-3-(4-isopropylphenyl)-2-oxo-2,3-dihydro-1H-benzo[d]imidazol-1-yl)methyl)benzoic acid

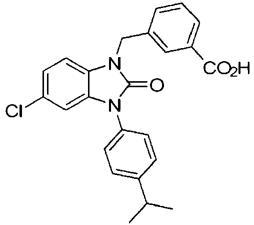

The process of the following steps A and B gave the title compound.

### Step A: Preparation of 3-((5-chloro-3-(4-isopropylphenyl)-2-oxo-2,3-dihydro-1H-benzo[d]imidazol-1-yl)methyl)benzoic acid methyl ester

The title compound (55 mg, yield: 80%) was obtained in the same manner as in step A of Example 2 by using 3-((5-chloro-2-oxo-2,3-dihydro-1*H*-benzo[*d*]imidazol-1-yl)methyl)benzoic acid methyl ester (50 mg, 0.158 mmol) obtained in Preparation Example 1 and (4-isopropylphenyl)boronic acid (51.8 mg, 0.316 mmol).
MS [M+H] = 435 (M+1)
¹H NMR (500 MHz, CD₃OD) δ8.07 (s, 1H), 8.00 (d, 1H), 7.59 (d, 1H), 7.46 (m, 3H), 7.42 (m, 2H), 7.09 (s, 1H), 7.03 (d, 1H), 6.82 (d, 1H), 5.18 (s, 2H), 3.94 (s, 3H), 3.01 (m, 1H), 1.33 (d, 6H)

### Step B: Preparation of 3-((5-chloro-3-(4-isopropylphenyl)-2-oxo-2,3-dihydro-1H-benzo[d]imidazol-1-yl)methyl)benzoic acid

The title compound (49 mg, yield: 92%) was obtained in the same manner as in step B of Example 2 by using 3-((5-chloro-3-(4-isopropylphenyl)-2-oxo-2,3-dihydro-1*H-*benzo[*d*]imidazol-1-yl)methyl)benzoic acid methyl ester (55 mg, 0.126 mmol) obtained in step A above.
MS [M+H] = 421 (M+1)
¹H NMR (500 MHz, DMSO-D6) δ13.08 (s, 1H), 7.97 (s, 1H), 7.87 (d, 1H), 7.63 (d, 1H), 7.50 (d, 3H), 7.47 (d, 2H), 7.27 (d, 1H), 7.16 (d, 1H), 7.01 (s, 1H), 5.22 (s, 2H), 3.00 (m, 1H), 1.27 (d, 6H)

### Example 18: Preparation of 3-((5-chloro-3-(4-(2-hydroxypropan-2-yl)phenyl)-2-oxo-2,3-dihydro-1H-benzo[d]imidazol-1-yl)methyl)benzoic acid

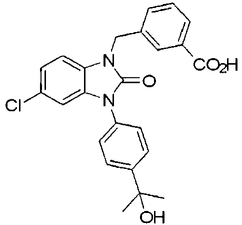

The process of the following steps A and B gave the title compound.

### Step A: Preparation of 3-((5-chloro-3-(4-(2-hydroxypropan-2-yl)phenyl)-2-oxo-2,3-dihydro-1H-benzo[d]imidazol-1-yl)methyl)benzoic acid methyl ester

The title compound (52 mg, yield: 73%) was obtained in the same manner as in step A of Example 2 by using 3-((5-chloro-2-oxo-2,3-dihydro-1*H*-benzo[*d*]imidazol-1-yl)methyl)benzoic acid methyl ester (50 mg, 0.158 mmol) obtained in Preparation Example 1 and (4-(2-hydroxypropan-2-yl)phenyl)boronic acid (56.8 mg, 0.316 mmol).
MS [M+H] = 451 (M+1)
¹H NMR (500 MHz, CD₃OD) δ8.08 (s, 1H), 8.00 (d, 1H), 7.70 (d, 2H), 7.59 (d, 1H), 7.53 (d, 2H), 7.45 (t, 1H), 7.10 (s, 1H), 7.06 (d, 1H), 6.83 (d, 1H), 5.18 (s, 2H), 3.93 (s, 3H), 1.66 (s, 6H)

### Step B: Preparation of 3-((5-chloro-3-(4-(2-hydroxypropan-2-yl)phenyl)-2-oxo-2,3-dihydro-1H-benzo[d]imidazol-1-yl)methyl)benzoic acid

The title compound (27 mg, yield: 54%) was obtained in the same manner as in step B of Example 2 by using 3-((5-chloro-3-(4-(2-hydroxypropan-2-yl)phenyl)-2-oxo-2,3-dihydro-1*H*-benzo[*d*]imidazol-1-yl)methyl)benzoic acid methyl ester (52 mg, 0.115 mmol) obtained in step A above.
MS [M+H] = 437 (M+1)
¹H NMR (500 MHz, DMSO-D6) δ7.95 (s, 1H), 7.85 (d, 1H), 7.68 (d, 2H), 7.57 (d, 1H), 7.51 (d, 2H), 7.46 (t, 1H), 7.26 (d, 1H), 7.16 (d, 1H), 7.01 (s, 1H), 5.20 (s, 2H), 1.49 (s, 6H)

### Example 19: Preparation of 3-((5-chloro-3-(4-(methylsulfonamido)phenyl)-2-oxo-2,3-dihydro-1H-benzo[d]imidazol-1-yl)methyl)benzoic acid

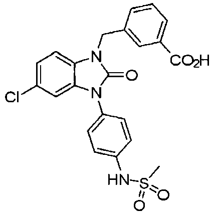

The process of the following steps A and B gave the title compound.

### Step A: Preparation of 3-((5-chloro-3-(4-(methylsulfonamido)phenyl)-2-oxo-2,3-dihydro-1H-benzo[d]imidazol-1-yl)methyl)benzoic acid methyl ester

The title compound (25 mg, yield: 32%) was obtained in the same manner as in step A of Example 2 by using 3-((5-chloro-2-oxo-2,3-dihydro-1*H*-benzo[*d*]imidazol-1-yl)methyl)benzoic acid methyl ester (50 mg, 0.158 mmol) obtained in Preparation Example 1 and (4-(methylsulfonamido)phenyl)boronic acid (67.9 mg, 0.316 mmol).
MS [M+H] = 486 (M+1)
¹H NMR (400 MHz, DMSO-D6) δ9.98 (s, 1H), 7.96 (s, 1H), 7.84 (d, 1H), 7.61 (d, 1H), 7.51 (m, 2H), 7.47 (d, 1H), 7.34 (d, 2H), 7.21 (d, 1H), 7.12 (d, 1H), 6.99 (s, 1H), 5.17 (s, 2H), 3.8 (s, 3H), 3.04 (s, 3H)

### Step B: Preparation of 3-((5-chloro-3-(4-(methylsulfonamido)phenyl)-2-oxo-2,3-dihydro-1H-benzo[d]imidazol-1-yl)methyl)benzoic acid

The title compound (8 mg, yield: 33%) was obtained in the same manner as in step B of Example 2 by using 3-((5-chloro-3-(4-(methylsulfonamido)phenyl)-2-oxo-2,3-dihydro-1*H-*benzo[*d*]imidazol-1-yl)methyl)benzoic acid methyl ester (24 mg, 0.049 mmol) obtained in step A above.
MS [M+H] = 472 (M+1)
¹H NMR (500 MHz, DMSO-D6) δ7.94 (s, 1H), 7.84 (d, 1H), 7.59 (m, 2H), 7.50 (d, 1H), 7.41 (m, 3H), 7.25 (d, 1H), 7.17 (d, 1H), 7.05 (s, 1H), 5.19 (s, 2H), 3.09 (s, 3H)

### Example 20: Preparation of 3-((5-chloro-3-(4-ethoxyphenyl)-2-oxo-2,3-dihydro-1H-benzo[d]imidazol-1-yl)methyl)benzoic acid

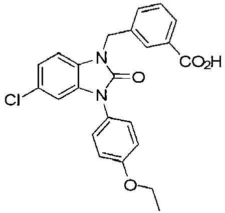

The process of the following steps A and B gave the title compound.

### Step A: Preparation of 3-((5-chloro-3-(4-ethoxyphenyl)-2-oxo-2,3-dihydro-1H-benzo[d]imidazol-1-yl)methyl)benzoic acid methyl ester

The title compound (49 mg, yield: 71%) was obtained in the same manner as in step A of Example 2 by using 3-((5-chloro-2-oxo-2,3-dihydro-1*H*-benzo[*d*]imidazol-1-yl)methyl)benzoic acid methyl ester (50 mg, 0.158 mmol) obtained in Preparation Example 1 and (4-ethoxyphenyl)boronic acid (52.4 mg, 0.316 mmol).
MS [M+H] = 437 (M+1)
¹H NMR (500 MHz, CD₃OD) δ8.07 (s, 1H), 8.00 (d, 1H), 7.58 (d, 1H), 7.43 (d, 3H), 7.07 (d, 2H), 7.01 (m, 2H), 6.81 (d, 1H), 5.17 (s, 2H), 4.12 (q, 2H), 3.94 (s, 3H), 1.48 (t, 3H)

### Step B: Preparation of 3-((5-chloro-3-(4-ethoxyphenyl)-2-oxo-2,3-dihydro-1H-benzo[d]imidazol-1-yl)methyl)benzoic acid

The title compound (41 mg, yield: 86%) was obtained in the same manner as in step B of Example 2 by using 3-((5-chloro-3-(4-ethoxyphenyl)-2-oxo-2,3-dihydro-1*H-*benzo[*d*]imidazol-1-yl)methyl)benzoic acid methyl ester (49 mg, 0.112 mmol) obtained in step A above.
MS [M+H] = 423 (M+1)
¹H NMR (500 MHz, DMSO-D6) δ13.08 (s, 1H), 7.98 (s, 1H), 7.87 (d, 1H), 7.62 (d, 1H), 7.49 (dd, 3H), 7.25 (d, 1H), 7.13 (m, 3H), 6.93 (s, 1H), 5.21 (s, 2H), 4.11 (q, 2H), 1.38 (t, 3H)

### Example 21: Preparation of 3-((5-chloro-3-(4-(dimethylcarbamoyl)phenyl)-2-oxo-2,3-dihydro-1H-benzo[d]imidazol-1-yl)methyl)benzoic acid

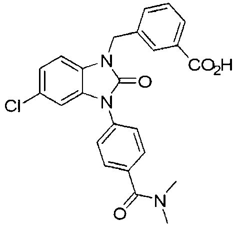

The process of the following steps A and B gave the title compound.

### Step A: Preparation of 3-((5-chloro-3-(4-(dimethylcarbamoyl)phenyl)-2-oxo-2,3-dihydro-1H-benzo[d]imidazol-1-yl)methyl)benzoic acid methyl ester

The title compound (52 mg, yield: 71%) was obtained in the same manner as in step A of Example 2 by using 3-((5-chloro-2-oxo-2,3-dihydro-1*H*-benzo[*d*]imidazol-1-yl)methyl)benzoic acid methyl ester (50 mg, 0.158 mmol) obtained in Preparation Example 1 and (4-(dimethylcarbamoyl)phenyl)boronic acid (60.9 mg, 0.316 mmol).
MS [M+H] = 464 (M+1)
¹H NMR (500 MHz, CD₃OD) δ8.08 (s, 1H), 8.01 (d, 1H), 7.64 (m, 4H), 7.59 (d, 1H), 7.46 (t, 1H), 7.14 (s, 1H), 7.07 (d, 1H), 6.86 (d, 1H), 5.19 (s, 2H), 3.94 (s, 3H), 3.18 (s, 3H), 3.08 (s, 4H)

### Step B: Preparation of 3-((5-chloro-3-(4-(dimethylcarbamoyl)phenyl)-2-oxo-2,3-dihydro-1H-benzo[d]imidazol-1-yl)methyl)benzoic acid

The title compound (41 mg, yield: 83%) was obtained in the same manner as in step B of Example 2 by using 3-((5-chloro-3-(4-(dimethylcarbamoyl)phenyl)-2-oxo-2,3-dihydro-1*H-*benzo[*d*]imidazol-1-yl)methyl)benzoic acid methyl ester (51 mg, 0.110 mmol) obtained in step A above.
MS [M+H] = 450 (M+1)
¹H NMR (500 MHz, DMSO-D6) δ13.08 (s, 1H), 7.99 (s, 1H), 7.87 (d, 1H), 7.64 (m, 5H), 7.50 (t, 1H), 7.29 (d, 1H), 7.19 (d, 1H), 7.16 (s, 1H), 5.23 (s, 2H), 3.01 (d, 6H)

### Example 22: Preparation of 3-((5-chloro-3-(4-(methylcarbamoyl)phenyl)-2-oxo-2,3-dihydro-1H-benzo[d]imidazol-1-yl)methyl)benzoic acid

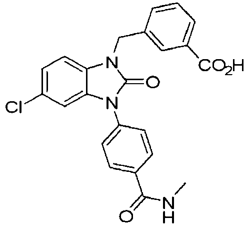

The process of the following steps A and B gave the title compound.

### Step A: Preparation of 3-((5-chloro-3-(4-(methylcarbamoyl)phenyl)-2-oxo-2,3-dihydro-1H-benzo[d]imidazol-1-yl)methyl)benzoic acid methyl ester

The title compound (55 mg, yield: 77%) was obtained in the same manner as in step A of Example 2 by using 3-((5-chloro-2-oxo-2,3-dihydro-1*H*-benzo[*d*]imidazol-1-yl)methyl)benzoic acid methyl ester (50 mg, 0.158 mmol) obtained in Preparation Example 1 and (4-(methylcarbamoyl)phenyl)boronic acid (56.5 mg, 0.316 mmol).
MS [M+H] = 450 (M+1)
¹H NMR (500 MHz, CD₃OD) δ8.07 (s, 1H), 8.01 (d, 1H), 7.98 (d, 2H), 7.68 (d, 2H), 7.58 (d, 1H), 7.46 (t, 1H), 7.15 (s, 1H), 7.08 (d, 1H), 6.86 (d, 1H), 6.23 (d, 1H), 5.19 (s, 2H), 3.94 (s, 3H), 3.08 (d, 3H)

### Step B: Preparation of 3-((5-chloro-3-(4-(methylcarbamoyl)phenyl)-2-oxo-2,3-dihydro-1H-benzo[d]imidazol-1-yl)methyl)benzoic acid

The title compound (46 mg, yield: 85%) was obtained in the same manner as in step B of Example 2 by using 3-((5-chloro-3-(4-(methylcarbamoyl)phenyl)-2-oxo-2,3-dihydro-1*H-*benzo[*d*]imidazol-1-yl)methyl)benzoic acid methyl ester (55 mg, 0.122 mmol) obtained in step A above.
MS [M+H] = 436 (M+1)
¹H NMR (500 MHz, DMSO-D6) δ13.07 (s, 1H), 8.59 (s, 1H), 8.03 (d, 2H), 7.99 (s, 1H), 7.87 (d, 1H), 7.72 (d, 2H), 7.64 (d, 1H), 7.50 (t, 1H), 7.29 (d, 1H), 7.19 (d, 1H), 7.14 (s, 1H), 5.23 (s, 2H), 2.83 (d, 3H)

### Example 23: Preparation of 3-((5-chloro-2-oxo-3-(4-((2,2,2-trifluoroethyl)carbamoyl)phenyl)-2,3-dihydro-1H-benzo[d]imidazol-1-yl)methyl)benzoic acid

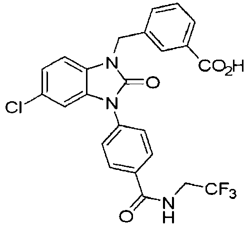

The process of the following steps A and B gave the title compound.

### Step A: Preparation of 3-((5-chloro-2-oxo-3-(4-((2,2,2-trifluoroethyl)carbamoyl)phenyl)-2,3-dihydro-1H-benzo[d]imidazol-1-yl)methyl)benzoic acid methyl ester

The title compound (75 mg, yield: 92%) was obtained in the same manner as in step A of Example 2 by using 3-((5-chloro-2-oxo-2,3-dihydro-1*H*-benzo[*d*]imidazol-1-yl)methyl)benzoic acid methyl ester (50 mg, 0.158 mmol) obtained in Preparation Example 1 and (4-((2,2,2-trifluoroethyl)carbamoyl)phenyl)boronic acid (78 mg, 0.316 mmol).
MS [M+H] = 518 (M+1)
¹H NMR (500 MHz, CD₃OD) δ8.09 (s, 1H), 8.03 (m, 3H), 7.73 (m, 2H), 7.59 (d, 1H), 7.48 (t, 1H), 7.16 (s, 1H), 7.09 (d, 1H), 6.88 (t, 1H), 6.55 (s, 1H), 5.20 (s, 2H), 4.19 (m, 2H), 3.92 (s, 3H)

### Step B: Preparation of 3-((5-chloro-2-oxo-3-(4-((2,2,2-trifluoroethyl)carbamoyl)phenyl)-2,3-dihydro-1H-benzo[d]imidazol-1-yl)methyl)benzoic acid

The title compound (44 mg, yield: 81%) was obtained in the same manner as in step B of Example 2 by using 3-((5-chloro-2-oxo-3-(4-((2,2,2-trifluoroethyl)carbamoyl)phenyl)-2,3-dihydro-1*H*-benzo[*d*]imidazol-1-yl)methyl)benzoic acid methyl ester (55 mg, 0.106 mmol) obtained in step A above.
MS [M+H] = 504 (M+1)
¹H NMR (500 MHz, DMSO-D6) δ9.27 (t, 1H), 8.10 (d, 2H), 7.96 (s, 1H), 7.85 (d, 1H), 7.75 (d, 2H), 7.55 (d, 1H), 7.43 (t, 1H), 7.27 (d, 1H), 7.18 (m, 2H), 5.21 (s, 2H), 4.14 (m, 2H)

### Example 24: Preparation of 3-((5-chloro-4-fluoro-3-(4-morpholinophenyl)-2-oxo-2,3-dihydro-1H-benzo[d]imidazol-1-yl)methyl)benzoic acid

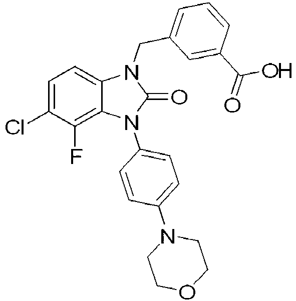

The process of the following steps A and B gave the title compound.

### Step A: Preparation of 3-((5-chloro-4-fluoro-3-(4-morpholinophenyl)-2-oxo-2,3-dihydro-1H-benzo[d]imidazol-1-yl)methyl)benzoic acid methyl ester

6-Chloro-7-fluoro-1-(4-morpholinophenyl)-1,3-dihydro-2*H*-benzo[*d*]imidazol-2-one (0.99 g, 2.85 mmol) obtained in Preparation Example 11 and 3-(bromomethyl)benzoic acid methyl ester (0.65 g, 2.85 mmol) were dissolved in 15 mL of AN, and then K₂CO₃ (0.78 g, 5.68 mmol) was added thereto, followed by stirring at 70 °C for 16 hours. After the reaction was terminated, the resulting solid was filtered, and the filtrate was purified by MPLC to give the title compound (0.90 g, yield: 64%).

¹H-NMR (400 MHz, DMSO-d6) δ8.01 (d, 1H), 7.89 (dd, 1H), 7.65 (dd, 1H), 7.53 (t, 1H), 7.39 (d, 2H), 7.22 (dd, 1H), 7.10 (d, 1H), 7.05 (d, 2H), 5.21 (s, 2H), 3.85 (s, 3H), 3.77 (m, 4H), 3.19 (m, 4H)

### Step B: Preparation of 3-((5-chloro-4-fluoro-3-(4-morpholinophenyl)-2-oxo-2,3-dihydro-1H-benzo[d]imidazol-1-yl)methyl)benzoic acid

To 3-((5-Chloro-4-fluoro-3-(4-morpholinophenyl)-2-oxo-2,3-dihydro-1*H*-benzo[*d*]imidazol-1-yl)methyl)benzoic acid methyl ester (0.90 g, 1.8 mmol) obtained in step A above were added 30 mL of EtOH, 10 mL of THF, and 5.4 mL of an aqueous 1 N NaOH solution, and the resulting mixture was stirred at room temperature for 16 hours. After the reaction was terminated, the reaction solution was concentrated under reduced pressure and adjusted to pH 3 with a 1 N aqueous hydrochloric acid solution. The precipitated solid was filtered and then dried to give the title compound (0.84 g, yield: 96%).
MS [M+H] = 482 (M+1)
¹H-NMR (400 MHz, DMSO-d6) δ7.94 (d, 1H), 7.84 (dd, 1H), 7.55 (dd, 1H), 7.40 (t, 1H), 7.37 (d, 2H), 7.24 (dd, 1H), 7.08 (d, 1H), 7.05 (d, 2H), 5.18 (s, 2H), 3.77 (m, 4H), 3.19 (m, 4H)

### Example 25: Preparation of 3-((5-chloro-3-(4-(dimethylamino)phenyl)-4-fluoro-2-oxo-2,3-dihydro-1H-benzo[d]imidazol-1-yl)methyl)benzoic acid

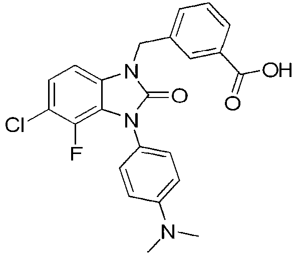

The process of the following steps A and B gave the title compound.

### Step A: Preparation of 3-((5-chloro-3-(4-(dimethylamino)phenyl)-4-fluoro-2-oxo-2,3-dihydro-1H-benzo[d]imidazol-1-yl)methyl)benzoic acid methyl ester

The title compound (3.93 g, yield: 81%) was obtained in the same manner as in step A of Example 24 by using 6-chloro-1-(4-(dimethylamino)phenyl)-7-fluoro-1,3-dihydro-2*H-*benzo[*d*]imidazol-2-one (3.25 g, 10.6 mmol) obtained in Preparation Example 8 and 3-(bromomethyl)benzoic acid methyl ester (2.43 g, 10.6 mmol).
¹H-NMR (400 MHz, DMSO-d6) δ8.00 (d, 1H), 7.89 (dd, 1H), 7.63 (dd, 1H), 7.52 (t, 1H), 7.33 (d, 2H), 7.24 (dd, 1H), 7.10 (d, 1H), 6.80 (d, 2H), 5.21 (s, 2H), 3.85 (s, 3H), 2.97 (s, 6H)

### Step B: Preparation of 3-((5-chloro-3-(4-(dimethylamino)phenyl)-4-fluoro-2-oxo-2,3-dihydro-1H-benzo[d]imidazol-1-yl)methyl)benzoic acid

The title compound (2.26 g, yield: 64%) was obtained in the same manner as in step B of Example 24 by using 3-((5-chloro-3-(4-(dimethylamino)phenyl)-4-fluoro-2-oxo-2,3-dihydro-1*H*-benzo[*d*]imidazol-1-yl)methyl)benzoic acid methyl ester (3.62 g, 7.98 mmol) obtained in step A above.
MS [M+H] = 440 (M+1)
¹H-NMR (400 MHz, DMSO-d6) δ7.97 (d, 1H), 7.86 (dd, 1H), 7.50 (dd, 1H), 7.48 (t, 1H), 7.31 (d, 2H), 7.22 (dd, 1H), 7.20 (d, 1H), 6.80 (d, 2H), 5.20 (s, 2H), 2.97 (s, 6H)

### Example 26: Preparation of 3-((5-chloro-2-oxo-3-(4-((2,2,2-trifluoroethyl)sulfonamido)phenyl)-2,3-dihydro-1H-benzo[d]imidazol-1-yl)methyl)benzoic acid

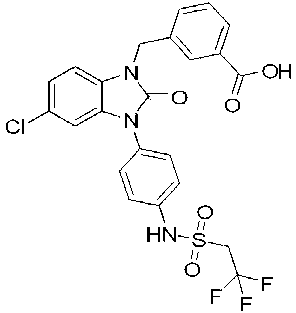

The process of the following steps A, B, C and D gave the title compound.

### Step A: Preparation of 3-((5-chloro-3-(4-nitrophenyl)-2-oxo-2,3-dihydro-1H-benzo[d]imidazol-1-yl)methyl)benzoic acid methyl ester

The title compound (0.22 g, yield: 99%) was obtained in the same manner as in step A of Example 2 by using 3-((5-chloro-2-oxo-2,3-dihydro-1*H*-benzo[*d*]imidazol-1-yl)methyl)benzoic acid methyl ester (0.157 g, 0.496 mmol) obtained in Preparation Example 1 and (4-nitrophenyl)boronic acid (0.165 g, 0.991 mmol).
¹H-NMR (400 MHz, CDCl3) δ8.43 (d, 2H), 8.05 (d, 1H), 8.00 (dd, 1H), 7.84 (d, 2H), 7.56 (dd, 1H), 7.46 (t, 1H), 7.20 (d, 1H), 7.10 (dd, 1H), 6.87 (d, 1H), 5.17 (s, 2H), 3.92 (s, 3H)

### Step B: Preparation of 3-((3-(4-aminophenyl)-5-chloro-2-oxo-2,3-dihydro-1H-benzo[d]imidazol-1-yl)methyl)benzoic acid methyl ester

The title compound (0.19 g, yield: 95%) was obtained in the same manner as in step B of Preparation Example 7 by using 3-((5-chloro-3-(4-nitrophenyl)-2-oxo-2,3-dihydro-1*H-*benzo[*d*]imidazol-1-yl)methyl)benzoic acid methyl ester (0.22 g, 0.50 mmol) obtained in step A above.
¹H-NMR (400 MHz, CDCl3) δ8.05 (d, 1H), 7.97 (dd, 1H), 7.55 (dd, 1H), 7.42 (t, 1H), 7.26 (d, 2H), 7.00 (2H), 6.81 (d, 2H), 6.77 (d, 1H), 5.15 (s, 2H), 3.92 (s, 3H), 3.85 (brs, 2H)

### Step C: Preparation of 3-((5-chloro-2-oxo-3-(4-((2,2,2-trifluoroethyl)sulfonamido)phenyl)-2,3-dihydro-1H-benzo[d]imidazol-1-yl)methyl)benzoic acid methyl ester

3-((3-(4-aminophenyl)-5-chloro-2-oxo-2,3-dihydro-1*H-*benzo[*d*]imidazol-1-yl)methyl)benzoic acid methyl ester (0.086 g, 0.21 mmol) obtained in Step B above was dissolved in 2 mL of DCM, then cooled to 0 °C, and 2,2,2-trifluoroethane-1-sulfonyl chloride (0.046 g, 0.25 mmol) and DIPEA (0.037 mL, 0.21 mmol) were sequentially added thereto, followed by stirring for 1 hour. After the reaction was terminated, water and DCM were added thereto and then an organic layer was separated. The organic layer was dried and then purified by MPLC to give the title compound (0.01 g, yield: 9%).
¹H-NMR (400 MHz, CDCl3) δ8.16 (d, 1H), 8.00 (dd, 1H), 7.57 (3H), 7.44 (3H), 7.07 (2H), 7.00 (dd, 1H), 5.17 (s, 2H), 3.92 (s, 3H), 3.86 (q, 2H)

### Step D: Preparation of 3-((5-chloro-2-oxo-3-(4-((2,2,2-trifluoroethyl)sulfonamido)phenyl)-2,3-dihydro-1H-benzo[d]imidazol-1-yl)methyl)benzoic acid

The title compound (0.007 g, yield: 77%) was obtained in the same manner as in step B of Example 24 by using 3-((5-chloro-2-oxo-3-(4-((2,2,2-trifluoroethyl)sulfonamido)phenyl)-2,3-dihydro-1*H*-benzo[*d*]imidazol-1-yl)methyl)benzoic acid methyl ester (0.01 g, 0.018 mmol) obtained in step C above.
¹H-NMR (400 MHz, CDCl3) δ8.17 (d, 1H), 8.03 (dd, 1H), 7.61 (2H), 7.45 (3H), 7.08 (2H), 6.85 (dd, 1H), 5.18 (s, 2H), 4.13 (q, 3H)

### Example 27: Preparation of 3-((5-chloro-3-(4-(cyclobutylmethoxy)phenyl)-2-oxo-2,3-dihydro-1H-benzo[d]imidazol-1-yl)methyl)benzoic acid

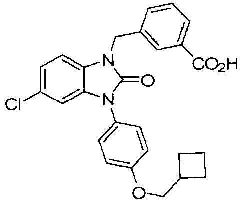

The process of the following steps A and B gave the title compound.

### Step A: Preparation of 3-((5-chloro-3-(4-(cyclobutylmethoxy)phenyl)-2-oxo-2,3-dihydro-1H-benzo[d]imidazol-1-yl)methyl)benzoic acid methyl ester

To 3-((5-chloro-3-(4-hydroxyphenyl)-2-oxo-2,3-dihydro-1*H*-benzo[*d*]imidazol-1-yl)methyl)benzoic acid methyl ester (50 mg, 0.122 mmol) obtained in step A of Example 13 was added 2 mL of DMF, then (bromomethyl)cyclobutane (36.5 mg, 0.245 mmol) and K₂CO₃ (33.8 mg, 0.245 mmol) were added thereto, and the resulting mixture was stirred at 80 °C for 16 hours. The resulting solid was filtered and then the filtrate was purified by MPLC to give the title compound (41 mg, yield: 70%) .
MS [M+H] = 477 (M+1)
¹H NMR (500 MHz, CD₃OD) δ8.07 (s, 1H), 8.00 (d, 1H), 7.58 (d, 1H), 7.44 (m, 3H), 7.08 (d, 2H), 7.02 (m, 2H), 6.81 (d, 1H), 5.18 (s, 2H), 4.01 (d, 2H), 3.94 (s, 3H), 2.84 (m, 1H), 2.21 (m, 2H), 1.99 (m, 4H)

### Step B: Preparation of 3-((5-chloro-3-(4-(cyclobutylmethoxy)phenyl)-2-oxo-2,3-dihydro-1H-benzo[d]imidazol-1-yl)methyl)benzoic acid

The title compound (35 mg, yield: 88%) was obtained in the same manner as in step B of Example 2 by using 3-((5-chloro-3-(4-(cyclobutylmethoxy)phenyl)-2-oxo-2,3-dihydro-1*H-*benzo[d]imidazol-1-yl)methyl)benzoic acid methyl ester (41 mg, 0.086 mmol) obtained in step A above.
MS [M+H] = 463 (M+1)
¹H NMR (500 MHz, DMSO-D6) δ13.02 (s, 1H), 7.98 (s, 1H), 7.87 (d, 1H), 7.63 (d, 1H), 7.47 (m, 3H), 7.25 (d, 1H), 7.12 (m, 3H), 6.93 (d, 1H), 5.21 (s, 2H), 4.03 (d, 2H), 2.76 (m, 1H), 2.13 (m, 2H), 1.92 (m, 4H)

### Example 28: Preparation of 3-((5-chloro-2-oxo-3-(4-(2-oxopiperidin-1-yl)phenyl)-2,3-dihydro-1H-benzo[d]imidazol-1-yl)methyl)benzoic acid

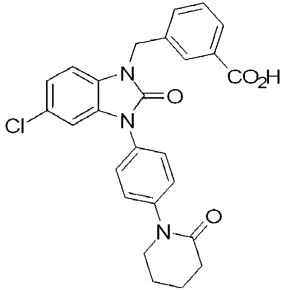

The process of the following steps A and B gave the title compound.

### Step A: Preparation of 3-((5-chloro-2-oxo-3-(4-(2-oxopiperidin-1-yl)phenyl)-2,3-dihydro-1H-benzo[d]imidazol-1-yl)methyl)benzoic acid methyl ester

The title compound (0.055 g, 32% yield) was obtained in the same manner as in step B of Preparation Example 8 and step A of Example 24 by using 1-(4-((5-chloro-2-nitrophenyl)amino)phenyl)piperidin-2-one (0.12 g, 0.35 mmol) obtained in Preparation Example 16.
¹H-NMR (400 MHz, CDCl3) δ8.05 (d, 1H), 7.98 (dd, 1H), 7.58 (d, 2H), 7.55 (dd, 1H), 7.47 (d, 2H), 7.42 (dd, 1H), 7.16 (d, 1H), 7.03 (dd, 1H), 6.81 (d, 1H), 5.16 (s, 2H), 3.92 (s, 2H), 3.73 (m, 2H), 2.611 (m, 2H), 1.99 (m, 4H)

### Step B: Preparation of 3-((5-chloro-2-oxo-3-(4-(2-oxopiperidin-1-yl)phenyl)-2,3-dihydro-1H-benzo[d]imidazol-1-yl)methyl)benzoic acid

The title compound (0.020 g, yield: 37%) was obtained in the same manner as in step B of Example 24 by using 3-((5-chloro-2-oxo-3-(4-(2-oxopiperidin-1-yl)phenyl)-2,3-dihydro-1H-benzo[d]imidazol-1-yl)methyl)benzoic acid methyl ester (0.055 g, 0.11 mmol) obtained in step A above.
MS [M+H] = 476 (M+1)
¹H-NMR (400 MHz, CDCl3) δ8.09 (d, 1H), 8.02 (dd, 1H), 7.59 (3H), 7.47 (d, 2H), 7.45 (t, 1H), 7.16 (d, 1H), 7.03 (dd, 1H), 6.82 (d, 1H), 5.16 (s, 2H), 3.71 (m, 2H), 2.63 (m, 2H), 1.99 (m, 4H)

### Example 29: Preparation of 3-((5-chloro-2-oxo-3-(4-(pyrrolidin-1-yl)phenyl)-2,3-dihydro-1H-benzo[d]imidazol-1-yl)methyl)benzoic acid

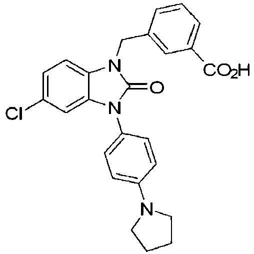

The process of the following steps A, B, and C gave the title compound.

### Step A: Preparation of 3-((5-chloro-2-oxo-3-(4-(((trifluoromethyl)sulfonyl)oxy)phenyl)-2,3-dihydro-1H-benzo[d]imidazol-1-yl)methyl)benzoic acid methyl ester

To 3-((5-chloro-3-(4-hydroxyphenyl)-2-oxo-2,3-dihydro-1*H*-benzo[*d*]imidazol-1-yl)methyl)benzoic acid methyl ester (70 mg, 0.171 mmol) obtained in step A of Example 13 was added 3 mL of toluene, then TEA (26.0 mg, 0.257 mmol) and trifluoromethanesulfonic anhydride (72.5 mg, 0.257 mmol) were added thereto at 0 °C, and the resulting mixture was stirred at room temperature for 2 hours. After the reaction was terminated, water was added thereto, and then an organic layer was separated and dried over Na₂SO₄. The resulting solid was filtered, and the filtrate was distilled under reduced pressure and then purified by MPLC to give the title compound (82 mg, yield: 89%).
MS [M+H] = 541 (M+1)

### Step B: Preparation of 3-((5-chloro-2-oxo-3-(4-(pyrrolidin-1-yl)phenyl)-2,3-dihydro-1H-benzo[d]imidazol-1-yl)methyl)benzoic acid methyl ester

To 3-((5-chloro-2-oxo-3-(4-(((trifluoromethyl)sulfonyl)oxy)phenyl)-2,3-dihydro-1*H-*benzo[d]imidazol-1-yl)methyl)benzoic acid methyl ester (80 mg, 0.148 mmol) obtained in step A was added 3 mL of toluene, and then pyrrolidine (12.6 mg, 0.177 mmol), Cs₂CO₃ (72.3 mg, 0.222 mmol), Xantphos (17.1 mg, 0.030 mmol), and Pd₂(dba)₃ (27.1 mg, 0.030 mmol) were added thereto, and the resulting mixture was stirred at 95 °C for 16 hours. After the reaction was terminated, a solid was filtered and the filtrate was purified by MPLC to give the title compound (39 mg, yield: 57%).
MS [M+H] = 462 (M+1)
¹H NMR (500 MHz, CD₃OD) δ8.07 (s, 1H), 7.99 (d, 1H), 7.58 (d, 1H), 7.45 (t, 1H), 7.32 (d, 2H), 6.98 (s, 2H), 6.78 (d, 1H), 6.68 (d, 2H), 5.17 (s, 2H), 3.94 (s, 3H), 3.36 (m, 4H), 2.07 (m, 4H)

### Step C: Preparation of 3-((5-chloro-2-oxo-3-(4-(pyrrolidin-1-yl)phenyl)-2,3-dihydro-1H-benzo[d]imidazol-1-yl)methyl)benzoic acid

The title compound (12 mg, yield: 33%) was obtained in the same manner as in step B of Example 2 by using 3-((5-chloro-2-oxo-3-(4-(pyrrolidin-1-yl)phenyl)-2,3-dihydro-1H-benzo[d]imidazol-1-yl)methyl)benzoic acid methyl ester (38 mg, 0.082 mmol) obtained in step B above.
MS [M+H] = 448 (M+1)
¹H NMR (500 MHz, DMSO-D6) δ7.96 (s, 1H), 7.87 (d, 1H), 7.62 (d, 1H), 7.50 (d, 1H), 7.30 (d, 2H), 7.23 (d, 1H), 7.12 (d, 1H), 6.84 (s, 1H), 6.68 (d, 2H), 5.20 (s, 2H), 3.30 (m, 4H), 2.00 (m, 4H)

### Example 30: Preparation of 3-((5-chloro-3-(4-(cyclobutylmethoxy)-3-fluorophenyl)-2-oxo-2,3-dihydro-1H-benzo[d]imidazol-1-yl)methyl)benzoic acid

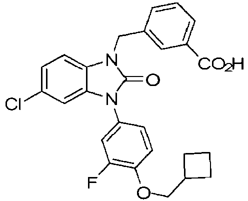

The process of the following steps A, B, and C gave the title compound.

### Step A: Preparation of 3-((5-chloro-3-(3-fluoro-4-hydroxyphenyl)-2-oxo-2,3-dihydro-1H-benzo[d]imidazol-1-yl)methyl)benzoic acid methyl ester

The title compound (77 mg, yield: 58%) was obtained in the same manner as in step A of Example 2 by using 3-((5-chloro-2-oxo-2,3-dihydro-1*H*-benzo[*d*]imidazol-1-yl)methyl)benzoic acid methyl ester (99 mg, 0.311 mmol) obtained in Preparation Example 1 and (3-fluoro-4-hydroxyphenyl)boronic acid (97 mg, 0.622 mmol).
MS [M+H] = 427 (M+1)
¹H NMR (500 MHz, CD₃OD) δ8.08 (s, 1H), 8.01 (d, 1H), 7.58 (d, 1H), 7.47 (t, 1H), 7.28 (d, 4H), 7.19 (d, 1H), 7.12 (t, 1H), 7.06 (d, 1H), 7.03 (s, 1H), 6.85 (d, 1H), 6.11 (s, 1H), 5.19 (s, 2H), 3.94 (d, 3H)

### Step B: Preparation of 3-((5-chloro-3-(4-(cyclobutylmethoxy)-3-fluorophenyl)-2-oxo-2,3-dihydro-1H-benzo[d]imidazol-1-yl)methyl)benzoic acid methyl ester

To 3-((5-chloro-3-(3-fluoro - 4-hydroxyphenyl)-2-oxo-2,3-dihydro-1*H*-benzo[*d*]imidazol-1-yl)methyl)benzoic acid methyl ester (40 mg, 0.094 mmol) obtained in step A above was added 2 mL of DMF, then (bromomethyl)cyclobutane (27.9 mg, 0.187 mmol) and K₂CO₃ (25.9 mg, 0.187 mmol) were added thereto, and the resulting mixture was stirred at 80 °C for 16 hours. The resulting solid was filtered and then the filtrate was purified by MPLC to give the title compound (39 mg, yield: 83%).
MS [M+H] = 495 (M+1)
¹H NMR (500 MHz, CD₃OD) δ8.03 (s, 1H), 7.97 (d, 1H), 7.55 (d, 1H), 7.41 (t, 1H), 7.30 (m, 1H), 7.24 (m, 1H), 7.21 (d, 1H), 7.09 (t, 1H), 7.01 (m, 2H), 6.79 (d, 1H), 5.13 (s, 2H), 4.05 (d, 2H), 3.90 (s, 3H), 2.84 (m, 1H), 2.17 (m, 2H), 1.93 (m, 4H)

### Step C: Preparation of 3-((5-chloro-3-(4-(cyclobutylmethoxy)-3-fluorophenyl)-2-oxo-2,3-dihydro-1H-benzo[d]imidazol-1-yl)methyl)benzoic acid

The title compound (32 mg, yield: 80%) was obtained in the same manner as in step B of Example 2 by using 3-((5-chloro-3-(4-(cyclobutylmethoxy)-3-fluorophenyl)-2-oxo-2,3-dihydro-1*H*-benzo[*d*]imidazol-1-yl)methyl)benzoic acid methyl ester (41 mg, 0.083 mmol) obtained in step B above.
MS [M+H] = 481 (M+1)
¹H NMR (500 MHz, DMSO-D6) 513.05 (s, 1H), 7.99 (s, 1H), 7.87 (d, 1H), 7.62 (d, 1H), 7.53 (d, 1H), 7.49 (t, 1H), 7.36 (m, 2H), 7.25 (d, 1H), 7.16 (d, 1H), 7.03 (s, 1H), 5.20 (s, 2H), 4.12 (d, 2H), 2.78 (m, 1H), 2.10 (m, 2H), 1.90 (m, 4H)

### Example 31: Preparation of 3-(1-(5-chloro-2-oxo-3-(4-propylphenyl)-2,3-dihydro-1H-benzo[d]imidazol-1-yl)cyclopropyl)benzoic acid

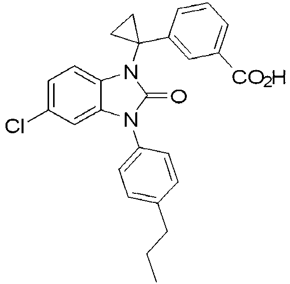

The title compound (32 mg, yield: 47%) was obtained in the same manner as in steps A and B of Example 1 by using 3-(1-(5-chloro-2-oxo-2,3-dihydro-1*H*-benzo[*d*]imidazol-1-yl)chloropropyl)benzoic acid methyl ester (52 mg, 0.152 mmol) obtained in Preparation Example 3 and (4-propylphenyl)boronic acid.
¹H NMR (400 MHz, MeOD) δ 7.82 (m, 2H), 7.35 (m, 6H), 7.23 (m, 1H), 7.12 (m, 1H), 6.96 (m, 1H), 2.67 (m, 2H), 1.70 (m, 6H), 0.97 (t, 3H)

### Example 32: Preparation of 3-((5-chloro-2-oxo-3-phenyl-2,3-dihydro-1H-benzo[d]imidazol-1-yl)methyl)-4-fluorobenzoic acid

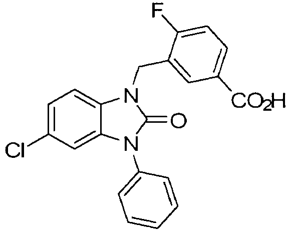

The title compound (25 mg, yield: 51%) was obtained in the same manner as in step A of Preparation Example 21 and step B of Example 1 by using 6-chloro-1-phenyl-1,3-dihydro-2*H-*benzo[*d*]imidazol-2-one (30 mg, 0.123 mmol) obtained in Preparation Example 5 and 3-(bromomethyl)-4-fluorobenzoic acid methyl ester.
¹H NMR (400 MHz, MeOD) δ 7.99 (m, 2H), 7.54 (m, 5H), 7.21 (t, 1H), 7.10 (m, 2H), 7.00 (m, 1H), 5.25 (s, 2H).

### Example 33: Preparation of 3-((5-chloro-2-oxo-3-phenyl-2,3-dihydro-1H-benzo[d]imidazol-1-yl)methyl)-2-fluorobenzoic acid

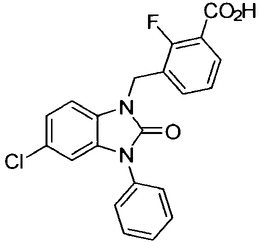

The title compound (21 mg, yield: 43%) was obtained in the same manner as in step A of Preparation Example 21 and step B of Example 1 by using 6-chloro-1-phenyl-1,3-dihydro-2*H-*benzo[*d*]imidazol-2-one (30 mg, 0.123 mmol) obtained in Preparation Example 5 and 3-(aminomethyl)-2-fluorobenzoic acid methyl ester.
¹H NMR (400 MHz, MeOD) δ 7.84 (t, 1H), 7.56 (m, 2H), 7.50 (m, 4 H), 7.21 (t, 1H), 7.12 (m, 2H), 7.00 (m, 1H), 5.25 (s, 2H).

### Example 34: Preparation of 3-((5-chloro-2-oxo-3-phenyl-2,3-dihydro-1H-benzo[d]imidazol-1-yl)methyl)-5-fluorobenzoic acid

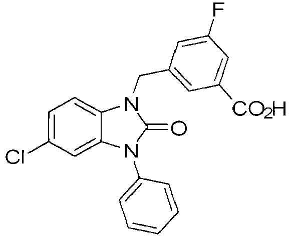

The title compound (13 mg, yield: 27%) was obtained in the same manner as in step A of Preparation Example 21 and step B of Example 1 by using 6-chloro-1-phenyl-1,3-dihydro-2*H-*benzo[*d*]imidazol-2-one (30 mg, 0.123 mmol) obtained in Preparation Example 5 and 3-(bromomethyl)-5-fluorobenzoic acid methyl ester.
¹H NMR (400 MHz, MeOD) δ 7.83 (t, 1H), 7.56 (m, 6H), 7.32 (m, 1H), 7.11 (m, 2H), 7.04 (m, 1H), 5.21 (s, 2H).

### Example 35: Preparation of 5-((5-chloro-2-oxo-3-phenyl-2,3-dihydro-1H-benzo[d]imidazol-1-yl)methyl)-2-fluorobenzoic acid

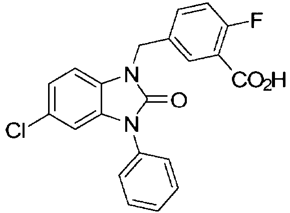

The title compound (31 mg, yield: 64%) was obtained in the same manner as in step A of Preparation Example 21 and step B of Example 1 by using 6-chloro-1-phenyl-1,3-dihydro-2*H-*benzo[d]imidazol-2-one (30 mg, 0.123 mmol) obtained in Preparation Example 5 and 5-(bromomethyl)-2-fluorobenzoic acid methyl ester.
¹H NMR (400 MHz, MeOD) δ 7.93 (t, 1H), 7.54 (m, 6H), 7.17 (m, 1 H), 7.11 (m, 2H), 7.00 (m, 1H), 5.17 (s, 2H)

### Example 36: Preparation of 3-((5-chloro-3-(4-(dimethylcarbamoyl)-3-fluorophenyl)-2-oxo-2,3-dihydro-1H-benzo[d]imidazol-1-yl)methyl)benzoic acid

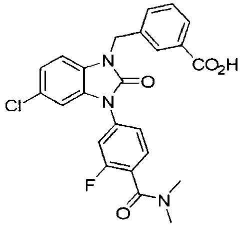

The process of the following steps A, B, and C gave the title compound.

### Step A: Preparation of methyl 3-((5-chloro-3-(3-fluoro-4-(methylcarbamoyl)phenyl)-2-oxo-2,3-dihydro-1H-benzo[d]imidazol-1-yl)methyl)benzoic acid methyl ester

The title compound (55 mg, yield: 75%) was obtained in the same manner as in step A of Example 2 by using 3-((5-chloro-2-oxo-2,3-dihydro-1*H*-benzo[*d*]imidazol-1-yl)methyl)benzoic acid methyl ester (50 mg, 0.158 mmol) obtained in Preparation Example 1 and (3-fluoro-4-(methylcarbamoyl)phenyl)boronic acid (62.2 mg, 0.316 mmol).
MS [M+H] = 468 (M+1)
¹H NMR (500 MHz, CD₃OD) δ8.18 (t, 1H), 8.07 (s, 1H), 8.02 (d, 1H), 7.58 (d, 1H), 7.51 (m, 2H), 7.47 (d, 1H), 7.23 (s, 1H), 7.11 (d, 1H), 6.88 (d, 1H), 5.18 (s, 2H), 4.00 (s, 3H), 3.94 (s, 3H)

### Step B: Preparation of 3-((5-chloro-3-(4-(dimethylcarbamoyl)-3-fluorophenyl)-2-oxo-2,3-dihydro-1H-benzo[d]imidazol-1-yl)methyl)benzoic acid methyl ester

To 3-((5-chloro-3-(3-fluoro-4-(methylcarbamoyl)phenyl)-2-oxo-2,3-dihydro-1*H-*benzo[*d*]imidazol-1-yl)methyl)benzoic acid methyl ester (55 mg, 0.118 mmol) obtained in step A above was added 2 mL of THF, and then NaOH (7.05 mg, 0.294 mmol) was added thereto at 0 °C, followed by stirring for 30 minutes. Then, iodomethane (25.0 mg, 0.176 mmol) was added thereto, and the resulting mixture was stirred at room temperature for 16 hours. After the reaction was terminated, water was added thereto, and an organic layer was separated and dried over Na₂SO₄. The resulting solid was removed, and the filtrate was distilled under reduced pressure and then separated by MPLC to give the title compound (29 mg, yield: 51%).
MS [M+H] = 482 (M+1)
¹H NMR (500 MHz, CD₃OD) δ8.07 (s, 1H), 8.01 (d, 1H), 7.62 (t, 1H), 7.58 (d, 1H), 7.48 (m, 2H), 7.43 (d, 1H), 7.18 (s, 1H), 7.09 (d, 1H), 6.86 (d, 1H), 5.18 (s, 2H), 3.94 (s, 3H), 3.19 (s, 3H), 3.04 (s, 3H)

### Step C: Preparation of 3-((5-chloro-3-(4-(dimethylcarbamoyl)-3-fluorophenyl)-2-oxo-2,3-dihydro-1H-benzo[d]imidazol-1-yl)methyl)benzoic acid

The title compound (18 mg, yield: 64%) was obtained in the same manner as in step B of Example 2 by using 3-((5-chloro-3-(4-(dimethylcarbamoyl)-3-fluorophenyl)-2-oxo-2,3-dihydro-1*H*-benzo[*d*]imidazol-1-yl)methyl)benzoic acid methyl ester (29 mg, 0.060 mmol) obtained in step B above.
MS [M+H] = 468 (M+1)
¹H NMR (500 MHz, DMSO-D6) δ7.99 (s, 1H), 7.87 (d, 1H), 7.63 (m, 3H), 7.56 (d, 1H), 7.50 (t, 1H), 7.27 (m, 2H), 7.20 (d, 1H), 5.22 (s, 2H), 3.04 (s, 3H), 2.93 (s, 3H)

### Example 37: Preparation of 3-((6-chloro-3-(4-(dimethylamino)phenyl)-4-fluoro-2-oxo-2,3-dihydro-1H-benzo[d]imidazol-1-yl)methyl)benzoic acid

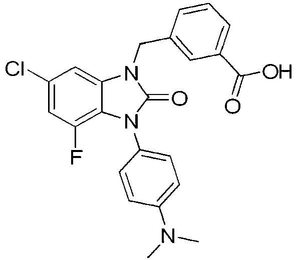

The title compound (0.020 g, yield: 28%) was obtained in the same manner as in steps A and B of Example 24 by using 5-chloro-1-(4-(dimethylamino)phenyl)-7-fluoro-1,3-dihydro-2*H-*benzo[*d*]imidazol-2-one (0.050 g, 0.16 mmol) obtained in Preparation Example 27 and 3-(bromomethyl)benzoic acid methyl ester (0.037 g, 0.16 mmol).
MS [M+H] = 440 (M+1)
¹H-NMR (400 MHz, CDCl3) δ8.05 (2H), 7.60 (dd, 1H), 7.48 (t, 1H), 7.32 (d, 2H), 6.85 (dd, 1H), 6.78 (d, 2H), 6.71 (d, 1H), 5.15 (s, 2H), 3.01 (s, 6H)

### Example 38: Preparation of 3-((5-chloro-3-(4-(dimethylcarbamoyl)phenyl)-2-oxo-2,3-dihydro-1H-benzo[d]imidazol-1-yl)methyl)-2-fluorobenzoic acid

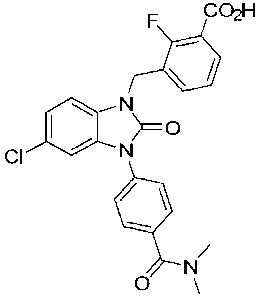

The title compound (43 mg, yield: 62%) was obtained in the same manner as in steps A and B of Example 1 by using 3-((5-chloro-2-oxo-2,3-dihydro-1*H*-benzo[*d*]imidazol-1-yl)methyl)-2-fluorobenzoic acid methyl ester (50 mg, 0.149 mmol) obtained in step C of Preparation Example 2 and (4-dimethylcarbamoyl)phenyl)boronic acid.
¹H NMR (500 MHz, MeOD) δ 8.27 (m, 1H), 7.89 (m, 1H), 7.72 (m, 3H), 7.57 (m, 1H), 7.22 (m, 4H), 5.30 (s, 2H), 3.10 (d, 6H)

### Example 39: Preparation of 3-((5-chloro-3-(4-(dimethylamino)phenyl)-2-oxo-2,3-dihydro-1H-benzo[d]imidazol-1-yl)methyl)-2-fluorobenzoic acid

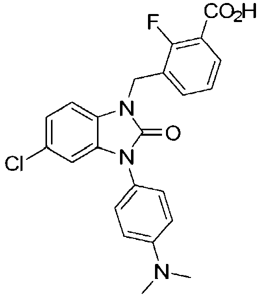

The title compound (39 mg, yield: 60%) was obtained in the same manner as in steps A and B of Example 1 by using 3-((5-chloro-2-oxo-2,3-dihydro-1*H*-benzo[*d*]imidazol-1-yl)methyl)-2-fluorobenzoic acid methyl ester (50 mg, 0.149 mmol) obtained in step C of Preparation Example 2 and (4-dimethylamino)phenyl)boronic acid.
¹H NMR (500 MHz, MeOD) δ 7.90 (t, 1H), 7.55 (t, 1H), 7.26 (m, 3H), 7.13 (m, 2H), 6.93 (m, 3H), 5.28 (s, 2H), 3.04 (s, 6H)

### Example 40: Preparation of 3-((5-chloro-3-(4-isopropoxyphenyl)-2-oxo-2,3-dihydro-1H-benzo[d]imidazol-1-yl)methyl)-2-fluorobenzoic acid

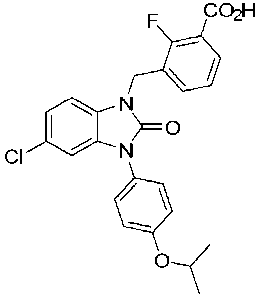

The title compound (35 mg, yield: 52%) was obtained in the same manner as in steps A and B of Example 1 by using 3-((5-chloro-2-oxo-2,3-dihydro-1*H*-benzo[*d*]imidazol-1-yl)methyl)-2-fluorobenzoic acid methyl ester (50 mg, 0.149 mmol) obtained in step C of Preparation Example 2 and (4-isopropoxyphenyl)boronic acid.

¹H NMR (500 MHz, MeOD) δ 7.90 (t, 1H), 7.56 (t, 1H), 7.42 (d, 2H), 7.25 (t, 1H), 7.12 (m, 4H), 6.97 (s, 1H), 5.28 (s, 2H), 4.71 (m, 1H), 1.38 (d, 6H).

### Example 41: Preparation of 3-((5-chloro-3-(4-(methylsulfonamido)phenyl)-2-oxo-2,3-dihydro-1H-benzo[d]imidazol-1-yl)methyl)-2-fluorobenzoic acid

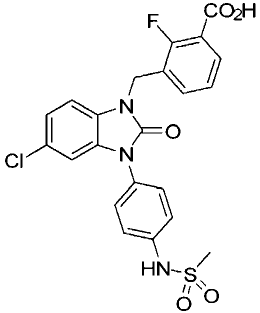

The title compound (31 mg, yield: 43%) was obtained in the same manner as in steps A and B of Example 1 by using 3-((5-chloro-2-oxo-2,3-dihydro-1*H*-benzo[*d*]imidazol-1-yl)methyl)-2-fluorobenzoic acid methyl ester (50 mg, 0.149 mmol) obtained in step C of Preparation Example 2 and 4-(methylsulfonamido)phenyl)boronic acid.
¹H NMR (500 MHz, MeOD) δ 7.90 (t, 1H), 7.53 (m, 5H), 7.25 (t, 1H), 7.15 (m, 2H), 7.05 (s, 1H), 5.28 (s, 2H), 3.07 (s, 3H)

### Example 42: Preparation of 3-((5-chloro-3-(4-(cyclopropylmethoxy)phenyl)-2-oxo-2,3-dihydro-1H-benzo[d]imidazol-1-yl)methyl)benzoic acid

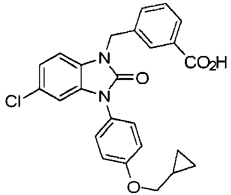

The process of the following steps A and B gave the title compound.

### Step A: Preparation of 3-((5-chloro-3-(4-(cyclopropylmethoxy)phenyl)-2-oxo-2,3-dihydro-1H-benzo[d]imidazol-1-yl)methyl)benzoic acid methyl ester

The title compound (47 mg, yield: 59%) was obtained in the same manner as in step A of Example 27 by using 3-((5-chloro-3-(4-hydroxyphenyl)-2-oxo-2,3-dihydro-1*H-*benzo[*d*]imidazol-1-yl)methyl)benzoic acid methyl ester (70 mg, 0.171 mmol) obtained in step A of Example 13 and (bromomethyl)cyclopropane (46.2 mg, 0.342 mmol).
MS [M+H] = 463 (M+1)
¹H NMR (500 MHz, CD₃OD) δ8.07 (s, 1H), 8.00 (d, 1H), 7.56 (d, 1H), 7.42 (m, 3H), 7.08 (d, 2H), 7.01 (m, 2H), 6.81 (d, 1H), 5.17 (s, 2H), 3.94 (s, 3H), 3.88 (d, 2H), 1.33 (m, 1H), 0.70 (m, 2H), 0.41 (m, 2H)

### Step B: Preparation of 3-((5-chloro-3-(4-(cyclopropylmethoxy)phenyl)-2-oxo-2,3-dihydro-1H-benzo[d]imidazol-1-yl)methyl)benzoic acid

The title compound (39 mg, yield: 98%) was obtained in the same manner as in step B of Example 2 by using 3-((5-chloro-3-(4-(cyclopropylmethoxy)phenyl)-2-oxo-2,3-dihydro-1H-benzo[d]imidazol-1-yl)methyl)benzoic acid methyl ester (41 mg, 0.089 mmol) obtained in step A above.
MS [M+H] = 449 (M+1)
¹H NMR (400 MHz, DMSO-D6) δ7.98 (s, 1H), 7.81 (d, 1H), 7.58 (d, 1H), 7.42 (m, 3H), 7.20 (d, 1H), 7.08 (m, 3H), 6.87 (s, 1H), 5.15 (s, 2H), 3.85 (d, 2H), 1.22 (m, 1H), 0.55 (m, 2H), 0.31 (m, 2H)

### Example 43: Preparation of 3- ((5-chloro-2-oxo-3- (4-(piperidine-1-carbonyl)phenyl) -2,3-dihydro-1H-benzo[d]imidazol-1-yl)methyl)benzoic acid

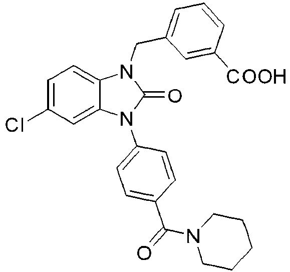

The process of the following steps A and B gave the title compound.

### Step A: Preparation of 3-((5-chloro-2-oxo-3-(4-(piperidine-1-carbonyl)phenyl)-2,3-dihydro-1H-benzo[d]imidazol-1-yl)methyl)benzoic acid methyl ester

The title compound (62.1 mg, yield: 44%) was obtained in the same manner as in step A of Preparation Example 1 by using 6-chloro-1-(4-(piperidine-1-carbonyl)phenyl)-1,3-dihydro-*2H*-benzo[*d*]imidazol-2-one (100.0 mg, 0.28 mmol) obtained in Preparation Example 22 and 3-(bromomethyl)benzoic acid methyl ester.
MS [M+H] = 504 (M+1)
¹H NMR (500 MHz, CDCl₃) δ8.05 (s, 1H), 7.99 (d, 1H), 7.62 (m, 5H), 7.45 (m, 1H), 7.11 (s, 1H), 7.05 (d, 1H), 6.84 (d, 1H), 5.16 (s, 2H), 3.92 (s, 3H), 3.74 (m, 2H), 3.48 (m, 2H), 1.71 (m, 4H), 1.56 (m, 2H)

### Step B: Preparation of 3-((5-chloro-2-oxo-3-(4-(piperidin-1-carbonyl)phenyl)-2,3-dihydro-1H-benzo[d]imidazol-1-yl)methyl)benzoic acid

The title compound (54.4 mg, yield: 90%) was obtained in the same manner as in step B of Example 1 by using methyl 3-((5-chloro-2-oxo-3-(4-(piperidine-1-carbonyl)phenyl)-2,3-dihydro-1*H*-benzo[*d*]imidazol-1-yl)methyl)benzoic acid methyl ester (62.0 mg, 0.12 mmol) obtained in step A above.
MS [M+H] = 490 (M+1)
¹H NMR (500 MHz, CDCl₃) δ 8.10 (s, 1H), 8.04 (d, 1H), 7.61 (m, 5H), 7.48 (t, 1H), 7.11 (s, 1H), 7.06 (d, 1H), 6.85 (s, 1H) 5.18 (s, 2H), 3.75 (m, 2H), 3.42 (m, 2H), 1.71 (m, 4H), 1.56 (m, 2H)

### Example 44: Preparation of 3-((5-chloro-3-(4-(morpholine-4-carbonyl)phenyl)-2-oxo-2,3-dihydro-1H-benzo[d]imidazol-1-yl)methyl)benzoic acid

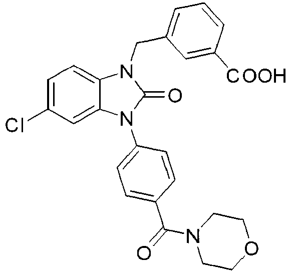

The process of the following steps A and B gave the title compound.

### Step A: Preparation of 3-((5-chloro-3-(4-(morpholine-4-carbonyl)phenyl)-2-oxo-2,3-dihydro-1H-benzo[d]imidazol-1-yl)methyl)benzoic acid methyl ester

The title compound (96.9 mg, yield: 69%) was obtained in the same manner as in step A of Preparation Example 1 by using 6-chloro-1-(4-(morpholine-4-carbonyl)phenyl)-1,3-dihydro-2*H*-benzo[*d*]imidazol-2-one (100.0 mg, 0.28 mmol) obtained in Preparation Example 23 and 3-(bromomethyl)benzoic acid methyl ester.
MS [M+H] = 506 (M+1)
¹H NMR (500 MHz, CDCl₃) δ8.05 (s, 1H), 7.99 (d, 1H), 7.65 (m, 4H), 7.57 (d, 1H), 7.45 (m, 1H), 7.11 (s, 1H), 7.06 (d, 1H), 6.84 (d, 1H), 5.16 (s, 2H), 3.91 (s, 3H), 3.80-3.45 (m, 8H)

### Step B: Preparation of 3-((5-chloro-3-(4-(morpholine-4-carbonyl)phenyl)-2-oxo-2,3-dihydro-1H-benzo[d]imidazol-1-yl)methyl)benzoic acid

The title compound (78.4 mg, yield: 84%) was obtained in the same manner as in step B of Example 1 by using 3-((5-chloro-3-(4-(morpholine-4-carbonyl)phenyl)-2-oxo-2,3-dihydro-1H-benzo[d]imidazol-1-yl)methyl)benzoic acid methyl ester (96.0 mg, 0.19 mmol) obtained in step A above.
MS [M+H] = 492 (M+1)
¹H NMR (500 MHz, CDCl₃) δ 8.10 (1, 1H), 8.05 (d, 1H), 7.65 (m, 5H), 7.48 (m, 1H), 7.12 (s, 1H), 7.07 (d, 1H), 6.86 (d, 1H), 5.18 (s, 2H), 3.80-3.49 (m, 8H)

### Example 45: Preparation of 3-((5-chloro-2-oxo-3-(4-(pyrrolidine-1-carbonyl)phenyl)-2,3-dihydro-1H-benzo[d]imidazol-1-yl)methyl)benzoic acid

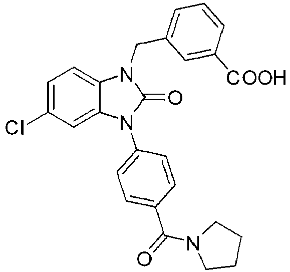

The process of the following steps A and B gave the title compound.

### Step A: Preparation of 3-((5-chloro-2-oxo-3-(4-(pyrrolidine-1-carbonyl)phenyl)-2,3-dihydro-1H-benzo[d]imidazol-1-yl)methyl)benzoic acid methyl ester

The title compound (68.0 mg, yield: 86%) was obtained in the same manner as in step A of Preparation Example 1 by using 6-chloro-1-(4-(pyrrolidine-1-carbonyl)phenyl)-1,3-dihydro-2*H*-benzo[*d*]imidazol-2-one (55.0 mg, 0.16 mmol) obtained in Preparation Example 24 and 3-(bromomethyl)benzoic acid methyl ester.
MS [M+H] = 490 (M+1)
¹H NMR (500 MHz, CDCl₃) δ8.05 (s, 1H), 7.99 (d, 1H), 7.73 (d, 2H), 7.62 (d, 2H), 7.57 (t, 1H), 7.11 (s, 1H), 7.05 (d, 1H), 6.83 (d, 1H), 5.16 (d, 2H), 3.92 (s, 3H), 3.70 (t, 2H), 3.52 (t, 2H), 2.00 (m, 2H), 1.93 (m, 2H)

### Step B: Preparation of 3-((5-chloro-2-oxo-3-(4-(pyrrolidine-1-carbonyl)phenyl)-2,3-dihydro-1H-benzo[d]imidazol-1-yl)methyl)benzoic acid

The title compound (63.6 mg, yield: 96%) was obtained in the same manner as in step B of Example 1 by using 3-((5-chloro-2-oxo-3-(4-(pyrrolidine-1-carbonyl)phenyl)-2,3-dihydro-1*H*-benzo[*d*]imidazol-1-yl)methyl)benzoic acid methyl ester (68.0 mg, 0.14 mmol) obtained in step A above.
MS [M+H] = 476 (M+1)
¹H NMR (500 MHz, CDCl₃) δ 8.10 (s, 2H), 8.04 (d, 1H), 7.74 (d, 2H), 7.63 (m, 3H), 7.47 (m, 1H), 7.11 (s, 1H), 7.06 (d, 1H), 6.85 (d, 1H), 5.18 (s, 2H), 3.71 (t, 2H), 3.52 (t, 2H), 2.01 (m, 2H), 1.93 (m, 2H)

### Example 46: Preparation of 4-(3-(3-carboxybenzyl)-6-chloro-2-oxo-2,3-dihydro-1H-benzo[d]imidazol-1-yl)-3-fluorobenzoic acid

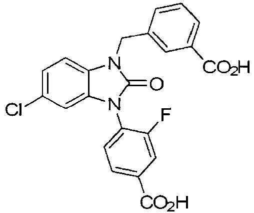

The process of the following steps A and B gave the title compound.

### Step A: Preparation of 3-((5-chloro-3-(2-fluoro-4-(methylcarbamoyl)phenyl)-2-oxo-2,3-dihydro-1H-benzo[d]imidazol-1-yl)methyl)benzoic acid methyl ester

The title compound (25 mg, yield: 11%) was obtained in the same manner as in step A of Example 2 by using 3-((5-chloro-2-oxo-2,3-dihydro-1*H*-benzo[*d*]imidazol-1-yl)methyl)benzoic acid methyl ester (150 mg, 0.474 mmol) obtained in Preparation Example 1 and (2-fluoro-4-(methylcarbamoyl)phenyl)boronic acid (187 mg, 0.947 mmol).
MS [M+H] = 468 (M+1)
¹H NMR (500 MHz, CD₃OD) δ8.19 (d, 2H), 7.91 (t, 1H), 7.64 (d, 3H), 7.20 (t, 1H), 7.13 (m, 2H), 7.03 (d, 1H), 5.22 (s, 2H), 3.97 (s, 3H), 1.64 (s, 3H)

### Step B: Preparation of 4-(3-(3-carboxybenzyl)-6-chloro-2-oxo-2,3-dihydro-1H-benzo[d]imidazol-1-yl)-3-fluorobenzoic acid

The title compound (10 mg, yield: 91%) was obtained in the same manner as in step B of Example 2 by using 3-((5-chloro-3-(2-fluoro-4-(methylcarbamoyl)phenyl)-2-oxo-2,3-dihydro-1*H*-benzo[*d*]imidazol-1-yl)methyl)benzoic acid methyl ester (12 mg, 0.025 mmol) obtained in step A above.
MS [M+H] = 441 (M+1)
¹H NMR (500 MHz, DMSO-D6) δ7.98 (m, 3H), 7.86 (m, 2H), 7.63 (d, 1H), 7.50 (t, 1H), 7.30 (d, 1H), 7.20 (d, 1H), 7.03 (s, 1H), 5.24 (s, 2H)

### Example 47: Preparation of 3-((5-chloro-3-(4-morpholinophenyl)-2-oxo-2,3-dihydro-1H-benzo[d]imidazol-1-yl)methyl)benzoic acid

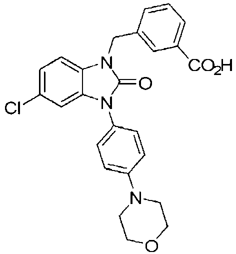

The process of the following steps A and B gave the title compound.

### Step A: Preparation of 3-((5-chloro-3-(4-morpholinophenyl)-2-oxo-2,3-dihydro-1H-benzo[d]imidazol-1-yl)methyl)benzoic acid methyl ester

The title compound (17 mg, yield: 38%) was obtained in the same manner as in step B of Example 29 by using 3-((5-chloro-2-oxo-3-(4-(((trifluoromethyl)sulfonyl)oxy)phenyl)-2,3-dihydro-1*H*-benzo[*d*]imidazol-1-yl)methyl)benzoic acid methyl ester (50 mg, 0.092 mmol) obtained in step A of Example 29 and morpholine (8.05 mg, 0.092 mmol).
MS [M+H] = 478 (M+1)
¹H NMR (500 MHz, CD₃OD) δ8.07 (s, 1H), 8.00 (d, 1H), 7.65 (m, 1H), 7.59 (d, 1H), 7.51 (1H), 7.46 (m, 2H), 7.06 (d, 2H), 7.01 (s, 1H), 6.80 (d, 1H), 5.17 (s, 2H), 3.94 (s, 3H), 3.92 (m, 4H), 3.25 (m, 4H)

### Step B: Preparation of 3-((5-chloro-3-(4-morpholinophenyl)-2-oxo-2,3-dihydro-1H-benzo[d]imidazol-1-yl)methyl)benzoic acid

The title compound (5 mg, yield: 32%) was obtained in the same manner as in step B of Example 2 by using 3-((5-chloro-3-(4-morpholinophenyl)-2-oxo-2,3-dihydro-1*H-*benzo[*d*]imidazol-1-yl)methyl)benzoic acid methyl ester (16 mg, 0.033 mmol) obtained in step A above.
MS [M+H] = 464 (M+1)
¹H NMR (500 MHz, DMSO-D6) 7.95 (s, 1H), 7.85 (d, 1H), 7.58 (d, 1H), 7.46 (t, 1H), 7.40 (d, 2H), 7.23 (d, 1H), 7.12 (m, 3H), 6.91 (s, 1H), 5.19 (s, 2H), 3.78 (m, 4H), 3.20 (m, 4H)

### Example 48: Preparation of 5-((5-chloro-4-fluoro-3-(4-morpholinophenyl)-2-oxo-2,3-dihydro-1H-benzo[d]imidazol-1-yl)methyl)-2-fluorobenzoic acid

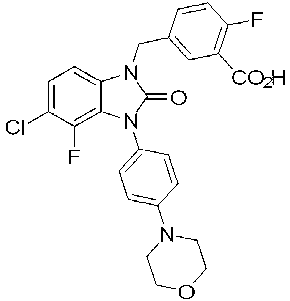

The title compound (21 mg, yield: 70%) was obtained in the same manner as in step C of Preparation Example 7 and step B of Example 1 by using 6-chloro-7-fluoro-1-(4-morpholinophenyl)-1,3-dihydro-2*H*-benzo[*d*]imidazol-2-one (30 mg, 0.086 mmol) obtained in step D of Preparation Example 11.
¹H NMR (500 MHz, MeOD) δ 7.95 (m, 1H), 7.59 (m, 1H), 7.39 (m, 2H), 7.19 (m, 2H), 7.12 (m, 2H), 7.00 (m, 1H), 5.19 (s, 2H), 3.88 (m, 4H), 3.26 (m, 4H)

### Example 49: Preparation of 3-((5-chloro-4-fluoro-3-(4-morpholinophenyl)-2-oxo-2,3-dihydro-1H-benzo[d]imidazol-1-yl)methyl)-5-fluorobenzoic acid

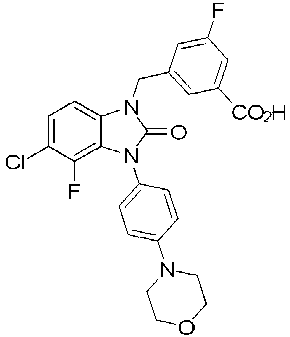

The title compound (17 mg, yield: 55%) was obtained in the same manner as in step C of Preparation Example 7 and step B of Example 1 by using 6-chloro-7-fluoro-1-(4-morpholinophenyl)-1,3-dihydro-*2H*-benzo[*d*]imidazol-2-one (30 mg, 0.086 mmol) obtained in step D of Preparation Example 11 and 3-(bromomethyl)-5-fluorobenzoic acid methyl ester.
¹H NMR (500 MHz, CDCl3) δ 7.85 (s, 1H), 7.63 (d, 1H), 7.37 (d, 3H), 7.17 (t, 1H), 7.10 (d, 1H), 6.96 (d, 1H), 5.22 (s, 2H), 3.85 (m, 4H), 3.23 (m, 4H).

### Example 50: Preparation of 3-((5-chloro-4-fluoro-3-(4-morpholinophenyl)-2-oxo-2,3-dihydro-1H-benzo[d]imidazol-1-yl)methyl)-4-fluorobenzoic acid

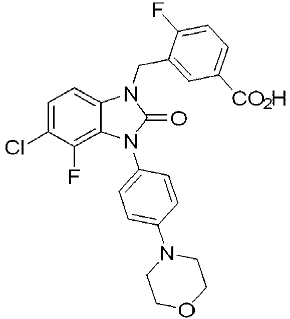

The title compound (26 mg, yield: 72%) was obtained in the same manner as in step C of Preparation Example 7 and step B of Example 1 by using 6-chloro-7-fluoro-1-(4-morpholinophenyl)-1,3-dihydro-2*H*-benzo[*d*]imidazol-2-one (30 mg, 0.086 mmol) obtained in step D of Preparation Example 11 and 3-(bromomethyl)-4-fluorobenzoic acid methyl ester.
¹H NMR (500 MHz, CDCl3) δ 8.01 (m, 2H), 7.36 (d, 2H), 7.26 (t, 1H), 7.18 (t, 1H), 7.10 (d, 2H), 6.96 (d, 1H), 5.26 (s, 2H), 3.85 (m, 4H), 3.24 (m, 4H)

### Example 51: Preparation of 3-((5-chloro-4-fluoro-3-(4-morpholinophenyl)-2-oxo-2,3-dihydro-1H-benzo[d]imidazol-1-yl)methyl)-2-fluorobenzoic acid

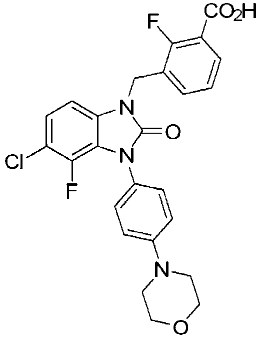

The title compound (25 mg, yield: 67%) was obtained in the same manner as in step C of Preparation Example 7 and step B of Example 1 by using 6-chloro-7-fluoro-1-(4-morpholinophenyl)-1,3-dihydro-2*H*-benzo[*d*]imidazol-2-one (30 mg, 0.086 mmol) obtained in step D of Preparation Example 11 and 3-(bromomethyl)-2-fluorobenzoic acid methyl ester.
¹H NMR (500 MHz, MeOD) δ 7.83 (m, 1H), 7.50 (m, 1H), 7.36 (d, 3H), 7.19 (m, 2H), 7.07 (d, 1H), 6.98 (d, 1H), 5.25 (s, 2H), 3.86 (m, 4H), 3.24 (m, 4H)

### Example 52: Preparation of 6-((5-chloro-4-fluoro-3-(4-morpholinophenyl)-2-oxo-2,3-dihydro-1H-benzo[d]imidazol-1-yl)methyl)picolinic acid

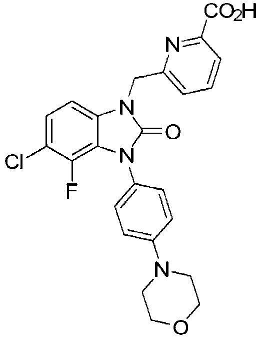

The title compound (17 mg, yield: 74%) was obtained in the same manner as in step C of Preparation Example 7 and step B of Example 1 by using 6-chloro-7-fluoro-1-(4-morpholinophenyl)-1,3-dihydro-2*H*-benzo[*d*]imidazol-2-one (30 mg, 0.086 mmol) obtained in step D of Preparation Example 11 and 6-(bromomethyl)picolinic acid methyl ester.
¹H NMR (500 MHz, MeOD) δ 8.07 (m, 1H), 7.96 (m, 1H), 7.52 (m, 1H), 7.41 (m, 2H), 7.17 (m, 1H), 7.12 (m, 2H), 7.05 (m, 1H), 5.35 (s, 2H), 3.89 (m, 4H), 3.26 (m, 4H)

### Example 53: Preparation of 3-((5-chloro-4-fluoro-3-(4-(3-hydroxyazetidin-1-yl)phenyl)-2-oxo-2,3-dihydro-1H-benzo[d]imidazol-1-yl)methyl)benzoic acid

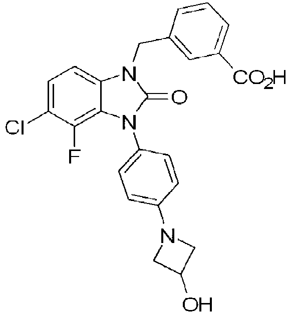

The title compound (0.022 g, yield: 44%) was obtained in the same manner as in steps A and B of Example 24 by using 1-(4-(6-chloro-7-fluoro-2-oxo-2,3-dihydro-1*H-*benzo[*d*]imidazol-1-yl)phenyl)azetidin-3-yl acetate (0.04 g, 0.10 mmol) obtained in Preparation Example 14 and 3-(bromomethyl)benzoic acid methyl ester (0.024 g, 0.10 mmol).
MS [M+H] = 468 (M+1)
¹H-NMR (500 MHz, MeOH-d4) δ8.01 (m, 1H), 7.96 (m, 1H), 7.57 (m, 1H), 7.45 (t, 1H), 7.29 (d, 2H), 7.13 (m, 1H), 6.92 (m, 1H), 6.60 (d, 2H), 5.21 (s, 2H), 4.70 (m, 1H), 4.20 (m, 2H), 3.68 (m, 2H)

### Example 54: Preparation of 3-((5-chloro-3-(4-(dimethylcarbamoyl)phenyl)-7-fluoro-2-oxo-2,3-dihydro-1H-benzo[d]imidazol-1-yl)methyl)benzoic acid

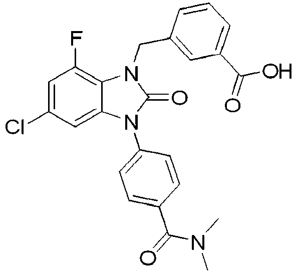

The process of the following steps A and B gave the title compound.

### Step A: Preparation of 3-((5-chloro-3-(4-(dimethylcarbamoyl)phenyl)-7-fluoro-2-oxo-2,3-dihydro-1H-benzo[d]imidazol-1-yl)methyl)benzoic acid methyl ester

The title compound (0.095 g, yield: 88%) was obtained in the same manner as in step A of Example 2 by using 3-((5-chloro-7-fluoro-2-oxo-2,3-dihydro-1*H*-benzo[*d*]imidazol-1-yl)methyl)benzoic acid methyl ester (0.075 g, 0.22 mmol) obtained in Preparation Example 15 and (4-(dimethylcarbamoyl)phenyl)boronic acid (0.086 g, 0.44 mmol).
¹H-NMR (500 MHz, MeOH-d4) δ8.02 (m, 1H), 7.97 (m, 1H), 7.62 (m, 1H), 7.48 (m, 1H), 7.30 (d, 2H), 7.06 (m, 1H), 6.99 (m, 1H), 6.82 (d, 2H), 5.30 (s, 2H), 3.89 (s, 3H), 3.07 (s, 6H)

### Step B: Preparation of 3-((5-chloro-3-(4-(dimethylcarbamoyl)phenyl)-7-fluoro-2-oxo-2,3-dihydro-1H-benzo[d]imidazol-1-yl)methyl)benzoic acid

The title compound (0.020 g, yield: 21%) was obtained in the same manner as in step B of Example 24 by using 3-((5-chloro-3-(4-(dimethylcarbamoyl)phenyl)-7-fluoro-2-oxo-2,3-dihydro-1H-benzo[d]imidazol-1-yl)methyl)benzoic acid methyl ester (0.095 g, 0.20 mmol) obtained in step A above.
MS [M+H] = 468 (M+1)
¹H-NMR (400 MHz, MeOH-d4) δ 8.02 (m, 1H), 7.95 (dd, 1H), 7.68 (m, 4H), 7.58 (dd, 1H), 7.45 (t, 1H), 7.06 (dd, 1H), 6.98 (d, 1H), 5.30 (s, 2H), 3.14 (s, 3H), 3.07 (s, 3H)

### Example 55: Preparation of 3-((5-chloro-3-(4-(methylthio)phenyl)-2-oxo-2,3-dihydro-1H-benzo[d]imidazol-1-yl)methyl)-2-fluorobenzoic acid

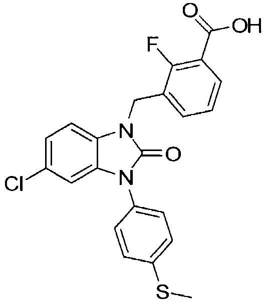

The process of the following steps A and B gave the title compound.

### Step A: Preparation of 3-((5-chloro-3-(4-(methylthio)phenyl)-2-oxo-2,3-dihydro-1H-benzo[d]imidazol-1-yl)methyl)-2-fluorobenzoic acid methyl ester

The title compound (58 mg, yield: 85%) was obtained in the same manner as in step A of Example 2 by using 3-((5-chloro-2-oxo-2,3-dihydro-1*H*-benzo[*d*]imidazol-1-yl)methyl)-2-fluorobenzoic acid methyl ester (50 mg, 0.15 mmol) obtained in Preparation Example 2 and (4-(methylthio)phenyl)boronic acid (38 mg, 0.22 mmol).

### Step B: Preparation of 3-((5-chloro-3-(4-(methylthio)phenyl)-2-oxo-2,3-dihydro-1H-benzo[d]imidazol-1-yl)methyl)-2-fluorobenzoic acid

The title compound (39 mg, yield: 70%) was obtained in the same manner as in step B of Example 2 by using 3-((5-chloro-3-(4-(methylthio)phenyl)-2-oxo-2,3-dihydro-1H-benzo[d]imidazol-1-yl)methyl)-2-fluorobenzoic acid methyl ester (58 mg, 0.12 mmol) obtained in step A above.
¹H NMR (500 MHz, DMSO-D6) δ 7.76 (t, 1H), 7.48 (m, 3H), 7.41 (d, 2H), 7.17 (m, 2H), 7.14 (d, 1H), 6.98 (s, 1H), 5.17 (s, 2H), 2.50 (s, 3H)

### Example 56: Preparation of 3-((5-chloro-2-oxo-3-(4-(pyrrolidine-1-carbonyl)phenyl)-2,3-dihydro-1H-benzo[d]imidazol-1-yl)methyl)-2-fluorobenzoic acid

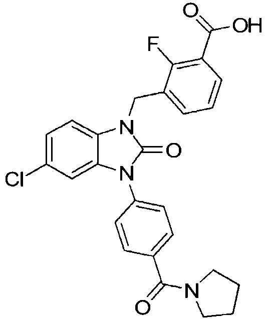

The process of the following steps A, B, C, and D gave the title compound.

### Step A: Preparation of 3-((3-(4-(tert-butoxycarbonyl)phenyl)-5-chloro-2-oxo-2,3-dihydro-1H-benzo[d]imidazol-1-yl)methyl)-2-fluorobenzoic acid methyl ester

The title compound (120 mg, yield: 40%) was obtained in the same manner as in step A of Example 2 by using 3-((5-chloro-2-oxo-2,3-dihydro-1*H*-benzo[*d*]imidazol-1-yl)methyl)-2-fluorobenzoic acid methyl ester (200 mg, 0.60 mmol) obtained in Preparation Example 2 and (4-(*tert-*butoxycarbonylphenyl)boronic acid (265 mg, 1.20 mmol).
¹H-NMR (500 MHz, CDCl3) δ8.17 (d, 2H), 7.89 (t, 1H), 7.61 (d, 3H), 7.18 (t, 1H), 7.11 (d, 2H), 7.00 (d, 1H), 5.20 (s, 2H), 3.94 (s, 3H), 1.62 (s, 9H)

### Step B: Preparation of 4-(6-chloro-3-(2-fluoro-3-(methoxycarbonyl)benzyl)-2-oxo-2,3-dihydro-1H-benzo[d]imidazol-1-yl)benzoic acid

3-((3-(4-(*tert*-Butoxycarbonyl)phenyl)-5-chloro-2-oxo-2,3-dihydro-1*H*-benzo[*d*]imidazol-1-yl)methyl)-2-fluorobenzoic acid methyl ester (120 mg, 0.23 mmol) obtained in step A above was dissolved in 1 mL of DCM and 1 mL of TFA, and then the solution was stirred at room temperature for 2 hours. After the reaction was terminated, the reaction solution was concentrated under reduced pressure and purified by MPLC to give the title compound (105 mg, yield: 98%).

### Step C: Preparation of 3-((5-chloro-2-oxo-3-(4-(pyrrolidine-1-carbonyl)phenyl)-2,3-dihydro-1H-benzo[d]imidazol-1-yl)methyl)-2-fluorobenzoic acid methyl ester

To 4-(6-chloro-3-(2-fluoro-3-(methoxycarbonyl)benzyl)-2-oxo-2,3-dihydro-1*H-*benzo[*d*]imidazol-1-yl)benzoic acid (50 mg, 0.11 mmol) obtained in step B above were added 2 mL of DMF, pyrrolidine (7.8 mg, 0.11 mmol), HATU (63 mg, 0.16 mmol), and TEA (30 µL, 0.22 mmol), and then the resulting mixture was stirred at room temperature for 16 hours. To the reaction solution were added water and EtOAc, and then an organic layer was separated and dried over anhydrous Na₂SO₄. The organic layer was purified by MPLC to give the title compound (57 mg, yield: 99%).
¹H-NMR (500 MHz, CDCl3) δ7.89 (t, 1H), 7.71 (d, 2H), 7.60 (m, 3H), 7.18 (t, 1H), 7.11 (m, 2H), 7.00 (d, 1H), 5.20 (s, 2H), 3.94 (s, 3H), 3.68 (t, 2H), 3.49 (t, 2H), 1.99 (m, 2H), 1.92 (m, 2H)

### Step D: Preparation of 3-((5-chloro-2-oxo-3-(4-(pyrrolidine-1-carbonyl)phenyl)-2,3-dihydro-1H-benzo[d]imidazol-1-yl)methyl)-2-fluorobenzoic acid

The title compound (35 mg, yield: 62%) was obtained in the same manner as in step B of Example 2 by using 3-((5-chloro-2-oxo-3-(4-(pyrrolidine-1-carbonyl)phenyl)-2,3-dihydro-1*H*-benzo[*d*]imidazol-1-yl)methyl)-2-fluorobenzoic acid methyl ester (57 mg, 0.11 mmol) obtained in step C above.
¹H NMR (400 MHz, DMSO-D6) δ7.78 (t, 1H), 7.68 (d, 2H), 7.62 (d, 2H), 7.52 (t, 1H), 7.20 (m, 3H), 7.11 (s, 1H), 5.20 (s, 2H), 3.45 (m, 4H), 1.82 (m, 4H)

### Example 57: Preparation of 3-((5-chloro-2-oxo-3-(4-(piperidine-1-carbonyl)phenyl)-2,3-dihydro-1H-benzo[d]imidazol-1-yl)methyl)-2-fluorobenzoic acid

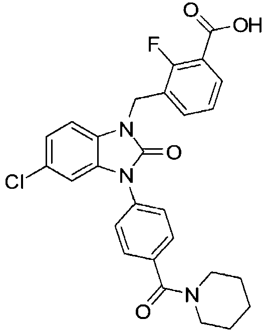

The process of the following steps A and B gave the title compound.

### Step A: Preparation of 3-((5-chloro-2-oxo-3-(4-(piperidine-1-carbonyl)phenyl)-2,3-dihydro-1H-benzo[d]imidazol-1-yl)methyl)-2-fluorobenzoic acid methyl ester

The title compound (61 mg, yield: 99%) was obtained in the same manner as in step C of Example 56 by using 4-(6-chloro-3-(2-fluoro-3-(methoxycarbonyl)benzyl)-2-oxo-2,3-dihydro-1*H*-benzo[*d*]imidazol-1-yl)benzoic acid (50 mg, 0.11 mmol) obtained in step B of Example 56 and piperidine (14 mg, 0.16 mmol).

¹H-NMR (500 MHz, CDCl3) 57.89 (t, 1H), 7.62 (t, 1H), 7.57 (m, 4H), 7.18 (t, 1H), 7.01 (m, 2H), 6.74 (m, 1H), 5.20 (s, 2H), 3.94 (s, 3H), 3.74 (br, 2H), 3.41 (br, 2H), 1.70 (br, 4H), 1.55 (br, 2H)

### Step B: Preparation of 3-((5-chloro-2-oxo-3-(4-(piperidine-1-carbonyl)phenyl)-2,3-dihydro-1H-benzo[d]imidazol-1-yl)methyl)-2-fluorobenzoic acid

The title compound (46 mg, yield: 78%) was obtained in the same manner as in step B of Example 2 by using 3-((5-chloro-2-oxo-3-(4-(piperidine-1-carbonyl)phenyl)-2,3-dihydro-1*H*-benzo[*d*]imidazol-1-yl)methyl)-2-fluorobenzoic acid methyl ester (60 mg, 0.11 mmol) obtained in step A above.
¹H-NMR (400 MHz, CDCl3) δ7.95 (t, 1H), 7.66 (t, 1H), 7.58 (m, 4H), 7.19 (t, 1H), 7.10 (m, 2H), 7.01 (d, 1H), 5.20 (s, 2H), 3.75 (br, 2H), 3.40 (br, 2H), 1.70 (br, 2H), 1.55 (br, 2H)

### Example 58: Preparation of 3-((3-(4-(azetidine-1-carbonyl)phenyl)-5-chloro-2-oxo-2,3-dihydro-1H-benzo[d]imidazol-1-yl)methyl)benzoic acid

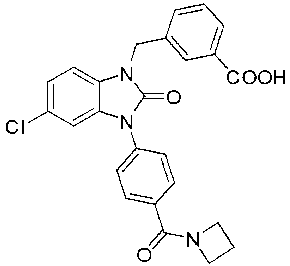

The process of the following steps A and B gave the title compound.

### Step A: Preparation of 3-((5-chloro-2-oxo-3-(4-(pyrrolidine-1-carbonyl)phenyl)-2,3-dihydro-1H-benzo[d]imidazol-1-yl)methyl)benzoic acid methyl ester

4-(6-Chloro-3-(3-(methoxycarbonyl)benzyl)-2-oxo-2,3-dihydro-1*H*-benzo[*d*]imidazol-1-yl)benzoic acid (43.6 mg, 0.10 mmol) obtained in Preparation Example 25 and azetidine (6.27 mg, 0.11 mmol) were dissolved in DMF and cooled to 0 °C. HATU (45.5 mg, 0.12 mmol) and DIPEA (52.3 µL, 0.30 mmol) were added thereto, and then the resulting mixture was stirred while raising the temperature to room temperature. After the reaction was terminated, DMF was removed by the distillation under reduced pressure, and diluted again with EtOAc and then an organic layer was extracted with EtOAc and a saturated aqueous NH₄Cl solution and dried over Na₂SO₄. The resulting solid was filtered, and the filtrate was distilled under reduced pressure and then purified by MPLC to give the title compound (43.0 mg, yield: 90%).
MS [M+H] = 476 (M+1)
¹H NMR (500 MHz, CDCl₃) δ 8.05 (s, 1H), 7.99 (d, 1H), 7.83 (d, 2H), 7.63 (d, 2H), 7.56 (d, 1H), 7.45 (t, 1H), 7.12 (s, 1H), 7.06 (d, 1H), 6.84 (d, 1H), 5.16 (s, 2H), 4.37 (m, 2H), 4.27 (m, 2H), 3.91 (s, 3H), 2.40 (m, 2H)

### Step B: Preparation of 3-((3-(4-(azetidine-1-carbonyl)phenyl)-5-chloro-2-oxo-2,3-dihydro-1H-benzo[d]imidazol-1-yl)methyl)benzoic acid

The title compound (38.2 mg, yield: 92%) was obtained in the same manner as in step B of Example 1 by using 3-((5-chloro-2-oxo-3-(4-(pyrrolidine-1-carbonyl)phenyl)-2,3-dihydro-1*H*-benzo[*d*]imidazol-1-yl)methyl)benzoic acid methyl ester (43.0 mg, 0.090 mmol) obtained in step A above.
MS [M+H] = 462 (M+1)
¹H NMR (500 MHz, CDCl₃) δ 8.09 (s, 1H), 8.04 (d, 1H), 7.84 (d, 2H), 7.64 (m, 3H), 7.48 (t, 1H), 7.13 (s, 1H), 7.07 (d, 1H), 6.85 (d, 1H), 5.18 (s, 2H), 4.37 (m, 2H), 4.28 (m, 2H), 2.40 (m, 2H)

### Example 59: Preparation of 3-((5-chloro-3-(4-(3-methoxypyrrolidine-1-carbonyl)phenyl)-2-oxo-2,3-dihydro-1H-benzo[d]imidazol-1-yl)methyl)benzoic acid

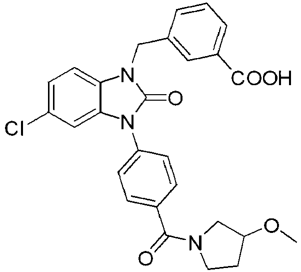

The process of the following steps A and B gave the title compound.

### Step A: Preparation of 3-((5-chloro-3-(4-(3-methoxypyrrolidine-1-carbonyl)phenyl)-2-oxo-2,3-dihydro-1H-benzo[d]imidazol-1-yl)methyl)benzoic acid methyl ester

The title compound (51.2 mg, yield: 99%) was obtained in the same manner as in step A of Example 58 by using 4-(6-chloro-3-(3-(methoxycarbonyl)benzyl)-2-oxo-2,3-dihydro-1*H-*benzo[*d*]imidazol-1-yl)benzoic acid (43.6 mg, 0.10 mmol) obtained in Preparation Example 25 and 3-methoxypyrrolidine (11.10 mg, 0.11 mmol).
MS [M+H] = 520 (M+1)
¹H NMR (400 MHz, CDCl3) δ 8.05 (s, 1H), 8.01 (d, 1H), 7.75 (m, 2H), 7.63 (d, 2H), 7.57 (d, 1H), 7.45 (m, 1H), 7.11 (s, 1H), 7.05 (d, 1H), 6.84 (d, 1H), 5.16 (s, 2H), 4.07 (s, 1H), 3.91 (s, 3H), 3.79 (m, 2H), 3.69 (m, 1H), 3.59 (m, 1H), 3.39 (s, 3H), 2.13 (m, 2H)

### Step B: Preparation of 3-((5-chloro-3-(4-(3-methoxypyrrolidine-1-carbonyl)phenyl)-2-oxo-2,3-dihydro-1H-benzo[d]imidazol-1-yl)methyl)benzoic acid

The title compound (38.2 mg, yield: 92%) was obtained in the same manner as in step B of Example 1 by using methyl 3-((5-chloro-3-(4-(3-methoxypyrrolidine-1-carbonyl)phenyl)-2-oxo-2,3-dihydro-1*H*-benzo[*d*]imidazol-1-yl)methyl)benzoic acid methyl ester (43.0 mg, 0.090 mmol) obtained in step A above.
MS [M+H] = 506 (M+1)
¹H NMR (500 MHz, CDCl₃) δ 8.10 (s, 1H), 8.05 (d, 1H), 7.76 (m, 2H), 7.63 (m, 3H), 7.48 (t, 1H), 7.12 (s, 1H), 7.06 (d, 1H), 6.85 (d, 1H) 5.18 (s, 2H), 4.07 (s, 1H), 3.83 (m, 2H), 3.70 (m, 1H), 3.57 (m, 1H), 3.39 (s, 3H), 2.13 (m, 2H)

### Example 60: Preparation of 3-((5-chloro-3-(4-(4-methylpiperazine-1-carbonyl)phenyl)-2-oxo-2,3-dihydro-1H-benzo[d]imidazol-1-yl)methyl)benzoic acid

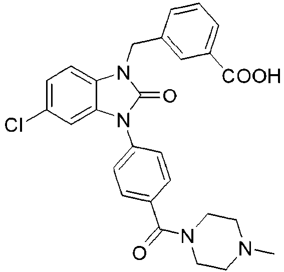

The process of the following steps A and B gave the title compound.

### Step A: Preparation of 3-((5-chloro-3-(4-(4-methylpiperazine-1-carbonyl)phenyl)-2-oxo-2,3-dihydro-1H-benzo[d]imidazol-1-yl)methyl)benzoic acid methyl ester

The title compound (43.3 mg, yield: 83%) was obtained in the same manner as in step A of Example 58 by using 4-(6-chloro-3-(3-(methoxycarbonyl)benzyl)-2-oxo-2,3-dihydro-1*H-*benzo[*d*]imidazol-1-yl)benzoic acid (43.6 mg, 0.10 mmol) obtained in Preparation Example 25 and 1-methylpiperazine (11.00 mg, 0.110 mmol).
MS [M+H] = 520 (M+1)
¹H NMR (500 MHz, CDCl₃) δ 8.05 (s, 1H), 7.99 (d, 1H), 7.63 (m, 4H), 7.57 (d, 1H), 7.45 (t, 1H), 7.11 (s, 1H), 7.06 (d, 1H), 6.84 (d, 1H), 5.16 (s, 2H), 3.91 (s, 3H), 3.84 (m, 2H), 3.53 (m, 2H), 2.53 (m, 2H), 2.41 (m, 2H), 2.35 (s, 3H)

### Step B: Preparation of 3-((5-chloro-3-(4-(4-methylpiperazin-1-carbonyl)phenyl)-2-oxo-2,3-dihydro-1H-benzo[d]imidazol-1-yl)methyl)benzoic acid

The title compound (17.0 mg, yield: 92%) was obtained in the same manner as in step B of Example 1 by using 3-((5-chloro-3-(4-(4-methylpiperazine-1-carbonyl)phenyl)-2-oxo-2,3-dihydro-1*H*-benzo[*d*]imidazol-1-yl)methyl)benzoic acid methyl ester (43.3 mg, 0.083 mmol) obtained in step A above.
MS [M+H] = 505 (M+1)
¹H NMR (400 MHz, DMSO-d6) δ 7.97 (s, 1H), 7.87 (d, 1H), 7.70-7.60 (m, 6H), 7.51 (t, 1H), 7.30 (d, 1H), 7.20 (m, 2H), 5.22 (s, 2H), 3.56 (m, 2H), 3.49 (m, 2H), 2.53 (m, 2H), 2.41 (m, 2H), 2.35 (s, 3H)

### Example 61: Preparation of 3-((5-chloro-3-(4-(4-hydroxypiperidin-1-carbonyl)phenyl)-2-oxo-2,3-dihydro-1H-benzo[d]imidazol-1-yl)methyl)benzoic acid

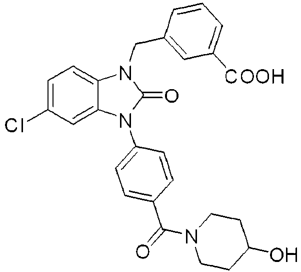

The process of the following steps A and B gave the title compound.

### Step A: Preparation of 3-((5-chloro-3-(4-(4-hydroxypiperidin-1-carbonyl)phenyl)-2-oxo-2,3-dihydro-1H-benzo[d]imidazol-1-yl)methyl)benzoic acid methyl ester

4-(6-Chloro-3-(3-(methoxycarbonyl)benzyl)-2-oxo-2,3-dihydro-1*H*-benzo[*d*]imidazol-1-yl)benzoic acid (43.6 mg, 0.10 mmol) obtained in Preparation Example 25 was dissolved in DCM, then cooled to 0 °C, and SOCl₂ (59.49 mg, 0.50 mmol) and a catalytic amount of DMF were added thereto, and the resulting mixture was stirred for 3 hours while raising the temperature to room temperature. Thereafter, the residual solvent and the reactant were removed under reduced pressure, and the condensed reactant was dissolved again in DCM. The reactant was cooled to 0 °C, 4-hydroxypiperidine (11.13 mg, 0.110 mmol) and TEA (20.24 mg, 0.20 mmol) were added thereto, and then the resulting mixture was stirred while raising the temperature to room temperature. After the reaction was terminated, an organic layer was extracted with DCM and a saturated aqueous NaCl solution, and dried over Na₂SO₄. The resulting solid was filtered, and the filtrate was distilled under reduced pressure and then purified by MPLC to give the title compound (43.1 mg, yield: 91%).
MS [M+H] = 520 (M+1)
¹H NMR (500 MHz, CDCl₃) δ 8.05 (s, 1H), 7.99 (m, 1H), 7.63 (m, 4H), 7.57 (m, 1H), 7.45 (t, 1H), 7.11 (s, 1H), 7.05 (d, 1H), 6.84 (d, 1H), 5.16 (s, 2H), 4.21 (m, 1H), 4.02 (m, 1H), 3.92 (s, 3H), 3.77 (m, 1H), 3.44 (m, 2H), 2.02 (m, 2H), 1.65 (m, 2H)

### Step B: Preparation of 3-((5-chloro-3-(4-(4-hydroxypiperidin-1-carbonyl)phenyl)-2-oxo-2,3-dihydro-1H-benzo[d]imidazol-1-yl)methyl)benzoic acid

The title compound (25.4 mg, yield: 61%) was obtained in the same manner as in step B of Example 1 by using 3-((5-chloro-3-(4-(4-hydroxypiperidine-1-carbonyl)phenyl)-2-oxo-2,3-dihydro-1*H*-benzo[*d*]imidazol-1-yl)methyl)benzoic acid methyl ester (43.1 mg, 0.083 mmol) obtained in step A above.
MS [M+H] = 506 (M+1)
¹H NMR (500 MHz, CDCl₃) δ 8.09 (s, 1H), 8.04 (d, 1H), 7.62 (m, 5H), 7.48 (d, 1H), 7.12 (s, 1H), 7.07 (d, 1H), 6.85 (d, 1H), 5.18 (s, 2H), 4.22 (m, 1H), 4.02 (m, 1H), 3.75 (m, 1H), 3.45 (m, 2H), 2.00 (m, 2H), 1.66 (m, 2H)

### Example 62: Preparation of 3-((5-chloro-3-(4-(4-methoxypiperidine-1-carbonyl)phenyl)-2-oxo-2,3-dihydro-1H-benzo[d]imidazol-1-yl)methyl)benzoic acid

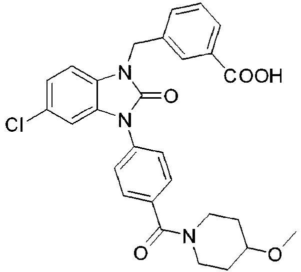

The process of the following steps A and B gave the title compound.

### Step A: Preparation of 3-((5-chloro-3-(4-(4-methoxypiperidine-1-carbonyl)phenyl)-2-oxo-2,3-dihydro-1H-benzo[d]imidazol-1-yl)methyl)benzoic acid methyl ester

The title compound (46.0 mg, yield: 86%) was obtained in the same manner as in step A of Example 58 by using 4-(6-chloro-3-(3-(methoxycarbonyl)benzyl)-2-oxo-2,3-dihydro-1*H-*benzo[*d*]imidazol-1-yl)benzoic acid (43.6 mg, 0.10 mmol) obtained in Preparation Example 25 and 4-methoxypiperidine (12.64 mg, 0.110 mmol).
MS [M+H] = 535 (M+1)

### Step B: Preparation of 3-((5-chloro-3-(4-(4-methoxypiperidin-1-carbonyl)phenyl)-2-oxo-2,3-dihydro-1H-benzo[d]imidazol-1-yl)methyl)benzoic acid

The title compound (29.5 mg, yield: 66%) was obtained in the same manner as in step B of Example 1 by using 3-((5-chloro-3-(4-(4-methoxypiperidine-1-carbonyl)phenyl)-2-oxo-2,3-dihydro-1*H*-benzo[*d*]imidazol-1-yl)methyl)benzoic acid methyl ester (43.0 mg, 0.090 mmol) obtained in step A above.
MS [M+H] = 520 (M+1)
¹H NMR (500 MHz, CDCl₃) δ 8.10 (s, 1H), 8.04 (d, 1H), 7.63 (m, 5H), 7.48 (t, 1H), 7.12 (s, 1H), 7.07 (d, 1H), 6.85 (d, 1H), 5.18 (d, 2H), 4.04 (m, 1H), 3.68 (m, 1H), 3.58 (m, 1H), 3.51 (m, 1H), 3.38 (s, 3H), 3.31 (m, 1H), 1.96-1.60 (m, 4H)

### Example 63: Preparation of 3-(1-(5-chloro-2-oxo-3-(4-(pyrrolidine-1-carbonyl)phenyl)-2,3-dihydro-1H-benzo[d]imidazol-1-yl)cyclopropyl)benzoic acid

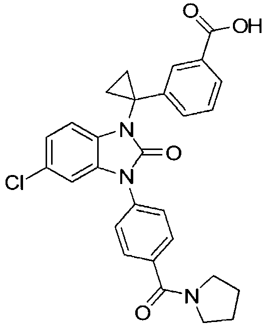

The process of the following steps A, B, C, and D gave the title compound.

### Step A: Preparation of 3-(1-(3-(4-(tert-butoxycarbonyl)phenyl)-5-chloro-2-oxo-2,3-dihydro-1H-benzo[d]imidazol-1-yl)cyclopropyl)benzoic acid methyl ester

The title compound (181 mg, yield: 60%) was obtained in the same manner as in step A of Example 2 by using 3-(1-(5-chloro-2-oxo-2,3-dihydro-1*H*-benzo[*d*]imidazol-1-yl)cyclopropyl)benzoic acid methyl ester (200 mg, 0.58 mmol) obtained in Preparation Example 3 and (4-(*tert-*butoxycarbonylphenyl)boronic acid (194 mg, 0.88 mmol).
¹H-NMR (500 MHz, CDCl3) δ8.16 (d, 2H), 7.90 (d, 1H), 7.88 (s, 1H), 7.62 (d, 2H), 7.50 (d, 1H), 7.36 (t, 1H), 7.12 (m, 3H), 3.90 (s, 3H), 1.73 (m, 2H), 1.68 (m, 2H), 1.62 (s, 9H)

### Step B: Preparation of 4-(6-chloro-3-(1-(3-(methoxycarbonyl)phenyl)cyclopropyl)-2-oxo-2,3-dihydro-1H-benzo[d]imidazol-1-yl)benzoic acid

The title compound (161 mg, yield: 99%) was obtained in the same manner as in step B of Example 56 by using 3-(1-(3-(4-(*tert*-butoxycarbonyl)phenyl)-5-chloro-2-oxo-2,3-dihydro-1*H*-benzo[*d*]imidazol-1-yl)cyclopropyl)benzoic acid methyl ester (181 mg, 0.35 mmol) obtained in step A above.

### Step C: Preparation of 3-(1-(5-chloro-2-oxo-3-(4-(pyrrolidine-1-carbonyl)phenyl)-2,3-dihydro-1H-benzo[d]imidazol-1-yl)cyclopropyl)benzoic acid methyl ester

The title compound (54 mg, yield: 98%) was obtained in the same manner as in step C of Example 56 by using 4-(6-chloro-3-(1-(3-(methoxycarbonyl)phenyl)cyclopropyl)-2-oxo-2,3-dihydro-1*H*-benzo[*d*]imidazol-1-yl)benzoic acid (50 mg, 0.11 mmol) obtained in step B above and pyrrolidine (9.2 mg, 0.13 mmol).
¹H-NMR (500 MHz, CDCl3) δ7.90 (d, 1H), 7.88 (s, 1H), 7.71 (d, 2H), 7.60 (d, 2H), 7.50 (d, 1H), 7.36 (t, 1H), 7.15 (d, 1H), 7.10 (d, 2H), 3.90 (s, 3H), 3.68 (t, 2H), 3.49 (t, 2H), 1.98 (m, 2H), 1.93 (m, 2H), 1.73 (m, 2H), 1.68 (m, 2H)

### Step D: Preparation of 3-(1-(5-chloro-2-oxo-3-(4-(pyrrolidine-1-carbonyl)phenyl)-2,3-dihydro-1H-benzo[d]imidazol-1-yl)cyclopropyl)benzoic acid

The title compound (20 mg, yield: 38%) was obtained in the same manner as in step B of Example 2 by using 3-(1-(5-chloro-2-oxo-3-(4-(pyrrolidine-1-carbonyl)phenyl)-2,3-dihydro-1H-benzo[d]imidazol-1-yl)cyclopropyl)benzoic acid methyl ester (54 mg, 0.10 mmol) obtained in step C above.
¹H NMR (500 MHz, DMSO-D6) δ7.81 (d, 1H), 7.73 (d, 3H), 7.66 (d, 2H), 7.46 (m, 2H), 7.28 (d, 1H), 7.20 (d, 1H), 7.16 (s, 1H), 3.51 (m, 2H), 3.47 (m, 2H), 1.87 (m, 2H), 1.84 (m, 2H), 1.69 (m, 4H)

### Example 64: Preparation of 3-(1-(5-chloro-2-oxo-3-(4-(piperidin-1-carbonyl)phenyl)-2,3-dihydro-1H-benzo[d]imidazol-1-yl)cyclopropyl)benzoic acid

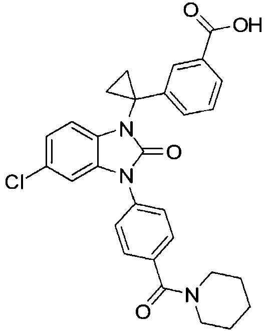

The process of the following steps A and B gave the title compound.

### Step A: Preparation of 3-(1-(5-chloro-2-oxo-3-(4-(piperidin-1-carbonyl)phenyl)-2,3-dihydro-1H-benzo[d]imidazol-1-yl)cyclopropyl)benzoic acid methyl ester

The title compound (53 mg, yield: 92%) was obtained in the same manner as in step C of Example 56 by using 4-(6-chloro-3-(1-(3-(methoxycarbonyl)phenyl)cyclopropyl)-2-oxo-2,3-dihydro-1*H*-benzo[*d*]imidazol-1-yl)benzoic acid (53 mg, 0.10 mmol) obtained in step B of Example 63 and piperidine (11 mg, 0.13 mmol).
¹H-NMR (500 MHz, CDCl3) δ7.92 (m, 2H), 7.60 (m, 4H), 7.53 (d, 1H), 7.38 (t, 1H), 7.17 (d, 1H), 7.13 (d, 2H), 3.92 (s, 3H), 3.76 (br, 2H), 3.43 (br, 2H), 1.71 (m, 6H), 1.58 (m, 4H)

### Step B: Preparation of 3-(1-(5-chloro-2-oxo-3-(4-(piperidin-1-carbonyl)phenyl)-2,3-dihydro-1H-benzo[d]imidazol-1-yl)cyclopropyl)benzoic acid

The title compound (31 mg, yield: 60%) was obtained in the same manner as in step B of Example 2 by using 3-(1-(5-chloro-2-oxo-3-(4-(piperidin-1-carbonyl)phenyl)-2,3-dihydro-1H-benzo[d]imidazol-1-yl)cyclopropyl)benzoic acid methyl ester (53 mg, 0.10 mmol) obtained in step A above.
¹H NMR (500 MHz, DMSO-D6) δ13.04 (s, 1H), 7.81 (d, 1H), 7.72 (s, 1H), 7.66 (d, 2H), 7.58 (d, 2H), 7.46 (m, 2H), 7.28 (d, 1H), 7.19 (m, 2H), 3.62 (br, 2H), 3.32 (br, 2H), 1.64 (m, 6H), 1.54 (m, 4H)

### Example 65: Preparation of 3-((5-chloro-3-(4-(dimethylamino)phenyl)-7-fluoro-2-oxo-2,3-dihydro-1H-benzo[d]imidazol-1-yl)methyl)-5-fluorobenzoic acid

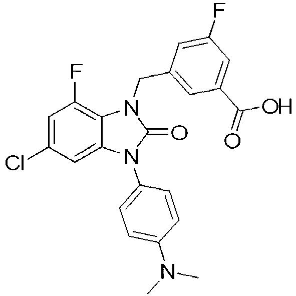

The process of the following steps A and B gave the title compound.

### Step A: Preparation of 3-((5-chloro-3-(4-(dimethylamino)phenyl)-7-fluoro-2-oxo-2,3-dihydro-1H-benzo[d]imidazol-1-yl)methyl)-5-fluorobenzoic acid methyl ester

The title compound (15 mg) was obtained in the same manner as in steps A and B of Preparation Example 8 and step B of Example 2 in turn by using 5-chloro-1,2-difluoro-3-nitrobenzene (0.641 g, 3.31 mmol) and 3-(aminomethyl)-5-fluorobenzoic acid methyl ester (0.607 g, 3.31 mmol) obtained in Preparation Example 6.

¹H-NMR (400 MHz, CDCl3) 57.88 (d, 1H), 7.64 (dd, 1H), 7.30 (dd, 1H), 7.27 (d, 2H), 6.82 (4H), 5.23 (s, 2H), 3.93 (s, 3H), 3.00 (s, 6H)

### Step B: Preparation of 3-((5-chloro-3-(4-(dimethylamino)phenyl)-7-fluoro-2-oxo-2,3-dihydro-1H-benzo[d]imidazol-1-yl)methyl)-5-fluorobenzoic acid

The title compound (7 mg, yield: 48%) was obtained in the same manner as in step B of Example 24 by using 3-((5-chloro-3-(4-(dimethylamino)phenyl)-7-fluoro-2-oxo-2,3-dihydro-1*H*-benzo[*d*]imidazol-1-yl)methyl)-5-fluorobenzoic acid methyl ester (15 mg, 0.032 mmol) obtained in step A above.
MS [M+H] = 458 (M+1)
¹H-NMR (400 MHz, CDCl3) δ7.95 (d, 1H), 7.68 (dd, 1H), 7.36 (dd, 1H), 7.27 (d, 2H), 6.83 (4H), 5.26 (s, 2H), 3.01 (s, 6H)

### Example 66: Preparation of 3-((5-chloro-3-(4-(dimethylamino)phenyl)-7-fluoro-2-oxo-2,3-dihydro-1H-benzo[d]imidazol-1-yl)methyl)benzoic acid

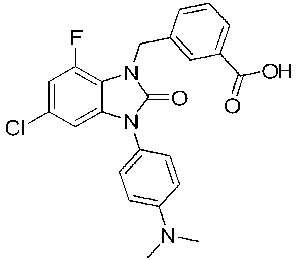

The process of the following steps A and B gave the title compound.

### Step A: Preparation of 3-((5-chloro-3-(4-(dimethylamino)phenyl)-7-fluoro-2-oxo-2,3-dihydro-1H-benzo[d]imidazol-1-yl)methyl)benzoic acid methyl ester

The title compound (0.09 g, yield: 83%) was obtained in the same manner as in step A of Example 2 by using 3-((5-chloro-7-fluoro-2-oxo-2,3-dihydro-1H-benzo[d]imidazol-1-yl)methyl)benzoic acid methyl ester (0.080 g, 0.24 mmol) obtained in Preparation Example 15 and (4-(dimethylamino)phenyl)boronic acid (0.079 g, 0.48 mmol).
¹H-NMR (500 MHz, CDCl3) δ8.08 (d, 1H), 7.97 (dd, 1H), 7.61 (dd, 1H), 7.41 (t, 1H), 7.28 (d, 2H), 6.80 (4H), 5.25 (s, 2H), 3.91 (s, 3H), 3.01 (s, 6H)

### Step B: Preparation of 3-((5-chloro-3-(4-(dimethylamino)phenyl)-7-fluoro-2-oxo-2,3-dihydro-1H-benzo[d]imidazol-1-yl)methyl)benzoic acid

The title compound (0.075 g, yield: 86%) was obtained in the same manner as in step B of Example 24 by using 3-((5-chloro-3-(4-(dimethylamino)phenyl)-7-fluoro-2-oxo-2,3-dihydro-1*H*-benzo[*d*]imidazol-1-yl)methyl)benzoic acid methyl ester (0.09 g, 0.20 mmol) obtained in step A above.
MS [M+H] = 440 (M+1)
¹H-NMR (400 MHz, CDCl3) δ8.14 (d, 1H), 8.01 (dd, 1H), 7.66 (dd, 1H), 7.44 (t, 1H), 7.30 (d, 2H), 6.84 (4H), 5.27 (s, 2H), 3.02 (s, 6H)

### Example 67: Preparation of 3-(1-(5-chloro-2-oxo-3-(4-(pyrrolidine-1-carbonyl)phenyl)-2,3-dihydro-1H-benzo[d]imidazol-1-yl)cyclopropyl)-2-fluorobenzoic acid

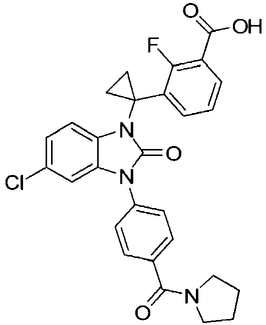

The process of the following steps A, B, C, and D gave the title compound.

### Step A: Preparation of 3-(1-(3-(4-(tert-butoxycarbonyl)phenyl)-5-chloro-2-oxo-2,3-dihydro-1H-benzo[d]imidazol-1-yl)cyclopropyl)-2-fluorobenzoic acid methyl ester

The title compound (198 mg, yield: 67%) was obtained in the same manner as in step A of Example 2 by using 3-(1-(5-chloro-2-oxo-2,3-dihydro-1*H*-benzo[*d*]imidazol-1-yl)cyclopropyl)-2-fluorobenzoic acid methyl ester (200 mg, 0.55 mmol) obtained in Preparation Example 4 and (4-(*tert-*butoxycarbonylphenyl)boronic acid (194 mg, 0.88 mmol).
¹H-NMR (400 MHz, CDCl3) δ8.12 (m, 3H), 7.85 (m, 2H), 7.53 (d, 3H), 7.18 (t, 2H), 7.05 (d, 1H), 3.90 (s, 3H), 1.68 (m, 2H), 1.63 (m, 2H), 1.58 (s, 9H)

### Step B: Preparation of 4-(6-chloro-3-(1-(2-fluoro-3-(methoxycarbonyl)phenyl)cyclopropyl)-2-oxo-2,3-dihydro-1H-benzo[d]imidazol-1-yl)benzoic acid

The title compound (156 mg, yield: 88%) was obtained in the same manner as in step B of Example 56 by using 3-(1-(3-(4-(*tert*-butoxycarbonyl)phenyl)-5-chloro-2-oxo-2,3-dihydro-1*H*-benzo[*d*]imidazol-1-yl)cyclopropyl)-2-fluorobenzoic acid methyl ester (198 mg, 0.37 mmol) obtained in step A above.
¹H-NMR (500 MHz, CDCl3) δ8.25 (d, 2H), 8.15 (t, 1H), 7.86 (t, 1H), 7.63 (d, 2H), 7.57 (d, 1H), 7.20 (m, 2H), 7.10 (s, 1H), 3.91 (s, 3H), 1.66(m, 2H), 1.64 (m 2H)

### Step C: Preparation of 3-(1-(5-chloro-2-oxo-3-(4-(pyrrolidine-1-carbonyl)phenyl)-2,3-dihydro-1H-benzo[d]imidazol-1-yl)cyclopropyl)-2-fluorobenzoic acid methyl ester

The title compound (50 mg, yield: 89%) was obtained in the same manner as in step C of Example 56 by using 4-(6-chloro-3-(1-(2-fluoro-3-(methoxycarbonyl)phenyl)cyclopropyl)-2-oxo-2,3-dihydro-1*H*-benzo[*d*]imidazol-1-yl)benzoic acid (50 mg, 0.10 mmol) obtained in step B above and pyrrolidine (8.9 mg, 0.12 mmol).
¹H-NMR (500 MHz, CDCl3) δ8.15 (t, 1H), 7.85 (t, 1H), 7.67 (d, 2H), 7.53 (m, 3H), 7.19 (m, 2H), 7.04 (s, 1H), 3.90 (s, 3H), 3.66 (t, 2H), 3.46 (t, 2H), 1.98 (m, 2H0, 1.91 (m, 2H), 1.68 (m, 2H), 1.64 (m, 2H)

### Step D: Preparation of 3-(1-(5-chloro-2-oxo-3-(4-(pyrrolidine-1-carbonyl)phenyl)-2,3-dihydro-1H-benzo[d]imidazol-1-yl)cyclopropyl)-2-fluorobenzoic acid

The title compound (42 mg, yield: 87%) was obtained in the same manner as in step B of Example 2 by using 3-(1-(5-chloro-2-oxo-3-(4-(pyrrolidine-1-carbonyl)phenyl)-2,3-dihydro-1*H*-benzo[*d*]imidazol-1-yl)cyclopropyl)-2-fluorobenzoic acid methyl ester (50 mg, 0.09 mmol) obtained in step C above.
MS [M+H] = 520 (M+1)
¹H-NMR (400 MHz, DMSO-d6) δ7.92 (t, 1H), 7.73 (t, 1H), 7.65 (dt, 2H), 7.55 (d, 2H), 7.46 (dd, 1H), 7.23 (m, 2H), 7.03 (d, 1H), 3.45 (t, 2H), 3.40 (t, 2H), 1.83 (m, 4H), 1.60 (d, 4H)

### Example 68: Preparation of 3-(1-(5-chloro-2-oxo-3-(4-(piperidine-1-carbonyl)phenyl)-2,3-dihydro-1H-benzo[d]imidazol-1-yl)cyclopropyl)-2-fluorobenzoic acid

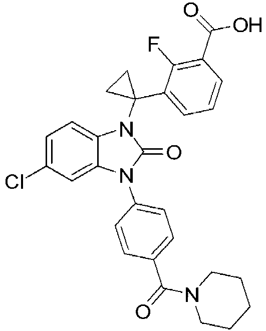

The process of the following steps A and B gave the title compound.

### Step A: Preparation of 3-(1-(5-chloro-2-oxo-3-(4-(piperidine-1-carbonyl)phenyl)-2,3-dihydro-1H-benzo[d]imidazol-1-yl)cyclopropyl)-2-fluorobenzoic acid methyl ester

The title compound (53 mg, yield: 92%) was obtained in the same manner as in step C of Example 56 by using 4-(6-chloro-3-(1-(2-fluoro-3-(methoxycarbonyl)phenyl)cyclopropyl)-2-oxo-2,3-dihydro-1*H*-benzo[*d*]imidazol-1-yl)benzoic acid (50 mg, 0.10 mmol) obtained in step B of Example 67 and piperidine (8.9 mg, 0.10 mmol).
MS [M+H] = 549 (M+1)
¹H-NMR (500 MHz, CDCl3) δ8.14 (t, 1H), 7.85 (t, 1H), 7.52 (m, 5H), 7.18 (m, 2H), 7.03 (s, 1H), 3.90 (s, 3H), 3.72 (m, 2H), 3.38 (m, 2H), 1.69 (m, 6H), 1.63 (m, 2H), 1.56 (m, 2H)

### Step B: Preparation of 3-(1-(5-chloro-2-oxo-3-(4-(piperidine-1-carbonyl)phenyl)-2,3-dihydro-1H-benzo[d]imidazol-1-yl)cyclopropyl)-2-fluorobenzoic acid

The title compound (48 mg, yield: 87%) was obtained in the same manner as in step B of Example 2 by using 3-(1-(5-chloro-2-oxo-3-(4-(piperidine-1-carbonyl)phenyl)-2,3-dihydro-1*H*-benzo[*d*]imidazol-1-yl)cyclopropyl)-2-fluorobenzoic acid methyl ester (53 mg, 0.096 mmol) obtained in step A above.
MS [M+H] = 534 (M+1)
¹H-NMR (500 MHz, CDCl3) δ8.22 (t, 1H), 7.95 (t, 1H), 7.57 (d, 3H), 7.54 (d, 2H), 7.23 (t, 1H), 7.18 (d, 1H), 7.05 (s, 1H), 3.76 (m, 2H), 3.41 (m, 2H), 1.72 (m, 6H), 1.65 (m, 2H), 1.55 (m, 2H)

### Example 69: Preparation of 3-(1-(5-chloro-3-(4-(dimethylamino)phenyl)-2-oxo-2,3-dihydro-1H-benzo[d]imidazol-1-yl)cyclopropyl)benzoic acid

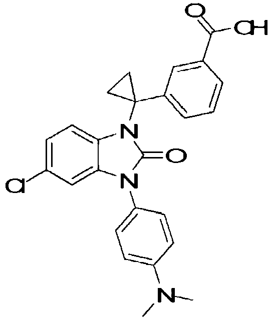

The title compound (20 mg, yield: 38%) was obtained in the same manner as in steps A and B of Example 1 by using 3-(1-(5-chloro-2-oxo-2,3-dihydro-1*H*-benzo[*d*]imidazol-1-yl)cyclopropyl)benzoic acid methyl ester (40 mg, 0.117 mmol) obtained in Preparation Example 3 and (4-dimethylamino)phenyl)boronic acid.
¹H NMR (500 MHz, CDCl₃) δ 7.96 (d, 1H), 7.92 (s, 1H), 7.56 (d, 1H), 7.41 (t, 1H), 7.35 (d, 2H), 7.09 (dd, 2H), 7.03 (s, 1H), 7.85 (m, 2H), 3.04 (s, 6H), 1.74 (m, 4H)

### Example 70: Preparation of 3-(1-(5-chloro-3-(4-(dimethylamino)phenyl)-2-oxo-2,3-dihydro-1H-benzo[d]imidazol-1-yl)cyclopropyl)-2-fluorobenzoic acid

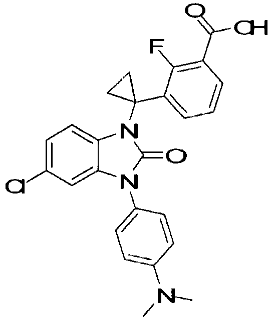

The title compound (10 mg, yield: 19%) was obtained in the same manner as in steps A and B of Example 1 by using 3-(1-(5-chloro-2-oxo-2,3-dihydro-*1H*-benzo[*d*]imidazol-1-yl)cyclopropyl)-2-fluorobenzoic acid methyl ester (40 mg, 0.111 mmol) obtained in Preparation Example 4 and (4-dimethylamino)phenyl)boronic acid.
¹H NMR (500 MHz, CDCl₃) δ 8.26 (t, 1H), 7.95 (t, 1H), 7.53 (d, 1H), 7.24 (m, 3H), 7.14 (d, 1H), 6.95 (s, 1H), 6.81 (d, 2H), 3.01 (s, 6H), 1.69 (m, 4H)

### Example 71: Preparation of 3-(1-(5-chloro-3-(4-(dimethylcarbamoyl)phenyl)-2-oxo-2,3-dihydro-1H-benzo[d]imidazol-1-yl)cyclopropyl)benzoic acid

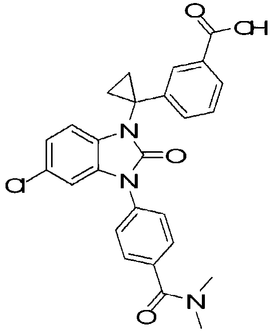

The title compound (20 mg, yield: 36%) was obtained in the same manner as in steps A and B of Example 1 by using 3-(1-(5-chloro-2-oxo-2,3-dihydro-1*H*-benzo[*d*]imidazol-1-yl)cyclopropyl)benzoic acid methyl ester (40 mg, 0.117 mmol) obtained in Preparation Example 3 and (4-dimethylcarbamoyl)phenyl)boronic acid.
¹H NMR (500 MHz, CDCl₃) δ 7.97 (d, 1H), 7.94 (s, 1H), 7.63 (m, 4H), 7.55 (d, 1H), 7.41 (t, 1H), 7.15 (m, 3H), 3.13 (d, 6H), 1.74 (m, 4H).

### Example 72: Preparation of 3-(1-(5-chloro-3-(4-(dimethylcarbamoyl)phenyl)-2-oxo-2,3-dihydro-1H-benzo[d]imidazol-1-yl)cyclopropyl)-2-fluorobenzoic acid

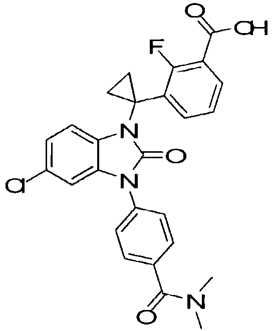

The title compound (25 mg, yield: 46%) was obtained in the same manner as in steps A and B of Example 1 by using 3-(1-(5-chloro-2-oxo-2,3-dihydro-*1H*-benzo[*d*]imidazol-1-yl)cyclopropyl)-2-fluorobenzoic acid methyl ester (40 mg, 0.111 mmol) obtained in Preparation Example 4 and (4-dimethylcarbamoyl)phenyl)boronic acid.
¹H NMR (500 MHz, CDCl₃) δ 8.22 (t, 1H), 7.95 (t, 1H), 7.58 (m, 5H), 7.23 (t, 1H), 7.19 (d, 1H), 7.05 (s, 1H), 3.11 (d, 6H), 1.69 (m, 4H)

### Example 73: Preparation of 3-((5-chloro-2-oxo-3-(pyridin-3-yl)-2,3-dihydro-1H-benzo[d]imidazol-1-yl)methyl)benzoic acid

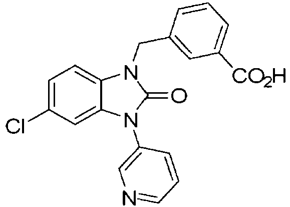

The process of the following steps A and B gave the title compound.

### Step A: Preparation of 3-((5-chloro-2-oxo-3-(pyridin-3-yl)-2,3-dihydro-1H-benzo[d]imidazol-1-yl)methyl)benzoic acid methyl ester

The title compound (11 mg, yield: 18%) was obtained in the same manner as in step A of Example 2 by using 3-((5-chloro-2-oxo-2,3-dihydro-1*H*-benzo[*d*]imidazol-1-yl)methyl)benzoic acid methyl ester (50 mg, 0.158 mmol) obtained in Preparation Example 1 and pyridin-3-ylboronic acid (19.4 mg, 0.158 mmol).
MS [M+H] = 394 (M+1)
¹H NMR (400 MHz, CD₃OD) δ8.89 (s, 1H), 8.70 (s, 1H), 8.04 (s, 1H), 7.98 (dt, 1H), 7.94 (d, 1H), 7.56 (d, 2H), 7.42 (t, 1H), 7.09 (d, 1H), 7.04 (dd, 1H), 6.82 (d, 1H), 5.19 (s, 2H), 3.90 (s, 3H)

### Step B: Preparation of 3-((5-chloro-2-oxo-3-(pyridin-3-yl)-2,3-dihydro-1H-benzo[d]imidazol-1-yl)methyl)benzoic acid

The title compound (10 mg, yield: 94%) was obtained in the same manner as in step B of Example 2 by using 3-((5-chloro-2-oxo-3-(pyridin-3-yl)-2,3-dihydro-1*H-*benzo[*d*]imidazol-1-yl)methyl)benzoic acid methyl ester (11 mg, 0.028 mmol) obtained in step A above.
MS [M+H] = 380 (M+1)
¹H NMR (500 MHz, DMSO-D6) δ8.80 (d, 1H), 8.63 (dd, 1H), 8.05 (dq, 1H), 7.95 (s, 1H), 7.82 (d, 1H), 7.60 (dd, 2H), 7.44 (t, 1H), 7.25 (d, 1H), 7.15 (dd, 1H), 7.10 (d, 1H), 5.18 (s, 2H)

### Example 74: Preparation of 3-((5-chloro-3-(6-fluoropyridin-3-yl)-2-oxo-2,3-dihydro-1H-benzo[d]imidazol-1-yl)methyl)benzoic acid

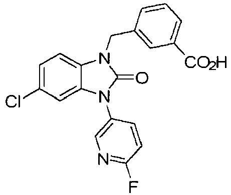

The process of the following steps A and B gave the title compound.

### Step A: Preparation of 3-((5-chloro-3-(6-fluoropyridin-3-yl)-2-oxo-2,3-dihydro-1H-benzo[d]imidazol-1-yl)methyl)benzoic acid methyl ester

The title compound (64 mg, yield: 41%) was obtained in the same manner as in step A of Example 2 by using methyl 3-((5-chloro-2-oxo-2,3-dihydro-1*H*-benzo[*d*]imidazol-1-yl)methyl)benzoic acid methyl ester (120 mg, 0.379 mmol) obtained in Preparation Example 1 and (6-fluoropyridin-3-yl)boronic acid (107 mg, 0.758 mmol).
MS [M+H] = 412 (M+1)
¹H NMR (400 MHz, CD₃OD) δ8.45 (s, 1H), 8.03 (m, 2H), 7.98 (d, 1H), 7.53 (d, 1H), 7.42 (t, 1H), 7.15 (dd, 1H), 7.07 (d, 1H), 7.04 (d, 1H), 6.84 (d, 1H), 5.15 (s, 2H), 3.90 (s, 3H)

### Step B: Preparation of 3-((5-chloro-3-(6-fluoropyridin-3-yl)-2-oxo-2,3-dihydro-1H-benzo[d]imidazol-1-yl)methyl)benzoic acid

The title compound (58 mg, yield: 94%) was obtained in the same manner as in step B of Example 2 by using 3-((5-chloro-3-(6-fluoropyridin-3-yl)-2-oxo-2,3-dihydro-1*H-*benzo[*d*]imidazol-1-yl)methyl)benzoic acid methyl ester (64 mg, 0.155 mmol) obtained in step A above.
MS [M+H] = 398 (M+1)
¹H NMR (400 MHz, DMSO-D6) δ8.49 (d, 1H), 8.25 (ddd, 1H), 7.96 (s, 1H), 7.83 (m, 1H), 7.59 (d, 1H), 7.44 (t, 1H), 7.40 (dd, 1H), 7.23 (m, 1H), 7.14 (m, 2H), 5.18 (s, 2H)

### Example 75: Preparation of 3-((5-chloro-3-(6-ethoxypyridin-3-yl)-2-oxo-2,3-dihydro-1H-benzo[d]imidazol-1-yl)methyl)benzoic acid

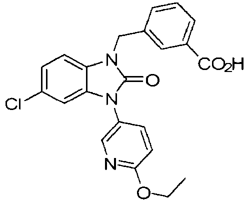

To 3-((5-chloro-3-(6-fluoropyridin-3-yl)-2-oxo-2,3-dihydro-1*H*-benzo[*d*]imidazol-1-yl)methyl)benzoic acid (25 mg, 0.063 mmol) obtained in Example 74 was added 2 mL of DMF, and then NaOH (2.41 mg, 0.101 mmol) was added thereto at 0 °C, followed by stirring for 30 minutes. Then, EtOH (4.34 mg, 0.094 mmol) was added thereto, and the resulting mixture was stirred at room temperature for 16 hours. After the reaction was terminated, water was added thereto, and an organic layer was separated and dried over Na₂SO₄. The resulting solid was removed, and the filtrate was distilled under reduced pressure and then separated by MPLC to give the title compound (17 mg, yield: 63%).
MS [M+H] = 424 (M+1)
¹H NMR (400 MHz, CD₃OD) δ8.33 (d, 1H), 7.94 (s, 1H), 7.89 (dd, 1H), 7.82 (d, 1H), 7.57 (d, 1H), 7.44 (t, 1H), 7.21 (d, 1H), 7.12 (dd, 1H), 6.98 (d, 1H), 6.96 (d, 1H), 5.16 (s, 2H), 4.34 (q, 2H), 1.32 (t, 3H)

### Example 76: Preparation of 3-((5-chloro-2-oxo-3-(6-propoxypyridin-3-yl)-2,3-dihydro-1H-benzo[d]imidazol-1-yl)methyl)benzoic acid

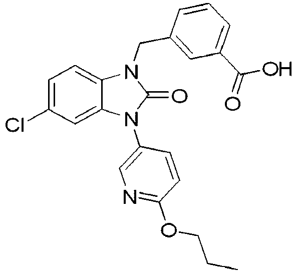

The title compound (0.10 g, yield: 35%) was obtained in the same manner as in steps A and B of Example 24 by using 6-chloro-1-(6-propoxypyridin-3-yl)-1,3-dihydro-2*H-*benzo[d]imidazol-2-one (0.20 g, 0.66 mmol) obtained in Preparation Example 7 and 3-(bromomethyl)benzoic acid methyl ester (0.17 g, 0.72 mmol).
MS [M-H] = 436 (M-1)
¹H-NMR (400 MHz, CDCl3) δ8.32 (d, 1H), 8.10 (d, 1H), 8.03 (dd, 1H), 7.74 (dd, 1H), 7.60 (m, 1H), 7.46 (t, 1H), 7.03 (dd, 1H), 7.00 (d, 1H), 6.91 (d, 1H), 6.83 (d, 1H), 5.17 (s, 2H), 4.32 (t, 2H), 1.83 (m, 2H), 1.05 (t, 3H)

### Example 77: Preparation of 3-((5-chloro-3-(6-(3-hydroxyazetidin-1-yl)pyridin-3-yl)-2-oxo-2,3-dihydro-1H-benzo[d]imidazol-1-yl)methyl)benzoic acid

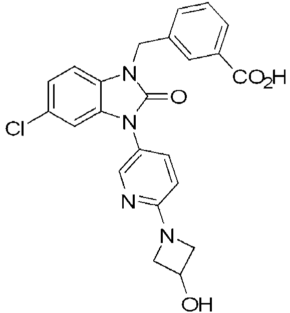

The process of the following steps A and B gave the title compound.

### Step A: Preparation of 3-((5-chloro-3-(6-(3-hydroxyazetidin-1-yl)pyridin-3-yl)-2-oxo-2,3-dihydro-1H-benzo[d]imidazol-1-yl)methyl)benzoic acid methyl ester

The title compound (0.020 g, yield: 27%) was obtained in the same manner as in step A of Example 24 by using 3-((5-chloro-3-(6-fluoropyridin-3-yl)-2-oxo-2,3-dihydro-1*H-*benzo[*d*]imidazol-1-yl)methyl)benzoic acid methyl ester (0.065 g, 0.16 mmol) obtained in step A of Example 74 and azetidin-3-ol hydrochloride (0.052 g, 0.47 mmol).
¹H-NMR (400 MHz, CDCl3) δ8.28 (m, 1H), 8.05 (m, 1H), 7.97 (m, 1H), 7.59 (m, 2H), 7.43 (m, 1H), 7.02 (m, 1H), 6.96 (m, 1H), 6.80 (m, 1H), 6.45 (m, 1H), 5.15 (s, 2H), 4.85 (m, 1H), 4.38 (m, 2H), 3.97 (m, 2H), 3.92 (s, 3H)

### Step B: Preparation of 3-((5-chloro-3-(6-(3-hydroxyazetidin-1-yl)pyridin-3-yl)-2-oxo-2,3-dihydro-1H-benzo[d]imidazol-1-yl)methyl)benzoic acid

The title compound (0.01 g, yield: 52%) was obtained in the same manner as in step B of Example 24 by using 3-((5-chloro-3-(6-(3-hydroxyazetidin-1-yl)pyridin-3-yl)-2-oxo-2,3-dihydro-1*H*-benzo[*d*]imidazol-1-yl)methyl)benzoic acid methyl ester (0.02 g, 0.043 mmol) obtained in step A above.
MS [M+H] = 451 (M+1)
¹H-NMR (400 MHz, MeOH-d4) δ8.18 (d, 1H), 7.03 (d, 1H), 7.96 (dd, 1H), 7.69 (dd, 1H), 7,61 (dd, 1H), 7.47 (t, 1H), 7.10 (2H), 6.95 (d, 1H), 6.61 (d, 1H), 5.22 (s, 2H), 4.70 (m, 1H), 4.35 (m, 2H), 3.91 (m, 2H)

### Example 78: Preparation of 3-((5-chloro-3-(6-morpholinopyridin-3-yl)-2-oxo-2,3-dihydro-1H-benzo[d]imidazol-1-yl)methyl)benzoic acid

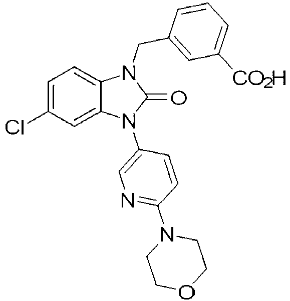

The process of the following steps A and B gave the title compound.

### Step A: Preparation of 3-((5-chloro-3-(6-morpholinopyridin-3-yl)-2-oxo-2,3-dihydro-1H-benzo[d]imidazol-1-yl)methyl)benzoic acid methyl ester

The title compound (0.063 g, yield: 45%) was obtained in the same manner as in step A of Example 24 by using 3-((5-chloro-3-(6-fluoropyridin-3-yl)-2-oxo-2,3-dihydro-1*H-*benzo[*d*]imidazol-1-yl)methyl)benzoic acid methyl ester (0.12 g, 0.29 mmol) obtained in step A of Example 74 and morpholine (0.051 g, 0.58 mmol).
¹H-NMR (400 MHz, CDCl3) δ 8.33 (dd, 1H), 8.05 (d, 1H), 7.98 (dd, 1H), 7.65 (dd, 1H), 7.56 (dd, 1H), 7.43 (t, 1H), 7.00 (2H), 6.78 (2H), 5.15 (s, 2H), 3.92 (s, 3H), 3.85 (m, 4H), 3.59 (m, 4H)

### Step B: Preparation of 3-((5-chloro-3-(6-morpholinopyridin-3-yl)-2-oxo-2,3-dihydro-1H-benzo[d]imidazol-1-yl)methyl)benzoic acid

The title compound (0.052 g, 89%) was obtained in the same manner as in step B of Example 24 by using 3-((5-chloro-3-(6-morpholinopyridin-3-yl)-2-oxo-2,3-dihydro-1*H-*benzo[d]imidazol-1-yl)methyl)benzoic acid methyl ester (0.06 g, 0.12 mmol) obtained in step A above.
MS [M+H] = 465 (M+1)
¹H-NMR (400 MHz, CDCl3) δ8.34 (d, 1H), 8.09 (d, 1H), 8.03 (dd, 1H), 7.65 (dd, 1H), 7.60 (dd, 1H), 7.49 (t, 1H), 7.03 (dd, 1H), 7.01 (d, 1H), 6.80 (2H), 5.17 (s, 2H), 3.86 (m, 4H), 3.50 (m, 4H)

### Example 79: Preparation of 3-((5-chloro-3-(6-(dimethylamino)pyridin-3-yl)-2-oxo-2,3-dihydro-1H-benzo[d]imidazol-1-yl)methyl)benzoic acid

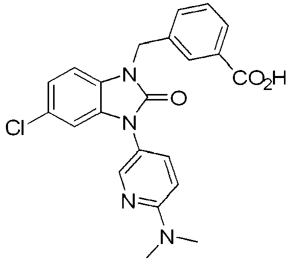

The process of the following steps A and B gave the title compound.

### Step A: Preparation of 3-((5-chloro-3-(6-(dimethylamino)pyridin-3-yl)-2-oxo-2,3-dihydro-1H-benzo[d]imidazol-1-yl)methyl)benzoic acid methyl ester

The title compound (0.022 g, yield: 30%) was obtained in the same manner as in step A of Example 24 by using 3-((5-chloro-3-(6-fluoropyridin-3-yl)-2-oxo-2,3-dihydro-1*H-*benzo[*d*]imidazol-1-yl)methyl)benzoic acid methyl ester (0.07 g, 0.17 mmol) obtained in step A of Example 74 and dimethylamine hydrochloride (0.042 g, 0.51 mmol).
¹H-NMR (400 MHz, CDCl3) δ 8.28 (dd, 1H), 8.06 (m, 1H), 8.00 (dd, 1H), 7.55 (2H), 7.43 (t, 1H), 6.98 (2H), 6.78 (d, 1H), 6.64 (dd, 1H), 5.15 (s, 2H), 3.92 (s, 3H), 3.15 (s, 6H)

### Step B: Preparation of 3-((5-chloro-3-(6-(dimethylamino)pyridin-3-yl)-2-oxo-2,3-dihydro-1H-benzo[d]imidazol-1-yl)methyl)benzoic acid

The title compound (0.018 g, yield: 85%) was obtained in the same manner as in step B of Example 24 by using 3-((5-chloro-3-(6-(dimethylamino)pyridin-3-yl)-2-oxo-2,3-dihydro-1*H*-benzo[*d*]imidazol-1-yl)methyl)benzoic acid methyl ester (0.022 g, 0.05 mmol) obtained in step A above.
MS [M+H] = 423 (M+1)
¹H-NMR (400 MHz, CDCl3) δ8.35 (d, 1H), 8.07 (d, 1H), 8.02 (dd, 1H), 7.70 (m, 1H), 7.59 (dd, 1H), 7.45 (t, 1H), 7.03 (dd, 1H), 7.00 (d, 1H), 6.83 (d, 1H), 6.74 (m, 1H), 5.16 (s, 2H), 3.25 (s, 6H)

### Example 80: Preparation of 3-((5-chloro-3-(6-(3,3-difluoropyrrolidin-1-yl)pyridin-3-yl)-2-oxo-2,3-dihydro-1H-benzo[d]imidazol-1-yl)methyl)benzoic acid

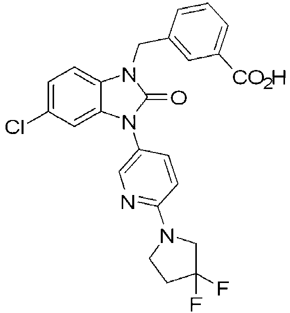

The title compound (0.005 g, yield: 23%) was obtained in the same manner as in steps A and B of Example 24 by using 3-((5-chloro-3-(6-fluoropyridin-3-yl)-2-oxo-2,3-dihydro-1*H-*benzo[*d*]imidazol-1-yl)methyl)benzoic acid methyl ester (0.07 g, 0.17 mmol) obtained in step A of Example 74 and 3,3-difluoropyrrolidine hydrochloride (0.066 g, 0.51 mmol).
MS [M+H] = 485 (M+1)
¹H-NMR (400 MHz, CDCl3) δ8.34 (d, 1H), 8.09 (d, 1H), 8.03 (dd, 1H), 7.65 (dd, 1H), 7.60 (dd, 1H), 7.46 (t, 1H), 7.02 (dd, 1H), 6.97 (d, 1H), 6.81 (d, 1H), 6.53 (d, 1H), 5.17 (s, 2H), 3.92 (t, 2H), 3.76 (t, 2H), 2.54 (m, 2H)

### Example 81: Preparation of 3-((5-chloro-3-(6-(4-methylpiperazin-1-yl)pyridin-3-yl)-2-oxo-2,3-dihydro-1H-benzo[d]imidazol-1-yl)methyl)benzoic acid

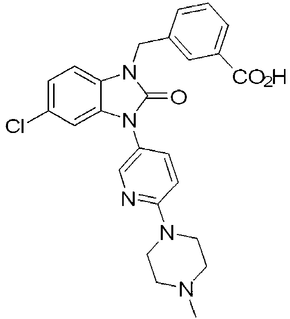

The process of the following steps A and B gave the title compound.

### Step A: Preparation of 3-((5-chloro-3-(6-(4-methylpiperazin-1-yl)pyridin-3-yl)-2-oxo-2,3-dihydro-1H-benzo[d]imidazol-1-yl)methyl)benzoic acid methyl ester

The title compound (0.033 g, yield: 40%) was obtained in the same manner as in step A of Example 24 by using 3-((5-chloro-3-(6-fluoropyridin-3-yl)-2-oxo-2,3-dihydro-1*H-*benzo[*d*]imidazol-1-yl)methyl)benzoic acid methyl ester (0.07 g, 0.17 mmol) obtained in step A of Example 74 and 1-methylpiperazine (0.051 g, 0.051 mmol).
¹H-NMR (400 MHz, CDCl3) δ8.31 (d, 1H), 8.05 (d, 1H), 7.97 (dd, 1H), 7.63 (dd, 1H), 7.56 (dd, 1H), 7.42 (t, 1H), 7.00 (2H), 6.78 (2H), 5.15 (s, 2H), 3.92 (s, 3H), 3.66 (m, 4H), 2.55 (m, 4H), 2.37 (s, 3H)

### Step B: Preparation of 3-((5-chloro-3-(6-(4-methylpiperazin-1-yl)pyridin-3-yl)-2-oxo-2,3-dihydro-1H-benzo[d]imidazol-1-yl)methyl)benzoic acid

The title compound (0.028 g, 85%) was obtained in the same manner as in step B of Example 24 by using 3-((5-chloro-3-(6-(4-methylpiperazin-1-yl)pyridin-3-yl)-2-oxo-2,3-dihydro-1*H*-benzo[*d*]imidazol-1-yl)methyl)benzoic acid methyl ester (0.033 g, 0.067 mmol) obtained in step A above.
MS [M+H] = 478 (M+1)
¹H-NMR (400 MHz, DMSO-d6) δ8.26 (d, 1H), 7.96 (d, 1H), 7.84 (dd, 1H), 7.71 (dd, 1H), 7.60 (dd, 1H), 7.50 (t, 1H), 7.20 (d, 1H), 7.13 (d, 1H), 7.01 (d, 1H), 6.93 (d, 1H), 5.18 (s, 2H), 3.60 (m, 4H), 2.52 (m, 4H), 2.31 (s, 3H)

### Example 82: Preparation of 3-((5-chloro-3-(6-(4,4-difluoropiperidin-1-yl)pyridin-3-yl)-2-oxo-2,3-dihydro-1H-benzo[d]imidazol-1-yl)methyl)benzoic acid

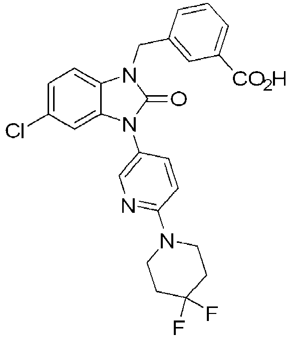

The process of the following steps A and B gave the title compound.

### Step A: Preparation of 3-((5-chloro-3-(6-(4,4-difluoropiperidin-1-yl)pyridin-3-yl)-2-oxo-2,3-dihydro-1H-benzo[d]imidazol-1-yl)methyl)benzoic acid methyl ester

The title compound (0.032 g, yield: 37%) was obtained in the same manner as in step A of Example 24 by using 3-((5-chloro-3-(6-fluoropyridin-3-yl)-2-oxo-2,3-dihydro-1*H-*benzo[*d*]imidazol-1-yl)methyl)benzoic acid methyl ester (0.07 g, 0.17 mmol) obtained in step A of Example 74 and 4,4-difluoropiperidine hydrochloride (0.070 g, 0.51 mmol).
¹H-NMR (400 MHz, CDCl3) δ8.32 (d, 1H), 8.05 (d, 1H), 7.98 (dd, 1H), 7.65 (dd, 1H), 7.55 (dd, 1H), 7.43 (t, 1H), 7.02 (2H), 6.85 (d, 1H), 6.80 (d, 1H), 5.15 (s, 2H), 3.92 (s, 3H), 3.82 (m, 4H), 2.05 (m, 4H)

### Step B: Preparation of 3-((5-chloro-3-(6-(4,4-difluoropiperidin-1-yl)pyridin-3-yl)-2-oxo-2,3-dihydro-1H-benzo[d]imidazol-1-yl)methyl)benzoic acid

The title compound (10 mg, yield: 32%) was obtained in the same manner as in step B of Example 24 by using 3-((5-chloro-3-(6-(4,4-difluoropiperidin-1-yl)pyridin-3-yl)-2-oxo-2,3-dihydro-1*H*-benzo[d]imidazol-1-yl)methyl)benzoic acid methyl ester (0.032 g, 0.17 mmol) obtained in step A above.
MS [M+H] = 499 (M+1)
¹H-NMR (400 MHz, CDCl3) δ8.33 (d, 1H), 8.10 (d, 1H), 8.04 (dd, 1H), 7.66 (dd, 1H), 7.63 (dd, 1H), 7.46 (t, 1H), 7.03 (dd, 1H), 7.00 (d, 1H), 6.85 (d, 1H), 6.82 (d, 1H), 5.18 (s, 2H), 3.82 (m, 4H), 2.05 (m, 4H)

### Example 83: Preparation of 3-((5-chloro-3-(2-morpholinopyrimidin-5-yl)-2-oxo-2,3-dihydro-1H-benzo[d]imidazol-1-yl)methyl)benzoic acid

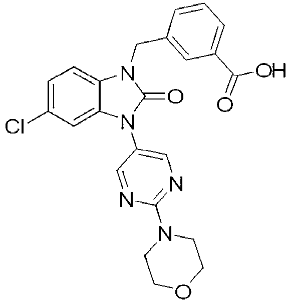

The title compound (0.040 g, yield: 63%) was obtained in the same manner as in steps A and B of Example 24 by using 6-chloro-1-(2-morpholinopyrimidin-5-yl)-1,3-dihydro-2*H-*benzo[*d*]imidazol-2-one (0.045 g, 0.136 mmol) obtained in Preparation Example 26 and 3-(bromomethyl)benzoic acid methyl ester (0.031 g, 0.136 mmol).
MS [M+H] = 466 (M+1)
¹H-NMR (400 MHz, MeOH-d4) δ8.52 (s, 2H), 8.03 (d, 1H), 7.95 (dd, 1H), 7.60 (dd, 1H), 7.47 (t, 1H), 7.11 (m, 2H), 7.01 (d, 1H), 5.22 (s, 2H), 3.88 (m, 4H), 3.77 (m, 4H)

### Example 84: Preparation of 3-((5-chloro-2-oxo-3-(1-propyl-1H-pyrazol-4-yl)-2,3-dihydro-1H-benzo[d]imidazol-1-yl)methyl)benzoic acid

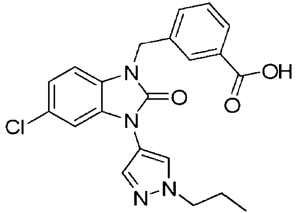

The process of the following steps A and B gave the title compound.

### Step A: Preparation of 3-((5-chloro-2-oxo-3-(1-propyl-1H-pyrazol-4-yl)-2,3-dihydro-1H-benzo[d]imidazol-1-yl)methyl)benzoic acid methyl ester

The title compound (0.011 g, yield: 46%) was obtained in the same manner as in step A of Example 21 by using 6-chloro-1-(1-propyl-1*H*-pyrazol-4-yl)-1,3-dihydro-2H-benzo[*d*]imidazol-2-one (0.015 g, 0.054 mmol) obtained in Preparation Example 12 and 3-(bromomethyl)benzoic acid methyl ester (0.013 g, 0.054 mmol).
¹H-NMR (500 MHz, CDCl3) δ8.00 (2H), 7.82 (s, 1H), 7.78 (s, 1H), 7.53 (m, 1H), 7.40 (m, 1H), 7.11 (s, 1H), 7.02 (d, 1H), 6.79 (d, 1H), 5.14 (s, 2H), 4.16 (t, 2H), 3.91 (s, 3H), 1.97 (m, 2H), 0.99 (t, 3H)

### Step B: Preparation of 3-((5-chloro-2-oxo-3-(1-propyl-1H-pyrazol-4-yl)-2,3-dihydro-1H-benzo[d]imidazol-1-yl)methyl)benzoic acid

The title compound (5 mg, yield: 61%) was obtained in the same manner as in step B of Example 24 by using 3-((5-chloro-2-oxo-3-(1-propyl-1H-pyrazol-4-yl)-2,3-dihydro-1H-benzo[*d*]imidazol-1-yl)methyl)benzoic acid methyl ester (8.5 mg, 0.02 mmol) obtained in step A above.
MS [M+H] = 411 (M+1)
¹H-NMR (400 MHz, CDCl3) δ8.00 (m, 1H), 7.97 (m, 1H), 7.75 (s, 1H), 7.75 (s, 1H), 7.48 (m, 1H), 7.40 (m, 1H), 7.08 (m, 1H), 7.00 (m, 1H), 6.78 (m, 1H), 5.11 (s, 2H), 4.12 (t, 2H), 1.94 (m, 2H), 0.96 (t, 3H)

### Example 85: Preparation of 3-((5-cyclopropyl-2-oxo-3-(1-(tetrahydro-2H-pyran-4-yl)-1H-pyrazol-4-yl)-2,3-dihydro-1H-benzo[d]imidazol-1-yl)methyl)benzoic acid

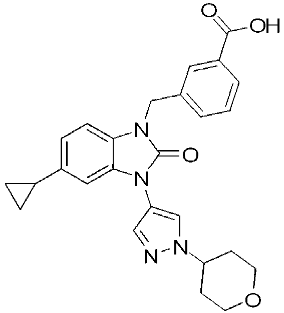

The title compound (0.020 g, yield: 20%) was obtained in the same manner as in steps A and B of Example 24 by using 6-cyclopropyl-1-(1-(tetrahydro-2*H*-pyran-4-yl)-1*H*-pyrazol-4-yl)-1,3-dihydro-2*H*-benzo[*d*]imidazol-2-one (0.075 g, 0.23 mmol) obtained in Preparation Example 17 and 3-(bromomethyl)benzoic acid methyl ester (0.064 g, 0.28 mmol).
MS [M+H] = 459 (M+1)
¹H-NMR (400 MHz, CDCl3) δ8.10 (m, 1H), 8.01 (m, 1H), 7.91 (s, 1H), 7.83 (s, 1H), 7.58 (m, 1H), 7.43 (t, 1H), 6.88 (m, 1H), 6.80 (m, 2H), 5.15 (s, 2H), 4.42 (m, 1H), 4.15 (m, 2H), 3.57 (m, 2H), 2.17 (m, 4H), 1.90 (m, 1H), 0.92 (m, 2H), 0.63 (m, 2H)

### Example 86: Preparation of 3-((5-chloro-2-oxo-3-(1-(tetrahydro-2H-pyran-4-yl)-1H-pyrazol-4-yl)-2,3-dihydro-1H-benzo[d]imidazol-1-yl)methyl)benzoic acid

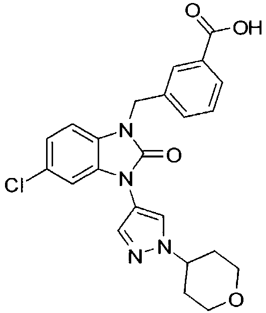

The process of the following steps A and B gave the title compound.

### Step A: Preparation of 3-((5-chloro-2-oxo-3-(1-(tetrahydro-2H-pyran-4-yl)-1H-pyrazol-4-yl)-2,3-dihydro-1H-benzo[d]imidazol-1-yl)methyl)benzoic acid methyl ester

The title compound (0.090 g, yield: 56%) was obtained in the same manner as in step A of Example 24 by using 6-chloro-1-(1-(tetrahydro-2*H*-pyran-4-yl)-1*H*-pyrazol-4-yl)-1,3-dihydro-2*H*-benzo[d]imidazol-2-one (0.11 g, 0.34 mmol) obtained in Preparation Example 9 and 3-(bromomethyl)benzoic acid methyl ester (0.10 g, 0.45 mmol).
¹H-NMR (400 MHz, CDCl3) δ8.03 (m, 1H), 7.98 (dd, 1H), 7.87 (s, 1H), 7.80 (s, 1H), 7.53 (dd, 1H), 7.42 (t, 1H), 7.11 (d, 1H), 7.03 (dd, 1H), 6.80 (d, 1H), 5.14 (s, 2H), 4.40 (m, 1H), 4.14 (m, 2H), 3.91 (s, 3H), 3.60 (m, 2H), 2.15 (m, 4H)

### Step B: Preparation of 3-((5-chloro-2-oxo-3-(1-(tetrahydro-2H-pyran-4-yl)-1H-pyrazol-4-yl)-2,3-dihydro-1H-benzo[d]imidazol-1-yl)methyl)benzoic acid

The title compound (0.035 g, yield: 40%) was obtained in the same manner as in step B of Example 24 by using 3-((5-chloro-2-oxo-3-(1-(tetrahydro-2*H*-pyran-4-yl)-1*H*-pyrazol-4-yl)-2,3-dihydro-1*H*-benzo[d]imidazol-1-yl)methyl)benzoic acid methyl ester (0.09 g, 0.19 mmol) obtained in step A above.
MS [M+H] = 453 (M+1)
¹H-NMR (400 MHz, CDCl3) δ8.19 (d, 1H), 8.03 (dd, 1H), 7.89 (s, 1H), 7.81 (s, 1H), 7.58 (m, 1H), 7.48 (t, 1H), 7.12 (d, 1H), 7.06 (dd, 1H), 6.82 (d, 1H), 5.17 (s, 2H), 4.41 (m, 1H), 4.14 (m, 2H), 3.58 (m, 2H), 2.17 (m, 4H)

### Example 87: Preparation of 3-((5-chloro-2-oxo-3-(1-(2,2,2-trifluoroethyl)-1H-pyrazol-4-yl)-2,3-dihydro-1H-benzo[d]imidazol-1-yl)methyl)benzoic acid

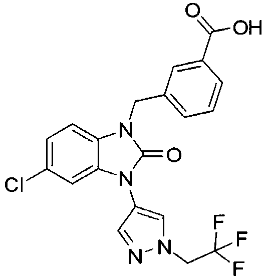

The process of the following steps A, B, and C gave the title compound.

### Step A: Preparation of N-(5-chloro-2-nitrophenyl)-1-(2,2,2-trifluoroethyl)-1H-pyrazol-4-amine

N-(5-chloro-2-nitrophenyl)-1*H*-pyrazol-4-amine (0.16 g, 0.67 mmol) obtained in Preparation Example 10 was dissolved in 3 mL of DMF, then cooled to 0 °C, NaH (0.021 g, 0.87 mmol) was added thereto, and then the resulting mixture was stirred for 20 minutes. To the mixture was added 4-methylbenzenesulfonic acid 2,2,2-trifluoroethyl ester (0.20 g, 0.80 mmol), and the resultant mixture was stirred at room temperature for 16 hours. After the reaction was terminated, an aqueous ammonium chloride solution was added thereto, and then the resulting solution was extracted with EtOAc. An organic layer was separated and purified by MPLC to give the title compound (0.069 g, yield: 32%).
¹H-NMR (400 MHz, CDCl3) δ9.19 (brs, 1H), 8.16 (d, 1H), 7.63 (s, 1H), 7.60 (s, 1H), 6.75 (d, 1H), 6.73 (dd, 1H), 4.74 (q, 2H)

### Step B: Preparation of 6-chloro-1-(1-(2,2,2-trifluoroethyl)-1H-pyrazol-4-yl)-1,3-dihydro-2H-benzo[d]imidazol-2-one

The title compound (0.024 g, yield: 37%) was obtained in the same manner as in step B of Preparation Example 7 and step C of Preparation Example 3 by using N-(5-chloro-2-nitrophenyl)-1-(2,2,2-trifluoroethyl)-1H-pyrazol-4-amine (0.065 g, 0.20 mmol) obtained in step A above.
¹H-NMR (400 MHz, CDCl3) δ8.19 (brs, 1H), 7.97 (s, 1H), 7.90 (s, 1H), 7.12 (2H), 7.00 (d, 1H), 4.78 (q, 2H)

### Step C: Preparation of 3-((5-chloro-2-oxo-3-(1-(2,2,2-trifluoroethyl)-1H-pyrazol-4-yl)-2,3-dihydro-1H-benzo[d]imidazol-1-yl)methyl)benzoic acid

The title compound (0.010 g, yield: 29%) was obtained in the same manner as in steps A and B of Example 24 by using 6-chloro-1-(1-(2,2,2-trifluoroethyl)-1*H*-pyrazol-4-yl)-1,3-dihydro-2*H*-benzo[*d*]imidazol-2-one (0.024 g, 0.076 mmol) obtained in step B above and 3-(bromomethyl)benzoic acid methyl ester (0.017 g, 0.076 mmol).
MS [M+H] = 451 (M+1)
¹H-NMR (400 MHz, CDCl3) δ8.17-8.02 (3H), 7.92 (s, 1H), 7.58 (m, 1H), 7.47 (m, 1H), 7.15 (d, 1H), 7.06 (dd, 1H), 6.83 (d, 1H), 5.15 (s, 2H), 4.80 (q, 2H)

### Example 88: Preparation of 3-((5-chloro-4-fluoro-2-oxo-3-(1-propyl-1H-pyrazol-4-yl)-2,3-dihydro-1H-benzo[d]imidazol-1-yl)methyl)benzoic acid

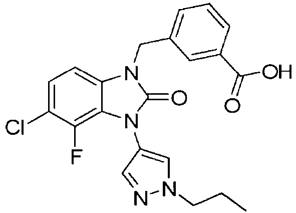

The title compound (0.097 g, yield: 80%) was obtained in the same manner as in steps A and B of Example 24 by using 6-chloro-7-fluoro-1-(1-propyl-1*H*-pyrazol-4-yl)-1,3-dihydro-2*H*-benzo[*d*]imidazol-2-one (0.083 g, 0.28 mmol) obtained in Preparation Example 13 and 3-(bromomethyl)benzoic acid methyl ester (0.065 g, 0.28 mmol).
MS [M+H] = 430 (M+1)
¹H-NMR (400 MHz, MeOH-d4) δ8.03 (m, 1H), 8.01 (m, 1H), 7.95 (dd, 1H), 7.74 (d, 1H), 7.57 (m, 1H), 7.47 (t, 1H), 7.16 (dd, 1H), 6.94 (d, 1H), 5.21 (s, 2H), 4.18 (t, 2H), 1.92 (m, 2H), 0.95 (t, 3H)

### Example 89: Preparation of 3-((5-chloro-3-(1-isopropyl-1H-pyrazol-4-yl)-2-oxo-2,3-dihydro-1H-benzo[d]imidazol-1-yl)methyl)benzoic acid

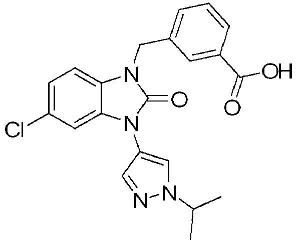

The process of the following steps A and B gave the title compound.

### Step A: Preparation of 3-((5-chloro-3-(1-isopropyl-1H-pyrazol-4-yl)-2-oxo-2,3-dihydro-1H-benzo[d]imidazol-1-yl)methyl)benzoic acid methyl ester

The title compound (15.4 mg, yield: 67%) was obtained in the same manner as in step A of Example 1 by using 6-chloro-1-(1-isopropyl-1H-pyrazol-4-yl)-1,3-dihydro-2H-benzo[d]imidazol-2-one (15.6 mg, 0.054 mmol) obtained in Preparation Example 18 and 3-(bromomethyl)benzoic acid methyl ester.
MS [M+H] = 425 (M+1)

### Step B: Preparation of 3-((5-chloro-3-(1-isopropyl-1H-pyrazol-4-yl)-2-oxo-2,3-dihydro-1H-benzo[d]imidazol-1-yl)methyl)benzoic acid

The title compound (9.9 mg, yield: 68%) was obtained in the same manner as in step B of Example 1 by using methyl 3-((5-chloro-3-(1-isopropyl-1*H*-pyrazol-4-yl)-2-oxo-2,3-dihydro-1*H*-benzo[*d*]imidazol-1-yl)methyl)benzoic acid methyl ester (15.4 mg, 0.035 mmol) obtained in step A above.
MS [M+H] = 411 (M+1)
¹H NMR (400 MHz, CDCl₃) δ 8.09 (s, 1H), 8.04 (d, 1H), 7.87 (s, 1H), 7.80 (s, 1H), 7.59 (d, 1H), 7.46 (t, 1H), 7.12 (m, 1H), 7.04 (m, 1H), 6.83 (d, 1H), 5.16 (s, 1H), 4.63 (m, 1H), 1.59 (d, 6H)

### Example 90: Preparation of 3-((5-chloro-3-(1-cyclopropylmethyl-1H-pyrazol-4-yl)-2-oxo-2,3-dihydro-1H-benzo[d]imidazol-1-yl)methyl)benzoic acid

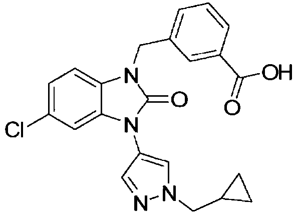

The process of the following steps A and B gave the title compound.

### Step A: Preparation of 3-((5-chloro-3-(1-(cyclopropylmethyl)-1H-pyrazol-4-yl)-2-oxo-2,3-dihydro-1H-benzo[d]imidazol-1-yl)methyl)benzoic acid methyl ester

The title compound (52.1 mg, yield: 63%) was obtained in the same manner as in step A of Preparation Example 1 by using 6-chloro-1-(1-(cyclopropylmethyl)-1*H*-pyrazol-4-yl)-1,3-dihydro-2*H*-benzo[d]imidazol-2-one (55.3 mg, 0.19 mmol) obtained in Preparation Example 19 and 3-(bromomethyl)benzoic acid methyl ester.
MS [M+H] = 437 (M+1)

### Step B: Preparation of 3-((5-chloro-3-(1-(cyclopropylmethyl)-1H-pyrazol-4-yl)-2-oxo-2,3-dihydro-1H-benzo[d]imidazol-1-yl)methyl)benzoic acid

The title compound (25.6 mg, yield: 52%) was obtained in the same manner as in step B of Example 1 by using 3-((5-chloro-3-(1-(cyclopropylmethyl)-1*H*-pyrazol-4-yl)-2-oxo-2,3-dihydro-1*H*-benzo[d]imidazol-1-yl)methyl)benzoic acid methyl ester (52.1 mg, 0.12 mmol) obtained in step A above.
MS [M+H] = 423 (M+1)
¹H NMR (500 MHz, CDCl₃) δ 8.07 (s, 1H), 8.03 (d, 1H), 7.95 (s, 1H), 7.78 (s, 1H), 7.59 (d, 1H), 7.47 (m, 1H), 7.13 (s, 1H), 7.04 (d, 1H), 6.81 (d, 1H), 5.16 (s, 2H), 4.07 (d, 2H), 1.36 (m, 1H), 0.88 (m, 2H), 0.45 (m, 2H)

### Example 91: Preparation of 3-((5-chloro-3-(1-cyclobutylmethyl-1H-pyrazol-4-yl)-2-oxo-2,3-dihydro-1H-benzo[d]imidazol-1-yl)methyl)benzoic acid

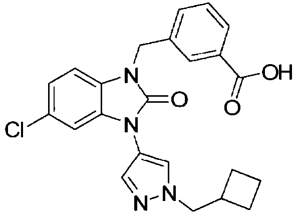

The process of the following steps A and B gave the title compound.

### Step A: Preparation of 3-((5-chloro-3-(1-(cyclobutylmethyl)-1H-pyrazol-4-yl)-2-oxo-2,3-dihydro-1H-benzo[d]imidazol-1-yl)methyl)benzoic acid methyl ester

The title compound (10.1 mg, yield: 68%) was obtained in the same manner as in step A of Example 1 by using 6-chloro-1-(1-(cyclobutylmethyl)-1*H*-pyrazol-4-yl)-1,3-dihydro-2*H-*benzo[*d*]imidazol-2-one (10.6 mg, 0.033 mmol) obtained in Preparation Example 20 and 3-(bromomethyl)benzoic acid methyl ester.
MS [M+H] = 451 (M+1)
¹H NMR (500 MHz, CDCl₃) δ8.02 (s, 1H), 7.97 (d, 2H), 7.79 (m, 2H), 7.54 (m, 1H), 7.43 (m, 1H), 7.11 (s, 1H), 7.02 (d, 1h), 6.79 (d, 1H), 5.14 (s, 2H), 4.21 (d, 2H), 3.91 (s, 3H), 2.91 (m, 1H), 2.16 (m, 2H), 1.97 (m, 4H)

### Step B: Preparation of 3-((5-chloro-3-(1-(cyclobutylmethyl)-1H-pyrazol-4-yl)-2-oxo-2,3-dihydro-1H-benzo[d]imidazol-1-yl)methyl)benzoic acid

The title compound (7.6 mg, yield: 79%) was obtained in the same manner as in step B of Example 1 by using 3-((5-chloro-3-(1-(cyclobutylmethyl)-1*H*-pyrazol-4-yl)-2-oxo-2,3-dihydro-1*H*-benzo[d]imidazol-1-yl)methyl)benzoic acid methyl ester (10.1 mg, 0.022 mmol) obtained in step A above.
MS [M+H] = 437 (M+1)
¹H NMR (500 MHz, CDCl₃) δ 8.08 (s, 1H), 8.04 (d, 1H), 7.80 (d, 2H), 7.58 (d, 2H), 7.46 (t, 1H), 7.12 (s, 1H), 7.04 (d, 1H), 6.82 (d, 1H), 5.16 (s, 2H), 4.22 (d, 2H), 2.92 (m, 1H), 2.14 (m, 2H), 1.96 (m, 4H)

### Example 92: Preparation of 3-((5-chloro-4-fluoro-2-oxo-3-(1-propyl-1H-pyrazol-4-yl)-2,3-dihydro-1H-benzo[d]imidazol-1-yl)methyl)-2-fluorobenzoic acid

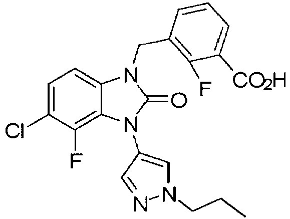

The title compound (0.052 g, yield: 61%) was obtained in the same manner as in steps A and B of Example 24 by using 6-chloro-1-(1-propyl-1*H*-pyrazol-4-yl)-1,3-dihydro-2H-benzo[*d*]imidazol-2-one (0.056 g, 0.19 mmol) obtained in Preparation Example 12 and 3-(bromomethyl)-2-fluorobenzoic acid methyl ester (0.047 g, 0.190 mmol).
MS [M+H] = 447 (M+1)
¹H-NMR (500 MHz, DMSO-d6) δ13.5 (brs, 1H), 8.13 (m, 1H), 7.69 (s, 1H), 7.65 (m, 1H), 7.28 (m, 1H), 7.25 (m, 1H), 7.16 (m, 1H), 7.07 (m, 1H), 5.17 (s, 2H), 4.11 (t, 2H), 1.82 (m, 2H), 0.84 (t, 3H)

### Example 93: Preparation of 3-(1-(3-(4-(7-oxa-2-azaspiro[3.5]nonane-2-carbonyl)phenyl)-5-chloro-2-oxo-2,3-dihydro-1H-benzo[d]imidazol-1-yl)cyclopropyl)benzoic acid

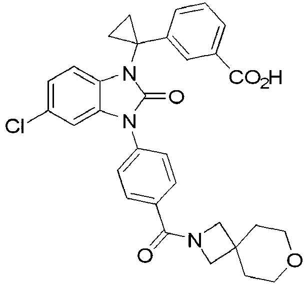

The process of the following steps A and B gave the title compound.

### Step A: Preparation of 3-(1-(3-(4-(7-oxa-2-azaspiro[3.5]nonane-2-carbonyl)phenyl)-5-chloro-2-oxo-2,3-dihydro-1H-benzo[d]imidazol-1-yl)cyclopropyl)benzoic acid methyl ester

The title compound (63 mg, yield: 51%) was obtained in the same manner as in step C of Example 56 by using 4-(6-chloro-3-(1-(3-(methoxycarbonyl)phenyl)cyclopropyl)-2-oxo-2,3-dihydro-1*H*-benzo[*d*]imidazol-1-yl)benzoic acid (100 mg, 0.22 mmol) obtained in step B of Example 63 and 7-oxa-2-azaspiro[3.5]nonane (32 mg, 0.26 mmol).
¹H NMR (400 MHz, CDCl₃) δ7.91 (m, 1H), 7.89 (m, 1H), 7.83 (d, 2H), 7.62 (d, 2H), 76.49 (m, 1H), 7.37 (t, 1H), 7.13 (3H), 4.13 (s, 2H), 4.03 (s, 2H), 3.91 (s, 3H), 3.65 (m, 4H), 1.84 (m, 4H), 1.70 (m, 4H)

### Step B: Preparation of 3-(1-(3-(4-(7-oxa-2-azaspiro[3.5]nonane-2-carbonyl)phenyl)-5-chloro-2-oxo-2,3-dihydro-1H-benzo[d]imidazol-1-yl)cyclopropyl)benzoic acid

The title compound (43 mg, yield: 70%) was obtained in the same manner as in step B of Example 2 by using 3-(1-(3-(4-(7-oxa-2-azaspiro[3.5]nonane-2-carbonyl)phenyl)-5-chloro-2-oxo-2,3-dihydro-1*H*-benzo[*d*]imidazol-1-yl)cyclopropyl)benzoic acid methyl ester (63 mg, 0.11 mmol) obtained in step A above.
¹H NMR (400 MHz, DMSO-d6) δ7.87 (d, 2H), 7.85 (m, 1H), 7.68 (3H), 7.45 (2H), 7.28 (d, 1H), 7.21 (m, 1H), 7.15 (m, 1H), 4.14 (s, 2H), 3.83 (s, 2H), 3.53 (4H), 1.70 (8H)

### Example 94: Preparation of 3-((5-chloro-3-(4-(4,4-difluoropiperidin-1-yl)phenyl)-2-oxo-2,3-dihydro-1H-benzo[d]imidazol-1-yl)methyl)benzoic acid

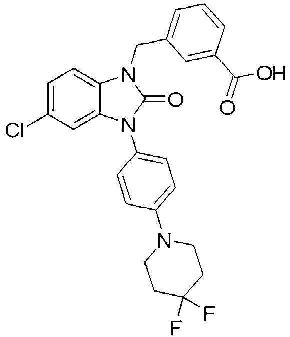

The process of the following steps A and B gave the title compound.

### Step A: Preparation of 3-((5-chloro-3-(4-(4,4-difluoropiperidin-1-yl)phenyl)-2-oxo-2,3-dihydro-1H-benzo[d]imidazol-1-yl)methyl)benzoic acid methyl ester

The title compound (0.35 g, yield: 99%) was obtained in the same manner as in step A of Example 24 by using 6-chloro-1-(4-(4,4-difluoropiperidin-1-yl)phenyl)-1,3-dihydro-2H-benzo[d]imidazol-2-one (0.25 g, 0.68 mmol) obtained in Preparation Example 28 and 3-(bromomethyl)benzoic acid methyl ester (0.16 g, 0.68 mmol).
¹H-NMR (400 MHz, CDCl3) δ 8.05 (d, 1H), 7.97 (dd, 1H), 7.56 (dd, 1H), 7.43 (d, 1H), 7.40 (d, 2H), 7.07 (d, 2H), 7.00 (d, 2H), 6.78 (d, 1H), 5.15 (s, 2H), 3.92 (s, 3H), 3.44 (m, 4H), 2.12 (m, 4H)

### Step B: Preparation of 3-((5-chloro-3-(4-(4,4-difluoropiperidin-1-yl)phenyl)-2-oxo-2,3-dihydro-1H-benzo[d]imidazol-1-yl)methyl)benzoic acid

The title compound (0.34 g, yield: 99%) was obtained in the same manner as in step B of Example 24 by using 3-((5-chloro-3-(4-(4,4-difluoropiperidin-1-yl)phenyl)-2-oxo-2,3-dihydro-1*H*-benzo[*d*]imidazol-1-yl)methyl)benzoic acid methyl ester (0.35 g, 0.68 mmol) obtained in step A above.
MS [M+H] = 498 (M+1)
¹H-NMR (400 MHz, DMSO-d6) δ 7.96 (d, 1H), 7.85 (dd, 1H), 7.60 (dd, 1H), 7.47 (t, 1H), 7.39 (d, 2H), 7.23 (d, 1H), 7.18 (d, 2H), 7.13 (dd, 1H), 6.92 (d, 1H), 5.19 (s, 2H), 3.45 (m, 4H), 2.08 (m, 4H)

### Example 95: Preparation of 3-((5-chloro-3-(4-(4-(cyclopropanecarbonyl)piperazin-1-yl)phenyl)-2-oxo-2,3-dihydro-1H-benzo[d]imidazol-1-yl)methyl)benzoic acid

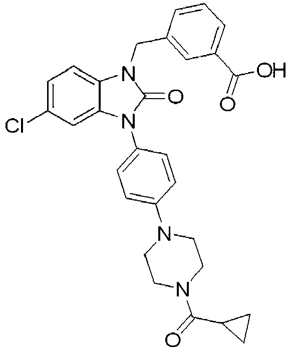

The process of the following steps A and B gave the title compound.

### Step A: Preparation of 3-((5-chloro-3-(4-(4-(cyclopropanecarbonyl)piperazin-1-yl)phenyl)-2-oxo-2,3-dihydro-1H-benzo[d]imidazol-1-yl)methyl)benzoic acid methyl ester

The title compound (0.083 g, yield: 78%) was obtained in the same manner as in step C of Example 26 by using 3-((5-chloro-2-oxo-3-(4-(piperazin-1-yl)phenyl)-2,3-dihydro-1*H-*benzo[*d*]imidazol-1-yl)methyl)benzoic acid methyl ester hydrochloride (0.10 g, 0.19 mmol) obtained in Preparation Example 29 and cyclopropanecarbonyl chloride (0.022 g, 0.21 mmol).
¹H-NMR (400 MHz, CDCl3) δ 8.05 (d, 1H), 7.97 (dd, 1H), 7.56 (dd, 1H), 7.42 (3H), 7.06 (d, 2H), 7.00 (2H), 6.79 (d, 1H), 5.15 (s, 2H), 3.92 (s, 3H), 3.88 (m, 4H), 3.28 (m, 4H), 1.80 (m, 1H), 1.04 (m, 2H), 0.81 (m, 2H)

### Step B: Preparation of 3-((5-chloro-3-(4-(4-(cyclopropanecarbonyl)piperazin-1-yl)phenyl)-2-oxo-2,3-dihydro-1H-benzo[d]imidazol-1-yl)methyl)benzoic acid

The title compound (0.065 g, yield: 80%) was obtained in the same manner as in step B of Example 24 by using 3-((5-chloro-3-(4-(4-(cyclopropanecarbonyl)piperazin-1-yl)phenyl)-2-oxo-2,3-dihydro-1*H*-benzo[*d*]imidazol-1-yl)methyl)benzoic acid methyl ester (0.083 g, 0.15 mmol) obtained in step A above.
MS [M+H] = 531 (M+1)
¹H-NMR (400 MHz, CDCl3) δ 8.10 (d, 1H), 8.01 (dd, 1H), 7.59 (dd, 1H), 7.45 (3H), 7.12 (d, 2H), 7.02 (2H), 6.82 (d, 1H), 5.18 (s, 2H), 3.91 (m, 4H), 3.31 (m, 4H), 1.80 (m, 1H), 1.06 (m, 2H), 0.84 (m, 2H)

### Example 96: Preparation of 3-((5-chloro-3-(4-(4-(isopropoxycarbonyl)piperazin-1-yl)phenyl)-2-oxo-2,3-dihydro-1H-benzo[d]imidazol-1-yl)methyl)benzoic acid

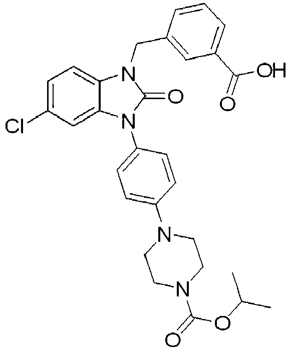

The process of the following steps A and B gave the title compound.

### Step A: Preparation of 3-((5-chloro-3-(4-(4-(isopropoxycarbonyl)piperazin-1-yl)phenyl)-2-oxo-2,3-dihydro-1H-benzo[d]imidazol-1-yl)methyl)benzoic acid methyl ester

To triphosgene (0.058 g, 0.19 mmol) was added 3 mL of DCM and DIPEA (0.068 mL, 0.39 mmol), and then the resulting mixture was cooled to 0 °C. 2-Propanol (0.030 mL, 0.39 mmol) was slowly added thereto, and then the resultant mixture was stirred at room temperature for 2 hours. The reaction solution was concentrated under reduced pressure, and then 5 mL of DCM, 3-((5-chloro-2-oxo-3-(4-(piperazin-1-yl)phenyl)-2,3-dihydro-1*H*-benzo[d]imidazol-1-yl)methyl)benzoic acid methyl ester hydrochloride (0.10 g, 0.19 mmol) obtained in Preparation Example 29, and DIPEA (0.068 mL, 0.39 mmol) were added thereto, followed by stirring at room temperature for 1 hour. To the reaction solution was added water, and then an organic layer was separated and purified by MPLC to give the title compound (0.044 g, yield: 40%).
¹H-NMR (400 MHz, CDCl3) δ 8.05 (d, 1H), 7.97 (dd, 1H), 7.55 (dd, 1H), 7.42 (3H), 7.05 (d, 2H), 7.00 (2H), 6.79 (d, 1H), 5.15 (s, 2H), 4.97 (m, 1H), 3.92 (s, 3H), 3.66 (m, 4H), 3.23 (m, 4H), 1.27 (d, 6H)

### Step B: Preparation of 3-((5-chloro-3-(4-(4-(isopropoxycarbonyl)piperazin-1-yl)phenyl)-2-oxo-2,3-dihydro-1H-benzo[d]imidazol-1-yl)methyl)benzoic acid

The title compound (0.032 g, yield: 83%) was obtained in the same manner as in step B of Example 24 by using 3-((5-chloro-3-(4-(4-(isopropoxycarbonyl)piperazin-1-yl)phenyl)-2-oxo-2,3-dihydro-1*H*-benzo[d]imidazol-1-yl)methyl)benzoic acid methyl ester (0.044 g, 0.075 mmol) obtained in step A above.
MS [M-H] = 547 (M-1)
¹H-NMR (400 MHz, CDCl3) δ 8.11 (d, 1H), 8.03 (dd, 1H), 7.60 (dd, 1H), 7.45 (t, 1H), 7.41 (d, 2H), 7.06 (d, 2H), 7.01 (2H), 6.81 (d, 1H), 5.18 (s, 2H), 4.98 (m, 1H), 3.66 (m, 4H), 3.22 (m, 4H), 1.28 (d, 6H)

### Example 97: Preparation of 3-((5-chloro-3-(4-(4-methylpiperazin-1-yl)phenyl)-2-oxo-2,3-dihydro-1H-benzo[d]imidazol-1-yl)methyl)benzoic acid

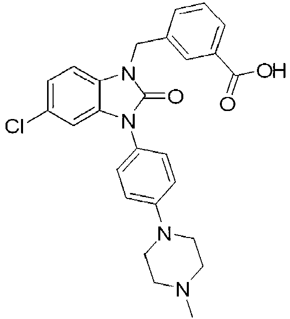

The process of the following steps A and B gave the title compound.

### Step A: Preparation of 3-((5-chloro-3-(4-(4-methylpiperazin-1-yl)phenyl)-2-oxo-2,3-dihydro-1H-benzo[d]imidazol-1-yl)methyl)benzoic acid methyl ester

To 3-((5-chloro-2-oxo-3-(4-(piperazin-1-yl)phenyl)-2,3-dihydro-1*H*-benzo[d]imidazol-1-yl)methyl)benzoic acid methyl ester hydrochloride (0.15 g, 0.29 mmol) obtained in Preparation Example 29 were added 6 mL of dichloroethane, paraformaldehyde (0.044 g, 1.46 mmol) and acetic acid (0.033 mL, 0.58 mmol), and then the resulting mixture was stirred at room temperature for 2 hours. Sodium triacetoxyborohydride (0.155 g, 0.73 mmol) was added thereto, and the resulting mixture was stirred at room temperature for 16 hours. To the filtrate was added a saturated aqueous sodium bicarbonate solution to separate an organic layer, and the organic layer was dried over anhydrous Na₂SO₄ to give the title compound (0.075 g, yield: 52%).
¹H-NMR (400 MHz, CDCl3) δ 8.05 (d, 1H), 7.97 (dd, 1H), 7.55 (dd, 1H), 7.42 (t, 1H), 7.38 (d, 2H), 7.05 (d, 2H), 7.00 (2H), 6.78 (d, 1H), 5.15 (s, 2H), 3.91 (s, 3H), 3.32 (4H), 2.65 (4H), 2.40 (s, 3H)

### Step B: Preparation of 3-((5-chloro-3-(4-(4-methylpiperazin-1-yl)phenyl)-2-oxo-2,3-dihydro-1H-benzo[d]imidazol-1-yl)methyl)benzoic acid

The title compound (0.044 g, yield: 60%) was obtained in the same manner as in step B of Example 24 by using 3-((5-chloro-3-(4-(4-methylpiperazin-1-yl)phenyl)-2-oxo-2,3-dihydro-1*H*-benzo[*d*]imidazol-1-yl)methyl)benzoic acid methyl ester (0.075 g, 0.15 mmol) obtained in step A above.
MS [M+H] = 477 (M+1)
¹H-NMR (400 MHz, DMSO-d6) δ 7.96 (d, 1H), 7.86 (dd, 1H), 7.61 (dd, 1H), 7.49 (t, 1H), 7.37 (d, 2H), 7.23 (d, 1H), 7.12 (3H), 6.91 (d, 1H), 5.20 (s, 2H), 3.25 (m, 4H), 2.55 (m, 4H), 2.29 (s, 3H)

### Example 98: Preparation of 3-(1-(5-chloro-3-(4-cyclohexylphenyl)-2-oxo-2,3-dihydro-1H-benzo[d]imidazol-1-yl)cyclopropyl)benzoic acid

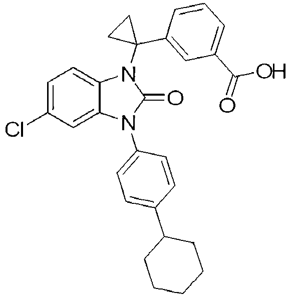

The title compound (20 mg, yield: 28%) was obtained in the same manner as in steps A and B of Example 1 by using 3-(1-(5-chloro-2-oxo-2,3-dihydro-1*H*-benzo[*d*]imidazol-1-yl)cyclopropyl)benzoic acid methyl ester (50 mg, 0.145 mmol) obtained in Preparation Example 3 and (4-cyclohexylphenyl)boronic acid (59 mg, 0.29 mmol).
¹H-NMR (400 MHz, CDCl3) δ7.97 (d, 1H), 7.94 (s, 1H), 7.57 (d, 1H), 7.41 (m, 5H), 7.13 (m, 3H), 2.60 (m, 1H), 1.92 (m, 4H), 1.75 (m, 6H), 1.45 (m, 4H).

### Example 99: Preparation of 3-(1-(5-chloro-2-oxo-3-(4-(tetrahydro-2H-pyran-4-yl)phenyl)-2,3-dihydro-1H-benzo[d]imidazol-1-yl)cyclopropyl)benzoic acid

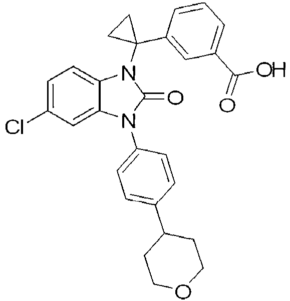

The process of the following steps A and B gave the title compound.

### Step A: Preparation of 3-(1-(5-chloro-2-oxo-3-(4-(tetrahydro-2H-pyran-4-yl)phenyl)-2,3-dihydro-1H-benzo[d]imidazol-1-yl)cyclopropyl)benzoic acid methyl ester

The title compound (58 mg, yield: 50%) was obtained in the same manner as in step A of Example 1 by using 3-(1-(5-chloro-2-oxo-2,3-dihydro-1*H*-benzo[d]imidazol-1-yl)cyclopropyl)benzoic acid methyl ester (78 mg, 0.23 mmol) obtained in Preparation Example 3 and (4-(tetrahydro-2*H*-pyran-4-yl)boronic acid (60 mg, 0.29 mmol).
¹H-NMR (400 MHz, CDCl3) δ 8.05 (d, 1H), 7.98 (dd, 1H), 7.56 (dd, 1H), 7.48 (d, 2H), 7.40 (3H), 7.01 (d, 1H), 7.03 (dd, 1H), 6.81 (d, 1H), 5.16 (s, 2H), 4.11 (m, 2H), 3.82 (s, 3H), 3.58 (m, 2H), 2.85 (m, 1H), 1.83 (m, 4H)

### Step B: Preparation of 3-(1-(5-chloro-2-oxo-3-(4-(tetrahydro-2H-pyran-4-yl)phenyl)-2,3-dihydro-1H-benzo[d]imidazol-1-yl)cyclopropyl)benzoic acid

The title compound (46 mg, yield: 82%) was obtained in the same manner as in step B of Example 1 by using 3-(1-(5-chloro-2-oxo-3-(4-(tetrahydro-2*H*-pyran-4-yl)phenyl)-2,3-dihydro-1*H*-benzo[*d*]imidazol-1-yl)cyclopropyl)benzoic acid methyl ester (58 mg, 0.115 mmol) obtained in step A above.
¹H-NMR (400 MHz, CDCl3) δ 7.94 (dd, 1H), 7.91 (d, 1H), 7.54 (dd, 1H), 7.47 (d, 2H), 7.40 (3H), 7.13 (d, 1H), 7.08 (2H), 4.10 (m, 2H), 3.56 (m, 2H), 2.83 (m, 1H), 1.85 (4H), 1.74 (m, 2H), 1.68 (m, 2H)

### Example 100: Preparation of 3-((5-chloro-3-(4-(1-(cyclopropanecarbonyl)piperidin-4-yl)phenyl)-2-oxo-2,3-dihydro-1H-benzo[d]imidazol-1-yl)methyl)benzoic acid

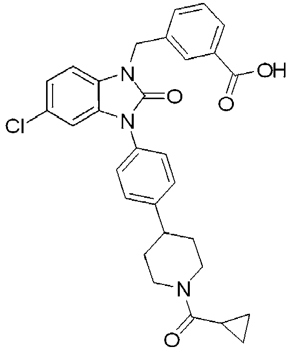

The process of the following steps A and B gave the title compound.

### Step A: Preparation of 3-((5-chloro-3-(4-(1-(cyclopropanecarbonyl)piperidin-4-yl)phenyl)-2-oxo-2,3-dihydro-1H-benzo[d]imidazol-1-yl)methyl)benzoic acid methyl ester

To 3-((5-chloro-2-oxo-3-(4-(piperidin-4-yl)phenyl)-2,3-dihydro-1*H*-benzo[d]imidazol-1-yl)methyl)benzoic acid methyl ester hydrochloride (0.10 g, 0.19 mmol) obtained in Preparation Example 30 were added 2 mL of DCM, cyclopropanecarbonyl chloride (19 µL, 0.22 mmol) and DIPEA (85 µL, 0.49 mmol), and then the resulting mixture was stirred at room temperature for 1 hour. To the reaction solution was added water, and an organic layer was separated and then purified by MPLC to give the title compound (0.095 g, yield: 89%) .
¹H-NMR (400 MHz, CDCl3) δ 8.05 (d, 1H), 7.98 (dd, 1H), 7.56 (dd, 1H), 7.48 (d, 2H), 7.43 (t, 1H), 7.39 (d, 2H), 7.07 (d, 1H), 7.02 (dd, 1H), 6.81 (d, 1H), 5.16 (s, 2H), 4.82 (m, 1H), 4.40 (m, 1H), 3.91 (s, 3H), 3.24 (m, 1H), 2.87 (m, 1H), 2.70 (m, 1H), 1.98 (m, 2H), 1.81 (m, 1H), 1.71 (m, 2H), 1.02 (m, 2H), 0.79 (m, 2H)

### Step B: Preparation of 3-((5-chloro-3-(4-(1-(cyclopropanecarbonyl)piperidin-4-yl)phenyl)-2-oxo-2,3-dihydro-1H-benzo[d]imidazol-1-yl)methyl)benzoic acid

The title compound (74 mg, yield: 80%) was obtained in the same manner as in step B of Example 24 by using 3-((5-chloro-3-(4-(1-(cyclopropanecarbonyl)piperidin-4-yl)phenyl)-2-oxo-2,3-dihydro-1*H*-benzo[d]imidazol-1-yl)methyl)benzoic acid methyl ester (95 mg, 0.17 mmol) obtained in step A above.
MS [M+H] = 530 (M+1)
¹H-NMR (400 MHz, CDCl3) δ 8.11 (d, 1H), 8.03 (dd, 1H), 7.60 (dd, 1H), 7.46 (3H), 7.39 (d, 2H), 7.08 (d, 1H), 7.04 (dd, 1H), 6.83 (d, 1H), 5.18 (s, 2H), 4.80 (m, 1H), 4.40 (m, 1H), 3.20 (m, 1H), 2.87 (m, 1H), 2.70 (m, 1H), 1.96 (m, 2H), 1.81 (m, 1H), 1.70 (m, 2H), 1.04 (m, 2H), 0.80 (m, 2H)

### Example 101: Preparation of 3-((5-chloro-3-(4-(1-(cyclobutanecarbonyl)piperidin-4-yl)phenyl)-2-oxo-2,3-dihydro-1H-benzo[d]imidazol-1-yl)methyl)benzoic acid

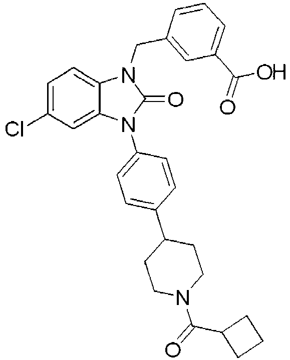

The process of the following steps A and B gave the title compound.

### Step A: Preparation of 3-((5-chloro-3-(4-(1-(cyclobutanecarbonyl)piperidin-4-yl)phenyl)-2-oxo-2,3-dihydro-1H-benzo[d]imidazol-1-yl)methyl)benzoic acid methyl ester

To 3-((5-chloro-2-oxo-3-(4-(piperidin-4-yl)phenyl)-2,3-dihydro-1H-benzo[d]imidazol-1-yl)methyl)benzoic acid methyl ester hydrochloride (100 mg, 0.19 mmol) obtained in Preparation Example 30 were added 2 mL of DCM, cyclobutanecarboxylic acid (21 mg, 0.22 mmol), HATU (82 mg, 0.22 mmol), and DIPEA (85 µL, 0.49 mmol), and then the resulting mixture was stirred at room temperature for 3 hours. The reaction solution was concentrated under reduced pressure and then purified by MPLC to give the title compound (90 mg, yield: 83%).
¹H-NMR (400 MHz, CDCl3) δ 8.05 (d, 1H), 7.98 (dd, 1H), 7.56 (dd, 1H), 7.48 (d, 2H), 7.45 (t, 1H), 7.36 (d, 2H), 7.07 (d, 1H), 7.02 (dd, 1H), 6.80 (d, 1H), 5.16 (s, 2H), 4.81 (m, 1H), 3.92 (s, 3H), 3.87 (m, 1H), 3.30 (m, 1H), 3.08 (m, 1H), 2.82 (m, 1H), 2.66 (m, 1H), 2.38 (m, 2H), 2.20 (m, 1H), 1.94 (m, 4H), 1.60 (m, 3H)

### Step B: Preparation of 3-((5-chloro-3-(4-(1-(cyclobutanecarbonyl)piperidin-4-yl)phenyl)-2-oxo-2,3-dihydro-1H-benzo[d]imidazol-1-yl)methyl)benzoic acid

The title compound (88 mg, yield: 99%) was obtained in the same manner as in step B of Example 24 by using 3-((5-chloro-3-(4-(1-(cyclobutanecarbonyl)piperidin-4-yl)phenyl)-2-oxo-2,3-dihydro-1*H*-benzo[*d*]imidazol-1-yl)methyl)benzoic acid methyl ester (90 mg, 0.16 mmol) obtained in step A above.
MS [M+H] =544 (M+1)
¹H-NMR (400 MHz, CDCl3) δ 8.10 (d, 1H), 8.03 (dd, 1H), 7.60 (dd, 1H), 7.46 (3H), 7.36 (d, 2H), 7.07 (d, 1H), 7.03 (dd, 1H), 6.82 (d, 1H), 5.18 (s, 2H), 4.80 (m, 1H), 3.85 (m, 1H), 3.32 (m, 1H), 3.08 (m, 1H), 2.82 (m, 1H), 2.67 (m, 1H), 2.40 (m, 2H), 2.17 (m, 2H), 1.92 (4H), 1.62 (m, 2H)

### Example 102: Preparation of 3-((5-chloro-2-oxo-3-(4-(1-propionylpiperidin-4-yl)phenyl)-2,3-dihydro-1H-benzo[d]imidazol-1-yl)methyl)benzoic acid

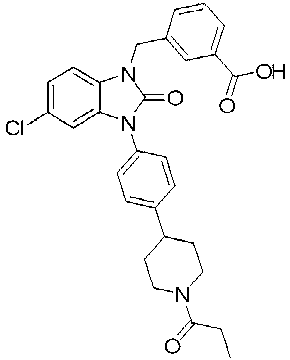

The process of the following steps A and B gave the title compound.

### Step A: Preparation of 3-((5-chloro-2-oxo-3-(4-(1-propionylpiperidin-4-yl)phenyl)-2,3-dihydro-1H-benzo[d]imidazol-1-yl)methyl)benzoic acid methyl ester

To 3-((5-chloro-2-oxo-3-(4-(piperidin-4-yl)phenyl)-2,3-dihydro-1*H*-benzo[*d*]imidazol-1-yl)methyl)benzoic acid methyl ester hydrochloride (100 mg, 0.19 mmol) obtained in Preparation Example 30 were added 2 mL of DCM, propionyl chloride (20 mg, 0.22 mmol), and DIPEA (85 µL, 0.49 mmol), and then the resulting mixture was stirred at room temperature for 1 hour. To the reaction solution was added water, and an organic layer was separated and then purified by MPLC to give the title compound (82 mg, yield: 79%).
¹H-NMR (400 MHz, CDCl3) δ 8.05 (d, 1H), 7.98 (dd, 1H), 7.56 (dd, 1H), 7.48 (d, 2H), 7.43 (t, 1H), 7.37 (d, 2H), 7.07 (d, 1H), 7.02 (dd, 1H), 6.81 (d, 1H), 5.16 (s, 2H), 4.85 (m, 1H), 4.04 (m, 1H), 3.90 (s, 3H), 3.16 (m, 1H), 2.85 (m, 1H), 2.66 (m, 1H), 2.42 (q, 2H), 1.95 (m, 2H), 1.66 (m, 2H), 1.32 (t, 3H)

### Step B: Preparation of 3-((5-chloro-2-oxo-3-(4-(1-propionylpiperidin-4-yl)phenyl)-2,3-dihydro-1H-benzo[d]imidazol-1-yl)methyl)benzoic acid

The title compound (75 mg, yield: 96%) was obtained in the same manner as in step B of Example 24 by using 3-((5-chloro-2-oxo-3-(4-(1-propionylpiperidin-4-yl)phenyl)-2,3-dihydro-1*H*-benzo[d]imidazol-1-yl)methyl)benzoic acid methyl ester (80 mg, 0.15 mmol) obtained in step A above.
MS [M+H] =518 (M+1)
¹H-NMR (400 MHz, CDCl3) δ 8.10 (d, 1H), 8.03 (dd, 1H), 7.61 (dd, 1H), 7.48 (3H), 7.37 (d, 2H), 7.07 (d, 1H), 7.03 (dd, 1H), 6.82 (d, 1H), 5.18 (s, 2H), 4.87 (m, 1H), 4.02 (m, 1H), 3.17 (m, 1H), 2.83 (m, 1H), 2.68 (m, 1H), 2.42 (q, 2H), 1.96 (m, 2H), 1.67 (m, 2H), 1.18 (t, 3H)

### Example 103: Preparation of 3-((5-chloro-2-oxo-3-(4-(tetrahydro-2H-pyran-4-yl)phenyl)-2,3-dihydro-1H-benzo[d]imidazol-1-yl)methyl)benzoic acid

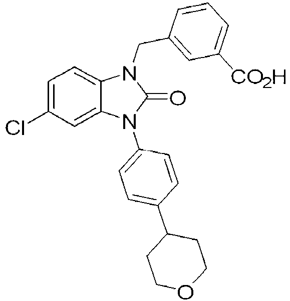

The process of the following steps A and B gave the title compound.

### Step A: Preparation of 3-((5-chloro-2-oxo-3-(4-(tetrahydro-2H-pyran-4-yl)phenyl)-2,3-dihydro-1H-benzo[d]imidazol-1-yl)methyl)benzoic acid methyl ester

The title compound (114 mg, yield: 75%) was obtained in the same manner as in step A of Example 1 by using 3-((5-chloro-2-oxo-2,3-dihydro-1H-benzo[d]imidazol-1-yl)methyl)benzoic acid methyl ester (100 mg, 0.32 mmol) obtained in Preparation Example 1 and (4-(tetrahydro-2H-pyran-4-yl)phenyl)boronic acid (130 mg, 0.63 mmol).
¹H-NMR (400 MHz, CDCl3) δ 8.05 (d, 1H), 7.98 (dd, 1H), 7.56 (dd, 1H), 7.48 (d, 2H), 7.40 (3H), 7.01 (d, 1H), 7.03 (dd, 1H), 6.81 (d, 1H), 5.16 (s, 2H), 4.11 (m, 2H), 3.82 (s, 3H), 3.58 (m, 2H), 2.85 (m, 1H), 1.83 (m, 4H)

### Step B: Preparation of 3-((5-chloro-2-oxo-3-(4-(tetrahydro-2H-pyran-4-yl)phenyl)-2,3-dihydro-1H-benzo[d]imidazol-1-yl)methyl)benzoic acid

The title compound (100 mg, yield: 91%) was obtained in the same manner as in step B of Example 24 by using 3-((5-chloro-2-oxo-3-(4-(tetrahydro-2*H*-pyran-4-yl)phenyl)-2,3-dihydro-1*H*-benzo[*d*]imidazol-1-yl)methyl)benzoic acid methyl ester (114 mg, 0.29 mmol) obtained in step A above.
MS [M+H] =463 (M+1)
¹H-NMR (400 MHz, CDCl3) δ 8.11 (d, 1H), 8.03 (dd, 1H), 7.62 (dd, 1H), 7.48 (3H), 7.41 (d, 2H), 7.08 (d, 1H), 7.03 (dd, 1H), 6.83 (d, 1H), 5.18 (s, 2H), 4.12 (m, 2H), 3.57 (m, 2H), 2.85 (m, 1H), 1.83 (4H)

### Example 104: Preparation of 3-((5-chloro-3-(4-(3,6-dihydro-2H-pyran-4-yl)phenyl)-2-oxo-2,3-dihydro-1H-benzo[d]imidazol-1-yl)methyl)benzoic acid

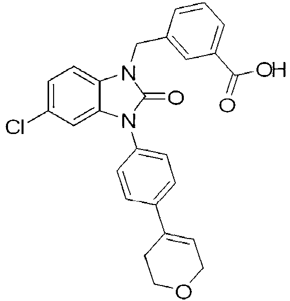

The process of the following steps A and B gave the title compound.

### Step A: Preparation of 3-((5-chloro-3-(4-(3,6-dihydro-2H-pyran-4-yl)phenyl)-2-oxo-2,3-dihydro-1H-benzo[d]imidazol-1-yl)methyl)benzoic acid methyl ester

The title compound (60 mg, yield: 69%) was obtained in the same manner as in step C of Preparation Example 7 by using 6-chloro-1-(4-(3,6-dihydro-2H-pyran-4-yl)phenyl)-1,3-dihydro-2H-benzo[d]imidazol-2-one (60 mg, 0.184 mmol) obtained in Preparation Example 31 and 3-(bromomethyl)benzoic acid methyl ester (42 mg, 0.184 mmol).
¹H-NMR (500 MHz, CDCl3) δ8.09 (s, 1H), 8.00 (d, 1H), 7.54 (m, 4H), 7.45 (m, 2H), 7.11 (s, 1H), 7.05 (d, 1H), 6.83 (d, 1H), 6.28 (s, 1H), 5.18 (s, 2H), 4.38 (m, 2H), 3.99 (m, 2H), 2.59 (m, 2H)

### Step B: Preparation of 3-((5-chloro-3-(4-(3,6-dihydro-2H-pyran-4-yl)phenyl)-2-oxo-2,3-dihydro-1H-benzo[d]imidazol-1-yl)methyl)benzoic acid

The title compound (11 mg, yield: 19%) was obtained in the same manner as in step B of Example 24 by using 3-((5-chloro-3-(4-(3,6-dihydro-2H-pyran-4-yl)phenyl)-2-oxo-2,3-dihydro-1H-benzo[d]imidazol-1-yl)methyl)benzoic acid methyl ester (60 mg, 0.126 mmol) obtained in step A above.
MS [M+H] =461 (M+1)
¹H-NMR (400 MHz, DMSO-d6) δ7.98 (s, 1H), 7.87 (d, 1H), 7.66 (d, 2H), 7.62 (d, 1H), 7.59 (d, 2H), 7.50 (m, 1H), 7.27 (d, 1H), 7.17 (d, 1H), 7.06 (s, 1H), 6.38 (s, 1H), 5.22 (s, 2H), 4.27 (m, 2H), 3.86 (t, 2H), 2.50 (m, 2H)

### Example 105: Preparation of 3-((5-chloro-3-(4-(3,3-difluoropyrrolidin-1-yl)phenyl)-4-fluoro-2-oxo-2,3-dihydro-1H-benzo[d]imidazol-1-yl)methyl)benzoic acid

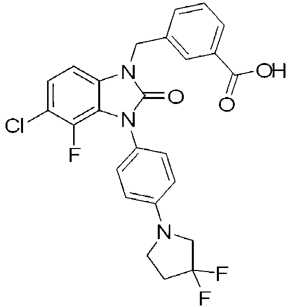

The process of the following steps A and B gave the title compound.

### Step A: Preparation of 3-((5-chloro-3-(4-(3,3-difluoropyrrolidin-1-yl)phenyl)-4-fluoro-2-oxo-2,3-dihydro-1H-benzo[d]imidazol-1-yl)methyl)benzoic acid methyl ester

The title compound (70 mg, yield: 99%) was obtained in the same manner as in step C of Preparation Example 7 by using 6-chloro-1-(4-(3,3-difluoropyrrolidin-1-yl)phenyl)-7-fluoro-1,3-dihydro-2H-benzo[d]imidazol-2-one (50 mg, 0.136 mmol) obtained in Preparation Example 32 and 3-(bromomethyl)benzoic acid methyl ester (31 mg, 0.136 mmol).
¹H-NMR (400 MHz, CDCl3) δ8.03 (d, 1H), 7.98 (dd, 1H), 7.57 (dd, 1H), 7.36 (t, 1H), 7.34 (d, 2H), 7.02 (dd, 1H), 6.64 (3H), 5.14 (s, 2H), 3.92 (s, 3H), 3.73 (t, 2H), 3.58 (t, 2H), 2.52 (m, 2H)

### Step B: Preparation of 3-((5-chloro-3-(4-(3,3-difluoropyrrolidin-1-yl)phenyl)-4-fluoro-2-oxo-2,3-dihydro-1H-benzo[d]imidazol-1-yl)methyl)benzoic acid

The title compound (35 mg, yield: 51%) was obtained in the same manner as in step B of Example 24 by using 3-((5-chloro-3-(4-(3,3-difluoropyrrolidin-1-yl)phenyl)-4-fluoro-2-oxo-2,3-dihydro-1H-benzo[d]imidazol-1-yl)methyl)benzoic acid methyl ester (70 mg, 0.136 mmol) obtained in step A above.
MS [M+H] =502 (M+1)
¹H-NMR (400 MHz, CDCl3+MeOH-d4) δ8.06 (d, 1H), 8.00 (dd, 1H), 7.58 (dd, 1H), 7.45 (t, 1H), 7.35 (d, 2H), 7.03 (m, 1H), 6.64 (3H), 5.16 (s, 2H), 3.73 (t, 2H), 3.59 (t, 2H), 2.53 (m, 2H)

### Example 106: Preparation of 3-(1-(5-chloro-2-oxo-3-(4-(tetrahydro-2H-pyran-4-yl)phenyl)-2,3-dihydro-1H-benzo[d]imidazol-1-yl)cyclopropyl)-2-fluorobenzoic acid

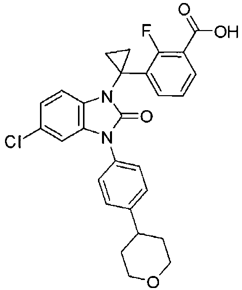

The title compound (20 mg, yield: 24%) was obtained as in the same manner as in step A of Example 1 and step B of Example 24 by using 3-(1-(5-chloro-2-oxo-2,3-dihydro-1*H-*benzo[*d*]imidazol-1-yl)cyclopropyl)-2-fluorobenzoic acid methyl ester (59 mg, 0.16 mmol) obtained in Preparation Example 4 and (4-(tetrahydro-2H-pyran-4-yl)phenyl)boronic acid (67 mg, 0.32 mmol).

¹H-NMR (400 MHz, CDCl3) δ8.10 (t, 1H), 7.81 (t, 1H), 7.47 (d, 1H), 7.35 (dd, 4H), 7.09 (t, 1H), 7.04 (d, 1H), 6.97 (s, 1H), 4.10 (d, 2H), 3.53 (t, 2H), 2.79 (m, 1H), 1.79 (m, 4H), 1.59 (m, 4H)

### Example 107: Preparation of 3-(1-(3-(4-(7-oxa-2-azaspiro[3.5]nonane-2-carbonyl)phenyl)-5-chloro-2-oxo-2,3-dihydro-1H-benzo[d]imidazol-1-yl)cyclopropyl)-2-fluorobenzoic acid

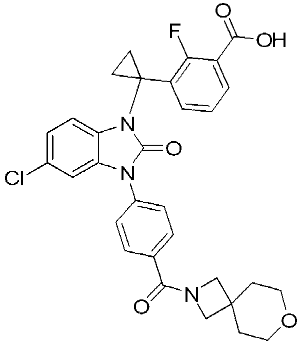

The title compound (16 mg, yield: 28%) was obtained in the same manner as in step C of Example 56 and step B of Example 24 by using 4-(6-chloro-3-(1-(2-fluoro-3-(methoxycarbonyl)phenyl)cyclopropyl)-2-oxo-2,3-dihydro-1*H-*benzo[*d*]imidazol-1-yl)benzoic acid (50 mg, 0.10 mmol) obtained in step B of Example 67 and 7-oxa-2-azaspiro[3.5]nonane (16 mg, 0.13 mmol).
MS [M+H] =576 (M+1)
¹H-NMR (400 MHz, DMSO-d6) δ7.93 (m, 1H), 7.83 (d, 2H), 7.75 (m, 1H), 7.61 (dd, 2H), 7.50 (m, 1H), 7.27 (m, 2H), 7.06 (d, 1H), 4.12 (s, 2H), 3.82 (s, 2H), 3.50 (m, 4H), 1.82 (m, 4H), 1.64 (m, 4H)

### Example 108: Preparation of 3-((5-chloro-2-oxo-3-(4-(tetrahydro-2H-pyran-4-yl)phenyl)-2,3-dihydro-1H-benzo[d]imidazol-1-yl)methyl)-2-fluorobenzoic acid

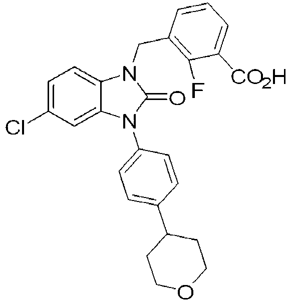

The process of the following steps A and B gave the title compound.

### Step A: Preparation of 3-((5-chloro-2-oxo-3-(4-(tetrahydro-2H-pyran-4-yl)phenyl)-2,3-dihydro-1H-benzo[d]imidazol-1-yl)methyl)-2-fluorobenzoic acid methyl ester

The title compound (0.028 g, yield: 38%) was obtained in the same manner as in step A of Example 1 by using 3-((5-chloro-2-oxo-2,3-dihydro-1*H*-benzo[d]imidazol-1-yl)methyl)-2-fluorobenzoic acid methyl ester (0.050 g, 0.149 mmol) obtained in Preparation Example 2 and (4-(tetrahydro-2*H*-pyran-4-yl)phenyl)boronic acid (0.092 g, 0.448 mmol).
¹H-NMR (400 MHz, CDCl3) δ7.89 (m, 1H), 7.62 (m, 1H), 7.45 (d, 2H), 7.40 (d, 2H), 7.17 (t, 1H), 7.07 (m, 1H), 6.97 (d, 1H), 6.72 (d, 1H), 5.20 (s, 2H), 4.10 (m, 2H), 3.95 (s, 3H), 3.55 (m, 2H), 2.85 (m, 1H), 1.84 (m, 4H)

### Step B: Preparation of 3-((5-chloro-2-oxo-3-(4-(tetrahydro-2H-pyran-4-yl)phenyl)-2,3-dihydro-1H-benzo[d]imidazol-1-yl)methyl)-2-fluorobenzoic acid

The title compound (0.027 g, yield: 99%) was obtained in the same manner as in step B of Example 24 by using 3-((5-chloro-2-oxo-3-(4-(tetrahydro-2*H*-pyran-4-yl)phenyl)-2,3-dihydro-1*H*-benzo[*d*]imidazol-1-yl)methyl)-2-fluorobenzoic acid methyl ester (0.028 g, 0.057 mmol) obtained in step A above.
MS [M-H] = 479 (M-1)
¹H-NMR (400 MHz, DMSO-d6) δ7.80 (td, 1H), 7.50 (5H), 7.25 (t, 1H), 7.22 (d, 1H), 7.16 (dd, 1H), 7.01 (d, 1H), 5.22 (s, 2H), 3.98 (m, 2H), 3.48 (m, 2H), 2.88 (m, 1H), 1.75 (m, 4H)

### Example 109: Preparation of 3-((5-chloro-2-oxo-3-(2',3',4',5'-tetrahydro-[1,1'-biphenyl]-4-yl)-2,3-dihydro-1H-benzo[d]imidazol-1-yl)methyl)benzoic acid

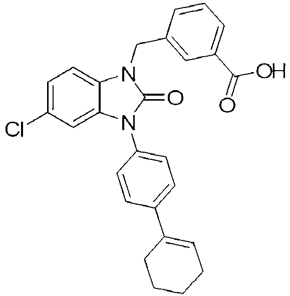

The process of the following steps A, B, and C gave the title compound.

### Step A: Preparation of 3-((3-(4-bromophenyl)-5-chloro-2-oxo-2,3-dihydro-1H-benzo[d]imidazol-1-yl)methyl)benzoic acid methyl ester

The title compound (193 mg, yield: 76%) was obtained in the same manner as in step A of Preparation Example 25 by using 3-((5-chloro-2-oxo-2,3-dihydro-1H-benzo[d]imidazol-1-yl)methyl)benzoic acid methyl ester (170 mg, 0.537 mmol) obtained in Preparation Example 1 and (4-bromophenyl)boronic acid (216 mg, 1.07 mmol).
MS [M+H] = 472 (M+1)
¹H-NMR (400 MHz, CDCl3) δ8.03 (s, 1H), 7.97 (d, 1H), 7.68 (d, 2H), 7.53 (d, 1H), 7.43 (m, 3H), 7.05 (m, 2H), 6.80 (d, 1H), 5.14 (s, 2H), 3.90 (s, 3H)

### Step B: Preparation of 3-((5-chloro-2-oxo-3-(2',3',4',5'-tetrahydro-[1,1'-biphenyl]-4-yl)-2,3-dihydro-1H-benzo[d]imidazol-1-yl)methyl)benzoic acid methyl ester

To 3-((3-(4-bromophenyl)-5-chloro-2-oxo-2,3-dihydro-1H-benzo[*d*]imidazol-1-yl)methyl)benzoic acid methyl ester (60 mg, 0.127 mmol) obtained in step A above were added 2-(cyclohex-1-en-1-yl)-4,4,5,5-tetramethyl-1,3,2-dioxaborolane (29.1 mg, 0.14 mmol), Cs₂CO₃ (124 mg, 0.382 mmol), and 2.5 mL of 1,4-dioxane, and then the resulting mixture was charged with nitrogen gas. To the mixture was added PdCl₂(dppf)-CH₂Cl₂ (9.31 mg, 0.013 mmol), and then the resulting mixture was stirred at 80 °C for 16 hours. After the reaction was terminated, the resulting solid was filtered through Celite and the filtrate was purified by MPLC to give the title compound (28 mg, yield: 46.5%).
¹H-NMR (400 MHz, CDCl3) δ8.04 (s, 1H), 7.96 (d, 1H), 7.53 (m, 3H), 7.43 (m, 3H), 7.06 (s, 1H), 7.00 (d, 1H), 6.79 (d, 1H), 6.18 (s, 1H), 5.14 (s, 2H), 3.90 (s, 3H), 2.43 (m, 2H), 2.23 (m, 2H), 1.80 (m, 2H), 1.68 (m, 2H)

### Step C: Preparation of 3-((5-chloro-2-oxo-3-(2',3',4',5'-tetrahydro-[1,1'-biphenyl]-4-yl)-2,3-dihydro-1H-benzo[d]imidazol-1-yl)methyl)benzoic acid

The title compound (25 mg, yield: 91.5%) was obtained in the same manner as in step B of Example 24 by using 3-((5-chloro-2-oxo-3-(2',3',4',5'-tetrahydro-[1,1'-biphenyl]-4-yl)-2,3-dihydro-1H-benzo[d]imidazol-1-yl)methyl)benzoic acid methyl ester (28 mg, 0.059 mmol) obtained in step B above.
MS [M+H] =459 (M+1)
¹H-NMR (500 MHz, DMSO-d6) δ7.99 (s, 1H), 7.87 (d, 1H), 7.61 (m, 3H), 7.51 (m, 3H), 7.27 (d, 1H), 7.18 (d, 1H), 7.03 (s, 1H), 6.28 (s, 1H), 5.21 (s, 2H), 2.43 (m, 2H), 2.22 (m, 2H), 1.76 (m, 2H), 1.63 (m, 2H)

### Example 110: Preparation of 3-((5-chloro-3-(4-(2,5-dihydrofuran-3-yl)phenyl)-2-oxo-2,3-dihydro-1H-benzo[d]imidazol-1-yl)methyl)-2-fluorobenzoic acid

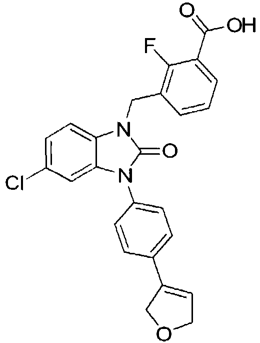

The process of the following steps A, B, and C gave the title compound.

### Step A: Preparation of 3-((3-(4-bromophenyl)-5-chloro-2-oxo-2,3-dihydro-1H-benzo[d]imidazol-1-yl)methyl)-2-fluorobenzoic acid methyl ester

The title compound (385 mg, yield: 88%) was obtained in the same manner as in step A of Preparation Example 25 by using 3-((5-chloro-2-oxo-2,3-dihydro-1H-benzo[d]imidazol-1-yl)methyl)-2-fluorobenzoic acid methyl ester (300 mg, 0.896 mmol) obtained Preparation Example 2 and (4-bromophenyl)boronic acid (360 mg, 1.79 mmol).

### Step B: Preparation of 3-((5-chloro-3-(4-(2,5-dihydrofuran-3-yl)phenyl)-2-oxo-2,3-dihydro-1H-benzo[d]imidazol-1-yl)methyl)-2-fluorobenzoic acid methyl ester

The title compound (7.9 mg, yield: 16.2%) was obtained in the same manner as in step B of Example 109 by using 3-((3-(4-bromophenyl)-5-chloro-2-oxo-2,3-dihydro-1H-benzo[d]imidazol-1-yl)methyl)-2-fluorobenzoic acid methyl ester (50 mg, 0.102 mmol) obtained in step A above and 2-(2,5-dihydrofuran-3-yl)-4,4,5,5-tetramethyl-1,3,2-dioxaborolane (22 mg, 0.112 mmol).
¹H-NMR (500 MHz, CDCl3) δ7.91 (t, 1H), 7.64 (t, 1H), 7.53 (t, 4H), 7.20 (t, 1H), 7.11(m, 2H), 7.02 (d, 1H), 6.33 (s, 1H), 5.22 (s, 2H), 5.06 (s, 2H), 4.91 (s, 2H), 3.97 (s, 3H)

### Step C: Preparation of 3-((5-chloro-3-(4-(2,5-dihydrofuran-3-yl)phenyl)-2-oxo-2,3-dihydro-1H-benzo[d]imidazol-1-yl)methyl)-2-fluorobenzoic acid

The title compound (7 mg, yield: 91%) was obtained in the same manner as in step B of Example 24 by using 3-((5-chloro-3-(4-(2,5-dihydrofuran-3-yl)phenyl)-2-oxo-2,3-dihydro-1H-benzo[d]imidazol-1-yl)methyl)-2-fluorobenzoic acid methyl ester (7.9 mg, 0.016 mmol) obtained in step B above.
MS [M+H] =465 (M+1)
¹H-NMR (500 MHz, DMSO-d6) δ7.80 (t, 1H), 7.71 (m, 1H), 7.61 (m, 3H), 7.54 (m, 1H), 7.28 (m, 2H), 7.17 (m, 1H), 7.08 (s, 1H), 6.61 (s, 1H), 5.23 (s, 2H), 4.96 (s, 2H), 4.78 (s, 2H)

### Example 111: Preparation of 3-((5-chloro-3-(4'-methyl-2',3',4',5'-tetrahydro-[1,1'-biphenyl]-4-yl)-2-oxo-2,3-dihydro-1H-benzo[d]imidazol-1-yl)methyl)-2-fluorobenzoic acid

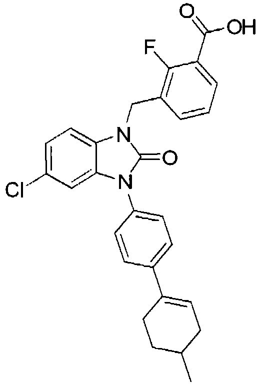

The process of the following steps A and B gave the title compound.

### Step A: Preparation of 3-((5-chloro-3-(4'-methyl-2',3',4',5'-tetrahydro-[1,1'-biphenyl]-4-yl)-2-oxo-2,3-dihydro-1H-benzo[d]imidazol-1-yl)methyl)-2-fluorobenzoic acid methyl ester

The title compound (32 mg, yield: 62%) was obtained in the same manner as in step B of Example 109 by using 3-((3-(4-bromophenyl)-5-chloro-2-oxo-2,3-dihydro-1*H*-benzo[*d*]imidazol-1-yl)methyl)-2-fluorobenzoic acid methyl ester (50 mg, 0.102 mmol) obtained in step A of Example 110 and (4-methylcyclohex-1-en-1-yl)boronic acid (15.7 mg, 0.112 mmol).
¹H-NMR (500 MHz, CDCl3) δ7.90 (t, 1H), 7.64 (t, 1H), 7.56 (d, 2H), 7.46 (d, 2H), 7.19 (t, 1H), 7.09 (m, 2H), 7.00 (d, 1H), 6.18 (s, 1H), 5.22 (s, 2H), 3.97 (s, 3H), 2.50 (m, 2H), 2.35 (d, 1H), 1.90 (m, 2H), 1.78 (m, 1H), 1.45 (m, 1H), 1.05 (d, 3H)

### Step B: Preparation of 3-((5-chloro-3-(4'-methyl-2',3',4',5'-tetrahydro-[1,1'-biphenyl]-4-yl)-2-oxo-2,3-dihydro-1H-benzo[d]imidazol-1-yl)methyl)-2-fluorobenzoic acid

The title compound (25 mg, yield: 81%) was obtained in the same manner as in step B of Example 24 by using 3-((5-chloro-3-(4'-methyl-2',3',4',5'-tetrahydro-[1,1'-biphenyl]-4-yl)-2-oxo-2,3-dihydro-1H-benzo[d]imidazol-1-yl)methyl)-2-fluorobenzoic acid methyl ester (32 mg, 0.063 mmol) obtained in step A above.
MS [M+H] =491 (M+1)
¹H-NMR (500 MHz, DMSO-d6) δ7.80 (t, 1H), 7.61 (d, 2H), 7.52 (m, 3H), 7.28 (m, 1H), 7.25 (m, 1H), 7.18 (d, 1H), 7.03 (s, 1H), 6.25 (s, 1H), 5.22 (s, 2H), 2.48 (m, 2H), 2.31 (d, 1H), 1.85 (m, 2H), 1.70 (m, 1H), 1.35 (m, 1H), 1.01 (d, 3H)

### Example 112: Preparation of 3-((5-chloro-3-(4',4'-dimethyl-2',3',4',5'-tetrahydro-[1,1'-biphenyl]-4-yl)-2-oxo-2,3-dihydro-1H-benzo[d]imidazol-1-yl)methyl)-2-fluorobenzoic acid

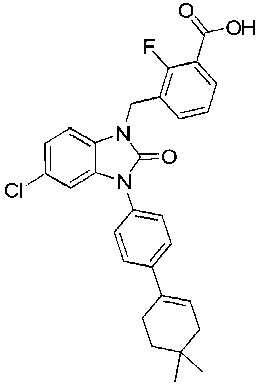

The process of the following steps A and B gave the title compound.

### Step A: Preparation of 3-((5-chloro-3-(4',4'-dimethyl-2',3',4',5'-tetrahydro-[1,1'-biphenyl]-4-yl)-2-oxo-2,3-dihydro-1H-benzo[d]imidazol-1-yl)methyl)-2-fluorobenzoic acid methyl ester

The title compound (71 mg, yield: 67%) was obtained in the same manner as in step B of Example 109 by using 3-((3-(4-bromophenyl)-5-chloro-2-oxo-2,3-dihydro-1*H*-benzo[*d*]imidazol-1-yl)methyl)-2-fluorobenzoic acid methyl ester (100 mg, 0.204 mmol) obtained in step A of Example 110 and 2-(4,4-dimethylcyclohex-1-en-1-yl)-4,4,5,5-tetramethyl-1,3,2-dioxaborolane (53 mg, 0.225 mmol).
¹H-NMR (500 MHz, CDCl3) δ7.87 (t, 1H), 7.62 (t, 1H), 7.53 (m, 2H), 7.43 (d, 2H), 7.15 (t, 1H), 7.05 (m, 2H), 6.97 (s, 1H), 6.12 (s, 1H), 5.18 (s, 2H), 3.93 (s, 3H), 2.44 (m, 2H), 2.02 (m, 2H), 1.54 (m, 2H), 0.97 (s, 6H)

### Step B: Preparation of 3-((5-chloro-3-(4',4'-dimethyl-2',3',4',5'-tetrahydro-[1,1'-biphenyl]-4-yl)-2-oxo-2,3-dihydro-1H-benzo[d]imidazol-1-yl)methyl)-2-fluorobenzoic acid

The title compound (61 mg, yield: 88%) was obtained in the same manner as in step B of Example 24 by using 3-((5-chloro-3-(4',4'-dimethyl-2',3',4',5'-tetrahydro-[1,1'-biphenyl]-4-yl)-2-oxo-2,3-dihydro-1H-benzo[d]imidazol-1-yl)methyl)-2-fluorobenzoic acid methyl ester (71 mg, 0.137 mmol) obtained in step A above.
MS [M+H] =505 (M+1)
¹H-NMR (500 MHz, DMSO-d6) δ7.75 (t, 1H), 7.59 (d, 2H), 7.48 (m, 3H), 7.22 (m, 2H), 7.14 (d, 1H), 6.99 (s, 1H), 6.18 (s, 1H), 5.18 (s, 2H), 2.40 (m, 2H), 1.97 (m, 2H), 1.47 (m, 2H), 0.91 (s, 6H)

### Example 113: Preparation of 3-(2-(5-chloro-3-(4-(dimethylcarbamoyl)phenyl)-2-oxo-2,3-dihydro-1H-benzo[d]imidazol-1-yl)ethyl)benzoic acid

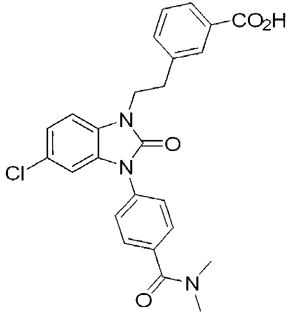

The process of the following steps A and B gave the title compound.

### Step A: Preparation of 3-(2-(5-chloro-3-(4-(dimethylcarbamoyl)phenyl)-2-oxo-2,3-dihydro-1H-benzo[d]imidazol-1-yl)ethyl)benzoic acid methyl ester

The title compound (97 mg, yield: 67%) was obtained in the same manner as in step A of Example 2 by using 3-(2-(5-chloro-2-oxo-2,3-dihydro-1*H*-benzo[*d*]imidazol-1-yl)ethyl)benzoic acid methyl ester (100 mg, 0.30 mmol) obtained in Preparation Example 36 and (4-(dimethylcarbamoyl)phenyl)boronic acid (117 mg, 0.60 mmol).
¹H-NMR (400 MHz, CDCl3) δ7.91 (2H), 7.61 (d, 2H), 7.55 (d, 2H), 7.42 (dd, 1H), 7.36 (t, 1H), 7.07 (2H), 6.82 (d, 1H), 4.17 (t, 2H), 3.91 (s, 3H), 3.16 (5H), 3.05 (s, 3H)

### Step B: Preparation of 3-(2-(5-chloro-3-(4-(dimethylcarbamoyl)phenyl)-2-oxo-2,3-dihydro-1H-benzo[d]imidazol-1-yl)ethyl)benzoic acid

The title compound (35 mg, yield: 37%) was obtained in the same manner as in step B of Example 24 by using 3-(2-(5-chloro-3-(4-(dimethylcarbamoyl)phenyl)-2-oxo-2,3-dihydro-1*H-*benzo[*d*]imidazol-1-yl)ethyl)benzoic acid methyl ester (97 mg, 0.20 mmol) obtained in step A above.
MS [M+H] = 464 (M+1)
¹H-NMR (400 MHz, CDCl3) δ7.95 (2H), 7.60 (d, 2H), 7.54 (d, 2H), 7.43 (dd, 1H), 7.37 (t, 1H), 7.07 (2H), 6.84 (d, 1H), 4.18 (t, 2H), 3.16 (5H), 3.05 (s, 3H)

### Example 114: Preparation of 3-((5-chloro-3-(2,2-dimethyl-3-oxo-2,3-dihydro-1H-inden-5-yl)-2-oxo-2,3-dihydro-1H-benzo[d]imidazol-1-yl)methyl)benzoic acid

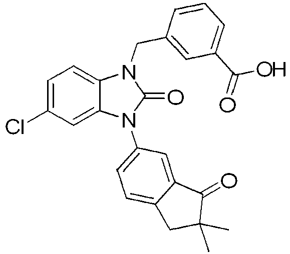

The process of the following steps A and B gave the title compound.

### Step A: Preparation of 3-((5-chloro-3-(2,2-dimethyl-3-oxo-2,3-dihydro-1H-inden-5-yl)-2-oxo-2,3-dihydro-1H-benzo[d]imidazol-1-yl)methyl)benzoic acid methyl ester

3-((5-Chloro-2-oxo-2,3-dihydro-1*H*-benzo[*d*]imidazol-1-yl)methyl)benzoic acid methyl ester obtained in Preparation Example 1 was dissolved in toluene, then 6-bromo-2,2-dimethyl-2,3-dihydro-1*H*-inden-1-one (28.6 mg, 0.12 mmol), DMEDA (22 µL, 0.20 mmol), CuI (22.4 mg, 0.11 mmol) and K₂CO₃ (33.1 mg, 0.23 mmol) were added thereto, and the resulting mixture was charged with nitrogen and then stirred at 115 °C. After the reaction was terminated, EtOAc and water were added thereto, and an organic layer was separated and dried over anhydrous MgSO₄. The filtrate was distilled under reduced pressure and then purified by MPLC to give the title compound (41.5 mg, yield: 88%) .
¹H NMR (500 MHz, CDCl₃) δ8.05 (s, 1H), 7.99 (d, 1H), 7.95 (d, 1H), 7.68 (s, 1H), 7.59 (t, 2H), 7.45 (t, 1H), 7.17 (s, 1H), 7.08 (d, 1H), 6.86 (d, 1H), 5.16 (s, 2H), 4.12 (s, 3H), 3.08 (s, 2H), 1.29 (s, 6H)

### Step B: Preparation of 3-((5-chloro-3-(2,2-dimethyl-3-oxo-2,3-dihydro-1H-inden-5-yl)-2-oxo-2,3-dihydro-1H-benzo[d]imidazol-1-yl)methyl)benzoic acid

The title compound (27.0 mg, yield: 67%) was obtained in the same manner as in step B of Example 2 by using 3-((5-chloro-3-(2,2-dimethyl-3-oxo-2,3-dihydro-1*H*-inden-5-yl)-2-oxo-2,3-dihydro-1*H*-benzo[*d*]imidazol-1-yl)methyl)benzoic acid methyl ester (41.5 mg, 0.087 mmol) obtained in step A above.
MS [M+H] = 461 (M+1)
¹H NMR (500 MHz, DMSO-d6) δ7.98 (s, 1H), 7.91 (d, 1H), 7.86 (d, 1H), 7.82 (s, 1H), 7.77 (d, 1H), 7.63 (d, 1H), 7.49 (m, 1H), 7.26 (d, 1H), 7.17 (d, 1H), 7.08 (s, 1H), 5.21 (s, 2H), 3.09 (s, 2H), 1.20 (s, 6H)

### Example 115: Preparation of 3-((5-chloro-3-(2,2-dimethyl-1-oxo-2,3-dihydro-1H-inden-5-yl)-2-oxo-2,3-dihydro-1H-benzo[d]imidazol-1-yl)methyl)benzoic acid

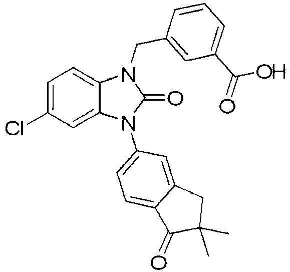

The process of the following steps A and B gave the title compound.

### Step A: Preparation of 3-((5-chloro-3-(2,2-dimethyl-3-oxo-2,3-dihydro-1H-inden-5-yl)-2-oxo-2,3-dihydro-1H-benzo[d]imidazol-1-yl)methyl)benzoic acid methyl ester

3-((5-Chloro-2-oxo-2,3-dihydro-1*H*-benzo[*d*]imidazol-1-yl)methyl)benzoic acid methyl ester obtained in Preparation Example 1 was dissolved in toluene, then 6-bromo-2,2-dimethyl-2,3-dihydro-1*H*-inden-1-one (28.6 mg, 0.12 mmol), DMEDA (22 µL, 0.20 mmol), CuI (22.4 mg, 0.11 mmol) and K₂CO₃ (33.1 mg, 0.23 mmol) were added thereto, and the resulting mixture was charged with nitrogen and then stirred at 115 °C. After the reaction was terminated, EtOAc and water were added thereto, and an organic layer was separated and dried over anhydrous MgSO₄. The filtrate was distilled under reduced pressure and then purified by MPLC to give the title compound (41.5 mg, yield: 88%) .
¹H NMR (500 MHz, CDCl₃) δ8.05 (s, 1H), 7.99 (d, 1H), 7.95 (d, 1H), 7.68 (s, 1H), 7.59 (t, 2H), 7.45 (t, 1H), 7.17 (s, 1H), 7.08 (d, 1H), 6.86 (d, 1H), 5.16 (s, 2H), 4.12 (s, 3H), 3.08 (s, 2H), 1.29 (s, 6H)

### Step B: Preparation of 3-((5-chloro-3-(2,2-dimethyl-3-oxo-2,3-dihydro-1H-inden-5-yl)-2-oxo-2,3-dihydro-1H-benzo[d]imidazol-1-yl)methyl)benzoic acid

The title compound (27.0 mg, yield: 67%) was obtained in the same manner as in step B of Example 2 by using 3-((5-chloro-3-(2,2-dimethyl-3-oxo-2,3-dihydro-1*H*-inden-5-yl)-2-oxo-2,3-dihydro-1*H*-benzo[*d*]imidazol-1-yl)methyl)benzoic acid methyl ester (41.5 mg, 0.087 mmol) obtained in step A above.
MS [M+H] = 461 (M+1)
¹H NMR (500 MHz, DMSO-d6) δ7.98 (s, 1H), 7.91 (d, 1H), 7.86 (d, 1H), 7.82 (s, 1H), 7.77 (d, 1H), 7.63 (d, 1H), 7.49 (m, 1H), 7.26 (d, 1H), 7.17 (d, 1H), 7.08 (s, 1H), 5.21 (s, 2H), 3.09 (s, 2H), 1.20 (s, 6H)

### Example 116: Preparation of 3-((5-chloro-3-(2-methyl-1-oxoisoindolin-5-yl)-2-oxo-2,3-dihydro-1H-benzo[d]imidazol-1-yl)methyl)benzoic acid

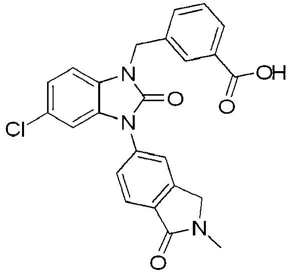

The process of the following steps A and B gave the title compound.

### Step A: Preparation of 3-((5-chloro-3-(2-methyl-1-oxoisoindolin-5-yl)-2-oxo-2,3-dihydro-1H-benzo[d]imidazol-1-yl)methyl)benzoic acid methyl ester

The title compound (108 mg, yield: 77%) was obtained in the same manner as in step C of Preparation Example 7 by using 6-chloro-1-(2-methyl-1-oxoisoindolidin-5-yl)-1,3-dihydro-2H-benzo[*d*]imidazol-2-one (95 mg, 0.303 mmol) obtained in Preparation Example 35 and 3-(bromomethyl)benzoic acid methyl ester (69 mg, 0.303 mmol).
¹H-NMR (400 MHz, CDCl3) δ8.05 (d, 1H), 8.02 (d, 1H), 8.00 (dd, 1H), 7.70 (d, 1H), 7.64 (dd, 1H), 7.56 (dd, 1H), 7.44 (t, 1H), 7.12 (d, 1H), 7.06 (dd, 1H), 6.85 (d, 1H), 5.17 (s, 2H), 4.48 (s, 2H), 3.92 (s, 3H), 3.25 (s, 3H)

### Step B: Preparation of 3-((5-chloro-3-(2-methyl-1-oxoisoindolin-5-yl)-2-oxo-2,3-dihydro-1H-benzo[d]imidazol-1-yl)methyl)benzoic acid

The title compound (65 mg, yield: 67%) was obtained in the same manner as in step B of Example 24 by using 3-((5-chloro-3-(2-methyl-1-oxoisoindolin-5-yl)-2-oxo-2,3-dihydro-1*H*-benzo[*d*]imidazol-1-yl)methyl)benzoic acid methyl ester (100 mg, 0.216 mmol) in step A above.
MS [M-H] = 446 (M-1)
¹H-NMR (400 MHz, DMSO-d6) δ7.99 (d, 1H), 7.85 (3H), 7.71 (dd, 1H), 7.64 (dd, 1H), 7.50 (t, 1H), 7.29 (d, 1H), 7.19 (dd, 1H), 7.17 (d, 1H), 5.23 (s, 2H), 4.56 (s, 2H), 3.11 (s, 3H)

### Example 117: Preparation of 3-((5-chloro-3-(2-methyl-1-oxoisoindolin-5-yl)-2-oxo-2,3-dihydro-1H-benzo[d]imidazol-1-yl)methyl)-2-fluorobenzoic acid

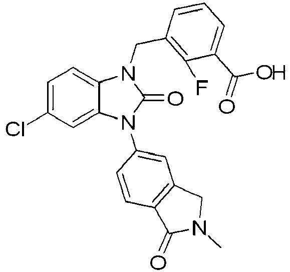

The process of the following steps A and B gave the title compound.

### Step A: Preparation of 3-((5-chloro-3-(2-methyl-1-oxoisoindolin-5-yl)-2-oxo-2,3-dihydro-1H-benzo[d]imidazol-1-yl)methyl)-2-fluorobenzoic acid methyl ester

The title compound (68 mg, yield: 47%) was obtained in the same manner as in step C of Preparation Example 7 by using 6-chloro-1-(2-methyl-1-oxoisoindolidin-5-yl)-1,3-dihydro-2*H-*benzo[*d*]imidazol-2-one (95 mg, 0.303 mmol) obtained in Preparation Example 35 and 3-(bromomethyl)-2-fluorobenzoic acid methyl ester (75 mg, 0.303 mmol).
¹H-NMR (400 MHz, CDCl3) δ8.00 (d, 1H), 7.89 (td, 1H), 7.67 (d, 1H), 7.61 (2H), 7.18 (t, 1H), 7.12 (2H), 7.03 (dd, 1H), 5.20 (s, 2H), 4.47 (s, 2H), 3.95 (s, 3H), 3.24 (s, 3H)

### Step B: Preparation of 3-((5-chloro-3-(2-methyl-1-oxoisoindolin-5-yl)-2-oxo-2,3-dihydro-1H-benzo[d]imidazol-1-yl)methyl)-2-fluorobenzoic acid

The title compound (42 mg, yield: 66%) was obtained in the same manner as in step B of Example 24 by using 3-((5-chloro-3-(2-methyl-1-oxoisoindolin-5-yl)-2-oxo-2,3-dihydro-1*H*-benzo[*d*]imidazol-1-yl)methyl)-2-fluorobenzoic acid methyl ester (65 mg, 0.13 mmol) obtained in step A above.
MS [M-H] = 464 (M-1)
¹H-NMR (400 MHz, DMSO-d6) δ7.84 (2H), 7.80 (dd, 1H), 7.69 (dd, 1H), 7.56 (m, 1H), 7.27 (2H), 7.21 (dd, 1H), 7.17 (d, 1H), 5.24 (s, 2H), 4.56 (s, 2H), 3.11 (s, 3H)

### Example 118: Preparation of 3-((5-chloro-3-(4-(4,4-difluoropiperidin-1-yl)phenyl)-2-oxo-2,3-dihydro-1H-benzo[d]imidazol-1-yl)methyl)-2-fluorobenzoic acid

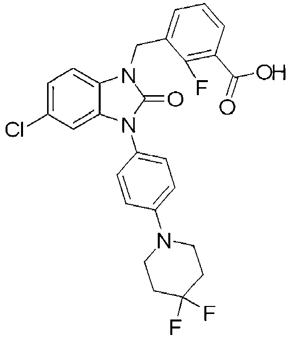

The process of the following steps A and B gave the title compound.

### Step A: Preparation of 3-((5-chloro-3-(4-(4,4-difluoropiperidin-1-yl)phenyl)-2-oxo-2,3-dihydro-1H-benzo[d]imidazol-1-yl)methyl)-2-fluorobenzoic acid methyl ester

The title compound (60 mg, yield: 56%) was obtained in the same manner as in step C of Preparation Example 7 by using 6-chloro-1-(4-(4,4-difluoropiperidin-1-yl)phenyl)-1,3-dihydro-2*H*-benzo[*d*]imidazol-2-one (73 mg, 0.20 mmol) obtained in Preparation Example 28 and 3-(bromomethyl)-2-fluorobenzoic acid methyl ester (50 mg, 0.20 mmol).
¹H-NMR (400 MHz, CDCl3) δ7.88 (td, 1H), 7.62 (td, 1H), 7.37 (d, 2H), 7.17 (t, 1H), 7.04 (3H), 7.00 (d, 1H), 6.96 (d, 1H), 5.19 (s, 2H), 3.95 (s, 3H), 3.43 (m, 4H), 2.11 (m, 4H)

### Step B: Preparation of 3-((5-chloro-3-(4-(4,4-difluoropiperidin-1-yl)phenyl)-2-oxo-2,3-dihydro-1H-benzo[d]imidazol-1-yl)methyl)-2-fluorobenzoic acid

The title compound (40 mg, yield: 68%) was obtained in the same manner as in step B of Example 24 by using 3-((5-chloro-3-(4-(4,4-difluoropiperidin-1-yl)phenyl)-2-oxo-2,3-dihydro-1*H*-benzo[*d*]imidazol-1-yl)methyl)-2-fluorobenzoic acid methyl ester (60 mg, 0.11 mmol) obtained in step A above.
MS [M+H] = 516 (M+1)
¹H-NMR (400 MHz, CDCl3) δ7.91 (m, 1H), 7.58 (m, 1H), 7.37 (m, 2H), 7.15 (t, 1H), 7.06 (3H), 6.98 (2H), 5.19 (s, 2H), 3.44 (m, 4H), 2.11 (m, 4H)

### Example 119: Preparation of 3-((5-chloro-3-(4-(3,3-difluoropyrrolidin-1-yl)phenyl)-4-fluoro-2-oxo-2,3-dihydro-1H-benzo[d]imidazol-1-yl)methyl)-2-fluorobenzoic acid

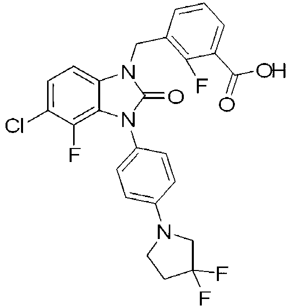

The process of the following steps A and B gave the title compound.

### Step A: Preparation of 3-((5-chloro-3-(4-(3,3-difluoropyrrolidin-1-yl)phenyl)-4-fluoro-2-oxo-2,3-dihydro-1H-benzo[d]imidazol-1-yl)methyl)-2-fluorobenzoic acid methyl ester

The title compound (74 mg, yield: 69%) was obtained in the same manner as in step C of Preparation Example 7 by using 6-chloro-1-(4-(3,3-difluoropyrrolidin-1-yl)phenyl)-7-fluoro-1,3-dihydro-2*H*-benzo[*d*]imidazol-2-one (74 mg, 0.20 mmol) obtained in Preparation Example 32 and 3-(bromomethyl)-2-fluorobenzoic acid methyl ester (50 mg, 0.20 mmol).
¹H-NMR (400 MHz, CDCl3) δ7.88 (td, 1H), 7.62 (td, 1H), 7.32 (dd, 2H), 7.17 (t, 1H), 7.08 (dd, 1H), 6.81 (dd, 1H), 6.62 (d, 2H), 5.17 (s, 2H), 3.95 (s, 3H), 3.72 (t, 2H), 3.58 (t, 2H), 2.52 (m, 2H)

### Step B: Preparation of 3-((5-chloro-3-(4-(3,3-difluoropyrrolidin-1-yl)phenyl)-4-fluoro-2-oxo-2,3-dihydro-1H-benzo[d]imidazol-1-yl)methyl)-2-fluorobenzoic acid

The title compound (69 mg, yield: 96%) was obtained in the same manner as in step B of Example 24 by using 3-((5-chloro-3-(4-(3,3-difluoropyrrolidin-1-yl)phenyl)-4-fluoro-2-oxo-2,3-dihydro-1*H*-benzo[*d*]imidazol-1-yl)methyl)-2-fluorobenzoic acid methyl ester (74 mg, 0.14 mmol) obtained in step A above.
MS [M+H] = 520 (M+1)
¹H-NMR (400 MHz, DMSO-d6) δ7.80 (m, 1H), 7.53 (m, 1H), 7.36 (d, 2H), 7.27 (2H), 7.07 (d, 1H), 6.70 (d, 2H), 5.21 (s, 2H), 3.76 (t, 2H), 3.54 (t, 2H), 2.55 (m, 2H)

### Example 120: Preparation of 3-((5-chloro-4-fluoro-3-(4-(3-hydroxyazetidin-1-yl)phenyl)-2-oxo-2,3-dihydro-1H-benzo[d]imidazol-1-yl)methyl)-2-fluorobenzoic acid

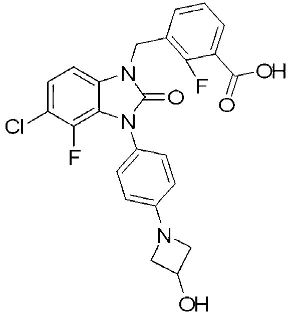

The process of the following steps A and B gave the title compound.

### Step A: Preparation of 3-((5-chloro-4-fluoro-3-(4-(3-hydroxyazetidin-1-yl)phenyl)-2-oxo-2,3-dihydro-1H-benzo[d]imidazol-1-yl)methyl)-2-fluorobenzoic acid methyl ester

The title compound (71 mg, yield: 66%) was obtained in the same manner as in step C of Preparation Example 7 by using 1-(4-(6-chloro-7-fluoro-2-oxo-2,3-dihydro-1*H-*benzo[*d*]imidazol-1-yl)phenyl)azetidin-3-yl acetate (75 mg, 0.20 mmol) obtained in Preparation Example 14 and 3-(bromomethyl)-2-fluorobenzoic acid methyl ester (50 mg, 0.20 mmol).
¹H-NMR (400 MHz, CDCl3) δ7.88 (td, 1H), 7.61 (td, 1H), 7.27 (m, 2H), 7.17 (t, 1H), 7.07 (dd, 1H), 6.80 (d, 1H), 6.52 (d, 2H), 5.33 (m, 1H), 5.17 (s, 2H), 4.29 (m, 2H), 3.95 (s, 3H), 3.86 (m, 2H), 2.12 (s, 3H)

### Step B: Preparation of 3-((5-chloro-4-fluoro-3-(4-(3-hydroxyazetidin-1-yl)phenyl)-2-oxo-2,3-dihydro-1H-benzo[d]imidazol-1-yl)methyl)-2-fluorobenzoic acid

The title compound (59 mg, yield: 93%) was obtained in the same manner as in step B of Example 24 by using 3-((5-chloro-4-fluoro-3-(4-(3-hydroxyazetidin-1-yl)phenyl)-2-oxo-2,3-dihydro-1*H*-benzo[*d*]imidazol-1-yl)methyl)-2-fluorobenzoic acid methyl ester (71 mg, 0.13 mmol) obtained in step A above.
MS [M+H] = 486 (M+1)
¹H-NMR (400 MHz, DMSO-d6) δ7.80 (m, 1H), 7.51 (m, 1H), 7.27 (4H), 7.08 (d, 1H), 6.51 (d, 2H), 5.63 (m, 1H), 5.20 (s, 2H), 4.59 (m, 1H), 4.13 (t, 2H), 3.57 (m, 2H)

### Example 121: Preparation of 3-(1-(5-chloro-3-(4-(3,6-dihydro-2H-pyran-4-yl)phenyl)-2-oxo-2,3-dihydro-1H-benzo[d]imidazol-1-yl)cyclopropyl)benzoic acid

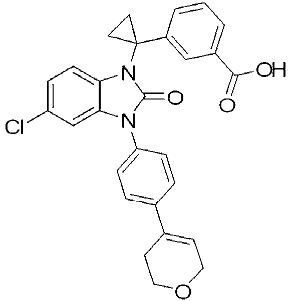

The process of the following steps A, B, and C gave the title compound.

### Step A: Preparation of 3-(1-(3-(4-bromophenyl)-5-chloro-2-oxo-2,3-dihydro-1H-benzo[d]imidazol-1-yl)cyclopropyl)benzoic acid methyl ester

The title compound (204 mg, yield: 70%) was obtained in the same manner as in step A of Preparation Example 25 by using 3-(1-(5-chloro-2-oxo-2,3-dihydro-1*H*-benzo[*d*]imidazol-1-yl)cyclopropyl)benzoic acid methyl ester (200 mg, 0.583 mmol) obtained in Preparation Example 3 and (4-bromophenyl)boronic acid (176 mg, 0.875 mmol).
¹H-NMR (500 MHz, CDCl3) δ7.92 (d, 1H), 7.89 (s, 1H), 7.69 (d, 2H), 7.51 (d, 1H), 7.45 (d, 2H), 7.38 (t, 1H), 7.16 (d, 1H), 7.12 (d, 1H), 7.08 (s, 1H), 3.92 (s, 3H), 1.74 (m, 2H), 1.70 (m, 2H)

### Step B: Preparation of 3-(1-(5-chloro-3-(4-(3,6-dihydro-2H-pyran-4-yl)phenyl)-2-oxo-2,3-dihydro-1H-benzo[d]imidazol-1-yl)cyclopropyl)benzoic acid methyl ester

The title compound (34 mg, yield: 48.3%) was obtained in the same manner as in step B of Example 109 by using 3-(1-(3-(4-bromophenyl)-5-chloro-2-oxo-2,3-dihydro-1*H-*benzo[*d*]imidazol-1-yl)cyclopropyl)benzoic acid methyl ester (70 mg, 0.141 mmol) obtained in step A above and 2-(3,6-dihydro-2*H*-pyran-4-yl)-4,4,5,5-tetramethyl-1,3,2-dioxaborolane (44.3 mg, 0.211 mmol).
¹H-NMR (500 MHz, CDCl3) δ7.91 (m, 2H), 7.57 (d, 2H), 7.52 (d, 3H), 7.38 (t, 1H), 7.16 (d, 1H), 7.11 (m, 2H), 6.22 (s, 1H), 4.38 (s, 2H), 3.99 (t, 2H), 3.92 (s, 3H), 2.58 (m 2H), 1.75 (m, 2H), 1.70 (m, 2H)

### Step C: Preparation of 3-(1-(5-chloro-3-(4-(3,6-dihydro-2H-pyran-4-yl)phenyl)-2-oxo-2,3-dihydro-1H-benzo[d]imidazol-1-yl)cyclopropyl)benzoic acid

The title compound (15.6 mg, yield: 47.2%) was obtained in the same manner as in step B of Example 24 by using 3-(1-(5-chloro-3-(4-(3,6-dihydro-2*H*-pyran-4-yl)phenyl)-2-oxo-2,3-dihydro-1*H*-benzo[*d*]imidazol-1-yl)cyclopropyl)benzoic acid methyl ester (34 mg, 0.068 mmol) obtained in step B above.
MS [M+H] =487 (M+1)
¹H-NMR (500 MHz, CDCl3) 7.95 (m, 2H), 7.54 (m, 5H), 7.38 (t, 1H), 7.16 (d, 1H), 7.10 (d, 2H), 6.21 (s, 1H), 4.37 (s, 2H), 3.98 (t, 2H), 2.57 (m, 2H), 1.75 (s, 2H), 1.69 (s, 2H)

### Example 122: Preparation of 3-(1-(5-chloro-3-(4-(3,6-dihydro-2H-pyran-4-yl)phenyl)-2-oxo-2,3-dihydro-1H-benzo[d]imidazol-1-yl)cyclopropyl)-2-fluorobenzoic acid

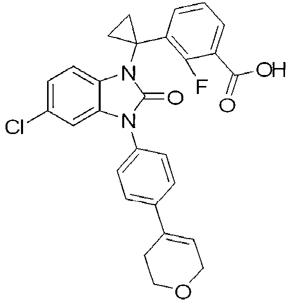

The process of the following steps A, B, and C gave the title compound.

### Step A: Preparation of 3-(1-(3-(4-bromophenyl)-5-chloro-2-oxo-2,3-dihydro-1H-benzo[d]imidazol-1-yl)cyclopropyl)-2-fluorobenzoic acid methyl ester

The title compound (187 mg, yield: 65.4%) was obtained in the same manner as in step A of Preparation Example 25 by using 3-(1-(5-chloro-2-oxo-2,3-dihydro-1*H*-benzo[*d*]imidazol-1-yl)cyclopropyl)-2-fluorobenzoic acid methyl ester (200 mg, 0.554 mmol) obtained in Preparation Example 4 and (4-bromophenyl)boronic acid (167 mg, 0.832 mmol).

### Step B: Preparation of 3-(1-(5-chloro-3-(4-(3,6-dihydro-2H-pyran-4-yl)phenyl)-2-oxo-2,3-dihydro-1H-benzo[d]imidazol-1-yl)cyclopropyl)-2-fluorobenzoic acid methyl ester

The title compound (44.7 mg, yield: 40.4%) was obtained in the same manner as in step B of Example 109 by using 3-(1-(3-(4-bromophenyl)-5-chloro-2-oxo-2,3-dihydro-1*H-*benzo[*d*]imidazol-1-yl)cyclopropyl)-2-fluorobenzoic acid methyl ester (110 mg, 0.213 mmol) obtained in step A above and 2-(3,6-dihydro-2*H*-pyran-4-yl)-4,4,5,5-tetramethyl-1,3,2-dioxaborolane (67.2 mg, 0.32 mmol).
¹H-NMR (500 MHz, CDCl3) δ8.18 (t, 1H), 7.87 (t, 1H), 7.54 (m, 3H), 7.45 (d, 2H), 7.19 (m, 2H), 7.04 (s, 1H), 6.19 (s, 1H), 4.37 (m, 2H), 3.98 (t, 2H), 3.93 (s, 3H), 2.56 (m, 2H), 1.69 (m, 2H), 1.65 (m, 2H)

### Step C: Preparation of 3-(1-(5-chloro-3-(4-(3,6-dihydro-2H-pyran-4-yl)phenyl)-2-oxo-2,3-dihydro-1H-benzo[d]imidazol-1-yl)cyclopropyl)-2-fluorobenzoic acid

The title compound (41.3 mg, yield: 95.3%) was obtained in the same manner as in step B of Example 24 by using 3-(1-(5-chloro-3-(4-(3,6-dihydro-2*H*-pyran-4-yl)phenyl)-2-oxo-2,3-dihydro-1*H*-benzo[*d*]imidazol-1-yl)cyclopropyl)-2-fluorobenzoic acid methyl ester (44.7 mg, 0.086 mmol) obtained in step B above.
MS [M+H] = 505 (M+1)
¹H-NMR (500 MHz, CDCl3) δ8.21 (t, 1H), 7.92 (t, 1H), 7.51 (m, 3H), 7.41 (d, 2H), 7.20 (t, 1H), 7.14 (dd, 1H), 7.0 (d, 1H), 6.15 (s, 1H), 4.33 (m, 2H), 3.94 (m, 2H), 2.52 (m, 2H), 1.69 (m, 2H), 1.62 (m, 2H)

### Example 123: Preparation of 3-(1-(5-chloro-3-(4-(2,5-dihydrofuran-3-yl)phenyl)-2-oxo-2,3-dihydro-1H-benzo[d]imidazol-1-yl)cyclopropyl)-2-fluorobenzoic acid

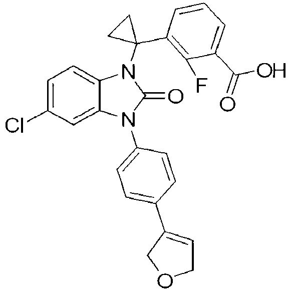

The process of the following steps A and B gave the title compound.

### Step A: Preparation of 3-(1-(5-chloro-3-(4-(2,5-dihydrofuran-3-yl)phenyl)-2-oxo-2,3-dihydro-1H-benzo[d]imidazol-1-yl)cyclopropyl)-2-fluorobenzoic acid methyl ester

The title compound (48 mg, yield: 44.6%) was obtained in the same manner as in step B of Example 109 by using 3-(1-(3-(4-bromophenyl)-5-chloro-2-oxo-2,3-dihydro-1*H-*benzo[*d*]imidazol-1-yl)cyclopropyl)-2-fluorobenzoic acid methyl ester (110 mg, 0.213 mmol) obtained in step A of Example 122 and 2-(2,5-dihydrofuran-3-yl)-4,4,5,5-tetramethyl-1,3,2-dioxaborolane (62.7 mg, 0.32 mmol).
¹H-NMR (500 MHz, CDCl3) δ8.17 (t, 1H), 7.87 (t, 1H), 7.56 (d, 1H), 7.48 (m, 4H), 7.19 (m, 2H), 7.04 (s, 1H), 6.31 (s, 1H), 5.04 (s, 2H), 4.90 (s, 2H), 3.93 (s, 3H), 1.71 (m, 2H), 1.65 (m, 2H)

### Step B: Preparation of 3-(1-(5-chloro-3-(4-(2,5-dihydrofuran-3-yl)phenyl)-2-oxo-2,3-dihydro-1H-benzo[d]imidazol-1-yl)cyclopropyl)-2-fluorobenzoic acid

The title compound (22.5 mg, yield: 48.4%) was obtained in the same manner as in step B of Example 24 by using 3-(1-(5-chloro-3-(4-(2,5-dihydrofuran-3-yl)phenyl)-2-oxo-2,3-dihydro-1*H*-benzo[*d*]imidazol-1-yl)cyclopropyl)-2-fluorobenzoic acid methyl ester (48 mg, 0.095 mmol) obtained in step A above.
MS [M+H] = 491 (M+1)
¹H-NMR (500 MHz, CDCl3) δ7.99 (t, 1H), 7.73 (t, 1H), 7.41 (m, 5H), 6.98 (m, 3H), 6.25 (s, 1H), 4.98 (s, 2H), 4.87 (s, 2H), 1.57 (m, 2H), 1.49 (m, 2H)

### Example 124: Preparation of 3-(1-(5-chloro-3-(4-(2,5-dihydrofuran-3-yl)phenyl)-2-oxo-2,3-dihydro-1H-benzo[d]imidazol-1-yl)cyclopropyl)benzoic acid

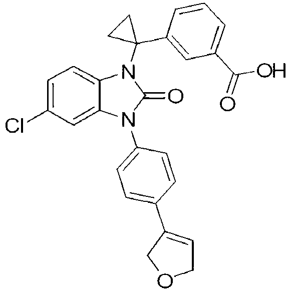

The process of the following steps A and B gave the title compound.

### Step A: Preparation of 3-(1-(5-chloro-3-(4-(2,5-dihydrofuran-3-yl)phenyl)-2-oxo-2,3-dihydro-1H-benzo[d]imidazol-1-yl)cyclopropyl)benzoic acid methyl ester

The title compound (148 mg, yield: 76%) was obtained in the same manner as in step B of Example 109 by using 3-(1-(3-(4-bromophenyl)-5-chloro-2-oxo-2,3-dihydro-1*H-*benzo[*d*]imidazol-1-yl)cyclopropyl)benzoic acid methyl ester (200 mg, 0.402 mmol) obtained in step A of Example 121 and 2-(2,5-dihydrofuran-3-yl)-4,4,5,5-tetramethyl-1,3,2-dioxaborolane (118 mg, 0.603 mmol).
¹H-NMR (400 MHz, CDCl3) δ7.89 (d, 1H), 7.86 (s, 1H), 7.50 (m, 5H), 7.35 (t, 1H), 7.11 (d, 1H), 7.08 (m, 2H), 6.29 (m, 1H), 5.02 (m, 2H), 4.88 (m, 2H), 3.88 (s, 3H), 1.73 (m, 2H), 1.67 (m, 2H)

### Step B: Preparation of 3-(1-(5-chloro-3-(4-(2,5-dihydrofuran-3-yl)phenyl)-2-oxo-2,3-dihydro-1H-benzo[d]imidazol-1-yl)cyclopropyl)benzoic acid

The title compound (60 mg, yield: 44.1%) was obtained in the same manner as in step B of Example 24 by using 3-(1-(5-chloro-3-(4-(2,5-dihydrofuran-3-yl)phenyl)-2-oxo-2,3-dihydro-1*H*-benzo[*d*]imidazol-1-yl)cyclopropyl)benzoic acid methyl ester (140 mg, 0.288 mmol) obtained in step A above.
MS [M+H] = 473 (M+1)
¹H-NMR (500 MHz, CDCl3) δ7.98 (d, 1H), 7.94 (s, 1H), 7.55 (m, 3H), 7.51 (d, 2H), 7.42 (t, 1H), 7.17 (d, 1H,), 7.12 (s, 2H), 6.33 (s, 1H), 5.06 (s, 2H), 4.91 (s, 2H), 1.77 (s, 2H), 1.71 (s, 2H)

### Example 125: Preparation of 4-((5-chloro-4-fluoro-3-(4-morpholinophenyl)-2-oxo-2,3-dihydro-1H-benzo[d]imidazol-1-yl)methyl)benzoic acid

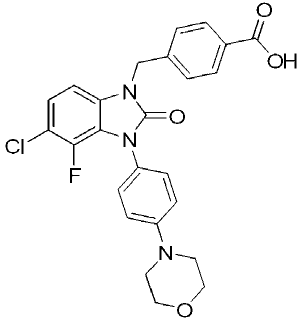

The process of the following steps A and B gave the title compound.

### Step A: Preparation of 4-((5-chloro-4-fluoro-3-(4-morpholinophenyl)-2-oxo-2,3-dihydro-1H-benzo[d]imidazol-1-yl)methyl)benzoic acid methyl ester

The title compound (50 mg, yield: 70%) was obtained in the same manner as in step C of Preparation Example 7 by using 6-chloro-7-fluoro-1-(4-morpholinophenyl)-1,3-dihydro-2*H-*benzo[*d*]imidazol-2-one (50 mg, 0.144 mmol) obtained in Preparation Example 11 and 4-(bromomethyl)benzoic acid methyl ester (33 mg, 0.144 mmol).
¹H-NMR (400 MHz, CDCl3) δ8.02 (d, 2H), 7.41 (d, 2H), 7.37 (dd, 2H), 7.04 (dd, 1H), 7.00 (d, 2H), 6.60 (dd, 1H), 5.16 (s, 2H), 3.93 (s, 3H), 3.89 (m, 4H), 3.23 (m, 4H)

### Step B: Preparation of 4-((5-chloro-4-fluoro-3-(4-morpholinophenyl)-2-oxo-2,3-dihydro-1H-benzo[d]imidazol-1-yl)methyl)benzoic acid

The title compound (24 mg, yield: 49%) was obtained in the same manner as in step B of Example 24 by using 4-((5-chloro-4-fluoro-3-(4-morpholinophenyl)-2-oxo-2,3-dihydro-1*H-*benzo[*d*]imidazol-1-yl)methyl)benzoic acid methyl ester (50 mg, 0.10 mmol) obtained in step A above.
MS [M+H] = 482 (M+1)
¹H-NMR (400 MHz, DMSO-d6) δ7.93 (d, 2H), 7.49 (d, 2H), 7.40 (dd, 2H), 7.25 (m, 1H), 7.08 (3H), 5.21 (s, 2H), 3.77 (m, 4H), 3.20 (m, 4H)

### Example 126: Preparation of 3-(1-(5-chloro-3-(4-(morpholine-4-carbonyl)phenyl)-2-oxo-2,3-dihydro-1H-benzo[d]imidazol-1-yl)cyclopropyl)benzoic acid

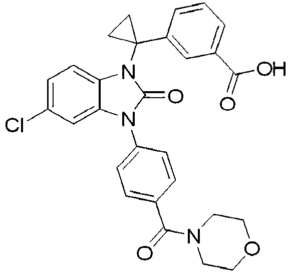

The process of the following steps A and B gave the title compound.

### Step A: Preparation of 3-(1-(5-chloro-3-(4-(morpholine-4-carbonyl)phenyl)-2-oxo-2,3-dihydro-1H-benzo[d]imidazol-1-yl)cyclopropyl)benzoic acid methyl ester

The title compound (46 mg, yield: 88%) was obtained in the same manner as in step C of Example 56 by using 4-(6-chloro-3-(1-(3-(methoxycarbonyl)phenyl)cyclopropyl)-2-oxo-2,3-dihydro-1*H*-benzo[*d*]imidazol-1-yl)benzoic acid (50 mg, 0.18 mmol) obtained step B of Example 63 and morpholine (17.1 mg, 0.196 mmol).
¹H-NMR (500 MHz, CDCl3) δ7.92 (m, 2H), 7.63 (m, 4H), 7.52 (d, 1H), 7.39 (t, 1H), 7.18 (d, 1H), 7.14 (m, 2H), 3.92 (s, 3H), 3.79 (m, 6H), 3.73 (m, 2H), 1.75 (m, 2H), 1.71 (m, 2H)

### Step B: Preparation of 3-(1-(5-chloro-3-(4-(morpholine-4-carbonyl)phenyl)-2-oxo-2,3-dihydro-1H-benzo[d]imidazol-1-yl)cyclopropyl)benzoic acid

The title compound (30 mg, yield: 67.4%) was obtained in the same manner as in step B of Example 2 by using 3-(1-(5-chloro-3-(4-(morpholine-4-carbonyl)phenyl)-2-oxo-2,3-dihydro-1*H*-benzo[*d*]imidazol-1-yl)cyclopropyl)benzoic acid methyl ester (46 mg, 0.086 mmol) obtained used step A above.
¹H NMR (500 MHz, CDCl3) δ7.95 (m, 1H), 7.91 (d, 1H), 7.61 (m, 4H), 7.57 (d, 1H), 7.39 (m, 1H), 7.11 (m, 3H), 3.79 (m, 6H), 3.66 (m, 2H), 1.73 (m, 2H), 1.68 (m, 2H)

### Example 127: Preparation of (S)-3-(1-(5-chloro-3-(4-(3-methylmorpholine-4-carbonyl)phenyl)-2-oxo-2,3-dihydro-1H-benzo[d]imidazol-1-yl)cyclopropyl)benzoic acid

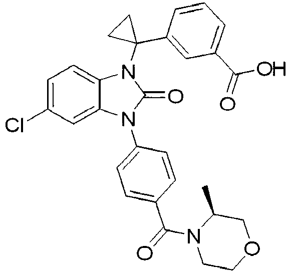

The title compound (15 mg, yield: 26%) was obtained in the same manner as in step C of Example 56 and step B of Example 24 by using 4-(6-chloro-3-(1-(3-(methoxycarbonyl)phenyl)cyclopropyl)-2-oxo-2,3-dihydro-1*H-*benzo[*d*]imidazol-1-yl)benzoic acid (50 mg, 0.108 mmol) obtained step B of Example 63 and (*S*)-3-methylmorpholine.
MS [M+H] = 532 (M+1)
¹H-NMR (400 MHz, MeOH-d4) δ7.89 (m, 1H), 7.84 (d, 1H), 7.70 (d, 2H), 7.64 (d, 2H), 7.42 (2H), 7.28 (d, 1H), 7.18 (dd, 1H), 7.15 (m, 1H), 3.90 (m, 1H), 3.70 (m, 2H), 3.51 (2H), 3.30 (m, 2H) 1.74 (m, 4H), 1.40 (d, 3H)

### Example 128: Preparation of 3-(1-(5-chloro-3-(4-(3-hydroxyazetidine-1-carbonyl)phenyl)-2-oxo-2,3-dihydro-1H-benzo[d]imidazol-1-yl)cyclopropyl)benzoic acid

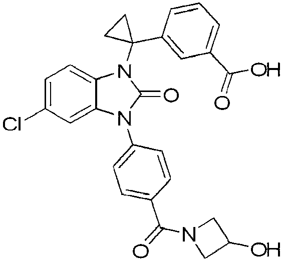

The title compound (13 mg, yield: 24%) was obtained in the same manner as in step C of Example 56 and step B of Example 24 by using 4-(6-chloro-3-(1-(3-(methoxycarbonyl)phenyl)cyclopropyl)-2-oxo-2,3-dihydro-1*H-*benzo[*d*]imidazol-1-yl)benzoic acid (50 mg, 0.108 mmol) obtained in step B of Example 63 and 3-hydroxyazetidine hydrochloride.
MS [M+H] = 504 (M+1)
¹H-NMR (400 MHz, MeOH-d4) δ7.88 (3H), 7.83 (m, 1H), 7.71 (d, 2H), 7.42 (2H), 7.28 (d, 1H), 7.18 (dd, 1H), 7.15 (m, 1H), 4.63 (m, 2H), 4.43 (m, 1H), 4.23 (m, 1H), 4.00 (m, 1H), 1.74 (m, 4H)

### Example 129: Preparation of 3-(1-(5-chloro-3-(4-(3,3-difluoroazetidine-1-carbonyl)phenyl) -2-oxo-2,3-dihydro-1H-benzo[d]imidazol-1-yl)cyclopropyl)benzoic acid

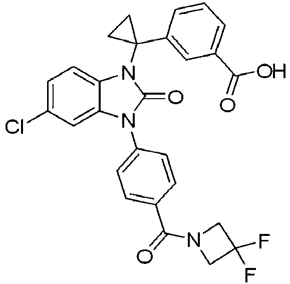

The title compound (15 mg, yield: 21%) was obtained in the same manner as in step C of Example 56 and step B of Example 2 by using 4-(6-chloro-3-(1-(3-(methoxycarbonyl)phenyl)cyclopropyl)-2-oxo-2,3-dihydro-1*H-*benzo[*d*]imidazol-1-yl)benzoic acid (60 mg, 0.13 mmol) obtained in step B of Example 63 and 3,3-difluoroazetidine hydrochloride (25 mg, 0.19 mmol).
¹H NMR (400 MHz, MeOD) δ8.25 (d, 2H), 7.90 (d, 1H), 7.86 (s, 1H), 7.72 (d, 2H), 7.44 (m, 2H), 7.30 (d, 1H), 7.20 (m, 2H), 1.77 (m, 4H), 1.33 (m, 2H), 1.92 (m, 2H)

### Example 130: Preparation of 3-(1-(5-chloro-3-(4-(3,3-difluoropyrrolidine-1-carbonyl)phenyl)-2-oxo-2,3-dihydro-1H-benzo[d]imidazol-1-yl)cyclopropyl)benzoic acid

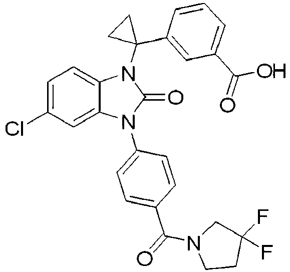

The title compound (23 mg, yield: 33%) was obtained in the same manner as in step C of Example 56 and step B of Example 2 by using 4-(6-chloro-3-(1-(3-(methoxycarbonyl)phenyl)cyclopropyl)-2-oxo-2,3-dihydro-1*H-*benzo[*d*]imidazol-1-yl)benzoic acid (60 mg, 0.13 mmol) obtained in step B of Example 63 and 3,3-difluoropyrrolidine hydrochloride (28 mg, 0.19 mmol).
¹H NMR (400 MHz, MeOD) δ7.88 (m, 2H), 7.79 (m, 2H), 7.73 (m, 2H), 7.31 (m, 3H), 7.17 (m, 2H), 3.92 (m, 4H), 2.49 (m, 2H), 1.72 (m, 4H)

### Example 131: Preparation of 3-(1-(5-chloro-3-(4-(4,4-difluoropiperidine-1-carbonyl)phenyl)-2-oxo-2,3-dihydro-1H-benzo[d]imidazol-1-yl)cyclopropyl)benzoic acid

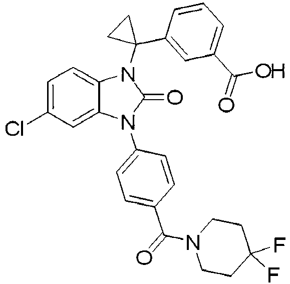

The title compound (27 mg, yield: 38%) was obtained in the same manner as in step C of Example 56 and step B of Example 2 by using 4-(6-chloro-3-(1-(3-(methoxycarbonyl)phenyl)cyclopropyl)-2-oxo-2,3-dihydro-1*H-*benzo[*d*]imidazol-1-yl)benzoic acid (60 mg, 0.13 mmol) obtained in step B of Example 63 and 4,4-difluoropiperidine hydrochloride (30 mg, 0.19 mmol).
¹H NMR (400 MHz, MeOD) δ7.88 (m, 2H), 7.71 (m, 4H), 7.31 (m, 3H), 7.17 (m, 2H), 3.78 (m, 4H), 2.05 (m, 4H), 1.72 (m, 4H)

### Example 132: Preparation of 3-(1-(5-chloro-3-(4-(3,3-difluoropyrrolidine-1-carbonyl)phenyl)-2-oxo-2,3-dihydro-1H-benzo[d]imidazol-1-yl)cyclopropyl)-2-fluorobenzoic acid

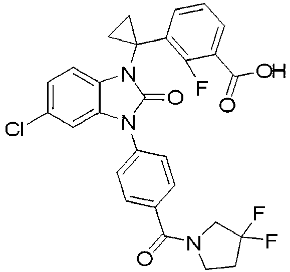

The process of the following steps A and B gave the title compound.

### Step A: Preparation of 3-(1-(5-chloro-3-(4-(3,3-difluoropyrrolidine-1-carbonyl)phenyl)-2-oxo-2,3-dihydro-1H-benzo[d]imidazol-1-yl)cyclopropyl)-2-fluorobenzoic acid methyl ester

The title compound (27.7 mg, yield: 46.7%) was obtained in the same manner as in step C of Example 56 by using 4-(6-chloro-3-(1-(2-fluoro-3-(methoxycarbonyl)phenyl)cyclopropyl)-2-oxo-2,3-dihydro-1*H*-benzo[*d*]imidazol-1-yl)benzoic acid (50 mg, 0.10 mmol) obtained in step B of Example 67 and 3,3-difluoropyrrolidine hydrochloride (30 mg, 0.208 mmol).
¹H-NMR (500 MHz, CDCl3) δ8.16 (t, 1H), 7.88 (t, 1H), 7.69 (m, 2H), 7.58 (m, 3H), 7.21 (m, 2H), 7.07 (s, 1H), 4.04 (m, 2H), 3.94 (s, 3H), 3.80 (m, 2H), 2.45 (m, 2H), 1.71 (m, 2H), 1.65 (m, 2H)

### Step B: Preparation of 3-(1-(5-chloro-3-(4-(3,3-difluoropyrrolidine-1-carbonyl)phenyl)-2-oxo-2,3-dihydro-1H-benzo[d]imidazol-1-yl)cyclopropyl)-2-fluorobenzoic acid

The title compound (12 mg, yield: 45.6%) was obtained in the same manner as in step B of Example 2 by using 3-(1-(5-chloro-3-(4-(3,3-difluoropyrrolidine-1-carbonyl)phenyl)-2-oxo-2,3-dihydro-1*H*-benzo[*d*]imidazol-1-yl)cyclopropyl)-2-fluorobenzoic acid methyl ester (27 mg, 0.047 mmol) obtained in step A above.
¹H-NMR (500 MHz, DMSO-d6) δ7.94 (m, 1H), 7.73 (m, 3H), 7.63 (m, 2H), 7.51 (d, 1H), 7.27 (m, 2H), 7.10 (d, 1H), 3.95 (m, 2H), 3.74 (m, 2H), 2.50 (m, 2H), 1.65 (m, 2H), 1.63 (m, 2H)

### Example 133: Preparation of 3-(1-(5-chloro-3-(4-(4,4-difluoropiperidine-1-carbonyl)phenyl)-2-oxo-2,3-dihydro-1H-benzo[d]imidazol-1-yl)cyclopropyl)-2-fluorobenzoic acid

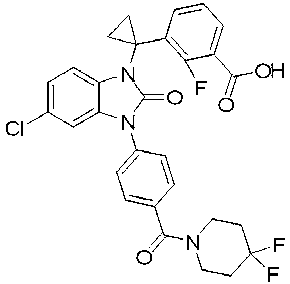

The process of the following steps A and B gave the title compound.

### Step A: Preparation of 3-(1-(5-chloro-3-(4-(4,4-difluoropiperidine-1-carbonyl)phenyl)-2-oxo-2,3-dihydro-1H-benzo[d]imidazol-1-yl)cyclopropyl)-2-fluorobenzoic acid methyl ester

The title compound (35.7 mg, yield: 58.8%) was obtained in the same manner as in step C of Example 56 by using 4-(6-chloro-3-(1-(2-fluoro-3-(methoxycarbonyl)phenyl)cyclopropyl)-2-oxo-2,3-dihydro-1*H*-benzo[*d*]imidazol-1-yl)benzoic acid (50 mg, 0.10 mmol) obtained in step B of Example 67 and 4,4-difluoropiperidine (25.2 mg, 0.208 mmol).
¹H-NMR (500 MHz, CDCl3) δ8.14 (t, 1H), 7.86 (t, 1H), 7.56 (m, 5H), 7.18 (m, 2H), 6.85 (d, 1H), 3.96 (s, 3H), 3.60 (m, 2H), 2.04 (m, 5H), 1.69 (m, 2H), 1.67 (m, 2H)

### Step B: Preparation of 3-(1-(5-chloro-3-(4-(4,4-difluoropiperidine-1-carbonyl)phenyl)-2-oxo-2,3-dihydro-1H-benzo[d]imidazol-1-yl)cyclopropyl)-2-fluorobenzoic acid

The title compound (14 mg, yield: 41%) was obtained in the same manner as in step B of Example 2 by using 3-(1-(5-chloro-3-(4-(4,4-difluoropiperidine-1-carbonyl)phenyl)-2-oxo-2,3-dihydro-1*H*-benzo[*d*]imidazol-1-yl)cyclopropyl)-2-fluorobenzoic acid methyl ester (35 mg, 0.06 mmol) obtained in step A above.
¹H-NMR (500 MHz, DMSO-d6) δ7.94 (s, 1H), 7.73 (m, 3H), 7.63 (m, 2H), 7.51 (d, 1H), 7.26 (d, 2H), 7.20 (d, 1H), 3.96 (m, 2H), 3.74 (m, 2H), 2.50 (m, 4H), 1.65 (m, 2H), 1.63 (m, 2H)

### Example 134: Preparation of 3-(1-(5-chloro-3-(4-(3-hydroxyazetidine-1-carbonyl)phenyl)-2-oxo-2,3-dihydro-1H-benzo[d]imidazol-1-yl)cyclopropyl)-2-fluorobenzoic acid

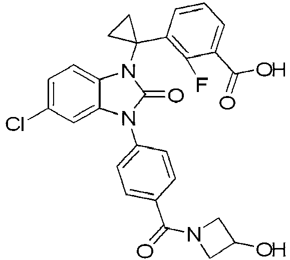

The process of the following steps A and B gave the title compound.

### Step A: Preparation of 3-(1-(5-chloro-3-(4-(3-hydroxyazetidine-1-carbonyl)phenyl)-2-oxo-2,3-dihydro-1H-benzo[d]imidazol-1-yl)cyclopropyl)-2-fluorobenzoic acid methyl ester

The title compound (28 mg, yield: 50.2%) was obtained in the same manner as in step C of Example 56 by using 4-(6-chloro-3-(1-(2-fluoro-3-(methoxycarbonyl)phenyl)cyclopropyl)-2-oxo-2,3-dihydro-1*H*-benzo[*d*]imidazol-1-yl)benzoic acid (50 mg, 0.10 mmol) obtained in step B of Example 67 and azetidin-3-ol hydrochloride (22.8 mg, 0.208 mmol).
¹H-NMR (500 MHz, CDCl3) δ8.14 (t, 1H), 7.85 (t, 1H), 7.78 (d, 2H), 7.55 (m, 3H), 7.20 (m, 2H), 7.05 (s, 1H), 4.75 (m, 1H), 4.49 (t, 2H), 4.20 (m, 1H), 4.11 (m, 1H), 3.91 (s, 3H), 1.69 (m, 2H), 1.63 (m, 2H)

### Step B: Preparation of 3-(1-(5-chloro-3-(4-(3-hydroxyazetidine-1-carbonyl)phenyl)-2-oxo-2,3-dihydro-1H-benzo[d]imidazol-1-yl)cyclopropyl)-2-fluorobenzoic acid

The title compound (3.8 mg, yield: 14%) was obtained in the same manner as in step B of Example 2 by using 3-(1-(5-chloro-3-(4-(3-hydroxyazetidine-1-carbonyl)phenyl)-2-oxo-2,3-dihydro-1H-benzo[*d*]imidazol-1-yl)cyclopropyl)-2-fluorobenzoic acid methyl ester (28 mg, 0.05 mmol) obtained in step A above.
¹H-NMR (500 MHz, DMSO-d6) δ7.95 (t, 1H), 7.78 (m, 3H), 7.62 (m, 2H), 7.51 (d, 1H), 7.27 (m, 2H), 7.08 (s, 1H), 5.79 (s, 1H), 4.51 (m, 2H), 4.28 (m, 1H), 4.09 (m, 1H), 3.81 (m, 1H), 1.65 (m, 2H), 1.63 (m, 2H)

### Example 135: Preparation of 3-(1-(3-(4-(2-oxa-6-azaspiro[3.3]heptane-6-carbonyl)phenyl)-5-chloro-2-oxo-2,3-dihydro-1H-benzo[d]imidazol-1-yl)cyclopropyl)-2-fluorobenzoic acid

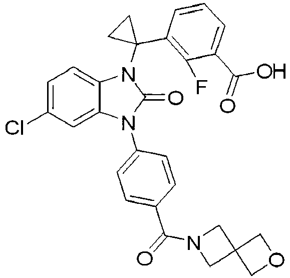

The process of the following steps A and B gave the title compound.

### Step A: Preparation of 3-(1-(3-(4-(2-oxa-6-azaspiro[3.3]heptane-6-carbonyl)phenyl)-5-chloro-2-oxo-2,3-dihydro-1H-benzo[d]imidazol-1-yl)cyclopropyl)-2-fluorobenzoic acid methyl ester

The title compound (10.5 mg, yield: 18%) was obtained in the same manner as in step C of Example 56 by using 4-(6-chloro-3-(1-(2-fluoro-3-(methoxycarbonyl)phenyl)cyclopropyl)-2-oxo-2,3-dihydro-1*H*-benzo[*d*]imidazol-1-yl)benzoic acid (50 mg, 0.10 mmol) obtained in step B of Example 67 and 2-oxa-6-azaspiro[3.3]heptane (20.6 mg, 0.208 mmol).
¹H-NMR (500 MHz, CDCl3) δ8.16 (t, 1H), 7.88 (t, 1H), 7.79 (m, 2H), 7.58 (m, 3H), 7.21 (m, 2H), 7.07 (s, 1H), 4.87 (s, 2H), 4.83 (s, 2H), 4.49 (s, 2H), 4.40 (s, 2H), 3.93 (s, 3H), 1.71 (s, 2H), 1.65 (s, 2H)

### Step B: Preparation of 3-(1-(3-(4-(2-oxa-6-azaspiro[3.3]heptane-6-carbonyl)phenyl)-5-chloro-2-oxo-2,3-dihydro-1H-benzo[d]imidazol-1-yl)cyclopropyl)-2-fluorobenzoic acid

The title compound (4.7 mg, yield: 48.2%) was obtained in the same manner as in step B of Example 2 by using 3-(1-(3-(4-(2-oxa-6-azaspiro[3.3]heptane-6-carbonyl)phenyl)-5-chloro-2-oxo-2,3-dihydro-1*H*-benzo[*d*]imidazol-1-yl)cyclopropyl)-2-fluorobenzoic acid methyl ester (10 mg, 0.018 mmol) obtained in step A above.
¹H-NMR (500 MHz, DMSO-d6) δ8.10 (d, 1H), 7.96 (t, 1H) 7.77 (m, 2H), 7.68 (d, 1H), 7.62 (d, 1H), 7.51 (d, 1H), 7.28 (m, 2H), 7.10 (m, 1H), 4.69 (s, 4H), 4.53 (s, 2H), 4.24 (s, 2H), 1.65 (m, 2H), 1.63 (m, 2H)

### Example 136: Preparation of (S)-3-(1-(5-chloro-3-(4-(3-methylmorpholine-4-carbonyl)phenyl)-2-oxo-2,3-dihydro-1H-benzo[d]imidazol-1-yl)cyclopropyl)-2-fluorobenzoic acid

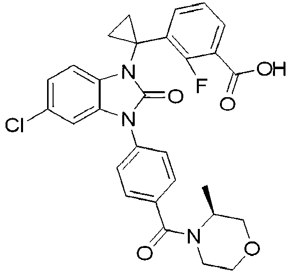

The process of the following steps A and B gave the title compound.

### Step A: Preparation of (5)-3-(1-(5-chloro-3-(4-(3-methylmorpholine-4-carbonyl)phenyl)-2-oxo-2,3-dihydro-1H-benzo[d]imidazol-1-yl)cyclopropyl)-2-fluorobenzoic acid methyl ester

The title compound (26 mg, yield: 44.3%) was obtained in the same manner as in step C of Example 56 by using 4-(6-chloro-3-(1-(2-fluoro-3-(methoxycarbonyl)phenyl)cyclopropyl)-2-oxo-2,3-dihydro-1*H*-benzo[*d*]imidazol-1-yl)benzoic acid (50 mg, 0.10 mmol) obtained in step B of Example 67 and (S)-3-methylmorpholine (21 mg, 0.208 mmol).
¹H-NMR (500 MHz, CDCl3) δ8.16 (t, 1H), 7.88 (t, 1H), 7.57 (m, 5H), 7.20 (m, 2H), 7.06 (s, 1H), 3.93 (s, 3H), 3.90 (m, 1H), 3.68 (m, 2H), 3.52 (m, 2H), 3.40 (m, 2H), 1.71 (m, 2H), 1.65 (m, 2H), 1.41 (d, 3H)

### Step B: Preparation of (S)-3-(1-(5-chloro-3-(4-(3-methylmorpholine-4-carbonyl)phenyl)-2-oxo-2,3-dihydro-1H-benzo[d]imidazol-1-yl)cyclopropyl)-2-fluorobenzoic acid

The title compound (23 mg, yield: 91%) was obtained in the same manner as in step B of Example 2 by using (*S*)-3-(1-(5-chloro-3-(4-(3-methylmorpholine-4-carbonyl)phenyl)-2-oxo-2,3-dihydro-1*H*-benzo[*d*]imidazol-1-yl)cyclopropyl)-2-fluorobenzoic acid methyl ester (26 mg, 0.046 mmol) obtained in step A above.
¹H-NMR (500 MHz, DMSO-d6) δ7.93 (t, 1H), 7.75 (m, 1H), 7.61 (d, 2H), 7.56 (d, 2H), 7.51 (d, 1H), 7.27 (m, 2H), 7.08 (s, 1H), 3.85 (m, 1H), 3.60 (m, 2H), 3.42 (m, 2H), 2.50 (m, 2H), 1.65 (m, 2H), 1.63 (m, 2H), 1.28 (d, 3H)

### Example 137: Preparation of 3-(1-(3-(4-(2-oxa-6-azaspiro[3.3]heptane-6-carbonyl)phenyl)-5-chloro-2-oxo-2,3-dihydro-1H-benzo[d]imidazol-1-yl)cyclopropyl)benzoic acid

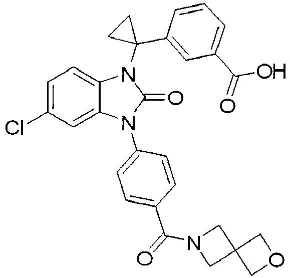

The title compound (5 mg, yield: 10%) was obtained in the same manner as in step C of Example 56 and step B of Example 24 by using 4-(6-chloro-3-(1-(3-(methoxycarbonyl)phenyl)cyclopropyl)-2-oxo-2,3-dihydro-1*H-*benzo[*d*]imidazol-1-yl)benzoic acid (50 mg, 0.108 mmol) obtained in step B of Example 63 and 3-hydroxyazetidine hydrochloride.
MS [M-H] = 528 (M-1)
¹H-NMR (400 MHz, MeOH-d4) δ8.36 (dd, 1H), 7.88 (3H), 7.71 (d, 2H), 7.44 (m, 2H), 7.28 (d, 1H), 7.17 (2H), 4.81 (m, 4H), 4.72 (s, 2H), 4.37 (s, 2H), 1.75 (m, 4H)

### Example 138: Preparation of 3-(1-(5-chloro-3-(4-(5-(4,4-difluorocyclohexyl)-1,3,4-oxadiazol-2-yl)phenyl)-2-oxo-2,3-dihydro-1H-benzo[d]imidazol-1-yl)cyclopropyl)benzoic acid

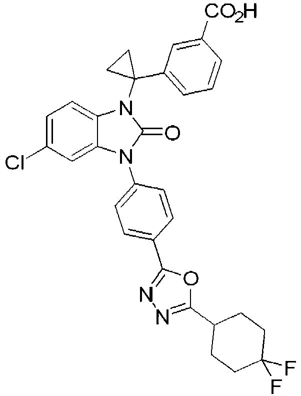

The process of the following steps A, B, and C gave the title compound.

### Step A: Preparation of 3-(1-(5-chloro-3-(4-(2-(4,4-difluorocyclohexane-1-carbonyl)hydrazine-1-carbonyl)phenyl)-2-oxo-2,3-dihydro-1H-benzo[d]imidazol-1-yl)cyclopropyl)benzoic acid methyl ester

To 4-(6-chloro-3-(1-(3-(methoxycarbonyl)phenyl)cyclopropyl)-2-oxo-2,3-dihydro-1*H-*benzo[*d*]imidazol-1-yl)benzoic acid (100 mg, 0.21 mmol) obtained in step B of Example 63 was added 1 mL of DMF, then 4,4-difluorocyclohexene-1-carboxyhydrazide (42 mg, 0.24 mmol), pyBOP (124 mg, 0.24 mmol), and *N*-methylmorpholine (24 mg, 0.24 mmol) were added thereto, and then the resulting mixture was stirred at room temperature for 16 hours. The reaction solution was concentrated under reduced pressure, then water was added thereto, and the resulting solid was dried to give the title compound (125 mg, yield: 93%).
¹H-NMR (400 MHz, DMSO-d6) δ10.49 (d, 1H), 10.02 (d, 1H), 8.08 (d, 2H), 7.82 (m, 1H), 7.72 (3H), 7.50 (2H), 7.28 (dd, 1H), 7.19 (2H), 3.93 (s, 3H), 2.45 (m, 1H), 2.09 (m, 2H), 1.90 (m, 6H), 1.65 (m, 4H)

### Step B: Preparation of 3-(1-(5-chloro-3-(4-(5-(4,4-difluorocyclohexyl)-1,3,4-oxadiazol-2-yl)phenyl)-2-oxo-2,3-dihydro-1H-benzo[d]imidazol-1-yl)cyclopropyl)benzoic acid methyl ester

To 3-(1-(5-chloro-3-(4-(2-(4,4-difluorocyclohexane-1-carbonyl)hydrazine-1-carbonyl)phenyl)-2-oxo-2,3-dihydro-1*H-*benzo[*d*]imidazol-1-yl)cyclopropyl)benzoic acid methyl ester (120 mg, 0.19 mmol) obtained in step A above were added 2 mL of ATN and POCl₃ (0.036 mL, 0.39 mmol), and then the resulting mixture was stirred under reflux for 2 hours. The reaction solution was concentrated under reduced pressure and extracted by the addition of an aqueous NaHCO₃ solution and EtOAc. An organic layer was separated and purified by MPLC to give the title compound (75 mg, yield: 64%).
¹H-NMR (400 MHz, CDCl3) δ8.22 (d, 2H), 7.89 (2H), 7.74 (d, 2H), 7.51 (dd, 1H), 7.37 (t, 1H), 7.15 (3H), 3.91 (s, 3H), 3.15 (m, 1H), 2.30-1.80 (8H), 1.71 (m, 4H)

### Step C: Preparation of 3-(1-(5-chloro-3-(4-(5-(4,4-difluorocyclohexyl)-1,3,4-oxadiazol-2-yl)phenyl)-2-oxo-2,3-dihydro-1H-benzo[d]imidazol-1-yl)cyclopropyl)benzoic acid

The title compound (30 mg, yield: 41%) was obtained in the same manner as in step B of Example 24 by using 3-(1-(5-chloro-3-(4-(5-(4,4-difluorocyclohexyl)-1,3,4-oxadiazol-2-yl)phenyl)-2-oxo-2,3-dihydro-1*H*-benzo[*d*]imidazol-1-yl)cyclopropyl)benzoic acid methyl ester (75 mg, 0.12 mmol) obtained in step B above.
MS [M+H] = 591 (M+1)
¹H-NMR (400 MHz, CDCl3) 8.17 (d, 2H), 7.90 (m, 1H), 7.85 (m, 1H), 7.70 (d, 2H), 7.43 (m, 1H), 7.25 (m, 1H), 7.13 (2H), 7.06 (m, 1H), 3.14 (m, 1H), 2.27 (m, 4H), 2.10 (m, 2H), 1.90 (m, 2H), 1.63 (m, 4H)

### Example 139: Preparation of 3-(1-(5-chloro-3-(4-(2-(dimethylamino)-2-oxoethyl)phenyl)-2-oxo-2,3-dihydro-1H-benzo[d]imidazol-1-yl)cyclopropyl)benzoic acid

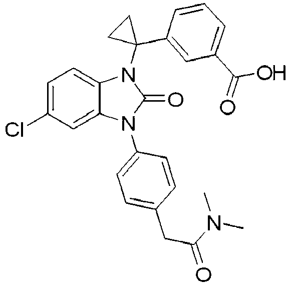

The process of the following steps A and B gave the title compound.

### Step A: Preparation of 3-(1-(5-chloro-3-(4-(2-(dimethylamino)-2-oxoethyl)phenyl)-2-oxo-2,3-dihydro-1H-benzo[d]imidazol-1-yl)cyclopropyl)benzoic acid methyl ester

To 3-(1-(5-chloro-2-oxo-2,3-dihydro-1*H-*benzo[*d*]imidazol-1-yl)cyclopropyl)benzoic acid methyl ester (100 mg, 0.29 mmol) obtained in Preparation Example 3 and 2-(4-bromophenyl)-*N*,*N*-dimethylacetamide (78 mg, 0.32 mmol) obtained in Preparation Example 37 was added 1.5 mL of toluene, and then the resulting mixture was charged with nitrogen gas at 70 °C. To the mixture were added K₂CO₃ (100 mg, 0.73 mmol), DMEDA (13 µL, 0.12 mmol), and CuI (6 mg, 30 µmol) sequentially, and the resulting mixture was charged with nitrogen gas. The reaction solution was heated to 100 °C and stirred for 16 hours, then the solid was removed through Celite and the filtrate was purified by MPLC to give the title compound (118 mg, yield: 80%).
¹H-NMR (400 MHz, CDCl3) δ7.90 (m, 1H), 7.88 (m, 1H), 7.49 (3H), 7.43 (d, 2H), 7.36 (t, 1H), 7.13 (d, 1H), 7.08 (2H), 3.90 (s, 3H), 3.79 (s, 2H), 3.06 (s, 3H), 3.00 (s, 3H), 1.73 (m, 2H), 1.68 (m, 2H)

### Step B: Preparation of 3-(1-(5-chloro-3-(4-(2-(dimethylamino)-2-oxoethyl)phenyl)-2-oxo-2,3-dihydro-1H-benzo[d]imidazol-1-yl)cyclopropyl)benzoic acid

The title compound (100 mg, yield: 89%) was obtained in the same manner as in step B of Example 24 by using 3-(1-(5-chloro-3-(4-(2-(dimethylamino)-2-oxoethyl)phenyl)-2-oxo-2,3-dihydro-1*H*-benzo[*d*]imidazol-1-yl)cyclopropyl)benzoic acid methyl ester (115 mg, 0.23 mmol) obtained in step A above.
MS [M+Na] = 513 (M+23)
¹H-NMR (400 MHz, DMSO-d6) δ7.80 (m, 1H), 7.69 (d, 1H), 7.50 (d, 2H), 7.44 (4H), 7.26 (d, 1H), 7.17 (dd, 1H), 7.03 (d, 1H), 3.80 (s, 2H), 3.06 (s, 3H), 2.86 (s, 3H), 1.68 (m, 4H)

### Example 140: Preparation of 3-((5-chloro-3-(4-(4-(cyclopropanecarbonyl)piperazin-1-yl)phenyl)-2-oxo-2,3-dihydro-1H-benzo[d]imidazol-1-yl)methyl)-2-fluorobenzoic acid

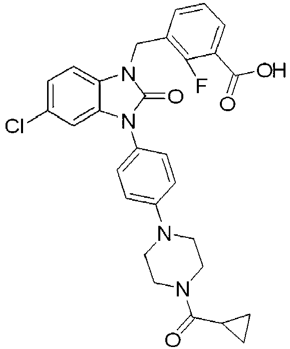

The process of the following steps A and B gave the title compound.

### Step A: Preparation of 3-((5-chloro-3-(4-(4-(cyclopropanecarbonyl)piperazin-1-yl)phenyl)-2-oxo-2,3-dihydro-1H-benzo[d]imidazol-1-yl)methyl)-2-fluorobenzoic acid methyl ester

The title compound (65 mg, 61%) was obtained in the same manner as in step A of Example 100 by using 3-((5-chloro-2-oxo-3-(4-(piperazin-1-yl)phenyl)-2,3-dihydro-1*H-*benzo[*d*]imidazol-1-yl)methyl)-2-fluorobenzoic acid methyl ester hydrochloride (100 mg, 0.188 mmol) obtained in Preparation Example 42 and cyclopropanecarbonyl chloride (22 mg, 0.21 mmol).
¹H-NMR (400 MHz, CDCl3) δ8.88 (td, 1H), 7.61 (td, 1H), 7.39 (dd, 2H), 7.17 (t, 1H), 7.06 (3H), 7.00 (d, 1H), 6.97 (d, 1H), 5.19 (s, 2H), 3.95 (s, 3H), 3.86 (m, 4H), 3.28 (m, 4H), 1.78 (m, 1H), 1.04 (m, 2H), 0.82 (m, 2H)

### Step B: Preparation of 3-((5-chloro-3-(4-(4-(cyclopropanecarbonyl)piperazin-1-yl)phenyl)-2-oxo-2,3-dihydro-1H-benzo[d]imidazol-1-yl)methyl)-2-fluorobenzoic acid

The title compound (55 mg, yield: 87%) was obtained in the same manner as in step B of Example 24 by using 3-((5-chloro-3-(4-(4-(cyclopropanecarbonyl)piperazin-1-yl)phenyl)-2-oxo-2,3-dihydro-1*H*-benzo[*d*]imidazol-1-yl)methyl)-2-fluorobenzoic acid methyl ester (65 mg, 0.11 mmol) obtained in step A above.
MS [M+Na] = 571 (M+23)
¹H-NMR (400 MHz, DMSO-d6) δ7.80 (td, 1H), 7.51 (td, 1H), 7.40 (dd, 2H), 7.26 (t, 1H), 7.20 (d, 1H), 7.15 (3H), 6.92 (d, 1H), 5.21 (s, 2H), 3.86 (m, 2H), 3.64 (m, 2H), 3.35 (m, 2H), 3.21 (m, 2H), 2.05 (m, 1H), 0.75 (m, 4H)

### Example 141: Preparation of 3-((5-chloro-2-oxo-3-(4-(4-propionylpiperazin-1-yl)phenyl)-2,3-dihydro-1H-benzo[d]imidazol-1-yl)methyl)-2-fluorobenzoic acid

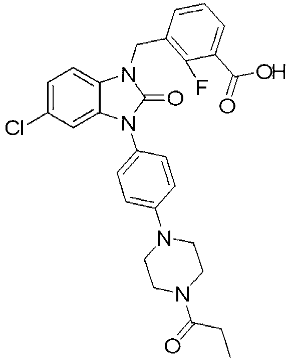

The process of the following steps A and B gave the title compound.

### Step A: Preparation of 3-((5-chloro-2-oxo-3-(4-(4-propionylpiperazin-1-yl)phenyl)-2,3-dihydro-1H-benzo[d]imidazol-1-yl)methyl)-2-fluorobenzoic acid methyl ester

The title compound (75 mg, 72%) was obtained in the same manner as in step A of Example 100 by using 3-((5-chloro-2-oxo-3-(4-(piperazin-1-yl)phenyl)-2,3-dihydro-1*H-*benzo[*d*]imidazol-1-yl)methyl)-2-fluorobenzoic acid methyl ester hydrochloride (100 mg, 0.188 mmol) obtained in Preparation Example 42 and propionyl chloride (19 mg, 0.21 mmol).
¹H-NMR (400 MHz, CDCl3) δ7.88 (td, 1H), 7.61 (td, 1H), 7.40 (dd, 2H), 7.17 (t, 1H), 7.05 (3H), 7.00 (d, 1H), 6.97 (d, 1H), 5.19 (s, 2H), 3.95 (s, 3H), 3.80 (m, 2H), 3.66 (m, 2H), 3.25 (m, 4H), 2.40 (q, 2H), 1.20 (t, 3H)

### Step B: Preparation of 3-((5-chloro-2-oxo-3-(4-(4-propionylpiperazin-1-yl)phenyl)-2,3-dihydro-1H-benzo[d]imidazol-1-yl)methyl)-2-fluorobenzoic acid

The title compound (65 mg, yield: 89%) was obtained in the same manner as in step B of Example 24 by using 3-((5-chloro-2-oxo-3-(4-(4-propionylpiperazin-1-yl)phenyl)-2,3-dihydro-1H-benzo[*d*]imidazol-1-yl)methyl)-2-fluorobenzoic acid methyl ester (75 mg, 0.14 mmol) obtained in step A above.
MS [M+H] = 537 (M+1)
¹H-NMR (400 MHz, DMSO-d6) δ 7.79 (td, 1H), 7.51 (td, 1H), 7.39 (dd, 1H), 7.26 (t, 1H), 7.20 (d, 1H), 7.14 (3H), 6.91 (d, 1H), 5.21 (s, 2H), 3.62 (m, 4H), 3.26 (m, 2H), 3.20 (m, 2H), 2.39 (q, 2H), 1.03 (t, 3H)

### Example 142: Preparation of 3-((5-chloro-3-(4-(4-(cyclobutanecarbonyl)piperazin-1-yl)phenyl)-2-oxo-2,3-dihydro-1H-benzo[d]imidazol-1-yl)methyl)-2-fluorobenzoic acid

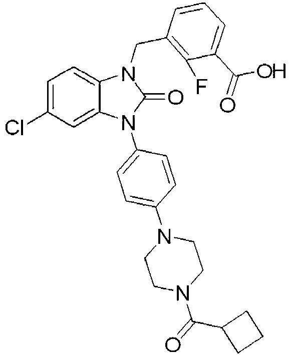

The process of the following steps A and B gave the title compound.

### Step A: Preparation of 3-((5-chloro-3-(4-(4-(cyclobutanecarbonyl)piperazin-1-yl)phenyl)-2-oxo-2,3-dihydro-1H-benzo[d]imidazol-1-yl)methyl)-2-fluorobenzoic acid methyl ester

To 3-((5-chloro-2-oxo-3-(4-(piperazin-1-yl)phenyl)-2,3-dihydro-1*H*-benzo[*d*]imidazol-1-yl)methyl)-2-fluorobenzoic acid methyl ester hydrochloride (100 mg, 0.188 mmol) obtained in Preparation Example 42 and cyclobutane carboxylic acid (21 mg, 0.21 mmol) were added 2 mL of DCM, EDC (43 mg, 0.23 mmol), HOBt (35 mg, 0.23 mmol), and DIPEA (0.12 mL, 0.66 mol), and then the resulting mixture was stirred at room temperature for 16 hours. After the reaction was terminated, water was added thereto and then an organic layer was separated. The organic layer was purified by MPLC to give the title compound (100 mg, 92%).
¹H-NMR (400 MHz, CDCl3) δ7.88 (td, 1H), 7.61 (td, 1H), 7.37 (dd, 2H), 7.17 (t, 1H), 7.05 (3H), 7.00 (d, 1H), 6.98 (d, 1H), 5.19 (s, 2H), 3.95 (s, 3H), 3.80 (m, 2H), 3.54 (m, 2H), 3.32 (m, 1H), 3.20 (m, 4H), 2.40 (m, 2H), 2.20 (m, 2H), 2.00 (m, 1H), 1.90 (m, 1H)

### Step B: Preparation of 3-((5-chloro-3-(4-(4-(cyclobutanecarbonyl)piperazin-1-yl)phenyl)-2-oxo-2,3-dihydro-1H-benzo[d]imidazol-1-yl)methyl)-2-fluorobenzoic acid

The title compound (73 mg, yield: 74%) was obtained in the same manner as in step B of Example 24 by using 3-((5-chloro-3-(4-(4-(cyclobutanecarbonyl)piperazin-1-yl)phenyl)-2-oxo-2,3-dihydro-1H-benzo[d]imidazol-1-yl)methyl)-2-fluorobenzoic acid methyl ester (100 mg, 0.17 mmol) obtained in step A above.
MS [M+Na] = 585 (M+23)
¹H-NMR (400 MHz, DMSO-d6) δ 7.79 (td, 1H), 7.51 (td, 1H), 7.38 (dd, 2H), 7.26 (t, 1H), 7.20 (d, 1H), 7.13 (3H), 6.91 (d, 1H), 5.20 (s, 2H), 3.61 (m, 4H), 3.21 (5H), 2.19 (m, 2H), 2.12 (m, 2H), 1.92 (m, 1H), 1.75 (m, 1H)

### Example 143: Preparation of 3-((5-chloro-3-(4-(4-(4,4-difluorocyclohexane-1-carbonyl)piperazin-1-yl)phenyl)-2-oxo-2,3-dihydro-1H-benzo[d]imidazol-1-yl)methyl)-2-fluorobenzoic acid

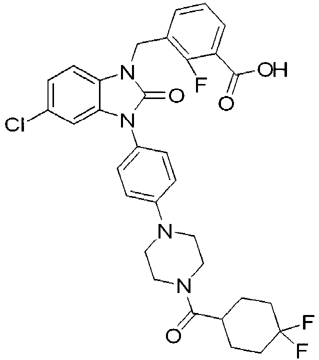

The process of the following steps A and B gave the title compound.

### Step A: Preparation of 3-((5-chloro-3-(4-(4-(4,4-difluorocyclohexane-1-carbonyl)piperazin-1-yl)phenyl)-2-oxo-2,3-dihydro-1H-benzo[d]imidazol-1-yl)methyl)-2-fluorobenzoic acid methyl ester

The title compound (85 mg, yield: 71%) was obtained in the same manner as in step A of Example 142 by using 3-((5-chloro-2-oxo-3-(4-(piperazin-1-yl)phenyl)-2,3-dihydro-1*H-*benzo[*d*]imidazol-1-yl)methyl)-2-fluorobenzoic acid methyl ester hydrochloride (100 mg, 0.188 mmol) obtained in Preparation Example 42 and 4,4-difluorocyclohexane-1-carboxylic acid (34 mg, 0.21 mmol).
¹H-NMR (400 MHz, CDCl3) δ7.88 (td, 1H), 7.71 (td, 1H), 7.41 (dd, 2H), 7.17 (t, 1H), 7.05 (3H), 7.00 (d, 1H), 6.98 (d, 1H), 5.19 (s, 2H), 3.95 (s, 3H), 3.82 (m, 2H), 3.70 (m, 2H), 3.25 (m, 4H), 2.53 (m, 1H), 2.24 (m, 2H), 2.00-1.70 (6H)

### Step B: Preparation of 3-((5-chloro-3-(4-(4-(4,4-difluorocyclohexane-1-carbonyl)piperazin-1-yl)phenyl)-2-oxo-2,3-dihydro-1H-benzo[d]imidazol-1-yl)methyl)-2-fluorobenzoic acid

The title compound (62 mg, yield: 75%) was obtained in the same manner as in step B of Example 24 by using 3-((5-chloro-3-(4-(4-(4,4-difluorocyclohexane-1-carbonyl)piperazin-1-yl)phenyl)-2-oxo-2,3-dihydro-1*H*-benzo[*d*]imidazol-1-yl)methyl)-2-fluorobenzoic acid methyl ester (85 mg, 0.13 mmol) obtained in step A above.
MS [M+H] = 627 (M+1)
¹H-NMR (400 MHz, DMSO-d6) δ 7.79 (td, 1H), 7.51 (td, 1H), 7.40 (dd, 2H), 7.26 (t, 1H), 7.20 (d, 1H), 7.15 (3H), 6.91 (d, 1H), 5.21 (s, 2H), 3.71 (m, 2H), 3.63 (m, 2H), 3.26 (m, 2H), 3.20 (m, 2H), 2.88 (m, 1H), 2.04 (m, 2H), 1.91 (m, 2H), 1.76 (m, 2H), 1.60 (m, 2H)

### Example 144: Preparation of 3-(1-(3-(benzo[d]thiazol-2-yl)-5-chloro-2-oxo-2,3-dihydro-1H-benzo[d]imidazol-1-yl)cyclopropyl)-2-fluorobenzoic acid

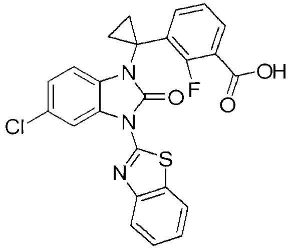

The process of the following steps A and B gave the title compound.

### Step A: Preparation of 3-(1-(3-(benzo[d]thiazol-2-yl)-5-chloro-2-oxo-2,3-dihydro-1H-benzo[d]imidazol-1-yl)cyclopropyl)-2-fluorobenzoic acid methyl ester

To 3-(1-(5-chloro-2-oxo-2,3-dihydro-1*H-*benzo[*d*]imidazol-1-yl)cyclopropyl)-2-fluorobenzoic acid methyl ester (50 mg, 0.14 mmol) obtained in Preparation Example 4 and 2-chlorobenzo[d]thiazole (47 mg, 0.277 mmol) were added 3 mL of DMF and K₂CO₃ (38.3 mg, 0.277 mmol), and the resulting mixture was stirred at 80 °C for 16 hours. A solid was filtered and then the filtrate was purified by MPLC to give the title compound (60 g, yield: 88%).
¹H-NMR (500 MHz, CDCl3) δ8.81 (s, 1H), 8.20 (t, 1H), 8.02 (d, 1H), 7.89 (m, 2H), 7.61 (d, 1H), 7.52 (t, 1H), 7.39 (t, 1H), 7.33 (d, 1H), 7.24 (m, 1H), 3.93 (s, 3H), 1.76 (m, 2H), 1.69 (m, 2H)

### Step B: Preparation of 3-(1-(3-(benzo[d]thiazol-2-yl)-5-chloro-2-oxo-2,3-dihydro-1H-benzo[d]imidazol-1-yl)cyclopropyl)-2-fluorobenzoic acid

The title compound (55 mg, yield: 94%) was obtained in the same manner as in step B of Example 24 by using 3-(1-(3-(benzo[*d*]thiazol-2-yl)-5-chloro-2-oxo-2,3-dihydro-1*H-*benzo[*d*]imidazol-1-yl)cyclopropyl)-2-fluorobenzoic acid methyl ester (60 mg, 0.121 mmol) obtained in step A above.
¹H-NMR (500 MHz, DMSO-d6) δ 8.65 (s, 1H), 8.12 (d, 1H), 8.02 (d, 1H), 7.98 (t, 1H), 7.79 (t, 1H), 7.59 (d, 1H), 7.53 (t, 1H), 7.45 (d, 1H), 7.41 (t, 1H), 7.30 (t, 1H), 1.71 (m, 2H), 1.69 (m, 2H)

### Example 145: Preparation of 3-(1-(5-chloro-3-(4-(morpholinomethyl)phenyl)-2-oxo-2,3-dihydro-1H-benzo[d]imidazol-1-yl)cyclopropyl)benzoic acid

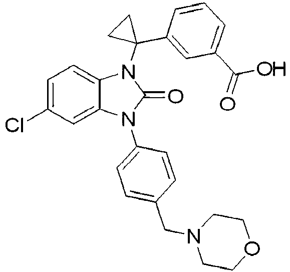

The process of the following steps A, B, and C gave the title compound.

### Step A: Preparation of 3-(1-(5-chloro-3-(4-formylphenyl)-2-oxo-2,3-dihydro-1H-benzo[d]imidazol-1-yl)cyclopropyl)benzoic acid methyl ester

The title compound (68.3 mg, yield: 52.4%) was obtained in the same manner as in step A of Preparation Example 25 by using 3-(1-(5-chloro-2-oxo-2,3-dihydro-1*H*-benzo[*d*]imidazol-1-yl)cyclopropyl)benzoic acid methyl ester (100 mg, 0.292 mmol) obtained in Preparation Example 3 and (4-formylphenyl)boronic acid (65.6 mg, 0.438 mmol).
¹H-NMR (500 MHz, CDCl3) δ10.07 (s, 1H), 8.07 (d, 2H), 7.90 (d, 1H), 7.89 (s, 1H), 7.78 (d, 2H), 7.51 (d, 1H), 7.37 (t, 1H), 7.18 (d, 2H), 7.14 (d, 1H), 3.90 (s, 3H), 1.73 (m, 2H), 1.70 (m, 2H)

### Step B: Preparation of 3-(1-(5-chloro-3-(4-(morpholinomethyl)phenyl)-2-oxo-2,3-dihydro-1H-benzo[d]imidazol-1-yl)cyclopropyl)benzoic acid methyl ester

To 3-(1-(5-chloro-3-(4-formylphenyl)-2-oxo-2,3-dihydro-1*H*-benzo[*d*]imidazol-1-yl)cyclopropyl)benzoic acid methyl ester (60 mg, 0.134 mmol) obtained in step A above was added 2 mL of DCM, then acetic acid (0.001mL, 0.013mmol), morpholine (12.8 mg, 0.148 mmol), and NaBH₃CN (8.4 mg, 0.134 mmol) were added thereto, and the resulting mixture was stirred at room temperature for 16 hours. After the reaction was terminated, water was added thereto and then an organic layer was separated. The organic layer was purified by MPLC to give the title compound (63 mg, 91%).
¹H-NMR (500 MHz, CDCl3) δ7.92 (d, 1H), 7.89 (s, 1H), 7.51 (m, 5H), 7.38 (t, 1H), 7.15 (d, 1H), 7.10 (m, 2H), 3.92 (s, 3H), 3.76 (m, 4H), 3.58 (s, 2H), 2.51 (m, 4H), 1.75 (m, 2H), 1.70 (m, 2H)

### Step C: Preparation of 3-(1-(5-chloro-3-(4-(morpholinomethyl)phenyl)-2-oxo-2,3-dihydro-1H-benzo[d]imidazol-1-yl)cyclopropyl)benzoic acid

The title compound (46 mg, yield: 75%) was obtained in the same manner as in step B of Example 24 by using 3-(1-(5-chloro-3-(4-(morpholinomethyl)phenyl)-2-oxo-2,3-dihydro-1*H-*benzo[d]imidazol-1-yl)cyclopropyl)benzoic acid methyl ester (63 mg, 0.122 mmol) obtained in step B above.
¹H-NMR (500 MHz, CDCl3) δ7.96 (d, 1H), 7.93 (s, 1H), 7.64 (m, 2H), 7.56 (d, 2H), 7.52 (d, 1H), 7.40 (t, 1H), 7.17 (d, 1H), 7.11 (s, 2H), 3.89 (m, 4H), 3.85 (s, 2H), 2.77 (m, 4H), 1.75 (m, 2H), 1.71 (m, 2H)

### Example 146: Preparation of 3-(1-(5-chloro-2-oxo-3-(4-(pyrrolidin-1-yl)phenyl)-2,3-dihydro-1H-benzo[d]imidazol-1-yl)cyclopropyl)benzoic acid

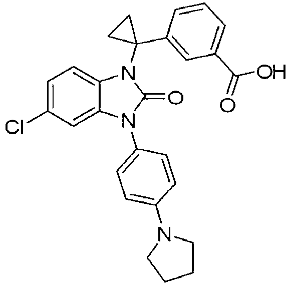

The process of the following steps A and B gave the title compound.

### Step A: Preparation of 3-(1-(5-chloro-2-oxo-3-(4-(pyrrolidin-1-yl)phenyl)-2,3-dihydro-1H-benzo[d]imidazol-1-yl)cyclopropyl)benzoic acid methyl ester

To 3-(1-(3-(4-bromophenyl)-5-chloro-2-oxo-2,3-dihydro-1*H*-benzo[*d*]imidazol-1-yl)cyclopropyl)benzoic acid methyl ester (50 mg, 0.1 mmol) obtained in step A of Example 121 was added 2 mL of DMF, then pyrrolidine (7.14 mg, 0.1 mmol), CuI (19.1 mg, 0.1 mmol), L-proline (23.1 mg, 0.2 mmol), and K₂CO₃ (27.8 mg, 0.2 mmol), and the resulting mixture was stirred at 130 °C for 1 hours using microwave. After the reaction was terminated, water was added thereto and then an organic layer was separated. The organic layer was purified by MPLC to give the title compound (25 mg, 51%).
¹H-NMR (500 MHz, CDCl3) δ7.91 (d, 1H), 7.87 (s, 1H), 7.51 (d, 1H), 7.37 (t, 1H), 7.31 (d, 2H), 7.10 (d, 1H), 7.05 (d, 1H), 6.99 (s, 1H), 6.67 (d, 2H), 3.92 (s, 3H), 3.35 (m, 2H), 3.35 (m, 4H), 2.06 (m, 4H), 1.75 (m, 2H), 1.68 (m, 2H)

### Step B: Preparation of 3-(1-(5-chloro-2-oxo-3-(4-(pyrrolidin-1-yl)phenyl)-2,3-dihydro-1H-benzo[d]imidazol-1-yl)cyclopropyl)benzoic acid

The title compound (20 mg, yield: 82%) was obtained in the same manner as in step B of Example 24 by using 3-(1-(5-chloro-2-oxo-3-(4-(pyrrolidin-1-yl)phenyl)-2,3-dihydro-1*H-*benzo[*d*]imidazol-1-yl)cyclopropyl)benzoic acid methyl ester (25 mg, 0.051 mmol) obtained in step A above.
¹H-NMR (500 MHz, CDCl3) δ7.95 (d, 1H), 7.91 (s, 1H), 7.55 (d, 1H), 7.40 (t, 1H), 7.31 (d, 2H), 7.11 (d, 1H), 7.06 (d, 1H), 7.00 (s, 1H), 6.67 (d, 2H), 3.35 (m, 4H), 2.07 (m, 4H), 1.77 (m, 2H), 1.69 (m, 2H)

### Example 147: Preparation of 3-(1-(5-chloro-3-(4-morpholinophenyl)-2-oxo-2,3-dihydro-1H-benzo[d]imidazol-1-yl)cyclopropyl)benzoic acid

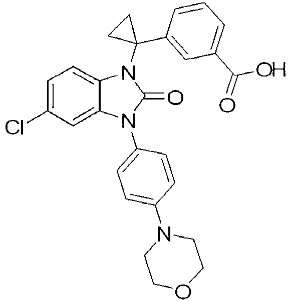

The process of the following steps A and B gave the title compound.

### Step A: Preparation of 3-(1-(5-chloro-3-(4-morpholinophenyl)-2-oxo-2,3-dihydro-1H-benzo[d]imidazol-1-yl)cyclopropyl)benzoic acid methyl ester

The title compound (0.73 g, yield: 50%) was obtained in the same manner as in step A of Example 2 by using 3-(1-(5-chloro-2-oxo-2,3-dihydro-1*H*-benzo[*d*]imidazol-1-yl)cyclopropyl)benzoic acid methyl ester (1.00 g, 2.92 mmol) obtained in Preparation Example 3 and (4-morpholinophenyl)boronic acid (1.51 g, 7.29 mmol).
MS [M+H] = 504 (M+1)
¹H-NMR (400 MHz, CDCl3) δ 7.88 (dd, 1H), 7.86 (d, 1H), 7.50 (dd, 1H), 7.36 (3H), 7.11 (d, 1H), 7.03 (4H), 3.90 (s, 3H), 3.89 (m, 4H), 3.23 (m, 4H), 1.73 (m, 2H), 1.67 (m, 2H)

### Step B: Preparation of 3-(1-(5-chloro-3-(4-morpholinophenyl)-2-oxo-2,3-dihydro-1H-benzo[d]imidazol-1-yl)cyclopropyl)benzoic acid

The title compound (0.66 g, yield: 93%) was obtained in the same manner as in step B of Example 24 by using 3-(1-(5-chloro-3-(4-morpholinophenyl)-2-oxo-2,3-dihydro-1*H-*benzo[*d*]imidazol-1-yl)cyclopropyl)benzoic acid methyl ester (0.73 g, 1.45 mmol) obtained in step A above.
¹H-NMR (400 MHz, DMSO-d6) δ 7.80 (dd, 1H), 7.68 (d, 1H), 7.43 (2H), 7.41 (d, 2H), 7.23 (d, 1H), 7.11 (3H), 6.92 (d, 1H), 3.77 (m, 4H), 3.20 (m, 4H) 1.67 (m, 4H)

### Example 148: Preparation of 3-(1-(3-(4-(7-oxa-2-azaspiro[3.5]nonane-2-yl)phenyl)-5-chloro-2-oxo-2,3-dihydro-1H-benzo[d]imidazol-1-yl)cyclopropyl)benzoic acid

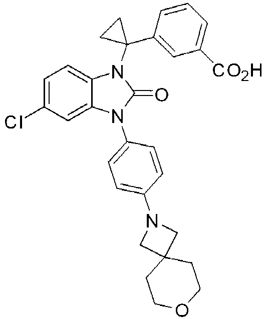

The process of the following steps A and B gave the title compound.

### Step A: Preparation of 3-(1-(3-(4-(7-oxa-2-azaspiro[3.5]nonane-2-yl)phenyl)-5-chloro-2-oxo-2,3-dihydro-1H-benzo[d]imidazol-1-yl)cyclopropyl)benzoic acid methyl ester

The title compound (54 mg, yield: 49%) was obtained in the same manner as in Example 139 by using 3-(1-(5-chloro-2-oxo-2,3-dihydro-1*H*-benzo[*d*]imidazol-1-yl)cyclopropyl)benzoic acid methyl ester (70 mg, 0.20 mmol) obtained in Preparation Example 3 and 2-(4-bromophenyl)-7-oxa-2-azaspiro[3.5]nonane (58 mg, 0.20 mmol) obtained in Preparation Example 39.
¹H-NMR (400 MHz, CDCl3) δ7.89 (m, 1H), 7.85 (m, 1H), 7.49 (m, 1H), 7.35 (t, 1H), 7.29 (m, 2H), 7.09 (d, 1H), 7.04 (m, 1H), 6.97 (m, 1H), 6.54 (d, 2H), 3.89 (s, 3H), 3.71 (s, 4H), 3.67 (m, 4H), 1.86 (m, 4H), 1.73 (m, 2H), 1.66 (m, 2H)

### Step B: Preparation of 3-(1-(3-(4-(7-oxa-2-azaspiro[3.5]nonane-2-yl)phenyl)-5-chloro-2-oxo-2,3-dihydro-1H-benzo[d]imidazol-1-yl)cyclopropyl)benzoic acid

The title compound (50 mg, yield: 94%) was obtained in the same manner as in step B of Example 24 by using 3-(1-(3-(4-(7-oxa-2-azaspiro[3.5]nonane-2-yl)phenyl)-5-chloro-2-oxo-2,3-dihydro-1*H*-benzo[*d*]imidazol-1-yl)cyclopropyl)benzoic acid methyl ester (54 mg, 0.1 mmol) in step A above.
MS [M+H] = 530 (M+1)
¹H-NMR (400 MHz, CDCl3) δ7.93 (dd, 1H), 7.88 (m, 1H), 7.51 (m, 1H), 7.40 (m, 1H), 7.30 (d, 2H), 7.11 (d, 1H), 7.06 (dd, 1H), 6.97 (d, 1H), 6.54 (d, 2H), 3.70 (s, 4H), 3.67 (m, 4H), 1.86 (m, 4H), 1.70 (m, 4H)

### Example 149: Preparation of 3-(1-(3-(4-(2-oxa-6-azaspiro[3.3]heptane-6-yl)phenyl)-5-chloro-2-oxo-2,3-dihydro-1H-benzo[d]imidazol-1-yl)cyclopropyl)benzoic acid

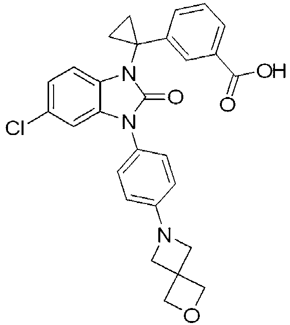

The process of the following steps A and B gave the title compound.

### Step A: Preparation of 3-(1-(3-(4-(2-oxa-6-azaspiro[3.3]heptane-6-yl)phenyl)-5-chloro-2-oxo-2,3-dihydro-1H-benzo[d]imidazol-1-yl)cyclopropyl)benzoic acid methyl ester

The title compound (50 mg, yield: 41%) was obtained in the same manner as in step A of Example 139 by using 3-(1-(5-chloro-2-oxo-2,3-dihydro-1*H*-benzo[d]imidazol-1-yl)cyclopropyl)benzoic acid methyl ester (81 mg, 0.23 mmol) obtained in Preparation Example 3 and 6-(4-bromophenyl)-2-oxa-6-azaspiro[3.3]heptane (60 mg, 0.23 mmol) obtained in Preparation Example 39.
¹H-NMR (400 MHz, CDCl3) δ7.90 (dd, 1H), 7.86 (m, 1H), 7.49 (dd, 1H), 7.35 (t, 1H), 7.30 (d, 2H), 7.10 (d, 1H), 7.04 (dd, 1H), 6.96 (d, 1H), 6.55 (d, 2H), 4.87 (s, 4H), 4.09 (s, 4H), 3.90 (s, 3H), 1.72 (m, 2H), 1.67 (m, 2H)

### Step B: Preparation of 3-(1-(3-(4-(2-oxa-6-azaspiro[3.3]heptane-6-yl)phenyl)-5-chloro-2-oxo-2,3-dihydro-1H-benzo[d]imidazol-1-yl)cyclopropyl)benzoic acid

The title compound (44 mg, yield: 99%) was obtained in the same manner as in step B of Example 24 by using 3-(1-(3-(4-(2-oxa-6-azaspiro[3.3]heptane-6-yl)phenyl)-5-chloro-2-oxo-2,3-dihydro-1*H*-benzo[*d*]imidazol-1-yl)cyclopropyl)benzoic acid methyl ester (45 mg, 0.87 mmol) obtained in step A above.
MS [M+H] = 502 (M+1)
¹H-NMR (400 MHz, DMSO-d6) δ7.78 (dd, 1H), 7.67 (d, 1H), 7.38 (t, 1H), 7.32 (3H), 7.21 (d, 1H), 7.12 (dd, 1H), 6.84 (d, 1H), 6.58 (d, 2H), 4.74 (s, 4H), 4.05 (s, 4H), 1.64 (m, 4H)

### Example 150: Preparation of 3-((5-chloro-3-(4-(1-(cyclopropanecarbonyl)piperidin-4-yl)phenyl)-4-fluoro-2-oxo-2,3-dihydro-1H-benzo[d]imidazol-1-yl)methyl)-2-fluorobenzoic acid

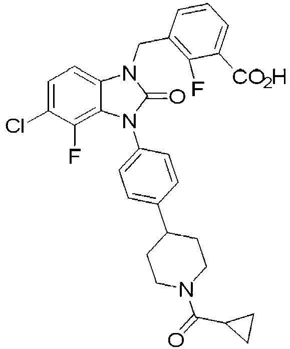

The title compound (50 mg, yield: 58%) was obtained in the same manner as in step A of Example 100 and step B of Example 24 by using 3-((5-chloro-4-fluoro-2-oxo-3-(4-(piperidin-4-yl)phenyl)-2,3-dihydro-1*H*-benzo[*d*]imidazol-1-yl)methyl)-2-fluorobenzoic acid methyl ester hydrochloride (85 mg, 0.15 mmol) obtained in Preparation Example 43 and cyclopropanecarbonyl chloride (17 mg, 0.17 mmol).
MS [M+Na] = 589 (M+23)
¹H-NMR (400 MHz, CDCl3) δ7.97 (td, 1H), 7.69 (td, 1H), 7.41 (dd, 2H), 7.34 (d, 2H), 7.21 (t, 1H), 7.13 (dd, 1H), 6.86 (d, 1H), 5.21 (s, 2H), 4.61 (m, 2H), 3.00 (m, 2H), 2.85 (m, 1H), 1.97 (m, 2H), 1.82 (m, 1H), 1.70 (m, 2H), 1.05 (m, 2H), 0.80 (m, 2H)

### Example 151: Preparation of 3-((5-chloro-4-fluoro-2-oxo-3-(4-(1-propionylpiperidin-4-yl)phenyl)-2,3-dihydro-1H-benzo[d]imidazol-1-yl)methyl)-2-fluorobenzoic acid

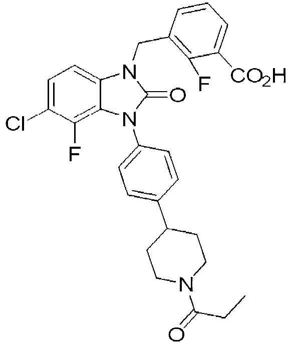

The process of the following steps A and B gave the title compound.

### Step A: Preparation of 3-((5-chloro-4-fluoro-2-oxo-3-(4-(1-propionylpiperidin-4-yl)phenyl)-2,3-dihydro-1H-benzo[d]imidazol-1-yl)methyl)-2-fluorobenzoic acid methyl ester

The title compound (55 mg, yield: 65%) was obtained in the same manner as in step A of Example 102 by using 3-((5-chloro-4-fluoro-2-oxo-3-(4-(piperidin-4-yl)phenyl)-2,3-dihydro-1*H*-benzo[*d*]imidazol-1-yl)methyl)-2-fluorobenzoic acid methyl ester hydrochloride (85 mg, 0.15 mmol) obtained in Preparation Example 43 and propionyl chloride (17 mg, 0.17 mmol).
¹H-NMR (400 MHz, CDCl3) δ7.90 (td, 1H), 7.63 (td, 1H), 7.40 (dd, 2H), 7.31 (d, 2H), 7.18 (t, 1H), 7.12 (dd, 1H), 6.84 (d, 1H), 5.19 (s, 2H), 4.84 (m, 1H), 4.00 (m, 1H), 3.95 (s, 3H), 3.14 (m, 1H), 2.82 (m, 1H), 2.65 (m, 1H), 2.40 (q, 2H), 1.94 (m, 2H), 1.65 (m, 2H), 1.19 (t, 3H)

### Step B: Preparation of 3-((5-chloro-4-fluoro-2-oxo-3-(4-(1-propionylpiperidin-4-yl)phenyl)-2,3-dihydro-1H-benzo[d]imidazol-1-yl)methyl)-2-fluorobenzoic acid

The title compound (50 mg, yield: 93%) was obtained in the same manner as in step B of Example 24 by using 3-((5-chloro-4-fluoro-2-oxo-3-(4-(1-propionylpiperidin-4-yl)phenyl)-2,3-dihydro-1*H*-benzo[*d*]imidazol-1-yl)methyl)-2-fluorobenzoic acid methyl ester (55 mg, 0.097 mmol) obtained in step A above.
MS [M+Na] = 576 (M+23)
¹H-NMR (400 MHz, CDCl3) δ7.98 (td, 1H), 7.69 (td, 1H), 7.41 (dd, 1H), 7.32 (d, 2H), 7.21 (t, 1H), 7.13 (dd, 1H), 6.86 (d, 1H), 5.20 (s, 2H), 4.85 (m, 1H), 4.05 (m, 1H), 3.16 (m, 1H), 2.83 (m, 1H), 2.67 (m, 1H), 2.43 (q, 2H), 1.95 (m, 2H), 1.65 (m, 2H), 1.20 (t, 3H)

### Example 152: Preparation of 3-((5-chloro-3-(4-(1-(cyclobutanecarbonyl)piperidin-4-yl)phenyl)-4-fluoro-2-oxo-2,3-dihydro-1H-benzo[d]imidazol-1-yl)methyl)-2-fluorobenzoic acid

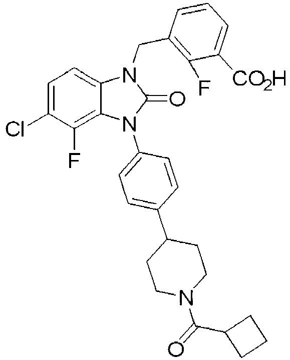

The title compound (40 mg, yield: 44%) was obtained in the same manner as in step A of Example 142 and step B of Example 24 by using 3-((5-chloro-4-fluoro-2-oxo-3-(4-(piperidin-4-yl)phenyl)-2,3-dihydro-1*H*-benzo[*d*]imidazol-1-yl)methyl)-2-fluorobenzoic acid methyl ester hydrochloride (85 mg, 0.15 mmol) obtained in Preparation Example 43 and cyclobutanecarboxylic acid (17 mg, 0.17 mmol).
MS [M+H] = 580 (M+1)
¹H-NMR (400 MHz, CDCl3) δ7.95 (td, 1H), 7.64 (td, 1H), 7.40 (dd, 2H), 7.30 (d, 2H), 7.18 (t, 1H), 7.10 (dd, 1H), 6.84 (d, 1H), 5.19 (s, 2H), 4.80 (m, 1H), 3.86 (m, 1H), 3.33 (m, 1H), 3.08 (m, 1H), 2.80 (m, 1H), 2.67 (m, 1H), 2.40 (m, 2H), 2.18 (m, 2H), 2.00-1.85 (4H), 1.60 (m, 2H)

### Example 153: Preparation of 3-(2-(5-chloro-3-(4-(dimethylamino)phenyl)-2-oxo-2,3-dihydro-1H-benzo[d]imidazol-1-yl)propane-2-yl)benzoic acid

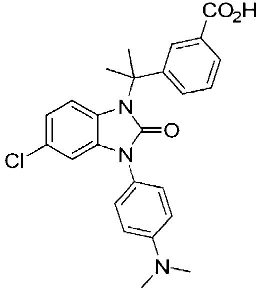

The process of the following steps A and B gave the title compound.

### Step A: Preparation of 3-(2-(5-chloro-3-(4-(dimethylamino)phenyl)-2-oxo-2,3-dihydro-1H-benzo[d]imidazol-1-yl)propane-2-yl)benzoic acid methyl ester

The title compound (46 mg, yield: 56%) was obtained in the same manner as in step A of Example 1 by using 3-(2-(5-chloro-2-oxo-2,3-dihydro-1*H*-benzo[d]imidazol-1-yl)propan-2-yl)benzonitrile (60 mg, 0.19 mmol) obtained in Preparation Example 41 and (4-(dimethylamino)phenyl)boronic acid (64 mg, 0.385 mmol).
¹H-NMR (400 MHz, CDCl3) δ7.70 (m, 1H), 7.64 (m, 1H), 7.60 (m, 1H), 7.48 (t, 1H), 7.30 (d, 2H), 6.91 (d, 1H), 6.82 (d, 2H), 6.73 (dd, 1H), 6.14 (dd, 1H), 3.02 (s, 6H), 2.11 (s, 6H)

### Step B: Preparation of 3-(2-(5-chloro-3-(4-(dimethylamino)phenyl)-2-oxo-2,3-dihydro-1H-benzo[d]imidazol-1-yl)propane-2-yl)benzoic acid methyl ester

To 3-(2-(5-chloro-3-(4-(dimethylamino)phenyl)-2-oxo-2,3-dihydro-1*H*-benzo[d]imidazol-1-yl)propane-2-yl)benzoic acid methyl ester (46 mg, 0.11 mmol) obtained in step A above were added 2 mL of EtOH and an excess KOH solid, and the resulting mixture was stirred under reflux for 4 hours. To the reaction solution was added water, and then the reaction solution was adjusted to pH 3, and then extracted with EtOAc. An organic layer was separated and purified by MPLC to give the title compound (18 mg, yield: 38%).
MS [M+H] = 450 (M+1)
¹H-NMR (500 MHz, CDCl3) 8.18 (m, 1H), 8.05 (m, 1H), 7.63 (m, 1H), 7.48 (m, 1H), 7.3 1(m, 2H), 6.89 (m, 1H), 6.83 (m, 2H), 6.68 (m, 1H), 6.06 (d, 1H), 3.01 (s, 6H), 2.05 (s, 6H)

### Example 154: Preparation of 3-(2-(5-chloro-3-(4-(3-hydroxyazetidin-1-yl)phenyl)-2-oxo-2,3-dihydro-1H-benzo[d]imidazol-1-yl)propane-2-yl)benzoic acid

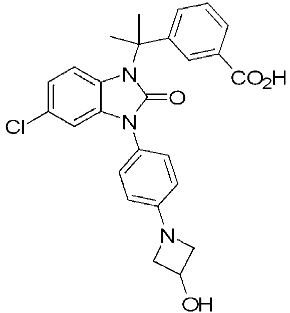

The process of the following steps A and B gave the title compound.

### Step A: Preparation of 3-(2-(5-chloro-3-(4-(3-hydroxyazetidin-1-yl)phenyl)-2-oxo-2,3-dihydro-1H-benzo[d]imidazol-1-yl)propane-2-yl)benzoic acid methyl ester

The title compound (57 mg, yield: 65%) was obtained in the same manner as in step A of Example 139 by using 3-(2-(5-chloro-2-oxo-2,3-dihydro-1*H*-benzo[*d*]imidazol-1-yl)propan-2-yl)benzonitrile (60 mg, 0.19 mmol) obtained in Preparation Example 41 and 1-(4-bromophenyl)azetidin-3-ol (44 mg, 0.19 mmol) obtained in Preparation Example 38.
¹H-NMR (400 MHz, CDCl3) δ7.66 (m, 1H), 7.65 (m, 1H), 7.60 (m, 1H), 7.48 (t, 1H), 7.29 (d, 2H), 6.89 (dd, 1H), 6.7 (dd, 1H), 6.47 (d, 2H), 6.14 (d, 1H), 4.78 (m, 1H), 4.23 (t, 2H), 3.74 (m, 2H), 2.32 (m, 1H), 2.08 (s, 6H)

### Step B: Preparation of 3-(2-(5-chloro-3-(4-(3-hydroxyazetidin-1-yl)phenyl)-2-oxo-2,3-dihydro-1H-benzo[d]imidazol-1-yl)propane-2-yl)benzoic acid

The title compound (4 mg, yield: 6%) was obtained in the same manner as in step B of Example 153 by using 3-(2-(5-chloro-3-(4-(3-hydroxyazetidin-1-yl)phenyl)-2-oxo-2,3-dihydro-1*H*-benzo[*d*]imidazol-1-yl)propane-2-yl)benzoic acid methyl ester (57 mg, 0.12 mmol) obtained in step A above.
MS [M+H] = 478 (M+1)
¹H-NMR (400 MHz, CDCl3) δ8.18 (m, 1H), 8.04 (m, 1H), 7.62 (m, 1H), 7.46 (m, 1H), 7.30 (d, 2H), 6.87 (m, 1H), 6.69 (m, 1H), 6.49 (d, 2H), 6.07 (m, 1H), 4.80 (m, 1H), 4.24 (m, 2H), 3.38 (m, 2H), 2.12 (s, 6H)

### Example 155: Preparation of 3-(1-(5-chloro-2-oxo-3-(4-(tetrahydro-2H-pyran-4-yl)phenyl)-2,3-dihydro-1H-benzo[d]imidazol-1-yl)cyclobutyl)benzoic acid

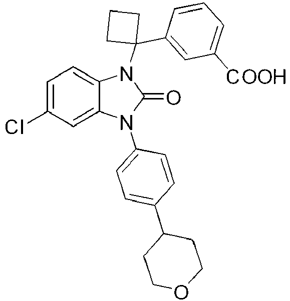

The process of the following steps A and B gave the title compound.

### Step A: Preparation of 3-(1-(5-chloro-2-oxo-3-(4-(tetrahydro-2H-pyran-4-yl)phenyl)-2,3-dihydro-1H-benzo[d]imidazol-1-yl)cyclobutyl)benzoic acid methyl ester

The title compound (11.4 mg, yield: 79%) was obtained in the same manner as in step A of Example 2 by using 3-(1-(5-chloro-2-oxo-2,3-dihydro-1*H*-benzo[*d*]imidazol-1-yl)cyclobutyl)benzoic acid methyl ester (9.99 mg, 0.0280 mmol) obtained in Preparation Example 46 and (4-(tetrahydro-2*H-*pyran-4-yl)phenyl)boronic acid (11.54 mg, 0.056 mmol).

### Step B: Preparation of 3-(1-(5-chloro-2-oxo-3-(4-(tetrahydro-2H-pyran-4-yl)phenyl)-2,3-dihydro-1H-benzo[d]imidazol-1-yl)cyclobutyl)benzoic acid

The title compound (4.3 mg, 41%) was obtained in the same manner as in step B of Example 2 by using 3-(1-(5-chloro-2-oxo-3-(4-(tetrahydro-2*H*-pyran-4-yl)phenyl)-2,3-dihydro-1*H-*benzo[*d*]imidazol-1-yl)cyclobutyl)benzoic acid methyl ester (10.86 mg, 0.021 mmol) obtained in step A above.
MS [M+H] = 503 (M+1)
¹H NMR (500 MHz, CDCl3) δ8.42 (s, 1H), 8.04 (d, 1H), 7.97 (d,1H), 7.48 (t, 1H), 7.44 (d, 2H), 7.38 (d, 2H), 7.03 (m, 2H), 6.93 (m, 1H), 4.12 (d, 2H), 3.55 (t, 2H), 3.20 (m, 2H), 3.06 (m, 2H), 2.82 (m, 1H), 2.06 (m, 2H), 1.84 (m, 4H)

### Example 156: Preparation of 3-(1-(5-chloro-3-(4-(dimethylamino)phenyl)-2-oxo-2,3-dihydro-1H-benzo[d]imidazol-1-yl)cyclobutyl)benzoic acid

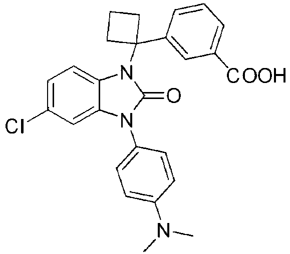

The process of the following steps A and B gave the title compound.

### Step A: Preparation of 3-(1-(5-chloro-3-(4-(dimethylamino)phenyl)-2-oxo-2,3-dihydro-1H-benzo[d]imidazol-1-yl)cyclobutyl)benzoic acid methyl ester

The title compound (13.3 mg, yield: 60%) was obtained in the same manner as in step A of Example 2 by using 3-(1-(5-chloro-2-oxo-2,3-dihydro-1*H*-benzo[d]imidazol-1-yl)cyclobutyl)benzoic acid methyl ester (9.99 mg, 0.0280 mmol) obtained in Preparation Example 46 and (4-(dimethylamino)phenyl)boronic acid (9.24 mg, 0.056 mmol).
¹H NMR (500 MHz, CDCl3) δ8.36 (s, 1H), 7.97 (d, 1H), 7.94 (d, 1H), 7.42 (t, 1H), 7.31 (d, 2H), 6.97 (m, 2H), 6.88 (m, 3H), 3.92 (s, 3H), 3.17 (m, 2H), 3.05 (m, 2H), 3.00 (s, 6H), 2.05 (m, 2H)

### Step B: Preparation of 3-(1-(5-chloro-3-(4-(dimethylamino)phenyl)-2-oxo-2,3-dihydro-1H-benzo[d]imidazol-1-yl)cyclobutyl)benzoic acid

The title compound (7.6 mg, 98%) was obtained in the same manner as in step B of Example 2 by using 3-(1-(5-chloro-3-(4-(dimethylamino)phenyl)-2-oxo-2,3-dihydro-1H-benzo[*d*]imidazol-1-yl)cyclobutyl)benzoic acid methyl ester (8.00 mg, 0.017 mmol) obtained in step A above.
MS [M+H] = 462 (M+1)
¹H NMR (500 MHz, CDCl3) δ8.42 (s, 1H), 8.03 (d, 1H), 7.97 (d, 1H), 7.46 (t, 1H), 7.30 (d, 2H), 6.98 (m, 2H), 6.90 (m, 1H), 6.81 (d, 2H), 3.21 (m, 2H), 3.06 (m, 2H), 2.99 (s, 6H), 2.09 (m, 2H)

### Example 157: Preparation of 3-(1-(5-chloro-3-(4-(dimethylcarbamoyl)phenyl)-2-oxo-2,3-dihydro-1H-benzo[d]imidazol-1-yl)cyclobutyl)benzoic acid

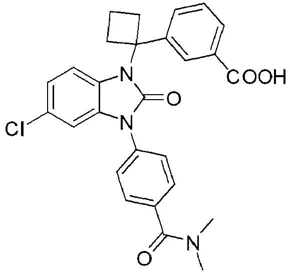

The process of the following steps A and B gave the title compound.

### Step A: Preparation of 3-(1-(5-chloro-3-(4-(dimethylcarbamoyl)phenyl)-2-oxo-2,3-dihydro-1H-benzo[d]imidazol-1-yl)cyclobutyl)benzoic acid methyl ester

The title compound (12.6 mg, yield: 89%) was obtained in the same manner as in step A of Example 2 by using 3-(1-(5-chloro-2-oxo-2,3-dihydro-1*H*-benzo[*d*]imidazol-1-yl)cyclobutyl)benzoic acid methyl ester (9.99 mg, 0.0280 mmol) obtained in Preparation Example 46 and (4-(dimethylcarbamoyl)phenyl)boronic acid (10.81 mg, 0.056 mmol).
¹H NMR (500 MHz, CDCl3) δ8.37 (s, 1H), 7.98 (d, 1H), 7.91 (d, 1H), 7.58 (1, 4H), 7.45 (t, 1H), 7.05 (s, 1H), 7.03 (d, 1H), 6.95 (d, 1H), 3.92 (s, 3H), 3.21 (m, 4H), 3.03 (s, 6H), 2.05 (m, 2H)

### Step B: Preparation of 3-(1-(5-chloro-3-(4-(dimethylcarbamoyl)phenyl)-2-oxo-2,3-dihydro-1H-benzo[d]imidazol-1-yl)cyclobutyl)benzoic acid

The title compound (6.3 mg, yield: 54%) was obtained in the same manner as in step B of Example 2 by using 3-(1-(5-chloro-3-(4-(dimethylcarbamoyl)phenyl)-2-oxo-2,3-dihydro-1*H-*benzo[*d*]imidazol-1-yl)cyclobutyl)benzoic acid methyl ester (12.00 mg, 0.024 mmol) obtained in step A above.
MS [M+H] = 490 (M+1)
¹H NMR (500 MHz, CDCl3) δ8.42 (s, 1H), 8.04 (d, 1H), 7.95 (d, 1H), 7.60 (m, 4H), 7.48 (t, 1H), 7.05 (m, 2H), 6.95 (d, 1H), 3.18 (m, 4H), 3.04 (s, 6H), 2.06 (m, 2H)

### Example 158: Preparation of 3-(2-(5-chloro-2-oxo-3-(4-(tetrahydro-2H-pyran-4-yl)phenyl)-2,3-dihydro-1H-benzo[d]imidazol-1-yl)propan-2-yl)benzoic acid

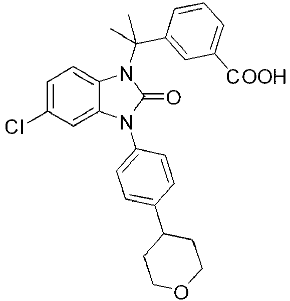

The process of the following steps A and B gave the title compound.

### Step A: Preparation of 3-(2-(5-chloro-2-oxo-3-(4-(tetrahydro-2H-pyran-4-yl)phenyl)-2,3-dihydro-1H-benzo[d]imidazol-1-yl)propan-2-yl)benzoic acid methyl ester

The title compound (9.0 mg, yield: 61%) was obtained in the same manner as in step A of Example 2 by using 3-(2-(5-chloro-2-oxo-2,3-dihydro-1*H*-benzo[*d*]imidazol-1-yl)propan-2-yl)benzoic acid methyl ester (10.0 mg, 0.029 mmol) obtained in Preparation Example 47 and (4-(tetrahydro-2*H*-pyran-4-yl)phenyl)boronic acid (11.95 mg, 0.058 mmol).

### Step B: Preparation of 3-(2-(5-chloro-2-oxo-3-(4-(tetrahydro-2H-pyran-4-yl)phenyl)-2,3-dihydro-1H-benzo[d]imidazol-1-yl)propan-2-yl)benzoic acid

The title compound (5.7 mg, yield: 65%) was obtained in the same manner as in step B of Example 2 by using 3-(2-(5-chloro-2-oxo-3-(4-(tetrahydro-2*H*-pyran-4-yl)phenyl)-2,3-dihydro-1*H*-benzo[*d*]imidazol-1-yl)propan-2-yl)benzoic acid methyl ester (9.0 mg, 0.018 mmol) obtained in step A above.
MS [M+H] = 491 (M+1)
¹H NMR (500 MHz, CDCl3) δ8.17 (s, 1H), 8.06 (d, 1H), 7.64 (d, 1H), 7.49 (m, 3H), 7.40 (d, 2H), 6.97 (s, 1H), 6.73 (d, 1H), 6.11 (d, 1H), 4.14 (d, 2H), 3.59 (t, 2H), 2.87 (m, 1H), 2.13 (s, 6H), 1.86 (m, 4H)

### Example 159: Preparation of 3-(1-(5-chloro-3-(4-(3-hydroxyazetidin-1-yl)phenyl)-2-oxo-2,3-dihydro-1H-benzo[d]imidazol-1-yl)cyclopropyl)benzoic acid

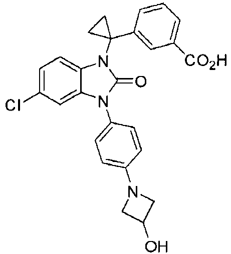

The process of the following steps A and B gave the title compound.

### Step A: Preparation of 3-(1-(5-chloro-3-(4-(3-hydroxyazetidin-1-yl)phenyl)-2-oxo-2,3-dihydro-1H-benzo[d]imidazol-1-yl)cyclopropyl)benzoic acid methyl ester

The title compound (12 mg, 18.6%) was obtained in the same manner as in step A of Example 139 by using 3-(1-(5-chloro-2-oxo-2,3-dihydro-1*H*-benzo[*d*]imidazol-1-yl)cyclopropyl)benzoic acid methyl ester (45 mg, 0.132 mmol) obtained in Preparation Example 3 and 1-(4-bromophenyl)azetidin-3-ol (30 mg, 0.132 mmol) obtained in Preparation Example 38.
¹H-NMR (500 MHz, CDCl3) δ7.91 (d, 1H), 7.87 (s, 1H), 7.51 (d, 1H), 7.37 (t, 1H), 7.32 (d, 2H), 7.12 (d, 1H), 7.08 (d, 1H), 6.99 (s, 1H), 6.59 (d, 2H), 4.80 (m, 1H), 4.25 (t, 1H), 3.92 (s, 3H), 3.76 (t, 2H), 1.74 (m, 2H), 1.70 (m, 2H)

### Step B: Preparation of 3-(1-(5-chloro-3-(4-(3-hydroxyazetidin-1-yl)phenyl)-2-oxo-2,3-dihydro-1H-benzo[d]imidazol-1-yl)cyclopropyl)benzoic acid

The title compound (12 mg, yield: 41%) was obtained in the same manner as in step B of Example 24 by using 3-(1-(5-chloro-3-(4-(3-hydroxyazetidin-1-yl)phenyl)-2-oxo-2,3-dihydro-1*H*-benzo[*d*]imidazol-1-yl)cyclopropyl)benzoic acid methyl ester (30 mg, 0.061 mmol) obtained in step A above.
¹H-NMR (500 MHz, CDCl3) δ7.95 (d, 1H), 7.91 (s, 1H), 7.53 (d, 1H), 7.40 (t, 1H), 7.33 (d, 2H), 7.13 (d, 1H), 7.08 (d, 1H), 7.00 (s, 1H), 6.59 (d, 2H), 4.80 (m, 1H), 4.24 (t, 2H), 3.76 (t, 2H), 1.76 (m, 2H), 1.72 (m, 2H)

### Example 160: Preparation of 3-(1-(5-chloro-3-(4-(4,4-difluoropiperidin-1-yl)phenyl)-2-oxo-2,3-dihydro-1H-benzo[d]imidazol-1-yl)cyclopropyl)benzoic acid

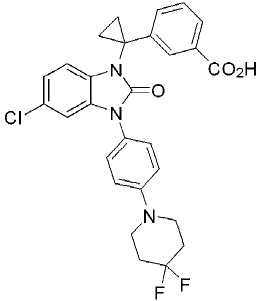

The process of the following steps A and B gave the title compound.

### Step A: Preparation of 3-(1-(5-chloro-3-(4-(4,4-difluoropiperidin-1-yl)phenyl)-2-oxo-2,3-dihydro-1H-benzo[d]imidazol-1-yl)cyclopropyl)benzoic acid methyl ester

The title compound (12 mg, 22%) was obtained in the same manner as in step A of Example 146 by using 3-(1-(3-(4-bromophenyl)-5-chloro-2-oxo-2,3-dihydro-1*H*-benzo[*d*]imidazol-1-yl)cyclopropyl)benzoic acid methyl ester (50 mg, 0.1 mmol) obtained in step A of Example 121 and 4,4-difluoropiperidine (12 mg, 0.1 mmol).
¹H-NMR (500 MHz, CDCl3) δ7.91 (d, 1H), 7.88 (s, 1H), 7.52 (d, 1H), 7.38 (m, 3H), 7.14 (d, 1H), 7.08 (m, 3H), 7.04 (s, 1H), 3.92 (s, 3H), 3.45 (m, 4H), 2.15 (m, 4H), 1.75 (m, 2H), 1.69 (m, 2H)

### Step B: Preparation of 3-(1-(5-chloro-3-(4-(4,4-difluoropiperidin-1-yl)phenyl)-2-oxo-2,3-dihydro-1H-benzo[d]imidazol-1-yl)cyclopropyl)benzoic acid

The title compound (13 mg, yield: 67.5%) was obtained in the same manner as in step B of Example 24 by using 3-(1-(5-chloro-3-(4-(4,4-difluoropiperidin-1-yl)phenyl)-2-oxo-2,3-dihydro-1*H*-benzo[*d*]imidazol-1-yl)cyclopropyl)benzoic acid methyl ester (20 mg, 0.037 mmol) obtained in step A above.
¹H-NMR (500 MHz, CDCl3) δ7.97 (d, 1H), 7.93 (s, 1H), 7.56 (d, 1H), 7.41 (m, 3H), 7.14 (d, 1H), 7.09 (m, 3H), 7.05 (s, 1H), 3.45 (m, 4H), 2.14 (m, 4H), 1.76 (m, 2H), 1.70 (m, 2H)

### Example 161: Preparation of 3-((6-chloro-1-(4-(dimethylamino)phenyl)-2-oxo-1,2-dihydro-3H-imidazo[4,5-b]pyridin-3-yl)methyl)benzoic acid

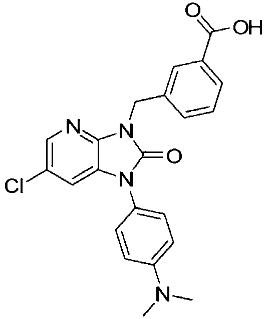

The title compound (17 mg, yield: 32%) was obtained in the same manner as in step A of Example 1 and step B of Example 24 by using 3-((6-chloro-2-oxo-1,2-dihydro-3*H*-imidazo[4,5-*b*]pyridin-3-yl)methyl)benzoic acid methyl ester (40 mg, 0.13 mmol) obtained in Preparation Example 48 and (4-(dimethylamino)phenyl)boronic acid (42 mg, 0.25 mmol).
¹H-NMR (500 MHz, DMSO-d6) δ8.08 (s, 1H), 7.97 (s, 1H), 7.86 (d, 1H), 7.63 (d, 1H), 7.48 (t, 1H), 7.34 (d, 2H), 7.32 (s, 1H), 6.84 (d, 2H), 5.17 (s, 2H), 2.97 (s, 6H).

### Example 162: Preparation of 3-((6-chloro-1-(4-morpholinophenyl)-2-oxo-1,2-dihydro-3H-imidazo[4,5-b]pyridin-3-yl)methyl)benzoic acid

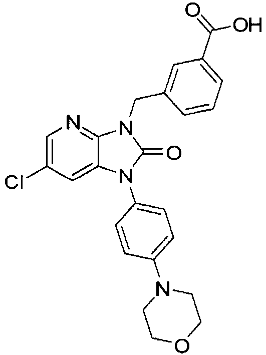

The title compound (25 mg, yield: 43%) was obtained in the same manner as in step A of Example 1 and step B of Example 24 by using 3-((6-chloro-2-oxo-1,2-dihydro-3*H*-imidazo[4,5-*b*]pyridin-3-yl)methyl)benzoic acid methyl ester (40 mg, 0.13 mmol) obtained in Preparation Example 48 and (4-(tetrahydro-2*H*-pyran-4-yl)phenyl)boronic acid (52 mg, 0.25 mmol).
¹H-NMR (500 MHz, CDCl3) δ8.31 (s, 1H), 8.13 (s, 1H), 8.04 (d, 1H), 7.80 (d, 1H), 7.45 (m, 5H), 7.33 (s, 1H), 5.30 (s, 2H), 4.14 (d, 2H), 3.58 (t, 2H), 2.87 (m, 1H), 1.87 (m, 4H)

### Example 163: Preparation of 3-((5-chloro-3-(5-cyclopropylpyridin-2-yl)-2-oxo-2,3-dihydro-1H-benzo[d]imidazol-1-yl)methyl)benzoic acid

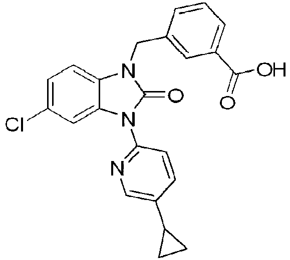

The process of the following steps A and B gave the title compound.

### Step A: Preparation of 3-((3-(5-bromopyridin-2-yl)-5-chloro-2-oxo-2,3-dihydro-1H-benzo[d]imidazol-1-yl)methyl)benzoic acid methyl ester

To 3-((5-Chloro-2-oxo-2,3-dihydro-1*H-*benzo[*d*]imidazol-1-yl)methyl)benzoic acid methyl ester (0.50 g, 1.58 mmol) obtained in Preparation Example 1 and 5-bromo-2-fluoropyridine (0.30 g, 1.73 mmol) were added 16 mL of DMF and Cs₂CO₃ (1.03 g, 3.16 mmol), and the resulting mixture was stirred at 100 °C for 6 hours. To the reaction solution was added an aqueous ammonium chloride solution, and the resulting solid was dried to give the title compound (0.10 g, yield: 13%).
¹H-NMR (400 MHz, CDCl3) δ8.63 (d, 1H), 8.20 (2H), 8.03 (d, 1H), 7.98 (2H), 7.53 (dd, 1H), 7.41 (t, 1H), 7.08 (dd, 1H), 6.81 (d, 1H), 5.15 (s, 2H), 3.91 (s, 3H)

### Step B: Preparation of 3-((5-chloro-3-(5-cyclopropylpyridin-2-yl)-2-oxo-2,3-dihydro-1H-benzo[d]imidazol-1-yl)methyl)benzoic acid methyl ester

To 3-((3-(5-bromopyridin-2-yl)-5-chloro-2-oxo-2,3-dihydro-1*H*-benzo[*d*]imidazol-1-yl)methyl)benzoic acid methyl ester (77 mg, 0.16 mmol) obtained in step A above were added 1.4 mL of toluene, cyclopropylboronic acid (28 mg, 0.32 mmol), and tricyclohexylphosphine (4 mg, 0.013 mmol), and then the resulting mixture was charged with nitrogen gas. To the mixture were added K₃PO₄ (0.24 mL, 2.0 M aqueous solution) and Pd(OAc)₂ (1.5 mg, 0.007 mmol), followed by stirring at 100 °C for 16 hours. The resulting solid was removed through Celite and the filtrate was purified by MPLC to give the title compound (26 mg, yield: 37%).
¹H-NMR (400 MHz, CDCl3) δ8.39 (d, 1H), 8.05 (d, 1H), 8.03-7.96 (3H), 7.50 (2H), 7.41 (t, 1H), 7.05 (dd, 1H), 6.78 (d, 1H), 5.15 (s, 2H), 3.91 (s, 3H), 1.95 (m, 1H), 1.08 (m, 2H), 0.78 (m, 2H)

### Step C: Preparation of 3-((5-chloro-3-(5-cyclopropylpyridin-2-yl)-2-oxo-2,3-dihydro-1H-benzo[d]imidazol-1-yl)methyl)benzoic acid

The title compound (17 mg, yield: 70%) was obtained in the same manner as in step B of Example 24 by using 3-((5-chloro-3-(5-cyclopropylpyridin-2-yl)-2-oxo-2,3-dihydro-1*H-*benzo[*d*]imidazol-1-yl)methyl)benzoic acid methyl ester (25 mg, 0.058 mmol) obtained in step B above.
MS [M+H] = 420 (M+1)
¹H-NMR (400 MHz, CDCl3) δ 8.39 (d, 1H), 8.10-7.96 (4H), 7.58 (dd, 1H), 7.45 (2H), 7.06 (dd, 1H), 6.80 (d, 1H), 5.17 (s, 2H), 1.97 (m, 1H), 1.07 (m, 2H), 0.78 (m, 2H)

### Example 164: Preparation of 3-((5-chloro-3-(5-(dimethylamino)pyridin-2-yl)-2-oxo-2,3-dihydro-1H-benzo[d]imidazol-1-yl)methyl)benzoic acid

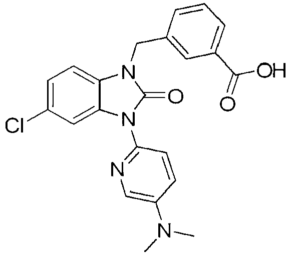

The process of the following steps A, B, and C gave the title compound.

### Step A: Preparation of 3-((5-chloro-3-(5-nitropyridin-2-yl)-2-oxo-2,3-dihydro-1H-benzo[d]imidazol-1-yl)methyl)benzoic acid methyl ester

The title compound (0.30 g, yield: 72%) was obtained in the same manner as in step A of Example 163 by using 3-((5-Chloro-2-oxo-2,3-dihydro-1*H*-benzo[*d*]imidazol-1-yl)methyl)benzoic acid methyl ester (0.30 g, 0.95 mmol) obtained in Preparation Example 1 and 2-fluoro-5-nitropyridine.
¹H-NMR (400 MHz, DMSO-d6) δ9.47 (d, 1H), 8.81 (dd, 1H), 8.56 (d, 1H), 8.38 (d, 1H), 8.03 (d, 1H), 7.90 (dd, 1H), 7.67 (dd, 1H), 7.51 (t, 1H), 7.31 (2H), 5.27 (s, 2H), 3.83 (s, 3H)

### Step B: Preparation of 3-((3-(5-aminopyridin-2-yl)-5-chloro-2-oxo-2,3-dihydro-1H-benzo[d]imidazol-1-yl)methyl)benzoic acid methyl ester

The title compound (0.23 g, yield: 85%) was obtained in the same manner as in step B of Preparation Example 7 by using 3-((5-chloro-3-(5-nitropyridin-2-yl)-2-oxo-2,3-dihydro-1*H*-benzo[*d*]imidazol-1-yl)methyl)benzoic acid methyl ester (0.30 g, 0.68 mmol) obtained in step A above.
¹H-NMR (400 MHz, DMSO-d6) δ7.98 (d, 1H), 7.92 (d, 1H), 7.88 (dd, 1H), 7.53 (dd, 1H), 7.53 (3H), 7.23 (d, 1H), 7.15 (2H), 5.57 (brs, 2H), 5.21 (s, 2H), 3.84 (s, 3H)

### Step C: Preparation of 3-((5-chloro-3-(5-(dimethylamino)pyridin-2-yl)-2-oxo-2,3-dihydro-1H-benzo[d]imidazol-1-yl)methyl)benzoic acid

To 3-((3-(5-aminopyridin-2-yl)-5-chloro-2-oxo-2,3-dihydro-1*H-*benzo[*d*]imidazol-1-yl)methyl)benzoic acid methyl ester (0.10 g, 0.24 mmol) obtained in step B above was added 5 mL of THF, and then the resulting mixture was cooled to 0 °C, NaH (55 wt% in oil, 34 mg, 0.78 mmol) was added thereto, followed by stirring at room temperature for 30 minutes. To the mixture was added iodomethane (0.11 mL, 1.7 mmol), and then the resulting mixture was stirred under reflux for 16 hours. After the reaction was terminated, an aqueous ammonium chloride solution was added thereto, and the resultant mixture was extracted with EtOAc to separate an organic layer. The organic layer was purified by MPLC to give the title compound (6 mg, yield: 6%).
MS [M+H] = 423 (M+1)
¹H-NMR (400 MHz, CDCl3) δ8.08 (d, 1H), 8.04 (d, 1H), 7.97 (dd, 1H), 7.74 (2H), 7.52 (dd, 1H), 7.37 (t, 1H), 7.15 (dd, 1H), 7.01 (dd, 1H), 6.77 (d, 1H), 5.13 (s, 2H), 3.02 (s, 6H)

### Example 165: Preparation of 3-(1-(5-chloro-3-(6-(3,3-difluoropyrrolidin-1-yl)pyridin-3-yl)-2-oxo-2,3-dihydro-1H-benzo[d]imidazol-1-yl)cyclopropyl)benzoic acid

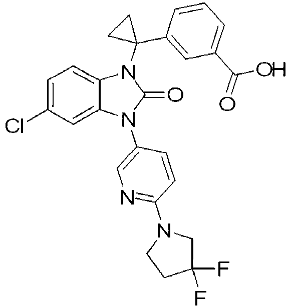

The process of the following steps A and B gave the title compound.

### Step A: Preparation of 3-(1-(5-chloro-3-(6-fluoropyridin-3-yl)-2-oxo-2,3-dihydro-1H-benzo[d]imidazol-1-yl)cyclopropyl)benzoic acid methyl ester

The title compound (0.10 g, yield: 39%) was obtained in the same manner as in step A of Example 1 by using 3-(1-(5-chloro-2-oxo-2,3-dihydro-1*H*-benzo[*d*]imidazol-1-yl)cyclopropyl)benzoic acid methyl ester (0.20 g, 0.58 mmol) obtained in Preparation Example 3 and (6-fluoropyridin-3-yl)boronic acid (0.16 g, 1.16 mmol).
¹H-NMR (400 MHz, CDCl3) δ8.45 (m, 1H), 8.03 (td, 1H), 7.91 (dd, 1H), 7.88 (m, 1H), 7.48 (dd, 1H), 7.37 (t, 1H), 7.15 (3H), 7.05 (d, 1H), 3.91 (s, 3H), 1.70 (m, 4H)

### Step B: Preparation of 3-(1-(5-chloro-3-(6-(3,3-difluoropyrrolidin-1-yl)pyridin-3-yl)-2-oxo-2,3-dihydro-1H-benzo[d]imidazol-1-yl)cyclopropyl)benzoic acid

The title compound (13 mg, yield: 11%) was obtained in the same manner as in step A of Preparation Example 1 and step B of Example 24 by using 3-(1-(5-chloro-3-(6-fluoropyridin-3-yl)-2-oxo-2,3-dihydro-1*H*-benzo[*d*]imidazol-1-yl)cyclopropyl)benzoic acid methyl ester (100 mg, 0.23 mmol) obtained in step A and 3,3-difluoropyrrolidine hydrochloride (66 mg, 0.46 mmol).
MS [M+H] = 511 (M+1)
¹H-NMR (400 MHz, MeOH-d4) δ8.25 (d, 1H), 7.90 (m, 1H), 7.83 (d, 1H), 7.72 (dd, 1H), 7.43 (2H), 7.25 (d, 1H), 7.16 (dd, 1H), 6.96 (d, 1H), 6.73 (dd, 1H), 3.91 (t, 2H), 3.76 (t, 2H), 2.60 (m, 2H), 1.73 (4H)

### Example 166: Preparation of 3-((5-chloro-3-(6-(3,3-difluoropyrrolidine-1-yl)pyridin-3-yl)-2-oxo-2,3-dihydro-1H-benzo[d]imidazol-1-yl)methyl)-2-fluorobenzoic acid

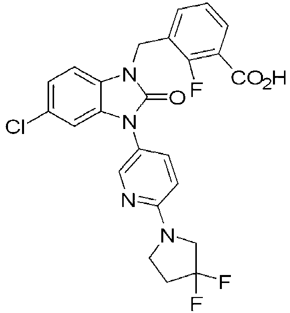

The title compound (122 mg, yield: 85%) was obtained in the same manner as in step C of Preparation Example 7 and step B of Example 24 by using 6-chloro-1-(6-(3,3-difluoropyrrolidin-1-yl)pyridin-3-yl)-1,3-dihydro-2*H-*benzo[d]imidazol-2-one (100 mg, 0.28 mmol) obtained in Preparation Example 44 and 3-(bromomethyl)-2-fluorobenzoic acid methyl ester (70 mg, 0.28 mmol).
MS [M+H] = 503 (M+1)
¹H-NMR (400 MHz, DMSO-d6) δ8.28 (d, 1H), 7.76 (2H), 7.52 (td, 1H), 7.27 (t, 1H), 7.21 (dd, 1H), 7.16 (dd, 1H), 6.92 (d, 1H), 6.73 (dd, 1H), 5.21 (s, 2H), 3.91 (t, 2H), 3.69 (t, 2H), 2.60 (m, 2H)

### Example 167: Preparation of 3-((3-(4-(dimethylamino)phenyl)-5-methyl-2-oxo-2,3-dihydro-1H-imidazo[4,5-b]pyridin-1-yl)methyl)-2-fluorobenzoic acid

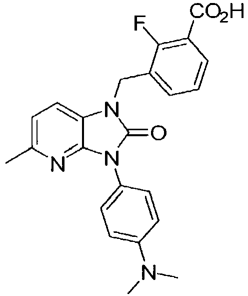

The process of the following steps A and B gave the title compound.

### Step A: Preparation of 3-((3-(4-(dimethylamino)phenyl)-5-methyl-2-oxo-2,3-dihydro-1H-imidazo[4,5-b]pyridin-1-yl)methyl)-2-fluorobenzoic acid methyl ester

The title compound (70 mg, yield: 66%) was obtained in the same manner as in step C of Preparation Example 7 by using 3-(4-(dimethylamino)phenyl)-5-methyl-1,3-dihydro-2*H-*imidazo[4,5-*b*]pyridin-2-one (65 mg, 0.24 mmol) obtained in Preparation Example 45 and 3-(bromomethyl)-2-fluorobenzoic acid methyl ester (60 mg, 0.24 mmol).
¹H-NMR (400 MHz, CDCl3) δ7.88 (m, 1H), 7.66 (m, 1H), 7.50 (d, 2H), 7.15 (2H), 6.83 (3H), 5.19 (s, 2H), 3.95 (s, 3H), 3.00 (s, 6H), 2.47 (s, 3H)

### Step B: Preparation of 3-((3-(4-(dimethylamino)phenyl)-5-methyl-2-oxo-2,3-dihydro-1H-imidazo[4,5-b]pyridin-1-yl)methyl)-2-fluorobenzoic acid

The title compound (25 mg, yield: 37%) was obtained in the same manner as in step B of Example 24 by using 3-((3-(4-(dimethylamino)phenyl)-5-methyl-2-oxo-2,3-dihydro-1*H-*imidazo[4,5-*b*]pyridin-1-yl)methyl)-2-fluorobenzoic acid methyl ester (70 mg, 0.16 mmol) obtained in step A above.
MS [M+H] = 421 (M+1)
¹H-NMR (400 MHz, DMSO-d6) δ7.79 (m, 1H), 7.53 (dd, 1H), 7.40 (d, 1H), 7.34 (d, 2H), 7.26 (t, 1H), 6.95 (d, 1H), 6.83 (d, 2H), 5.20 (s, 2H), 2.96 (s, 6H), 2.36 (s, 3H)

### Example 168: Preparation of 3-((3-(4-(dimethylamino)phenyl)-5-methyl-2-oxo-2,3-dihydro-1H-imidazo[4,5-b]pyridin-1-yl)methyl)benzoic acid

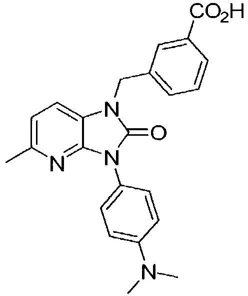

The process of the following steps A and B gave the title compound.

### Step A: Preparation of 3-((3-(4-(dimethylamino)phenyl)-5-methyl-2-oxo-2,3-dihydro-1H-imidazo[4,5-b]pyridin-1-yl)methyl)benzoic acid methyl ester

The title compound (67 mg, yield: 60%) was obtained in the same manner as in step C of Preparation Example 7 by using 3-(4-(dimethylamino)phenyl)-5-methyl-1,3-dihydro-2*H-*imidazo[4,5-*b*]pyridin-2-one (65 mg, 0.24 mmol) obtained in Preparation Example 45 and 3-(bromomethyl)benzoic acid methyl ester (55 mg, 0.24 mmol).
¹H-NMR (400 MHz, CDCl3) δ8.05 (d, 1H), 7.97 (dd, 1H), 7.58 (dd, 1H), 7.53 (d, 2H), 7.42 (t, 1H), 6.94 (d, 1H), 6.85 (d, 2H), 6.77 (dd, 1H), 5.16 (s, 2H), 3.92 (s, 3H), 3.00 (s, 6H), 2.46 (s, 3H)

### Step B: Preparation of 3-((3-(4-(dimethylamino)phenyl)-5-methyl-2-oxo-2,3-dihydro-1H-imidazo[4,5-b]pyridin-1-yl)methyl)benzoic acid

The title compound (20 mg, yield: 35%) was obtained in the same manner as in step B of Example 24 by using 3-((3-(4-(dimethylamino)phenyl)-5-methyl-2-oxo-2,3-dihydro-1*H-*imidazo[4,5-*b*]pyridin-1-yl)methyl)benzoic acid methyl ester (60 mg, 0.14 mmol) obtained in step A above.
MS [M+H] = 403 (M+1)
¹H-NMR (400 MHz, DMSO-d6) δ7.94 (m, 1H), 7.85 (dd, 1H), 7.58 (dd, 1H), 7.48 (t, 1H), 7.41 (d, 1H), 7.35 (d, 2H), 6.93 (d, 1H), 6.84 (d, 2H), 5.18 (s, 2H), 2.96 (s, 6H), 2.38 (s, 3H)

### Example 169: Preparation of 3-((6-chloro-2-oxo-1-(4-(pyrrolidine-1-carbonyl)phenyl)-1,2-dihydro-3H-imidazo[4,5-b]pyridin-3-yl)methyl)benzoic acid

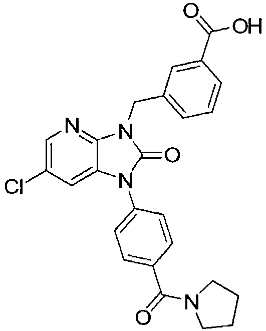

The title compound (39 mg, yield: 53%) was obtained in the same manner as in Example 56 by using 3-((6-chloro-2-oxo-1,2-dihydro-3*H*-imidazo[4,5-*b*]pyridin-3-yl)methyl)benzoic acid methyl ester (90 mg, 0.28 mmol) obtained in Preparation Example 48.
¹H-NMR (400 MHz, CDCl3) δ8.24 (s, 1H), 8.08 (s, 1H), 7.98 (d, 1H), 7.71 (m, 3H), 7.55 (d, 2H), 7.40 (m, 1H), 7.30 (s, 1H), 5.23 (s, 2H), 3.67 (t, 2H), 3.46 (t, 2H), 1.94 (m, 4H) .

### Example 170: Preparation of 3-(1-(5-chloro-2-oxo-3-(4-(2-oxo-2-(pyrrolidin-1-yl)ethyl)phenyl)-2,3-dihydro-1H-benzo[d]imidazol-1-yl)cyclopropyl)benzoic acid

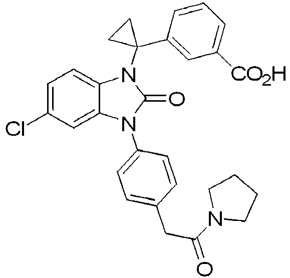

The title compound (47 mg, yield: 31%) was obtained in the same manner as in Example 139 by using 3-(1-(5-chloro-2-oxo-2,3-dihydro-1*H*-benzo[*d*]imidazol-1-yl)cyclopropyl)benzoic acid methyl ester (100 mg, 0.29 mmol) obtained in Preparation Example 3 and 2-(4-bromophenyl)-1-(pyrrolidin-1-yl)ethan-1-one (80 mg, 0.32 mmol).
¹H-NMR (500 MHz, DMSO-d6) δ7.81 (m, 1H), 7.70 (d, 1H), 7.51 (m, 2H), 7.44 (4H), 7.26 (m, 1H), 7.17 (m, 1H), 7.04 (d, 1H), 4.03 (d, 2H), 3.54 (m, 2H), 3.30 (m, 2H), 1.90 (m, 2H), 1.81 (m, 2H), 1.68 (4H)

### Example 171: Preparation of 3-(1-(5-chloro-2-oxo-3-(4-(2-oxo-2-(piperidin-1-yl)ethyl)phenyl)-2,3-dihydro-1H-benzo[d]imidazol-1-yl)cyclopropyl)benzoic acid

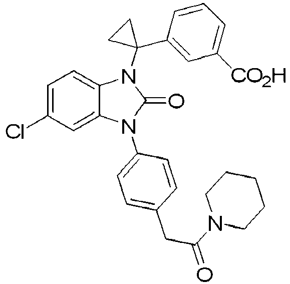

The title compound (30 mg, yield: 20%) was obtained in the same manner as in Example 139 by using 3-(1-(5-chloro-2-oxo-2,3-dihydro-1*H*-benzo[*d*]imidazol-1-yl)cyclopropyl)benzoic acid methyl ester (100 mg, 0.29 mmol) obtained in Preparation Example 3 and 2-(4-bromophenyl)-1-(piperidin-1-yl)ethan-1-one (91 mg, 0.32 mmol).
¹H-NMR (500 MHz, DMSO-d6) δ7.81 (m, 1H), 7.69 (d, 1H), 7.51 (m, 2H), 7.44 (4H), 7.26 (m, 1H), 7.17 (m, 1H), 7.02 (d, 1H), 3.80 (d, 2H), 3.45 (m, 4H), 1.68 (m, 4H), 1.56 (m, 2H), 1.43 (m, 4H)

### Example 172: Preparation of 3-(1-(5-chloro-3-(4-(2-morpholino-2-oxoethyl)phenyl)-2-oxo-2,3-dihydro-1H-benzo[d]imidazol-1-yl)cyclopropyl)benzoic acid

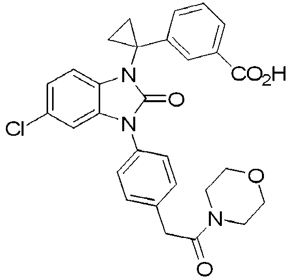

The title compound (48 mg, yield: 15%) was obtained in the same manner as in Example 139 by using 3-(1-(5-chloro-2-oxo-2,3-dihydro-1*H*-benzo[*d*]imidazol-1-yl)cyclopropyl)benzoic acid methyl ester (200 mg, 0.58 mmol) obtained in Preparation Example 3 and 2-(4-bromophenyl)-1-morpholinoethan-1-one (182 mg, 0.64 mmol).
¹H-NMR (500 MHz, DMSO-d6) δ7.80 (d, 1H), 7.69 (d, 1H), 7.51 (m, 2H), 7.43 (4H), 7.26 (m, 1H), 7.18 (m, 1H), 7.03 (d, 1H), 3.83 (s, 2H), 3.56 (6H), 3.49 (2H), 1.68 (4H)

### Example 173: Preparation of 3-(1-(5-chloro-3-(4-(2-(4,4-difluoropiperidin-1-yl)-2-oxoethyl)phenyl)-2-oxo-2,3-dihydro-1H-benzo[d]imidazol-1-yl)cyclopropyl)benzoic acid

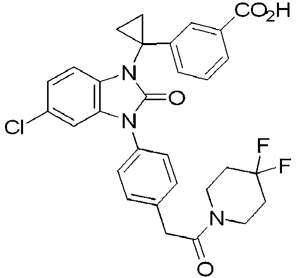

The title compound (42 mg, yield: 25%) was obtained in the same manner as in Example 139 by using 3-(1-(5-chloro-2-oxo-2,3-dihydro-1*H*-benzo[*d*]imidazol-1-yl)cyclopropyl)benzoic acid methyl ester (100 mg, 0.29 mmol) obtained in Preparation Example 3 and 2-(4-bromophenyl)-1-(4,4-difluoropiperidin-1-yl)ethan-1-one (93 mg, 0.29 mmol) obtained in Preparation Example 65.
¹H-NMR (500 MHz, CDCl3) δ7.97 (d, 1H), 7.93 (s, 1H), 7.54 (m, 3H), 7.45 (m, 2H), 7.41 (t, 1H), 7.16 (d, 1H), 7.12 (m, 2H), 3.85 (s, 2H), 3.80 (m, 2H), 3.62 (m, 2H), 2.00 (m, 2H), 1.90 (m, 2H), 1.76 (m, 2H), 1.71 (m, 2H)

### Example 174: Preparation of 3-(1-(5-chloro-3-(4-(2-(3-hydroxyazetidin-1-yl)-2-oxoethyl)phenyl)-2-oxo-2,3-dihydro-1H-benzo[d]imidazol-1-yl)cyclopropyl)benzoic acid

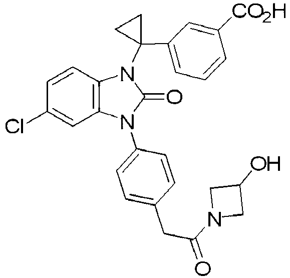

The title compound (72 mg, yield: 24%) was obtained in the same manner as in Example 139 by using 3-(1-(5-chloro-2-oxo-2,3-dihydro-1*H*-benzo[*d*]imidazol-1-yl)cyclopropyl)benzoic acid methyl ester (190 mg, 0.55 mmol) obtained in Preparation Example 3 and 2-(4-bromophenyl)-1-(3-hydroxyazetidin-1-yl)ethan-1-one (150 mg, 0.55 mmol) obtained in Preparation Example 64.
¹H-NMR (500 MHz, CDCl3) δ7.96 (d, 1H), 7.93 (s, 1H), 7.48 (m, 5H), 7.41 (t, 1H), 7.15 (d, 1H), 7.11 (m, 2H), 5.32 (s, 2H), 4.63 (m, 1H), 4.35 (t, 1H), 4.25 (m, 1H), 4.03 (dd, 1H), 3.89 (dd, 1H), 1.76 (m, 2H), 1.69 (m, 2H)

### Example 175: Preparation of (R)-3-(1-(5-chloro-3-(4-(2-(2-methylmorpholino)-2-oxoethyl)phenyl)-2-oxo-2,3-dihydro-1H-benzo[d]imidazol-1-yl)cyclopropyl)benzoic acid

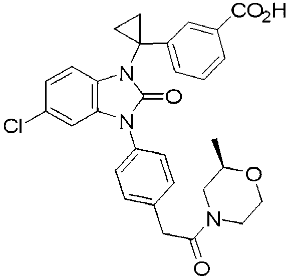

The title compound (8 mg, yield: 3%) was obtained in the same manner as in steps A and B of Example 139 by using 3-(1-(5-chloro-2-oxo-2,3-dihydro-1*H*-benzo[*d*]imidazol-1-yl)cyclopropyl)benzoic acid methyl ester (170 mg, 0.50 mmol) obtained in Preparation Example 3 and (R)-2-(4-bromophenyl)-1-(2-methylmorpholino)ethan-1-one (150 mg, 0.50 mmol) obtained in Preparation Example 68.
¹H-NMR (500 MHz, CDCl3) δ7.95 (m, 1H), 7.91 (m, 1H), 7.51 (m, 3H), 7.41 (m, 3H), 7.12 (m, 3H), 4.49 (dd, 1H), 3.90 (dd, 1H), 3.80 (s, 2H), 3.71 (t, 1H), 3.51 (m, 1H), 3.41 (m, 1H), 2.83 (m, 2H), 2.47 (m, 1H), 1.75 (m, 2H), 1.69 (m, 2H), 1.19 (3H)

### Example 176: Preparation of (S)-3-(1-(5-chloro-3-(4-(2-(2-methylmorpholino)-2-oxoethyl)phenyl)-2-oxo-2,3-dihydro-1H-benzo[d]imidazol-1-yl)cyclopropyl)benzoic acid

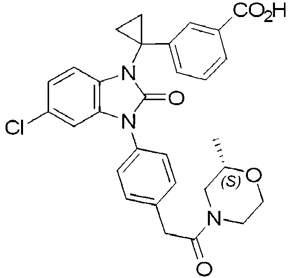

The title compound (78 mg, yield: 31%) was obtained in the same manner as in Example 139 by using 3-(1-(5-chloro-2-oxo-2,3-dihydro-1*H*-benzo[*d*]imidazol-1-yl)cyclopropyl)benzoic acid methyl ester (150 mg, 0.44 mmol) obtained in Preparation Example 3 and (*S*)-2-(4-bromophenyl)-1-(2-methylmorpholino)ethan-1-one (130 mg, 0.44 mmol) obtained in Preparation Example 69.
¹H-NMR (500 MHz, CDCl3) δ7.97 (d, 1H), 7.93 (s, 1H), 7.53 (m, 3H), 7.44 (d, 2H), 7.40 (t, 1H), 7.16 (d, 1H), 7.11 (m, 2H), 4.49 (dd, 1H), 3.94 (dd, 1H), 3.82 (s, 2H), 3.70 (t, 1H), 3.52 (m, 1H), 3.42 (m, 1H), 2.93 (m, 2H), 2.49 (m, 1H), 1.76 (m, 2H), 1.70 (m, 2H), 1.20 (3H)

### Example 177: Preparation of (S)-3-(1-(5-chloro-3-(4-(2-(3-methylmorpholino)-2-oxoethyl)phenyl)-2-oxo-2,3-dihydro-1H-benzo[d]imidazol-1-yl)cyclopropyl)benzoic acid

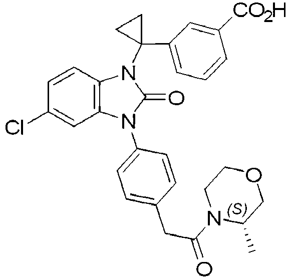

The title compound (14 mg, yield: 5%) was obtained in the same manner as in steps A and B of Example 139 by using 3-(1-(5-chloro-2-oxo-2,3-dihydro-1*H*-benzo[*d*]imidazol-1-yl)cyclopropyl)benzoic acid methyl ester (150 mg, 0.44 mmol) obtained in Preparation Example 3 and (*S*)-2-(4-bromophenyl)-1-(3-methylmorpholino)ethan-1-one (130 mg, 0.44 mmol) obtained in Preparation Example 66.
¹H-NMR (500 MHz, CDCl3) δ7.95 (d, 1H), 7.91 (s, 1H), 7.51 (m, 3H), 7.40 (m, 3H), 7.14 (d, 1H), 7.09 (m, 2H), 4.34 (m, 1H), 3.94 (m, 1H), 3.84 (m, 1H), 3.78 (s, 2H), 3.70 (m, 1H), 3.49 (m, 2H), 3.35 (m, 1H), 3.08 (m, 1H), 1.74 (m, 2H), 1.69 (m, 2H), 1.28 (3H)

### Example 178: Preparation of (R)-3-(1-(5-chloro-3-(4-(2-(3-methylmorpholino)-2-oxoethyl)phenyl)-2-oxo-2,3-dihydro-1H-benzo[d]imidazol-1-yl)cyclopropyl)benzoic acid

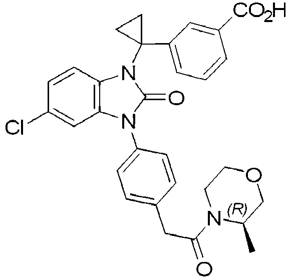

The title compound (140 mg, yield: 44%) was obtained in the same manner as in steps A and B of Example 139 by using 3-(1-(5-chloro-2-oxo-2,3-dihydro-1*H*-benzo[*d*]imidazol-1-yl)cyclopropyl)benzoic acid methyl ester (200 mg, 0.58 mmol) obtained in Preparation Example 3 and (*R*)-2-(4-bromophenyl)-1-(3-methylmorpholino)ethan-1-one (174 mg, 0.58 mmol) obtained in Preparation Example 67.
¹H-NMR (500 MHz, CDCl3) δ7.95 (d, 1H), 7.90 (s, 1H), 7.51 (m, 3H), 7.44 (d, 1H), 7.39 (m, 2H), 7.14 (d, 1H), 7.09 (m, 2H), 4.34 (m, 1H), 3.93 (m, 1H), 3.84 (m, 1H), 3.80 (s, 2H), 3.70 (m, 1H), 3.47 (m, 2H), 3.35 (m, 1H), 3.07 (m, 1H), 1.74 (m, 2H), 1.69 (m, 2H), 1.28 (3H)

### Example 179: Preparation of 3-(1-(3-(4-((3r,5r,7r)-adamantan-1-yl)phenyl)-5-chloro-2-oxo-2,3-dihydro-1H-benzo[d]imidazol-1-yl)cyclopropyl)benzoic acid

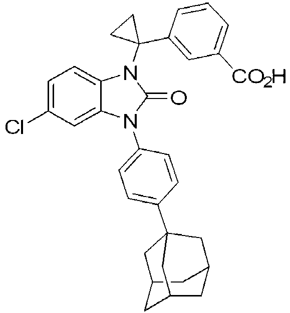

The title compound (16 mg, yield: 10.2%) was obtained in the same manner as in Example 139 by using 3-(1-(5-chloro-2-oxo-2,3-dihydro-1*H*-benzo[*d*]imidazol-1-yl)cyclopropyl)benzoic acid methyl ester (100 mg, 0.29 mmol) obtained in Preparation Example 3 and (3r,5r,7r)-1-(4-bromophenyl)adamantane (93 mg, 0.32 mmol).
¹H-NMR (500 MHz, CDCl3) δ7.97 (d, 1H), 7.93 (s, 1H), 7.57 (d, 1H), 7.54 (d, 2H), 7.48 (d, 2H), 7.41 (t, 1H), 7.12 (m, 3H), 2.14 (m, 3H), 1.97 (m, 6H), 1.80 (m, 9H), 1.71 (m, 2H)

### Example 180: Preparation of 3-(1-(5-chloro-3-(4-(methylsulfonyl)phenyl)-2-oxo-2,3-dihydro-1H-benzo[d]imidazol-1-yl)cyclopropyl)benzoic acid

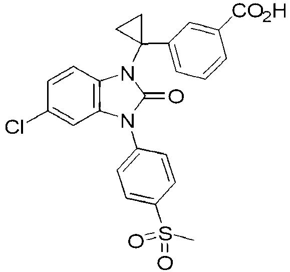

The title compound (97 mg, yield: 69.3%) was obtained in the same manner as in steps A and B of Example 1 by using 3-(1-(5-chloro-2-oxo-2,3-dihydro-1*H*-benzo[*d*]imidazol-1-yl)cyclopropyl)benzoic acid methyl ester (100 mg, 0.29 mmol) obtained in Preparation Example 3 and (4-(methylsulfonyl)phenyl)boronic acid (88 mg, 0.44 mmol).
¹H-NMR (500 MHz, CDCl3) δ8.16 (d, 2H), 7.99 (d, 1H), 7.96 (s, 1H), 7.85 (d, 2H), 7.56 (d, 1H), 7.43 (t, 1H), 7.20 (m, 3H), 3.13 (s, 3H), 1.76 (m, 2H), 1.73 (m, 2H)

### Example 181: Preparation of 3-(1-(3-(4-(2-oxa-5-azabicyclo[2.2.1]heptan-5-yl)phenyl)-5-chloro-2-oxo-2,3-dihydro-1H-benzo[d]imidazol-1-yl)cyclopropyl)benzoic acid

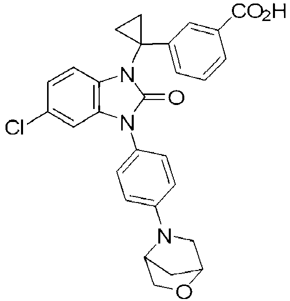

The process of the following steps A and B gave the title compound.

### Step A: Preparation of 3-(1-(3-(4-(2-oxa-5-azabicyclo[2.2.1]heptan-5-yl)phenyl)-5-chloro-2-oxo-2,3-dihydro-1H-benzo[d]imidazol-1-yl)cyclopropyl)benzoic acid methyl ester

To 3-(1-(3-(4-bromophenyl)-5-chloro-2-oxo-2,3-dihydro-1*H*-benzo[*d*]imidazol-1-yl)cyclopropyl)benzoic acid methyl ester (100 mg, 0.20 mmol) obtained in step A of Example 121, 2-oxa-5-azabicyclo[2.2.1]heptane hydrochloride (30 mg, 0.22 mmol), and BINAP (13 mg, 0.02 mmol) was added 2 mL of toluene, and then the resulting mixture was charged with nitrogen gas. To the mixture were added Pd₂(dba)₃ (10 mg, 10 mmol) and Cs₂CO₃ (140 mg, 0.44 mmol), followed by stirring at 100 °C for 16 hours. After the reaction solution was cooled, the resulting solid was filtered through Celite, and the filtrate was purified by MPLC to give the title compound (7 mg, yield: 7%).
¹H-NMR (400 MHz, CDCl3) 7.90 (m, 1H), 7.86 (m, 1H), 7.48 (m, 1H), 7.36 (d, 1H), 7.31 (m, 2H), 7.10 (d, 1H), 7.03 (dd, 1H), 6.99 (m, 1H), 6.69 (m, 2H), 4.69 (m, 1H), 4.45 (m, 1H), 3.96 (m, 1H), 3.90 (s, 3H), 3.89 (m, 1H), 3.59 (dd, 1H), 3.21 (dd, 1H), 2.04 (m, 2H), 1.73 (m, 2H), 1.66 (m, 2H)

### Step B: Preparation of 3-(1-(3-(4-(2-oxa-5-azabicyclo[2.2.1]heptan-5-yl)phenyl)-5-chloro-2-oxo-2,3-dihydro-1H-benzo[d]imidazol-1-yl)cyclopropyl)benzoic acid

The title compound (2 mg, yield: 30%) was obtained in the same manner as in step B of Example 24 by using 3-(1-(3-(4-(2-oxa-5-azabicyclo[2.2.1]heptan-5-yl)phenyl)-5-chloro-2-oxo-2,3-dihydro-1*H*-benzo[*d*]imidazol-1-yl)cyclopropyl)benzoic acid methyl ester (7 mg, 0.014 mmol) obtained in step A above.
¹H-NMR (400 MHz, CDCl3) δ7.79 (2H), 7.32 (m, 2H), 7.28 (m, 1H), 7.22 (m, 2H), 7.10 (dd, 1H), 6.90 (d, 1H), 6.81 (d, 2H), 4.68 (m, 1H), 4.60 (m, 1H), 3.89 (m, 2H), 3.61 (m, 1H), 3.12 (m, 1H), 2.05 (m, 1H), 2.00 (m, 1H), 1.70 (m, 4H)

### Example 182: Preparation of 3-(1-(3-(4-(8-oxa-3-azabicyclo[3.2.1]octan-3-yl)phenyl)-5-chloro-2-oxo-2,3-dihydro-1H-benzo[d]imidazol-1-yl)cyclopropyl)benzoic acid

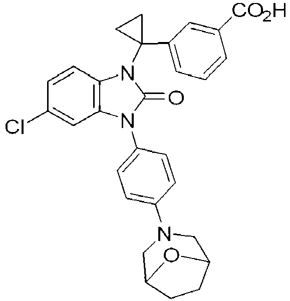

The title compound (5 mg, yield: 5%) was obtained in the same manner as in steps A and B of Example 181 by using 3-(1-(3-(4-bromophenyl)-5-chloro-2-oxo-2,3-dihydro-1*H-*benzo[*d*]imidazol-1-yl)cyclopropyl)benzoic acid methyl ester (100 mg, 0.20 mmol) obtained in step A of Example 121 and 8-oxa-3-azabicyclo[3.2.1]octane hydrochloride (33 mg, 0.22 mmol).
MS [M+H] = 516 (M+1)
¹H-NMR (400 MHz, CDCl3) δ7.93 (m, 1H), 7.90 (m, 1H), 7.52 (m, 1H), 7.37 (3H), 7.11 (d, 1H), 7.05 (dd, 1H), 7.01 (m, 1H), 6.92 (d, 2H), 4.52 (m, 2H), 3.38 (m, 2H), 3.08 (m, 2H), 2.00 (4H), 1.74 (m, 2H), 1.67 (m, 2H)

### Example 183: Preparation of 3-(1-(3-(4-(1,4-oxazepan-4-yl)phenyl)-5-chloro-2-oxo-2,3-dihydro-1H-benzo[d]imidazol-1-yl)cyclopropyl)benzoic acid

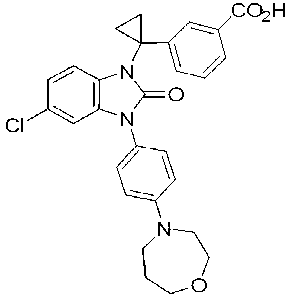

The title compound (5 mg) was obtained in the same manner as in steps A and B of Example 139 by using 3-(1-(5-chloro-2-oxo-2,3-dihydro-1*H*-benzo[*d*]imidazol-1-yl)cyclopropyl)benzoic acid methyl ester (100 mg, 0.29 mmol) obtained in Preparation Example 3 and 4-(4-bromophenyl)-1,4-oxazepane (140 mg, 0.55 mmol) obtained in Preparation Example 51.
¹H-NMR (400 MHz, MeOH-d4) δ7.88 (m, 1H), 7.81 (m, 1H), 7.42 (m, 2H), 7.29 (d, 2H), 7.23 (d, 1H), 7.11 (dd, 1H), 6.93 (3H), 3.86 (m, 2H), 3.72 (m, 6H), 2.04 (m, 2H), 1.70 (m, 4H)

### Example 184: Preparation of 3-(1-(3-(4-(2-azaspiro[3.3]heptan-2-yl)phenyl)-5-chloro-2-oxo-2,3-dihydro-1H-benzo[d]imidazol-1-yl)cyclopropyl)benzoic acid

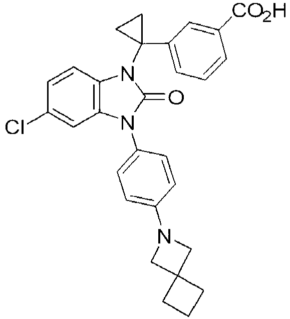

The title compound (13 mg, yield: 10%) was obtained in the same manner as in steps A and B of Example 139 by using 3-(1-(5-chloro-2-oxo-2,3-dihydro-1*H*-benzo[*d*]imidazol-1-yl)cyclopropyl)benzoic acid methyl ester (100 mg, 0.29 mmol) obtained in Preparation Example 3 and 2-(4-bromophenyl)-2-azaspiro[3.3]heptane (120 mg, 0.47 mmol) obtained in Preparation Example 52.
¹H-NMR (400 MHz, DMSO-d6) δ7.80 (dd, 1H), 7.67 (d, 1H), 7.43 (2H), 7.28 (m, 2H), 7.22 (d, 1H), 7.14 (dd, 1H), 6.85 (d, 1H), 6.54 (d, 2H), 3.84 (s, 4H), 2.20 (m, 4H), 1.85 (m, 2H) 1.66 (m, 4H)

### Example 185: Preparation of 3-(1-(5-chloro-3-(4-(3,3-difluoroazetidin-1-yl)phenyl)-2-oxo-2,3-dihydro-1H-benzo[d]imidazol-1-yl)cyclopropyl)benzoic acid

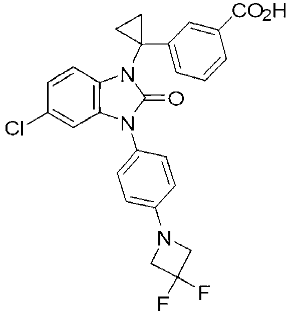

The title compound (14 mg, yield: 14%) was obtained in the same manner as in steps A and B of Example 181 by using 3-(1-(3-(4-bromophenyl)-5-chloro-2-oxo-2,3-dihydro-1*H-*benzo[*d*]imidazol-1-yl)cyclopropyl)benzoic acid methyl ester (100 mg, 0.20 mmol) obtained in step A of Example 121 and 3,3-difluoroazetidine hydrochloride (52 mg, 0.40 mmol).
¹H-NMR (500 MHz, DMSO-d6) δ13.03 (brs, 1H), 7.80 (dd, 1H), 7.68 (d, 1H), 7.43 (4H), 7.23 (dd, 1H), 7.14 (dd, 1H), 6.89 (d, 1H), 6.74 (d, 2H), 4.35 (t, 4H), 1.67 (m, 4H)

### Example 186: Preparation of 3-(1-(3-(4-(7-acetyl-2,7-diazaspiro[3.5]nonan-2-yl)phenyl)-5-chloro-2-oxo-2,3-dihydro-1H-benzo[d]imidazol-1-yl)cyclopropyl)benzoic acid

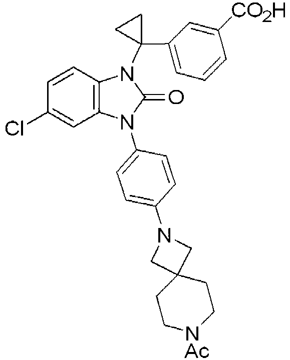

The process of the following steps A, B, and C gave the title compound.

### Step A: Preparation of 2-(4-(6-chloro-3-(1-(3-(methoxycarbonyl)phenyl)cyclopropyl)-2-oxo-2,3-dihydro-1H-benzo[d]imidazol-1-yl)phenyl)-2,7-diazaspiro[3.5]nonane-7-carboxylic acid tert-butyl ester

The title compound (82 mg, yield: 53%) was obtained in the same manner as in step A of Example 181 by using 3-(1-(3-(4-bromophenyl)-5-chloro-2-oxo-2,3-dihydro-1*H-*benzo[*d*]imidazol-1-yl)cyclopropyl)benzoic acid methyl ester (120 mg, 0.24 mmol) obtained in step A of Example 121 and 2,7-diazaspiro[3.5]nonane-7-carboxylic acid *tert*-butyl ester hydrochloride (0.127 g, 0.482 mmol).
¹H-NMR (400 MHz, CDCl3) δ7.89 (dd, 1H), 7.86 (m, 1H), 7.48 (dd, 1H), 7.35 (t, 1H), 7.28 (m, 2H), 7.09 (d, 1H), 7.03 (dd, 1H), 6.97 (d, 1H), 6.54 (d, 2H), 3.90 (s, 3H), 3.68 (s, 4H), 3.42 (m, 4H), 1.80 (m, 4H), 1.72 (m, 2H), 1.66 (m, 2H), 1.47 (s, 9H)

### Step B: Preparation of 3-(1-(3-(4-(7-acetyl-2,7-diazaspiro[3.5]nonan-2-yl)phenyl)-5-chloro-2-oxo-2,3-dihydro-1H-benzo[d]imidazol-1-yl)cyclopropyl)benzoic acid methyl ester

To 2-(4-(6-chloro-3-(1-(3-(methoxycarbonyl)phenyl)cyclopropyl)-2-oxo-2,3-dihydro-1*H-*benzo[*d*]imidazol-1-yl)phenyl)-2,7-diazaspiro[3.5]nonane-7-carboxylic acid *tert*-butyl ester (82 mg, 0.127 mmol) obtained in step A above were added 1 mL of DCM and 0.16 mL of hydrochloric acid (4 M EtOAc solution), and the resulting mixture was stirred at room temperature for 1 hour, then concentrated under reduced pressure, and then 3 mL of DCM, acetic anhydride (0.022 mL, 0.23 mmol), and TEA (0.108 mL, 0.78 mmol) were added thereto, follwed by stirring at room temperature for 1 hour. To the reaction solution was added an aqueous NaHCO₃ solution, and then the reaction solution was extracted with DCM to separate an organic layer. The organic layer was purified by MPLC to give the title compound (70 mg, yield: 99%).
¹H-NMR (400 MHz, CDCl3) δ7.80 (dd, 1H), 7.86 (m, 1H), 7.48 (dd, 1H), 7.35 (t, 1H), 7.30 (m, 2H), 7.10 (d, 1H), 7.04 (dd, 1H), 6.97 (m, 1H), 6.55 (m, 2H), 3.90 (s, 3H), 3.71 (s, 4H), 3.61 (m, 2H), 3.45 (m, 2H), 2.12 (s, 3H), 1.88 (m, 2H), 1.83 (m, 2H), 1.73 (m, 2H), 1.66 (m, 2H)

### Step C: Preparation of 3-(1-(3-(4-(7-acetyl-2,7-diazaspiro[3.5]nonan-2-yl)phenyl)-5-chloro-2-oxo-2,3-dihydro-1H-benzo[d]imidazol-1-yl)cyclopropyl)benzoic acid

The title compound (45 mg, yield: 66%) was obtained in the same manner as in step B of Example 24 by using 3-(1-(3-(4-(7-acetyl-2,7-diazaspiro[3.5]nonan-2-yl)phenyl)-5-chloro-2-oxo-2,3-dihydro-1*H*-benzo[*d*]imidazol-1-yl)cyclopropyl)benzoic acid methyl ester (70 mg, 0.12 mmol) obtained in step B above.
¹H-NMR (400 MHz, CDCl3) δ7.80 (dd, 1H), 7.67 (d, 1H), 7.42 (2H), 7.30 (m, 2H), 7.23 (dd, 1H), 7.13 (dd, 1H), 6.86 (d, 1H), 6.57 (m, 2H), 3.67 (s, 4H), 3.43 (m, 4H), 2.10 (s, 3H), 1.79 (m, 2H), 1.66 (m, 6H)

### Example 187: Preparation of 3-(1-(5-chloro-3-(4-morpholinophenyl)-2-oxo-2,3-dihydro-1H-benzo[d]imidazol-1-yl)cyclopropyl)-5-fluorobenzoic acid

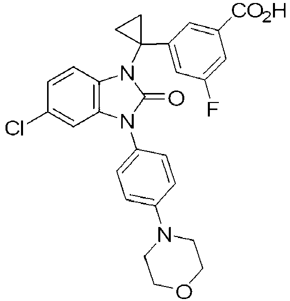

The title compound (14 mg, yield: 14%) was obtained in the same manner as in steps A and B of Example 24 by using 3-(1-(5-chloro-2-oxo-2,3-dihydro-1*H*-benzo[*d*]imidazol-1-yl)cyclopropyl)-5-fluorobenzoic acid ethyl ester (70 mg, 0.18 mmol) obtained in Preparation Example 59 and (4-morpholinophenyl)boronic acid (77 mg, 0.37 mmol).
MS [M+H] = 508 (M+1)
¹H-NMR (400 MHz, CDCl3) δ7.67 (m, 1H), 7.61 (m, 1H), 7.42 (d, 2H), 7.20 (dd, 1H), 7.07 (5H), 3.91 (m, 4H), 3.25 (m, 4H), 1.80 (4H)

### Example 188: Preparation of 3-(1-(5-chloro-3-(4-(dimethylamino)phenyl)-2-oxo-2,3-dihydro-1H-benzo[d]imidazol-1-yl)cyclopropyl)-5-fluorobenzoic acid

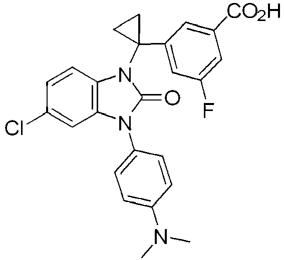

The process of the following steps A and B gave the title compound.

### Step A: Preparation of 3-(1-(5-chloro-3-(4-(dimethylamino)phenyl)-2-oxo-2,3-dihydro-1H-benzo[d]imidazol-1-yl)cyclopropyl)-5-fluorobenzoic acid ethyl ester

The title compound (55 mg, yield: 70%) was obtained in the same manner as in step A of Example 139 by using 3-(1-(5-chloro-2-oxo-2,3-dihydro-1H-benzo[d]imidazol-1-yl)cyclopropyl)-5-fluorobenzoic acid ethyl ester (60 mg, 0.16 mmol) obtained in Preparation Example 59 and 4-bromo-*N*,*N-*dimethylaniline (32 mg, 0.16 mmol).
¹H-NMR (400 MHz, CDCl3) δ7.61 (m, 1H), 7.56 (m, 1H), 7.33 (m, 2H), 7.14 (dt, 1H), 7.05 (2H), 7.01 (d, 1H), 6.82 (m, 2H), 4.36 (q, 2H), 3.02 (s, 6H), 1.76 (2H), 1.68 (2H), 1.38 (t, 3H)

### Step B: Preparation of 3-(1-(5-chloro-3-(4-(dimethylamino)phenyl)-2-oxo-2,3-dihydro-1H-benzo[d]imidazol-1-yl)cyclopropyl)-5-fluorobenzoic acid

The title compound (52 mg, yield: 99%) was obtained in the same manner as in step B of Example 24 by using 3-(1-(5-chloro-3-(4-(dimethylamino)phenyl)-2-oxo-2,3-dihydro-1*H-*benzo[d]imidazol-1-yl)cyclopropyl)-5-fluorobenzoic acid ethyl ester (55 mg, 0.11 mmol) obtained in step A above.
MS [M+H] = 466 (M+1)
¹H-NMR (400 MHz, CDCl3) δ7.64 (m, 1H), 7.58 (m, 1H), 7.33 (m, 2H), 7.19 (dt, 1H), 7.07 (m, 2H), 7.02 (m, 1H), 6.83 (d, 2H), 3.02 (s, 6H), 1.78 (m, 2H), 1.70 (m, 2H)

### Example 189: Preparation of 3-(1-(5-chloro-3-(4-(2-(dimethylamino)-2-oxoethyl)phenyl)-2-oxo-2,3-dihydro-1H-benzo[d]imidazol-1-yl)cyclopropyl)-5-fluorobenzoic acid

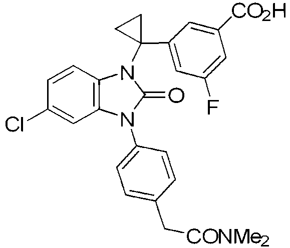

The process of the following steps A and B gave the title compound.

### Step A: Preparation of 3-(1-(5-chloro-3-(4-(2-(dimethylamino)-2-oxoethyl)phenyl)-2-oxo-2,3-dihydro-1H-benzo[d]imidazol-1-yl)cyclopropyl)-5-fluorobenzoic acid ethyl ester

The title compound (55 mg, yield: 64%) was obtained in the same manner as in step A of Example 139 by using 3-(1-(5-chloro-2-oxo-2,3-dihydro-1H-benzo[d]imidazol-1-yl)cyclopropyl)-5-fluorobenzoic acid ethyl ester (60 mg, 0.16 mmol) obtained in Preparation Example 59 and 2-(4-bromophenyl)-*N,N*-dimethylacetamide (39 mg, 0.16 mmol).
¹H-NMR (400 MHz, CDCl3) δ7.62 (m, 1H), 7.56 (m, 1H), 7.46 (4H), 7.15 (dt, 1H), 7.11 (m, 1H), 7.09 (m, 2H), 4.37 (q, 2H), 3.79 (s, 2H), 3.07 (s, 3H), 3.00 (s, 3H), 1.75 (4H), 1.37 (t, 3H)

### Step B: Preparation of 3-(1-(5-chloro-3-(4-(2-(dimethylamino)-2-oxoethyl)phenyl)-2-oxo-2,3-dihydro-1H-benzo[d]imidazol-1-yl)cyclopropyl)-5-fluorobenzoic acid

The title compound (52 mg, yield: 99%) was obtained in the same manner as in step B of Example 24 by using 3-(1-(5-chloro-3-(4-(2-(dimethylamino)-2-oxoethyl)phenyl)-2-oxo-2,3-dihydro-1H-benzo[d]imidazol-1-yl)cyclopropyl)-5-fluorobenzoic acid ethyl ester (55 mg, 0.10 mmol) obtained in step A above.
MS [M+Na] = 530 (M+23)
¹H-NMR (400 MHz, CDCl3) δ7.68 (d, 1H), 7.60 (m, 1H), 7.48 (4H), 7.20 (m, 1H), 7.10 (3H), 3.80 (s, 2H), 3.07 (s, 3H), 3.01 (s, 3H), 1.77 (m, 2H), 1.70 (m, 2H)

### Example 190: Preparation of 3-(1-(5-chloro-2-oxo-3-(4-(tetrahydro-2H-pyran-4-yl)phenyl)-2,3-dihydro-1H-benzo[d]imidazol-1-yl)cyclopropyl)-5-fluorobenzoic acid

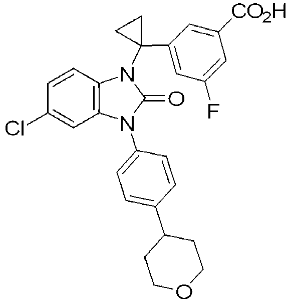

The title compound (60 mg, yield: 44%) was obtained in the same manner as in steps A and B of Example 1 by using 3-(1-(5-chloro-2-oxo-2,3-dihydro-1*H*-benzo[*d*]imidazol-1-yl)cyclopropyl)-5-fluorobenzoic acid ethyl ester (0.10 g, 0.26 mmol) obtained in Preparation Example 59 and (4-(tetrahydro-2H-pyran-4-yl)phenyl)boronic acid (0.11 g, 0.53 mmol).
MS [M+H] = 507 (M+1)
¹H-NMR (400 MHz, CDCl3) δ7.68 (d, 1H), 7.61 (m, 1H), 7.48 (d, 2H), 7.40 (d, 2H), 7.18 (m, 1H), 7.10 (2H), 7.05 (m, 1H), 4.13 (td, 2H), 3.57 (td, 2H), 2.84 (m, 1H), 1.85 (4H), 1.72 (4H)

### Example 191: Preparation of 3-(1-(3-(4-(2-oxa-6-azaspiro[3.3]heptan-6-yl)phenyl)-5-chloro-2-oxo-2,3-dihydro-1H-benzo[d]imidazol-1-yl)cyclopropyl)-5-fluorobenzoic acid

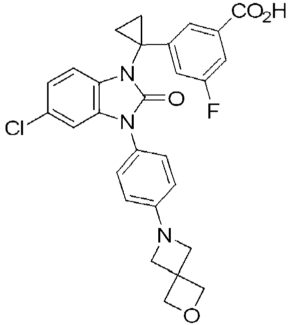

The title compound (62 mg, yield: 45%) was obtained in the same manner as in steps A and B of Example 139 by using 3-(1-(5-chloro-2-oxo-2,3-dihydro-1H-benzo[d]imidazol-1-yl)cyclopropyl)-5-fluorobenzoic acid ethyl ester (0.10 g, 0.26 mmol) obtained in Preparation Example 59 and 6-(4-bromophenyl)-2-oxa-6-azaspiro[3.3]heptane (68 mg, 0.26 mmol) obtained in Preparation Example 39.
MS [M+H] = 520 (M+1)
¹H-NMR (400 MHz, DMSO-d6) δ7.53 (m, 1H), 7.48 (m, 1H), 7.32 (3H), 7.24 (d, 1H), 7.14 (dd, 1H), 6.85 (d, 1H), 6.59 (d, 2H), 4.74 (s, 4H), 4.06 (s, 4H), 1.72 (4H)

### Example 192: Preparation of 3-(1-(3-(4-(8-oxa-3-azabicyclo[3.2.1]octan-3-yl)phenyl)-5-chloro-2-oxo-2,3-dihydro-1H-benzo[d]imidazol-1-yl)cyclopropyl)-5-fluorobenzoic acid

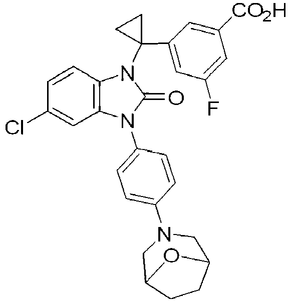

The title compound (100 mg, yield: 72%) was obtained in the same manner as in steps A and B of Example 139 by using 3-(1-(5-chloro-2-oxo-2,3-dihydro-1H-benzo[d]imidazol-1-yl)cyclopropyl)-5-fluorobenzoic acid ethyl ester (0.10 g, 0.26 mmol) obtained in Preparation Example 59 and 3-(4-bromophenyl)-8-oxa-3-azabicyclo[3.2.1]octane (72 mg, 0.26 mmol) obtained in Preparation Example 60.
MS [M+H] = 534 (M+1)
¹H-NMR (400 MHz, DMSO-d6) δ7.52 (m, 1H), 7.50 (m, 1H), 7.34 (3H), 7.23 (d, 1H), 7.14 (dd, 1H), 7.00 (d, 2H), 6.91 (d, 1H), 4.46 (m, 2H), 3.48 (2H), 2.89 (m, 2H), 1.85 (4H), 1.72 (4H)

### Example 193: Preparation of 3-(1-(3-(4-(7-oxa-2-azaspiro[3.5]nonan-2-yl)phenyl)-5-chloro-2-oxo-2,3-dihydro-1H-benzo[d]imidazol-1-yl)cyclopropyl)-5-fluorobenzoic acid

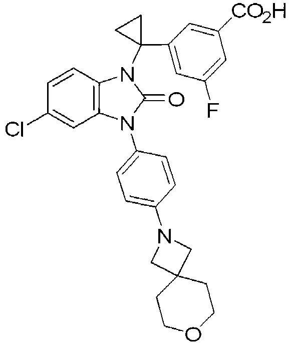

The title compound (80 mg, yield: 56%) was obtained in the same manner as in steps A and B of Example 139 by using 3-(1-(5-chloro-2-oxo-2,3-dihydro-1*H*-benzo[*d*]imidazol-1-yl)cyclopropyl)-5-fluorobenzoic acid ethyl ester (0.10 g, 0.26 mmol) obtained in Preparation Example 59 and 2-(4-bromophenyl)-7-oxa-2-azaspiro[3.5]nonane (78 mg, 0.27 mmol) obtained in Preparation Example 40.
¹H-NMR (400 MHz, DMSO-d6) δ7.52 (m, 1H), 7.49 (m, 1H), 7.30 (3H), 7.23 (d, 1H), 7.14 (dd, 1H), 6.86 (m, 1H), 6.57 (d, 2H), 3.67 (s, 4H), 3.57 (m, 4H), 1.78 (s, 4H), 1.72 (4H)

### Example 194: Preparation of 3-(1-(5-chloro-3-(4-(3,3-difluoroazetidin-1-yl)phenyl)-2-oxo-2,3-dihydro-1H-benzo[d]imidazol-1-yl)cyclopropyl)-5-fluorobenzoic acid

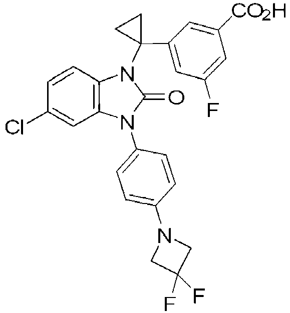

The title compound (90 mg, yield: 67%) was obtained in the same manner as in steps A and B of Example 139 by using 3-(1-(5-chloro-2-oxo-2,3-dihydro-1*H*-benzo[*d*]imidazol-1-yl)cyclopropyl)-5-fluorobenzoic acid ethyl ester (0.10 g, 0.26 mmol) obtained in Preparation Example 59 and 1-(4-bromophenyl)-3,3-difluoroazetidine (70 mg, 0.27 mmol) obtained in Preparation Example 61.
¹H-NMR (400 MHz, CDCl3) δ7.65 (m, 1H), 7.60 (dd, 1H), 7.39 (d, 2H), 7.18 (m, 1H), 7.08 (2H), 7.01 (d, 1H), 6.63 (d, 2H), 4.28 (t, 4H), 1.72 (4H)

### Example 195: Preparation of 3-(1-(3-(4-(7-(tert-butoxycarbonyl)-2,7-diazaspiro[3.5]nonan-2-yl)phenyl)-5-chloro-2-oxo-2,3-dihydro-1H-benzo[d]imidazol-1-yl)cyclopropyl)-5-fluorobenzoic acid

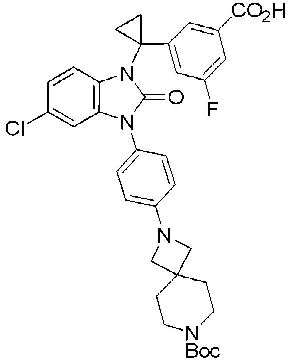

The title compound (70 mg, yield: 77%) was obtained in the same manner as in steps A and B of Example 139 by using 3-(1-(5-chloro-2-oxo-2,3-dihydro-1H-benzo[d]imidazol-1-yl)cyclopropyl)-5-fluorobenzoic acid ethyl ester (0.40 g, 1.1 mmol) obtained in Preparation Example 59 and 2-(4-bromophenyl)-2,7-diazaspiro[3.5]nonane-7-carboxylic acid tert-butyl ester (423 mg, 1.1 mmol) obtained in Preparation Example 63.
¹H-NMR (400 MHz, CDCl3) δ7.64 (m, 1H), 7.58 (m, 1H), 7.30 (d, 2H), 7.19 (m, 1H), 7.06 (2H), 7.00 (m, 1H), 6.54 (d, 2H), 3.69 (s, 4H), 3.42 (m, 4H), 1.80 (6H), 1.69 (2H), 1.47 (s, 9H)

### Example 196: Preparation of 3-(1-(3-(4-(2-azaspiro[3.3]heptan-2-yl)phenyl)-5-chloro-2-oxo-2,3-dihydro-1H-benzo[d]imidazol-1-yl)cyclopropyl)-5-fluorobenzoic acid

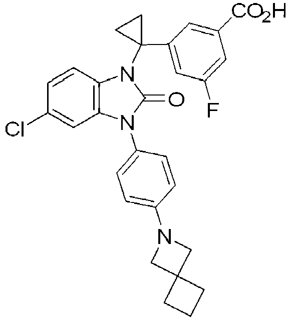

The title compound (90 mg, yield: 67%) was obtained in the same manner as in steps A and B of Example 139 by using 3-(1-(5-chloro-2-oxo-2,3-dihydro-1*H*-benzo[d]imidazol-1-yl)cyclopropyl)-5-fluorobenzoic acid ethyl ester (0.10 g, 0.26 mmol) obtained in Preparation Example 59 and 2-(4-bromophenyl)-2-azaspiro[3.3]heptane (70 mg, 0.27 mmol) obtained in Preparation Example 52.
¹H-NMR (400 MHz, DMSO-d6) δ7.52 (m, 1H), 7.55 (m, 1H), 7.30 (3H), 7.23 (d, 1H), 7.13 (dd, 1H), 6.86 (d, 1H), 6.54 (d, 2H), 3.85 (s, 4H), 2.20 (m, 4H), 1.85 (m, 2H), 1.72 (4H)

### Example 197: Preparation of 3-(1-(5-chloro-3-(4-(4,4-difluoropiperidin-1-yl)phenyl)-2-oxo-2,3-dihydro-1H-benzo[d]imidazol-1-yl)cyclopropyl)-5-fluorobenzoic acid

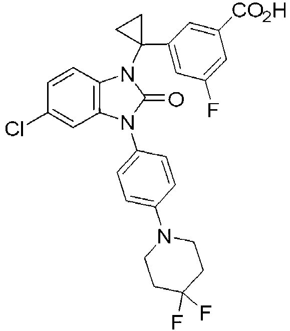

The title compound (100 mg, yield: 71%) was obtained in the same manner as in steps A and B of Example 139 by using 3-(1-(5-chloro-2-oxo-2,3-dihydro-1*H*-benzo[*d*]imidazol-1-yl)cyclopropyl)-5-fluorobenzoic acid ethyl ester (0.10 g, 0.26 mmol) obtained in Preparation Example 59 and 1-(4-bromophenyl)-4,4-difluoropiperidine (77 mg, 0.27 mmol) obtained in Preparation Example 62.
¹H-NMR (400 MHz, DMSO-d6) δ7.52 (m, 1H), 7.50 (m, 1H), 7.39 (d, 2H), 7.32 (m, 1H), 7.24 (d, 1H), 7.15 (3H), 6.94 (m, 1H), 3.45 (m, 4H), 2.08 (m, 4H), 1.72 (4H)

### Example 198: Preparation of 5-((5-chloro-4-fluoro-3-(4-morpholinophenyl)-2-oxo-2,3-dihydro-1H-benzo[d]imidazol-1-yl)methyl)isoxazole-3-carboxylic acid

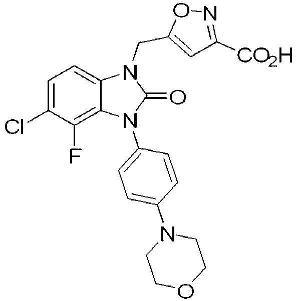

The title compound (70 mg, yield: 51%) was obtained in the same manner as in steps A and B of Example 24 by using 6-chloro-7-fluoro-1-(4-morpholinophenyl)-1,3-dihydro-2H-benzo[d]imidazol-2-one (100 mg, 0.29 mmol) obtained in Preparation Example 53 and 5-(bromomethyl)isoxazole-3-carboxylic acid methyl ester (0.10 g, 0.31 mmol) obtained in Preparation Example 55.
MS [M+H] = 473 (M+1)
¹H-NMR (400 MHz, MeOH-d4) δ 7.35 (m, 2H), 7.25 (dd, 1H), 7.10 (3H), 6.81 (s, 1H), 5.38 (s, 2H), 3.85 (m, 4H), 3.24 (m, 4H)

### Example 199: Preparation of 2-((5-chloro-4-fluoro-3-(4-morpholinophenyl)-2-oxo-2,3-dihydro-1H-benzo[d]imidazol-1-yl)methyl)oxazole-4-carboxylic acid

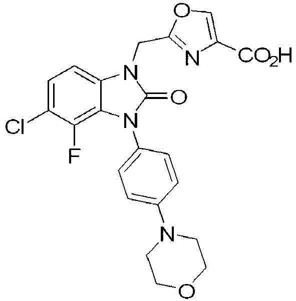

The title compound (70 mg, yield: 51%) was obtained in the same manner as in steps A and B of Example 24 by using 6-chloro-7-fluoro-1-(4-morpholinophenyl)-1,3-dihydro-2H-benzo[d]imidazol-2-one (100 mg, 0.29 mmol) obtained in Preparation Example 53 and 2-(bromomethyl)oxazole-4-carboxylic acid methyl ester (70 mg, 0.31 mmol) obtained in Preparation Example 56.
MS [M+H] = 473 (M+1)
¹H-NMR (400 MHz, MeOH-d4) δ8.52 (s, 1H), 7.38 (m, 2H), 7.21 (m, 1H), 7.12 (d, 2H), 7.06 (m, 1H), 5.36 (s, 2H), 3.87 (m, 4H), 3.25 (m, 4H)

### Example 200: Preparation of 5-((5-chloro-3-(4-morpholinophenyl)-2-oxo-2,3-dihydro-1H-benzo[d]imidazol-1-yl)methyl)isoxazole-3-carboxylic acid

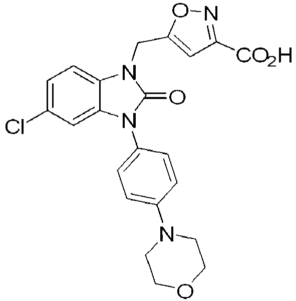

The title compound (35 mg, yield: 25%) was obtained in the same manner as in steps A and B of Example 24 by using 6-chloro-1-(4-morpholinophenyl)-1,3-dihydro-2H-benzo[d]imidazol-2-one (100 mg, 0.30 mmol) obtained Preparation Example 54 and 5-(bromomethyl)isoxazole-3-carboxylic acid methyl ester (0.11 g, 0.33 mmol) obtained in Preparation Example 55.
MS [M+H] = 455 (M+1)
¹H-NMR (500 MHz, MeOH-d4) δ7.38 (m, 2H), 7.27 (m, 1H), 7.17 (3H), 6.96 (m, 1H), 6.73 (m, 1H), 5.37 (s, 2H), 3.86 (m, 4H), 3.24 (m, 4H)

### Example 201: Preparation of 2-((5-chloro-3-(4-morpholinophenyl)-2-oxo-2,3-dihydro-1H-benzo[d]imidazol-1-yl)methyl)oxazole-4-carboxylic acid

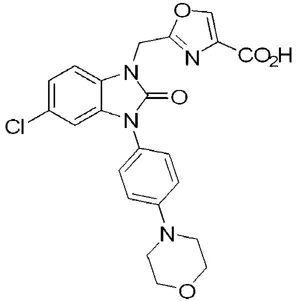

The title compound (30 mg, yield: 18%) was obtained in the same manner as in steps A and B of Example 24 by using 6-chloro-1-(4-morpholinophenyl)-1,3-dihydro-2H-benzo[d]imidazol-2-one (100 mg, 0.30 mmol) obtained Preparation Example 54 and 2-(bromomethyl)oxazole-4-carboxylic acid methyl ester (73 mg, 0.33 mmol) obtained in Preparation Example 56.
MS [M+H] = 455 (M+1)
¹H-NMR (400 MHz, CDCl3) δ8.27 (s, 1H), 7.38 (d, 2H), 7.00 (5H), 5.33 (s, 2H), 3.90 (m, 4H), 3.24 (m, 4H)

### Example 202: Preparation of 5-((5-chloro-4-fluoro-3-(4-morpholinophenyl)-2-oxo-2,3-dihydro-1H-benzo[d]imidazol-1-yl)methyl)-2,3-difluorobenzoic acid

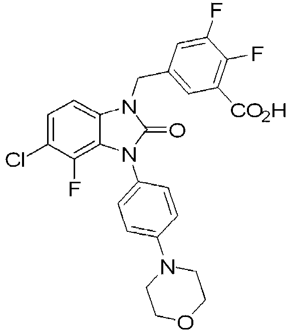

The title compound (104 mg, yield: 70%) was obtained in the same manner as in steps A and B of Example 24 by using 6-chloro-7-fluoro-1-(4-morpholinophenyl)-1,3-dihydro-2H-benzo[d]imidazol-2-one (100 mg, 0.29 mmol) obtained in Preparation Example 53 and 5-(bromomethyl)-2,3-difluorobenzoic acid ethyl ester (88 mg, 0.31 mmol) obtained in Preparation Example 57.
MS [M+H] = 518 (M+1)
¹H-NMR (500 MHz, DMSO-d6) δ13.70 (brs, 1H), 7.72 (2H), 7.39 (d, 2H), 7.26 (m, 1H), 7.14 (m, 1H), 7.05 (d, 2H), 5.16 (s, 2H), 3.77 (m, 4H), 3.19 (m, 4H)

### Example 203: Preparation of 5-((5-chloro-3-(4-morpholinophenyl)-2-oxo-2,3-dihydro-1H-benzo[d]imidazol-1-yl)methyl)-2,3-difluorobenzoic acid

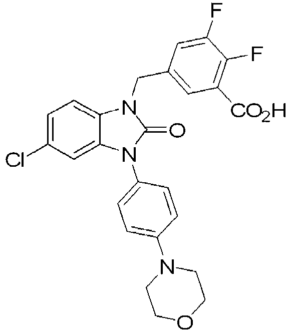

The title compound (100 mg, yield: 66%) was obtained in the same manner as in steps A and B of Example 24 by using 6-chloro-1-(4-morpholinophenyl)-1,3-dihydro-2H-benzo[d]imidazol-2-one (100 mg, 0.30 mmol) obtained in Preparation Example 54 and 5-(bromomethyl)-2,3-difluorobenzoic acid ethyl ester (85 mg, 0.30 mmol) obtained in Preparation Example 57.
MS [M+H] = 500 (M+1)
¹H-NMR (500 MHz, DMSO-d6) 513.69 (brs, 1H), 7.71 (2H), 7.40 (d, 2H), 7.27 (m, 1H), 7.14 (3H), 6.91 (d, 1H), 5.16 (s, 2H), 3.77 (m, 4H), 3.20 (m, 4H)

### Example 204: Preparation of 3-((5-chloro-4-fluoro-3-(4-morpholinophenyl)-2-oxo-2,3-dihydro-1H-benzo[d]imidazol-1-yl)methyl)-2,6-difluorobenzoic acid

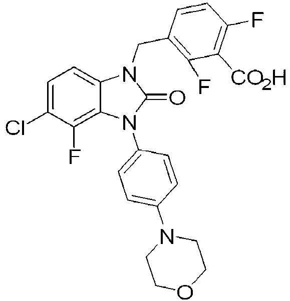

The title compound (70 mg, yield: 47%) was obtained in the same manner as in steps A and B of Example 24 by using 6-chloro-7-fluoro-1-(4-morpholinophenyl)-1,3-dihydro-2H-benzo[d]imidazol-2-one (100 mg, 0.29 mmol) obtained in Preparation Example 53 and 3-(bromomethyl)-2,6-difluorobenzoic acid ethyl ester (88 mg, 0.31 mmol) obtained in Preparation Example 58.
¹H-NMR (400 MHz, DMSO-d6) δ7.52 (m, 1H), 7.37 (d, 2H), 7.27 (dd, 1H), 7.19 (t, 1H), 7.10 (d, 1H), 7.04 (d, 2H), 5.18 (s, 2H), 3.76 (m, 4H), 3.19 (m, 4H)

### Example 205: Preparation of 3-((5-chloro-3-(4-morpholinophenyl)-2-oxo-2,3-dihydro-1H-benzo[d]imidazol-1-yl)methyl)-2,6-difluorobenzoic acid

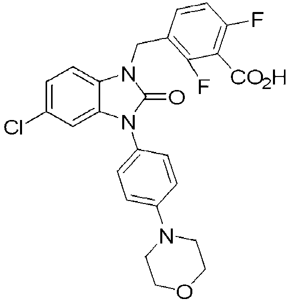

The title compound (90 mg, yield: 59%) was obtained in the same manner as in steps A and B of Example 24 by using 6-chloro-1-(4-morpholinophenyl)-1,3-dihydro-2*H-*benzo[*d*]imidazol-2-one (100 mg, 0.30 mmol) obtained in Preparation Example 54 and 3-(bromomethyl)-2,6-difluorobenzoic acid ethyl ester (93 mg, 0.33 mmol) obtained in Preparation Example 58.
¹H-NMR (400 MHz, DMSO-d6) δ7.51 (m, 1H), 7.38 (d, 2H), 7.21 (2H), 7.16 (dd, 1H), 7.10 (d, 2H), 6.91 (dd, 1H), 5.17 (s, 2H), 3.77 (m, 4H), 3.20 (m, 4H)

### Example 206: Preparation of 3-(1-(3-(4-(dimethylamino)phenyl)-2-oxo-5-(trifluoromethyl)-2,3-dihydro-1H-benzo[d]imidazol-1-yl)cyclopropyl)benzoic acid

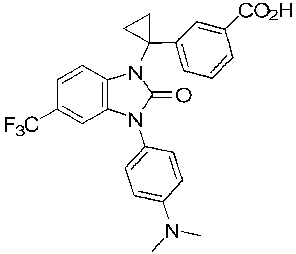

The process of the following steps A and B gave the title compound.

### Step A: Preparation of 3-(1-(3-(4-(dimethylamino)phenyl)-2-oxo-5-(trifluoromethyl)-2,3-dihydro-1H-benzo[d]imidazol-1-yl)cyclopropyl)benzoic acid methyl ester

The title compound (55 mg, yield: 60%) was obtained in the same manner as in step A of Example 1 by using 3-(1-(2-oxo-5-(trifluoromethyl)-2,3-dihydro-1*H*-benzo[d]imidazol-1-yl)cyclopropyl)benzoic acid methyl ester (70 mg, 0.18 mmol) obtained in Preparation Example 49 and (4-(dimethylamino)phenyl)boronic acid (61 mg, 0.372 mmol).
¹H-NMR (400 MHz, CDCl3) δ7.91 (m, 1H), 7.89 (m, 1H), 7.53 (dt, 1H), 7.35 (4H), 7.27 (d, 1H), 7.21 (d, 1H), 6.83 (m, 2H), 3.90 (s, 3H), 3.02 (s, 6H), 1.70 (4H)

### Step B: Preparation of 3-(1-(3-(4-(dimethylamino)phenyl)-2-oxo-5-(trifluoromethyl)-2,3-dihydro-1H-benzo[d]imidazol-1-yl)cyclopropyl)benzoic acid

The title compound (27 mg, yield: 50%) was obtained in the same manner as in step B of Example 24 by using 3-(1-(3-(4-(dimethylamino)phenyl)-2-oxo-5-(trifluoromethyl)-2,3-dihydro-1H-benzo[d]imidazol-1-yl)cyclopropyl)benzoic acid methyl ester (55 mg, 0.11 mmol) obtained in step A above.
MS [M+H] = 482 (M+1)
¹H-NMR (400 MHz, CDCl3) δ7.95 (m, 1H), 7.93 (m, 1H), 7.57 (dt, 1H), 7.40 (t, 1H), 7.36 (m, 1H), 7.33 (m, 2H), 7.28 (m, 1H), 7.22 (d, 1H), 6.84 (m, 2H), 2.99 (s, 6H), 1.72 (4H)

### Example 207: Preparation of 3-(1-(3-(4-morpholinophenyl)-2-oxo-5-(trifluoromethyl)-2,3-dihydro-1H-benzo[d]imidazol-1-yl)cyclopropyl)benzoic acid

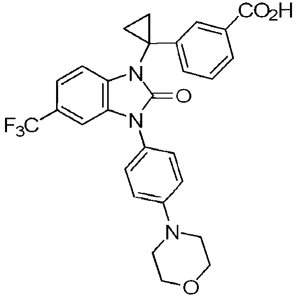

The process of the following steps A and B gave the title compound.

### Step A: Preparation of 3-(1-(3-(4-morpholinophenyl)-2-oxo-5-(trifluoromethyl)-2,3-dihydro-1H-benzo[d]imidazol-1-yl)cyclopropyl)benzoic acid methyl ester

The title compound (60 mg, yield: 60%) was obtained in the same manner as in step A of Example 1 by using 3-(1-(2-oxo-5-(trifluoromethyl)-2,3-dihydro-1*H*-benzo[*d*]imidazol-1-yl)cyclopropyl)benzoic acid methyl ester (70 mg, 0.18 mmol) obtained in Preparation Example 49 and (4-morpholinophenyl)boronic acid (77 mg, 0.37 mmol).
¹H-NMR (400 MHz, CDCl3) δ7.91 (m, 1H), 7.90 (m, 1H), 7.53 (dt, 1H), 7.38 (4H), 7.28 (dd, 1H), 7.23 (d, 1H), 7.05 (m, 2H), 3.90 (s, 3H), 3.88 (m, 4H), 3.24 (m, 4H), 1.71 (4H)

### Step B: Preparation of 3-(1-(3-(4-morpholinophenyl)-2-oxo-5-(trifluoromethyl)-2,3-dihydro-1H-benzo[d]imidazol-1-yl)cyclopropyl)benzoic acid

The title compound (35 mg, yield: 60%) was obtained in the same manner as in step B of Example 24 by using 3-(1-(3-(4-morpholinophenyl)-2-oxo-5-(trifluoromethyl)-2,3-dihydro-1H-benzo[d]imidazol-1-yl)cyclopropyl)benzoic acid methyl ester (60 mg, 0.11 mmol) obtained in step A above.
MS [M+H] = 524 (M+1)
¹H-NMR (400 MHz, CDCl3) δ7.96 (m, 1H), 7.95 (m, 1H), 7.56 (m, 1H), 7.41 (4H), 7.28 (m, 1H), 7.24 (m, 1H), 7.00 (m, 2H), 3.90 (m, 4H), 3.23 (m, 4H), 1.73 (4H)

### Example 208: Preparation of 3-(1-(3-(4-(dimethylamino)phenyl)-2-oxo-5-(trifluoromethoxy)-2,3-dihydro-1H-benzo[d]imidazol-1-yl)cyclopropyl)benzoic acid

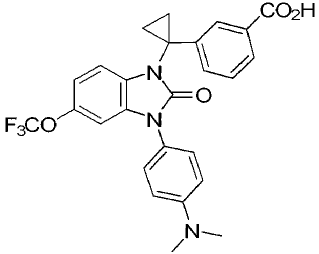

The process of the following steps A and B gave the title compound.

### Step A: Preparation of 3-(1-(3-(4-(dimethylamino)phenyl)-2-oxo-5-(trifluoromethoxy)-2,3-dihydro-1H-benzo[d]imidazol-1-yl)cyclopropyl)benzoic acid methyl ester

The title compound (66 mg, yield: 63%) was obtained in the same manner as in step A of Example 1 by using 3-(1-(2-oxo-5-(trifluoromethoxy)-2,3-dihydro-1*H*-benzo[*d*]imidazol-1-yl)cyclopropyl)benzoic acid methyl ester (80 mg, 0.20 mmol) obtained in Preparation Example 50 and (4-(dimethylamino)phenyl)boronic acid (67 mg, 0.40 mmol).
¹H-NMR (400 MHz, CDCl3) δ7.91 (m, 1H), 7.89 (m, 1H), 7.54 (dt, 1H), 7.36 (t, 1H), 7.32 (m, 2H), 7.15 (d, 1H), 6.96 (m, 1H), 6.89 (m, 1H), 6.82 (m, 2H), 3.90 (s, 3H), 3.02 (s, 6H), 1.70 (4H)

### Step B: Preparation of 3-(1-(3-(4-(dimethylamino)phenyl)-2-oxo-5-(trifluoromethoxy)-2,3-dihydro-1H-benzo[d]imidazol-1-yl)cyclopropyl)benzoic acid

The title compound (37 mg, yield: 57%) was obtained in the same manner as in step B of Example 24 by using 3-(1-(3-(4-(dimethylamino)phenyl)-2-oxo-5-(trifluoromethoxy)-2,3-dihydro-1H-benzo[d]imidazol-1-yl)cyclopropyl)benzoic acid methyl ester (66 mg, 0.13 mmol) obtained in step A above.
MS [M+H] = 498 (M+1)
¹H-NMR (400 MHz, CDCl3) δ7.95 (m, 1H), 7.93 (m, 1H), 7.59 (dt, 1H), 7.40 (t, 1H), 7.33 (m, 2H), 7.16 (d, 1H), 6.96 (m, 1H), 6.90 (m, 1H), 6.83 (m, 2H), 3.02 (s, 6H), 1.70 (4H)

### Example 209: Preparation of 3-(1-(3-(4-morpholinophenyl)-2-oxo-5-(trifluoromethoxy)-2,3-dihydro-1H-benzo[d]imidazol-1-yl)cyclopropyl)benzoic acid

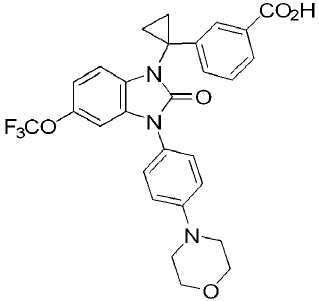

The process of the following steps A and B gave the title compound.

### Step A: Preparation of 3-(1-(3-(4-morpholinophenyl)-2-oxo-5-(trifluoromethoxy)-2,3-dihydro-1H-benzo[d]imidazol-1-yl)cyclopropyl)benzoic acid methyl ester

The title compound (66 mg, yield: 63%) was obtained in the same manner as in step A of Example 1 by using 3-(1-(2-oxo-5-(trifluoromethoxy)-2,3-dihydro-1*H*-benzo[*d*]imidazol-1-yl)cyclopropyl)benzoic acid methyl ester (80 mg, 0.20 mmol) obtained in Preparation Example 50 and (4-morpholinophenyl)boronic acid (84 mg, 0.40 mmol).
¹H-NMR (400 MHz, CDCl3) δ7.91 (m, 1H), 7.89 (m, 1H), 7.54 (dt, 1H), 7.40 (m, 2H), 7.36 (t, 1H), 7.17 (d, 1H), 7.02 (m, 2H), 6.97 (dd, 1H), 6.90 (d, 1H), 3.90 (s, 3H), 3.87 (m, 4H), 3.23 (m, 4H), 1.70 (4H)

### Step B: Preparation of 3-(1-(3-(4-morpholinophenyl)-2-oxo-5-(trifluoromethoxy)-2,3-dihydro-1H-benzo[d]imidazol-1-yl)cyclopropyl)benzoic acid

The title compound (40 mg, yield: 59%) was obtained in the same manner as in step B of Example 24 by using 3-(1-(3-(4-morpholinophenyl)-2-oxo-5-(trifluoromethoxy)-2,3-dihydro-1H-benzo[d]imidazol-1-yl)cyclopropyl)benzoic acid methyl ester (70 mg, 0.12 mmol) obtained in step A above.
MS [M+H] = 540 (M+1)
¹H-NMR (400 MHz, CDCl3) δ7.96 (m, 1H), 7.94 (m, 1H), 7.58 (dt, 1H), 7.40 (3H), 7.18 (d, 1H), 7.04 (m, 2H), 6.98 (m, 1H), 6.92 (m, 1H), 3.89 (m, 4H), 3.23 (m, 4H), 1.70 (4H)

### Example 210: Preparation of 3-(1-(5-chloro-3-(4-(N,N-dimethylsulfamoyl)phenyl)-2-oxo-2,3-dihydro-1H-benzo[d]imidazol-1-yl)cyclopropyl)benzoic acid

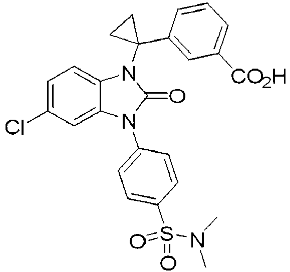

The title compound (44 mg, yield: 30%) was obtained in the same manner as in steps A and B of Example 139 by using 3-(1-(5-chloro-2-oxo-2,3-dihydro-1*H*-benzo[*d*]imidazol-1-yl)cyclopropyl)benzoic acid methyl ester (247 mg, 0.72 mmol) obtained in Preparation Example 3 and 4-bromo-N,N-dimethylbenzenesulfonamide (190 mg, 0.72 mmol) obtained in Preparation Example 70.
¹H-NMR (400 MHz, CDCl3) δ1.95 (m, 4H), 7.77 (d, 2H), 7.53 (d, 1H), 7.40 (t, 1H), 7.15 (m, 3H), 2.77 (s, 6H), 1.73 (m, 2H), 1.70 (m, 2H)

### Example 211: Preparation of 3- (1- (5-chloro-3- (4- (N-cyclopropylsulfamoyl)phenyl)-2-oxo-2,3-dihydro-1H-benzo[d]imidazol-1-yl)cyclopropyl)benzoic acid

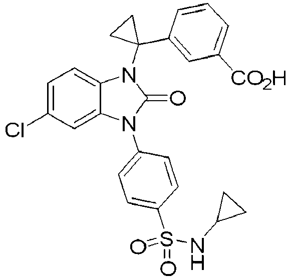

The title compound (157 mg, yield: 90%) was obtained in the same manner as in steps A and B of Example 139 by using 3-(1-(5-chloro-2-oxo-2,3-dihydro-1H-benzo[d]imidazol-1-yl)cyclopropyl)benzoic acid methyl ester (298 mg, 0.87 mmol) obtained in Preparation Example 3 and 4-bromo-N-cyclopropylbenzenesulfonamide (240 mg, 0.87 mmol) obtained in Preparation Example 71.
¹H-NMR (400 MHz, CDCl3) δ8.09 (d, 2H), 7.95 (d, 1H), 7.92 (s, 1H), 7.76 (d, 2H), 7.50 (d, 1H), 7.41 (t, 1H), 7.16 (m, 3H), 5.10 (brs, 1H), 2.29 (m, 1H), 1.73 (m, 2H), 1.69 (m, 2H), 0.64 (m, 4H)

### Example 212: Preparation of 3-(1-(5-chloro-3-(4-(morpholinosulfonyl)phenyl)-2-oxo-2,3-dihydro-1H-benzo[d]imidazol-1-yl)cyclopropyl)benzoic acid

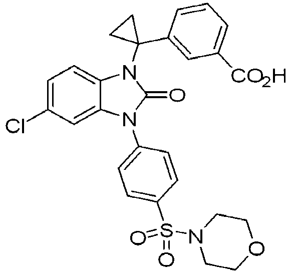

The title compound (114 mg, yield: 63%) was obtained in the same manner as in steps A and B of Example 139 by using 3-(1-(5-chloro-2-oxo-2,3-dihydro-1*H*-benzo[d]imidazol-1-yl)cyclopropyl)benzoic acid methyl ester (280 mg, 0.82 mmol) obtained in Preparation Example 3 and 4-((4-bromophenyl)sulfonyl)morpholine (250 mg, 0.82 mmol) obtained in Preparation Example 72.
¹H-NMR (400 MHz, CDCl3) δ7.93 (m, 4H), 7.80 (d, 2H), 7.52 (d, 1H), 7.40 (t, 1H), 7.18 (m, 3H), 3.76 (m, 4H), 3.07 (m, 4H), 1.73 (m, 2H), 1.70 (m, 2H)

### Example 213: Preparation of 3-(1-(5-chloro-3-(4-(N-(cyclobutylmethyl)sulfamoyl)phenyl)-2-oxo-2,3-dihydro-1H-benzo[d]imidazol-1-yl)cyclopropyl)benzoic acid

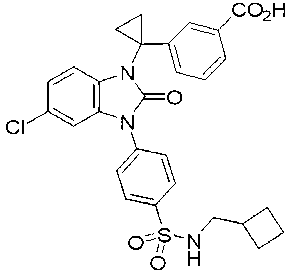

The title compound (22 mg, yield: 8.7%) was obtained in the same manner as in steps A and B of Example 139 by using 3-(1-(5-chloro-2-oxo-2,3-dihydro-1*H*-benzo[*d*]imidazol-1-yl)cyclopropyl)benzoic acid methyl ester (156 mg, 0.45 mmol) obtained in Preparation Example 3 and 4-bromo-N-(cyclobutylmethyl)benzenesulfonamide (138 mg, 0.45 mmol) obtained in Preparation Example 73.
¹H-NMR (500 MHz, CDCl3) δ8.06 (d, 2H), 7.99 (d, 1H), 7.95 (s, 1H), 7.78 (d, 2H), 7.54 (d, 1H), 7.44 (t, 1H), 7.19 (m, 3H), 4.66 (t, 1H), 3.05 (t, 2H), 2.48 (m, 1H), 2.06 (m, 2H), 1.91 (m, 2H), 1.77 (m, 2H), 1.73 (m, 2H), 1.69 (m, 2H)

### Example 214: Preparation of 3-(1-(5-chloro-2-oxo-3-(4-(piperidin-1-ylsulfonylphenyl)-2,3-dihydro-1H-benzo[d]imidazol-1-yl)cyclopropyl)benzoic acid

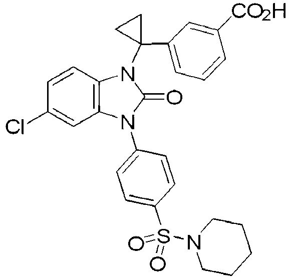

The title compound (32 mg, yield: 12.2%) was obtained in the same manner as in steps A and B of Example 139 by using 3-(1-(5-chloro-2-oxo-2,3-dihydro-1H-benzo[d]imidazol-1-yl)cyclopropyl)benzoic acid methyl ester (163 mg, 0.48 mmol) obtained in Preparation Example 3 and 1-((4-bromophenyl)sulfonyl)piperidine (145 mg, 0.48 mmol) obtained in Preparation Example 74.
¹H-NMR (500 MHz, CDCl3) δ7.96 (d, 1H), 7.95 (m, 3H), 7.79 (d, 2H), 7.57 (d, 1H), 7.44 (t, 1H), 7.19 (m, 3H), 3.07 (m, 4H), 1.77 (m, 2H), 1.73 (m, 2H), 1.70 (m, 4H), 1.49 (m, 2H)

### Example 215: Preparation of 3-(1-(5-chloro-2-oxo-3-(4-(pyrrolidin-1-ylsulfonyl)phenyl)-2,3-dihydro-1H-benzo[d]imidazol-1-yl)cyclopropyl)benzoic acid

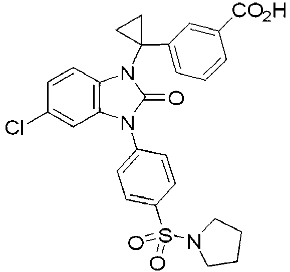

The title compound (15.5 mg, yield: 8.3%) was obtained in the same manner as in steps A and B of Example 139 by using 3-(1-(5-chloro-2-oxo-2,3-dihydro-1H-benzo[d]imidazol-1-yl)cyclopropyl)benzoic acid methyl ester (118 mg, 0.35 mmol) obtained in Preparation Example 3 and 1-((4-bromophenyl)sulfonyl)pyrrolidine (100 mg, 0.35 mmol) obtained in Preparation Example 75.
¹H-NMR (500 MHz, CDCl3) δ8.06 (d, 2H), 7.99 (d, 1H), 7.95 (s, 1H), 7.79 (d, 2H), 7.56 (d, 1H), 7.43 (t, 1H), 7.18 (m, 3H), 3.32 (m, 4H), 1.85 (m, 4H), 1.77 (m, 2H), 1.73 (m, 2H)

### Example 216: Preparation of 3-(1-(5-chloro-3-(4-(2-hydroxy-2-methylpropyl)phenyl)-2-oxo-2,3-dihydro-1H-benzo[d]imidazol-1-yl)cyclopropyl)benzoic acid

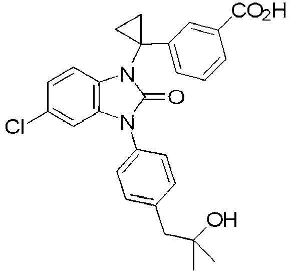

The title compound (80 mg, yield: 57%) was obtained in the same manner as in steps A and B of Example 139 by using 3-(1-(5-chloro-2-oxo-2,3-dihydro-1H-benzo[d]imidazol-1-yl)cyclopropyl)benzoic acid methyl ester (100 mg, 0.29 mmol) obtained in Preparation Example 3 and 1-(4-bromophenyl)-2-methylpropan-2-ol (67 mg, 0.29 mmol).
MS [M+H] = 477 (M+1)
¹H-NMR (400 MHz, DMSO-d₆) δ7.80 (m, 1H), 7.68 (d, 1H), 7.45 (6H), 7.26 (m, 1H), 7.18 (dd, 1H), 7.02 (d, 1H), 7.42 (s, 1H), 2.84 (s, 2H), 1.68 (4H), 1.12 (s, 6H)

### Example 217: Preparation of 3-(1-(5-chloro-3-(4-(2-fluoro-2-methylpropyl)phenyl)-2-oxo-2,3-dihydro-1H-benzo[d]imidazol-1-yl)cyclopropyl)benzoic acid

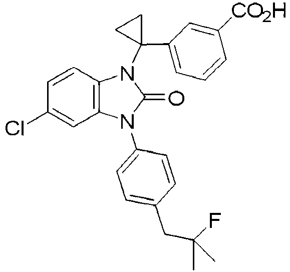

The title compound (80 mg, yield: 57%) was obtained in the same manner as in steps A and B of Example 139 by using 3-(1-(5-chloro-2-oxo-2,3-dihydro-1*H*-benzo[*d*]imidazol-1-yl)cyclopropyl)benzoic acid methyl ester (100 mg, 0.29 mmol) obtained in Preparation Example 3 and 1-bromo-4-(2-fluoro-2-methylpropyl)benzene (67 mg, 0.29 mmol).
MS [M+H] = 479 (M+1)
¹H-NMR (400 MHz, DMSO-d₆) δ7.82 (m, 1H), 7.70 (d, 1H), 7.51 (m, 2H), 7.45 (4H), 7.26 (d, 1H), 7.18 (dd, 1H), 7.05 (d, 1H), 2.99 (d, 2H, J=21.6 Hz), 1.68 (4H), 1.34 (d, 6H, J=21.6 Hz)

### Example 218: Preparation of 3-(1-(5-chloro-3-(4-(1-hydroxy-2-methylpropan-2-yl)phenyl)-2-oxo-2,3-dihydro-1H-benzo[d]imidazol-1-yl)cyclopropyl)benzoic acid

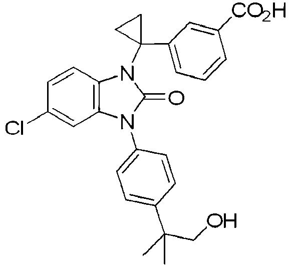

The title compound (25 mg, yield: 18%) was obtained in the same manner as in steps A and B of Example 139 by using 3-(1-(5-chloro-2-oxo-2,3-dihydro-1*H*-benzo[*d*]imidazol-1-yl)cyclopropyl)benzoic acid methyl ester (100 mg, 0.29 mmol) obtained in Preparation Example 3 and 2-(4-bromophenyl)-2-methylpropan-1-ol (67 mg, 0.29 mmol).
MS [M+H] = 477 (M+1)
¹H-NMR (400 MHz, CDCl₃) δ7.95 (2H), 7.56 (3H), 7.51 (m, 2H), 7.40 (m, 1H), 7.12 (3H), 3.67 (s, 2H), 1.72 (4H), 1.39 (s, 6H)

### Example 219: Preparation of 3-(1-(5-chloro-3-(4-(1-fluoro-2-methylpropan-2-yl)phenyl)-2-oxo-2,3-dihydro-1H-benzo[d]imidazol-1-yl)cyclopropyl)benzoic acid

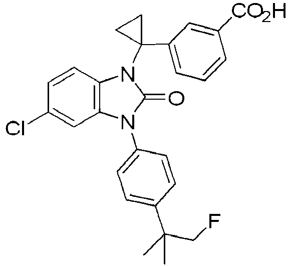

The title compound (80 mg, yield: 57%) was obtained in the same manner as in steps A and B of Example 139 by using 3-(1-(5-chloro-2-oxo-2,3-dihydro-1*H*-benzo[d]imidazol-1-yl)cyclopropyl)benzoic acid methyl ester (100 mg, 0.29 mmol) obtained in Preparation Example 3 and 1-bromo-4-(1-fluoro-2-methylpropan-2-yl)benzene (67 mg, 0.29 mmol).
MS [M+H] = 479 (M+1)
¹H-NMR (400 MHz, CDCl₃) δ7.95 (2H), 7.54 (m, 1H), 7.46 (m, 2H), 7.38 (3H), 7.13 (m, 1H), 7.10 (m, 2H), 2.97 (d, 2H, J=20.8 Hz), 1.72 (4H), 1.38 (d, 6H, J=21.6 Hz)

### Example 220: Preparation of 3-(1-(6-chloro-2-oxo-1-(4-(tetrahydro-2H-pyran-4-yl)phenyl)-1,2-dihydro-3H-imidazo[4,5-b]pyridin-3-yl)cyclopropyl)benzoic acid

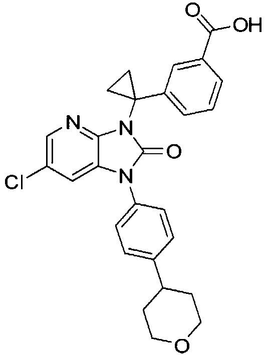

The title compound (44 mg, yield: 62%) was obtained in the same manner as in steps A and B of Example 1 by using 3-(1-(6-chloro-2-oxo-1,2-dihydro-3H-imidazo[4,5-b]pyridin-3-yl)cyclopropyl)benzoic acid methyl ester (50 mg, 0.14 mmol) obtained in Preparation Example 76 and (4-(tetrahydro-2H-pyran-4-yl)phenyl)boronic acid (60 mg, 0.29 mmol).
¹H-NMR (500 MHz, DMSO-d6) δ 8.08 (d, 1H), 7.75 (d, 1H), 7.71 (s, 1H), 7.42 (m, 7H), 3.93 (m, 2H), 2.83 (m, 1H), 1.65 (m, 8H)

### Example 221: Preparation of 3-(1-(6-chloro-1-(4-(dimethylamino)phenyl)-2-oxo-1,2-dihydro-3H-imidazo[4,5-b]pyridin-3-yl)cyclopropyl)benzoic acid

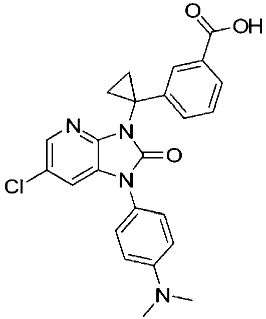

The title compound (42 mg, yield: 65%) was obtained in the same manner as in steps A and B of Example 1 by using 3-(1-(6-chloro-2-oxo-1,2-dihydro-3*H*-imidazo[4,5-b]pyridin-3-yl)cyclopropyl)benzoic acid methyl ester (50 mg, 0.14 mmol) obtained in Preparation Example 76 and (4-(dimethylamino)phenyl)boronic acid (48 mg, 0.29 mmol).
¹H-NMR (400 MHz, DMSO-d6) δ8.04 (d, 1H), 7.75 (d, 1H), 7.70 (s, 1H), 7.41 (m, 2H), 7.29 (d, 2H), 7.26 (d, 1H), 6.81 (d, 2H), 2.92 (s, 6H), 1.63 (m, 4H)

### Example 222: Preparation of 3-(1-(6-chloro-1-(4-morpholinophenyl)-2-oxo-1,2-dihydro-3H-imidazo[4,5-b]pyridin-3-yl)cyclopropyl)benzoic acid

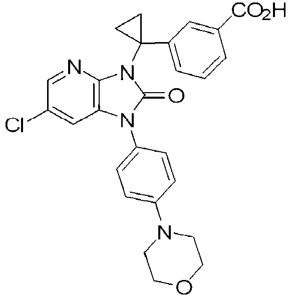

The title compound (23 mg, yield: 40%) was obtained in the same manner as in steps A and B of Example 1 by using 3-(1-(6-chloro-2-oxo-1,2-dihydro-3H-imidazo[4,5-b]pyridin-3-yl)cyclopropyl)benzoic acid methyl ester (40 mg, 0.12 mmol) obtained in Preparation Example 76 and (4-morpholinophenyl)boronic acid (48 mg, 0.23 mmol).
¹H-NMR (500 MHz, DMSO-d6) δ8.10 (s, 1H), 7.80 (d, 1H), 7.76 (s, 1H), 7.46 (m, 4H), 7.36 (s, 1H), 7.11 (d, 2H), 3.76 (s, 4H), 3.19 (s, 4H), 1.70 (m, 2H), 1.62 (m, 2H)

### Example 223: Preparation of 3-(1-(6-chloro-1-(4-(dimethylcarbamoyl)phenyl)-2-oxo-1,2-dihydro-3H-imidazo[4,5-b]pyridin-3-yl)cyclopropyl)benzoic acid

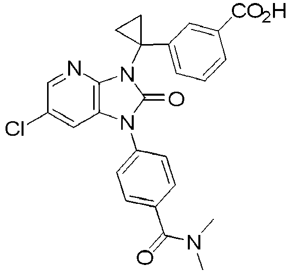

The title compound (12 mg, yield: 22%) was obtained in the same manner as in steps A and B of Example 1 by using 3-(1-(6-chloro-2-oxo-1,2-dihydro-3*H*-imidazo[4,5-b]pyridin-3-yl)cyclopropyl)benzoic acid methyl ester (40 mg, 0.12 mmol) obtained in Preparation Example 76 and (4-(dimethylcarbamoyl)phenyl)boronic acid (45 mg, 0.23 mmol).
¹H-NMR (500 MHz, DMSO-d6) δ8.15 (s, 1H), 7.79 (m, 2H), 7.68 (m, 2H), 7.61 (m, 3H), 7.54 (m, 1H), 7.44 (m, 1H), 3.00 (d, 6H), 1.72 (m, 2H), 1.64 (m, 2H)

### Example 224: Preparation of 3-(1-(6-chloro-1-(4-(3-hydroxyazetidine-1-carbonyl)phenyl)-2-oxo-1,2-dihydro-3H-imidazo[4,5-b]pyridin-3-yl)cyclopropyl)benzoic acid

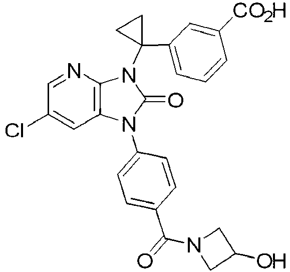

The title compound was obtained in the same manner as in Example 56 by using 3-(1-(6-chloro-2-oxo-1,2-dihydro-3H-imidazo[4,5-b]pyridin-3-yl)cyclopropyl)benzoic acid methyl ester obtained in Preparation Example 76 and 3-hydroxyazetidine hydrochloride.
¹H-NMR (500 MHz, DMSO-d6) δ 8.15 (d, 1H), 8.80 (m, 4H), 7.70 (d, 2H), 7.63 (s, 1H), 7.49 (m, 1H), 7.41 (t, 1H), 4.53 (m, 2H), 4.28 (m, 1H), 4.10 (m, 1H), 3.82 (m, 1H), 1.71 (m, 2H), 1.63 (m, 2H)

### Example 225: Preparation of 3-(1-(1-(4-(7-oxa-2-azaspiro[3.5]nonane-2-carbonyl)phenyl)-6-chloro-2-oxo-1,2-dihydro-3H-imidazo[4,5-b]pyridin-3-yl)cyclopropyl)benzoic acid

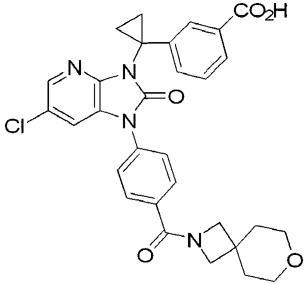

The title compound was obtained in the same manner as in Example 56 by using 3-(1-(6-chloro-2-oxo-1,2-dihydro-3*H-*imidazo[4,5-*b*]pyridin-3-yl)cyclopropyl)benzoic acid methyl ester obtained in Preparation Example 76 and 7-oxa-2-azaspiro[3.5]nonane.
¹H-NMR (500 MHz, DMSO-d6) δ 8.15 (d, 1H), 7.84 (m, 2H), 7.90 (m, 2H), 7.70 (m, 2H), 7.61 (d, 1H), 7.52 (m, 1H), 7.42 (t, 1H), 4.12 (m, 4H), 3.83 (m, 4H), 1.73 (m, 6H), 1.64 (m, 2H)

### Example 226: Preparation of 3-(1-(6-chloro-1-(4-(2-(dimethylamino)-2-oxoethyl)phenyl)-2-oxo-1,2-dihydro-3H-imidazo[4,5-b]pyridin-3-yl)cyclopropyl)benzoic acid

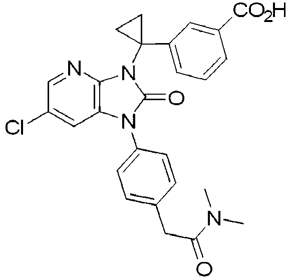

The title compound (41 mg, yield: 43%) was obtained in the same manner as in steps A and B of Example 139 by using 3-(1-(6-chloro-2-oxo-1,2-dihydro-3H-imidazo[4,5-b]pyridin-3-yl)cyclopropyl)benzoic acid methyl ester (67 mg, 0.20 mmol) obtained in Preparation Example 76 and 2-(4-bromophenyl)-N,N-dimethylacetamide (52 mg, 0.21 mmol).
¹H-NMR (500 MHz, CDCl₃) δ8.26 (m, 1H), 8.17 (m, 1H), 7.97 (m, 1H), 7.79 (m, 1H), 7.46 (m, 4H), 7.40 (m, 1H), 7.32 (m, 1H), 3.80 (s, 2H), 3.49 (d, 6H), 1.73 (m, 4H)

### Example 227: Preparation of 3-(1-(6-chloro-1-(4-(methylsulfonyl)phenyl)-2-oxo-1,2-dihydro-3H-imidazo[4,5-b]pyridin-3-yl)cyclopropyl)benzoic acid

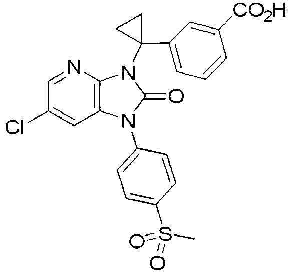

The title compound (14 mg, yield: 20%) was obtained in the same manner as in steps A and B of Example 1 by using 3-(1-(6-chloro-2-oxo-1,2-dihydro-3*H*-imidazo[4,5-b]pyridin-3-yl)cyclopropyl)benzoic acid methyl ester (50 mg, 0.15 mmol) obtained in Preparation Example 76 and (4-(methylsulfonyl)phenyl)boronic acid (58 mg, 0.29 mmol).
¹H-NMR (500 MHz, CDCl₃) δ8.28 (s, 1H), 8.23 (s, 1H), 8.15 (m, 2H), 7.99 (m, 1H), 7.81 (m, 3H), 7.43 (m, 2H), 3.13 (s, 3H), 1.75 (d, 4H)

### Example 228: Preparation of 3-(1-(6-chloro-2-oxo-1-(4-(tetrahydro-2H-pyran-4-yl)phenyl)-1,2-dihydro-3H-imidazo[4,5-b]pyridin-3-yl)cyclopropyl)-2-fluorobenzoic acid

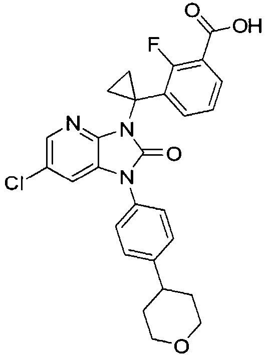

The title compound (33 mg, yield: 74%) was obtained in the same manner as in steps A and B of Example 1 by using 3-(1-(6-chloro-2-oxo-1,2-dihydro-3H-imidazo[4,5-b]pyridin-3-yl)cyclopropyl)-2-fluorobenzoic acid methyl ester (32 mg, 0.09 mmol) obtained in Preparation Example 77 and (4-(tetrahydro-2H-pyran-4-yl)boronic acid (37 mg, 0.18 mmol).
¹H-NMR (400 MHz, MeOD-d4) δ8.07 (d, 1H), 7.95 (dt, 1H), 7.78 (dt, 1H), 7.43 (m, 4H), 7.28 (d, 1H), 7.18 (t, 1H), 4.04 (m, 2H), 3.56 (m, 2H), 2.87 (m, 1H), 1.80 (m, 6H), 1.64 (m, 2H)

### Example 229: Preparation of 3-(1-(6-chloro-1-(4-(dimethylamino)phenyl)-2-oxo-1,2-dihydro-3H-imidazo[4,5-b]pyridin-3-yl)cyclopropyl)-2-fluorobenzoic acid

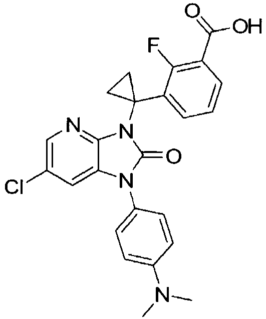

The title compound (11 mg, yield: 27%) was obtained in the same manner as in steps A and B of Example 1 by using 3-(1-(6-chloro-2-oxo-1,2-dihydro-3H-imidazo[4,5-b]pyridin-3-yl)cyclopropyl)-2-fluorobenzoic acid methyl ester (32 mg, 0.09 mmol) obtained in Preparation Example 77 and (4-dimethylamino)phenyl)boronic acid (30 mg, 0.18 mmol).
¹H-NMR (400 MHz, MeOD-d4) δ8.03 (d, 1H), 7.76 (dt, 1H), 7.57 (dt, 1H), 7.23 (m, 2H), 7.16 (d, 1H), 7.09 (t, 1H), 6.86 (m, 2H), 2.98 (s, 6H), 1.69 (m, 4H)

### Example 230: Preparation of 3-(1-(6-chloro-1-(4-(morpholinophenyl)-2-oxo-1,2-dihydro-3H-imidazo[4,5-b]pyridin-3-yl)cyclopropyl)-2-fluorobenzoic acid

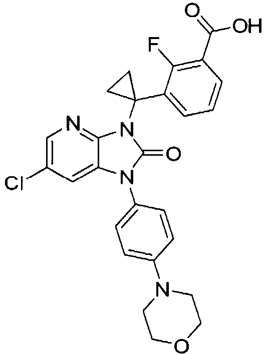

The title compound (14 mg, yield: 31%) was obtained in the same manner as in steps A and B of Example 1 by using 3-(1-(6-chloro-2-oxo-1,2-dihydro-3*H*-imidazo[4,5-b]pyridin-3-yl)cyclopropyl)-2-fluorobenzoic acid methyl ester (33 mg, 0.09 mmol) obtained in Preparation Example 77 and (4-morpholinophenyl)boronic acid (37 mg, 0.18 mmol).
¹H-NMR (500 MHz, CDCl₃) δ8.14 (s, 1H), 8.12 (m, 3H), 7.34 (m, 2H), 7.20 (m, 2H), 7.02 (m, 2H), 3.90 (s, 4H), 3.22 (s, 4H), 1.85 (s, 2H), 1.70 (s, 2H)

### Example 231: Preparation of 3-(1-(6-chloro-2-oxo-1-(4-(pyrrolidine-1-carbonyl)phenyl)-1,2-dihydro-3H-imidazo[4,5-b]pyridin-3-yl)cyclopropyl)-2-fluorobenzoic acid

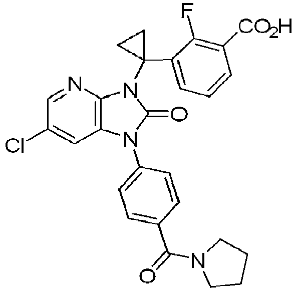

The title compound was obtained in the same manner as in Example 56 by using 3-(1-(6-chloro-2-oxo-1,2-dihydro-3H-imidazo[4,5-b]pyridin-3-yl)cyclopropyl)-2-fluorobenzoic acid methyl ester obtained in Preparation Example 77 and pyrrolidine.
¹H-NMR (400 MHz, MeOD-d4) 58.10 (d, 1H), 7.98 (dt, 1H), 7.80 (dt, 1H), 7.70 (m, 2H), 7.61 (m, 2H), 7.44 (d, 1H), 7.18 (t, 1H), 3.59 (m, 2H), 3.51 (m, 2H), 1.95 (m, 4H), 1.80 (m, 2H), 1.65 (m, 2H).

### Example 232: Preparation of 3-(1-(6-chloro-1-(4-(3-hydroxyazetidine-1-carbonyl)phenyl)-2-oxo-1,2-dihydro-3H-imidazo[4,5-b]pyridin-3-yl)cyclopropyl)-2-fluorobenzoic acid

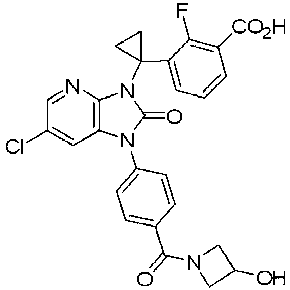

The title compound was obtained in the same manner as in Example 56 by using 3-(1-(6-chloro-2-oxo-1,2-dihydro-3H-imidazo[4,5-b]pyridin-3-yl)cyclopropyl)-2-fluorobenzoic acid methyl ester obtained in Preparation Example 77 and 3-hydroxyazetidine hydrochloride.
¹H-NMR (500 MHz, Acetone-d6) δ8.22 (d, 1H), 8.10 (m, 2H), 7.87 (m, 2H), 7.78 (m, 1H), 7.70 (m, 1H), 7.54 (dd, 1H), 7.25 (dt, 1H), 4.68 (m, 1H), 4.36 (m, 1H), 4.20 (m, 1H), 3.93 (m, 1H), 1.84 (m, 2H), 1.67 (m, 2H)

### Example 233: Preparation of 3-(1-(1-(4-(2-oxa-6-azaspiro[3.3]heptane-6-carbonyl)phenyl)-6-chloro-2-oxo-1,2-dihydro-3H-imidazo[4,5-b]pyridin-3-yl)cyclopropyl)-2-fluorobenzoic acid

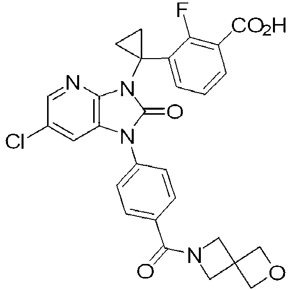

The title compound was obtained in the same manner as in Example 56 by using 3-(1-(6-chloro-2-oxo-1,2-dihydro-3*H-*imidazo[4,5-b]pyridin-3-yl)cyclopropyl)-2-fluorobenzoic acid methyl ester obtained in Preparation Example 77 and 2-oxa-6-azaspiro[3.3]heptane.
¹H-NMR (500 MHz, DMSO-d6) δ 8.18 (d, 1H), 8.11 (m, 2H), 7.92 (m, 1H), 7.73 (m, 4H), 7.60 (d, 1H), 7.25 (t, 1H), 4.69 (m, 2H), 4.53 (m, 1H), 4.25 (m, 1H), 3.5 (2H), 1.74 (m, 3H), 1.63 (m, 3H)

### Example 234: Preparation of 3-(1-(5-chloro-2-oxo-3-(4-(1-propionylpiperidin-4-yl)phenyl)-2,3-dihydro-1H-benzo[d]imidazol-1-yl)cyclopropyl)benzoic acid

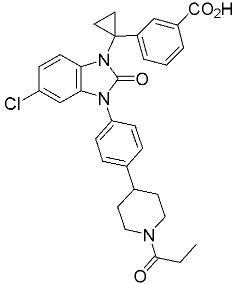

The process of the following steps A and B gave the title compound.

### Step A: Preparation of 4-(4-(6-chloro-3-(1-(3-(methoxycarbonyl)phenyl)cyclopropyl)-2-oxo-2,3-dihydro-1H-benzo[d]imidazol-1-yl)phenyl)piperidine-1-carboxylic acid tert-butyl ester

The title compound (90 mg, yield: 17%) was obtained in the same manner as in step A of Example 139 by using 3-(1-(5-chloro-2-oxo-2,3-dihydro-1H-benzo[d]imidazol-1-yl)cyclopropyl)benzoic acid methyl ester (302 mg, 0.88 mmol) obtained in Preparation Example 3 and 4-(4-bromophenyl(piperidine-1-carboxylic acid tert-butyl ester (300 mg, 0.88 mmol).

### Step B: Preparation of 3-(1-(5-chloro-2-oxo-3-(4-(1-propionylpiperidin-4-yl)phenyl)-2,3-dihydro-1H-benzo[d]imidazol-1-yl)cyclopropyl)benzoic acid

To 4-(4-(6-chloro-3-(1-(3-(methoxycarbonyl)phenyl)cyclopropyl)-2-oxo-2,3-dihydro-1H-benzo[d]imidazol-1-yl)phenyl)piperidine-1-carboxylic acid tert-butyl ester (90 mg, 0.15 mmol) obtained in step A above were added 2 mL of DCM and 0.15 mL of hydrochloric acid (4 M EtOAc solution), and the resulting mixture was stirred at room temperature for 1 hour, and then the reaction solution was concentrated under reduced pressure. This was dried to give the title compound (76 mg, yield: 94%) in the same manner as in steps A and B of Example 102.
¹H-NMR (400 MHz, CDCl₃) δ 7.94 (dd, 1H), 7.91 (d, 1H), 7.54 (dd, 1H), 7.47 (d, 2H), 7.38 (t, 1H), 7.35 (d, 2H), 7.14 (d, 1H), 7.08 (2H), 4.85 (m, 1H), 4.01 (m, 1H), 3.15 (td, 1H), 2.82 (td, 1H), 2.65 (td, 1H), 2.42 (q, 2H), 1.94 (m, 2H), 1.68 (6H), 1.17 (t, 3H)

### Example 235: Preparation of 3-(1-(5-chloro-2-oxo-3-(4-(1-propionylpiperidin-4-yl)phenyl)-2,3-dihydro-1H-benzo[d]imidazol-1-yl)cyclopropyl)-5-fluorobenzoic acid

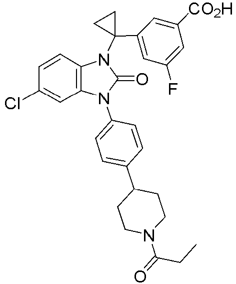

The process of the following steps A and B gave the title compound.

### Step A: Preparation of 4-(4-(6-chloro-3-(1-(3-fluoro-5-(methoxycarbonyl)phenyl)cyclopropyl)-2-oxo-2,3-dihydro-1H-benzo[d]imidazol-1-yl)phenyl)piperidine-1-carboxylic acid tert-butyl ester

The title compound (90 mg, yield: 17%) was obtained in the same manner as in step A of Example 139 by using 3-(1-(5-chloro-2-oxo-2,3-dihydro-1*H*-benzo[*d*]imidazol-1-yl)cyclopropyl)-2-fluorobenzoic acid methyl ester (318 mg, 0.88 mmol) obtained Preparation Example 4 and 4-(4-bromophenyl)piperidine-1-carboxylic acid tert-butyl ester (300 mg, 0.88 mmol).

### Step B: Preparation of 3-(1-(5-chloro-2-oxo-3-(4-(1-propionylpiperidin-4-yl)phenyl)-2,3-dihydro-1H-benzo[d]imidazol-1-yl)cyclopropyl)-5-fluorobenzoic acid

The title compound (47 mg, yield: 58%) was obtained in the same manner as in step B of Example 234 by using 4-(4-(6-chloro-3-(1-(3-fluoro-5-(methoxycarbonyl)phenyl)cyclopropyl)-2-oxo-2,3-dihydro-1H-benzo[d]imidazol-1-yl)phenyl)piperidine-1-carboxylic acid tert-butyl ester (90 mg, 0.145 mmol) obtained in step A above.
¹H-NMR (400 MHz, CDCl₃) δ 7.77 (d, 1H), 7.01 (dd, 1H), 7.47 (d, 2H), 7.37 (d, 2H), 7.21 (dd, 1H), 7.10 (3H), 4.86 (m, 1H), 4.02 (m, 1H), 3.16 (m, 1H), 2.83 (m, 1H), 2.66 (m, 1H), 2.43 (q, 2H), 1.95 (m, 2H), 1.70 (6H), 1.18 (t, 3H)

### Example 236: Preparation of 3-(1-(5-chloro-2-oxo-3-(4-((2-oxopyrrolidin-1-yl)methyl)phenyl)-2,3-dihydro-1H-benzo[d]imidazol-1-yl)cyclopropyl)benzoic acid

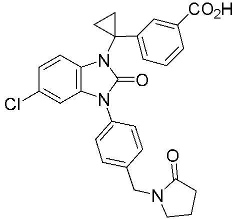

The process of the following steps A and B gave the title compound.

### Step A: Preparation of 3-(1-(5-chloro-2-oxo-3-(4-((2-oxopyrrolidin-1-yl)methyl)phenyl)-2,3-dihydro-1H-benzo[d]imidazol-1-yl)cyclopropyl)benzoic acid methyl ester

The title compound (124 mg, yield: 82%) was obtained in the same manner as in step A of Example 139 by using 3-(1-(5-chloro-2-oxo-2,3-dihydro-1*H*-benzo[*d*]imidazol-1-yl)cyclopropyl)benzoic acid methyl ester (100 mg, 0.29 mmol) obtained in Preparation Example 3 and 1-(4-bromobenzyl)pyrrolidine-2-one (0.074 g, 0.292 mmol).
¹H-NMR (400 MHz, CDCl₃) δ 7.90 (dd, 1H), 7.87 (d, 1H), 7.51 (3H), 7.42 (2H), 7.41 (t, 1H), 7.13 (d, 1H), 7.10 (2H), 4.52 (s, 2H), 3.90 (s, 3H), 3.28 (t, 2H), 2.47 (t, 2H), 2.03 (m, 2H), 1.70 (4H)

### Step B: Preparation of 3-(1-(5-chloro-2-oxo-3-(4-((2-oxopyrrolidin-1-yl)methyl)phenyl)-2,3-dihydro-1H-benzo[d]imidazol-1-yl)cyclopropyl)benzoic acid

The title compound (110 mg, yield: 91%) was obtained in the same manner as in step B of Example 139 by using 3-(1-(5-chloro-2-oxo-3-(4-((2-oxopyrrolidin-1-yl)methyl)phenyl)-2,3-dihydro-1H-benzo[d]imidazol-1-yl)cyclopropyl)benzoic acid methyl ester (124 mg, 0.24 mmol) obtained in step A above.
¹H-NMR (400 MHz, CDCl₃) δ 7.80 (m, 1H), 7.70 (d, 1H), 7.55 (d, 2H), 7.43 (4H), 7.26 (dd, 1H), 7.16 (dd, 1H), 7.05 (d, 1H), 4.46 (s, 2H), 3.30 (t, 2H), 2.33 (m, 2H), 1.97 (m, 2H), 1.68 (4H)

### Example 237: Preparation of 3-(1-(5-chloro-2-oxo-3-(4-((4-(trifluoromethyl)-1H-imidazol-1-yl)methyl)phenyl)-2,3-dihydro-1H-benzo[d]imidazol-1-yl)cyclopropyl)benzoic acid

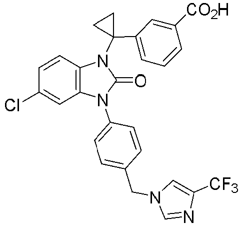

The process of the following steps A and B gave the title compound.

### Step A: Preparation of 3-(1-(5-chloro-2-oxo-3-(4-((4-(trifluoromethyl)-1H-imidazol-1-yl)methyl)phenyl)-2,3-dihydro-1H-benzo[d]imidazol-1-yl)cyclopropyl)benzoic acid methyl ester

The title compound (118 mg, yield: 71%) was obtained in the same manner as in step A of Example 139 by using 3-(1-(5-chloro-2-oxo-2,3-dihydro-1*H-*benzo[*d*]imidazol-1-yl)cyclopropyl)benzoic acid methyl ester (100 mg, 0.29 mmol) obtained in Preparation Example 3 and 1-(4-bromobenzyl)-4-(trifluoromethyl)-1H-imidazole (89 mg, 0.292 mmol).
¹H-NMR (400 MHz, CDCl₃) δ 7.90 (2H), 7.64 (d, 1H), 7.59 (d, 2H), 7.50 (dd, 1H), 7.37 (3H), 7.29 (s, 1H), 7.13 (d, 1H), 7.12 (2H), 5.22 (s, 2H), 3.90 (s, 3H), 1.70 (4H)

### Step B: Preparation of 3-(1-(5-chloro-2-oxo-3-(4-((4-(trifluoromethyl)-1H-imidazol-1-yl)methyl)phenyl)-2,3-dihydro-1H-benzo[d]imidazol-1-yl)cyclopropyl)benzoic acid

The title compound (110 mg, yield: 96%) was obtained in the same manner as in step B of Example 137 by using 3-(1-(5-chloro-2-oxo-3-(4-((4-(trifluoromethyl)-1H-imidazol-1-yl)methyl)phenyl)-2,3-dihydro-1*H*-benzo[*d*]imidazol-1-yl)cyclopropyl)benzoic acid methyl ester (118 mg, 0.21 mmol) obtained in step A above.
MS [M+H] = 553 (M+1)
¹H-NMR (400 MHz, DMSO-d₆) δ 8.06 (d, 1H), 7.98 (d, 1H), 7.77 (dd, 1H), 7.68 (d, 1H), 7.61 (d, 2H), 7.53 (2H), 7.36 (2H), 7.25 (d, 1H), 7.17 (dd, 1H), 7.06 (d, 1H), 5.36 (s, 2H), 1.65 (4H)

### Example 238: Preparation of 3-(1-(5-chloro-3-(4-(1-(hydroxymethyl)cyclopropyl)phenyl)-2-oxo-2,3-dihydro-1H-benzo[d]imidazol-1-yl)cyclopropyl)benzoic acid

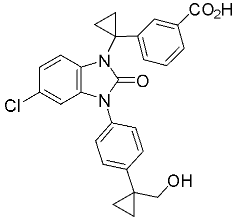

The title compound (70 mg, yield: 48%) was obtained in the same manner as in steps A and B of Example 139 by using 3-(1-(5-chloro-2-oxo-2,3-dihydro-1*H*-benzo[d]imidazol-1-yl)cyclopropyl)benzoic acid methyl ester (100 mg, 0.29 mmol) obtained in Preparation Example 3 and (1-(4-bromophenyl)cyclopropyl)methanol (66 mg, 0.29 mmol).
¹H-NMR (400 MHz, CDCl₃) δ 7.95 (dd, 1H), 7.93 (d, 1H), 7.54 (3H), 7.47 (d, 2H), 7.41 (t, 1H), 7.13 (d, 1H), 7.10 (2H), 3.72 (s, 2H), 1.72 (4H), 0.93 (4H)

### Example 239: Preparation of 3-(1-(5-chloro-3-(4-(1-(hydroxymethyl)cyclobutyl)phenyl)-2-oxo-2,3-dihydro-1H-benzo[d]imidazol-1-yl)cyclopropyl)benzoic acid

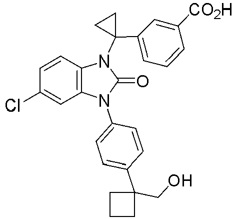

The title compound (60 mg, yield: 42%) was obtained in the same manner as in steps A and B of Example 139 by using 3-(1-(5-chloro-2-oxo-2,3-dihydro-1*H*-benzo[d]imidazol-1-yl)cyclopropyl)benzoic acid methyl ester (100 mg, 0.29 mmol) obtained in Preparation Example 3 and (1-(4-bromophenyl)cyclobutyl)methanol (0.070 g, 0.292 mmol).
¹H-NMR (400 MHz, CDCl₃) δ 7.95 (2H), 7.54 (dd, 1H), 7.48 (d, 2H), 7.39 (t, 1H), 7.31 (d, 2H), 7.15 (d, 1H), 7.10 (2H), 3.80 (s, 2H), 2.33 (4H), 2.10 (2H), 1.93 (m, 1H), 1.70 (4H)

### Example 240: Preparation of 3-(1-(5-chloro-3-(4-(oxetan-3-ylmethyl)phenyl)-2-oxo-2,3-dihydro-1H-benzo[d]imidazol-1-yl)cyclopropyl)benzoic acid

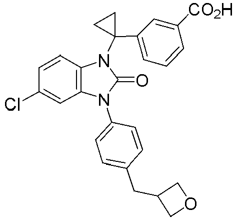

The title compound (50 mg, yield: 26%) was obtained in the same manner as in steps A and B of Example 139 by using 3-(1-(5-chloro-2-oxo-2,3-dihydro-1*H*-benzo[d]imidazol-1-yl)cyclopropyl)benzoic acid methyl ester (137 mg, 0.40 mmol) obtained in Preparation Example 3 and 3-(4-bromobenzyl)oxetane (91 mg, 0.40 mmol).
¹H-NMR (400 MHz, CDCl₃) δ 7.94 (dd, 1H), 7.91 (d, 1H), 7.54 (m, 1H), 7.44 (2H), 7.39 (t, 1H), 7.29 (d, 2H), 7.10 (3H), 4.85 (m, 2H), 4.54 (m, 2H), 3.34 (m, 1H), 3.10 (d, 2H), 1.70 (4H)

### Example 241: Preparation of 3-(1-(5-chloro-3-(4-((3-methyl-2-oxoimidazolin-1-yl)methyl)phenyl)-2-oxo-2,3-dihydro-1H-benzo[d]imidazol-1-yl)cyclopropyl)benzoic acid

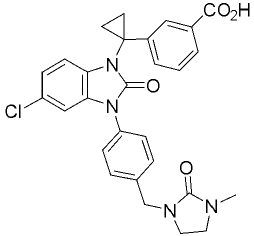

The title compound (120 mg, yield: 79%) was obtained in the same manner as in steps A and B of Example 139 by using 3-(1-(5-chloro-2-oxo-2,3-dihydro-1*H*-benzo[d]imidazol-1-yl)cyclopropyl)benzoic acid methyl ester (100 mg, 0.29 mmol) obtained in Preparation Example 3 and 1-(4-bromobenzyl)-3-methylimidazolidin-2-one (80 mg, 0.29 mmol).
¹H-NMR (400 MHz, DMSO-d6) δ 7.80 (m, 1H), 7.69 (d, 1H), 7.55 (d, 2H), 7.45 (4H), 7.26 (dd, 1H), 7.18 (dd, 1H), 7.05 (d, 1H), 4.35 (s, 2H), 3.25 (4H), 2.70 (s, 3H), 1.68 (4H)

### Example 242: Preparation of 3-(1-(5-chloro-3-(4-(4,4-dimethyl-2-oxooxazolidin-3-yl)phenyl)-2-oxo-2,3-dihydro-1H-benzo[d]imidazol-1-yl)cyclopropyl)benzoic acid

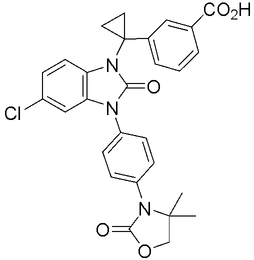

The title compound (74 mg, yield: 47%) was obtained in the same manner as in steps A and B of Example 139 by using 3-(1-(3-(4-bromophenyl)-5-chloro-2-oxo-2,3-dihydro-1*H-*benzo[d]imidazol-1-yl)cyclopropyl)benzoic acid methyl ester (100 mg, 0.20 mmol) obtained in step A of Example 121 and 4,4-dimethyloxazolidin-2-one (28 mg, 0.24 mmol).
¹H-NMR (400 MHz, MeOH-d4) δ 7.83 (m, 1H), 7.70 (m, 1H), 7.68 (d, 2H), 7.54 (d, 2H), 7.40 (2H), 7.27 (m, 1H), 7.17 (m, 1H), 7.14 (m, 1H), 4.31 (s, 2H), 1.73 (4H), 1.41 (s, 6H)

### Example 243: Preparation of 3-(1-(5-chloro-2-oxo-3-(4-(5-oxo-6-oxa-4-azaspiro[2.4]heptan-4-yl)phenyl)-2,3-dihydro-1H-benzo[d]imidazol-1-yl)cyclopropyl)benzoic acid

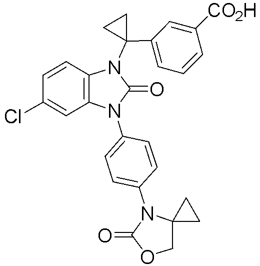

The title compound (25 mg, yield: 20%) was obtained in the same manner as in steps A and B of Example 139 by using 3-(1-(3-(4-bromophenyl)-5-chloro-2-oxo-2,3-dihydro-1*H-*benzo[*d*]imidazol-1-yl)cyclopropyl)benzoic acid methyl ester (100 mg, 0.20 mmol) obtained in step A of Example 121 and 6-oxa-4-azaspiro[2.4]heptan-5-one (26 mg, 0.24 mmol).
¹H-NMR (400 MHz, MeOH-d4) δ 7.86 (m, 1H), 7.83 (m, 1H), 7.68 (d, 2H), 7.46 (d, 2H), 7.40 (m, 2H), 7.27 (m, 1H), 7.15 (m, 1H), 7.13 (m, 1H), 4.57 (s, 2H), 1.73 (4H), 0.84 (4H)

### Example 244: Preparation of 3-(1-(5-chloro-2-oxo-3-(4-((3-(trifluoromethyl)-1H-pyrazol-1-yl)methyl)phenyl)-2,3-dihydro-1H-benzo[d]imidazol-1-yl)cyclopropyl)benzoic acid

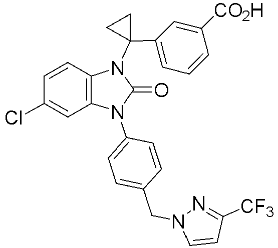

The title compound (74 mg, yield: 47%) was obtained in the same manner as in steps A and B of Example 139 by using 3-(1-(5-chloro-2-oxo-2,3-dihydro-1*H*-benzo[*d*]imidazol-1-yl)cyclopropyl)benzoic acid methyl ester (100 mg, 0.29 mmol) obtained in Preparation Example 3 and 1-(4-bromobenzyl)-3-(trifluoromethyl)-1*H*-pyrazole (90 mg, 0.29 mmol).
¹H-NMR (400 MHz, CDCl3) δ 7.96 (m, 1H), 7.91 (m, 1H), 7.56 (d, 2H), 7.53 (m, 1H), 7.46 (m, 1H), 7.42 (d, 2H), 7.39 (t, 1H), 7.15 (d, 1H), 7.10 (2H), 6.58 (d, 1H), 5.43 (s, 2H), 1.70 (4H)

### Example 245: Preparation of 3-(1-(5-chloro-3-(2-methyl-1-oxoisoindolin-5-yl)-2-oxo-2,3-dihydro-1H-benzo[d]imidazol-1-yl)cyclopropyl)benzoic acid

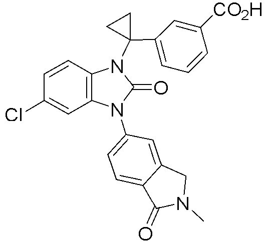

The title compound (80 mg, yield: 58%) was obtained in the same manner as in steps A and B of Example 139 by using 3-(1-(5-chloro-2-oxo-2,3-dihydro-1*H*-benzo[*d*]imidazol-1-yl)cyclopropyl)benzoic acid methyl ester (100 mg, 0.29 mmol) obtained in Preparation Example 3 and 5-bromo-2-methylisoindolin-1-one (66 mg, 0.29 mmol).
¹H-NMR (400 MHz, CDCl3) δ 8.01 (d, 1H), 7.95 (2H), 7.70 (d, 1H), 7.62 (dd, 1H), 7.53 (dd, 1H), 7.40 (t, 1H), 7.18 (d, 1H), 7.18 (2H), 4.46 (s, 2H), 3.24 (s, 3H), 1.70 (4H)

### Example 246: Preparation of 3-(1-(5-chloro-3-(4-(2-hydroxypropane-2-yl)phenyl)-2-oxo-2,3-dihydro-1H-benzo[d]imidazol-1-yl)cyclopropyl)benzoic acid

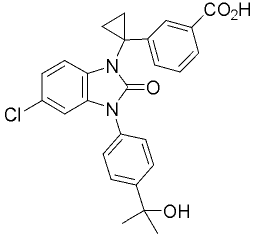

The title compound (88 mg, yield: 65%) was obtained in the same manner as in steps A and B of Example 139 by using 3-(1-(5-chloro-2-oxo-2,3-dihydro-1*H*-benzo[*d*]imidazol-1-yl)cyclopropyl)benzoic acid methyl ester (100 mg, 0.29 mmol) obtained in Preparation Example 3 and 2-(4-bromophenyl)propan-2-ol (63 mg, 0.29 mmol).
¹H-NMR (400 MHz, CDCl3) δ 7.95 (m, 1H), 7.91 (m, 1H), 7.66 (d, 2H), 7.54 (m, 1H), 7.50 (d, 2H), 7.48 (t, 1H), 7.14 (m, 1H), 7.10 (m, 2H), 1.70 (4H), 1.63 (s, 6H)

### Example 247: Preparation of 3-(1-(5-chloro-3-(4-(3-methyloxetan-3-yl)phenyl)-2-oxo-2,3-dihydro-1H-benzo[d]imidazol-1-yl)cyclopropyl)benzoic acid

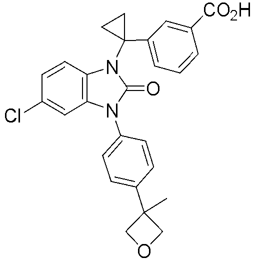

The title compound (100 mg, yield: 75%) was obtained in the same manner as in steps A and B of Example 139 by using 3-(1-(5-chloro-2-oxo-2,3-dihydro-1*H*-benzo[*d*]imidazol-1-yl)cyclopropyl)benzoic acid methyl ester (100 mg, 0.29 mmol) obtained in Preparation Example 3 and 3-(4-bromophenyl)-3-methyloxetane (63 mg, 0.29 mmol).
¹H-NMR (400 MHz, DMSO-d6) δ 7.81 (m, 1H), 7.70 (d, 1H), 7.56 (d, 2H), 7.45 (4H), 7.26 (d, 1H), 7.19 (dd, 1H), 7.06 (d, 1H), 4.87 (d, 2H), 4.60 (d, 2H), 1.69 (7H)

### Example 248: Preparation of 3-(1-(5-chloro-2-oxo-3-(4-(tetrahydrofuran-3-yl)phenyl)-2,3-dihydro-1H-benzo[d]imidazol-1-yl)cyclopropyl)benzoic acid

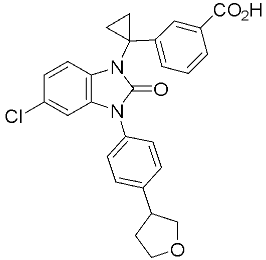

The title compound (90 mg, yield: 67%) was obtained in the same manner as in steps A and B of Example 139 by using 3-(1-(5-chloro-2-oxo-2,3-dihydro-1*H*-benzo[*d*]imidazol-1-yl)cyclopropyl)benzoic acid methyl ester (100 mg, 0.29 mmol) obtained in Preparation Example 3 and 3-(4-bromophenyl)tetrahydrofuran (66 mg, 0.29 mmol).
¹H-NMR (500 MHz, DMSO-d6) δ 13.00 (brs, 1H), 7.81 (m, 1H), 7.70 (m, 1H), 7.48 (4H), 7.26 (2H), 7.26 (dd, 1H), 7.16 (dd, 1H), 7.03 (d, 1H), 4.08 (m, 1H), 4.00 (m, 1H), 3.85 (m, 1H), 3.61 (m, 1H), 3.50 (m, 1H), 2.37 (m, 1H), 2.00 (m, 1H), 1.68 (4H)

### Example 249: Preparation of 3-(1-(5-chloro-2-oxo-3-(4-(tetrahydrofuran-3-yl)phenyl)-2,3-dihydro-1H-benzo[d]imidazol-1-yl)cyclopropyl)-5-fluorobenzoic acid

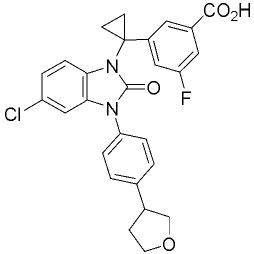

The title compound (20 mg, yield: 14%) was obtained in the same manner as in steps A and B of Example 139 by using 3-(1-(5-chloro-2-oxo-2,3-dihydro-1*H*-benzo[*d*]imidazol-1-yl)cyclopropyl)-5-fluorobenzoic acid methyl ester (100 mg, 0.28 mmol) obtained in the same manner as in Preparation Example 59 and 3-(4-bromophenyl)tetrahydrofuran (63 mg, 0.28 mmol).
¹H-NMR (400 MHz, CDCl3) δ 7.64 (m, 1H), 7.55 (dd, 1H), 7.46 (d, 2H), 7.42 (d, 2H), 7.16 (dd, 1H), 7.08 (3H), 4.17 (t, 1H), 4.11 (td, 1H), 3.94 (dd, 1H), 3.79 (dd, 1H), 3.47 (q, 1H), 2.41 (m, 1H), 2.04 (m, 1H), 1.70 (4H)

### Example 250: Preparation of 3-(1-(5-chloro-2-oxo-3-(4-(tetrahydrofuran-3-yl)phenyl)-2,3-dihydro-1H-benzo[d]imidazol-1-yl)cyclopropyl)-2-fluorobenzoic acid

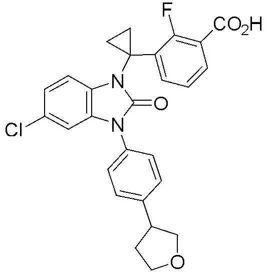

The title compound (50 mg, yield: 35%) was obtained in the same manner as in steps A and B of Example 139 by using 3-(1-(5-chloro-2-oxo-2,3-dihydro-*1H*-benzo[*d*]imidazol-1-yl)cyclopropyl)-2-fluorobenzoic acid methyl ester (100 mg, 0.28 mmol) obtained in Preparation Example 4 and 3-(4-bromophenyl)tetrahydrofuran (70 mg, 0.30 mmol).
¹H-NMR (500 MHz, CDCl3) δ 8.24 (t, 1H), 7.96 (t, 1H), 7.56 (d, 1H), 7.41 (m, 4H), 7.23 (t, 1H), 7.17 (dd, 1H), 7.02 (d, 1H), 4.15 (m, 1H), 4.13 (m, 1H), 3.97 (m, 1H), 3.79 (t, 1H), 3.48 (m, 1H), 2.42 (m, 1H), 2.05 (m, 1H), 1.73 (m, 2H), 1.65 (m, 2H)

### Example 251: Preparation of 3-(1-(5-chloro-3-(1,1-dimethyl-1,3-dihydroisobenzofuran-5-yl)-2-oxo-2,3-dihydro-1H-benzo[d]imidazol-1-yl)cyclopropyl)benzoic acid

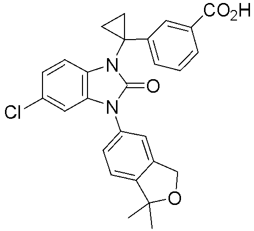

The title compound (48 mg, yield: 68%) was obtained in the same manner as in steps A and B of Example 139 by using 3-(1-(5-chloro-2-oxo-2,3-dihydro-1*H*-benzo[*d*]imidazol-1-yl)cyclopropyl)benzoic acid methyl ester (50 mg, 0.14 mmol) obtained in Preparation Example 3 and 5-bromo-1,1-dimethyl-1,3-dihydroisobenzofuran (33 mg, 0.14 mmol).
¹H-NMR (400 MHz, CDCl3) δ 7.95 (dd, 1H), 7.92 (d, 1H), 7.55 (dd, 1H), 7.41 (d, 1H), 7.40 (t, 1H), 7.36 (d, 1H), 7.27 (d, 1H), 7.15 (d, 1H), 7.10 (2H), 5.13 (s, 2H), 1.70 (4H), 1.55 (s, 6H)

### Example 252: Preparation of 3-(1-(5-chloro-3-(1,1-dimethyl-1,3-dihydroisobenzofuran-5-yl)-2-oxo-2,3-dihydro-1H-benzo[d]imidazol-1-yl)cyclopropyl)-5-fluorobenzoic acid

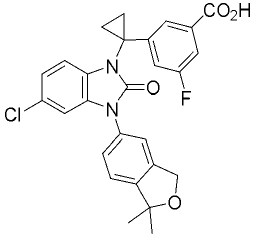

The title compound (44 mg, yield: 32%) was obtained in the same manner as in steps A and B of Example 139 by 3-(1-(5-chloro-2-oxo-2,3-dihydro-1*H*-benzo[*d*]imidazol-1-yl)cyclopropyl)-5-fluorobenzoic acid methyl ester (100 mg) obtained in the same manner as in Preparation Example 59 and 5-bromo-1,1-dimethyl-1,3-dihydroisobenzofuran (33 mg, 0.14 mmol).
¹H-NMR (400 MHz, CDCl₃) δ 7.65 (m, 1H), 7.48 (dd, 1H), 7.38 (dd, 1H), 7.34 (d, 1H), 7.24 (d, 1H), 7.08 (4H), 5.10 (s, 2H), 1.66 (4H), 1.52 (s, 6H)

### Example 253: Preparation of 3-(1-(5-chloro-3-(1,1-dimethyl-1,3-dihydroisobenzofuran-5-yl)-2-oxo-2,3-dihydro-1H-benzo[d]imidazol-1-yl)cyclopropyl)-2-fluorobenzoic acid

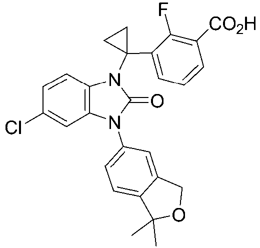

The title compound (8 mg, yield: 6%) was obtained in the same manner as in step A of Example 139 by using 3-(1-(5-chloro-2-oxo-2,3-dihydro-*1H*-benzo[*d*]imidazol-1-yl)cyclopropyl)-2-fluorobenzoic acid methyl ester (100 mg, 0.28 mmol) obtained in Preparation Example 4 and 5-bromo-1,1-dimethyl-1,3-dihydroisobenzofuran (69 mg, 0.30 mmol).
¹H-NMR (500 MHz, CDCl₃) δ 8.23 (t, 1H), 7.96 (t, 1H), 7.57 (d, 1H), 7.34-7.18 (5H), 7.03 (s, 1H), 5.12 (s, 2H), 1.73 (m, 2H), 1.65 (m, 2H), 1.55 (s, 6H)

### Example 254: Preparation of 3-(1-(5-chloro-2-oxo-3-(3-oxo-3,4-dihydro-2H-benzo[b][1,4]oxazin-7-yl)-2,3-dihydro-1H-benzo[d]imidazol-1-yl)cyclopropyl)-5-fluorobenzoic acid

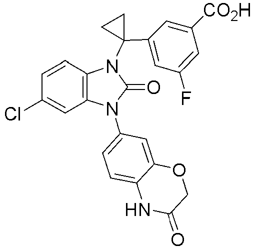

The title compound (49 mg, yield: 50%) was obtained in the same manner as in steps A and B of Example 139 by using 3-(1-(5-chloro-2-oxo-2,3-dihydro-1*H*-benzo[*d*]imidazol-1-yl)cyclopropyl)-5-fluorobenzoic acid methyl ester (72 mg, 0.20 mmol) obtained in the same manner as in Preparation Example 59 and 7-bromo-2*H*-benzo[*b*][1,4]oxazin-3(4*H*)-one (50 mg, 0.21 mmol).
¹H-NMR (400 MHz, CDCl3) δ 9.39 (brs, 1H), 7.63 (m, 1H), 7.62 (m, 1H), 7.15 (2H), 7.08 (3H), 7.04 (m, 1H), 6.98 (m, 1H), 4.68 (s, 2H), 1.75 (4H)

### Example 255: Preparation of 3-(1-(5-chloro-3-(4-methyl-3,4-dihydro-2H-benzo[b][1,4]oxazin-7-yl)-2-oxo-2,3-dihydro-1H-benzo[d]imidazol-1-yl)cyclopropyl)-5-fluorobenzoic acid

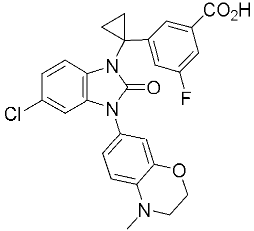

The title compound (16 mg, yield: 16%) was obtained in the same manner as in steps A and B of Example 139 by using 3-(1-(5-chloro-2-oxo-2,3-dihydro-1*H*-benzo[*d*]imidazol-1-yl)cyclopropyl)-5-fluorobenzoic acid methyl ester (72 mg, 0.20 mmol) obtained in the same manner as in Preparation Example 59 and 7-bromo-4-methyl-3,4-dihydro-2*H*-benzo[*b*][1,4]oxazine (50 mg, 0.21 mmol).
¹H-NMR (400 MHz, CDCl₃) δ 7.66 (m, 1H), 7.60 (dd, 1H), 7.19 (d, 1H), 7.06 (3H), 6.97 (dd, 1H), 6.89 (d, 1H), 6.76 (d, 1H), 4.34 (m, 2H), 3.33 (m, 2H), 2.96 (s, 3H), 1.70 (4H)

### Example 256: Preparation of 3-(1-(5-chloro-3-(4-(1-hydroxycyclobutyl)phenyl)-2-oxo-2,3-dihydro-1H-benzo[d]imidazol-1-yl)cyclopropyl)benzoic acid

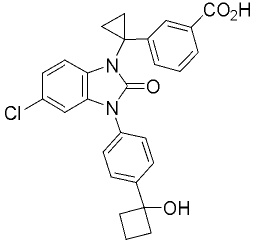

The title compound (80 mg, yield: 58%) was obtained in the same manner as in step A of Example 139 by using 3-(1-(5-chloro-2-oxo-2,3-dihydro-1*H*-benzo[*d*]imidazol-1-yl)cyclopropyl)benzoic acid methyl ester (100 mg, 0.29 mmol) obtained in Preparation Example 3 and 1-(4-bromophenyl)cyclobutan-1-ol (73 mg, 0.32 mmol).
¹H-NMR (400 MHz, DMSO-d6) δ 7.81 (m, 1H), 7.70 (3H), 7.53 (d, 2H), 7.45 (2H), 7.26 (d, 1H), 7.18 (dd, 1H), 7.03 (d, 1H), 2.46 (m, 2H), 2.33 (m, 2H), 1.96 (m, 1H), 1.75 (m, 1H), 1.72 (4H)

### Example 257: Preparation of 3-(1-(5-chloro-3-(4-(1-hydroxycyclobutyl)phenyl)-2-oxo-2,3-dihydro-1H-benzo[d]imidazol-1-yl)cyclopropyl)-5-fluorobenzoic acid

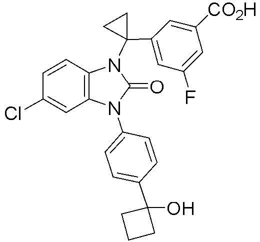

The title compound (90 mg, yield: 66%) was obtained in the same manner as in steps A and B of Example 139 by using 3-(1-(5-chloro-2-oxo-2,3-dihydro-1*H*-benzo[*d*]imidazol-1-yl)cyclopropyl)-5-fluorobenzoic acid methyl ester (100 mg, 0.27 mmol) obtained in the same manner as in Preparation Example 59 and 1-(4-bromophenyl)cyclobutan-1-ol (70 mg, 0.30 mmol).
¹H-NMR (400 MHz, CDCl3) δ 7.67 (m, 3H), 7.60 (m, 1H), 7.52 (m, 2H), 2.18 (m, 1H), 7.17 (3H), 2.58 (m, 2H), 2.41 (m, 2H), 2.05 (m, 1H), 1.73 (5H)

### Example 258: Preparation of 3-(1-(5-chloro-3-(4-(3-hydroxyoxetan-3-yl)phenyl)-2-oxo-2,3-dihydro-1H-benzo[d]imidazol-1-yl)cyclopropyl)benzoic acid

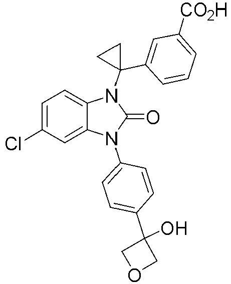

The title compound (50 mg, yield: 51%) was obtained in the same manner as in steps A and B of Example 139 by using 3-(1-(5-chloro-2-oxo-2,3-dihydro-1*H*-benzo[*d*]imidazol-1-yl)cyclopropyl)benzoic acid methyl ester (70 mg, 0.20 mmol) obtained in Preparation Example 3 and 3-(4-bromophenyl)oxetan-3-ol (47 mg, 0.20 mmol).
¹H-NMR (400 MHz, DMSO-d6) δ 7.82 (d, 2H), 7.80 (m, 1H), 7.70 (m, 1H), 7.61 (d, 2H), 7.42 (2H), 7.27 (d, 1H), 7.18 (dd, 1H), 7.06 (d, 1H), 6.54 (brs, 1H), 4.83 (d, 2H), 4.75 (d, 2H), 1.68 (4H)

### Example 259: Preparation of 3-((5-chloro-3-(4-(1-(oxetan-3-yl)piperidin-4-yl)phenyl)-2-oxo-2,3-dihydro-1H-benzo[d]imidazol-1-yl)methyl)benzoic acid

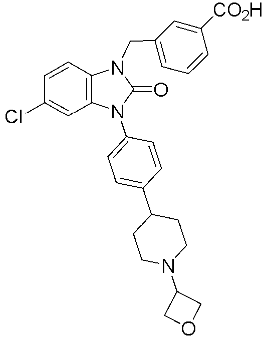

To 3-((5-chloro-2-oxo-3-(4-(piperidin-4-yl)phenyl)-2,3-dihydro-1*H*-benzo[*d*]imidazol-1-yl)methyl)benzoic acid methyl ester hydrochloride (117 mg, 0.23 mmol) obtained in Preparation Example 30 were added 2.2 mL of 1,2-dichloroethane and 0.2 mL of DIPEA, and then the resulting mixture was stirred at room temperature for 10 minutes. After the reaction solution was concentrated under reduced pressure, 2.2 mL of 1,2-dichloroethane, oxetan-3-one (0.021 mL, 0.34 mmol), and acetic acid (0.020 mL, 0.34 mmol) were added thereto, and the resulting mixture was stirred at room temperature for 1 hour. To the reaction solution was added NaBH(OAc)₃ (72 mg, 0.34 mmol), followed by stirring at room temperature for 16 hours. After the reaction was terminated, an aqueous NaHCO₃ solution was added thereto, and then an organic layer was separated and purified by MPLC to give methyl ester. By using this, the title compound (55 mg, yield: 46%) was obtained in the same manner as in step B of Example 139.
¹H-NMR (400 MHz, MeOH-d4) δ 8.03 (d, 1H), 7.95 (m, 1H), 7.59 (m, 1H), 7.49 (4H), 7.46 (t, 1H), 7.10 (2H), 7.00 (m, 1H), 5.22 (s, 2H), 4.78 (t, 2H), 4.72 (t, 2H), 3.82 (m, 1H), 3.14 (m, 2H), 2.81 (m, 1H), 2.33 (m, 2H), 2.00 (m, 2H), 1.92 (m, 2H)

### Example 260: Preparation of 3-(1-(5-chloro-3-(4-(1-(oxetan-3-yl)piperidin-4-yl)phenyl)-2-oxo-2,3-dihydro-1H-benzo[d]imidazol-1-yl)cyclopropyl)benzoic acid

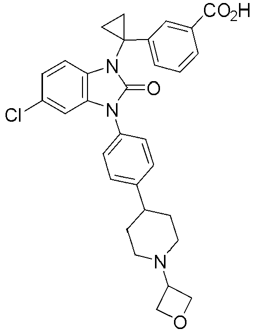

The title compound (60 mg, yield: 38%) was obtained in the same manner as in steps A and B of Example 139 by using 3-(1-(5-chloro-2-oxo-2,3-dihydro-1*H*-benzo[*d*]imidazol-1-yl)cyclopropyl)benzoic acid methyl ester (100 mg, 0.29 mmol) obtained in Preparation Example 3 and 4-(4-bromophenyl)-1-(oxetan-3-yl)piperidine (0.095 g, 0.32 mmol).
¹H-NMR (400 MHz, DMSO-d6) δ 7.81 (m, 1H), 7.70 (d, 1H), 7.50 (6H), 7.26 (d, 1H), 7.17 (dd, 1H), 7.03 (d, 1H), 4.60 (4H), 3.20 (1H), 3.00 (m, 2H), 2.70 (m, 1H), 1.85 (6H), 1.68 (4H)

### Example 261: Preparation of 3-(1-(5-chloro-2-oxo-3-(6-(tetrahydro-2H-pyran-4-yl)pyridin-3-yl)-2,3-dihydro-1H-benzo[d]imidazol-1-yl)cyclopropyl)benzoic acid

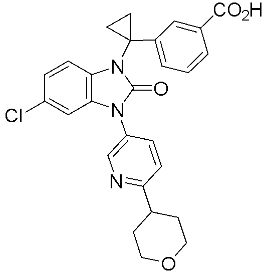

The title compound (46 mg, yield: 43%) was obtained in the same manner as in steps A and B of Example 139 by using 3-(1-(5-chloro-2-oxo-2,3-dihydro-1*H*-benzo[*d*]imidazol-1-yl)cyclopropyl)benzoic acid methyl ester (70 mg, 0.20 mmol) obtained in Preparation Example 3 and 5-bromo-2-(tetrahydro-2*H*-pyran-4-yl)pyridine (0.049 g, 0.204 mmol).
¹H-NMR (400 MHz, DMSO-d6) δ 8.73 (m, 1H), 7.99 (dd, 1H), 7.81 (dt, 1H), 7.71 (m, 1H), 7.53 (d, 1H), 7.46 (2H), 7.28 (d, 1H), 7.19 (dd, 1H), 7.13 (d, 1H), 3.98 (m, 2H), 3.50 (m, 2H), 3.05 (m, 1H), 1.83 (4H), 1.68 (4H)

### Example 262: Preparation of 3-(1-(5-chloro-2-oxo-3-(6-(tetrahydro-2H-pyran-4-yl)pyridin-3-yl)-2,3-dihydro-1H-benzo[d]imidazol-1-yl)cyclopropyl)-5-fluorobenzoic acid

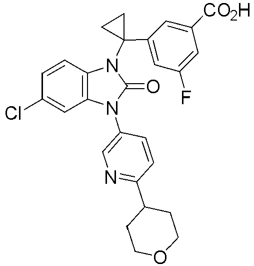

The title compound (18 mg, yield: 13%) was obtained in the same manner as in steps A and B of Example 139 by 3-(1-(5-chloro-2-oxo-2,3-dihydro-1*H*-benzo[*d*]imidazol-1-yl)cyclopropyl)-5-fluorobenzoic acid methyl ester (100 mg, 0.27 mmol) obtained in the same manner as in Preparation Example 59 and 5-bromo-2-(tetrahydro-2*H*-pyran-4-yl)pyridine (0.074 g, 0.27 mmol).
¹H-NMR (400 MHz, CDCl3) δ 8.77 (m, 1H), 7.87 (m, 1H), 7.68 (m, 1H), 7.41 (m, 1H), 7.38 (m, 1H), 7.10 (4H), 4.12 (m, 2H), 3.60 (m, 2H), 3.05 (m, 1H), 1.96 (4H), 1.70 (4H)

### Example 263: Preparation of 3-(1-(5-chloro-3-(4-(5-cyclopropyl-1,3,4-oxadiazol-2-yl)phenyl)-2-oxo-2,3-dihydro-1H-benzo[d]imidazol-1-yl)cyclopropyl)benzoic acid

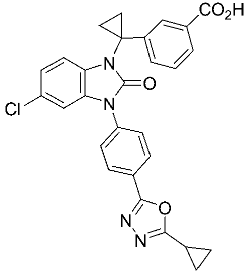

The title compound (162 mg, yield: 72%) was obtained in the same manner as in steps A and B of Example 139 by using 3-(1-(5-chloro-2-oxo-2,3-dihydro-1*H*-benzo[*d*]imidazol-1-yl)cyclopropyl)benzoic acid methyl ester (150 mg, 0.44 mmol) obtained in Preparation Example 3 and 2-(4-bromophenyl)-5-cyclopropyl-1,3,4-oxadiazole (140 mg, 0.53 mmol).
¹H-NMR (400 MHz, CDCl3) δ 8.19 (d, 2H), 7.95 (2H), 7.72 (d, 2H), 7.56 (m, 1H), 7.43 (t, 1H), 7.17 (3H), 2.26 (m, 1H), 1.72 (4H), 1.26 (4H)

### Example 264: Preparation of 3-(1-(5-chloro-3-(4-(5-cycloheptyl-1,3,4-oxadiazol-2-yl)phenyl)-2-oxo-2,3-dihydro-1H-benzo[d]imidazol-1-yl)cyclopropyl)benzoic acid

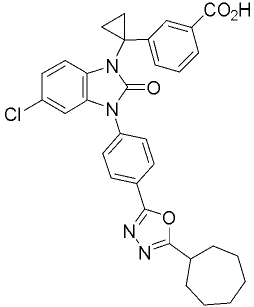

The title compound (4 mg, yield: 0.3%) was obtained in the same manner as in steps A and B of Example 139 by using 3-(1-(5-chloro-2-oxo-2,3-dihydro-1*H*-benzo[*d*]imidazol-1-yl)cyclopropyl)benzoic acid methyl ester (49 mg, 0.14 mmol) obtained in Preparation Example 3 and 2-(4-bromophenyl)-5-cycloheptyl-1,3,4-oxadiazole (40 mg, 0.15 mmol).
¹H-NMR (400 MHz, CDCl3) δ 8.21 (d, 2H), 7.94 (d, 1H), 7.92 (s, 1H), 7.72 (d, 2H), 7.55 (d, 1H), 7.39 (t, 1H), 7.13 (m, 3H), 3.21 (m, 1H), 2.15 (m, 2H), 1.95 (m, 2H), 1.85 (m, 2H), 1.72 (m, 2H), 1.69 (m, 2H), 1.64 (m, 6H)

### Example 265: Preparation of 3-(1-(5-chloro-3-(4-(5-cyclohexyl-1,3,4-oxadiazol-2-yl)phenyl)-2-oxo-2,3-dihydro-1H-benzo[d]imidazol-1-yl)cyclopropyl)benzoic acid

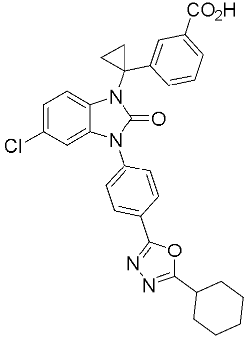

The title compound (2.6 mg, yield: 10%) was obtained in the same manner as in steps A and B of Example 139 by using 3-(1-(5-chloro-2-oxo-2,3-dihydro-1*H*-benzo[*d*]imidazol-1-yl)cyclopropyl)benzoic acid methyl ester (15 mg, 0.044 mmol) obtained in Preparation Example 3 and 2-(4-bromophenyl)-5-cyclohexyl-1,3,4-oxadiazole (15 mg, 0.05 mmol).
¹H-NMR (500 MHz, CDCl₃) δ 8.24 (d, 2H), 7.99 (d, 1H), 7.95 (s, 1H), 7.75 (d, 2H), 7.57 (d, 1H), 7.43 (t, 1H), 7.19 (d, 2H), 7.16 (d, 1H), 3.04 (m, 1H), 2.22 (m, 2H), 1.91 (m, 2H), 1.77 (m, 3H), 1.73 (m, 5H), 1.48 (m, 2H)

### Example 266: Preparation of 3-(1-(5-chloro-3-(4-(5-cyclopentyl-1,3,4-oxadiazol-2-yl)phenyl)-2-oxo-2,3-dihydro-1H-benzo[d]imidazol-1-yl)cyclopropyl)benzoic acid

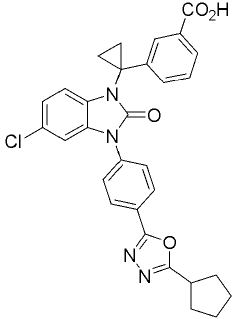

The title compound (4 mg, yield: 0.3%) was obtained in the same manner as in steps A and B of Example 139 by using 3-(1-(5-chloro-2-oxo-2,3-dihydro-1*H*-benzo[*d*]imidazol-1-yl)cyclopropyl)benzoic acid methyl ester (106 mg, 0.31 mmol) obtained in Preparation Example 3 and 2-(4-bromophenyl)-5-cyclopentyl-1,3,4-oxadiazole (100 mg, 0.77 mmol).
¹H-NMR (500 MHz, CDCl3) δ 8.24 (d, 2H), 7.98 (d, 1H), 7.95 (s, 1H), 7.75 (d, 2H), 7.57 (d, 1H), 7.43 (t, 1H), 7.19 (d, 2H), 7.15 (d, 1H), 3.44 (m, 1H), 2.22 (m, 2H), 2.04 (m, 2H), 1.89 (m, 2H), 1.79 (m, 4H), 1.67 (m, 2H)

### Example 267: Preparation of 3-(1-(5-chloro-2-oxo-3-(4-(5-(tetrahydro-2H-pyran-4-yl)-1,3,4-oxadiazol-2-yl)phenyl)-2,3-dihydro-1H-benzo[d]imidazol-1-yl)cyclopropyl)benzoic acid

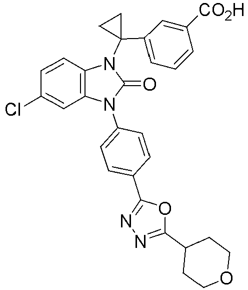

The title compound (20 mg, yield: 45%) was obtained in the same manner as in steps A and B of Example 139 by using 3-(1-(5-chloro-2-oxo-2,3-dihydro-1*H*-benzo[*d*]imidazol-1-yl)cyclopropyl)benzoic acid methyl ester (28 mg, 0.08 mmol) obtained in Preparation Example 3 and 2-(4-bromophenyl)-5-(tetrahydro-2*H*-pyran-4-yl)-1,3,4-oxadiazole (28 mg, 0.09 mmol).
¹H-NMR (500 MHz, CDCl3) δ 8.24 (d, 2H), 7.99 (d, 1H), 7.95 (s, 1H), 7.76 (d, 2H), 7.57 (d, 1H), 7.43 (t, 1H), 7.20 (d, 2H), 7.16 (d, 1H), 4.12 (m, 2H), 3.62 (m, 2H), 3.31 (m, 1H), 2.12 (m, 4H), 1.77 (m, 2H), 1.73 (m, 2H)

### Example 268: Preparation of 3-(1-(5-chloro-2-oxo-3-(4-(5-(tetrahydro-2H-pyran-4-yl)-1,3,4-oxadiazol-2-yl)phenyl)-2,3-dihydro-1H-benzo[d]imidazol-1-yl)cyclopropyl)-2-fluorobenzoic acid

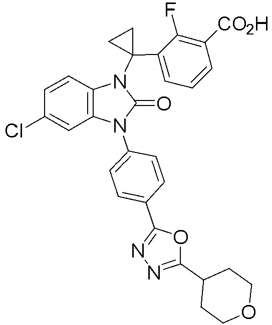

The title compound (28 mg, yield: 35%) was obtained in the same manner as in steps A and B of Example 139 by using 3-(1-(5-chloro-2-oxo-2,3-dihydro-*1H*-benzo[*d*]imidazol-1-yl)cyclopropyl)-2-fluorobenzoic acid methyl ester (50 mg, 0.14 mmol) obtained in Preparation Example 4 and 2-(4-bromophenyl)-5-(tetrahydro-2*H*-pyran-4-yl)-1,3,4-oxadiazole (47 mg, 0.15 mmol).
¹H-NMR (500 MHz, CDCl3) δ 8.22 (m, 3H), 7.96 (t, 1H), 7.68 (d, 2H), 7.60 (d, 1H), 7.25 (m, 1H), 7.22 (m, 1H), 7.11 (s, 1H), 4.11 (m, 2H), 3.61 (t, 2H), 3.30 (m, 1H), 2.12 (m, 4H), 1.75 (m, 2H), 1.68 (m, 2H)

### Example 269: Preparation of 3-(1-(5-chloro-2-oxo-3-(4-(1-propyl-1H-pyrazol-4-yl)phenyl)-2,3-dihydro-1H-benzo[d]imidazol-1-yl)cyclopropyl)benzoic acid

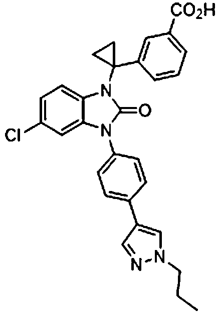

The process of the following steps A and B gave the title compound.

### Step A: Preparation of 4-(4-bromophenyl)-1-propyl-1H-pyrazole

To 1-bromo-4-iodobenzene (4.37 g, 15.46 mmol) and 4-pyrazolboronic acid pinacol ester (2.00 g, 10.31 mmol) were added 20 mL of DMF and 4 mL of water, and to the mixture were added K₂HPO₄ (3.59 g, 20.61 mmol) and PdCl₂ (dppf) (0.377 mmol, 0.515 mL), and the resulting mixture was stirred at 90 °C for 4 hours. After the reaction was terminated, the resulting solid was filtered through Celite, and then the filtrate was purified by MPLC to give 4-(4-bromophenyl)-1*H*-pyrazole (1.02 g, 44% yield). This 4-(4-bromophenyl)-1*H*-pyrazole (0.25 g, 1.12 mmol) was dissolved in THF and then cooled to 0 °C. NaH (40.3 mg, 1.68 mmol) was added slowly thereto and then stirred for 30 minutes. Thereafter, 1-bromopropane (0.21 g, 1.68 mmol) was added at 0 °C, and the resulting mixture was stirred while raising the temperature to room temperature. After the reaction was terminated, water was slowly added thereto while stirring at 0 °C, and an organic layer was extracted with EtOAc and a saturated aqueous NH₄Cl solution and dried over Na₂SO₄. The resulting solid was filtered and the filtrate was distilled under reduced pressure and then purified by MPLC to give the title compound (27 mg, yield: 9%).
¹H-NMR (400 MHz, CDCl3) δ 7.73 (s, 1H), 7.60 (s, 1H), 7.45 (m, 2H), 7.32 (m, 2H), 4.09 (t, 2H), 1.91 (m, 2H), 0.93 (t, 3H)

### Step B: Preparation of 3-(1-(5-chloro-2-oxo-3-(4-(1-propyl-1H-pyrazol-4-yl)phenyl)-2,3-dihydro-1H-benzo[d]imidazol-1-yl)cyclopropyl)benzoic acid

The title compound (29 mg, yield: 55%) was obtained in the same manner as in steps A and B of Example 139 by using 4-(4-bromophenyl)-1-propyl-1*H*-pyrazole (27 mg, 0.10 mmol) obtained in step A above and 3-(1-(5-chloro-2-oxo-2,3-dihydro-1*H*-benzo[*d*]imidazol-1-yl)cyclopropyl)benzoic acid methyl ester (35 mg, 0.10 mmol) obtained in Preparation Example 3.
¹H-NMR (400 MHz, CDCl3) δ 7.88 (m, 2H), 7.79 (s, 1H), 7.65 (s, 1H), 7.59 (m, 1H), 7.48 (m, 3H), 7.30 (t, 1H), 7.25 (m, 1H), 7.08 (m, 3H), 4.11 (t, 2H), 1.94 (m, 2H), 1.66 (m, 4H), 0.95 (t, 3H)

### Example 270: Preparation of 3-(1-(6-chloro-2-oxo-1-(4-(1-propyl-1H-pyrazol-4-yl)phenyl)-1,2-dihydro-3H-imidazo[4,5-b]pyridin-3-yl)cyclopropyl)benzoic acid

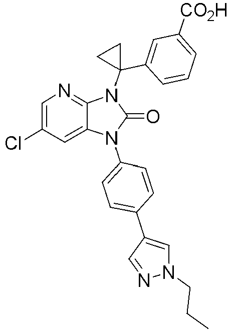

The title compound (11 mg, yield: 10%) was obtained in the same manner as in steps A and B of Example 139 by using 3-(1-(6-chloro-2-oxo-1,2-dihydro-3*H*-imidazo[4,5-*b*]pyridin-3-yl)cyclopropyl)benzoic acid methyl ester (70 mg, 0.20 mmol) obtained in Preparation Example 76 and 4-(4-bromophenyl)-1-propyl-1*H*-pyrazole (113 mg, 0.43 mmol) obtained in step A of Example 269.
¹H-NMR (400 MHz, CDCl3) δ 8.26 (s, 1H), 8.15 (m, 1H), 7.96 (d, 1H), 7.80 (m, 2H), 7.66 (s, 1H), 7.61 (d, 2H), 7.46 (d, 2H), 7.38 (t, 1H), 7.29 (d, 1H), 4.13 (t, 2H), 1.94 (m, 2H), 1.70 (m, 4H), 0.95 (t, 3H)

### Example 271: Preparation of 3-(1-(6-chloro-1-(4-(dimethylamino)phenyl)-2-oxo-1,2-dihydro-3H-imidazo[4,5-b]pyridin-3-yl)cyclopropyl)-5-fluorobenzoic acid

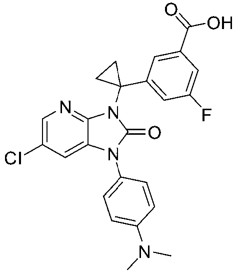

The title compound (35 mg, yield: 54%) was obtained in the same manner as in steps A and B of Example 1 by using 3-(1-(6-chloro-2-oxo-1,2-dihydro-3*H*-imidazo[4,5-*b*]pyridin-3-yl)cyclopropyl)-5-fluorobenzoic acid methyl ester (50 mg, 0.14 mmol) obtained in Preparation Example 78 and (4-dimethylamino)phenyl)boronic acid.
¹H-NMR (500 MHz, CDCl3) δ 8.18 (d, 1H), 8.07 (m, 1H), 7.67 (m, 1H), 7.55 (m, 1H), 7.31 (m, 2H), 7.25 (d, 1H), 6.83 (m, 2H), 3.04 (s, 6H), 1.81 (m, 2H), 1.73 (m, 2H)

### Example 272: Preparation of 3-(1-(6-chloro-1-(4-(dimethylcarbamoyl)phenyl)-2-oxo-1,2-dihydro-3H-imidazo[4,5-b]pyridin-3-yl)cyclopropyl)-5-fluorobenzoic acid

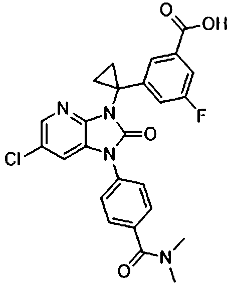

The title compound (42 mg, yield: 61%) was obtained in the same manner as in steps A and B of Example 1 by using 3-(1-(6-chloro-2-oxo-1,2-dihydro-3*H*-imidazo[4,5-*b*]pyridin-3-yl)cyclopropyl)-5-fluorobenzoic acid methyl ester (50 mg, 0.14 mmol) obtained in Preparation Example 78 and (4-dimethylcarbamoyl)phenyl)boronic acid.
¹H-NMR (500 MHz, CDCl3) δ 8.17 (d, 1H), 8.02 (s, 1H), 7.58 (m, 6H), 7.34 (d, 1H), 3.17 (s, 3H), 3.07 (s, 3H), 1.80 (m, 2H), 1.73 (m, 2H)

### Example 273: Preparation of 3-(1-(6-chloro-2-oxo-1-(4-(tetrahydro-2H-pyran-4-yl)phenyl)-1,2-dihydro-3H-imidazo[4,5-b]pyridin-3-yl)cyclopropyl)-5-fluorobenzoic acid

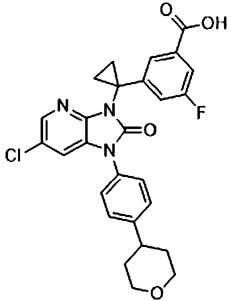

The title compound (45 mg, yield: 64%) was obtained in the same manner as in steps A and B of Example 1 by using 3-(1-(6-chloro-2-oxo-1,2-dihydro-3*H*-imidazo[4,5-*b*]pyridin-3-yl)cyclopropyl)-5-fluorobenzoic acid methyl ester (50 mg, 0.14 mmol) obtained in Preparation Example 78 and (4-(tetrahydro-2*H*-pyran-4-yl)phenyl)boronic acid.
¹H-NMR (500 MHz, CDCl3) δ 8.20 (d, 1H), 8.07 (m, 1H), 7.68 (m, 1H), 7.54 (m, 1H), 7.45 (m, 4H), 7.34 (d, 1H), 4.15 (m, 2H), 3.58 (m, 2H), 2.87 (m, 1H), 1.85 (m, 8H)

### Example 274: Preparation of 3-(1-(6-chloro-1-(4-morpholinophenyl)-2-oxo-1,2-dihydro-3H-imidazo[4,5-b]pyridin-3-yl)cyclopropyl)-5-fluorobenzoic acid

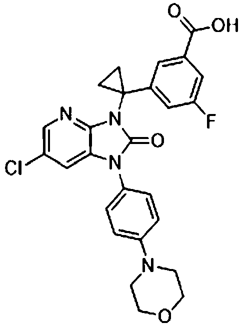

The title compound (45 mg, yield: 64%) was obtained in the same manner as in steps A and B of Example 1 by using 3-(1-(6-chloro-2-oxo-1,2-dihydro-3*H*-imidazo[4,5-*b*]pyridin-3-yl)cyclopropyl)-5-fluorobenzoic acid methyl ester (50 mg, 0.14 mmol) obtained in Preparation Example 78 and (4-morpholinophenyl)boronic acid.
¹H-NMR (500 MHz, CDCl3) δ 8.18 (m, 1H), 8.04 (s, 1H), 7.65 (m, 1H), 7.52 (m, 1H), 7.38 (m, 2H), 7.26 (d, 1H), 7.04 (d, 2H), 3.91 (m, 4H), 3.25 (m, 4H), 1.81 (m, 2H), 1.72 (m, 2H)

### Example 275: Preparation of 3-(1-(1-(4-(7-oxa-2-azaspiro[3.5]nonane-2-carbonyl)phenyl)-6-chloro-2-oxo-1,2-dihydro-3H-imidazo[4,5-b]pyridin-3-yl)cyclopropyl)-5-fluorobenzoic acid

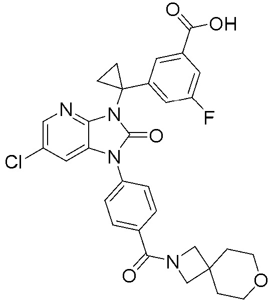

The title compound (33 mg, yield: 46%) was obtained in the same manner as in Example 56 by using 3-(1-(6-chloro-2-oxo-1,2-dihydro-3*H*-imidazo[4,5-*b*]pyridin-3-yl)cyclopropyl)-5-fluorobenzoic acid methyl ester (60 mg, 0.13 mmol) obtained in Preparation Example 78.
¹H-NMR (500 MHz, CDCl3) δ 8.17 (s, 1H), 7.99 (s, 1H), 7.84 (m, 2H), 7.60 (m, 3H), 7.44 (m, 1H), 7.36 (m, 1H), 4.01 (m 4H), 3.65 (m, 4H), 1.82 (m, 8H)

### Example 276: Preparation of 3-(1-(6-chloro-2-oxo-1-(4-(pyrrolidine-1-carbonyl)phenyl)-1,2-dihydro-3H-imidazo[4,5-b]pyridin-3-yl)cyclopropyl)-5-fluorobenzoic acid

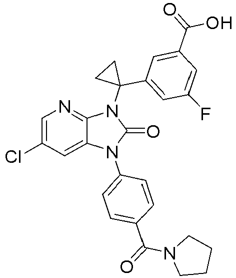

The title compound (33 mg, yield: 51%) was obtained in the same manner as in Example 56 by using 3-(1-(6-chloro-2-oxo-1,2-dihydro-3*H*-imidazo[4,5-*b*]pyridin-3-yl)cyclopropyl)-5-fluorobenzoic acid methyl ester (60 mg, 0.13 mmol) obtained in Preparation Example 78.
¹H-NMR (500 MHz, CDCl3) δ 8.13 (m, 1H), 7.98 (m, 1H), 7.70 (m, 2H), 7.56 (m, 3H), 7.40 (m, 1H), 7.30 (m, 1H), 3.69 (m, 2H), 3.48 (m, 2H), 1.95 (m, 4H), 1.69 (m, 4H)

### [Comparative Example]

### Comparative Example 1: Ziritaxestat, GLPG-1690

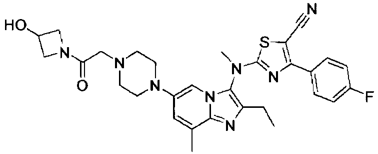

The title compound of Comparative Example 1 was purchased from Ambeed and used.

### [Experimental Example]

### Experimental Example 1: LysoPLD Activity Measurement

The inhibitory activity of the compound against the enzymatic activity of autotaxin was measured by using Recombinant Human ENPP-2/Autotaxin (Cat. No. 5255-EN, R&D systems) and substrate 14:0 LysoPC (Avanti, Cat. No. 855575P). Such a measurement method is a method of measuring the amount of choline, which is a reaction product in an autotaxin enzyme reaction, using a TOOS reagent. Depending on the activity of the autotaxin inhibitor, the amount of choline produced as a reaction product varies, which affects the final absorbance.

Specifically, in each well of the 96-well plate (Falcon, Cat. No. 351172 or corning, Cat. No. CT3370), 400 ng/mL autotaxin diluted with enzyme reaction buffer solution (50 mM Tris (pH 7.5), 50 mM CaCl₂, 0.01% Triton X-100) and various concentrations of autotaxin inhibitors were dispensed to be a volume of 50 µL and then allowed to stand at 37 °C for 30 minutes. After 30 min, 14:0 LysoPC of the substrate diluted to a concentration of 300 µM with the enzyme reaction buffer solution was dispensed into each well by 50 µL and allowed to stand at 37 °C for 90 minutes. At this time, negative control wells were dispensed with 1% DMSO, substrate, and autotaxin instead of the compound, and positive control wells were dispensed with 1% DMSO and substrate. After 90 minutes, TOOS reagent (50 mM Tris-HCl (pH 8.0), 4.5 mM MgCl₂, 2.7 mM TOOS, 4.5 mM 4-aminoantipyrine, 20 U/mL HRP, 3 U/mL choline oxidase) was dispensed into each well by 100 µL, and allowed to stand at room temperature for 15 minutes, and then the absorbance was measured at 555 nm. The absorbance which was decreased by the diluted inhibitor for each concentration was converted into a relative % value for the positive control (100% reaction inhibition) and the negative control (0% reaction inhibition), and the IC₅₀ value, which is the concentration of the inhibitor at which the enzyme activity is inhibited by 50%, was derived using PRISM (GraphPad).

The results of measuring the LysoPLD inhibitory activity of the compounds of Examples are shown in Table 2 below.
(A; IC₅₀ < 10 nM, B; 10 nM ≤ IC₅₀ < 100 nM, C; 100 nM ≤ IC₅₀ < 1 µM)

**[Table 2]**

| **Examples** | **IC₅₀** | **Example s** | **IC₅₀** | **Example s** | **IC₅₀** | **Example s** | **IC₅₀** | **Example s** | **IC₅₀** | **Examples** | **IC₅₀** |
|---|---|---|---|---|---|---|---|---|---|---|---|
| 1 | C | 47 | B | 96 | B | 142 | B | 188 | A | 235 | A |
| 2 | C | 48 | B | 97 | C | 143 | B | 189 | A | 236 | A |
| 3 | C | 49 | B | 98 | C | 144 | B | 190 | B | 237 | B |
| 4 | C | 50 | B | 99 | A | 145 | B | 191 | A | 238 | A |
| 5 | B | 51 | A | 100 | B | 146 | A | 192 | A | 239 | A |
| 7 | C | 52 | B | 101 | C | 147 | A | 193 | A | 240 | A |
| 8 | C | 53 | B | 102 | B | 148 | A | 194 | A | 241 | A |
| 9 | C | 54 | A | 103 | B | 149 | A | 195 | B | 242 | A |
| 10 | B | 55 | B | 104 | B | 150 | B | 196 | B | 243 | A |
| 11 | C | 56 | A | 105 | C | 151 | B | 197 | A | 244 | C |
| 12 | C | 57 | A | 106 | A | 152 | B | 198 | C | 245 | A |
| 13 | C | 58 | A | 107 | A | 153 | B | 199 | C | 246 | A |
| 14 | B | 59 | B | 108 | B | 154 | B | 202 | B | 247 | A |
| 15 | C | 60 | A | 109 | C | 155 | B | 203 | B | 248 | A |
| 16 | C | 61 | A | 110 | B | 156 | B | 204 | B | 249 | A |
| 17 | C | 62 | A | 111 | C | 157 | A | 205 | B | 250 | A |
| 18 | B | 63 | A | 112 | C | 158 | B | 206 | B | 251 | A |
| 19 | B | 64 | A | 113 | C | 159 | A | 207 | A | 252 | A |
| 20 | B | 65 | B | 115 | C | 160 | A | 208 | B | 253 | B |
| 21 | A | 66 | B | 116 | C | 161 | C | 209 | B | 254 | A |
| 22 | B | 67 | A | 117 | B | 162 | C | 210 | A | 255 | A |
| 23 | C | 68 | A | 118 | B | 163 | C | 211 | A | 256 | A |
| 24 | B | 69 | A | 119 | B | 164 | C | 212 | A | 257 | A |
| 25 | B | 70 | A | 120 | B | 165 | A | 213 | B | 258 | A |
| 26 | C | 71 | A | 121 | A | 166 | C | 214 | A | 259 | C |
| 27 | C | 72 | A | 122 | A | 168 | C | 215 | A | 260 | A |
| 28 | B | 75 | C | 123 | A | 169 | B | 216 | A | 261 | B |
| 29 | B | 76 | C | 124 | A | 170 | A | 217 | B | 262 | B |
| 30 | C | 77 | C | 125 | C | 171 | A | 218 | A | 263 | A |
| 31 | B | 78 | B | 126 | A | 172 | B | 219 | A | 264 | B |
| 32 | C | 79 | B | 127 | A | 173 | B | 220 | A | 265 | B |
| 33 | C | 80 | C | 128 | A | 174 | A | 221 | A | 266 | A |
| 34 | C | 81 | C | 129 | A | 175 | A | 222 | A | 267 | A |
| 35 | C | 82 | B | 130 | A | 176 | B | 223 | A | 268 | A |
| 36 | B | 83 | C | 131 | A | 177 | B | 224 | A | 269 | A |
| 37 | C | 84 | C | 132 | A | 178 | A | 225 | A | 270 | A |
| 38 | A | 86 | C | 133 | A | 179 | C | 226 | B | 271 | A |
| 39 | B | 88 | B | 134 | A | 180 | A | 227 | B | 272 | A |
| 40 | B | 89 | C | 135 | A | 181 | A | 228 | B | 273 | B |
| 41 | B | 90 | C | 136 | A | 182 | A | 229 | A | 274 | A |
| 42 | C | 91 | C | 137 | A | 183 | A | 230 | B | 275 | A |
| 43 | A | 92 | B | 138 | A | 184 | B | 231 | A | 276 | A |
| 44 | B | 93 | A | 139 | A | 185 | A | 232 | A | | |
| 45 | A | 94 | B | 140 | B | 186 | A | 233 | B | | |
| 46 | C | 95 | B | 141 | B | 187 | A | 234 | A | **Comparative Example 1** | **C** |

### Abbreviation

AN: acetonitrile
BOC₂O: di-tert-butyl dicarbonate
CDI: 1,1'-carbonyldiimidazole
Cs₂CO₃: cesium carbonate
DCM: dichloromethane
DIPEA: N,N-diisopropylethylamine
DMF: N,N-dimethylformamide
DMEDA: 1,2-dimethylethylenediamine
DMSO: dimethyl sulfoxide
EtOAc: ethyl acetate
EtOH: ethyl alcohol
HATU: 1-[bis(dimethylamino)methylene]-1*H*-1,2,3-triazolo[4,5-*b*]pyridinium 3-oxid hexafluorophosphate
K₂CO₃: potassium carbonate
KOtBu: potassium tert-butoxide
LiOH: lithium hydroxide
MeOH: methyl alcohol
MgSO₄: magnesium sulfate
MTBE: tert-butyl methyl ether
NaOH: sodium hydroxide
Na₂SO₄: sodium sulfate
Pd₂(dba)₃: tris(dibenzylideneacetone)dipalladium
pyBOP: (benzotriazol-1-yloxy)tripyrrolidinophosphonium hexafluorophosphate
TEA: triethylamine
THF: tetrahydrofuran
TFA: trifluoroacetic acid
Xantphos: 4,5-bis(diphenylphosphino)-9,9-dimethylxanthene
MPLC: medium pressure liquid chromatography (teledyne ISCO^{®}; RediSep^{®} Silica gel Flash Column, 4-220 g)

Although the preferred embodiments of the present invention have been exemplarily described, the scope of the present invention is not limited to the specific embodiments as described above. Therefore, it will be apparent to those skilled in the art that modifications and variations can be made without departing from the spirit and scope of the invention as defined by the appended claims.

## Claims

1. A compound having a chemical structure of Formula 1 below or a pharmaceutically acceptable salt thereof: 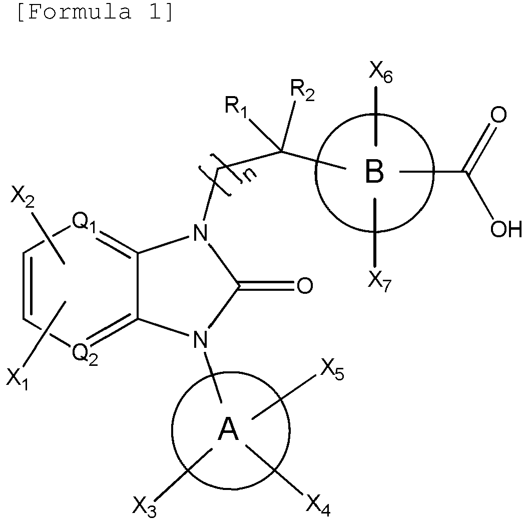 wherein, in Formula 1 above,
Q₁ and Q₂ are each independently any one selected from the group consisting of: carbon; and nitrogen,
X₁ and X₂ are each independently any one selected from the group consisting of: hydrogen; halogen; substituted or unsubstituted alkyl; substituted or unsubstituted cycloalkyl; and substituted or unsubstituted alkoxy,
A is aryl; or heteroaryl,
X₃, X₄, and X₅ are each independently any one selected from the group consisting of: hydrogen; halogen; hydroxy; oxo; carboxy; substituted or unsubstituted alkoxy; substituted or unsubstituted thio; substituted or unsubstituted amino; substituted or unsubstituted carbamoyl; substituted or unsubstituted sulfonyl; substituted or unsubstituted alkyl; substituted or unsubstituted cycloalkyl; substituted or unsubstituted tricycloalkyl; substituted or unsubstituted heterocycloalkyl; substituted or unsubstituted oxoheterocycloalkyl; substituted or unsubstituted heterobicycloalkyl; substituted or unsubstituted cycloalkenyl; substituted or unsubstituted heterocycloalkenyl; substituted or unsubstituted oxaazabicycloalkyl; substituted or unsubstituted aryl; substituted or unsubstituted heteroaryl; substituted or unsubstituted heterocycloalkylcarbonyl; and substituted or unsubstituted heterobicycloalkylcarbonyl,
the substituted functional groups in X₃, X₄, and X₅ above are each independently substituted with at least one selected from the group consisting of: halogen; hydroxy; alkoxy; alkoxycarbonyl; sulfonyl; alkylsulfonyl; haloalkylsulfonyl; oxo; alkyl; haloalkyl; hydroxyalkyl; cycloalkyl; halocycloalkyl; heterocycloalkyl; alkyloxoheterocycloalkyl; aryl; heteroaryl; haloalkylheteroaryl; acetyl; alkylcarbonyl; haloalkylcarbonyl; cycloalkylcarbonyl; halocycloalkylcarbonyl; heterocycloalkylcarbonyl; haloheterocycloalkylcarbonyl; hydroxyheterocycloalkylcarbonyl;
alkylheterocycloalkylcarbonyl; alkylamino; cycloalkylamino; cycloalkylalkylamino; and alkylaminocarbonyl,
n is an integer of 0 to 3,
R₁ and R₂ are each independently any one selected from the group consisting of: hydrogen; a substituted or unsubstituted alkyl group; and a substituted or unsubstituted cycloalkyl group, or R₁ and R₂ above are linked to each other to form a substituted or unsubstituted hydrocarbon ring,
B is aryl; or heteroaryl,
X₆ and X₇ are each independently any one selected from the group consisting of: hydrogen; halogen; hydroxy; oxo; substituted or unsubstituted alkoxy; substituted or unsubstituted amino; substituted or unsubstituted alkyl; and substituted or unsubstituted cycloalkyl, and
the substituted functional groups in X₁, X₂, X₆, X₇, R₁, and R₂ above are substituted with at least one selected from the group consisting of halogen, alkyl, hydroxyalkyl, alkoxyalkyl, haloalkyl, cycloalkyl, heterocycloalkyl, -OR₃, - (CO)-R₃, -(CO)-OR₃, alkylamino, and oxo, wherein R₃ is hydrogen or alkyl.

2. The compound or pharmaceutically acceptable salt thereof of claim 1, wherein both Q₁ and Q₂ are carbon, or either Q₁ or Q₂ is nitrogen,
X₁ and X₂ above are each independently any one selected from the group consisting of: hydrogen; halogen; alkyl unsubstituted or substituted with halogen; cycloalkyl unsubstituted or substituted with halogen; and alkoxy unsubstituted or substituted with halogen,
n is an integer of 0 to 1,
R₁ and R₂ above are each independently hydrogen; or a substituted or unsubstituted alkyl group; or R₁ and R₂ above are linked to each other to form any one selected from the group consisting of: substituted or unsubstituted cycloalkyl; substituted or unsubstituted heterocycloalkyl; substituted or unsubstituted aryl; and substituted or unsubstituted heterocycloaryl,
B is phenyl; or heteroaryl comprising at least one selected from the group consisting of N and O, and
X₆ and X₇ are each independently any one selected from the group consisting of: hydrogen; halogen; hydroxy; oxo; and substituted or unsubstituted alkyl.

3. The compound or pharmaceutically acceptable salt thereof of claim 2, wherein X₁ and X₂ above are each independently any one selected from the group consisting of: hydrogen; halogen; alkyl unsubstituted or substituted with halogen; unsubstituted cycloalkyl; and alkoxy substituted with halogen,
R₁ and R₂ above are each independently hydrogen; or a substituted or unsubstituted alkyl group having 1 to 6 carbon atoms, or R₁ and R₂ above are linked to each other to form substituted or unsubstituted cycloalkyl,
B above is any one selected from the group consisting of: phenyl; oxazole; and isoxazole, and
X₆ and X₇ above are each independently hydrogen or halogen.

4. The compound or pharmaceutically acceptable salt thereof of claim 3, wherein X₁ and X₂ above are each independently any one selected from the group consisting of: hydrogen; halogen; alkyl having 1 to 3 carbon atoms unsubstituted or substituted with halogen; unsubstituted cycloalkyl having 3 to 6 carbon atoms; and alkoxy having 1 to 3 carbon atoms substituted with halogen, and
R₁ and R₂ above are each independently hydrogen; or methyl; or R₁ and R₂ above are linked to each other to form cycloalkyl having 3 to 6 carbon atoms.

5. The compound or pharmaceutically acceptable salt thereof of claim 4, wherein A above is any one selected from the group consisting of: phenyl; benzodioxolyl; pyridinyl; pyrimidinyl; pyrazolyl; dihydroindenyl; isoindolinyl; benzothiazolyl; dihydroisobenzofuranyl; and dihydrobenzoxazinyl, and
X₃, X₄, and X₅ above are each independently any one selected from the group consisting of: hydrogen; halogen; hydroxy; oxo; carboxy; substituted or unsubstituted alkoxy; alkylthio; substituted or unsubstituted amino; substituted or unsubstituted carbamoyl; substituted or unsubstituted sulfonyl; substituted or unsubstituted alkyl; substituted or unsubstituted cycloalkyl; substituted or unsubstituted tricycloalkyl; substituted or unsubstituted morpholino; substituted or unsubstituted pyrrolidinyl; substituted or unsubstituted azetidinyl; substituted or unsubstituted piperazinyl; substituted or unsubstituted piperidinyl; substituted or unsubstituted oxazepanyl; substituted or unsubstituted oxetanyl; substituted or unsubstituted tetrahydrofuranyl; substituted or unsubstituted tetrahydropyranyl; substituted or unsubstituted oxopiperidinyl; substituted or unsubstituted oxooxazolidinyl; substituted or unsubstituted oxooxaazaspiroalkanyl; substituted or unsubstituted azaspiroalkanyl; substituted or unsubstituted oxaazaspiroalkanyl; substituted or unsubstituted diazaspiroalkanyl; substituted or unsubstituted cycloalkenyl; substituted or unsubstituted dihydropyranyl; substituted or unsubstituted dihydrofuranyl; substituted or unsubstituted oxaazabicycloalkyl; substituted or unsubstituted phenyl; substituted or unsubstituted oxadiazolyl; substituted or unsubstituted pyrazolyl; substituted or unsubstituted pyrrolidinecarbonyl; substituted or unsubstituted piperazinecarbonyl; substituted or unsubstituted piperidinecarbonyl; substituted or unsubstituted morpholinecarbonyl; substituted or unsubstituted azetidinecarbonyl; and substituted or unsubstituted oxaazaspiroalkanecarbonyl.

6. The compound or pharmaceutically acceptable salt thereof of claim 5, wherein A above is any one selected from the group consisting of: phenyl; benzo[*d*][1,3]dioxolyl; pyridin-2-yl; pyridin-3-yl; pyrimidin-5-yl; 1*H*-pyrazol-4-yl; 2,3-dihydro-1*H*-inden-5-yl; isoindolin-5-yl; benzo[d]thiazol-2-yl; 1,3-dihydroisobenzofuran-5-yl; and 3,4-dihydro-2*H-*benzo[*b*][1,4]oxazin-7-yl, and
X₃, X₄, and X₅ are each independently any one selected from the group consisting of hydrogen; halogen; hydroxy; oxo; carboxy; methoxy; ethoxy; propoxy; isopropoxy; cyclobutylmethoxy; cyclopropylmethoxy; methylthio; dimethylamino; isobutyrylamido; methylsulfonamido; (2,2,2-trifluoroethyl)sulfonamido; methylcarbamoyl; dimethylcarbamoyl; (2,2,2-trifluoroethyl)carbamoyl; methylsulfonyl; morpholinosulfonyl; piperidin-1-ylsulfonyl; pyrrolidin-1-ylsulfonyl; N,N-dimethylsulfamoyl; N-cyclopropylsulfamoyl; N-(cyclobutylmethyl)sulfamoyl; methyl; propyl; isopropyl; tert-butyl; butyl; 2-hydroxypropan-2-yl; trifluoromethyl; 2,2,2-trifluoroethyl; 2-hydroxy-2-methylpropyl; 2-fluoro-2-methylpropyl; 1-hydroxy-2-methylpropan-2-yl; 1-fluoro-2-methylpropan-2-yl; (2-oxopyrrolidin-1-yl)methyl; ((3-(trifluoromethyl)-1*H*-pyrazol-4-yl)methyl; (4-(trifluoromethyl)-1*H*-imidazol-1-yl)methyl; (3-methyl-2-oxo-imidazolin-1-yl)methyl; oxetan-3-ylmethyl; 2-(dimethylamino)-2-oxoethyl; 2-oxo-2-(pyrrolidin-1-yl)ethyl; morpholinomethyl; 2-morpholino-2-oxoethyl; 2-(4,4-difluoropiperidin-1-yl)-2-oxoethyl; 2-(3-hydroxyazetidin-1-yl)-2-oxoethyl; 2-(2-methylmorpholino)-2-oxoethyl; 2-(3-methylmorpholino)-2-oxoethyl; cyclopropylmethyl; cyclobutylmethyl; cyclopropyl; 1-(hydroxymethyl)cyclopropyl; 1-(hydroxymethyl)cyclobutyl; 1-hydroxycyclobutyl; cyclohexyl; (3r,5r,7r)-adamantan-1-yl; morpholino; 3-methyloxetan-3-yl; 3-hydroxyoxetan-3-yl; tetrahydro-2H-pyran-4-yl; tetrahydrofuran-3-yl; 2-oxo-piperidin-1-yl; pyrrolidin-1-yl; 3-hydroxyazetidin-1-yl; 3,3-difluoroazetidin-1-yl; 3,3-difluoropyrrolidin-1-yl; 4-methylpiperazin-1-yl; 4,4-difluoropiperidin-1-yl; 4-(cyclopropanecarbonyl)piperazin-1-yl; 4-(cyclobutanecarbonyl)piperazin-1-yl; 4-(isopropoxycarbonyl)piperazin-1-yl; 4-(4,4-difluorocyclohexane-1-carbonyl)piperazin-1-yl; 1-(oxetan-3-yl)piperidin-4-yl; 1-(cyclopropanecarbonyl)piperidin-4-yl; 1-(cyclobutanecarbonyl)piperidin-4-yl; 1-propionylpiperidin-4-yl; 4-propionylpiperazin-1-yl; 3,6-dihydro-2*H*-pyran-4-yl; 2,5-dihydrofuran-3-yl; 2-azaspiro[3.3]heptan-2-yl; 7-oxa-2-azaspiro[3.5]nonan-2-yl; 2-oxa-6-azaspiro[3.3]heptan-6-yl; 2-oxa-5-azabicyclo[2.2.1]heptan-5-yl; 8-oxa-3-azabicyclo[3.2.1]octan-3-yl; 1,4-oxazepan-4-yl; 7-acetyl-2,7-diazaspiro[3.5]nonan-2-yl; 7-(*tert-*butoxycarbonyl)-2,7-diazaspiro[3.5]nonan-2-yl; 4,4-dimethyl-2-oxo-oxazolidin-3-yl; 5-oxo-6-oxa-4-azaspiro[2.4]heptan-4-yl; cyclohexen-1-yl; 4-methyl-cyclohexen-1-yl; 4',4'-dimethyl-cyclohexen-1-yl; phenyl; chlorophenyl; 5-cyclopropyl-1,3,4-oxadiazol-2-yl; 5-cyclopentyl-1,3,4-oxadiazol-2-yl; 5-cyclohexyl-1,3,4-oxadiazol-2-yl; 5-cycloheptyl-1,3,4-oxadiazol-2-yl; 5-(tetrahydro-2*H*-pyran-4-yl)-1,3,4-oxadiazol-2-yl; 5-(4,4-difluorocyclohexyl)-1,3,4-oxadiazol-2-yl; 1-propyl-1*H-*pyrazol-4-yl; piperidine-1-carbonyl; morpholine-4-carbonyl; 3-methylmorpholine-4-carbonyl; pyrrolidine-1-carbonyl; azetidine-1-carbonyl; 3-hydroxyazetidine-1-carbonyl; 3,3-difluoroazetidine-1-carbonyl; 3-methoxypyrrolidine-1-carbonyl; 3,3-difluoropyrrolidine-1-carbonyl; 4-methylpiperazine-1-carbonyl; 4-hydroxypiperidine-1-carbonyl; 4-methoxypiperidine-1-carbonyl; 4,4-difluoropiperidine-1-carbonyl; 7-oxa-2-azaspiro[3.5]nonane-2-carbonyl; and 2-oxa-6-azaspiro[3.3]heptane-6-carbonyl.

7. The compound or pharmaceutically acceptable salt thereof of claim 6, wherein the compound having the chemical structure of Formula 1 above is at least one selected from the group consisting of compounds listed in Table 3 below:
**[Table 3]**
| **Number** | **Name** |
|---|---|
| 1 | 3-((5-chloro-2-oxo-3-phenyl-2,3-dihydro-*1H*-benzo[*d*]imidazol-1-yl)methyl)benzoic acid |
| 2 | 3-((5-chloro-2-oxo-3-(4-propylphenyl)-2,3-dihydro-1*H*- |
| | benzo[*d*]imidazol-1-yl)methyl)benzoic acid |
| 3 | 3-((5-chloro-3-(4-fluorophenyl)-2-oxo-2,3-dihydro-1*H-*benzo[*d*]imidazol-1-yl)methyl)benzoic acid |
| 4 | 3-((5-chloro-2-oxo-3-(4-(trifluoromethyl)phenyl)-2,3-dihydro-*1H*-benzo[*d*]imidazol-1-yl)methyl)benzoic acid |
| 5 | 3-((5-chloro-3-(4-methoxyphenyl)-2-oxo-2,3-dihydro-1*H-*benzo[*d*]imidazol-1-yl)methyl)benzoic acid |
| 6 | 3-((3-([1,1'-biphenyl]-3-yl)-5-chloro-2-oxo-2,3-dihydro-*1H-*benzo[*d*]imidazol-1-yl)methyl)benzoic acid |
| 7 | 3-((5-chloro-3-(4'-chloro-[1,1'-biphenyl]-4-yl)-2-oxo-2,3-dihydro-*1H*-benzo[*d*]imidazol-1-yl)methyl)benzoic acid |
| 8 | 3-((5-chloro-2-oxo-3-(*p*-tolyl)-2,3-dihydro-*1H*-benzo[*d*]imidazol-1-yl)methyl)benzoic acid |
| 9 | 3-((3-(benzo[*d*][1,3]dioxol-5-yl)-5-chloro-2-oxo-2,3-dihydro-*1H-*benzo[*d*]imidazol-1-yl)methyl)benzoic acid |
| 10 | 3-((5-chloro-3-(4-(dimethylamino)phenyl)-2-oxo-2,3-dihydro-*1H-*benzo[*d*]imidazol-1-yl)methyl)benzoic acid |
| 11 | 3-((5-chloro-3-(4-isobutylamidophenyl)-2-oxo-2,3-dihydro-*1H-*benzo[*d*]imidazol-1-yl)methyl)benzoic acid |
| 12 | 3-((3-(4-(*tert*-butyl)phenyl)-5-chloro-2-oxo-2,3-dihydro-*1H-*benzo[*d*]imidazol-1-yl)methyl)benzoic acid |
| 13 | 3-((5-chloro-3-(4-hydroxyphenyl)-2-oxo-2,3-dihydro-*1H-*benzo[*d*]imidazol-1-yl)methyl)benzoic acid |
| 14 | 3-((5-chloro-3-(4-isopropoxyphenyl)-2-oxo-2,3-dihydro-*1H-*benzo[*d*]imidazol-1-yl)methyl)benzoic acid |
| 15 | 3-((3-([1,1'-biphenyl]-4-yl)-5-chloro-2-oxo-2,3-dihydro-*1H-*benzo[*d*]imidazol-1-yl)methyl)benzoic acid |
| 16 | 3-((3-(4-butylphenyl)-5-chloro-2-oxo-2,3-dihydro-*1H-*benzo[*d*]imidazol-1-yl)methyl)benzoic acid |
| 17 | 3-((5-chloro-3-(4-isopropylphenyl)-2-oxo-2,3-dihydro-*1H-*benzo[*d*]imidazol-1-yl)methyl)benzoic acid |
| 18 | 3-((5-chloro-3-(4-(2-hydroxypropan-2-yl)phenyl)-2-oxo-2,3-dihydro-*1H*-benzo[*d*]imidazol-1-yl)methyl)benzoic acid |
| 19 | 3-((5-chloro-3-(4-(methylsulfonamido)phenyl)-2-oxo-2,3-dihydro-*1H*-benzo[*d*]imidazol-1-yl)methyl)benzoic acid |
| 20 | 3-((5-chloro-3-(4-ethoxyphenyl)-2-oxo-2,3-dihydro-*1H-*benzo[*d*]imidazol-1-yl)methyl)benzoic acid |
| 21 | 3-((5-chloro-3-(4-(dimethylcarbamoyl)phenyl)-2-oxo-2,3-dihydro-*1H*-benzo[*d*]imidazol-1-yl)methyl)benzoic acid |
| 22 | 3-((5-chloro-3-(4-(methylcarbamoyl)phenyl)-2-oxo-2,3-dihydro-*1H*-benzo[*d*]imidazol-1-yl)methyl)benzoic acid |
| 23 | 3-((5-chloro-2-oxo-3-(4-((2,2,2-trifluoroethyl)carbamoyl)phenyl)-2,3-dihydro-*1H-*benzo[*d*]imidazol-1-yl)methyl)benzoic acid |
| 24 | 3-((5-chloro-4-fluoro-3-(4-morpholinophenyl)-2-oxo-2,3-dihydro-*1H*-benzo[*d*]imidazol-1-yl)methyl)benzoic acid |
| 25 | 3-((5-chloro-3-(4-(dimethylamino)phenyl)-4-fluoro-2-oxo-2,3-dihydro-*1H*-benzo[*d*]imidazol-1-yl)methyl)benzoic acid |
| 26 | 3-((5-chloro-2-oxo-3-(4-((2,2,2-trifluoroethyl)sulfonamido)phenyl)-2,3-dihydro-*1H-*benzo[*d*]imidazol-1-yl)methyl)benzoic acid |
| 27 | 3-((5-chloro-3-(4-(cyclobutylmethoxy)phenyl)-2-oxo-2,3-dihydro-*1H*-benzo[*d*]imidazol-1-yl)methyl)benzoic acid |
| 28 | 3-((5-chloro-2-oxo-3-(4-(2-oxo-piperidin-1-yl)phenyl)-2,3-dihydro-*1H*-benzo[*d*]imidazol-1-yl)methyl)benzoic acid |
| 29 | 3-((5-chloro-2-oxo-3-(4-(pyrrolidin-1-yl)phenyl)-2,3-dihydro-*1H*-benzo[*d*]imidazol-1-yl)methyl)benzoic acid |
| 30 | 3-((5-chloro-3-(4-(cyclobutylmethoxy)-3-fluorophenyl)-2-oxo-2,3-dihydro-1*H*-benzo[*d*]imidazol-1-yl)methyl)benzoic acid |
| 31 | 3-(1-(5-chloro-2-oxo-3-(4-propylphenyl)-2,3-dihydro-*1H-*benzo[*d*]imidazol-1-yl)cyclopropyl)benzoic acid |
| 32 | 3-((5-chloro-2-oxo-3-phenyl-2,3-dihydro-1*H*-benzo[*d*]imidazol-1-yl)methyl)-4-fluorobenzoic acid |
| 33 | 3-((5-chloro-2-oxo-3-phenyl-2,3-dihydro-*1H*-benzo[*d*]imidazol-1-yl)methyl)-2-fluorobenzoic acid |
| 34 | 3-((5-chloro-2-oxo-3-phenyl-2,3-dihydro-*1H*-benzo[*d*]imidazol-1-yl)methyl)-5-fluorobenzoic acid |
| 35 | 5-((5-chloro-2-oxo-3-phenyl-2,3-dihydro-*1H*-benzo[*d*]imidazol-1-yl)methyl)-2-fluorobenzoic acid |
| 36 | 3-((5-chloro-3-(4-(dimethylcarbamoyl)-3-fluorophenyl)-2-oxo-2,3-dihydro-*1H*-benzo[*d*]imidazol-1-yl)methyl)benzoic acid |
| 37 | 3-((6-chloro-3-(4-(dimethylamino)phenyl)-4-fluoro-2-oxo-2,3-dihydro-*1H*-benzo[*d*]imidazol-1-yl)methyl)benzoic acid |
| 38 | 3-((5-chloro-3-(4-(dimethylcarbamoyl)phenyl)-2-oxo-2,3-dihydro-*1H*-benzo[*d*]imidazol-1-yl)methyl)-2-fluorobenzoic acid |
| 39 | 3-((5-chloro-3-(4-(dimethylamino)phenyl)-2-oxo-2,3-dihydro-*1H-*benzo[*d*]imidazol-1-yl)methyl)-2-fluorobenzoic acid |
| 40 | 3-((5-chloro-3-(4-isopropoxyphenyl)-2-oxo-2,3-dihydro-*1H-*benzo[*d*]imidazol-1-yl)methyl)-2-fluorobenzoic acid |
| 41 | 3-((5-chloro-3-(4-(methylsulfonamido)phenyl)-2-oxo-2,3-dihydro-*1H*-benzo[*d*]imidazol-1-yl)methyl)-2-fluorobenzoic acid |
| 42 | 3-((5-chloro-3-(4-(cyclopropylmethoxy)phenyl)-2-oxo-2,3-dihydro-*1H*-benzo[*d*]imidazol-1-yl)methyl)benzoic acid |
| 43 | 3-((5-chloro-2-oxo-3-(4-(piperidine-1-carbonyl)phenyl)-2,3-dihydro-*1H*-benzo[*d*]imidazol-1-yl)methyl)benzoic acid |
| 44 | 3-((5-chloro-3-(4-(morpholine-4-carbonyl)phenyl)-2-oxo-2,3-dihydro-*1H*-benzo[*d*]imidazol-1-yl)methyl)benzoic acid |
| 45 | 3-((5-chloro-2-oxo-3-(4-(pyrrolidine-1-carbonyl)phenyl)-2,3-dihydro-*1H*-benzo[*d*]imidazol-1-yl)methyl)benzoic acid |
| 46 | 4-(3-(3-carboxybenzyl)-6-chloro-2-oxo-2,3-dihydro-*1H-*benzo[*d*]imidazol-1-yl)-3-fluorobenzoic acid |
| 47 | 3-((5-chloro-3-(4-morpholinophenyl)-2-oxo-2,3-dihydro-*1H-*benzo[*d*]imidazol-1-yl)methyl)benzoic acid |
| 48 | 5-((5-chloro-4-fluoro-3-(4-morpholinophenyl)-2-oxo-2,3-dihydro-*1H*-benzo[*d*]imidazol-1-yl)methyl)-2-fluorobenzoic acid |
| 49 | 3-((5-chloro-4-fluoro-3-(4-morpholinophenyl)-2-oxo-2,3-dihydro- |
| | *1H*-benzo[*d*]imidazol-1-yl)methyl)-5-fluorobenzoic acid |
| 50 | 3-((5-chloro-4-fluoro-3-(4-morpholinophenyl)-2-oxo-2,3-dihydro-*1H*-benzo[*d*]imidazol-1-yl)methyl)-4-fluorobenzoic acid |
| 51 | 3-((5-chloro-4-fluoro-3-(4-morpholinophenyl)-2-oxo-2,3-dihydro-*1H*-benzo[*d*]imidazol-1-yl)methyl)-2-fluorobenzoic acid |
| 52 | 6-((5-chloro-4-fluoro-3-(4-morpholinophenyl)-2-oxo-2,3-dihydro-*1H*-benzo[*d*]imidazol-1-yl)methyl)picolinic acid |
| 53 | 3-((5-chloro-4-fluoro-3-(4-(3-hydroxyazetidin-1-yl)phenyl)-2-oxo-2,3-dihydro-*1H*-benzo[*d*]imidazol-1-yl)methyl)benzoic acid |
| 54 | 3-((5-chloro-3-(4-(dimethylcarbamoyl)phenyl)-7-fluoro-2-oxo-2,3-dihydro-*1H*-benzo[*d*]imidazol-1-yl)methyl)benzoic acid |
| 55 | 3-((5-chloro-3-(4-(methylthio)phenyl)-2-oxo-2,3-dihydro-*1H-*benzo[*d*]imidazol-1-yl)methyl)-2-fluorobenzoic acid |
| 56 | 3-((5-chloro-2-oxo-3-(4-(pyrrolidine-1-carbonyl)phenyl)-2,3-dihydro-*1H*-benzo[*d*]imidazol-1-yl)methyl)-2-fluorobenzoic acid |
| 57 | 3-((5-chloro-2-oxo-3-(4-(piperidine-1-carbonyl)phenyl)-2,3-dihydro-*1H*-benzo[*d*]imidazol-1-yl)methyl)-2-fluorobenzoic acid |
| 58 | 3-((3-(4-(azetidine-1-carbonyl)phenyl)-5-chloro-2-oxo-2,3-dihydro-*1H*-benzo[*d*]imidazol-1-yl)methyl)benzoic acid |
| 59 | 3-((5-chloro-3-(4-(3-methoxypyrrolidine-1-carbonyl)phenyl)-2-oxo-2,3-dihydro-*1H*-benzo[*d*]imidazol-1-yl)methyl)benzoic acid |
| 60 | 3-((5-chloro-3-(4-(4-methylpiperazine-1-carbonyl)phenyl)-2-oxo-2,3-dihydro-*1H*-benzo[*d*]imidazol-1-yl)methyl)benzoic acid |
| 61 | 3-((5-chloro-3-(4-(4-hydroxypiperidine-1-carbonyl)phenyl)-2-oxo-2,3-dihydro-*1H*-benzo[*d*]imidazol-1-yl)methyl)benzoic acid |
| 62 | 3-((5-chloro-3-(4-(4-methoxypiperidine-1-carbonyl)phenyl)-2-oxo-2,3-dihydro-*1H*-benzo[*d*]imidazol-1-yl)methyl)benzoic acid |
| 63 | 3-(1-(5-chloro-2-oxo-3-(4-(pyrrolidine-1-carbonyl)phenyl)-2,3-dihydro-*1H*-benzo[*d*]imidazol-1-yl)cyclopropyl)benzoic acid |
| 64 | 3-(1-(5-chloro-2-oxo-3-(4-(piperidine-1-carbonyl)phenyl)-2,3-dihydro-*1H*-benzo[*d*]imidazol-1-yl)cyclopropyl)benzoic acid |
| 65 | 3-((5-chloro-3-(4-(dimethylamino)phenyl)-7-fluoro-2-oxo-2,3-dihydro-*1H*-benzo[*d*]imidazol-1-yl)methyl)-5-fluorobenzoic acid |
| 66 | 3-((5-chloro-3-(4-(dimethylamino)phenyl)-7-fluoro-2-oxo-2,3-dihydro-*1H*-benzo[*d*]imidazol-1-yl)methyl)benzoic acid |
| 67 | 3-(1-(5-chloro-2-oxo-3-(4-(pyrrolidine-1-carbonyl)phenyl)-2,3-dihydro-*1H*-benzo[*d*]imidazol-1-yl)cyclopropyl)-2-fluorobenzoic acid |
| 68 | 3-(1-(5-chloro-2-oxo-3-(4-(piperidine-1-carbonyl)phenyl)-2,3-dihydro-*1H*-benzo[*d*]imidazol-1-yl)cyclopropyl)-2-fluorobenzoic acid |
| 69 | 3-(1-(5-chloro-3-(4-(dimethylamino)phenyl)-2-oxo-2,3-dihydro-*1H*-benzo[*d*]imidazol-1-yl)cyclopropyl)benzoic acid |
| 70 | 3-(1-(5-chloro-3-(4-(dimethylamino)phenyl)-2-oxo-2,3-dihydro-*1H*-benzo[*d*]imidazol-1-yl)cyclopropyl)-2-fluorobenzoic acid |
| 71 | 3-(1-(5-chloro-3-(4-(dimethylcarbamoyl)phenyl)-2-oxo-2,3-dihydro-*1H*-benzo[*d*]imidazol-1-yl)cyclopropyl)benzoic acid |
| 72 | 3-(1-(5-chloro-3-(4-(dimethylcarbamoyl)phenyl)-2-oxo-2,3-dihydro-*1H*-benzo[*d*]imidazol-1-yl)cyclopropyl)-2-fluorobenzoic acid |
| 73 | 3-((5-chloro-2-oxo-3-(pyridin-3-yl)-2,3-dihydro-*1H-*benzo[*d*]imidazol-1-yl)methyl)benzoic acid |
| 74 | 3-((5-chloro-3-(6-fluoropyridin-3-yl)-2-oxo-2,3-dihydro-*1H-*benzo[*d*]imidazol-1-yl)methyl)benzoic acid |
| 75 | 3-((5-chloro-3-(6-ethoxypyridin-3-yl)-2-oxo-2,3-dihydro-*1H-*benzo[*d*]imidazol-1-yl)methyl)benzoic acid |
| 76 | 3-((5-chloro-2-oxo-3-(6-propoxypyridin-3-yl)-2,3-dihydro-*1H-*benzo[d]imidazol-1-yl)methyl)benzoic acid |
| 77 | 3-((5-chloro-3-(6-(3-hydroxyazetidin-1-yl)pyridin-3-yl)-2-oxo-2,3-dihydro-*1H*-benzo[*d*]imidazol-1-yl)methyl)benzoic acid |
| 78 | 3-((5-chloro-3-(6-morpholinopyridin-3-yl)-2-oxo-2,3-dihydro-*1H-*benzo[*d*]imidazol-1-yl)methyl)benzoic acid |
| 79 | 3-((5-chloro-3-(6-(dimethylamino)pyridin-3-yl)-2-oxo-2,3-dihydro-*1H*-benzo[*d*]imidazol-1-yl)methyl)benzoic acid |
| 80 | 3-((5-chloro-3-(6-(3,3-difluoropyrrolidin-1-yl)pyridin-3-yl)-2-oxo-2,3-dihydro-*1H*-benzo[*d*]imidazol-1-yl)methyl)benzoic acid |
| 81 | 3-((5-chloro-3-(6-(4-methylpiperazin-1-yl)pyridin-3-yl)-2-oxo-2,3-dihydro-*1H*-benzo[*d*]imidazol-1-yl)methyl)benzoic acid |
| 82 | 3-((5-chloro-3-(6-(4,4-difluoropiperidin-1-yl)pyridin-3-yl)-2-oxo-2,3-dihydro-*1H*-benzo[*d*]imidazol-1-yl)methyl)benzoic acid |
| 83 | 3-((5-chloro-3-(2-morpholinopyrimidin-5-yl)-2-oxo-2,3-dihydro-*1H*-benzo[*d*]imidazol-1-yl)methyl)benzoic acid |
| 84 | 3-((5-chloro-2-oxo-3-(1-propyl-*1H*-pyrazol-4-yl)-2,3-dihydro-*1H-*benzo[d]imidazol-1-yl)methyl)benzoic acid |
| 85 | 3-((5-cyclopropyl-2-oxo-3-(1-(tetrahydro-2*H*-pyran-4-yl)-*1H-*pyrazol-4-yl)-2,3-dihydro-*1H*-benzo[*d*]imidazol-1-yl)methyl)benzoic acid |
| 86 | 3-((5-chloro-2-oxo-3-(1-(tetrahydro-2*H*-pyran-4-yl)-*1H*-pyrazol-4-yl)-2,3-dihydro-*1H*-benzo[*d*]imidazol-1-yl)methyl)benzoic acid |
| 87 | 3-((5-chloro-2-oxo-3-(1-(2,2,2-trifluoroethyl)-*1H*-pyrazol-4-yl)-2,3-dihydro-*1H*-benzo[*d*]imidazol-1-yl)methyl)benzoic acid |
| 88 | 3-((5-chloro-4-fluoro-2-oxo-3-(1-propyl-*1H*-pyrazol-4-yl)-2,3-dihydro-*1H*-benzo[*d*]imidazol-1-yl)methyl)benzoic acid |
| 89 | 3-((5-chloro-3-(1-isopropyl-*1H*-pyrazol-4-yl)-2-oxo-2,3-dihydro- |
| | *1H*-benzo[*d*]imidazol-1-yl)methyl)benzoic acid |
| 90 | 3-((5-chloro-3-(1-cyclopropylmethyl-*1H*-pyrazol-4-yl)-2-oxo-2,3-dihydro-*1H*-benzo[*d*]imidazol-1-yl)methyl)benzoic acid |
| 91 | 3-((5-chloro-3-(1-cyclobutylmethyl-*1H*-pyrazol-4-yl)-2-oxo-2,3-dihydro-*1H*-benzo[*d*]imidazol-1-yl)methyl)benzoic acid |
| 92 | 3-((5-chloro-4-fluoro-2-oxo-3-(1-propyl-*1H*-pyrazol-4-yl)-2,3-dihydro-*1H*-benzo[*d*]imidazol-1-yl)methyl)-2-fluorobenzoic acid |
| 93 | 3-(1-(3-(4-(7-oxa-2-azaspiro[3.5]nonane-2-carbonyl)phenyl)-5-chloro-2-oxo-2,3-dihydro-*1H*-benzo[*d*]imidazol-1-yl)cyclopropyl)benzoic acid |
| 94 | 3-((5-chloro-3-(4-(4,4-difluoropiperidin-1-yl)phenyl)-2-oxo-2,3-dihydro-*1H*-benzo[*d*]imidazol-1-yl)methyl)benzoic acid |
| 95 | 3-((5-chloro-3-(4-(4-(cyclopropanecarbonyl)piperazin-1-yl)phenyl)-2-oxo-2,3-dihydro-*1H*-benzo[*d*]imidazol-1-yl)methyl)benzoic acid |
| 96 | 3-((5-chloro-3-(4-(4-(isopropoxycarbonyl)piperazin-1-yl)phenyl)-2-oxo-2,3-dihydro-*1H*-benzo[*d*]imidazol-1-yl)methyl)benzoic acid |
| 97 | 3-((5-chloro-3-(4-(4-methylpiperazin-1-yl)phenyl)-2-oxo-2,3-dihydro-*1H*-benzo[*d*]imidazol-1-yl)methyl)benzoic acid |
| 98 | 3-(1-(5-chloro-3-(4-cyclohexylphenyl)-2-oxo-2,3-dihydro-*1H-*benzo[*d*]imidazol-1-yl)cyclopropyl)benzoic acid |
| 99 | 3-(1-(5-chloro-2-oxo-3-(4-(tetrahydro-2H-pyran-4-yl)phenyl)-2,3-dihydro-*1H*-benzo[*d*]imidazol-1-yl)cyclopropyl)benzoic acid |
| 100 | 3-((5-chloro-3-(4-(1-(cyclopropanecarbonyl)piperidin-4-yl)phenyl)-2-oxo-2,3-dihydro-*1H*-benzo[*d*]imidazol-1-yl)methyl)benzoic acid |
| 101 | 3-((5-chloro-3-(4-(1-(cyclobutanecarbonyl)piperidin-4-yl)phenyl)-2-oxo-2,3-dihydro-*1H*-benzo[*d*]imidazol-1-yl)methyl)benzoic acid |
| 102 | 3-((5-chloro-2-oxo-3-(4-(1-propionylpiperidin-4-yl)phenyl)-2,3-dihydro-*1H*-benzo[*d*]imidazol-1-yl)methyl)benzoic acid |
| 103 | 3-((5-chloro-2-oxo-3-(4-(tetrahydro-2H-pyran-4-yl)phenyl)-2,3-dihydro-*1H*-benzo[*d*]imidazol-1-yl)methyl)benzoic acid |
| 104 | 3-((5-chloro-3-(4-(3,6-dihydro-2*H*-pyran-4-yl)phenyl)-2-oxo-2,3-dihydro-*1H*-benzo[*d*]imidazol-1-yl)methyl)benzoic acid |
| 105 | 3-((5-chloro-3-(4-(3,3-difluoropyrrolidin-1-yl)phenyl)-4-fluoro-2-oxo-2,3-dihydro-*1H*-benzo[*d*]imidazol-1-yl)methyl)benzoic acid |
| 106 | 3-(1-(5-chloro-2-oxo-3-(4-(tetrahydro-2*H*-pyran-4-yl)phenyl)-2,3-dihydro-*1H*-benzo[*d*]imidazol-1-yl)cyclopropyl)-2-fluorobenzoic acid |
| 107 | 3-(1-(3-(4-(7-oxa-2-azaspiro[3.5]nonane-2-carbonyl)phenyl)-5-chloro-2-oxo-2,3-dihydro-*1H*-benzo[*d*]imidazol-1-yl)cyclopropyl)-2-fluorobenzoic acid |
| 108 | 3-((5-chloro-2-oxo-3-(4-(tetrahydro-2*H*-pyran-4-yl)phenyl)-2,3- |
| | dihydro-1*H*-benzo[*d*]imidazol-1-yl)methyl)-2-fluorobenzoic acid |
| 109 | 3-((5-chloro-2-oxo-3-(2',3',4',5'-tetrahydro-[1,1'-biphenyl]-4-yl)-2,3-dihydro-*1H*-benzo[*d*]imidazol-1-yl)methyl)benzoic acid |
| 110 | 3-((5-chloro-3-(4-(2,5-dihydrofuran-3-yl)phenyl)-2-oxo-2,3-dihydro-*1H*-benzo[*d*]imidazol-1-yl)methyl)-2-fluorobenzoic acid |
| 111 | 3-((5-chloro-3-(4'-methyl-2',3',4',5'-tetrahydro-[1,1'-biphenyl]-4-yl)-2-oxo-2,3-dihydro-*1H*-benzo[d]imidazol-1-yl)methyl)-2-fluorobenzoic acid |
| 112 | 3-((5-chloro-3-(4',4'-dimethyl-2',3',4',5'-tetrahydro-[1,1'-biphenyl]-4-yl)-2-oxo-2,3-dihydro-*1H*-benzo[d]imidazol-1-yl)methyl)-2-fluorobenzoic acid |
| 113 | 3-(2-(5-chloro-3-(4-(dimethylcarbamoyl)phenyl)-2-oxo-2,3-dihydro-*1H*-benzo[*d*]imidazol-1-yl)ethyl)benzoic acid |
| 114 | 3-((5-chloro-3-(2,2-dimethyl-3-oxo-2,3-dihydro-*1H*-inden-5-yl)-2-oxo-2,3-dihydro-*1H*-benzo[*d*]imidazol-1-yl)methyl)benzoic acid |
| 115 | 3-((5-chloro-3-(2,2-dimethyl-1-oxo-2,3-dihydro-*1H*-inden-5-yl)-2-oxo-2,3-dihydro-*1H*-benzo[*d*]imidazol-1-yl)methyl)benzoic acid |
| 116 | 3-((5-chloro-3-(2-methyl-1-oxoisoindolin-5-yl)-2-oxo-2,3-dihydro-*1H*-benzo[*d*]imidazol-1-yl)methyl)benzoic acid |
| 117 | 3-((5-chloro-3-(2-methyl-1-oxoisoindolin-5-yl)-2-oxo-2,3-dihydro-*1H*-benzo[d]imidazol-1-yl)methyl)-2-fluorobenzoic acid |
| 118 | 3-((5-chloro-3-(4-(4,4-difluoropiperidin-1-yl)phenyl)-2-oxo-2,3-dihydro-*1H*-benzo[*d*]imidazol-1-yl)methyl)-2-fluorobenzoic acid |
| 119 | 3-((5-chloro-3-(4-(3,3-difluoropyrrolidin-1-yl)phenyl)-4-fluoro-2-oxo-2,3-dihydro-*1H*-benzo[*d*]imidazol-1-yl)methyl)-2-fluorobenzoic acid |
| 120 | 3-((5-chloro-4-fluoro-3-(4-(3-hydroxyazetidin-1-yl)phenyl)-2-oxo-2,3-dihydro-*1H*-benzo[*d*]imidazol-1-yl)methyl)-2-fluorobenzoic acid |
| 121 | 3-(1-(5-chloro-3-(4-(3,6-dihydro-*2H*-pyran-4-yl)phenyl)-2-oxo-2,3-dihydro-*1H*-benzo[*d*]imidazol-1-yl)cyclopropyl)benzoic acid |
| 122 | 3-(1-(5-chloro-3-(4-(3,6-dihydro-2*H*-pyran-4-yl)phenyl)-2-oxo-2,3-dihydro-*1H*-benzo[*d*]imidazol-1-yl)cyclopropyl)-2-fluorobenzoic acid |
| 123 | 3-(1-(5-chloro-3-(4-(2,5-dihydrofuran-3-yl)phenyl)-2-oxo-2,3-dihydro-*1H*-benzo[d]imidazol-1-yl)cyclopropyl)-2-fluorobenzoic acid |
| 124 | 3-(1-(5-chloro-3-(4-(2,5-dihydrofuran-3-yl)phenyl)-2-oxo-2,3-dihydro-*1H*-benzo[*d*]imidazol-1-yl)cyclopropyl)benzoic acid |
| 125 | 4-((5-chloro-4-fluoro-3-(4-morpholinophenyl)-2-oxo-2,3-dihydro- |
| | *1H*-benzo[d]imidazol-1-yl)methyl)benzoic acid |
| 126 | 3-(1-(5-chloro-3-(4-(morpholine-4-carbonyl)phenyl)-2-oxo-2,3-dihydro-*1H*-benzo[*d*]imidazol-1-yl)cyclopropyl)benzoic acid |
| 127 | (S)-3-(1-(5-chloro-3-(4-(3-methylmorpholine-4-carbonyl)phenyl)-2-oxo-2,3-dihydro-*1H*-benzo[d]imidazol-1-yl)cyclopropyl)benzoic acid |
| 128 | 3-(1-(5-chloro-3-(4-(3-hydroxyazetidine-1-carbonyl)phenyl)-2-oxo-2,3-dihydro-*1H*-benzo[*d*]imidazol-1-yl)cyclopropyl)benzoic acid |
| 129 | 3-(1-(5-chloro-3-(4-(3,3-difluoroazetidine-1-carbonyl)phenyl)-2-oxo-2,3-dihydro-*1H*-benzo[d]imidazol-1-yl)cyclopropyl)benzoic acid |
| 130 | 3-(1-(5-chloro-3-(4-(3,3-difluoropyrrolidine-1-carbonyl)phenyl)-2-oxo-2,3-dihydro-*1H*-benzo[*d*]imidazol-1-yl)cyclopropyl)benzoic acid |
| 131 | 3-(1-(5-chloro-3-(4-(4,4-difluoropiperidine-1-carbonyl)phenyl)-2-oxo-2,3-dihydro-*1H-*benzo[*d*]imidazol-1-yl)cyclopropyl)benzoic acid |
| 132 | 3-(1-(5-chloro-3-(4-(3,3-difluoropyrrolidine-1-carbonyl)phenyl)-2-oxo-2,3-dihydro-*1H*-benzo[*d*]imidazol-1-yl)cyclopropyl)-2-fluorobenzoic acid |
| 133 | 3-(1-(5-chloro-3-(4-(4,4-difluoropiperidine-1-carbonyl)phenyl)-2-oxo-2,3-dihydro-*1H*-benzo[*d*]imidazol-1-yl)cyclopropyl)-2-fluorobenzoic acid |
| 134 | 3-(1-(5-chloro-3-(4-(3-hydroxyazetidine-1-carbonyl)phenyl)-2-oxo-2,3-dihydro-1H-benzo[d]imidazol-1-yl)cyclopropyl)-2-fluorobenzoic acid |
| 135 | 3-(1-(3-(4-(2-oxa-6-azaspiro[3.3]heptane-6-carbonyl)phenyl)-5-chloro-2-oxo-2,3-dihydro-1*H*-benzo[*d*]imidazol-1-yl)cyclopropyl)-2-fluorobenzoic acid |
| 136 | (S)-3-(1-(5-chloro-3-(4-(3-methylmorpholine-4-carbonyl)phenyl)-2-oxo-2,3-dihydro-*1H*-benzo[*d*]imidazol-1-yl)cyclopropyl)-2-fluorobenzoic acid |
| 137 | 3-(1-(3-(4-(2-oxa-6-azaspiro[3.3]heptane-6-carbonyl)phenyl)-5-chloro-2-oxo-2,3-dihydro-*1H*-benzo[*d*]imidazol-1-yl)cyclopropyl)benzoic acid |
| 138 | 3-(1-(5-chloro-3-(4-(5-(4,4-difluorocyclohexyl)-1,3,4-oxadiazol-2-yl)phenyl)-2-oxo-2,3-dihydro-*1H-*benzo[*d*]imidazol-1-yl)cyclopropyl)benzoic acid |
| 139 | 3-(1-(5-chloro-3-(4-(2-(dimethylamino)-2-oxoethyl)phenyl)-2-oxo-2,3-dihydro-*1H*-benzo[*d*]imidazol-1-yl)cyclopropyl)benzoic acid |
| 140 | 3-((5-chloro-3-(4-(4-(cyclopropanecarbonyl)piperazin-1-yl)phenyl)-2-oxo-2,3-dihydro-*1H*-benzo[*d*]imidazol-1-yl)methyl)-2-fluorobenzoic acid |
| 141 | 3-((5-chloro-2-oxo-3-(4-(4-propionylpiperazin-1-yl)phenyl)-2,3-dihydro-*1H*-benzo[*d*]imidazol-1-yl)methyl)-2-fluorobenzoic acid |
| 142 | 3-((5-chloro-3-(4-(4-(cyclobutanecarbonyl)piperazin-1-yl)phenyl)-2-oxo-2,3-dihydro-*1H*-benzo[*d*]imidazol-1-yl)methyl)-2-fluorobenzoic acid |
| 143 | 3-((5-chloro-3-(4-(4-(4,4-difluorocyclohexane-1-carbonyl)piperazin-1-yl)phenyl)-2-oxo-2,3-dihydro-*1H-*benzo[*d*]imidazol-1-yl)methyl)-2-fluorobenzoic acid |
| 144 | 3-(1-(3-(benzo[*d*]thiazol-2-yl)-5-chloro-2-oxo-2,3-dihydro-*1H-*benzo[*d*]imidazol-1-yl)cyclopropyl)-2-fluorobenzoic acid |
| 145 | 3-(1-(5-chloro-3-(4-(morpholinomethyl)phenyl)-2-oxo-2,3-dihydro*-1H*-benzo[*d*]imidazol-1-yl)cyclopropyl)benzoic acid |
| 146 | 3-(1-(5-chloro-2-oxo-3-(4-(pyrrolidin-1-yl)phenyl)-2,3-dihydro-*1H*-benzo[*d*]imidazol-1-yl)cyclopropyl)benzoic acid |
| 147 | 3-(1-(5-chloro-3-(4-morpholinophenyl)-2-oxo-2,3-dihydro-*1H-*benzo[*d*]imidazol-1-yl)cyclopropyl)benzoic acid |
| 148 | 3-(1-(3-(4-(7-oxa-2-azaspiro[3.5]nonan-2-yl)phenyl)-5-chloro-2-oxo-2,3-dihydro-*1H*-benzo[*d*]imidazol-1-yl)cyclopropyl)benzoic acid |
| 149 | 3-(1-(3-(4-(2-oxa-6-azaspiro[3.3]heptan-6-yl)phenyl)-5-chloro-2-oxo-2,3-dihydro-*1H*-benzo[*d*]imidazol-1-yl)cyclopropyl)benzoic acid |
| 150 | 3-((5-chloro-3-(4-(1-(cyclopropanecarbonyl)piperidin-4-yl)phenyl)-4-fluoro-2-oxo-2,3-dihydro-*1H-*benzo[*d*]imidazol-1-yl)methyl)-2-fluorobenzoic acid |
| 151 | 3-((5-chloro-4-fluoro-2-oxo-3-(4-(1-propionylpiperidin-4-yl)phenyl)-2,3-dihydro-*1H*-benzo[*d*]imidazol-1-yl)methyl)-2-fluorobenzoic acid |
| 152 | 3-((5-chloro-3-(4-(1-(cyclobutanecarbonyl)piperidin-4-yl)phenyl)-4-fluoro-2-oxo-2,3-dihydro-*1H-*benzo[*d*]imidazol-1-yl)methyl)-2-fluorobenzoic acid |
| 153 | 3-(2-(5-chloro-3-(4-(dimethylamino)phenyl)-2-oxo-2,3-dihydro-*1H*-benzo[d*]*imidazol-1-yl)propan-2-yl)benzoic acid |
| 154 | 3-(2-(5-chloro-3-(4-(3-hydroxyazetidin-1-yl)phenyl)-2-oxo-2,3-dihydro-*1H-*benzo[*d*]imidazol-1-yl)propan-2-yl)benzoic acid |
| 155 | 3-(1-(5-chloro-2-oxo-3-(4-(tetrahydro-*2H*-pyran-4-yl)phenyl)-2,3-dihydro-*1H*-benzo[*d*]imidazol-1-yl)cyclobutyl)benzoic acid |
| 156 | 3-(1-(5-chloro-3-(4-(dimethylamino)phenyl)-2-oxo-2,3-dihydro-*1H*-benzo[*d*]imidazol-1-yl)cyclobutyl)benzoic acid |
| 157 | 3-(1-(5-chloro-3-(4-(dimethylcarbamoyl)phenyl)-2-oxo-2,3-dihydro-*1H*-benzo[*d*]imidazol-1-yl)cyclobutyl)benzoic acid |
| 158 | 3-(2-(5-chloro-2-oxo-3-(4-(tetrahydro-*2H-*pyran-4-yl)phenyl)-2,3-dihydro-*1H*-benzo[*d*]imidazol-1-yl)propan-2-yl)benzoic acid |
| 159 | 3-(1-(5-chloro-3-(4-(3-hydroxyazetidin-1-yl)phenyl)-2-oxo-2,3-dihydro-*1H*-benzo[*d*]imidazol-1-yl)cyclopropyl)benzoic acid |
| 160 | 3-(1-(5-chloro-3-(4-(4,4-difluoropiperidin-1-yl)phenyl)-2-oxo-2,3-dihydro-*1H*-benzo[*d*]imidazol-1-yl)cyclopropyl)benzoic acid |
| 161 | 3-((6-chloro-1-(4-(dimethylamino)phenyl)-2-oxo-1,2-dihydro-*3H-*imidazo[4,5-*b*]pyridin-3-yl)methyl)benzoic acid |
| 162 | 3-((6-chloro-1-(4-morpholinophenyl)-2-oxo-1,2-dihydro-*3H-*imidazo[4,5-*b*]pyridin-3-yl)methyl)benzoic acid |
| 163 | 3-((5-chloro-3-(5-cyclopropylpyridin-2-yl)-2-oxo-2,3-dihydro-*1H*-benzo[*d*]imidazol-1-yl)methyl)benzoic acid |
| 164 | 3-((5-chloro-3-(5-(dimethylamino)pyridin-2-yl)-2-oxo-2,3-dihydro-*1H*-benzo[*d*]imidazol-1-yl)methyl)benzoic acid |
| 165 | 3-(1-(5-chloro-3-(6-(3,3-difluoropyrrolidin-1-yl)pyridin-3-yl)-2-oxo-2,3-dihydro-*1H*-benzo[*d*]imidazol-1-yl)cyclopropyl)benzoic acid |
| 166 | 3-((5-chloro-3-(6-(3,3-difluoropyrrolidin-1-yl)pyridin-3-yl)-2-oxo-2,3-dihydro-*1H*-benzo[*d*]imidazol-1-yl)methyl)-2-fluorobenzoic acid |
| 167 | 3-((3-(4-(dimethylamino)phenyl)-5-methyl-2-oxo-2,3-dihydro-*1H-*imidazo[4,5-*b*]pyridin-1-yl)methyl)-2-fluorobenzoic acid |
| 168 | 3-((3-(4-(dimethylamino)phenyl)-5-methyl-2-oxo-2,3-dihydro*-1H-*imidazo[4,5-*b*]pyridin-1-yl)methyl)benzoic acid |
| 169 | 3-((6-chloro-2-oxo-1-(4-(pyrrolidine-1-carbonyl)phenyl)-1,2-dihydro-3*H*-imidazo[4,5-*b*]pyridin-3-yl)methyl)benzoic acid |
| 170 | 3-(1-(5-chloro-2-oxo-3-(4-(2-oxo-2-(pyrrolidin-1-yl)ethyl)phenyl)-2,3-dihydro-*1H*-benzo[d]imidazol-1-yl)cyclopropyl)benzoic acid |
| 171 | 3-(1-(5-chloro-2-oxo-3-(4-(2-oxo-2-(piperidin-1-yl)ethyl)phenyl)-2,3-dihydro-*1H*-benzo[*d*]imidazol-1-yl)cyclopropyl)benzoic acid |
| 172 | 3-(1-(5-chloro-3-(4-(2-morpholino-2-oxoethyl)phenyl)-2-oxo-2,3-dihydro-*1H*-benzo[*d*]imidazol-1-yl)cyclopropyl)benzoic acid |
| 173 | 3-(1-(5-chloro-3-(4-(2-(4,4-difluoropiperidin-1-yl)-2-oxoethyl)phenyl)-2-oxo-2,3-dihydro-*1H*-benzo[*d*]imidazol-1-yl)cyclopropyl)benzoic acid |
| 174 | 3-(1-(5-chloro-3-(4-(2-(3-hydroxyazetidin-1-yl)-2-oxoethyl)phenyl)-2-oxo-2,3-dihydro-*1H*-benzo[*d*]imidazol-1-yl)cyclopropyl)benzoic acid |
| 175 | (*R*)-3-(1-(5-chloro-3-(4-(2-(2-methylmorpholino)-2-oxoethyl)phenyl)-2-oxo-2,3-dihydro-*1H*-benzo[*d*]imidazol-1-yl)cyclopropyl)benzoic acid |
| 176 | (*S*)-3-(1-(5-chloro-3-(4-(2-(2-methylmorpholino)-2-oxoethyl)phenyl)-2-oxo-2,3-dihydro-*1H*-benzo[*d*]imidazol-1-yl)cyclopropyl)benzoic acid |
| 177 | (*S*)-3-(1-(5-chloro-3-(4-(2-(3-methylmorpholino)-2-oxoethyl)phenyl)-2-oxo-2,3-dihydro-*1H*-benzo[*d*]imidazol-1-yl)cyclopropyl)benzoic acid |
| 178 | (R)-3-(1-(5-chloro-3-(4-(2-(3-methylmorpholino)-2-oxoethyl)phenyl)-2-oxo-2,3-dihydro-*1H*-benzo[*d*]imidazol-1-yl)cyclopropyl)benzoic acid |
| 179 | 3-(1-(3-(4-((3r,5r,7r)-adamantan-1-yl)phenyl)-5-chloro-2-oxo-2,3-dihydro-*1H*-benzo[*d*]imidazol-1-yl)cyclopropyl)benzoic |
| | acid |
| 180 | 3-(1-(5-chloro-3-(4-(methylsulfonyl)phenyl)-2-oxo-2,3-dihydro-*1H*-benzo[*d*]imidazol-1-yl)cyclopropyl)benzoic acid |
| 181 | 3-(1-(3-(4-(2-oxa-5-azabicyclo[2.2.1]heptan-5-yl)phenyl)-5-chloro-2-oxo-2,3-dihydro-*1H*-benzo[*d*]imidazol-1-yl)cyclopropyl)benzoic acid |
| 182 | 3-(1-(3-(4-(8-oxa-3-azabicyclo[3.2.1]octan-3-yl)phenyl)-5-chloro-2-oxo-2,3-dihydro-*1H*-benzo[*d*]imidazol-1-yl)cyclopropyl)benzoic acid |
| 183 | 3-(1-(3-(4-(1,4-oxazepan-4-yl)phenyl)-5-chloro-2-oxo-2,3-dihydro-*1H*-benzo[*d*]imidazol-1-yl)cyclopropyl)benzoic acid |
| 184 | 3-(1-(3-(4-(2-azaspiro[3.3]heptan-2-yl)phenyl)-5-chloro-2-oxo-2,3-dihydro-*1H*-benzo[*d*]imidazol-1-yl)cyclopropyl)benzoic acid |
| 185 | 3-(1-(5-chloro-3-(4-(3,3-difluoroazetidin-1-yl)phenyl)-2-oxo-2,3-dihydro-*1H*-benzo[*d*]imidazol-1-yl)cyclopropyl)benzoic acid |
| 186 | 3-(1-(3-(4-(7-acetyl-2,7-diazaspiro[3.5]nonan-2-yl)phenyl)-5-chloro-2-oxo-2,3-dihydro-*1H*-benzo[*d*]imidazol-1-yl)cyclopropyl)benzoic acid |
| 187 | 3-(1-(5-chloro-3-(4-morpholinophenyl)-2-oxo-2,3-dihydro-*1H-*benzo[*d*]imidazol-1-yl)cyclopropyl)-5-fluorobenzoic acid |
| 188 | 3-(1-(5-chloro-3-(4-(dimethylamino)phenyl)-2-oxo-2,3-dihydro-*1H*-benzo[*d*]imidazol-1-yl)cyclopropyl)-5-fluorobenzoic acid |
| 189 | 3-(1-(5-chloro-3-(4-(2-(dimethylamino)-2-oxoethyl)phenyl)-2-oxo-2,3-dihydro-*1H*-benzo[*d*]imidazol-1-yl)cyclopropyl)-5-fluorobenzoic acid |
| 190 | 3-(1-(5-chloro-2-oxo-3-(4-(tetrahydro-2*H*-pyran-4-yl)phenyl)-2,3-dihydro-*1H*-benzo[*d*]imidazol-1-yl)cyclopropyl)-5-fluorobenzoic acid |
| 191 | 3-(1-(3-(4-(2-oxa-6-azaspiro[3.3]heptan-6-yl)phenyl)-5-chloro-2-oxo-2,3-dihydro-*1H*-benzo[*d*]imidazol-1-yl)cyclopropyl)-5-fluorobenzoic acid |
| 192 | 3-(1-(3-(4-(8-oxa-3-azabicyclo[3.2.1]octan-3-yl)phenyl)-5-chloro-2-oxo-2,3-dihydro-*1H*-benzo[*d*]imidazol-1-yl)cyclopropyl)-5-fluorobenzoic acid |
| 193 | 3-(1-(3-(4-(7-oxa-2-azaspiro[3.5]nonan-2-yl)phenyl)-5-chloro-2-oxo-2,3-dihydro-*1H*-benzo[*d*]imidazol-1-yl)cyclopropyl)-5-fluorobenzoic acid |
| 194 | 3-(1-(5-chloro-3-(4-(3,3-difluoroazetidin-1-yl)phenyl)-2-oxo-2,3-dihydro-*1H*-benzo[*d*]imidazol-1-yl)cyclopropyl)-5-fluorobenzoic acid |
| 195 | 3-(1-(3-(4-(7-(*tert*-butoxycarbonyl)-2,7-diazaspiro[3.5]nonan-2-yl)phenyl)-5-chloro-2-oxo-2,3-dihydro-*1H-*benzo[*d*]imidazol-1-yl)cyclopropyl)-5-fluorobenzoic acid |
| 196 | 3-(1-(3-(4-(2-azaspiro[3.3]heptan-2-yl)phenyl)-5-chloro-2-oxo-2,3-dihydro-*1H*-benzo[*d*]imidazol-1-yl)cyclopropyl)-5- |
| | fluorobenzoic acid |
| 197 | 3-(1-(5-chloro-3-(4-(4,4-difluoropiperidin-1-yl)phenyl)-2-oxo-2,3-dihydro-*1H*-benzo[*d*]imidazol-1-yl)cyclopropyl)-5-fluorobenzoic acid |
| 198 | 5-((5-chloro-4-fluoro-3-(4-morpholinophenyl)-2-oxo-2,3-dihydro-*1H*-benzo[*d*]imidazol-1-yl)methyl)isoxazole-3-carboxylic acid |
| 199 | 2-((5-chloro-4-fluoro-3-(4-morpholinophenyl)-2-oxo-2,3-dihydro-*1H*-benzo[*d*]imidazol-1-yl)methyl)oxazole-4-carboxylic acid |
| 200 | 5-((5-chloro-3-(4-morpholinophenyl)-2-oxo-2,3-dihydro-*1H-*benzo[*d*]imidazol-1-yl)methyl)isoxazole-3-carboxylic acid |
| 201 | 2-((5-chloro-3-(4-morpholinophenyl)-2-oxo-2,3-dihydro-*1H-*benzo[*d*]imidazol-1-yl)methyl)oxazole-4-carboxylic acid |
| 202 | 5-((5-chloro-4-fluoro-3-(4-morpholinophenyl)-2-oxo-2,3-dihydro-*1H*-benzo[*d*]imidazol-1-yl)methyl)-2,3-difluorobenzoic acid |
| 203 | 5-((5-chloro-3-(4-morpholinophenyl)-2-oxo-2,3-dihydro-*1H-*benzo[*d*]imidazol-1-yl)methyl)-2,3-difluorobenzoic acid |
| 204 | 3-((5-chloro-4-fluoro-3-(4-morpholinophenyl)-2-oxo-2,3-dihydro-*1*H*-*benzo[*d*]imidazol-1-yl)methyl)-2,6-difluorobenzoic acid |
| 205 | 3-((5-chloro-3-(4-morpholinophenyl)-2-oxo-2,3-dihydro-*1H-*benzo[*d*]imidazol-1-yl)methyl)-2,6-difluorobenzoic acid |
| 206 | 3-(1-(3-(4-(dimethylamino)phenyl)-2-oxo-5-(trifluoromethyl)-2,3-dihydro-*1H*-benzo[*d*]imidazol-1-yl)cyclopropyl)benzoic acid |
| 207 | 3-(1-(3-(4-morpholinophenyl)-2-oxo-5-(trifluoromethyl)-2,3-dihydro-*1H*-benzo[*d*]imidazol-1-yl)cyclopropyl)benzoic acid |
| 208 | 3-(1-(3-(4-(dimethylamino)phenyl)-2-oxo-5-(trifluoromethoxy)-2,3-dihydro-*1H*-benzo[*d*]imidazol-1-yl)cyclopropyl)benzoic acid |
| 209 | 3-(1-(3-(4-morpholinophenyl)-2-oxo-5-(trifluoromethoxy)-2,3-dihydro-*1H*-benzo[*d*]imidazol-1-yl)cyclopropyl)benzoic acid |
| 210 | 3-(1-(5-chloro-3-(4-(*N*,*N*-dimethylsulfamoyl)phenyl)-2-oxo-2,3-dihydro-*1H*-benzo[*d*]imidazol-1-yl)cyclopropyl)benzoic acid |
| 211 | 3-(1-(5-chloro-3-(4-(*N*-cyclopropylsulfamoyl)phenyl)-2-oxo-2,3-dihydro-*1H*-benzo[d]imidazol-1-yl)cyclopropyl)benzoic acid |
| 212 | 3-(1-(5-chloro-3-(4-(morpholinosulfonyl)phenyl)-2-oxo-2,3-dihydro-*1H*-benzo[*d*]imidazol-1-yl)cyclopropyl)benzoic acid |
| 213 | 3-(1-(5-chloro-3-(4-(*N*-(cyclobutylmethyl)sulfamoyl)phenyl)-2-oxo-2,3-dihydro-*1H*-benzo[*d*]imidazol-1-yl)cyclopropyl)benzoic acid |
| 214 | 3-(1-(5-chloro-2-oxo-3-(4-(piperidin-1-ylsulfonylphenyl)-2,3-dihydro-*1H*-benzo[*d*]imidazol-1-yl)cyclopropyl)benzoic acid |
| 215 | 3-(1-(5-chloro-2-oxo-3-(4-(pyrrolidin-1-ylsulfonyl)phenyl)-2,3-dihydro-*1H*-benzo[*d*]imidazol-1-yl)cyclopropyl)benzoic acid |
| 216 | 3-(1-(5-chloro-3-(4-(2-hydroxy-2-methylpropyl)phenyl)-2-oxo-2,3-dihydro-*1H*-benzo[*d*]imidazol-1-yl)cyclopropyl)benzoic acid |
| 217 | 3-(1-(5-chloro-3-(4-(2-fluoro-2-methylpropyl)phenyl)-2-oxo-2,3- |
| | dihydro-*1H*-benzo[*d*]imidazol-1-yl)cyclopropyl)benzoic acid |
| 218 | 3-(1-(5-chloro-3-(4-(1-hydroxy-2-methylpropan-2-yl)phenyl)-2-oxo-2,3-dihydro-*1H*-benzo[*d*]imidazol-1-yl)cyclopropyl)benzoic acid |
| 219 | 3-(1-(5-chloro-3-(4-(1-fluoro-2-methylpropan-2-yl)phenyl)-2-oxo-2,3-dihydro-*1H*-benzo[*d*]imidazol-1-yl)cyclopropyl)benzoic acid |
| 220 | 3-(1-(6-chloro-2-oxo-1-(4-(tetrahydro-2*H*-pyran-4-yl)phenyl)-1,2-dihydro-3*H*-imidazo[4,5-*b*]pyridin-3-yl)cyclopropyl)benzoic acid |
| 221 | 3-(1-(6-chloro-1-(4-(dimethylamino)phenyl)-2-oxo-1,2-dihydro-3*H*-imidazo[4,5-*b*]pyridin-3-yl)cyclopropyl)benzoic acid |
| 222 | 3-(1-(6-chloro-1-(4-morpholinophenyl)-2-oxo-1,2-dihydro-3*H-*imidazo[4,5-*b*]pyridin-3-yl)cyclopropyl)benzoic acid |
| 223 | 3-(1-(6-chloro-1-(4-(dimethylcarbamoyl)phenyl)-2-oxo-1,2-dihydro-3*H*-imidazo[4,5-*b*]pyridin-3-yl)cyclopropyl)benzoic acid |
| 224 | 3-(1-(6-chloro-1-(4-(3-hydroxyazetidine-1-carbonyl)phenyl)-2-oxo-1,2-dihydro-3*H*-imidazo[4,5-*b*]pyridin-3-yl)cyclopropyl)benzoic acid |
| 225 | 3-(1-(1-(4-(7-oxa-2-azaspiro[3.5]nonane-2-carbonyl)phenyl)-6-chloro-2-oxo-1,2-dihydro-3*H*-imidazo[4,5*-b*]pyridin-3-yl)cyclopropyl)benzoic acid |
| 226 | 3-(1-(6-chloro-1-(4-(2-(dimethylamino)-2-oxoethyl)phenyl)-2-oxo-1,2-dihydro-3*H*-imidazo[4,5-*b*]pyridin-3-yl)cyclopropyl)benzoic acid |
| 227 | 3-(1-(6-chloro-1-(4-(methylsulfonyl)phenyl)-2-oxo-1,2-dihydro-3*H*-imidazo[4,5-*b*]pyridin-3-yl)cyclopropyl)benzoic acid |
| 228 | 3-(1-(6-chloro-2-oxo-1-(4-(tetrahydro-2*H*-pyran-4-yl)phenyl)-1,2-dihydro-3*H*-imidazo[4,5-*b*]pyridin-3-yl)cyclopropyl)-2-fluorobenzoic acid |
| 229 | 3-(1-(6-chloro-1-(4-(dimethylamino)phenyl)-2-oxo-1,2-dihydro-3*H*-imidazo[4,5-*b*]pyridin-3-yl)cyclopropyl)-2-fluorobenzoic acid |
| 230 | 3-(1-(6-chloro-1-(4-(morpholinophenyl)-2-oxo-1,2-dihydro-3*H-*imidazo[4,5-*b*]pyridin-3-yl)cyclopropyl)-2-fluorobenzoic acid |
| 231 | 3-(1-(6-chloro-2-oxo-1-(4-(pyrrolidine-1-carbonyl)phenyl)-1,2-dihydro-3*H*-imidazo[4,5-*b*]pyridin-3-yl)cyclopropyl)-2-fluorobenzoic acid |
| 232 | 3-(1-(6-chloro-1-(4-(3-hydroxyazetidine-1-carbonyl)phenyl)-2-oxo-1,2-dihydro-3*H*-imidazo[4,5-*b*]pyridin-3-yl)cyclopropyl)-2-fluorobenzoic acid |
| 233 | 3-(1-(1-(4-(2-oxa-6-azaspiro[3.3]heptane-6-carbonyl)phenyl)-6-chloro-2-oxo-1,2-dihydro-3*H*-imidazo[4,5-*b*]pyridin-3-yl)cyclopropyl)-2-fluorobenzoic acid |
| 234 | 3-(1-(5-chloro-2-oxo-3-(4-(1-propionylpiperidin-4-yl)phenyl)-2,3-dihydro-*1H*-benzo[*d*]imidazol-1-yl)cyclopropyl)benzoic acid |
| 235 | 3-(1-(5-chloro-2-oxo-3-(4-(1-propionylpiperidin-4-yl)phenyl)-2,3-dihydro-*1H*-benzo[*d*]imidazol-1-yl)cyclopropyl)-5-fluorobenzoic acid |
| 236 | 3-(1-(5-chloro-2-oxo-3-(4-((2-oxopyrrolidin-1-yl)methyl)phenyl)-2,3-dihydro-*1H*-benzo[*d*]imidazol-1-yl)cyclopropyl)benzoic acid |
| 237 | 3-(1-(5-chloro-2-oxo-3-(4-((4-(trifluoromethyl)-*1H*-imidazol-1-yl)methyl)phenyl)-2,3-dihydro-*1H*-benzo[*d*]imidazol-1-yl)cyclopropyl)benzoic acid |
| 238 | 3-(1-(5-chloro-3-(4-(1-(hydroxymethyl)cyclopropyl)phenyl)-2-oxo-2,3-dihydro-*1H*-benzo[*d*]imidazol-1-yl)cyclopropyl)benzoic acid |
| 239 | 3-(1-(5-chloro-3-(4-(1-(hydroxymethyl)cyclobutyl)phenyl)-2-oxo-2,3-dihydro-*1H*-benzo[*d*]imidazol-1-yl)cyclopropyl)benzoic acid |
| 240 | 3-(1-(5-chloro-3-(4-(oxetan-3-ylmethyl)phenyl)-2-oxo-2,3-dihydro-*1H*-benzo[*d*]imidazol-1-yl)cyclopropyl)benzoic acid |
| 241 | 3-(1-(5-chloro-3-(4-((3-methyl-2-oxo-imidazolin-1-yl)methyl)phenyl)-2-oxo-2,3-dihydro-*1H*-benzo[*d*]imidazol-1-yl)cyclopropyl)benzoic acid |
| 242 | 3-(1-(5-chloro-3-(4-(4,4-dimethyl-2-oxo-oxazolidin-3-yl)phenyl)-2-oxo-2,3-dihydro-*1H*-benzo[*d*]imidazol-1-yl)cyclopropyl)benzoic acid |
| 243 | 3-(1-(5-chloro-2-oxo-3-(4-(5-oxo-6-oxa-4-azaspiro[2.4]heptan-4-yl)phenyl)-2,3-dihydro-*1H*-benzo[*d*]imidazol-1-yl)cyclopropyl)benzoic acid |
| 244 | 3-(1-(5-chloro-2-oxo-3-(4-((3-(trifluoromethyl)-*1H*-pyrazol-1-yl)methyl)phenyl)-2,3-dihydro-*1H*-benzo[*d*]imidazol-1-yl)cyclopropyl)benzoic acid |
| 245 | 3-(1-(5-chloro-3-(2-methyl-1-oxoisoindolin-5-yl)-2-oxo-2,3-dihydro-*1H*-benzo[*d*]imidazol-1-yl)cyclopropyl)benzoic acid |
| 246 | 3-(1-(5-chloro-3-(4-(2-hydroxypropan-2-yl)phenyl)-2-oxo-2,3-dihydro-*1H*-benzo[*d*]imidazol-1-yl)cyclopropyl)benzoic acid |
| 247 | 3-(1-(5-chloro-3-(4-(3-methyloxetan-3-yl)phenyl)-2-oxo-2,3-dihydro-*1H*-benzo[*d*]imidazol-1-yl)cyclopropyl)benzoic acid |
| 248 | 3-(1-(5-chloro-2-oxo-3-(4-(tetrahydrofuran-3-yl)phenyl)-2,3-dihydro-*1H*-benzo[*d*]imidazol-1-yl)cyclopropyl)benzoic acid |
| 249 | 3-(1-(5-chloro-2-oxo-3-(4-(tetrahydrofuran-3-yl)phenyl)-2,3-dihydro-*1H*-benzo[*d*]imidazol-1-yl)cyclopropyl)-5-fluorobenzoic acid |
| 250 | 3-(1-(5-chloro-2-oxo-3-(4-(tetrahydrofuran-3-yl)phenyl)-2,3-dihydro-*1H*-benzo[*d*]imidazol-1-yl)cyclopropyl)-2-fluorobenzoic acid |
| 251 | 3-(1-(5-chloro-3-(1,1-dimethyl-1,3-dihydroisobenzofuran-5-yl)-2-oxo-2,3-dihydro-*1H*-benzo[*d*]imidazol-1-yl)cyclopropyl)benzoic acid |
| 252 | 3-(1-(5-chloro-3-(1,1-dimethyl-1,3-dihydroisobenzofuran-5-yl)-2-oxo-2,3-dihydro-*1H*-benzo[*d*]imidazol-1-yl)cyclopropyl)-5-fluorobenzoic acid |
| 253 | 3-(1-(5-chloro-3-(1,1-dimethyl-1,3-dihydroisobenzofuran-5-yl)-2-oxo-2,3-dihydro-*1H*-benzo[*d*]imidazol-1-yl)cyclopropyl)-2-fluorobenzoic acid |
| 254 | 3-(1-(5-chloro-2-oxo-3-(3-oxo-3,4-dihydro-2*H-*benzo[*b*][1,4]oxazin-7-yl)-2,3-dihydro-*1H-*benzo[*d*]imidazol-1-yl)cyclopropyl)-5-fluorobenzoic acid |
| 255 | 3-(1-(5-chloro-3-(4-methyl-3,4-dihydro-2*H*-benzo[*b*][1,4]oxazin-7-yl)-2-oxo-2,3-dihydro-*1H*-benzo[*d*]imidazol-1-yl)cyclopropyl)-5-fluorobenzoic acid |
| 256 | 3-(1-(5-chloro-3-(4-(1-hydroxycyclobutyl)phenyl)-2-oxo-2,3-dihydro-*1H*-benzo[*d*]imidazol-1-yl)cyclopropyl)benzoic acid |
| 257 | 3-(1-(5-chloro-3-(4-(1-hydroxycyclobutyl)phenyl)-2-oxo-2,3-dihydro-*1H*-benzo[*d*]imidazol-1-yl)cyclopropyl)-5-fluorobenzoic acid |
| 258 | 3-(1-(5-chloro-3-(4-(3-hydroxyoxetan-3-yl)phenyl)-2-oxo-2,3-dihydro-*1H*-benzo[*d*]imidazol-1-yl)cyclopropyl)benzoic acid |
| 259 | 3-((5-chloro-3-(4-(1-(oxetan-3-yl)piperidin-4-yl)phenyl)-2-oxo-2,3-dihydro-*1H*-benzo[*d*]imidazol-1-yl)methyl)benzoic acid |
| 260 | 3-(1-(5-chloro-3-(4-(1-(oxetan-3-yl)piperidin-4-yl)phenyl)-2-oxo-2,3-dihydro-*1H*-benzo[*d*]imidazol-1-yl)cyclopropyl)benzoic acid |
| 261 | 3-(1-(5-chloro-2-oxo-3-(6-(tetrahydro-2*H*-pyran-4-yl)pyridin-3-yl)-2,3-dihydro-*1H*-benzo[*d*]imidazol-1-yl)cyclopropyl)benzoic acid |
| 262 | 3-(1-(5-chloro-2-oxo-3-(6-(tetrahydro-2*H*-pyran-4-yl)pyridin-3-yl)-2,3-dihydro-*1H*-benzo[*d*]imidazol-1-yl)cyclopropyl)-5-fluorobenzoic acid |
| 263 | 3-(1-(5-chloro-3-(4-(5-cyclopropyl-1,3,4-oxadiazol-2-yl)phenyl)-2-oxo-2,3-dihydro-*1H*-benzo[*d*]imidazol-1-yl)cyclopropyl)benzoic acid |
| 264 | 3-(1-(5-chloro-3-(4-(5-cycloheptyl-1,3,4-oxadiazol-2-yl)phenyl)-2-oxo-2,3-dihydro-*1H*-benzo[*d*]imidazol-1-yl)cyclopropyl)benzoic acid |
| 265 | 3-(1-(5-chloro-3-(4-(5-cyclohexyl-1,3,4-oxadiazol-2-yl)phenyl)-2-oxo-2,3-dihydro-*1H*-benzo[*d*]imidazol-1-yl)cyclopropyl)benzoic acid |
| 266 | 3-(1-(5-chloro-3-(4-(5-cyclopentyl-1,3,4-oxadiazol-2-yl)phenyl)-2-oxo-2,3-dihydro-*1H*-benzo[*d*]imidazol-1-yl)cyclopropyl)benzoic acid |
| 267 | 3-(1-(5-chloro-2-oxo-3-(4-(5-(tetrahydro-2*H*-pyran-4-yl)-1,3,4-oxadiazol-2-yl)phenyl)-2,3-dihydro-*1H*-benzo[*d*]imidazol-1-yl)cyclopropyl)benzoic acid |
| 268 | 3-(1-(5-chloro-2-oxo-3-(4-(5-(tetrahydro*-2H-*pyran-4-yl)-1,3,4-oxadiazol-2-yl)phenyl)-2,3-dihydro-*1H*-benzo[*d*]imidazol-1-yl)cyclopropyl)-2-fluorobenzoic acid |
| 269 | 3-(1-(5-chloro-2-oxo-3-(4-(1-propyl-*1H*-pyrazol-4-yl)phenyl)-2,3-dihydro-*1H*-benzo[*d*]imidazol-1-yl)cyclopropyl)benzoic acid |
| 270 | 3-(1-(6-chloro-2-oxo-1-(4-(1-propyl-*1H*-pyrazol-4-yl)phenyl)-1,2-dihydro-*3H*-imidazo[4,5-*b*]pyridin-3-yl)cyclopropyl)benzoic acid |
| 271 | 3-(1-(6-chloro-1-(4-(dimethylamino)phenyl)-2-oxo-1,2-dihydro-3*H*-imidazo[4,5-*b*]pyridin-3-yl)cyclopropyl)-5-fluorobenzoic acid |
| 272 | 3-(1-(6-chloro-1-(4-(dimethylcarbamoyl)phenyl)-2-oxo-1,2-dihydro-3H-imidazo[4,5-*b*]pyridin-3-yl)cyclopropyl)-5-fluorobenzoic acid |
| 273 | 3-(1-(6-chloro-2-oxo-1-(4-(tetrahydro-2*H*-pyran-4-yl)phenyl)-1,2-dihydro-3*H*-imidazo[4,5-*b*]pyridin-3-yl)cyclopropyl)-5-fluorobenzoic acid |
| 274 | 3-(1-(6-chloro-1-(4-morpholinophenyl)-2-oxo-1,2-dihydro-3*H-*imidazo[4,5-*b*]pyridin-3-yl)cyclopropyl)-5-fluorobenzoic acid |
| 275 | 3-(1-(1-(4-(7-oxa-2-azaspiro[3.5]nonane-2-carbonyl)phenyl)-6-chloro-2-oxo-1,2-dihydro-3*H*-imidazo[4,5-*b*]pyridin-3-yl)cyclopropyl)-5-fluorobenzoic acid |
| 276 | 3-(1-(6-chloro-2-oxo-1-(4-(pyrrolidine-1-carbonyl)phenyl)-1,2-dihydro-3*H*-imidazo[4,5-*b*]pyridin-3-yl)cyclopropyl)-5-fluorobenzoic acid |

8. A pharmaceutical composition comprising: the compound according to any one of claims 1 to 7 or a pharmaceutically acceptable salt thereof; and a pharmaceutically acceptable carrier.

9. The pharmaceutical composition of claim 8, wherein the pharmaceutical composition is for use in the prevention or treatment of an autotaxin-mediated disease.

10. The pharmaceutical composition of claim 9, wherein the autotaxin-mediated disease is a disease caused by the overexpression or overactivation of autotaxin.

11. The pharmaceutical composition of claim 10, wherein the autotaxin-mediated disease is cancer or fibrosis.
